# EUROPEAN PATENT APPLICATION

(11) **EP 4 692 375 A2**
(43) Date of publication of application: **11.02.2026**
(21) Application number: 25218814.9
(22) Date of filing: 16.01.2020
(51) Int. Cl.: C12Q 1/6886

(54) **BIOMARKERS FOR RENAL CELL CARCINOMA**

(30) Priority: 16.01.2019 EP 19152126
(62) Divisional of application: 20701548.8
(71) Applicant: Biorek S.r.l., 20123 Milano (IT)
(72) Inventor: Trevisani, Francesco, 20123 Milano (IT); Cinque, Alessandra, 20123 Milano (IT); Larcher, Alessandro, 20123 Milano (IT); Rampoldi, Luca, 20123 Milano (IT); Fichera, Domenico, 20123 Milano (IT); Ripa, Francesco, 20123 Milano (IT)
(74) Representative: Currado, Luisa

(57) **Abstract**

The present invention relates to methods for the screening, diagnosis and/or prognosis of renal cell carcinoma. The methods of the invention are based on the determination of expression profiles of sets of miRNAs representative for renal cell carcinoma optionally associated with genotype analysis, in particular of uromodulin SNP variants. The invention also refers to a kit to perform such methods.

## Description

### TECHNICAL FIELD

The present invention relates to methods for the screening, diagnosis and/or prognosis of renal cell carcinoma. In particular, the methods of the invention allow (i) to predict the presence of a renal mass; (ii) to predict if such renal mass is benign or malignant, (iii) to predict its aggressiveness status and the clinical severity of RCC (stage, grade, and metastasis); (iv) to monitor the progression or regression of RCC in a subject (eg in "watchful waiting" before treatment or surgery or during an oncological therapy to evaluate the effect of the treatment), (v) to monitor the occurrence of RCC in a subject at risk (obese patients, autosomal dominant polycystic kidney disease patients, diabetes, etc), (vi) to detect the recurrence of RCC (eg after partial nephrectomy in the remnant part of the operated kidney or after radical nephrectomy in the contralateral organ or after any type of surgery in other distant organs for metastatic patients), (vii) to distinguish RCC from other renal functional diseases or other malignant diseases (eg, infections, inflammatory or fibrotic systemic diseases or other aetiology of tumors which involve the kidney, eg sarcoma, urothelial cancer, etc).

The methods of the invention are based on the determination of expression profiles of sets of miRNAs representative for renal cell carcinoma compared to a reference and it may be associated with genotype analysis, in particular of uromodulin SNP variants. The invention also refers to a kit to perform such methods.

### BACKGROUND ART

### Renal Cell Carcinoma

### Epidemiology

Renal cell carcinoma (RCC) in adults comprehends a heterogeneous group of tumours which derive from renal tubular epithelial cells and represents the most common type of kidney cancer (80%) [Znaor et al., 2015; Chow et al., 2010; Siegel et al., 2017].

Every year more than 350,000 patients have a new diagnosis worldwide, which made it the seventh most common site for tumours [Pantucka et al., 2001]. This cancer is associated with more than 140,000 deaths per year; only in Europe, it was estimated an incidence of 84,400 new cases of RCC and 34,700 kidney cancer-related deaths [Siemer et al., 2006]. RCC has a male-female ratio of 2:17 and occurs most commonly in the fifth to sixth decade, with cancer-specific mortality extremely heterogeneous according to host characteristics [Thompson et al., 2008]. The incidence of RCC has increased since the 1980s by an average of 3% per year, largely related to incidental detection secondary to the more prevalent use of ultrasonography and CT scan [Adams et al., 2008]. The most generally accepted risk factor for RCC is cigarette smoking, although the relative associated risks have been modest, ranging from 1.4 to 2.5 compared with controls [Cumberbatch et al., 2016]. Other established risk factors for RCC include excess body weight and hypertension [Lipworth et al., 2006]. Medical conditions that have been associated with RCC in epidemiological studies include chronic kidney disease, haemodialysis, kidney transplantation, acquired kidney cystic disease, a previous RCC diagnosis and, possibly, diabetes mellitus [Dellavalle et al., 2013].

### Histological classification

Renal cell carcinoma (RCC) classification is based on cytogenetic, genetic and histological studies on both familiar and sporadic tumors [Pantucka et al., 2001]. According to the World Health Organization there are three major histological subtypes of RCC, all differentiated by histological and molecular genetic changes: clear cell (70-80%), papillary (10-15%) and chromophobe (4-5%). The remaining 10-15% of renal tumors include a variety of uncommon, sporadic, familiar carcinomas, and a group of unclassified carcinomas [Cumberbatch et al., 2016].

Among the minor histological subtypes there are oncocytoma and angiomyolipoma which represent renal tumours with a benign prognosis [Ljungberg B et al. , 2016].

Clear cell renal cell carcinoma (ccRCC) represents the most common type of RCC. Most ccRCC are composed predominantly of cells containing clear cytoplasm, although eosinophilic cytoplasm predominates in some cells. The growth pattern may be solid, tubular and cystic. Grossly, ccRCC is well circumscribed, capsule is usually absent [D'Avella C et al., 2018]. The cut surface is golden-yellow, often with haemorrhage and necrosis. The indolent variant of ccRCC is multilocular cystic and accounts for approximately 4% of all ccRCC. Up to 95% of ccRCC are sporadic. The most common genetic alteration reported is loss of sequence in the short arm of chromosome 3 [Bex a et al., 2018] . This happens because of a deletion (3p-) or because of a chromosomic translocation (3;6, 3;8, 3;11) that results in the loss of chromosome 3 sequences from 3p12 to 3p26. This is the region where VHL gene is located (3p25.3). Other associated molecular changes may be duplication of the chromosome band 5q22, deletion of chromosome 6q, 8p, 9p and 14q. Sequencing studies have identified other driver genes that are involved in the pathogenesis of renal cell carcinoma, including PBRM1, BAP1, SETD2, TCEB1 and KDM5C32,33. Studies analyzing the effects of these mutations on clinical outcomes, however, are limited by small sample sizes, short-term follow-up, and lack of assessment of multiple markers in the same cohort [Lalani AA, et al., 2018].

Papillary renal cell carcinoma (pRCC) comprehends up to 10-15% of all renal cell carcinomas. Macroscopically, pRCC is well circumscribed with pseudocapsule, yellow or brown in color, and a soft structure. Its growth pattern is typically papillary and it can arise both in a sporadic or familiar way. Pseudocapsules and extensive necrotic changes cause a spherical tumor in the extra renal section [BhindiB et al., 2018]. Tumors with massive necrosis are fragile and vulnerable to spontaneous rupture or rupture resulting from minimal trauma followed by retroperitoneal bleeding. Necrosis cohere with a hypodense central area of tumor on postcontrast CT. This area is surrounded by a vital tumor tissue, seen as a serpiginous contrast-enhancing margin on CT. Papillary renal cell carcinoma is not associated with deletion of chromosome 3p. The most common cytogenetic abnormalities found are trisomies of chromosomes 3q, 7, 8, 12, 16, 17, the loss of Y chromosome in men within the sporadic form and the trisomy of chromosome 7 within the familiar one. Trisomy of chromosome 7, which contains both MET and HGF/SF genes, occurs in 95% of sporadic papillary renal carcinoma. Unlike clear cell RCC, papillary RCC is frequently multifocal from its origin [Wagener N et al., 2018]. Papillary RCCs can be divided into type 1 and type 2 lesions based upon histopathologic features. Type 1 and type 2 papillary carcinomas differ in both clinical features and in their underlying genetic abnormalities:
- Type 1 papillary RCC (p1RCC): p1RCC typically presents with stage I or II disease, and these patients have a relatively favourable prognosis. Although type 1 lesions usually occur in patients with hereditary papillary RCC, most these reports are sporadic. In the hereditary form this disease, activating germline mutations are seen in MET. In non-hereditary forms of the disease, somatic mutations in MET have been identified in approximately 10 to 20 percent of cases. Type 1 tumors are papillary lesions covered by small cells with pale cytoplasm and small oval nuclei with indistinct nucleoli [Pal SK et al., 2018].
- Type 2 papillary RCC (p2RCC): p2RCC is frequently associated with aggressive tumors that are stage III or IV at presentation and are associated with a poor prognosis. These tumors have also been seen in the hereditary leiomyomatosis and renal cell cancer syndrome, which is caused by germline mutation in the gene for fumarate hydratase (FH). Very few patients had alterations in the MET pathway. Type 2 tumors are papillary lesions covered by large cells with abundant eosinophilic cytoplasm. Type 2 cells are typified by pseudo stratification and large, spherical nuclei with distinct nucleoli [Pal SK et al., 2018].

Chromophobe (chRCC): The chromophobe variant of renal cell carcinoma also accounts for 4 percent of all cases of renal cell carcinoma and may have a benign course after surgery, provided that the tumor stage and grade are favorable. The cells of chRCC may have pail or eosinophilic granular cytoplasm. Growth usually occurs in solid sheets. The genetic characteristic of chromophobe RCC is a combination of loss of chromosomes 1, 2, 6, 10, 13 and 17. It is thought to arise from type B intercalated cells of the collecting ducts [Leibovich BC et al, 2018].

Oncocytoma: Oncocytomas, which are benign, account for about 4 percent of nephrectomies performed because renal cell carcinoma is suspected. Oncocytoma is thought to originate from type A intercalated cells of the collecting duct [Valdés Olmos RA et al., 2016].

### Diagnosis

The major clinical obstacles in RCC diagnosis and therapy are the unspecific symptoms and the absence of predictive biomarkers for RCC [Heidenreich et al., 2004]. In many cases, RCC remains clinically silent, as the simultaneous presence of haematuria, pain and palpable mass at the side, considered the classic presentation triad, is actually very rare (1%) [Ljungberg et al., 2014]. Symptoms of systemic disease, such as persistent cough, bone pain, cervical lymphadenopathy, constitutional symptoms, weight loss, fever, malaise, lead to a diagnosis in up to 30% of metastatic patients [Hollingsworth et al., 2007].

Historically, survival outcomes for patients with advanced disease have been poor even with oncological treatment. On the contrary, if renal cancer is detected at early stages, surgical treatment remains the unique resolutive therapy with a free survival from metastatic disease around 95% in five years. [Hollingsworth et al., 2007].

RCC is detected mostly by abdominal ultrasound scanning by chance in day to day clinical practice; however, due to its limitations in specificity and accuracy, only CT scan and MRI technologies represent the gold standard tools for renal masses identification [Kutikov et al., 2009]. Unfortunately, both techniques are still unable to distinguish the presence of a benign lesion (e.g. oncocytoma and fat-free angiomyolipoma) from a malignant one (RCC) and this misleading error leads every year up to 20% of unnecessary surgical operations [Linehan et al., 2007]. Percutaneous renal tumor biopsies are increasingly being used for histological diagnosis when the radiological exams are confused and not able to identify clearly a malignant neoplasm; however, due to RCC heterogenicity, renal biopsies do not determine the aggressiveness of cancer [Richard et al., 2015].

Therefore, there is still the need for clinical and molecular strategies capable of stratifying the population according to the risk of developing RCC.

### Staging and grading

RCC tends to spread either by direct invasion through the renal capsule into perinephric fat and adjacent visceral structures or by direct extension into the renal vein. The most common site of distant metastases is the lung [Taneja K et al., 2018] However, liver, bone, adrenal gland, brain, the contralateral kidney and the subcutaneous tissue are potential sites of disease spread. The current International Union Against Cancer (UICC) 2017 TNM (Tumour Node Metastasis) classification is recommended for the staging of RCC (Table 1), where stages I-II represent low risk tumours whereas stages III-IV are high risk neoplasm with a considerable metastatic power dissemination [Brookman-May SD et al.,2015] .

**Table 1. TNM classification system (2017).**

| **T** - **Primary tumor** | | | | |
|---|---|---|---|---|
| TX | Primary tumor cannot be assessed | | | |
| T0 | No evidence of primary tumor | | | |
| T1 | Tumor < 7 cm in greatest dimension, limited to the kidney | | | |
| | T1a | Tumor ≤ 4 cm in greatest dimension, limited to the kidney | | |
| | T1b | Tumor > 4 cm but ≤ 7 cm in greatest dimension | | |
| T2 | | Tumor > 7 cm in greatest dimension, limited to the kidney | | |
| | | T2a Tumor > 7 cm but ≤ 10 cm in greatest dimension, limited to the kidney | | |
| | | T2b Tumors > 10 cm, limited to the kidney | | |
| T3 | Tumor extends into major veins or perinephric tissues, but not into the ipsilateral adrenal gland and not beyond Gerota's fascia | | | |
| | | T3a Tumor extends into the renal vein or its segmental branches, or invades the pelvicalyceal system, or invades perirenal and/or renal sinus fat but not beyond Gerota's fascia | | |
| | | T3b Tumor extends into the vena cava below the diaphragm | | |
| | | T3c Tumor extends into the vena cava above the diaphragm or invades the wall of the vena cava | | |
| T4 | Tumor invades beyond Gerota's fascia (including contiguous extension into the ipsilateral adrenal gland) | | | |

| **N - Regional lymph nodes** | | | | |
|---|---|---|---|---|
| NX | Regional lymph nodes cannot be assessed | | | |
| N0 | No regional lymph node metastasis | | | |
| N1 | Metastasis in regional lymph node(s) | | | |

| **M - Distant metastasis** | | | | |
|---|---|---|---|---|
| M0 | No distant metastasis | | | |
| M1 | Distant metastasis | | | |

| **TNM stage grouping** | | | | |
|---|---|---|---|---|
| Stage I | | T1 | N0 M0 | |
| Stage II | | T2 | N0 M0 | |
| Stage III | | T1, T2 N1 | | M0 |
| | | T3 Any N | | M0 |
| Stage IV | | T4 Any N | | M0 |
| | | Any T Any | N M1 | |

The Fuhrman system was the most frequently used grading system in RCC but should not be applied for chromophobe RCC [Zhang G et al., 2018]. Furthermore, the Fuhrman system has not been validated for most of the new subtypes of renal carcinoma. For these reasons, the four-tiered WHO/ISUP grading system is recommended by the WHO. For grade 1-3 tumours, the system defines tumour grade based on nucleolar prominence. Grade 4 is defined by the presence of pronounced nuclear pleomorphism, tumour giant cells, and/or rhabdoid and/or sarcomatoid differentiation [Nini et al., 2018]. This grading system has been validated for ccRCC and papillary RCC. It has not yet been validated for other tumour types because of the small numbers of reported cases.

### Treatment

Surgery remains the basis of the curative treatment, despite advances in understanding the biology of RCC. Historically radical nephrectomy was the standard of care for management of renal tumours but the developing of chronic kidney disease and hypertension after the acute nephron mass loss in several patients have led to a more conservative approach with nephron sparing technique [Capitanio et al., 2015].

### Uromodulin

Tamm-Horsfall protein was discovered by Igor Tamm and Frank Horsfall in 1950 when they precipitated a protein from the urine that inhibited hemagglutination of viruses [Pennica et al., 1987]. This glycoprotein is exclusively expressed in the kidney, specifically by cells lining the thick ascending limb of the nephron [Muchmore et al., 1985]. The domain composition of uromodulin includes a leader peptide directing its entry in the secretory pathway, four epidermal growth factor (EGF)-like domains (EGF-II and EGF-III predicted to be calcium binding), a central domain of unknown function (named D8C as it contains eight conserved cysteines), a zona pellucida domain, and a glycosylphosphatidylinositol-anchoring site [Rampoldi et al., 2012].

Given its abundance, unique structure and evolutionary conservation through all vertebrate species, a number of physiological and pathological roles have been hypothesized for uromodulin [Bachmann et al., 1985]. These include its role in defence against urinary tract infection, prevention of kidney stone formation, modulation of salt and water retention, hypertension, and more recently its possible role in inflammation and immunomodulation during acute kidney injury as well as in the pathogenesis of CKD [El-achkar et al., 2012].

The potential link between uromodulin and kidney function has been emphasized by some innovative studies that investigated for the first time the role of UMOD variants in regulating UMOD excretion in urine and the possibility that this could affect tubular function and progressive chronic kidney disease [Rampoldi et al., 2003]. Later, several genome-wide association studies revealed that variants in the UMOD gene are strongly associated with markers of renal function and risk of developing CKD in the general population [Bleyer et al., 2010; Trudu et al., 2014]. The biological role of uromodulin in the background of renal cancer occurrence, onset and development has not been clarified yet. The first studies in this field demonstrated the presence of uromodulin in samples of renal cell carcinomas [Kottgen et al., 2010]. Recently, there has been a growing interest in applying proteomics approaches in research on clinical diagnostics and predictive medicine, and uromodulin was one of the investigated proteins in the context of renal cell carcinoma [Eikrem et al., 2016]. The main proteomic goals are to detect quantitative and qualitative differences between normal and pathological samples and to identify these differentially expressed proteins [Eikrem et al., 2016]. The possible role of uromodulin as a specific biomarker of RCC in biological fluids such as urine remains uncertain and demands further investigation.

### MicroRNAs

In biology, miRNAs are epigenetic regulators of gene expression able to modulate several cellular processes, from development to disease condition [Krol et al., 2010]. The process of epigenetic regulation is accomplished through binding of miRNAs to their target mRNAs by base-pairing and subsequently inducing either translational repression or mRNA destabilization [Lim et al., 2005]. The human miRNAome is composed of 1,881 precursors and 2,588 mature miRNAs, which regulate at least 60% of protein-coding genes [Bracken et al., 2016]. Because of their broader targeting characteristics, miRNAs are expected to be involved in most biological pathways and cellular processes including cell proliferation, apoptosis, cellular development and cellular signalling [Zhu et al., 2016]. Since 2010, the number of miRNAs included in miRBase has grown by approximately two-thirds owing to the advent of small RNA deep sequencing techniques[Lorenzen et al., 2012].

The majority of miRNAs are located within the tissue's cell, but some miRNAs, commonly known as circulating miRNAs or extracellular miRNAs, have also been found in extracellular environment, including various biological fluids and cell culture media [Trevisani et al., 2016]. Transcription of miRNAs occurs from individual or clustered genes, although some miRNAs can be encoded from distinct genomic loci. Genes encoding miRNAs are located in non-coding sequences or in introns of either protein-coding genes (miRtrons) or non-coding RNA [Lytle et al., 2007]. Intronic miRNAs are usually co-ordinately expressed with their host gene and most of the time they both affect the same signalling pathway [Baek, D. et al, 2008]. RNA polymerase II transcribes miRNAs in the nucleus as long capped and polyadenylated hairpin transcripts, called primary miRNAs (pri-miRNAs) [Eichhorn et al., 2004]. These are processed into smaller ~70 nucleotide stem-looped structures, called precursor miRNAs (pre-miRNAs) by the ribonuclease III-like enzyme Drosha together with the microprocessor complex subunit DGCR8. Pre-miRNAs are then exported to the cytoplasm by exportin-5/GTP-binding nuclear protein Ran, where the ribonuclease Dicer yields 22 nucleotide miRNA duplexes consisting of the guide and passenger strands [Acunzo et al., 2015]. The guide strands are finally assembled into the RNA-induced silencing complex (RISC) and bind through their 'seed sequence' (nucleotides 2-8) to fully or partially complementary sites within the 3' untranslated region of target mRNAs [Yamaguchi et al., 2017].

MiRNAs also have a key role as regulators of cellular crosstalk. They can be actively secreted into the extracellular microenvironment or into body fluids and captured by other cells, thus altering their transcriptional programme [Lampis et al., 2018]. In addition, circulating miRNAs can be derived from apoptotic cells, with the likely purpose of carrying alarm signals from apoptotic cells to other cells [Calin et al., 2004]. miRNAs circulate in blood, urine and other body fluids either packaged into microvesicles and/or exosomes or transported by RNA binding proteins (Argonaute2 complexes) and lipoproteins, which protect them from degradation by ribonucleases [Trionfini et al., 2015]. Given their ease of access, abundance, and stability, circulating miRNAs are novel promising minimally-invasive biomarkers for diagnosis and prognosis of plenty kinds of diseases, such as cancer [Mitchell et al., 2008]. Indeed, there is increasing evidence that miRNAs are involved in oncogenesis and tumour progression [lorio et al., 2012]. In addition, miRNA signatures in blood are similar in men and women, and independent of patients' age [Mooney et al., 2015; Hunter et al., 2008]. Specific expression profiles of miRNAs have been shown in a variety of cancers, including RCC [Weng et al., 2010; Schaefer et al., 2010]. More importantly, miRNAs are thought to be excellent biomarkers not only for the diagnosis, but also for the prognosis and the classification of cancer [Schaefer et al., 2010].Differences in the miRNA profiles of tumours versus adjacent renal tissues have been extensively explored [Tang et al., 2015; Li et al., 2015]. However, not always miRNAs deregulated in tissue are also deregulated in blood and vice versa [Keller et al., 2011]. Recently, few studies have assessed circulating levels of miRNA in the blood of RCC patients, but no consistent circulating miRNA signatures of renal cancer have emerged. Authors have often used a candidate miRNA approaches for their investigations [Zhao et al., 2013; Zhang et al., 2013; Zhai et al., 2012; Iwamoto et al., 2014; Hauser et al., 2012; Fedorko et al., 2015; Teixeira et al., 2014] and in the four genome-wide studies performed to date there was no obvious overlap between the miRNA expression profiles obtained [Chanud et et al., 2017; Wulfken et al., 2011;Redova et al., 2012; Wang et al.,2015].Variability in plasma/serum expression levels of miRNAs in different works could be due to several critical factors: sample collection and processing (es. serum samples could present an altered miRNAs composition due to the coagulation process [Wang et al., 2012]), RNA extraction, and normalization methods. In addition, in three out of four of the genome-wide studies mentioned before, screening was limited to small numbers of samples (6, 15 and 25 RCC patients respectively) [Wulfken et al., 2011;Redovaet al.,2012; Wang et al., 2015].

Consequently, lager and deeper research are needed in the novel field of circulating miRNAs in renal cancer.

### SUMMARY OF THE INVENTION

In the present invention, the inventors surprisingly found an association of UMOD SNP variants with renal mass presence, malignancy, and aggressiveness in patients. Further specific plasma-circulating miRNAs were found to discriminate renal cancer patients from controls, to discriminate patients with a malignant lesion from those with a benign mass, to determine aggressiveness of RCC.

UMOD genotype may be combined with miRNA profile and optionally together with clinical parameters to provide a screening test and to improve the accuracy and specificity of diagnosis, prognosis, and classification of renal cancer.

In particular, the methods of the invention allow (i) to predict the presence of a renal mass; (ii) to predict if such renal mass is benign or malignant, (iii) to predict its aggressiveness status and the clinical severity of RCC (stage, grade, and metastasis); (iv) to monitor the progression or regression of RCC in a subject (eg in "watchful waiting" before treatment or surgery or during an oncological therapy to evaluate the effect of the treatment), (v) to monitor the occurrence of RCC in a subject at risk (obese patients, autosomal dominant polycystic kidney disease patients, diabetes, etc), (vi) to detect the recurrence of RCC (eg after partial nephrectomy in the remnant part of the operated kidney or after radical nephrectomy in the contralateral organ or after any type of surgery in other distant organs for metastatic patients), (vii) to distinguish RCC from other renal functional diseases or other malignant diseases (eg, infections, inflammatory or fibrotic systemic diseases or other aetiology of tumors which involve the kidney, eg sarcoma, urothelial cancer, etc).

Therefore the invention provides a method to determine if a renal mass present in a subject is a benign or a malignant renal mass or a method to predict if a renal mass present in a subject is a renal cell carcinoma (RCC) or a method to detect the recurrence of a renal mass or a method to identify if a subject is affected by RCC or by other renal functional diseases (diseases that affects the renal parenchyma as a result of a systemic pathological process) or by other malignant diseases (other types of tumours that affect kidneys except RCC) said method comprising measuring the level of at least one miRNA or a combination thereof in a biological sample of the subject and comparing said measured level to a reference level, wherein the at least one miRNA is indicated in Table 11 or Table 21 or is selected from the group consisting of: hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548l, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-485-3p, hsa-miR-335-5p, hsa-miR-1290, hsa-miR-132-3p, hsa-miR-337-5p, hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, hsa-miR-181b-5p, hsa-miR-192-5p, hsa-miR-433-3p, hsa-miR-12136, hsa-miR-598-3p, hsa-miR-628-3p, hsa-miR-19a-3p, hsa-miR-122-5p, hsa-miR-196b-5p, hsa-miR-215-5p, hsa-miR-491-5p, and hsa-miR-153-3p and wherein said combination is indicated in Table 16 or Table 25.

Preferably the method according further comprises determining the genotype of UMOD SNPs rs71384446, rs9928757, rs11647727, rs28640218, rs7193058, rs76563024, rs11862974, rs13329952, rs7203642, rs7204342, and rs28688991 or of any other UMOD SNPs in linkage disequilibrium with them (preferably with a R² > 0.8) in any ethnic background, preferably said other UMOD SNPs are selected from the group consisting of: rs7204775, rs4997081, rs12922822, rs12917707, rs9933330, rs13334589, rs13333226, rs28362063, rs4293393, rs35650857, rs34882080, rs13335818, rs28544423, rs9928003, rs12934320, rs34356953, rs34115067, rs60136849, rs9928936, rs36060036, rs71149134, rs12934455, rs34262842, rs35830321, rs71149135, rs71377648, rs80159442, rs11342813, rs6497476, rs79245268, rs77875418, rs80142672, rs190450490, rs59027126, rs58768874, rs118059635, rs138755971, rs189908977, rs55808501, rs74842838, rs143657120, rs145915865, rs75645968, rs114333799, rs114112267, rs75459600, rs76625281, rs76236484, rs4500734, rs58351658, rs4490010, rs147846395, rs34857077, rs6497475, rs7204210, rs6497474, rs8060932, rs8062123, and rs4238595.

The invention also provides a method to discriminate a subject affected by a low stage RCC from a subject affected by a high stage RCC comprising measuring the level of at least one miRNA or a combination thereof in a biological sample of the subject and comparing said measured level to a reference level, wherein the at least one is indicated in Table 13 or Table 23 or is selected from the group consisting of: hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p, hsa-miR-181c-5p, hsa-miR-4306, hsa-miR-27a-3p, hsa-miR-134-5p, hsa-miR-1296-5p, hsa-miR-15a-5p hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p, hsa-miR-1296-5p, hsa-miR-1249-3p, hsa-miR-100-5p, hsa-miR-181c-5p, hsa-miR-323b-3p, hsa-miR-452-3p, hsa-miR-20b-5p, and hsa-miR-1301-3p and wherein said combination is indicated in Table 17 or Table 26.

Preferably said method further comprises determining the genotype of UMOD SNPs rs71384446, rs9928757, rs60136849, rs11647727, rs34356953, rs12934320, rs12934455, rs28640218, rs28544423, rs13335818, rs4293393, rs28362063, rs13333226, rs13329952, rs7203642, rs28688991, and rs12917707 or of any other UMOD SNPs in linkage disequilibrium with them (preferably with a R² > 0.8) in any ethnic background, preferably said other UMOD SNPs is selected from the group consisting of: rs12922822, rs9933330, rs13334589, rs34115067, rs9928936, rs71149134, rs7204775, rs71377648, rs9924088, rs9923990, rs9926225, rs111483946, rs112166439, rs113923200, rs554722277, rs111992415, rs544951514, rs113957052, rs111266260, rs192180621, rs12102292, rs116563128, rs145279202, rs9937393, rs150044343, rs7204342, rs80159442, rs11342813, rs11862974, rs6497476, rs76563024, rs79245268, rs77875418, rs80142672, rs190450490, rs59027126, rs58768874, rs118059635, rs138755971, rs189908977, rs8060932, rs8062123, rs4238595, rs7193058, rs34857077, rs6497475, rs6497474, rs181820305, rs117774819, rs528859496, rs149233634, rs147946789, rs181711435, rs184483102, rs183590048, rs187620954, rs191065984, rs182744661, rs185578691, rs182605229, rs191171015, rs185630961, rs192280820, rs182188833, rs186260541, rs185128245, rs77225526, rs74011925, rs78052469, rs75864273, rs150052437, rs74011921, and rs372456426.

The invention also provides a method to discriminate a subject affected by a low grade RCC from a subject affected by a high grade RCC comprising measuring the level of at least one miRNA or a combination thereof in a biological sample of the subject and comparing said measured level to a reference level, wherein the at least one miRNA is indicated in Table 12 or Table 22 or is selected from the group consisting of: hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-29b-3p, hsa-miR-590-5p, hsa-miR-340-5p, hsa-miR-15a-5p, hsa-miR-505-5p, hsa-miR-433-3p, hsa-miR-29c-5p, and hsa-miR-15a-3p and wherein the combination is indicated in Table 18 or Table 27.

Preferably said method further comprises determining the genotype of UMOD SNP rs13333226 or of any other UMOD SNPs in linkage disequilibrium with rs13333226 (preferably with a R² > 0.8) in any ethnic background, preferably said other UMOD SNP is selected from the group consisting of: rs7204775, rs7203642, rs4293393, rs4997081, rs28688991, rs28640218, rs13329952, rs13335818, rs34115067, rs60136849, rs9928936, rs35650857, rs34882080, rs71149135, rs34356953, rs11862974, rs6497476, rs11342813, rs7204342, rs76563024, rs80159442, rs79245268, rs77875418, rs80142672, rs190450490, rs59027126, rs58768874, rs118059635, rs138755971, rs189908977, rs55808501, rs28362063, rs13334589, rs9933330, rs12922822, rs12917707, rs28544423, rs9928003, rs12934320, rs36060036, rs71149134, rs12934455, rs34262842, rs35830321, rs71377648, rs9928757, and rs71384446.

The invention also provides a method to determine the presence of clinical metastasis in a RCC patient comprising measuring the level of at least one miRNA or a combination thereof in a biological sample of the subject and comparing said measured level to a reference level, wherein the at least one miRNA is indicated in Table 14 or is selected from the group consisting of: hsa-miR-3613-3p, hsa-miR-548l, hsa-miR-548k, hsa-miR-377-3p, hsa-miR-153-3p, hsa-miR-590-3p, hsa-miR-582-5p, hsa-miR-129, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, hsa-miR-382-5p, hsa-miR-342-3p, hsa-miR-532-5p, hsa-miR-197-3p, hsa-miR-126-3p, hsa-miR-150-5p, hsa-let-7i-3p, hsa-miR-222-3p, and wherein the combination is indicated in Table 19.

Preferably said method further comprises determining the genotype of UMOD SNPs rs71384446, rs9928757, rs60136849, rs34356953, rs12934320, rs12934455, rs28544423, rs13335818, rs4293393, rs28362063, rs13333226, rs13329952, rs7203642, rs28688991, and rs12917707 or of any other UMOD SNPs in linkage disequilibrium with them (preferably with a R² > 0.8) in any ethnic background, preferably rs12922822, rs9933330, rs13334589, rs35650857, rs34882080, rs9928003, rs34115067, rs9928936, rs36060036, rs71149135, rs71149134, rs34262842, rs35830321, rs7204775, rs4997081, rs28640218, rs71377648, rs9924088, rs9923990, rs9926225, rs369494277, rs111483946, rs112166439, rs188045848, rs111904030, rs113923200, rs149786037, rs554722277, rs113452476, rs111992415, rs377257136, rs114997829, rs544951514, rs113957052, rs111266260, rs192180621, rs12102292, rs116563128, rs145279202, rs9937393, rs150044343, rs7204342, rs80159442, rs11342813, rs11862974, rs6497476, rs76563024, rs79245268, rs77875418, rs80142672, rs190450490, rs59027126, rs58768874, rs118059635, rs138755971, rs189908977, rs55808501, rs372456426, rs6497475, rs34857077, rs77225526, rs74011925, rs78052469, rs75864273, rs150052437, rs74011921, rs181820305, rs4238595, rs117774819, rs528859496, rs149233634, rs147946789, rs181711435, rs184483102, rs183590048, rs187620954, rs191065984, rs182744661, rs185578691, rs182605229, rs191171015, rs185630961, rs192280820, rs182188833, rs186260541, rs185128245, rs8060932, and rs8062123.

The invention also provides a method for the prognosis of RCC or a method to monitor the effect of a therapy on RCC in a subject comprising measuring the level of at least one miRNA or a combination thereof in a biological sample of the subject and comparing said measured level to a reference level, wherein the at least one miRNA is indicated in Table 12 or Table 13 or Table 14 or Table 22 or Table 23 or is selected from the group consisting of: hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p, hsa-miR-181c-5p, hsa-miR-4306, hsa-miR-27a-3p, hsa-miR-134-5p, hsa-miR-1296-5p, and hsa-miR-15a-5p, hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-3613-3p, hsa-miR-548l, hsa-miR-548k, hsa-miR-377-3p, hsa-miR-153-3p, hsa-miR-590-3p, hsa-miR-582-5p, hsa-miR-129, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, hsa-miR-382-5p, hsa-miR-342-3p, hsa-miR-532-5p, hsa-miR-197-3p, hsa-miR-126-3p, hsa-miR-150-5p, hsa-let-7i-3p, hsa-miR-222-3p, hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p, hsa-miR-1296-5p, hsa-miR-1249-3p, hsa-miR-100-5p, hsa-miR-181c-5p, hsa-miR-323b-3p, hsa-miR-452-3p, hsa-miR-20b-5p, and hsa-miR-1301-3p, hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-29b-3p, hsa-miR-590-5p, hsa-miR-340-5p, hsa-miR-15a-5p, hsa-miR-505-5p, hsa-miR-433-3p, hsa-miR-29c-5p, and hsa-miR-15a-3p and wherein the combination is indicated in Table 17 or Table 18 or Table 19 or Table 26 or Table 27.

Preferably, said method further comprises determining the genotype of UMOD SNPs rs11647727, rs12917707, rs12934320, rs12934455, rs13329952, rs13333226, rs13335818, rs28362063, rs28544423, rs28640218, rs28688991, rs34356953, rs4293393, rs60136849, rs71384446, rs7203642, rs9928757 or of any other UMOD SNPs in linkage disequilibrium with them (preferably with a R² > 0.8) in any ethnic background, preferably rs111266260, rs111483946, rs111904030, rs111992415, rs112166439, rs11342813, rs113452476, rs113923200, rs113957052, rs114997829, rs116563128, rs117774819, rs118059635, rs11862974, rs12102292, rs12922822, rs13334589, rs138755971, rs145279202, rs147946789, rs149233634, rs149786037, rs150044343, rs150052437, rs181711435, rs181820305, rs182188833, rs182605229, rs182744661, rs183590048, rs184483102, rs185128245, rs185578691, rs185630961, rs186260541, rs187620954, rs188045848, rs189908977, rs190450490, rs191065984, rs191171015, rs192180621, rs192280820, rs34115067, rs34262842, rs34857077, rs34882080, rs35650857, rs35830321, rs36060036, rs369494277, rs372456426, rs377257136, rs4238595, rs4997081, rs528859496, rs544951514, rs554722277, rs55808501, rs58768874, rs59027126, rs6497474, rs6497475, rs6497476, rs71149134, rs71149135, rs71377648, rs7193058, rs7204342, rs7204775, rs74011921, rs74011925, rs75864273, rs76563024, rs77225526, rs77875418, rs78052469, rs79245268, rs80142672, rs80159442, rs8060932, rs8062123, rs9923990, rs9924088, rs9926225, rs9928003, rs9928936, rs9933330, rs9937393.

The invention also provides a method to predict the presence of a renal mass in a subject comprising measuring the level of at least one miRNA or a combination thereof in a biological sample of the subject and comparing said measured level to a reference level, wherein the at least one miRNA is indicated in Table 10 or Table 20 or is selected from the group consisting of: hsa-miR-197-3p, hsa-miR-378a-3p, hsa-miR-12136, hsa-miR-28-3p, hsa-miR-15b, 3p, hsa-miR-328-3p, hsa-miR-323a-3p, hsa-miR-550a-3-5p, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p, hsa-miR-377-3, hsa-miR-483-5p, hsa-miR-6803-3p, hsa-miR-181c-3p, hsa-miR-101-3p, hsa-miR-374a-5, hsa-miR-423-5p, hsa-miR-29a-3p, hsa-miR-7-5p, hsa-miR-378a-3p, hsa-miR-328-3p, hsa-miR-28-3p, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p, hsa-miR-18a-5p, hsa-miR-130a-3p, hsa-miR-4306, hsa-miR-423-3p, hsa-miR-6803-3p, hsa-miR-664a-3p, hsa-miR-323b-3p, hsa-miR-128-3p, and hsa-miR-494-3p and wherein the combination is indicated in Table 15 or Table 24.

Preferably in any of herein disclosed methods the subject is affected by obesity, autosomal polycystic kidney disease, multicystic kidney disease, hypertension or diabetes or wherein the subject is a smoker or has a family history of kidney cancer

In a still preferred embodiment any of disclosed herein method further comprises measuring the level of uromodulin in a biological sample of the subject and comparing said measured level to a reference level, preferably the biological sample is selected from the group consisting of: plasma, serum and urine sample.

In a still preferred embodiment any of disclosed herein method further comprises the analysis of a phenotypic characteristic, preferably selected from the group consisting of:
- clinical parameters, such as age at diagnosis/treatment, comorbidity index and specific comorbidities or history of previous renal or non-renal cancer;
- radiological parameters, such as clinical size defined as clinical diameter at axial imaging, tumor volume, clinical tumor stage, clinical nodal stage, PADUA score, RENAL score, ABC score or other nephrometric scores;
- biohumoral parameters, such as hemoglobin, platelets, creatinine, urea, blood electrolytes, total protein serum level and electrophoresis, measured and estimated glomerular filtration rate, proteinuria and urine tests.

In a still preferred embodiment, any of disclosed herein method the biological sample is selected from: blood, blood cells, serum, plasma, urine, saliva, sputum, tears, kidney tissue.

The present invention also provides a kit for use in a method according to any one of previous claim comprising means to quantify said miRNA and control means and optionally comprising means to determine at least one UMOD genotype, preferably said kit comprises an array.

Clinical metastasis is revealed by the most common and standard radiological imaging tests (CT, MRI) and is detected before surgery (e.g. renal tumor mass) or after diagnosis of renal tumor. In the present invention the reference level to which the measured miRNA is to be compared with is the value measured in a biological sample of a healthy subject, or the value measured in a biological sample of a subject that does not have a renal mass and/or any previous history of any type of cancer (non-renal mass control). It can also be the value measured in a biological sample of a subject without a renal mass, the value measured in a biological sample of a subject with oncocytoma or other benign renal mass, the value measured in a biological sample of a subject with RCC, the value measured in a biological sample of a subject with low or high grade RCC, the value measured in a biological sample of a subject with low stage or high stage RCC, the value measured in a biological sample of a subject with or without clinical metastasis, the value measured in a biological sample of a subject with RCC before starting a therapy or throughout the course of therapy treatment, the value measured in a biological sample of a subject with RCC in active surveillance, the value measured in a biological sample of a subject with a previous surgery for RCC. The skilled person will choose the appropriate reference value according to the comparison to be made.

Indicated miRNA can be found in the miRbase Version v22.

In the methods of the present invention, the nucleotide sequences of said miRNAs can be found in the miRbase Version v22 and are selected from the group consisting of (i) a nucleotide sequence according to the miRbase Version v22, (ii) a nucleotide sequence that is a fragment of the nucleotide sequence according to (i), preferably, a nucleotide sequence that is a fragment which is between 1 and 12, more preferably between 1 and 8, and most preferably between 1 and 5 or 1 and 3, i.e. 1, 2, 3, 4, 5, 6, 7, 8, 9, 10, 11, or 12, nucleotides shorter than the nucleotide sequence according to (i), and (iii) a nucleotide sequence that has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the nucleotide sequence according to (i) or nucleotide sequence fragment according to (ii).

MiRNAs of the present invention may be detected and quantify according to any method known in the art, including methods disclosed herein.

In some aspects, the miRNAs of the present invention are upregulated or overexpressed in renal cell carcinoma or depending on the stage/grade of the carcinoma, the presence or not of clinical metastasis, as well as the histotype of renal mass; In some aspects, the miRNAs of the present invention are downregulated or under-expressed in renal cell carcinoma or depending on the stage/grade of the carcinoma, the presence or not of clinical metastasis, as well as the histotype of renal mass.

The present invention also provides miRNA signatures or "expression signatures", as indicated in herein tables.

An "expression signature" is a group of two or more genes or gene-related molecules (e.g., DNA, RNA, amino acids and proteins) that exist in a cell, tissue fluid or other sample whose combined expression pattern is characteristic of a particular tissue or is characteristic of a particular condition, or disease state. The expression signature can be used to select or stratify a group of subjects based on, for example, a specific stage of a disease, a risk of developing a particular disease or state of disease or a probability or prediction of a prognosis, with sufficient accuracy to facilitate diagnosis or selection of treatment.

MiRNA is a group of small non-coding RNAs that regulate gene expression during development and differentiation (Bartel 2004). miRNA expression is very tissue-specific (Bartel 2004). Some miRNAs that are stably expressed in certain tissue types might be expressed differently in other tissue types. In some embodiments, the miRNA molecules are present in tissues and/or organs at normal physiological conditions but may be expressed at a much higher or lower level in diseased tissue or cells and are indicative of a change in health status. In other embodiments, the biomarkers may be absent in tissues and/or organs under normal physiological conditions but are specifically expressed in diseased tissue or cells. In other embodiments, the biomarkers may be specifically released to the bloodstream under conditions of RCC, and/or are over- or under-expressed as compared to normal levels. Measurement of miRNA, alone or in combination, in patient samples provides information that may correlate with a diagnosis of the selected disease. Therefore, a set of one or more miRNA molecules may be used in screening, diagnostic and prognostic assays.

As used herein the phrase "diagnostic" or means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay may be termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

As used herein the phrase "diagnosing" refers to classifying a disease or a symptom, determining a severity of the disease, monitoring disease progression, forecasting an outcome of a disease and/or prospects of recovery. The term "detecting" may also optionally encompass any of the above.

A "prognosis" may be measured as any outcome of illness. In some embodiments a prognosis is a measurement of or prediction of overall survival, recurrence-free survival, or progression of a disease. A good prognosis indicates, for example, that the disease is less likely to progress, the chance of survival after diagnosis or treatment is high or the length of time a subject is likely to live after diagnosis or after treatment is a relatively long period of time. A poor prognosis indicates, for example, that the disease is more likely to progress, the chance of survival after diagnosis or treatment is low or the length of time a subject is likely to live after diagnosis or after treatment is relatively short.

Alteration of miRNA expression has been shown in malignancies (Michael et al. 2003; Calin et al. 2005; Yanaihara et al. 2006) and plays an important role in tumorigenesis and cancer progression (Esquela-Kerscher & Slack 2006). Studies have shown that certain miRNAs play important roles in various steps of the metastatic cascade, such as the endothelial-mesenchymal transition (EMT), adhesion, migration, invasion, apoptosis and angiogenesis. Because the expression of multiple genes can be regulated by a single miRNA, miRNA expression profiles may be more accurate in determining cancer subtypes than RNA profiles of protein-coding genes (Calin& Croce 2006; Volinia et al. 2006). Expression signatures based on miRNA expression have been shown to aid in diagnosis and prognostication of cancer (Yu et al. 2008; Hiroki et al. 2010).

In the current study, miRNA expression levels in patients affected by a renal mass or RCC were profiled. MiRNA expression levels be profiled using a microarrays or PCR-based assay, as further discussed herein.

miRNA expression may be detected by any suitable method for RNA detection including, but not limited to, microarray, reverse transcriptase polymerase chain reaction (RT-PCR) or other PCR-based assays (e.g. quantitative or real time RT-PCR) or mass spectrometry.

A method for generating a miRNA expression signature may comprises performing a logistic regression analysis to identify a set of one or more miRNAs associated with presence of kidney cancer, presence of adverse phenotypic kidney cancer characteristics or a specific kidney cancer histotype.

A method for generating a miRNA expression signature may also include a step assigning a risk score to each patient with a renal mass by calculating a risk score formula and determining a cut-off risk score to stratify the cohort subjects into one or more risk groups (e.g., high risk, low risk, intermediate risk) as described in further detail in the examples below.

In other embodiments, methods for predicting a presence of kidney cancer, presence of adverse phenotypic kidney cancer characteristics or a specific kidney cancer histotype, hence aiding clinical decision making in the screening, diagnosis and prognostication of the disease, are provided. These methods may include detecting a test expression level of one or more miRNAs as part of a cancer-specific expression signature in a biological sample from a subject having a cancer, assigning a risk score to the test expression level; and predicting a presence of kidney cancer, presence of adverse phenotypic kidney cancer characteristics or specific kidney cancer histotype, hence aiding clinical decision making in the screening, diagnosis and prognostication of the disease.

When the test expression level is assigned a high-risk score, the subject is likely to have kidney cancer, presence of adverse phenotypic kidney cancer characteristics. When the test expression level is assigned a low risk score, the subject is not likely to have kidney cancer, presence of adverse phenotypic kidney cancer characteristics.

In some embodiments, the methods of the present invention provide for utilizing a biological sample of a subject or patient received from a third party; or obtaining a biological sample from a subject or a patient. Examples of suitable biological samples which may optionally be used with the embodiments of the present invention include but are not limited to blood, serum, plasma, blood cells, urine, sputum, saliva, stool, spinal fluid or CSF, lymph fluid, the external secretions of the skin, respiratory, intestinal, and genitourinary tracts, tears, milk, neuronal tissue, lung tissue, any human organ or tissue, including any tumor or normal tissue, any sample obtained by lavage (for example of the bronchial system or of the breast ductal system), and also samples of in vivo cell culture constituents. The sample can optionally be diluted with a suitable eluant before contacting the sample to an antibody and/or performing any other diagnostic assay.

Numerous well-known tissues or fluid collection methods can be utilized to collect a biological sample from the subject in order to genotype and/or sequence DNA regions, determine the level of RNA and/or polypeptide of the variant of interest in the subject. Examples include, but are not limited to, liquid biopsy, fine needle biopsy, needle biopsy, core needle biopsy and surgical biopsy (e.g., brain biopsy), and lavage. Regardless of the procedure employed or where the biopsy or sample is obtained, the level of the variant can be determined, and a diagnosis can thus be made.

The present invention will be illustrated by means of non-limiting examples in reference to the following figures.

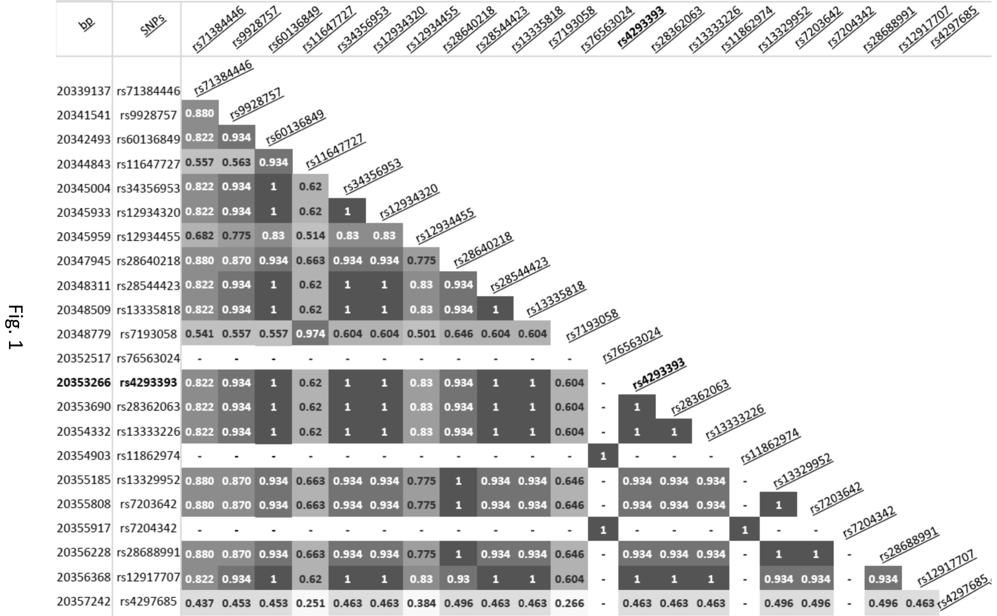

Figure 1. Pairwise r² values among the 22 UMOD SNPs genotyped in this study.

Linkage disequilibrium (LD) map for the 22 UMOD SNPs genotyped in this study. Variants are ordered by chromosomal position. A pairwise r² value is shown in each square. Darker blocks correspond to higher r² values. LD r² values were from 1000 Genomes Phase 3 genotypes for the Tuscan population (TSI, Toscani in Italy).

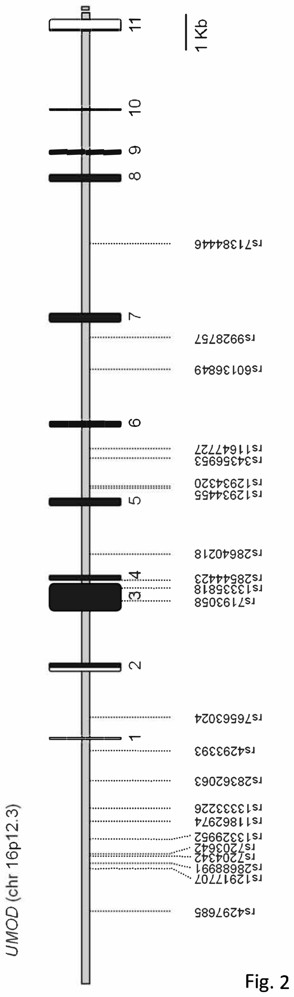

Figure 2. Schematic representation of human UMOD gene structure. The position of the 22 UMOD SNP variants is shown.

### DETAILED DESCRIPTION OF THE INVENTION

### Patients

The study population consisted of 276 patients with a diagnosis of a first episode of renal mass, elected for surgical treatment via nephron sparing surgery or radical nephrectomy, together to 83 non renal mass controls consisted of patients affected by urological functional diseases (kidney stones, benign prostate hypertrophy, etc).

This total cohort of patients was included in the analysis of UMOD genotypes. A subgroup of 120 cases (patients affected by renal masses) together to 78 controls (patients affected by urological benign disfunctions) were included in the analysis of circulating miRNAs. This cohort of patients was composed by a retrospective monocentric phase together with a prospective European multicentric phase.

The prospective cohort was formed by 110 patients (59 case and 51 controls). It is important to underline that each control patient was submitted to an abdominal CT scan to exclude the concomitant presence of a renal mass.

A retrospective cohort refers to patients whose biological samples have been already collected and stored in a biobank and clinical data have been reported in a dataset.

A prospective cohort refers to patients, which are followed over time, whose biological samples and clinical data are collected.

The study population was enrolled from 2011 to 2018 in a single tertiary care institution. From 2019, in addition to this center, other four European centers were involved into the prospectively maintained dataset and specimen collection.

### Study outcomes

The aim of the study was to identify novel biomarkers that can improve the management of patients with RCC at all the stages of clinical decision-making, in particular in 3 specific clinical settings:

### 1. Screening

The present predictive model based on UMOD genotype and/or circulating miRNAs can predict the presence of a renal mass, defined as a mass either benign or malignant recorded at axial imaging examination (either CT or MRI). In the model, the inventors compared the above-mentioned markers in patients diagnosed with a renal mass relative to controls. The clinical implication of this information is the identification of patients with high risk of a renal mass to axial imaging.

### 2. Diagnosis of kidney cancer

The present predictive model based on UMOD genotype and/or circulating miRNAs can predict the presence of RCC, defined as a renal malignancy. In the model, the inventors compared the above-mentioned markers in patients diagnosed with any RCC histological subtype (ccRCC, p1RCC, p2RCC, chRCC) relative to patients diagnosed with a benign renal mass (oncocytoma, angymyolipoma, benign cyst or other benign histotype). The clinical implication of this information is the selection or identification of patients with high risk of RCC and of patients with low risk of RCC to be inserted in specific treatment or surveillance protocol.

### 3. Prognostic

The present predictive model based on UMOD genotype and/or circulating miRNAs can predict the prognosis of patients diagnosed with aggressive RCC. Inventors relied on established predictors of RCC prognosis that are direct markers of clinical aggressiveness, namely
a. tumour stage, defined using the AJCC 2018staging system and grouped as pT1-pT2 (low stage) vs pT3-pT4 (high stage) [Edge et al., 2010];
b. pathologic N stage, defined using the AJCC 2018 staging system and grouped as pN0-pNx vs pN1 [Edge et al., 2010];
c. tumour grade, defined according to the Furhman classification system or WHO/ISUP and grouped as G1-G2 (low grade) vs G3-G4 (high grade) [Delahunt et al., 2013- Delahunt et al.,2016];
d. presence of clinical metastasis, defined as cM1 stage at systemic clinical staging [Edge et al., 2010].

The clinical implication of this information is the identification of patients with high risk of aggressive RCC for specific treatment. The model allows to spare an aggressive treatment in older and more frail patients. Moreover, the model allows the identification of patients that could benefit from post-treatment follow-up based on the aggressiveness of the disease.

### Study Population

The study population consisted of 276 patients with a diagnosis of a first episode of renal mass, elected for surgical treatment via nephron sparing surgery or radical nephrectomy, together to 83 non renal mass controls consisted of patients affected by urological functional diseases (kidney stones, benign prostate hypertrophy, etc).

Among those with a renal mass, the median age was 64 years old with a male predominance on female patients (65.6% vs 34.4%) as reported in literature. In the final pathology evaluation, the 16.3 % of patients demonstrated a benign lesion whereas the 83.7% a malignant one. High stage (pT3-pT4) was diagnosed in the 36.4 % while low stage (pT1-pT2) was reported in the 63.6%: in the 59.7% the population displayed low grade tumors (G1-G2) while 40.3% high grade neoplasms (G3-G4). The discrepancy (patients with T1 or T2 staging but G3 or G4 grading and vice versa) between TNM staging and Furman/WHO-ISUP grading was about 24.3%. The 12.3% of patients was affected by a metastatic process.

In the controls group, median age was 58 years old, with a male-female percentage of 62.7% and 37.3%, respectively (Table 2).

**Table 2. Descriptive analysis of UMOD genotypes total cohort of patients**

| | Renal mass patients | Controls |
|---|---|---|
| Number of patients, n | 276 | 83 |
| Age, median (range) | 64 (55-72) | 58 (50-66) |

| Gender, n (%) | | |
|---|---|---|
| Male | 181 (65.6%) | 52(62.7%) |
| Female | 95 (34.4%) | 31 (37.3%) |

| Malignancy, n (%) | | NA |
|---|---|---|
| Benign | 45 (16.3%) | |
| Malignant | 231 (83.7%) | |

| Pathologic T stage, n (%) | | NA |
|---|---|---|
| pT1-pT2 | 147 (63.6%) | |
| pT3-pT4 | 84 (36.4%) | |

| Pathologic grade, n (%) | | NA |
|---|---|---|
| G1-G2 | 138 (59.7%) | |
| G3-G4 | 93 (40.3%) | |

| Clinicalmetastasis, n (%) | | NA |
|---|---|---|
| cM0 | 242 (87.7%) | |
| cM1 | 34(12.3%) | |

In the subgroup of patients where miRNAs were analyzed the inventors considered 120 case patients vs 78 controls with the previous same clinical and pathological criteria.

Medium age was 66 years old for renal mass pts while 57 years old for controls. Male/female percentages were 54.2% vs 45.8% for cases and 62.82% vs 37.18% for controls.

Patients affected by a benign lesion were 21.7%. High stage tumours were 28.7% and high-grade neoplasms were 28.7%, with a discrepancy of 15% between stage and grade classifications. Metastatic patients were the 6.7% of the total malignant cancers (Table 3).

**Table 3. Descriptive analysis of miRNAs total cohort of patients**

| | **Renal mass patients** | **Controls** |
|---|---|---|
| **Number of patients, n** | 120 | 78 |
| **Age, median (range)** | 66 (54-73) | 57 (49-66) |

| **Gender, n (%)** | | |
|---|---|---|
| Male | 65 (54.2%) | 49 (62.8%) |
| Female | 55 (45.8%) | 29 (37.2%) |

| **Malignancy, n (%)** | | NA |
|---|---|---|
| Benign | 26 (21.7%) | |
| Malignant | 94(78.3%) | |

| **Pathologic T stage, n** (%) | | NA |
|---|---|---|
| pT1-pT2 | 67 (71.3%) | |
| pT3-pT4 | 27 (28.7%) | |

| **Pathologic grade, n (%)** | | NA |
|---|---|---|
| G1-G2 | 66 (71.3%) | |
| G3-G4 | 28 (28.7%) | |

| **Clinicalmetastasis, n** (%) | | NA |
|---|---|---|
| cM0 | 112 (93.3%) | |
| cM1 | 8 (6.7%) | |

Then, the inventors decided to consider for the miRNAs analysis also a restricted cohort of patients related to the prospective multicentric study (Table 4).

**Table 4. Descriptive analysis of miRNAs prospective multicentric cohort of patients**

| | **Renal mass patients** | **Controls** |
|---|---|---|
| **Number of patients, n** | 59 | 51 |
| **Age, median (range)** | 66 (59-75) | 59 (53-67) |

| **Gender, n (%)** | | |
|---|---|---|
| Male | 37 (62,7%) | 35 (68,6%) |
| Female | 22 (37,3%) | 16 (31,4%) |

| **Malignancy, n (%)** | | NA |
|---|---|---|
| Benign | 17 (28,8%) | |
| Malignant | 42 (71,2%) | |

| **Pathologic T stage, n (%)** | | NA |
|---|---|---|
| pT1-pT2 | 34 (81%) | |
| pT3-pT4 | 8 (19%) | |

| **Pathologic grade, n (%)** | | NA |
|---|---|---|
| G1-G2 | 31 (73,8%) | |
| G3-G4 | 11 (26,2%) | |

| **Clinical metastasis, n (%)** | | NA |
|---|---|---|
| cM0 | 58 (98,3%) | |
| cM1 | 1 (1,7%) | |

Medium age was 66 years old for renal mass pts while 59 years old for controls. Male/female percentages were 62.7% vs 37.3% for cases and 68.6% vs 31.4% for controls.

Patients affected by a benign lesion were 28.8%. High stage tumours were 19% and high-grade neoplasms were 26.2%, with a discrepancy of 15% between stage and grade classifications. Only one patient was affected by metastasis (1.7%).

### METHODS

### Single nucleotide polymorphisms (SNPs) genotyping in the UMOD gene

Genomic DNA was extracted from whole blood, leukocytes, or normal kidney tissue (depending on individual sample availability) using the QIAamp DNA Mini Kit (QIAGEN), according to manufacturer's instructions. Purity and concentration of recovered DNA were determined with a NanoDrop spectrophotometer (ND-1000, NanoDrop Technologies, Wilmington, DE, USA).

Genotyping of 22 single nucleotide polymorphisms (SNPs) in the UMOD gene (rs4293393 (ASSAY ID C_27865986_10), rs13335818 (ASSAY ID C_136431_10), rs28544423 (ASSAY ID C_136432_20), rs11647727 (ASSAY ID C_11844689_10), rs28640218 (ASSAY ID C_27398349_10), rs12934320 (ASSAY ID C_27435435_10), rs12934455 (ASSAY ID C_27435436_10), rs34356953 (ASSAY ID C__27435446_10), rs7193058 (ASSAY ID C__29095382_10), rs4297685 (ASSAY ID C__30368496_10), rs9928757 (ASSAY ID C_30613644_10), rs13333226 (ASSAY ID C_31122293_10), rs11862974 (ASSAY ID C_31122295_10), rs13329952 (ASSAY ID C_31122297_10),rs12917707 (ASSAY ID C_31122302_20), rs28362063 (ASSAY ID C_60216431_10), rs28688991 (ASSAY ID C_60216441_10), rs60136849 (ASSAY ID C_89248696_10), rs71384446 (ASSAY ID C_97852849_10), rs76563024 (ASSAY ID C_102020079_10), rs7203642 (ASSAY ID C_189262956_10), rs7204342 (ASSAY ID C_189263753_10), ThermoFisher, Cat. No.: 4351379) was performed by using the TaqMan Open Array technology (ThermoFisher), following the manufacturer's protocol. DNA was combined with TaqMan OpenArray Genotyping Master Mix, loaded onto the OpenArray^{™} plates (AccuFill^{™} system, Cat. No. 4471021) and amplified with the QuantStudio 12K Flex Real-Time PCR System (ThermoFisher, Cat. No. 4472379). All analyses were performed in duplicate to ensure reproducibility of the results. Human gDNA samples from the NHGRI Sample Repository for Human Genetic Research at Coriell Institute for Medical Research were also includedas positive controls for each variant, as well as non-template control (NTC), consisting of DNase-free double-distilled water.

Genotype clustering and calling were carried out by using TaqMan Genotyper Software version 1.5 (call rate 99.14%; ThermoFisher).

### TaqMan MicroRNA Profiling Using the QuantStudio 12K Flex Real-Time PCR System with OpenArray

Total RNA was isolated from 200 µL of plasma using miRNeasy Serum/Plasma Advanced Kit (QIAGEN, Cat No. 217204) according to manufacturer's instructions, with a little modification. Briefly, during the isolation procedure, 0.001 pmol of synthetic miRNA-39 from Caenorhabditis elegans (cel-miR-39, Accession number MIMAT0000010 miRBase v22, synthesized by ThermoFisher) was added as a spike-in control before the addition of Buffer RPP. In humans, a cel-miR-39-sequence does not exist and thus its quantification enables quality control of the RNA isolation, cDNA synthesis and PCR amplification steps.

cDNA was generated using 2 µl of purified total RNA with the TaqMan Advanced miRNA cDNA Synthesis kit (ThermoFisher, Cat. No. A28007). Expression levels of 668 miRNAs (hsa-let-7a-3p (Assay ID 477861_mir), hsa-let-7a-5p (Assay ID 478575_mir),hsa-let-7b-3p (Assay ID 478221_mir), hsa-let-7b-5p (Assay ID 478576_mir), hsa-let-7c-5p (Assay ID 478577_mir), hsa-let-7d-3p (Assay ID 477848_mir), hsa-let-7d-5p (Assay ID 478439_mir),hsa-let-7e-3p (Assay ID 479281_mir), hsa-let-7e-5p (Assay ID 478579_mir), hsa-let-7f-1-3p (Assay ID 477801_mir), hsa-let-7f-2-3p (Assay ID 477843_mir), hsa-let-7f-5p (Assay ID 478578_mir), hsa-let-7g-3p (Assay ID 477850_mir), hsa-let-7g-5p (Assay ID 478580_mir), hsa-let-7i-3p (Assay ID 477862_mir),hsa-let-7i-5p (Assay ID 478375_mir), hsa-miR-100-3p (Assay ID 478619_mir),hsa-miR-100-5p (Assay ID 478224_mir), hsa-miR-101-3p (Assay ID 477863_mir), hsa-miR-101-5p (Assay ID 478620_mir),hsa-miR-10399-3p (Assay ID 483065_mir), hsa-miR-10399-5p (Assay ID 483087_mir), hsa-miR-103a-2-5p (Assay ID 477864_mir), hsa-miR-103a-3p (Assay ID 478253_mir),hsa-miR-103b (Assay ID 478621_mir), hsa-miR-106a-3p (Assay ID 478623_mir),hsa-miR-106a-5p (Assay ID 478225_mir), hsa-miR-106b-3p (Assay ID 477866_mir), hsa-miR-106b-5p (Assay ID 478412_mir), hsa-miR-107 (Assay ID 478254_mir), hsa-miR-10a-3p (Assay ID 478624_mir), hsa-miR-10a-5p (Assay ID 479241_mir), hsa-miR-10b-3p (Assay ID 477868_mir), hsa-miR-10b-5p (Assay ID 478494_mir),hsa-miR-11400 (Assay ID 483092_mir), hsa-miR-1179 (Assay ID 478626_mir), hsa-miR-1180-3p (Assay ID 477869_mir),hsa-miR-1185-5p (Assay ID 480633_mir), hsa-miR-1197 (Assay ID 478630_mir), hsa-miR-12116 (Assay ID 483094_mir), hsa-miR-12118 (Assay ID 483119_mir), hsa-miR-12135 (Assay ID 483153_mir), hsa-miR-12136 (Assay ID 483118_mir),hsa-miR-122-3p (Assay ID 477874_mir), hsa-miR-122-5p (Assay ID 477855_mir), hsa-miR-1226-5p (Assay ID 478641_mir), hsa-miR-1227-3p (Assay ID 478642_mir),hsa-miR-1228-5p (Assay ID 478644_mir), hsa-miR-122b-3p (Assay ID 479677_mir), hsa-miR-124-3p (Assay ID 480901_mir),hsa-miR-1246 (Assay ID 477881_mir), hsa-miR-1247-5p (Assay ID 477882_mir),hsa-miR-1248 (Assay ID 478653_mir), hsa-miR-1249-3p (Assay ID 478654_mir), hsa-miR-1250-5p (Assay ID 478655_mir), hsa-miR-1255a (Assay ID 478661_mir), hsa-miR-1255b-5p (Assay ID 478662_mir), hsa-miR-1256 (Assay ID 478663_mir), hsa-miR-125a-3p (Assay ID 477883_mir),hsa-miR-125a-5p (Assay ID 477884_mir), hsa-miR-125b-2-3p (Assay ID 478666_mir), hsa-miR-125b-5p (Assay ID 477885_mir), hsa-miR-1260a (Assay ID 478476_mir), hsa-miR-1260b (Assay ID 477886_mir),hsa-miR-126-3p (Assay ID 477887_mir), hsa-miR-126-5p (Assay ID 477888_mir), hsa-miR-1268b (Assay ID 480790_mir), hsa-miR-1270 (Assay ID 478673_mir),hsa-miR-1271-5p (Assay ID 478674_mir), hsa-miR-1273h-3p (Assay ID 479563_mir), hsa-miR-127-3p (Assay ID 477889_mir), hsa-miR-1275 (Assay ID 477890_mir), hsa-miR-127-5p (Assay ID 477891_mir), hsa-miR-1277-3p (Assay ID 478219_mir), hsa-miR-1277-5p (Assay ID 480875_mir),hsa-miR-1283 (Assay ID 478685_mir),hsa-miR-128-3p (Assay ID 477892_mir), hsa-miR-1284 (Assay ID 478686_mir), hsa-miR-1285-3p (Assay ID 478687_mir), hsa-miR-1285-5p (Assay ID 479565_mir), hsa-miR-1287-5p (Assay ID 477894_mir), hsa-miR-1290 (Assay ID 477895_mir), hsa-miR-1291 (Assay ID 478690_mir),hsa-miR-129-1-3p (Assay ID 480873_mir), hsa-miR-129-2-3p (Assay ID 478544_mir), hsa-miR-1292-5p (Assay ID 478691_mir), hsa-miR-1293 (Assay ID 478692_mir),hsa-miR-1294 (Assay ID 478693_mir), hsa-miR-129-5p (Assay ID 477896_mir), hsa-miR-1296-5p (Assay ID 479451_mir), hsa-miR-1298-5p (Assay ID 479452_mir), hsa-miR-1299 (Assay ID 478696_mir),hsa-miR-1301-3p (Assay ID 477897_mir), hsa-miR-1302 (Assay ID 478697_mir), hsa-miR-1303 (Assay ID 478698_mir),hsa-miR-1306-3p (Assay ID 479575_mir), hsa-miR-1306-5p (Assay ID 478701_mir), hsa-miR-1307-3p (Assay ID 483036_mir), hsa-miR-1307-5p (Assay ID 479576_mir), hsa-miR-130a-3p (Assay ID 477851_mir), hsa-miR-130a-5p (Assay ID 483130_mir), hsa-miR-130b-3p (Assay ID 477840_mir),hsa-miR-130b-5p (Assay ID 477899_mir), hsa-miR-132-3p (Assay ID 477900_mir), hsa-miR-132-5p (Assay ID 478705_mir), hsa-miR-133a-3p (Assay ID 478511_mir), hsa-miR-133b (Assay ID 480871_mir), hsa-miR-134-5p (Assay ID 477901_mir), hsa-miR-135a-5p (Assay ID 478581_mir), hsa-miR-136-3p (Assay ID 477902_mir), hsa-miR-136-5p (Assay ID 478307_mir),hsa-miR-137-3p (Assay ID 477904_mir),hsa-miR-138-5p (Assay ID 477905_mir), hsa-miR-139-3p (Assay ID 477906_mir), hsa-miR-139-5p (Assay ID 478312_mir), hsa-miR-1-3p (Assay ID 477820_mir),hsa-miR-140-3p (Assay ID 477908_mir), hsa-miR-140-5p (Assay ID 477909_mir), hsa-miR-141-3p (Assay ID 478501_mir),hsa-miR-142-3p (Assay ID 477910_mir), hsa-miR-142-5p (Assay ID 477911_mir), hsa-miR-143-3p (Assay ID 477912_mir), hsa-miR-143-5p (Assay ID 478713_mir),hsa-miR-144-3p (Assay ID 477913_mir), hsa-miR-144-5p (Assay ID 477914_mir), hsa-miR-145-3p (Assay ID 477915_mir),hsa-miR-145-5p (Assay ID 477916_mir), hsa-miR-146a-3p (Assay ID 478714_mir), hsa-miR-146a-5p (Assay ID 478399_mir), hsa-miR-146b-3p (Assay ID 483103_mir), hsa-miR-146b-5p (Assay ID 483144_mir), hsa-miR-148a-3p (Assay ID 477814_mir), hsa-miR-148a-5p (Assay ID 478718_mir), hsa-miR-148b-3p (Assay ID 477824_mir), hsa-miR-148b-5p (Assay ID 478719_mir), hsa-miR-150-3p (Assay ID 478721_mir), hsa-miR-150-5p (Assay ID 477918_mir),hsa-miR-151a-3p (Assay ID 477919_mir), hsa-miR-151a-5p (Assay ID 478505_mir), hsa-miR-151b (Assay ID 477811_mir), hsa-miR-152-3p (Assay ID 477921_mir), hsa-miR-153-3p (Assay ID 477922_mir), hsa-miR-1537-3p (Assay ID 477923_mir), hsa-miR-154-3p (Assay ID 478725_mir),hsa-miR-154-5p (Assay ID 477925_mir), hsa-miR-155-5p (Assay ID 483064_mir), hsa-miR-15a-3p (Assay ID 477928_mir),hsa-miR-15a-5p (Assay ID 477858_mir), hsa-miR-15b-3p (Assay ID 477929_mir), hsa-miR-15b-5p (Assay ID 478313_mir), hsa-miR-16-1-3p (Assay ID 478727_mir),hsa-miR-16-2-3p (Assay ID 477931_mir), hsa-miR-16-5p (Assay ID 477860_mir),hsa-miR-17-3p (Assay ID 477932_mir), hsa-miR-17-5p (Assay ID 478447_mir), hsa-miR-181a-2-3p (Assay ID 479478_mir), hsa-miR-181a-3p (Assay ID 479405_mir), hsa-miR-181a-5p (Assay ID 477857_mir), hsa-miR-181b-5p (Assay ID 478583_mir), hsa-miR-181c-3p (Assay ID 477933_mir), hsa-miR-181c-5p (Assay ID 477934_mir), hsa-miR-181d-5p (Assay ID 479517_mir), hsa-miR-182-5p (Assay ID 477935_mir), hsa-miR-183-3p (Assay ID 477936_mir), hsa-miR-183-5p (Assay ID 477937_mir),hsa-miR-184 (Assay ID 477938_mir),hsa-miR-1843 (Assay ID 483104_mir), hsa-miR-185-3p (Assay ID 478732_mir), hsa-miR-185-5p (Assay ID 477939_mir), hsa-miR-186-3p (Assay ID 479544_mir),hsa-miR-186-5p (Assay ID 477940_mir), hsa-miR-187-3p (Assay ID 477941_mir),hsa-miR-188-3p (Assay ID 477942_mir), hsa-miR-188-5p (Assay ID 477943_mir), hsa-miR-18a-3p (Assay ID 477944_mir), hsa-miR-18a-5p (Assay ID 478551_mir), hsa-miR-18b-3p (Assay ID 478734_mir),hsa-miR-18b-5p (Assay ID 478584_mir), hsa-miR-1908-5p (Assay ID 478735_mir), hsa-miR-190a-5p (Assay ID 478358_mir), hsa-miR-190b-5p (Assay ID 483105_mir), hsa-miR-191-3p (Assay ID 477951_mir), hsa-miR-191-5p (Assay ID 477952_mir), hsa-miR-192-3p (Assay ID 478741_mir),hsa-miR-192-5p (Assay ID 478262_mir), hsa-miR-193a-3p (Assay ID 478306_mir), hsa-miR-193a-5p (Assay ID 477954_mir),hsa-miR-193b-3p (Assay ID 478314_mir),hsa-miR-193b-5p (Assay ID 478742_mir), hsa-miR-194-5p (Assay ID 477956_mir), hsa-miR-195-3p (Assay ID 478744_mir),hsa-miR-195-5p (Assay ID 477957_mir), hsa-miR-196a-5p (Assay ID 478230_mir), hsa-miR-196b-3p (Assay ID 477958_mir), hsa-miR-196b-5p (Assay ID 478585_mir), hsa-miR-197-3p (Assay ID 477959_mir), hsa-miR-1976 (Assay ID 478365_mir), hsa-miR-199a-3p (Assay ID 477961_mir),hsa-miR-199a-5p (Assay ID 478231_mir), hsa-miR-199b-5p (Assay ID 478486_mir), hsa-miR-19a-3p (Assay ID 479228_mir), hsa-miR-19b-1-5p (Assay ID 477962_mir), hsa-miR-19b-3p (Assay ID 478264_mir), hsa-miR-200a-3p (Assay ID 478490_mir), hsa-miR-200b-3p (Assay ID 477963_mir),hsa-miR-200c-3p (Assay ID 478351_mir), hsa-miR-202-5p (Assay ID 478755_mir), hsa-miR-203a-3p (Assay ID 478316_mir), hsa-miR-203b-3p (Assay ID 478757_mir), hsa-miR-203b-5p (Assay ID 478758_mir), hsa-miR-204-5p (Assay ID 478491_mir), hsa-miR-205-5p (Assay ID 477967_mir),hsa-miR-206 (Assay ID 477968_mir),hsa-miR-208a-3p (Assay ID 477819_mir), hsa-miR-208b-3p (Assay ID 477806_mir), hsa-miR-20a-3p (Assay ID 478317_mir), hsa-miR-20a-5p (Assay ID 478586_mir), hsa-miR-20b-5p (Assay ID 477804_mir), hsa-miR-210-3p (Assay ID 477970_mir),hsa-miR-2110 (Assay ID 477971_mir), hsa-miR-211-5p (Assay ID 478507_mir), hsa-miR-212-3p (Assay ID 478318_mir),hsa-miR-21-3p (Assay ID 477973_mir), hsa-miR-214-3p (Assay ID 477974_mir),hsa-miR-214-5p (Assay ID 478768_mir),hsa-miR-215-5p (Assay ID 478516_mir),hsa-miR-21-5p (Assay ID 477975_mir),hsa-miR-216b-5p (Assay ID 479456_mir),hsa-miR-218-5p (Assay ID 477977_mir),hsa-miR-219a-1-3p (Assay ID 477978_mir),hsa-miR-219a-5p (Assay ID 477980_mir),hsa-miR-219b-3p (Assay ID 478776_mir),hsa-miR-221-3p (Assay ID 477981_mir),hsa-miR-221-5p (Assay ID 478778_mir),hsa-miR-222-3p (Assay ID 477982_mir),hsa-miR-223-3p (Assay ID 477983_mir),hsa-miR-223-5p (Assay ID 477984_mir),hsa-miR-22-3p (Assay ID 477985_mir),hsa-miR-224-3p (Assay ID 478780_mir),hsa-miR-224-5p (Assay ID 483106_mir), hsa-miR-22-5p (Assay ID 477987_mir),hsa-miR-2355-3p (Assay ID 477989_mir),hsa-miR-23a-3p (Assay ID 478532_mir),hsa-miR-23a-5p (Assay ID 478782_mir), hsa-miR-23b-3p (Assay ID 483150_mir),hsa-miR-23b-5p (Assay ID 477991_mir), hsa-miR-24-1-5p (Assay ID 478784_mir), hsa-miR-24-2-5p (Assay ID 478785_mir), hsa-miR-24-3p (Assay ID 477992_mir),hsa-miR-25-3p (Assay ID 477994_mir),hsa-miR-25-5p (Assay ID 478786_mir), hsa-miR-26a-1-3p (Assay ID 478787_mir),hsa-miR-26a-5p (Assay ID 477995_mir),hsa-miR-26b-3p (Assay ID 483077_mir),hsa-miR-26b-5p (Assay ID 478418_mir), hsa-miR-27a-3p (Assay ID 478384_mir),hsa-miR-27a-5p (Assay ID 477998_mir),hsa-miR-27b-3p (Assay ID 478270_mir), hsa-miR-27b-5p (Assay ID 478789_mir),hsa-miR-28-3p (Assay ID 477999_mir),hsa-miR-28-5p (Assay ID 478000_mir),hsa-miR-296-5p (Assay ID 477836_mir),hsa-miR-299-3p (Assay ID 478792_mir),hsa-miR-299-5p (Assay ID 478793_mir),hsa-miR-29a-3p (Assay ID 478587_mir),hsa-miR-29a-5p (Assay ID 478002_mir),hsa-miR-29b-1-5p (Assay ID 478794_mir),hsa-miR-29b-2-5p (Assay ID 478003_mir),hsa-miR-29b-3p (Assay ID 478369_mir),hsa-miR-29c-3p (Assay ID 479229_mir), hsa-miR-29c-5p (Assay ID 478005_mir),hsa-miR-301a-3p (Assay ID 477815_mir),hsa-miR-301b-3p (Assay ID 477825_mir),hsa-miR-302b-3p (Assay ID 478591_mir),hsa-miR-302d-3p (Assay ID 478237_mir),hsa-miR-3065-3p (Assay ID 479605_mir),hsa-miR-3065-5p (Assay ID 478802_mir), hsa-miR-3074-5p (Assay ID 479606_mir),hsa-miR-30a-3p (Assay ID 478273_mir),hsa-miR-30a-5p (Assay ID 479448_mir),hsa-miR-30b-3p (Assay ID 478804_mir), hsa-miR-30b-5p (Assay ID 478007_mir),hsa-miR-30c-1-3p (Assay ID 479412_mir), hsa-miR-30c-5p(Assay ID 478008_mir), hsa-miR-30d-5p (Assay ID 478606_mir),hsa-miR-30e-3p (Assay ID 478388_mir), hsa-miR-30e-5p (Assay ID 479235_mir), hsa-miR-3115 (Assay ID 479607_mir),hsa-miR-3120-3p (Assay ID 478009_mir),hsa-miR-3120-5p (Assay ID 479611_mir), hsa-miR-3130-5p (Assay ID 479621_mir),hsa-miR-31-3p (Assay ID 478012_mir),hsa-miR-3140-3p (Assay ID 479626_mir), hsa-miR-3158-5p (Assay ID 479644_mir),hsa-miR-31-5p (Assay ID 478015_mir),hsa-miR-3173-5p (Assay ID 479656_mir), hsa-miR-3177-3p (Assay ID 479658_mir),hsa-miR-3184-3p (Assay ID 479662_mir),hsa-miR-3184-5p (Assay ID 478817_mir),hsa-miR-3194-5p (Assay ID 479673_mir),hsa-miR-320a-3p (Assay ID 478594_mir),hsa-miR-320b (Assay ID 478588_mir), hsa-miR-320e (Assay ID 478022_mir), hsa-miR-323a-3p (Assay ID 477853_mir),hsa-miR-323b-3p (Assay ID 478825_mir),hsa-miR-323b-5p (Assay ID 478826_mir),hsa-miR-32-3p (Assay ID 478827_mir), hsa-miR-324-3p (Assay ID 483109_mir),hsa-miR-324-5p (Assay ID 483066_mir),hsa-miR-325 (Assay ID 478025_mir), hsa-miR-32-5p (Assay ID 478026_mir),hsa-miR-326 (Assay ID 478027_mir),hsa-miR-328-3p (Assay ID 478028_mir), hsa-miR-329-3p (Assay ID 478029_mir),hsa-miR-330-3p (Assay ID 478030_mir),hsa-miR-330-5p (Assay ID 478830_mir),hsa-miR-331-3p (Assay ID 478323_mir), hsa-miR-331-5p (Assay ID 478032_mir),hsa-miR-335-3p (Assay ID 478033_mir), hsa-miR-335-5p (Assay ID 478324_mir), hsa-miR-337-3p (Assay ID 478035_mir),hsa-miR-337-5p (Assay ID 478036_mir),hsa-miR-338-3p (Assay ID 478037_mir),hsa-miR-338-5p (Assay ID 478038_mir),hsa-miR-339-3p (Assay ID 478325_mir),hsa-miR-339-5p (Assay ID 478040_mir),hsa-miR-33a-3p (Assay ID 478831_mir), hsa-miR-33a-5p (Assay ID 478347_mir),hsa-miR-33b-5p (Assay ID 478479_mir),hsa-miR-340-3p (Assay ID 478041_mir),hsa-miR-340-5p (Assay ID 478042_mir), hsa-miR-342-3p (Assay ID 478043_mir),hsa-miR-342-5p (Assay ID 478044_mir), hsa-miR-345-5p (Assay ID 478366_mir), hsa-miR-346 (Assay ID 478046_mir),hsa-miR-34a-3p (Assay ID 478047_mir),hsa-miR-34a-5p (Assay ID 478048_mir),hsa-miR-34c-3p (Assay ID 478051_mir), hsa-miR-34c-5p (Assay ID 478052_mir), hsa-miR-3529-3p (Assay ID 480652_mir),hsa-miR-3611 (Assay ID 480655_mir),hsa-miR-3613-3p (Assay ID 478434_mir), hsa-miR-3613-5p (Assay ID 479424_mir),hsa-miR-361-3p (Assay ID 478055_mir),hsa-miR-3615 (Assay ID 478837_mir), hsa-miR-361-5p (Assay ID 478056_mir), hsa-miR-362-3p (Assay ID 478058_mir),hsa-miR-362-5p (Assay ID 478059_mir), hsa-miR-363-3p (Assay ID 478060_mir),hsa-miR-363-5p (Assay ID 478840_mir), hsa-miR-365a-3p (Assay ID 478065_mir),hsa-miR-3667-5p (Assay ID 479704_mir), hsa-miR-367-3p (Assay ID 478066_mir),hsa-miR-369-3p (Assay ID 478067_mir),hsa-miR-369-5p (Assay ID 478068_mir), hsa-miR-370-3p (Assay ID 478326_mir),hsa-miR-373-3p (Assay ID 478363_mir), hsa-miR-373-5p (Assay ID 478073_mir), hsa-miR-374a-3p (Assay ID 478855_mir),hsa-miR-374a-5p (Assay ID 478238_mir),hsa-miR-374b-3p (Assay ID 479421_mir), hsa-miR-374b-5p (Assay ID 478389_mir), hsa-miR-374c-3p (Assay ID 478856_mir),hsa-miR-375-3p (Assay ID 478074_mir),hsa-miR-376a-2-5p (Assay ID 478858_mir), hsa-miR-376a-3p (Assay ID 478240_mir),hsa-miR-376a-5p (Assay ID 478859_mir),hsa-miR-376b-3p (Assay ID 478860_mir),hsa-miR-376b-5p (Assay ID 478861_mir),hsa-miR-376c-3p (Assay ID 478459_mir),hsa-miR-377-3p (Assay ID 478075_mir),hsa-miR-377-5p (Assay ID 478863_mir),hsa-miR-378a-3p (Assay ID 478349_mir),hsa-miR-378a-5p (Assay ID 478076_mir),hsa-miR-378c (Assay ID 478864_mir),hsa-miR-378i (Assay ID 478305_mir),hsa-miR-379-5p (Assay ID 478077_mir),hsa-miR-380-3p (Assay ID 477854_mir), hsa-miR-381-3p (Assay ID 477816_mir),hsa-miR-381-5p (Assay ID 478866_mir),hsa-miR-382-3p (Assay ID 479458_mir), hsa-miR-382-5p (Assay ID 478078_mir),hsa-miR-3913-3p (Assay ID 479734_mir),hsa-miR-3913-5p (Assay ID 479735_mir),hsa-miR-3928-3p (Assay ID 479749_mir),hsa-miR-3940-3p (Assay ID 479759_mir),hsa-miR-409-3p (Assay ID 478084_mir),hsa-miR-409-5p (Assay ID 478872_mir), hsa-miR-410-3p (Assay ID 478085_mir),hsa-miR-411-3p (Assay ID 479526_mir), hsa-miR-411-5p (Assay ID 478086_mir), hsa-miR-421 (Assay ID 478088_mir),hsa-miR-422a (Assay ID 478481_mir),hsa-miR-423-3p (Assay ID 478327_mir),hsa-miR-423-5p (Assay ID 478090_mir), hsa-miR-424-3p (Assay ID 478091_mir), hsa-miR-424-5p (Assay ID 478092_mir), hsa-miR-4253 (Assay ID 480774_mir),hsa-miR-425-3p (Assay ID 478093_mir),hsa-miR-425-5p (Assay ID 478094_mir),hsa-miR-4286 (Assay ID 478096_mir),hsa-miR-429 (Assay ID 477849_mir),hsa-miR-4306 (Assay ID 478392_mir), hsa-miR-431-3p (Assay ID 478888_mir),hsa-miR-431-5p (Assay ID 478889_mir), hsa-miR-432-3p (Assay ID 478892_mir),hsa-miR-432-5p (Assay ID 478101_mir), hsa-miR-433-3p (Assay ID 478102_mir), hsa-miR-4429 (Assay ID 480852_mir),hsa-miR-4433a-3p (Assay ID 478897_mir),hsa-miR-4433a-5p (Assay ID 479801_mir),hsa-miR-4433b-3p (Assay ID 479802_mir), hsa-miR-4433b-5p (Assay ID 479803_mir), hsa-miR-4444 (Assay ID 479810_mir),hsa-miR-4446-3p (Assay ID 479812_mir),hsa-miR-4448 (Assay ID 480683_mir), hsa-miR-4454 (Assay ID 478104_mir),hsa-miR-4466 (Assay ID 483160_mir),hsa-miR-448 (Assay ID 478105_mir),hsa-miR-4488 (Assay ID 478906_mir),hsa-miR-449a (Assay ID 478561_mir),hsa-miR-450a-5p (Assay ID 478106_mir),hsa-miR-450b-3p (Assay ID 478913_mir), hsa-miR-450b-5p (Assay ID 478914_mir), hsa-miR-4516 (Assay ID 478303_mir),hsa-miR-451a (Assay ID 478107_mir),hsa-miR-4523 (Assay ID 479852_mir),hsa-miR-452-3p (Assay ID 478917_mir),hsa-miR-452-5p (Assay ID 478109_mir),hsa-miR-4537 (Assay ID 480691_mir),hsa-miR-4538 (Assay ID 478110_mir),hsa-miR-454-3p (Assay ID 478329_mir), hsa-miR-454-5p (Assay ID 478919_mir),hsa-miR-455-3p (Assay ID 478112_mir),hsa-miR-455-5p (Assay ID 478113_mir),hsa-miR-4662a-5p (Assay ID 479896_mir), hsa-miR-4669 (Assay ID 478925_mir),hsa-miR-4684-5p (Assay ID 479920_mir),hsa-miR-4685-3p (Assay ID 479921_mir),hsa-miR-4689 (Assay ID 478117_mir),hsa-miR-4690-3p (Assay ID 480702_mir),hsa-miR-4732-3p (Assay ID 478118_mir),hsa-miR-4732-5p (Assay ID 478119_mir), hsa-miR-4747-5p (Assay ID 480001_mir),hsa-miR-483-3p (Assay ID 478122_mir),hsa-miR-483-5p (Assay ID 478432_mir),hsa-miR-484 (Assay ID 478308_mir), hsa-miR-485-3p (Assay ID 478125_mir),hsa-miR-485-5p (Assay ID 478126_mir),hsa-miR-486-3p (Assay ID 478422_mir), hsa-miR-486-5p (Assay ID 478128_mir),hsa-miR-487a-3p (Assay ID 477826_mir),hsa-miR-487b-3p (Assay ID 477835_mir),hsa-miR-489-3p (Assay ID 478130_mir),hsa-miR-490-3p (Assay ID 478131_mir),hsa-miR-491-5p (Assay ID 478132_mir),hsa-miR-493-3p (Assay ID 478134_mir), hsa-miR-493-5p (Assay ID 478943_mir),hsa-miR-494-3p (Assay ID 478135_mir), hsa-miR-495-3p (Assay ID 478136_mir), hsa-miR-496 (Assay ID 478335_mir),hsa-miR-497-5p (Assay ID 478138_mir), hsa-miR-499a-5p (Assay ID 478139_mir),hsa-miR-499b-3p (Assay ID 478949_mir),hsa-miR-500a-3p (Assay ID 478951_mir),hsa-miR-500a-5p (Assay ID 478309_mir),hsa-miR-5010-3p (Assay ID 480086_mir),hsa-miR-501-3p (Assay ID 478350_mir),hsa-miR-501-5p (Assay ID 478142_mir), hsa-miR-502-3p (Assay ID 478348_mir),hsa-miR-502-5p (Assay ID 478954_mir), hsa-miR-503-5p (Assay ID 478143_mir), hsa-miR-504-5p (Assay ID 478144_mir),hsa-miR-505-3p (Assay ID 478145_mir),hsa-miR-505-5p (Assay ID 478957_mir),hsa-miR-509-3-5p (Assay ID 478963_mir), hsa-miR-5100 (Assay ID 480099_mir),hsa-miR-513b-5p (Assay ID 479297_mir), hsa-miR-513c-5p (Assay ID 479349_mir),hsa-miR-515-3p (Assay ID 478976_mir),hsa-miR-516a-5p (Assay ID 478978_mir),hsa-miR-516b-5p (Assay ID 478979_mir), hsa-miR-517a-3p (Assay ID 479485_mir), hsa-miR-517c-3p (Assay ID 479487_mir),hsa-miR-5187-5p (Assay ID 480101_mir),hsa-miR-518c-3p (Assay ID 478982_mir),hsa-miR-518d-3p (Assay ID 479393_mir),hsa-miR-518d-5p (Assay ID 479530_mir),hsa-miR-518e-3p (Assay ID 479408_mir),hsa-miR-518f-3p (Assay ID 478984_mir), hsa-miR-519a-3p (Assay ID 479534_mir),hsa-miR-519d-3p (Assay ID 478986_mir),hsa-miR-520d-3p (Assay ID 478990_mir),hsa-miR-520d-5p (Assay ID 478616_mir),hsa-miR-523-3p (Assay ID 478994_mir),hsa-miR-524-3p (Assay ID 479338_mir),hsa-miR-525-3p (Assay ID 478995_mir),hsa-miR-532-3p (Assay ID 478336_mir),hsa-miR-532-5p (Assay ID 478151_mir),hsa-miR-539-5p (Assay ID 478152_mir), hsa-miR-541-3p (Assay ID 478999_mir),hsa-miR-542-3p (Assay ID 478153_mir),hsa-miR-542-5p (Assay ID 478337_mir),hsa-miR-543 (Assay ID 478155_mir), hsa-miR-544a (Assay ID 478156_mir),hsa-miR-545-3p (Assay ID 479002_mir), hsa-miR-545-5p (Assay ID 479003_mir), hsa-miR-548a-3p (Assay ID 478157_mir),hsa-miR-548a-5p (Assay ID 479501_mir),hsa-miR-548aa (Assay ID 479004_mir),hsa-miR-548aj-5p (Assay ID 479009_mir), hsa-miR-548am-5p (Assay ID 480872_mir),hsa-miR-548b-3p (Assay ID 479018_mir),hsa-miR-548b-5p (Assay ID 478589_mir),hsa-miR-548c-3p (Assay ID 479537_mir),hsa-miR-548d-3p (Assay ID 477833_mir),hsa-miR-548d-5p (Assay ID 480870_mir),hsa-miR-548e-3p (Assay ID 478362_mir),hsa-miR-548g-3p (Assay ID 479020_mir),hsa-miR-548h-5p (Assay ID 479503_mir),hsa-miR-548i (Assay ID 480874_mir),hsa-miR-548j-5p (Assay ID 479022_mir),hsa-miR-548k (Assay ID 479374_mir),hsa-miR-548l (Assay ID 479391_mir),hsa-miR-548m (Assay ID 479023_mir),hsa-miR-548n (Assay ID 479024_mir),hsa-miR-548p (Assay ID 479025_mir),hsa-miR-550a-3-5p (Assay ID 479031_mir),hsa-miR-550a-3p (Assay ID 479032_mir), hsa-miR-550a-5p (Assay ID 477852_mir),hsa-miR-550b-2-5p (Assay ID 479033_mir), hsa-miR-551a (Assay ID 478158_mir),hsa-miR-551b-3p (Assay ID 478159_mir),hsa-miR-552-3p (Assay ID 479036_mir),hsa-miR-553 (Assay ID 479038_mir), hsa-miR-556-3p (Assay ID 479041_mir),hsa-miR-5582-3p (Assay ID 480123_mir),hsa-miR-5588-5p (Assay ID 480135_mir),hsa-miR-562 (Assay ID 479047_mir),hsa-miR-570-3p (Assay ID 479053_mir),hsa-miR-574-3p (Assay ID 478163_mir),hsa-miR-574-5p (Assay ID 479357_mir), hsa-miR-576-3p (Assay ID 478164_mir),hsa-miR-576-5p (Assay ID 478165_mir), hsa-miR-579-3p (Assay ID 479059_mir), hsa-miR-580-3p (Assay ID 479061_mir),hsa-miR-582-5p (Assay ID 478166_mir),hsa-miR-583 (Assay ID 479065_mir),hsa-miR-584-5p (Assay ID 478167_mir),hsa-miR-585-3p (Assay ID 479067_mir),hsa-miR-589-3p (Assay ID 479072_mir), hsa-miR-589-5p (Assay ID 479073_mir), hsa-miR-590-3p (Assay ID 478168_mir),hsa-miR-590-5p (Assay ID 478367_mir),hsa-miR-592 (Assay ID 479075_mir),hsa-miR-593-3p (Assay ID 479076_mir),hsa-miR-597-5p (Assay ID 478339_mir),hsa-miR-598-3p (Assay ID 478172_mir), hsa-miR-605-5p (Assay ID 478174_mir), hsa-miR-606 (Assay ID 479087_mir),hsa-miR-608 (Assay ID 479089_mir), hsa-miR-615-3p (Assay ID 478175_mir),hsa-miR-616-5p (Assay ID 479099_mir),hsa-miR-618 (Assay ID 479101_mir),hsa-miR-619-5p (Assay ID 479103_mir),hsa-miR-624-3p (Assay ID 479108_mir), hsa-miR-624-5p (Assay ID 478178_mir),hsa-miR-625-3p (Assay ID 478179_mir), hsa-miR-625-5p (Assay ID 479469_mir), hsa-miR-627-3p (Assay ID 479111_mir),hsa-miR-627-5p (Assay ID 478427_mir),hsa-miR-628-3p (Assay ID 478181_mir),hsa-miR-628-5p (Assay ID 479112_mir), hsa-miR-629-3p (Assay ID 478182_mir),hsa-miR-629-5p (Assay ID 478183_mir), hsa-miR-633 (Assay ID 479115_mir),hsa-miR-636 (Assay ID 478185_mir),hsa-miR-641 (Assay ID 479120_mir), hsa-miR-642a-5p (Assay ID 479121_mir),hsa-miR-643 (Assay ID 479123_mir),hsa-miR-6501-5p (Assay ID 480194_mir),hsa-miR-6503-5p (Assay ID 480197_mir),hsa-miR-6513-5p (Assay ID 480213_mir),hsa-miR-651-5p (Assay ID 479131_mir),hsa-miR-652-3p (Assay ID 478189_mir),hsa-miR-652-5p (Assay ID 479132_mir),hsa-miR-653-5p (Assay ID 479134_mir),hsa-miR-654-3p (Assay ID 479135_mir), hsa-miR-654-5p (Assay ID 478368_mir),hsa-miR-655-3p (Assay ID 478191_mir),hsa-miR-656-3p (Assay ID 479137_mir),hsa-miR-660-3p (Assay ID 479143_mir), hsa-miR-660-5p (Assay ID 478192_mir),hsa-miR-663b (Assay ID 479146_mir),hsa-miR-664a-3p (Assay ID 478193_mir),hsa-miR-665 (Assay ID 479150_mir),hsa-miR-668-3p (Assay ID 479151_mir),hsa-miR-671-3p (Assay ID 478194_mir),hsa-miR-671-5p (Assay ID 483035_mir),hsa-miR-6727-5p (Assay ID 480235_mir),hsa-miR-6729-3p (Assay ID 480238_mir),hsa-miR-6734-5p (Assay ID 480247_mir),hsa-miR-6741-5p (Assay ID 480260_mir),hsa-miR-6748-3p (Assay ID 480270_mir),hsa-miR-675-5p (Assay ID 478196_mir),hsa-miR-6757-5p (Assay ID 480286_mir),hsa-miR-6765-5p (Assay ID 480300_mir),hsa-miR-6803-3p (Assay ID 480372_mir),hsa-miR-6852-5p (Assay ID 480461_mir),hsa-miR-6881-5p (Assay ID 480513_mir),hsa-miR-6882-5p (Assay ID 480515_mir), hsa-miR-708-3p (Assay ID 479162_mir),hsa-miR-708-5p (Assay ID 478197_mir), hsa-miR-7114-3p (Assay ID 480554_mir),hsa-miR-7-1-3p (Assay ID 478198_mir),hsa-miR-744-3p (Assay ID 479165_mir),hsa-miR-744-5p (Assay ID 478200_mir),hsa-miR-758-3p (Assay ID 479166_mir),hsa-miR-7-5p (Assay ID 483061_mir),hsa-miR-766-3p (Assay ID 478342_mir),hsa-miR-766-5p (Assay ID 479174_mir), hsa-miR-769-3p (Assay ID 479177_mir),hsa-miR-769-5p (Assay ID 478203_mir),hsa-miR-7704 (Assay ID 480576_mir),hsa-miR-770-5p (Assay ID 479178_mir), hsa-miR-7706 (Assay ID 480578_mir),hsa-miR-7847-3p (Assay ID 480584_mir),hsa-miR-7976 (Assay ID 480595_mir),hsa-miR-7977 (Assay ID 479180_mir),hsa-miR-8077 (Assay ID 480619_mir), hsa-miR-873-5p (Assay ID 478204_mir),hsa-miR-874-3p (Assay ID 478205_mir), hsa-miR-876-3p (Assay ID 479186_mir), hsa-miR-877-3p (Assay ID 477856_mir),hsa-miR-877-5p (Assay ID 478206_mir),hsa-miR-885-3p (Assay ID 479188_mir),hsa-miR-885-5p (Assay ID 478207_mir), hsa-miR-887-3p (Assay ID 479189_mir),hsa-miR-888-5p (Assay ID 479192_mir), hsa-miR-889-3p (Assay ID 478208_mir), hsa-miR-891a-5p (Assay ID 479196_mir),hsa-miR-92a-1-5p (Assay ID 479205_mir),hsa-miR-92a-3p (Assay ID 477827 _mir),hsa-miR-92b-3p (Assay ID 477823_mir),hsa-miR-92b-5p (Assay ID 479207_mir),hsa-miR-933 (Assay ID 479208_mir),hsa-miR-93-3p (Assay ID 478209_mir),hsa-miR-935 (Assay ID 479210_mir), hsa-miR-93-5p (Assay ID 478210_mir), hsa-miR-937-3p (Assay ID 479212_mir),hsa-miR-939-5p (Assay ID 478245_mir), hsa-miR-9-3p (Assay ID 478211_mir),hsa-miR-941 (Assay ID 479217_mir),hsa-miR-942-5p (Assay ID 478212_mir), hsa-miR-943 (Assay ID 479219_mir),hsa-miR-944 (Assay ID 479220_mir),hsa-miR-95-3p (Assay ID 478213_mir),hsa-miR-9-5p (Assay ID 478214_mir),hsa-miR-96-5p (Assay ID 478215_mir), hsa-miR-98-3p (Assay ID 479223_mir),hsa-miR-98-5p (Assay ID 478590_mir),hsa-miR-99a-3p (Assay ID 479224_mir), hsa-miR-99a-5p (Assay ID 478519_mir),hsa-miR-99b-3p (Assay ID 478216_mir),hsa-miR-99b-5p (Assay ID 478343_mir),hsa-miR-199a-3p (Assay ID 477961_mir),hsa-miR-365a-3p (Assay ID 478065_mir), ThermoFisher, Cat. No. A25576), including cel-miR-39-3p (Assay ID 478293_mir, ThermoFisher, Cat. No. A25576), were quantified using the TaqMan Open Array technology (ThermoFisher), as per the manufacturer's instructions. cDNA was diluted 1:20 in 0.1X TE (pH 8.0), combined with TaqMan OpenArray Real-TimePCR Master Mix, loaded onto the OpenArray^{™} plates (AccuFill^{™} system, Cat. No. 4471021) and amplified with the QuantStudio 12K Flex Real-Time PCR System (ThermoFisher, Cat. No. 4472379). Technical duplicates were performed in each OpenArray^{™} plates. OpenArray^{®} microRNA profiling data were processed and filtered using Python scripts. Briefly, QuantStudio 12K Flex Software assigns a Cq value and an Amplification score (Amp Score) and a Cq Confidence (Cq Conf.) value to each reaction for rapid assessment of data reliability. Data were filtered to exclude reactions with Amp Score < 1.00, Cq Conf. < 0.8, and Cq below 10 or above 28. In addition, a miRNA was considered as detected only if passes that criteria in at least 70% of the samples.

The concentrations from plasma samples were normalized using the global mean normalization method: the value of ΔCq was calculated by subtracting the mean of Cq values of all analyzed miRNAs from the mean of Cq values of each miRNA, in each patient sample. Statistical analysis was performed on the ΔCq values. In order to assess the change in gene expression, the values of ΔΔCq were then calculated by subtracting the mean of ΔCq of a group of samples from the mean of ΔCq of the comparison group of samples. The fold changes were calculated as 2^{-ΔΔCq}.

### Statistical analysis

As regarding the analysis of UMOD genotype, the chi-square test was used to determine significant differences in genotype frequencies in the UMOD gene between different patients' groups, defined on the basis of the different outcomes (screening, diagnosis, and prognosis).

As regarding the analysis of circulating plasma miRNAs, the Mann-Whitney test was used for the identification of statistically differentially regulated miRNAs between different patients' groups, defined on the basis of the different outcomes (screening, diagnosis, and prognosis). Any miRNA with Amp Score < 1.00 and/or Cq Conf. < 0.8 and/or Cq below 10 or above 28 in > 30% of the samples were filtered out, resulting in 274 miRNAs, including cel-miR-39-3p. miRNAs which exhibit high percentage of missing values were removed only after checking through statistical test that the missingness pattern is not associated with the outcome.

The small percentage (1.68%) of missing values were imputed through an iterative procedure based on Bayesian Linear Regression. All statistical tests were two-sided with a significance level set at p < 0.05 and were performed using python version 3.7.

In addition to this single biomarker analysis, a machine learning supervised classification of samples for the various forecasting tasks was performed by using Support Vector Machines (SVM) as implemented in the Scikit-Learn package for the python programming languages (python, version 3.7). As parameters, a linear kernel together with l1/l2 penalties and a cost parameter sampled from 0.01 to 10 was evaluated. As subset selection technique, a bootstrap simulation based on recursive feature elimination was used. This allowed to rank by importance the variables in analysis. A further reduction of the dimensionality of the obtained features was achieved by means of Bayesian optimisation. The most performing subsets were used to train the SVM and to carry out the prediction of the test samples in a 10-fold cross validation procedure (stratifying by outcome in order to account for class imbalances). To test for overfitting, permutation simulations were performed.

In order to evaluate the performances of each classification models the inventors investigated three different metrics: accuracy, recall, Receiver Operating Characteristic Area Under the Curve (ROC AUC). Accuracy is the ratio between the actual correctly predicted population on the total population. Recall is the ratio between the actual positive predicted population on all the positive predicted population. The ROC AUC is the area under the Receiver Operating Characteristic curve, created by plotting the recall against 1- specificity.

### EXAMPLES

Example 1: Variants in the UMOD gene associated with high risk of RCC and high risk of aggressive RCC

The inventors have previously demonstrated in a cohort of 211 patients diagnosed with a renal mass and treated with surgery that the TT-rs4293393 variant in the UMOD gene is associated with a more aggressive clinical and pathological RCC phenotype and, in particular, with clinical metastasis, and high stage. [Trevisani et al., 2017].

The inventors extended their analysis to other 21 UMOD SNP variants: 15 (rs12917707, rs12934320, rs12934455, rs13329952, rs13333226, rs13335818, rs28362063, rs28544423, rs28640218, rs28688991, rs34356953, rs60136849, rs71384446, rs7203642, rs9928757) and 3 (rs11647727, rs4297685, rs7193058) SNPs in linkage disequilibrium (LD) with rs4293393 with a r² > 0.8 and a 0.5 ≤ r2 ≤ 0.7, respectively; and 3 SNPs (rs11862974, rs7204342, rs76563024) not in LD with rs4293393 in the European/Caucasoid population.

Pairwise r² values for those SNPs are reported in Figure 1.

Those SNPs have a different location in the UMOD locus: 1 SNPs is upstream of UMOD gene promoter; 9 SNPs are in the UMOD gene promoter; 9 SNPs are intron variants, located in introns 1, 4, 5, and 7; and 3 SNPs are synonymous variant, two of them in the exon 3 and the other in the exon 4 (Figure 2).

All those variants are important for different reasons: variants located in the UMOD gene promoter could be associated with an effect on gene expression [Trudu et al., 2013]; intronic variants can lead to pseudo-exon inclusion, due to activation of non-canonical splice sites or changes in splicing regulatory elements, or disrupt transcription regulatory motifs and non-coding RNA genes [Vaz-Drago et al., 2017]; synonymous variants could affect protein function altering RNA secondary structures, affecting RNA stability, and subsequently reducing protein expression [Nackley et al., 2006] and possibly affecting protein folding and function [Kimchi-Sarfaty et al. 2007]; synonymous variants could also affect splicing regulatory sequences as enhancers or silencers of splicing.

A total of 276 renal mass patients and 83 controls were genotyped for the 22 SNPs listed above. Minor allele frequencies (MAF), ranging from 0.03 to 0.3 (MAF observed), were similar to those reported in the literature (MAF (100Genome, EUR) (Table 4).

**Table 4. Minor allele frequency (MAF) among the overall population (n = 359).**

| bp position | SNP | Minor Allele | MAF (1000Genome, EUR) | MAF (observed) |
|---|---|---|---|---|
| 20339137 | rs71384446 | G | *0.25* | 0.23 |
| 20341541 | rs9928757 | G | *0.23* | 0.20 |
| 20342493 | rs60136849 | G | *0.20* | 0.18 |
| 20344843 | rs11647727 | T | *0.28* | 0.30 |
| 20345004 | rs34356953 | A | *0.20* | 0.18 |
| 20345933 | rs12934320 | A | *0.20* | 0.18 |
| 20345959 | rs12934455 | A | *0.19* | 0.15 |
| 20347945 | rs28640218 | A | *0.21* | 0.20 |
| 20348311 | rs28544423 | A | *0.20* | 0.18 |
| 20348509 | rs13335818 | A | *0.20* | 0.18 |
| 20348779 | rs7193058 | C | *0.28* | 0.30 |
| 20352517 | rs76563024 | C | *0.01* | 0.03 |
| 20353266 | *rs4293393* | C | *0.20* | 0.18 |
| 20353690 | rs28362063 | G | *0.20* | 0.18 |
| 20354332 | rs13333226 | C | *0.20* | 0.19 |
| 20354903 | rs11862974 | T | *0.01* | 0.03 |
| 20355185 | rs13329952 | G | *0.22* | 0.21 |
| 20355808 | rs7203642 | C | *0.22* | 0.20 |
| 20355917 | rs7204342 | A | *0.01* | 0.04 |
| 20356228 | rs28688991 | A | *0.22* | 0.22 |
| 20356368 | rs12917707 | A | *0.20* | 0.18 |
| 20357242 | rs4297685 | G | *0.33* | 0.30 |

| | | | | |
|---|---|---|---|---|
| * Information available at: http://www.ncbi.nlm.nih.gov/projects/SNP/. | | | | |

For statistical analysis of the SNPs with a MAF between 0.03 and 0.23, the inventors grouped patients carrying one or two copies of the minor allele variant and compared them with patients homozygous for the major allele variant. For statistical analysis of rs11647727, rs7193058, and rs4297685 the inventors maintained the three genotypic classes.

As shown in Table 5, rs4293393 as well as 14 SNPs having strong or perfect LD (linkage disequilibrium) with rs4293393 (rs13333226, rs12917707, rs13335818, rs28362063, rs28544423, rs34356953, rs60136849, rs12934320, rs13329952, rs28688991, rs7203642, rs9928757, rs12934455, rs28640218) were significantly associated with pT3-pT4 stage, most of them showing a very similar or identical p value, as expected, ranging from 0.001 and 0.01.

Only rs71384446, even if in LD with rs4293393 with a r²> 0.8, were not significantly associated with pT3-pT4 stage with a p value of 0.06, which could reach statistical significance by increasing the study cohort.

On the other hand, none of the SNPs having a weak or no LD with rs4293393 were significantly associated with pT3-pT4 stage. Only rs11647727showed a p value of 0.05, at the limits of significance.

**Table 5. Association between UMOD SNP variants and higher pathologic T stage (pT3-pT4).**

| **SNP** | **Variable** | **RCC patients (n = 231)** | **Genotypic frequencies (%)** | | | | | | **p value** |
|---|---|---|---|---|---|---|---|---|---|
| rs71384446 | | | | | | | | | **0.06** |
| | pT3-pT4 | 84 | | | GG + GT | 30.95 | TT | 69.05 | |
| | pT1-pT2 | 147 | | | GG + GT | 43.54 | TT | 56.46 | |
| rs9928757 | | | | | | | | | **0.009** |
| | pT3-pT4 | 84 | | | GG + CG | 22.62 | CC | 77.38 | |
| | pT1-pT2 | 147 | | | GG + CG | 39.46 | CC | 60.54 | |
| rs60136849 | | | | | | | | | **0.004** |
| | pT3-pT4 | 84 | | | GG + GA | 19.05 | AA | 80.95 | |
| | pT1-pT2 | 147 | | | GG + GA | 37.41 | AA | 62.59 | |
| rs11647727 | | | | | | | | | **0.05** |
| | pT3-pT4 | 84 | TT | 2.38 | CT | 34.52 | CC | 63.10 | |
| | pT1-pT2 | 147 | TT | 6.80 | CT | 45.58 | CC | 47.62 | |
| rs34356953 | | | | | | | | | **0.003** |
| | pT3-pT4 | 84 | | | AA + AC | 19.05 | CC | 80.95 | |
| | pT1-pT2 | 147 | | | AA + AC | 38.10 | CC | 61.90 | |
| rs12934320 | | | | | | | | | **0.005** |
| | pT3-pT4 | 84 | | | AA + AG | 19.05 | GG | 80.95 | |
| | pT1-pT2 | 147 | | | AA + AG | 36.73 | GG | 63.27 | |
| rs12934455 | | | | | | | | | **0.01** |
| | pT3-pT4 | 84 | | | AA + AG | 16.67 | GG | 83.33 | |
| | pT1-pT2 | 147 | | | AA + AG | 31.97 | GG | 68.03 | |
| rs28640218 | | | | | | | | | **0.01** |
| | pT3-pT4 | 84 | | | AA + AC | 22.62 | CC | 77.38 | |
| | pT1-pT2 | 147 | | | AA + AC | 38.78 | CC | 61.22 | |
| rs28544423 | | | | | | | | | **0.003** |
| | pT3-pT4 | 84 | | | AA + AG | 19.05 | GG | 80.95 | |
| | pT1-pT2 | 147 | | | AA + AG | 38.10 | GG | 61.90 | |
| rs13335818 | | | | | | | | | **0.003** |
| | pT3-pT4 | 84 | | | AA + AG | 19.05 | GG | 80.95 | |
| | pT1-pT2 | 147 | | | AA + AG | 38.10 | GG | 61.90 | |
| rs7193058 | | | | | | | | | 0.2 |
| | pT3-pT4 | 84 | CC | 3.57 | CT | 34.52 | TT | 61.90 | |
| | pT1-pT2 | 147 | CC | 7.48 | CT | 42.86 | TT | 49.66 | |
| rs76563024 | | | | | | | | | 0.6 |
| | pT3-pT4 | 84 | | | CC + CT | 4.76 | TT | 95.24 | |
| | pT1-pT2 | 147 | | | CC + CT | 3.40 | TT | 96.60 | |
| *rs4293393* | | | | | | | | | **0.003** |
| | pT3-pT4 | 84 | | | CC + CT | 19.05 | TT | 80.95 | |
| | pT1-pT2 | 147 | | | CC + CT | 38.10 | TT | 61.90 | |
| rs28362063 | | | | | | | | | **0.003** |
| | pT3-pT4 | 84 | | | GG + AG | 19.05 | AA | 80.95 | |
| | pT1-pT2 | 147 | | | GG + AG | 38.10 | AA | 61.90 | |
| rs13333226 | | | | | | | | | **0.001** |
| | pT3-pT4 | 84 | | | CC + CT | 19.05 | TT | 80.95 | |
| | pT1-pT2 | 147 | | | CC + CT | 39.46 | TT | 60.54 | |
| rs11862974 | | | | | | | | | 0.8 |
| | pT3-pT4 | 84 | | | TT + GT | 4.76 | GG | 95.24 | |
| | pT1-pT2 | 147 | | | TT + GT | 5.44 | GG | 94.56 | |
| rs13329952 | | | | | | | | | **0.005** |
| | pT3-pT4 | 84 | | | GG + AG | 23.81 | AA | 76.19 | |
| | pT1-pT2 | 147 | | | GG + AG | 42.18 | AA | 57.82 | |
| rs7203642 | | | | | | | | | **0.007** |
| | pT3-pT4 | 84 | | | CC + CT | 23.81 | TT | 76.19 | |
| | pT1-pT2 | 147 | | | CC + CT | 41.50 | TT | 58.50 | |
| rs7204342 | | | | | | | | | 0.8 |
| | pT3-pT4 | 84 | | | AA + AC | 4.76 | CC | 95.24 | |
| | pT1-pT2 | 147 | | | AA + AC | 5.44 | CC | 94.56 | |
| rs28688991 | | | | | | | | | **0.005** |
| | pT3-pT4 | 84 | | | AA + AG | 23.81 | GG | 76.19 | |
| | pT1-pT2 | 147 | | | AA + AG | 42.18 | GG | 57.82 | |
| rs12917707 | | | | | | | | | **0.003** |
| | pT3-pT4 | 84 | | | AA + AC | 19.05 | CC | 80.95 | |
| | pT1-pT2 | 147 | | | AA + AC | 38.10 | CC | 61.90 | |
| rs4297685 | | | | | | | | | 0.1 |
| | pT3-pT4 | 84 | GG | 9.52 | AG | 33.33 | AA | 57.14 | |
| | pT1-pT2 | 147 | GG | 9.52 | AG | 46.26 | AA | 44.22 | |

Similarly, as shown in Table 6, rs4293393 as well as 14 SNPs having strong or perfect LD with rs4293393 (rs9928757, rs13333226, rs12917707, rs13335818, rs28362063, rs28544423, rs34356953, rs60136849, rs12934320, rs71384446,rs13329952,rs28688991,rs7203642, rs12934455) were significantly associated with clinical metastasis, most of them showing a very similar or identical p value, as expected, ranging from 0.005 and 0.03.

Only rs12934455 and rs28640218, even if in LD with rs4293393 with a r²> 0.8, were not significantly associated with clinical metastasis, with a p value of 0.06 and 0.1, respectively. However, rs12934455 only marginally failed to meet statistical significance. On the other hand, none of the SNPs having a weak or no LD with rs4293393 were significantly associated with clinical metastasis.

**Table 6. Association between UMOD SNP variants and presence of clinical metastasis (cM1).**

| **SNP** | **Variable** | **Renal mass patients (n = 276)** | **Genotypic frequencies (%)** | | | | | | **p value** |
|---|---|---|---|---|---|---|---|---|---|
| rs71384446 | | | | | | | | | 0.02 |
| | cM1 | 34 | | | GG + GT | 23.53 | TT | 76.47 | |
| | cM0 | 242 | | | GG + GT | 43.80 | TT | 56.20 | |
| rs9928757 | | | | | | | | | **0.005** |
| | cM1 | 34 | | | GG + CG | 14.71 | CC | 85.29 | |
| | cM0 | 242 | | | GG + CG | 39.67 | CC | 60.33 | |
| rs60136849 | | | | | | | | | **0.02** |
| | cM1 | 34 | | | GG + GA | 14.71 | AA | 85.29 | |
| | cM0 | 242 | | | GG + GA | 34.71 | AA | 65.29 | |
| rs11647727 | | | | | | | | | 0.3 |
| | cM1 | 34 | TT | 2.94 | CT | 35.29 | CC | 61.76 | |
| | cM0 | 242 | TT | 7.44 | CT | 42.98 | CC | 49.59 | |
| rs34356953 | | | | | | | | | **0.02** |
| | cM1 | 34 | | | AA + AC | 14.71 | CC | 85.29 | |
| | cM0 | 242 | | | AA + AC | 35.12 | CC | 64.88 | |
| rs12934320 | | | | | | | | | **0.02** |
| | cM1 | 34 | | | AA + AG | 14.71 | GG | 85.29 | |
| | cM0 | 242 | | | AA + AG | 34.30 | GG | 65.70 | |
| rs12934455 | | | | | | | | | **0.06** |
| | cM1 | 34 | | | AA + AG | 14.71 | GG | 85.29 | |
| | cM0 | 242 | | | AA + AG | 30.17 | GG | 69.83 | |
| rs28640218 | | | | | | | | | 0.1 |
| | cM1 | 34 | | | AA + AC | 23.53 | CC | 76.47 | |
| | cM0 | 242 | | | AA + AC | 37.19 | CC | 62.81 | |
| rs28544423 | | | | | | | | | **0.02** |
| | cM1 | 34 | | | AA + AG | 14.71 | GG | 85.29 | |
| | cM0 | 242 | | | AA + AG | 35.12 | GG | 64.88 | |
| rs13335818 | | | | | | | | | **0.02** |
| | cM1 | 34 | | | AA + AG | 14.71 | GG | 85.29 | |
| | cM0 | 242 | | | AA + AG | 35.12 | GG | 64.88 | |
| rs7193058 | | | | | | | | | 0.3 |
| | cM1 | 34 | CC | 5.88 | CT | 29.41 | TT | 64.71 | |
| | cM0 | 242 | CC | 8.26 | CT | 41.74 | TT | 50.00 | |
| rs76563024 | | | | | | | | | 0.9 |
| | cM1 | 34 | | | CC + CT | 5.88 | TT | 94.12 | |
| | cM0 | 242 | | | CC + CT | 5.37 | TT | 94.63 | |
| rs4293393 | | | | | | | | | **0.02** |
| | cM1 | 34 | | | CC + CT | 14.71 | TT | 85.29 | |
| | cM0 | 242 | | | CC + CT | 35.12 | TT | 64.88 | |
| rs28362063 | | | | | | | | | **0.02** |
| | cM1 | 34 | | | GG + AG | 14.71 | AA | 85.29 | |
| | cM0 | 242 | | | GG + AG | 35.12 | AA | 64.88 | |
| rs13333226 | | | | | | | | | **0.01** |
| | cM1 | 34 | | | CC + CT | 14.71 | TT | 85.29 | |
| | cM0 | 242 | | | CC + CT | 35.95 | TT | 64.05 | |
| rs11862974 | | | | | | | | | 0.9 |
| | cM1 | 34 | | | TT + GT | 5.88 | GG | 94.12 | |
| | cM0 | 242 | | | TT + GT | 6.61 | GG | 93.39 | |
| rs13329952 | | | | | | | | | **0.03** |
| | cM1 | 34 | | | GG + AG | 20.59 | AA | 79.41 | |
| | cM0 | 242 | | | GG + AG | 40.08 | AA | 59.92 | |
| rs7203642 | | | | | | | | | **0.03** |
| | cM1 | 34 | | | CC + CT | 20.59 | TT | 79.41 | |
| | cM0 | 242 | | | CC + CT | 39.67 | TT | 60.33 | |
| rs7204342 | | | | | | | | | 0.9 |
| | cM1 | 34 | | | AA + AC | 5.88 | CC | 94.12 | |
| | cM0 | 242 | | | AA + AC | 6.61 | CC | 93.39 | |
| rs28688991 | | | | | | | | | **0.03** |
| | cM1 | 34 | | | AA + AG | 20.59 | GG | 79.41 | |
| | cM0 | 242 | | | AA + AG | 40.08 | GG | 59.92 | |
| rs12917707 | | | | | | | | | **0.02** |
| | cM1 | 34 | | | AA + AC | 14.71 | CC | 85.29 | |
| | cM0 | 242 | | | AA + AC | 35.12 | CC | 64.88 | |
| rs4297685 | | | | | | | | | 0.7 |
| | cM1 | 34 | GG | 5.88 | AG | 41.18 | AA | 52.94 | |
| | cM0 | 242 | GG | 10.33 | AG | 42.15 | AA | 47.52 | |

No SNP showed a significant association with high grade (Table 7). However, rs13333226 variant showed a p value of 0.096, on the very limits of significance.

**Table 7. Association between UMOD SNP variants and higher pathologic grade (G3-G4).**

| **SNP** | **Variable** | **RCC patients (n = 231)** | **Genotypic frequencies (%)** | | | | | | **p value** |
|---|---|---|---|---|---|---|---|---|---|
| rs71384446 | | | | | | | | | 0.5 |
| | G3-G4 | 93 | | | GG + GT | 36.56 | TT | 63.44 | |
| | G1-G2 | 138 | | | GG + GT | 40.58 | TT | 59.42 | |
| rs9928757 | | | | | | | | | 0.3 |
| | G3-G4 | 93 | | | GG + CG | 29.03 | CC | 70.97 | |
| | G1-G2 | 138 | | | GG + CG | 36.23 | CC | 63.77 | |
| rs60136849 | | | | | | | | | 0.2 |
| | G3-G4 | 93 | | | GG + GA | 25.81 | AA | 74.19 | |
| | G1-G2 | 138 | | | GG + GA | 34.06 | AA | 65.94 | |
| rs11647727 | | | | | | | | | 0.3 |
| | G3-G4 | 93 | TT | 4.30 | CT | 36.56 | CC | 59.14 | |
| | G1-G2 | 138 | TT | 5.80 | CT | 44.93 | CC | 49.28 | |
| rs34356953 | | | | | | | | | 0.1 |
| | G3-G4 | 93 | | | AA + AC | 25.81 | CC | 74.19 | |
| | G1-G2 | 138 | | | AA + AC | 34.78 | CC | 65.22 | |
| rs12934320 | | | | | | | | | 0.2 |
| | G3-G4 | 93 | | | AA + AG | 25.81 | GG | 74.19 | |
| | G1-G2 | 138 | | | AA + AG | 33.33 | GG | 66.67 | |
| rs12934455 | | | | | | | | | 0.6 |
| | G3-G4 | 93 | | | AA + AG | 24.73 | GG | 75.27 | |
| | G1-G2 | 138 | | | AA + AG | 27.54 | GG | 72.46 | |
| rs28640218 | | | | | | | | | 0.6 |
| | G3-G4 | 93 | | | AA + AC | 31.18 | CC | 68.82 | |
| | G1-G2 | 138 | | | AA + AC | 34.06 | CC | 65.94 | |
| rs28544423 | | | | | | | | | 0.1 |
| | G3-G4 | 93 | | | AA + AG | 25.81 | GG | 74.19 | |
| | G1-G2 | 138 | | | AA + AG | 34.78 | GG | 65.22 | |
| rs13335818 | | | | | | | | | 0.1 |
| | G3-G4 | 93 | | | AA + AG | 25.81 | GG | 74.19 | |
| | G1-G2 | 138 | | | AA + AG | 34.78 | GG | 65.22 | |
| rs7193058 | | | | | | | | | 0.2 |
| | G3-G4 | 93 | CC | 5.38 | CT | 33.33 | TT | 61.29 | |
| | G1-G2 | 138 | CC | 6.52 | CT | 44.20 | TT | 49.28 | |
| rs76563024 | | | | | | | | | 0.8 |
| | G3-G4 | 93 | | | CC + CT | 4.30 | TT | 95.70 | |
| | G1-G2 | 138 | | | CC + CT | 3.62 | TT | 96.38 | |
| ***rs4293393*** | | | | | | | | | 0.1 |
| | G3-G4 | 93 | | | CC + CT | 25.81 | TT | 74.19 | |
| | G1-G2 | 138 | | | CC + CT | 34.78 | TT | 65.22 | |
| rs28362063 | | | | | | | | | 0.1 |
| | G3-G4 | 93 | | | GG + AG | 25.81 | AA | 74.19 | |
| | G1-G2 | 138 | | | GG + AG | 34.78 | AA | 65.22 | |
| rs13333226 | | | | | | | | | **0.096** |
| | G3-G4 | 93 | | | CC + CT | 25.81 | TT | 74.19 | |
| | G1-G2 | 138 | | | CC + CT | 36.23 | TT | 63.77 | |
| rs11862974 | | | | | | | | | 0.9 |
| | G3-G4 | 93 | | | TT + GT | 5.38 | GG | 94.62 | |
| | G1-G2 | 138 | | | TT + GT | 5.07 | GG | 94.93 | |
| rs13329952 | | | | | | | | | 0.3 |
| | G3-G4 | 93 | | | GG + AG | 31.18 | AA | 68.82 | |
| | G1-G2 | 138 | | | GG + AG | 38.41 | AA | 61.59 | |
| rs7203642 | | | | | | | | | 0.3 |
| | G3-G4 | 93 | | | CC + CT | 31.18 | TT | 68.82 | |
| | G1-G2 | 138 | | | CC + CT | 37.68 | TT | 62.32 | |
| rs7204342 | | | | | | | | | 0.9 |
| | G3-G4 | 93 | | | AA + AC | 5.38 | CC | 94.62 | |
| | G1-G2 | 138 | | | AA + AC | 5.07 | CC | 94.93 | |
| rs28688991 | | | | | | | | | 0.3 |
| | G3-G4 | 93 | | | AA + AG | 31.18 | GG | 68.82 | |
| | G1-G2 | 138 | | | AA + AG | 38.41 | GG | 61.59 | |
| rs12917707 | | | | | | | | | 0.1 |
| | G3-G4 | 93 | | | AA + AC | 25.81 | CC | 74.19 | |
| | G1-G2 | 138 | | | AA + AC | 34.78 | CC | 65.22 | |
| rs4297685 | | | | | | | | | 0.8 |
| | G3-G4 | 93 | GG | 10.75 | AG | 41.94 | AA | 47.31 | |
| | G1-G2 | 138 | GG | 8.70 | AG | 41.30 | AA | 50.00 | |

As shown in Table 8, there are 7 SNPs, rs76563024, rs9928757, rs7193058, rs11862974, rs7204342, rs28640218, and rs11647727, which showed a statistically significant association with patients with a malignant lesion (kidney cancer), with respect to patients with a benign mass. rs76563024, rs11862974, and rs7204342 were in strong LD among each other, as well as rs7193058 was in strong LD with rs11647727, as shown in Figure 1.

On the other hand, rs71384446, rs7203642, rs13329952, rs28688991, showed a similar association with malignant histotypes with a p value between 0.07 and 0.09 (Table 8). This suggest a trend for association that could become significant by increasing the study cohort.

**Table 8. Association between UMOD SNP variants and risk of RCC.**

| **SNP** | **Variable** | **Renal mass patients (n = 276)** | **Genotypic frequencies (%)** | | | | | | **p value** |
|---|---|---|---|---|---|---|---|---|---|
| rs71384446 | | | | | | | | | **0.07** |
| | Malignant lesion | 231 | | | GT + GG | 38.96 | TT | 61.04 | |
| | Benign mass | 45 | | | GT + GG | 53.33 | TT | 46.67 | |
| rs9928757 | | | | | | | | | **0.01** |
| | Malignant lesion | 231 | | | CG + GG | 33.33 | CC | 66.67 | |
| | Benign mass | 45 | | | CG + GG | 53.33 | CC | 46.67 | |
| rs60136849 | | | | | | | | | 0.2 |
| | Malignant lesion | 231 | | | GA + GG | 30.74 | AA | 69.26 | |
| | Benign mass | 45 | | | GA + GG | 40.00 | AA | 60.00 | |
| rs11647727 | | | | | | | | | **0.03** |
| | Malignant lesion | 231 | TT | 5.19 | CT | 41.56 | CC | 53.25 | |
| | Benign mass | 45 | TT | 15.56 | CT | 44.44 | CC | 40.00 | |
| rs34356953 | | | | | | | | | 0.2 |
| | Malignant lesion | 231 | | | AC + AA | 31.17 | CC | 68.83 | |
| | Benign mass | 45 | | | AC + AA | 40.00 | CC | 60.00 | |
| rs12934320 | | | | | | | | | 0.2 |
| | Malignant lesion | 231 | | | AG + AA | 30.30 | GG | 69.70 | |
| | Benign mass | 45 | | | AG + AA | 40.00 | GG | 60.00 | |
| rs12934455 | | | | | | | | | 0.1 |
| | Malignant lesion | 231 | | | AG + AA | 26.41 | GG | 73.59 | |
| | Benign mass | 45 | | | AG + AA | 37.78 | GG | 62.22 | |
| rs28640218 | | | | | | | | | **0.04** |
| | Malignant lesion | 231 | | | AC + AA | 32.90 | CC | 67.10 | |
| | Benign mass | 45 | | | AC + AA | 48.89 | CC | 51.11 | |
| rs28544423 | | | | | | | | | 0.2 |
| | Malignant lesion | 231 | | | AG + AA | 31.17 | GG | 68.83 | |
| | Benign mass | 45 | | | AG + AA | 40.00 | GG | 60.00 | |
| rs13335818 | | | | | | | | | 0.2 |
| | Malignant lesion | 231 | | | AG + AA | 31.17 | GG | 68.83 | |
| | Benign mass | 45 | | | AG + AA | 40.00 | GG | 60.00 | |
| rs7193058 | | | | | | | | | **0.02** |
| | Malignant lesion | 231 | CC | 6.06 | CT | 39.83 | TT | 54.11 | |
| | Benign mass | 45 | CC | 17.78 | CT | 42.22 | TT | 40.00 | |
| rs76563024 | | | | | | | | | **0.01** |
| | Malignant lesion | 231 | | | CT + CC | 3.90 | TT | 96.10 | |
| | Benign mass | 45 | | | CT + CC | 13.33 | TT | 86.67 | |
| ***rs4293393*** | | | | | | | | | 0.2 |
| | Malignant lesion | 231 | | | CT + CC | 31.17 | TT | 68.83 | |
| | Benign mass | 45 | | | CT + CC | 40.00 | TT | 60.00 | |
| rs28362063 | | | | | | | | | 0.2 |
| | Malignant lesion | 231 | | | AG + GG | 31.17 | AA | 68.83 | |
| | Benign mass | 45 | | | AG + GG | 40.00 | AA | 60.00 | |
| rs13333226 | | | | | | | | | 0.3 |
| | Malignant lesion | 231 | | | CT + CC | 32.03 | TT | 67.97 | |
| | Benign mass | 45 | | | CT + CC | 40.00 | TT | 60.00 | |
| rs11862974 | | | | | | | | | **0.04** |
| | Malignant lesion | 231 | | | GT + TT | 5.19 | GG | 94.81 | |
| | Benign mass | 45 | | | GT + TT | 13.33 | GG | 86.67 | |
| rs13329952 | | | | | | | | | **0.09** |
| | Malignant lesion | 231 | | | AG + GG | 35.50 | AA | 64.50 | |
| | Benign mass | 45 | | | AG + GG | 48.89 | AA | 51.11 | |
| rs7203642 | | | | | | | | | **0.08** |
| | Malignant lesion | 231 | | | CT + CC | 35.06 | TT | 64.94 | |
| | Benign mass | 45 | | | CT + CC | 48.89 | TT | 51.11 | |
| rs7204342 | | | | | | | | | **0.04** |
| | Malignant lesion | 231 | | | AC + AA | 5.19 | CC | 94.81 | |
| | Benign mass | 45 | | | AC + AA | 13.33 | CC | 86.67 | |
| rs28688991 | | | | | | | | | **0.09** |
| | Malignant lesion | 231 | | | AG + AA | 35.50 | GG | 64.50 | |
| | Benign mass | 45 | | | AG + AA | 48.89 | GG | 51.11 | |
| rs12917707 | | | | | | | | | 0.2 |
| | Malignant lesion | 231 | | | AC + AA | 31.17 | CC | 68.83 | |
| | Benign mass | 45 | | | AC + AA | 40.00 | CC | 60.00 | |
| rs4297685 | | | | | | | | | 0.8 |
| | Malignant lesion | 231 | GG | 9.52 | AG | 41.56 | AA | 48.92 | |
| | Benign mass | 45 | GG | 11.11 | AG | 44.44 | AA | 44.44 | |

As showed in Table 9, rs7193058 and rs11647727 variants showed a statistically significant association with renal mass patients with respect to controls, with a p value of 0.03 and 0.04, respectively. Of note, most of the controls are patients with kidney stones and it is known that some UMOD variants, especially those located in the UMOD gene promoter, are associated with reduced risk of kidney stones [Gudbjartsson et al., 2010]. Since the UMOD promoter top variant rs4293393 shows a higher p value of 0.2, the inventors believe that the association of rs11647727 and rs7193058 variants is not driven by an enrichment of renal lithiasis-related variants in the control population.

**Table 9. Association between UMOD SNP variants and risk of renal mass.**

| **SNP** | **Variable** | **Overall population (n = 359)** | **Genotypic frequencies (%)** | | | | | | **p value** |
|---|---|---|---|---|---|---|---|---|---|
| rs71384446 | | | | | | | | | 0.4 |
| | Renal mass patients | 276 | | | GT + GG | 41.30 | TT | 58.70 | |
| | Controls | 83 | | | GT + GG | 46.99 | TT | 53.01 | |
| rs9928757 | | | | | | | | | 0.5 |
| | Renal mass patients | 276 | | | CG + GG | 36.59 | CC | 63.41 | |
| | Controls | 83 | | | CG + GG | 40.96 | CC | 59.04 | |
| rs60136849 | | | | | | | | | 0.1 |
| | Renal mass patients | 276 | | | GA + GG | 32.25 | AA | 67.75 | |
| | Controls | 83 | | | GA + GG | 40.96 | AA | 59.04 | |
| rs11647727 | | | | | | | | | **0.04** |
| | Renal mass patients | 276 | TT | 6.88 | CT | 42.03 | CC | 51.09 | |
| | Controls | 83 | TT | 6.02 | CT | 57.83 | CC | 36.14 | |
| rs34356953 | | | | | | | | | 0.2 |
| | Renal mass patients | 276 | | | AC + AA | 32.61 | CC | 67.39 | |
| | Controls | 83 | | | AC + AA | 40.96 | CC | 59.04 | |
| rs12934320 | | | | | | | | | 0.1 |
| | Renal mass patients | 276 | | | AG + AA | 31.88 | GG | 68.12 | |
| | Controls | 83 | | | AG + AA | 40.96 | GG | 59.04 | |
| rs12934455 | | | | | | | | | 0.5 |
| | Renal mass patients | 276 | | | AG + AA | 28.26 | GG | 71.74 | |
| | Controls | 83 | | | AG + AA | 32.53 | GG | 67.47 | |
| rs28640218 | | | | | | | | | 0.2 |
| | Renal mass patients | 276 | | | AC + AA | 35.51 | CC | 64.49 | |
| | Controls | 83 | | | AC + AA | 43.37 | CC | 56.63 | |
| rs28544423 | | | | | | | | | 0.2 |
| | Renal mass patients | 276 | | | AG + AA | 32.61 | GG | 67.39 | |
| | Controls | 83 | | | AG + AA | 40.96 | GG | 59.04 | |
| rs13335818 | | | | | | | | | 0.2 |
| | Renal mass patients | 276 | | | AG + AA | 32.61 | GG | 67.39 | |
| | Controls | 83 | | | AG + AA | 39.76 | GG | 60.24 | |
| rs7193058 | | | | | | | | | **0.03** |
| | Renal mass patients | 276 | CC | 7.97 | CT | 40.22 | TT | 51.81 | |
| | Controls | 83 | CC | 6.02 | CT | 56.63 | TT | 37.35 | |
| rs76563024 | | | | | | | | | 0.5 |
| | Renal mass patients | 276 | | | CT + CC | 5.43 | TT | 94.57 | |
| | Controls | 83 | | | CT + CC | 7.23 | TT | 92.77 | |
| ***rs4293393*** | | | | | | | | | 0.2 |
| | Renal mass patients | 276 | | | CT + CC | 32.61 | TT | 67.39 | |
| | Controls | 83 | | | CT + CC | 40.96 | TT | 59.04 | |
| rs28362063 | | | | | | | | | 0.2 |
| | Renal mass patients | 276 | | | AG + GG | 32.61 | AA | 67.39 | |
| | Controls | 83 | | | AG + GG | 39.76 | AA | 60.24 | |
| rs13333226 | | | | | | | | | 0.1 |
| | Renal mass patients | 276 | | | CT + CC | 33.33 | TT | 66.67 | |
| | Controls | 83 | | | CT + CC | 42.17 | TT | 57.83 | |
| rs11862974 | | | | | | | | | 0.5 |
| | Renal mass patients | 276 | | | GT + TT | 6.52 | GG | 93.48 | |
| | Controls | 83 | | | GT + TT | 8.43 | GG | 91.57 | |
| rs13329952 | | | | | | | | | 0.3 |
| | Renal mass patients | 276 | | | AG + GG | 37.68 | AA | 62.32 | |
| | Controls | 83 | | | AG + GG | 44.58 | AA | 55.42 | |
| rs7203642 | | | | | | | | | 0.2 |
| | Renal mass patients | 276 | | | CT + CC | 37.32 | TT | 62.68 | |
| | Controls | 83 | | | CT + CC | 45.78 | TT | 54.22 | |
| rs7204342 | | | | | | | | | 0.3 |
| | Renal mass patients | 276 | | | AC + AA | 6.52 | CC | 93.48 | |
| | Controls | 83 | | | AC + AA | 9.64 | CC | 90.36 | |
| rs28688991 | | | | | | | | | 0.2 |
| | Renal mass patients | 276 | | | AG + AA | 37.68 | GG | 62.32 | |
| | Controls | 83 | | | AG + AA | 45.78 | GG | 54.22 | |
| rs12917707 | | | | | | | | | 0.2 |
| | Renal mass patients | 276 | | | AC + AA | 32.61 | CC | 67.39 | |
| | Controls | 83 | | | AC + AA | 39.76 | CC | 60.24 | |
| rs4297685 | | | | | | | | | 0.4 |
| | Renal mass patients | 276 | GG | 9.78 | AG | 42.03 | AA | 48.19 | |
| | Controls | 83 | GG | 4.82 | AG | 43.37 | AA | 51.81 | |

### Example 2: Circulating plasma miRNAs profiling in the total study population

To develop specific miRNA signatures for RCC screening, diagnosis, and prognosis, a TaqMan-based high-throughput screen of 668 miRNAs was performed in plasma samples of 120 patients with a renal mass (either benign or malignant) and 78 controls.

MiRNAs previously described as readily detectable in human plasma samples - including hsa-miR-16-5p, and hsa-miR-21-3p - were detected in all samples. Following the inventors' filtering criteria, a total of 273 miRNAs were subjected to differential expression analyses.

The inventors found a set of 54 miRNAs that was significantly differentially expressed (p value < 0.05) in plasma of patients with a renal mass (n = 115) compared with controls (n = 78). In addition, 18 miRNAs, even if did not reach statistical significance, showed a trend that could become significant by increasing the study cohort (0.05 < p value < 0.1) (Table 10).

**Table 10. Differentially expressed miRNAs in patients with a renal mass (n = 115) compared with controls (n = 78).**

| miRNA | FC* | p value |
|---|---|---|
| hsa-miR-423-5p | 1.28 | 0.0001 |
| hsa-miR-186-5p | 1.18 | 0.0002 |
| hsa-miR-877-5p | 1.46 | 0.0004 |
| hsa-miR-378a-3p | 1.28 | 0.0004 |
| hsa-miR-320e | 1.27 | 0.001 |
| hsa-miR-652-3p | 1.26 | 0.001 |
| hsa-miR-598-3p | 1.19 | 0.002 |
| hsa-miR-197-3p | 0.65 | 0.002 |
| hsa-miR-500a-5p | 1.39 | 0.003 |
| hsa-miR-205-5p | 0.71 | 0.003 |
| hsa-miR-6803-3p | 0.73 | 0.003 |
| hsa-m i R-125a-5 p | 0.86 | 0.003 |
| hsa-miR-320a-3p | 1.18 | 0.006 |
| hsa-let-7e-5p | 0.87 | 0.007 |
| hsa-miR-362-5p | 1.28 | 0.007 |
| hsa-miR-21-5p | 1.14 | 0.008 |
| hsa-miR-132-3p | 0.79 | 0.008 |
| hsa-miR-375-3p | 0.71 | 0.009 |
| hsa-miR-1260a | 0.73 | 0.01 |
| hsa-miR-331-3p | 0.85 | 0.01 |
| hsa-miR-128-3p | 1.12 | 0.01 |
| hsa-miR-532-5p | 1.23 | 0.01 |
| hsa-miR-29c-5p | 1.16 | 0.01 |
| hsa-miR-378a-5p | 0.82 | 0.01 |
| hsa-miR-28-3p | 1.24 | 0.01 |
| hsa-miR-145-5p | 0.85 | 0.01 |
| hsa-miR-151b | 0.91 | 0.01 |
| hsa-miR-361-5p | 1.22 | 0.02 |
| hsa-miR-501-5p | 1.26 | 0.02 |
| hsa-miR-369-3p | 0.84 | 0.02 |
| hsa-miR-4286 | 0.89 | 0.02 |
| hsa-miR-505-3p | 1.15 | 0.02 |
| hsa-miR-10a-5p | 0.86 | 0.02 |
| hsa-miR-2110 | 1.24 | 0.02 |
| hsa-miR-193b-3p | 0.83 | 0.02 |
| hsa-miR-224-5p | 0.80 | 0.02 |
| hsa-miR-214-3p | 0.77 | 0.02 |
| hsa-miR-181a-5p | 1.28 | 0.02 |
| hsa-miR-628-3p | 0.76 | 0.02 |
| hsa-miR-550a-3-5p | 1.57 | 0.03 |
| hsa-miR-339-3p | 1.18 | 0.03 |
| hsa-miR-323a-3p | 1.41 | 0.03 |
| hsa-miR-500a-3p | 1.15 | 0.03 |
| hsa-miR-125b-5p | 0.84 | 0.03 |
| hsa-miR-483-3p | 0.81 | 0.03 |
| hsa-miR-151a-3p | 1.20 | 0.04 |
| hsa-miR-499a-5p | 1.29 | 0.04 |
| hsa-miR-21-3p | 1.15 | 0.04 |
| hsa-miR-3940-3p | 0.89 | 0.04 |
| hsa-miR-10b-5p | 0.75 | 0.04 |
| hsa-miR-769-5p | 1.26 | 0.04 |
| hsa-miR-30d-5p | 1.09 | 0.04 |
| hsa-miR-181b-5p | 1.16 | 0.04 |
| hsa-miR-448 | 0.83 | 0.049 |
| hsa-miR-377-3p | 0.86 | 0.05 |
| hsa-miR-4306 | 1.16 | 0.05 |
| hsa-miR-22-5p | 1.08 | 0.05 |
| hsa-miR-380-3p | 0.84 | 0.05 |
| hsa-miR-103a-2-5p | 0.91 | 0.06 |
| hsa-miR-130b-5p | 0.81 | 0.06 |
| hsa-miR-146a-5p | 1.19 | 0.06 |
| hsa-miR-188-5p | 1.18 | 0.06 |
| hsa-miR-210-3p | 1.13 | 0.07 |
| hsa-miR-885-5p | 0.68 | 0.07 |
| hsa-miR-320b | 1.12 | 0.07 |
| hsa-miR-26b-3p | 0.88 | 0.07 |
| hsa-miR-421 | 0.89 | 0.08 |
| hsa-miR-7-5p | 0.85 | 0.08 |
| hsa-miR-579-3p | 0.78 | 0.08 |
| hsa-miR-17-3p | 1.08 | 0.08 |
| hsa-miR-24-3p | 1.16 | 0.09 |
| hsa-miR-660-5p | 1.18 | 0.09 |

| | | |
|---|---|---|
| *FC: fold changes (>1 increased; <1 decreased expression in in patients with a renal mass) | | |

Then, the inventors investigated whether patients with a benign mass were associated with a specific miRNA signature that significantly differs (p value < 0.05) from patients with a malignant lesion (RCC). The inventors observed 6 miRNAs significantly differentially expressed in patients with a benign mass (n = 24) compared with RCC patients (n = 91). In addition, 12 miRNAs showed a trend towards statistical significance (0.05 < p value < 0.1) (Table 11).

**Table 11. Differentially expressed miRNAs in patients with a benign mass (n = 24) compared with RCC patients (n = 91).**

| miRNA | FC* | p value* |
|---|---|---|
| hsa-miR-337-5p | 1.23 | 0.01 |
| hsa-miR-99a-3p | 1.68 | 0.02 |
| hsa-miR-331-5p | 1.52 | 0.02 |
| hsa-miR-221-5p | 1.26 | 0.03 |
| hsa-miR-1290 | 1.35 | 0.04 |
| hsa-miR-196b-5p | 1.36 | 0.048 |
| hsa-miR-550a-3-5p | 1.49 | 0.06 |
| hsa-miR-548l | 1.35 | 0.06 |
| hsa-miR-152-3p | 1.09 | 0.06 |
| hsa-miR-501-5p | 1.23 | 0.06 |
| hsa-miR-125b-5p | 0.79 | 0.08 |
| hsa-miR-34a-3p | 0.60 | 0.08 |
| hsa-miR-410-3p | 1.28 | 0.08 |
| hsa-miR-485-3p | 0.59 | 0.08 |
| hsa-miR-192-5p | 0.80 | 0.08 |
| hsa-miR-361-3p | 0.85 | 0.098 |
| hsa-miR-877-5p | 1.16 | 0.098 |
| hsa-miR-215-5p | 0.79 | 0.099 |

| | | |
|---|---|---|
| *fold changes (>1 increased; <1 decreased expression in RCC patients) | | |

The inventors also analyzed whether specific miRNA expression patterns were correlated to established predictors of RCC prognosis that are direct markers of clinical aggressiveness, namely tumour stage, tumour grade, and presence of clinical metastasis (cM1).

A panel of 43miRNAs was found to be significantly differentially expressed between low grade RCC patients (n = 64) and high grade cases (27). 36 miRNAs, even if did not reach statistical significance, showed a trend that could become significant by increasing the study cohort (Table 12).

**Table 12. Differentially expressed miRNAs (p value ≤ 0.05) in low grade RCC patients (n = 64) compared with high grade RCC patients (n = 27).**

| miRNA | FC* | p value |
|---|---|---|
| hsa-miR-374a-5p | 0.72 | 0.04 |
| hsa-miR-382-5p | 2.50 | 0.0004 |
| hsa-miR-18a-5p | 0.73 | 0.0006 |
| hsa-miR-125b-5p | 0.57 | 0.0009 |
| hsa-miR-20a-5p | 0.71 | 0.002 |
| hsa-miR-429 | 0.55 | 0.002 |
| hsa-miR-17-5p | 0.70 | 0.002 |
| hsa-miR-369-3p | 1.41 | 0.002 |
| hsa-miR-21-3p | 1.44 | 0.002 |
| hsa-miR-382-3p | 1.80 | 0.003 |
| hsa-miR-134-5p | 1.81 | 0.004 |
| hsa-miR-433-3p | 1.81 | 0.004 |
| hsa-miR-100-5p | 0.50 | 0.005 |
| hsa-miR-125a-5p | 0.79 | 0.006 |
| hsa-miR-584-5p | 1.24 | 0.006 |
| hsa-miR-93-5p | 0.79 | 0.006 |
| hsa-miR-20b-5p | 0.77 | 0.007 |
| hsa-miR-18a-3p | 0.80 | 0.008 |
| hsa-miR-374a-3p | 0.64 | 0.009 |
| hsa-miR-410-3p | 1.42 | 0.01 |
| hsa-let-7b-5p | 0.71 | 0.01 |
| hsa-miR-424-3p | 1.43 | 0.01 |
| hsa-miR-101-3p | 0.69 | 0.01 |
| hsa-miR-32-3p | 1.19 | 0.02 |
| hsa-miR-154-5p | 1.87 | 0.02 |
| hsa-miR-409-3p | 2.18 | 0.02 |
| hsa-miR-130b-3p | 1.10 | 0.02 |
| hsa-miR-193b-3p | 0.66 | 0.02 |
| hsa-miR-484 | 0.80 | 0.02 |
| hsa-miR-3613-3p | 0.81 | 0.02 |
| hsa-miR-629-5p | 1.35 | 0.02 |
| hsa-miR-151a-3p | 1.25 | 0.02 |
| hsa-miR-493-5p | 1.67 | 0.02 |
| hsa-let-7d-3p | 1.15 | 0.03 |
| hsa-miR-144-3p | 0.59 | 0.03 |
| hsa-miR-885-5p | 0.54 | 0.03 |
| hsa-miR-655-3p | 1.32 | 0.03 |
| hsa-miR-106b-3p | 0.85 | 0.03 |
| hsa-miR-942-5p | 1.23 | 0.04 |
| hsa-miR-148a-3p | 0.83 | 0.04 |
| hsa-let-7e-3p | 0.74 | 0.04 |
| hsa-miR-127-3p | 1.79 | 0.04 |
| hsa-miR-103a-3p | 0.77 | 0.046 |
| hsa-miR-323b-3p | 1.79 | 0.05 |
| hsa-miR-337-5p | 1.18 | 0.05 |
| hsa-miR-376c-3p | 1.54 | 0.05 |
| hsa-miR-195-5p | 0.83 | 0.06 |
| hsa-miR-181a-3p | 1.17 | 0.06 |
| hsa-miR-483-5p | 1.54 | 0.06 |
| hsa-miR-590-3p | 0.85 | 0.06 |
| hsa-let-7d-5p | 0.68 | 0.06 |
| hsa-miR-151a-5p | 1.22 | 0.06 |
| hsa-miR-324-5p | 0.83 | 0.06 |
| hsa-miR-500a-5p | 0.70 | 0.06 |
| hsa-miR-331-3p | 0.84 | 0.06 |
| hsa-miR-7-1-3p | 1.18 | 0.06 |
| hsa-miR-154-3p | 1.21 | 0.06 |
| hsa-miR-10a-5p | 0.78 | 0.07 |
| hsa-miR-194-5p | 0.71 | 0.07 |
| hsa-miR-22-5p | 1.11 | 0.07 |
| hsa-miR-423-5p | 1.25 | 0.07 |
| hsa-miR-142-3p | 0.79 | 0.07 |
| hsa-miR-30b-5p | 0.85 | 0.07 |
| hsa-miR-548a-3p | 1.32 | 0.07 |
| hsa-miR-1537-3p | 1.17 | 0.07 |
| hsa-miR-221-3p | 1.19 | 0.07 |
| hsa-miR-495-3p | 1.66 | 0.07 |
| hsa-miR-1290 | 1.44 | 0.08 |
| hsa-miR-455-3p | 0.64 | 0.08 |
| hsa-miR-377-3p | 1.30 | 0.08 |
| hsa-miR-451a | 0.70 | 0.08 |
| hsa-miR-16-5p | 0.78 | 0.08 |
| hsa-miR-301a-3p | 0.81 | 0.08 |
| hsa-miR-376a-3p | 1.43 | 0.09 |
| hsa-miR-122-5p | 0.57 | 0.09 |
| hsa-miR-1301-3p | 1.12 | 0.09 |
| hsa-miR-374b-5p | 0.81 | 0.09 |
| hsa-let-7g-5p | 0.83 | 0.098 |
| hsa-miR-144-5p | 0.74 | 0.098 |

| | | |
|---|---|---|
| *fold changes (>1 increased; <1 decreased expression in high grade RCC patients) | | |

Similarly, the inventors found that 38 miRNAs can significantly distinguish (p value < 0.05) between low stage RCC patients (n = 65) and high stage RCC patients (n = 26), while 20 miRNAs showed a trend towards statistical significance (0.05 < p value < 0.1)(Table 13).

**Table 13. Differentially expressed miRNAs in low stage RCC patients (n = 65) compared with high stage RCC patients (n = 26).**

| miRNA | FC* | p value |
|---|---|---|
| hsa-miR-374a-5p | 0.67 | 0.004 |
| hsa-miR-629-5p | 1.55 | 0.0004 |
| hsa-miR-320a-3p | 1.38 | 0.001 |
| hsa-miR-423-5p | 1.43 | 0.002 |
| hsa-miR-30b-5p | 0.78 | 0.002 |
| hsa-miR-320e | 1.44 | 0.003 |
| hsa-miR-100-5p | 0.47 | 0.005 |
| hsa-miR-374a-3p | 0.60 | 0.006 |
| hsa-miR-30c-5p | 0.81 | 0.007 |
| hsa-miR-2110 | 1.37 | 0.007 |
| hsa-miR-27b-3p | 0.74 | 0.008 |
| hsa-miR-664a-3p | 0.69 | 0.008 |
| hsa-let-7d-3p | 1.17 | 0.01 |
| hsa-miR-548k | 1.29 | 0.01 |
| hsa-miR-26b-5p | 0.75 | 0.01 |
| hsa-miR-374b-5p | 0.76 | 0.01 |
| hsa-miR-483-5p | 1.71 | 0.01 |
| hsa-miR-378a-3p | 1.31 | 0.01 |
| hsa-miR-769-5p | 1.54 | 0.02 |
| hsa-miR-34a-3p | 1.72 | 0.02 |
| hsa-miR-125a-5p | 0.83 | 0.02 |
| hsa-miR-224-5p | 0.70 | 0.02 |
| hsa-miR-320b | 1.29 | 0.02 |
| hsa-miR-27a-3p | 0.77 | 0.02 |
| hsa-miR-545-3p | 0.79 | 0.02 |
| hsa-miR-335-5p | 0.84 | 0.03 |
| hsa-miR-140-3p | 1.21 | 0.03 |
| hsa-miR-126-3p | 0.82 | 0.03 |
| hsa-miR-382-5p | 1.77 | 0.03 |
| hsa-miR-484 | 0.81 | 0.03 |
| hsa-miR-18a-5p | 0.85 | 0.03 |
| hsa-miR-29c-3p | 1.09 | 0.04 |
| hsa-miR-26a-5p | 0.74 | 0.04 |
| hsa-miR-339-3p | 1.33 | 0.04 |
| hsa-miR-202-5p | 1.38 | 0.04 |
| hsa-miR-133b | 0.55 | 0.045 |
| hsa-miR-590-3p | 0.83 | 0.048 |
| hsa-miR-15a-3p | 0.83 | 0.049 |
| hsa-miR-324-3p | 0.87 | 0.05 |
| hsa-miR-628-3p | 1.28 | 0.06 |
| hsa-miR-17-5p | 0.80 | 0.06 |
| hsa-miR-1290 | 1.33 | 0.06 |
| hsa-miR-502-3p | 1.26 | 0.07 |
| hsa-miR-424-3p | 1.28 | 0.07 |
| hsa-miR-301a-3p | 0.83 | 0.07 |
| hsa-miR-139-5p | 0.80 | 0.08 |
| hsa-miR-148b-3p | 0.88 | 0.08 |
| hsa-miR-142-3p | 0.78 | 0.08 |
| hsa-miR-133a-3p | 0.73 | 0.08 |
| hsa-miR-20a-5p | 0.83 | 0.08 |
| hsa-miR-942-5p | 1.19 | 0.08 |
| hsa-miR-190a-5p | 0.85 | 0.09 |
| hsa-miR-1307-5p | 0.73 | 0.09 |
| hsa-miR-885-5p | 0.64 | 0.09 |
| hsa-miR-1180-3p | 1.59 | 0.09 |
| hsa-miR-29c-5p | 1.10 | 0.09 |
| hsa-miR-4306 | 1.30 | 0.09 |
| hsa-miR-1249-3p | 0.77 | 0.098 |

| | | |
|---|---|---|
| *fold changes (>1 increased; <1 decreased expression in high stage RCC patients) | | |

Finally, 15 miRNAs were found to be significantly differentially expressed between cM0 RCC patients (n = 84) and cM1 RCC patients (n = 7), while 21 miRNAs show a p value between 0.05 and 0.098, indicating a trend towards statistical significance (Table 14).

**Table 14. Differentially expressed miRNAs in cM0 RCC patients (n = 84) compared with cM1 RCC patients (n = 7).**

| miRNA | FC* | p value |
|---|---|---|
| hsa-miR-374a-5p | 0.57 | 0.05 |
| hsa-miR-337-5p | 1.44 | 0.006 |
| hsa-miR-410-3p | 1.72 | 0.008 |
| hsa-miR-374a-3p | 0.52 | 0.01 |
| hsa-miR-590-3p | 0.71 | 0.01 |
| hsa-miR-377-3p | 1.92 | 0.02 |
| hsa-miR-548k | 1.49 | 0.02 |
| hsa-miR-382-5p | 2.68 | 0.03 |
| hsa-miR-655-3p | 1.49 | 0.03 |
| hsa-miR-378a-5p | 1.50 | 0.03 |
| hsa-let-7d-3p | 1.19 | 0.04 |
| hsa-miR-1290 | 2.05 | 0.04 |
| hsa-miR-483-5p | 1.75 | 0.04 |
| hsa-miR-323b-3p | 2.76 | 0.047 |
| hsa-miR-376c-3p | 2.00 | 0.048 |
| hsa-miR-590-5p | 0.75 | 0.048 |
| hsa-let-7g-3p | 0.67 | 0.05 |
| hsa-miR-18a-5p | 0.81 | 0.06 |
| hsa-miR-142-3p | 0.66 | 0.06 |
| hsa-miR-125a-5p | 0.79 | 0.07 |
| hsa-miR-130b-3p | 1.22 | 0.07 |
| hsa-miR-429 | 0.53 | 0.07 |
| hsa-miR-15a-3p | 0.73 | 0.08 |
| hsa-miR-20a-5p | 0.72 | 0.08 |
| hsa-miR-134-5p | 1.67 | 0.08 |
| hsa-miR-369-3p | 1.31 | 0.08 |
| hsa-miR-548l | 1.61 | 0.08 |
| hsa-miR-127-3p | 2.18 | 0.08 |
| hsa-miR-196b-5p | 1.41 | 0.08 |
| hsa-miR-877-5p | 1.60 | 0.08 |
| hsa-miR-671-5p | 1.47 | 0.09 |
| hsa-miR-17-5p | 0.75 | 0.09 |
| hsa-miR-532-3p | 0.76 | 0.09 |
| hsa-miR-409-3p | 2.40 | 0.09 |
| hsa-miR-484 | 0.78 | 0.09 |
| hsa-miR-376a-3p | 1.81 | 0.098 |

| | | |
|---|---|---|
| *fold changes (>1 increased; <1 decreased expression in cM1 RCC patients) | | |

Since in most cases it is very difficult to reach high accuracy, specificity and sensitivity for discrimination between two clinical conditions (i.e. renal mass patients vs controls, patients with a benign mass vs patients with a malignant lesion, patients with high grade RCC vs patients with low grade RCC, patients with high stage RCC vs patients with low stage RCC, patients with clinical metastasis (cM1) vs patients without clinical metastasis (cM0)) using a single miRNA biomarker, no simple threshold method can be used. Therefore, in addition to single biomarker analysis, the inventors identified unique sets of miRNAs (miRNA signatures), leading to a significant increase in sensitivity, specificity, accuracy and predictive power for screening, diagnosing and prognosing of RCC patients by using machine learning algorithms.

The inventors investigated also clinical parameters in association with miRNAs biomarkers, using the feature selection algorithm, and the variable age resulted predictive in association with some miRNAs in screening, and clinical metastasis.

It is important to underline that the single variable age, using the same evaluation metric, performed significantly less than the presented combinations with an accuracy of 52.10%, recall of 48.69%, and ROC AUC of 54.13% for screening and an accuracy of 74.44%, recall of 20%, and ROC AUC of 22.78% for clinical metastasis.

In addition, the inventors combined some of the identified miRNAs signatures (together or not with age) with SNPs, creating unique combinations leading to a further significant increase in sensitivity, specificity, accuracy and predictive power for screening, diagnosing and prognosing of RCC patients by using machine learning algorithms (Table 15, 16, 17, 18, 19).

This is the list of the best 29 mRNAs selected by the algorithm sorted by importance for screening outcome: hsa-miR-374a-5p, hsa-miR-197-3p, hsa-miR-378a-3p, hsa-miR-12136, hsa-miR-28-3p, hsa-miR-15b, 3p, hsa-miR-328-3p, hsa-miR-323a-3p, hsa-miR-550a-3-5p, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-423-5p, hsa-miR-29a-3p, and hsa-miR-7-5p. The inventors combined these miRNAs, together or not with age and UMOD SNP variant, creating unique combinations leading to a further significant increase in sensitivity, specificity, accuracy and predictive power for screening (Table 15).

**Table 15. Combinations that allow higher sensitivity, specificity, accuracy and predictive power for screening.**

| N | Combinations | Accuracy | ROC AUC | Recall |
|---|---|---|---|---|
| 1 | hsa-miR-197-3p | 63.42 | 63.58 | 86.89 |
| 2 | hsa-miR-197-3p, hsa-miR-378a-3p | 67.53 | 71.47 | 84.24 |
| 3 | hsa-miR-197-3p, hsa-miR-378a-3p, hsa-miR-12136 | 68.58 | 72.54 | 81.74 |
| 4 | hsa-miR-197-3p, hsa-miR-378a-3p, hsa-miR-12136, hsa-miR-28-3p | 72.29 | 78.01 | 82.5 |
| 5 | hsa-miR-197-3p, hsa-miR-378a-3p, hsa-miR-12136, hsa-miR-28-3p, hsa-miR-15b-3p | 75.42 | 78.52 | 81.67 |
| 6 | hsa-miR-197-3p, hsa-miR-378a-3p, hsa-miR-12136, hsa-miR-28-3p, hsa-miR-15b-3p, hsa-miR-328-3p | 74.37 | 78.5 | 80.0 |
| 7 | hsa-miR-197-3p, hsa-miR-378a-3p, hsa-miR-12136, hsa-miR-28-3p, hsa-miR-15b-3p, hsa-miR-328-3p, hsa-miR-323a-3p | 75.95 | 80.29 | 83.41 |
| 8 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p | 73.87 | 79.82 | 81.67 |
| 9 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age | 75.97 | 80.63 | 84.32 |
| 10 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p | 74.89 | 81.16 | 84.32 |
| 11 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p | 76.5 | 80.78 | 85.15 |
| 12 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326 | 74.95 | 81.94 | 83.33 |
| 13 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p | 74.39 | 80.91 | 83.41 |
| 14 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p | 75.95 | 82.08 | 81.67 |
| 15 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p | 75.95 | 81.96 | 80.83 |
| 16 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p | 77.00 | 84.11 | 84.24 |
| 17 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b | 76.47 | 83.69 | 83.33 |
| 18 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306 | 79.61 | 84.79 | 83.33 |
| 19 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p | 82.24 | 85.88 | 83.41 |
| 20 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p | 81.16 | 85.09 | 81.67 |
| 21 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p | 81.16 | 85.21 | 85.15 |
| 22 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p, hsa-miR-377-3p | 81.18 | 85.55 | 85.15 |
| 23 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p, hsa-miR-377-3p, hsa-miR-483-5p | 81.18 | 85.70 | 82.65 |
| 24 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p | 82.21 | 86.38 | 82.65 |
| 25 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p | 81.71 | 88.13 | 84.32 |
| 26 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p | 81.71 | 88.26 | 84.32 |
| 27 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p | 81.71 | 87.57 | 85.15 |
| 28 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-423-5p | 80.11 | 87.19 | 83.41 |
| 29 | hsa-miR-197-3p, hsa-miR-378a-3p, hsa-miR-12136, hsa-miR-28-3p, hsa-miR-15b-3p, hsa-miR-328-3p, hsa-miR-323a-3p, hsa-miR-550a-3-5p, age, hsa-miR-323b-3p, hsa-miR-30b-5p, hsa-miR-326, hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-423-5p, hsa-miR-29a-3p | 80.16 | 87.40 | 86.97 |
| 30 | hsa-miR-326, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, hsa-miR-550a-3-5p, age, | 81.76 | 85.90 | 86.06 |
| 31 | hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 81.74 | 85.94 | 86.21 |
| 32 | hsa-miR-142-5p, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 81.71 | 85.52 | 84.39 |
| 33 | hsa-miR-326, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 81.18 | 83.44 | 83.64 |
| 34 | hsa-miR-326, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 80.68 | 83.34 | 81.89 |
| 35 | hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 80.68 | 84.57 | 83.56 |
| 36 | hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, | 80.68 | 85.08 | 85.3 |
| 37 | hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, | 80.68 | 84.63 | 85.3 |
| 38 | hsa-miR-326, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 80.68 | 85.56 | 82.8 |
| 39 | hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, hsa-miR-323b-3p, | 80.68 | 84.00 | 85.38 |
| 40 | hsa-miR-326, hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 80.68 | 85.17 | 84.47 |
| 41 | hsa-miR-326, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 80.66 | 86.09 | 82.73 |
| 42 | hsa-miR-326, hsa-miR-142-5p, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, hsa-miR-550a-3-5p, age, hsa-miR-323b-3p, | 80.66 | 84.60 | 82.58 |
| 43 | hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-28-3p, hsa-miR-328-3p, hsa-miR-323a-3p, hsa-miR-550a-3-5p, age, hsa-miR-323b-3p, | 80.66 | 84.33 | 87.05 |
| 44 | hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, hsa-miR-323b-3p, | 80.66 | 85.02 | 83.64 |
| 45 | hsa-miR-181a-5p, hsa-miR-130a-3p, hsa-miR-1260b, hsa-miR-4306, hsa-miR-1301-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, hsa-miR-323b-3p, | 80.66 | 86.31 | 82.8 |
| 46 | hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-323a-3p, hsa-miR-550a-3-5p, age, | 80.63 | 85.41 | 86.14 |
| 47 | hsa-miR-326, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-28-3p, hsa-miR-328-3p, hsa-miR-323a-3p, hsa-miR-550a-3-5p, age, hsa-miR-323b-3p, | 80.63 | 84.69 | 85.38 |
| 48 | hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 80.18 | 85.15 | 81.89 |
| 49 | hsa-miR-326, hsa-miR-1260b, hsa-miR-4306, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 80.16 | 86.05 | 84.55 |
| 50 | hsa-miR-326, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, hsa-miR-550a-3-5p, age, hsa-miR-323b-3p, | 80.16 | 85.47 | 81.89 |
| 51 | hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, hsa-miR-323b-3p, | 80.16 | 81.61 | 82.58 |
| 52 | hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 80.13 | 84.73 | 83.48 |
| 53 | hsa-miR-181a-5p, hsa-miR-1260b, hsa-miR-4306, hsa-miR-21-5p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, hsa-miR-323b-3p, | 80.13 | 84.75 | 84.47 |
| 54 | hsa-miR-142-5p, hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-101-3p, hsa-miR-7-5p, hsa-miR-28-3p, hsa-miR-328-3p, hsa-miR-323a-3p, hsa-miR-550a-3-5p, age, hsa-miR-323b-3p, | 80.13 | 82.8 | 85.3 |
| 55 | hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, hsa-miR-323b-3p, | 80.13 | 84.05 | 81.74 |
| 56 | hsa-miR-142-5p, hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-101-3p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 80.13 | 83.98 | 85.3 |
| 57 | hsa-miR-142-5p, hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, | 80.13 | 84.72 | 84.39 |
| 58 | hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 80.13 | 85.77 | 83.56 |
| 59 | hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-374a-5p, hsa-miR-28-3p, hsa-miR-328-3p, hsa-miR-323a-3p, hsa-miR-550a-3-5p, age, hsa-miR-323b-3p, | 80.11 | 83.35 | 85.3 |
| 60 | hsa-miR-326, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-29a-3p, hsa-miR-28-3p, hsa-miR-328-3p, hsa-miR-323a-3p, hsa-miR-550a-3-5p, age, hsa-miR-323b-3p, | 80.11 | 84.36 | 83.64 |
| 61 | hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, hsa-miR-323b-3p, | 80.11 | 82.77 | 84.47 |
| 62 | hsa-miR-142-5p, hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-7-5p, hsa-miR-28-3p, hsa-miR-328-3p, hsa-miR-323a-3p, hsa-miR-550a-3-5p, age, hsa-miR-323b-3p, | 80.11 | 83.37 | 85.23 |
| 63 | hsa-miR-326, hsa-miR-181a-5p, hsa-miR-1260b, hsa-miR-1301-3p, hsa-miR-195-5p, hsa-miR-483-5p, hsa-miR-6803-3p, hsa-miR-181c-3p, hsa-miR-29a-3p, hsa-miR-7-5p, hsa-miR-28-3p, hsa-miR-15b-3p, hsa-miR-328-3p, hsa-miR-323a-3p, hsa-miR-550a-3-5p, hsa-miR-323b-3p, | 79.66 | 83.89 | 81.89 |
| 64 | hsa-miR-326, hsa-miR-181a-5p, hsa-miR-1260b, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, hsa-miR-323b-3p, | 79.66 | 82.6 | 81.82 |
| 65 | hsa-miR-30b-5p, hsa-miR-326, hsa-miR-1260b, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-15b-3p, hsa-miR-328-3p, hsa-miR-323a-3p, hsa-miR-550a-3-5p, age, hsa-miR-323b-3p, | 79.66 | 86.6 | 81.89 |
| 66 | hsa-miR-326, hsa-miR-142-5p, hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-28-3p, hsa-miR-328-3p, hsa-miR-323a-3p, hsa-miR-550a-3-5p, age, hsa-miR-323b-3p, | 79.66 | 85.2 | 83.56 |
| 67 | hsa-miR-181a-5p, hsa-miR-1260b, hsa-miR-4306, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, hsa-miR-323b-3p, | 79.66 | 83.25 | 84.55 |
| 68 | hsa-miR-181a-5p, hsa-miR-130a-3p, hsa-miR-4306, hsa-miR-1301-3p, hsa-miR-21-5p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 79.63 | 82.73 | 81.89 |
| 69 | hsa-miR-326, hsa-miR-181a-5p, hsa-miR-130a-3p, hsa-miR-1260b, hsa-miR-4306, hsa-miR-1301-3p, hsa-miR-21-5p, hsa-miR-195-5p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, hsa-miR-550a-3-5p, age, hsa-miR-323b-3p, | 79.63 | 86.36 | 82.05 |
| 70 | hsa-miR-30b-5p, hsa-miR-326, hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-15b-3p, hsa-miR-328-3p, hsa-miR-323a-3p, age, hsa-miR-323b-3p, | 79.63 | 84.26 | 81.97 |
| 71 | hsa-miR-326, hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-7-5p, hsa-miR-28-3p, hsa-miR-328-3p, hsa-miR-323a-3p, hsa-miR-550a-3-5p, age, hsa-miR-323b-3p, | 79.63 | 84.74 | 81.06 |
| 72 | hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-28-3p, hsa-miR-328-3p, hsa-miR-323a-3p, hsa-miR-550a-3-5p, age, hsa-miR-323b-3p, | 79.63 | 84.51 | 83.48 |
| 73 | hsa-miR-326, hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, age, hsa-miR-323b-3p, | 79.63 | 84.83 | 83.56 |
| 74 | hsa-miR-181a-5p, hsa-miR-130a-3p, hsa-miR-1260b, hsa-miR-4306, hsa-miR-1301-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 79.63 | 85.26 | 83.71 |
| 75 | hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, | 79.63 | 85.8 | 84.47 |
| 76 | hsa-miR-326, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, hsa-miR-550a-3-5p, age, hsa-miR-323b-3p, | 79.63 | 85.88 | 80.15 |
| 77 | hsa-miR-326, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 79.63 | 85.66 | 83.56 |
| 78 | hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-28-3p, hsa-miR-328-3p, hsa-miR-323a-3p, hsa-miR-550a-3-5p, age, hsa-miR-323b-3p, | 79.63 | 84.77 | 82.8 |
| 79 | hsa-miR-142-5p, hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-28-3p, hsa-miR-328-3p, hsa-miR-323a-3p, hsa-miR-550a-3-5p, age, hsa-miR-323b-3p, | 79.63 | 84.19 | 83.48 |
| 80 | hsa-miR-142-5p, hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, | 79.61 | 83.47 | 84.39 |
| 81 | hsa-miR-142-5p, hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-28-3p, hsa-miR-328-3p, hsa-miR-323a-3p, age, hsa-miR-323b-3p, | 79.61 | 83.84 | 84.39 |
| 82 | hsa-miR-326, hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-29a-3p, hsa-miR-28-3p, hsa-miR-328-3p, hsa-miR-323a-3p, age, hsa-miR-323b-3p, | 79.61 | 81.07 | 83.64 |
| 83 | hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-374a-5p, hsa-miR-28-3p, hsa-miR-15b-3p, hsa-miR-328-3p, hsa-miR-323a-3p, hsa-miR-550a-3-5p, age, hsa-miR-323b-3p, | 79.61 | 85.09 | 84.55 |
| 84 | hsa-miR-142-5p, hsa-miR-130a-3p, hsa-miR-1260b, hsa-miR-4306, hsa-miR-1301-3p, hsa-miR-21-5p, hsa-miR-377-3p, hsa-miR-483-5p, hsa-miR-6803-3p, hsa-miR-181c-3p, hsa-miR-101-3p, hsa-miR-29a-3p, hsa-miR-7-5p, hsa-miR-28-3p, hsa-miR-328-3p, hsa-miR-323a-3p, age, hsa-miR-323b-3p, | 79.61 | 83.51 | 85.3 |
| 85 | hsa-miR-326, hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 79.61 | 81 | 85.38 |
| 86 | hsa-miR-142-5p, hsa-miR-181a-5p, hsa-miR-130a-3p, hsa-miR-1260b, hsa-miR-4306, hsa-miR-1301-3p, hsa-miR-21-5p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 79.61 | 84.89 | 83.56 |
| 87 | hsa-miR-142-5p, hsa-miR-130a-3p, hsa-miR-4306, hsa-miR-1301-3p, hsa-miR-377-3p, hsa-miR-483-5p, hsa-miR-6803-3p, hsa-miR-181c-3p, hsa-miR-101-3p, hsa-miR-29a-3p, hsa-miR-7-5p, hsa-miR-28-3p, hsa-miR-328-3p, hsa-miR-323a-3p, age, hsa-miR-323b-3p, | 79.61 | 84.46 | 85.38 |
| 88 | hsa-miR-326, hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-423-5p, hsa-miR-7-5p, hsa-miR-28-3p, hsa-miR-328-3p, hsa-miR-323a-3p, hsa-miR-550a-3-5p, age, hsa-miR-323b-3p, | 79.58 | 83.01 | 84.55 |
| 89 | hsa-miR-326, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-28-3p, hsa-miR-323a-3p, hsa-miR-550a-3-5p, age, hsa-miR-323b-3p, | 79.58 | 84.99 | 84.39 |
| 90 | hsa-miR-142-5p, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, | 79.58 | 84.53 | 84.24 |
| 91 | hsa-miR-326, hsa-miR-181a-5p, hsa-miR-1260b, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-28-3p, hsa-miR-15b-3p, hsa-miR-328-3p, hsa-miR-323a-3p, hsa-miR-550a-3-5p, hsa-miR-323b-3p, | 79.16 | 85.87 | 83.79 |
| 92 | hsa-miR-326, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, age, | 79.16 | 82.77 | 81.89 |
| 93 | hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 79.13 | 84.56 | 84.55 |
| 94 | hsa-miR-142-5p, hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, | 79.13 | 85.02 | 85.3 |
| 95 | hsa-miR-142-5p, hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, age, hsa-miR-323b-3p, | 79.13 | 84.27 | 83.56 |
| 96 | hsa-miR-326, hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 79.11 | 85.8 | 83.71 |
| 97 | hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 79.11 | 84.98 | 82.73 |
| 98 | hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, | 79.11 | 84.75 | 82.65 |
| 99 | hsa-miR-30b-5p, hsa-miR-326, hsa-miR-142-5p, hsa-miR-1260b, hsa-miR-1301-3p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, hsa-miR-323b-3p, | 79.11 | 84.37 | 84.39 |
| 100 | hsa-miR-30b-5p, hsa-miR-142-5p, hsa-miR-181a-5p, hsa-miR-1260b, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-15b-3p, hsa-miR-323a-3p, hsa-miR-550a-3-5p, hsa-miR-323b-3p, | 79.11 | 84.37 | 85.98 |
| 101 | hsa-miR-142-5p, hsa-miR-181a-5p, hsa-miR-1260b, hsa-miR-4306, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-15b-3p, hsa-miR-328-3p, hsa-miR-323a-3p, age, hsa-miR-323b-3p, | 79.11 | 85.45 | 83.48 |
| 102 | hsa-miR-326, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 79.11 | 84.14 | 82.73 |
| 103 | hsa-miR-326, hsa-miR-142-5p, hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-423-5p, hsa-miR-28-3p, hsa-miR-328-3p, hsa-miR-323a-3p, hsa-miR-550a-3-5p, age, | 79.08 | 82.82 | 82.65 |
| 104 | hsa-miR-142-5p, hsa-miR-181a-5p, hsa-miR-4306, hsa-miR-1301-3p, hsa-miR-21-5p, hsa-miR-377-3p, hsa-miR-483-5p, hsa-miR-6803-3p, hsa-miR-101-3p, hsa-miR-29a-3p, hsa-miR-28-3p, hsa-miR-328-3p, hsa-miR-323a-3p, hsa-miR-550a-3-5p, age, hsa-miR-323b-3p, | 79.08 | 84.41 | 83.48 |
| 105 | hsa-miR-326, hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-423-5p, hsa-miR-29a-3p, hsa-miR-28-3p, hsa-miR-328-3p, hsa-miR-323a-3p, age, hsa-miR-323b-3p, | 79.08 | 81.54 | 83.64 |
| 106 | hsa-miR-142-5p, hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-323a-3p, hsa-miR-550a-3-5p, age, hsa-miR-323b-3p, | 79.08 | 85.14 | 83.56 |
| 107 | hsa-miR-326, hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-423-5p, hsa-miR-28-3p, hsa-miR-328-3p, hsa-miR-323a-3p, age, hsa-miR-323b-3p, | 79.08 | 83.49 | 84.55 |
| 108 | hsa-miR-142-5p, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, hsa-miR-323b-3p, | 79.08 | 85.37 | 84.39 |
| 109 | hsa-miR-142-5p, hsa-miR-181a-5p, hsa-miR-1260b, hsa-miR-4306, hsa-miR-21-5p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, hsa-miR-323b-3p, | 79.08 | 84.07 | 83.56 |
| 110 | hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, hsa-miR-323b-3p, | 79.08 | 84.77 | 80.98 |
| 111 | hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-323a-3p, hsa-miR-550a-3-5p, age, hsa-miR-323b-3p, | 79.08 | 84.99 | 85.38 |
| 112 | hsa-miR-142-5p, hsa-miR-130a-3p, hsa-miR-1260b, hsa-miR-4306, hsa-miR-1301-3p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, hsa-miR-323b-3p, | 79.08 | 84.37 | 84.39 |
| 113 | hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-101-3p, hsa-miR-423-5p, hsa-miR-29a-3p, hsa-miR-28-3p, hsa-miR-328-3p, hsa-miR-323a-3p, age, hsa-miR-323b-3p, | 79.08 | 81.09 | 83.56 |
| 114 | hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, | 79.08 | 84.61 | 86.06 |
| 115 | hsa-miR-326, hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-423-5p, hsa-miR-28-3p, hsa-miR-328-3p, hsa-miR-323a-3p, hsa-miR-550a-3-5p, age, hsa-miR-323b-3p, | 79.08 | 83.48 | 82.88 |
| 116 | hsa-miR-326, hsa-miR-181a-5p, hsa-miR-130a-3p, hsa-miR-1260b, hsa-miR-4306, hsa-miR-1301-3p, hsa-miR-21-5p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 79.08 | 83.31 | 83.71 |
| 117 | hsa-miR-142-5p, hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 79.08 | 84.69 | 82.65 |
| 118 | hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, hsa-miR-323b-3p, | 79.08 | 82.03 | 82.65 |
| 119 | hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-101-3p, hsa-miR-374a-5p, hsa-miR-28-3p, hsa-miR-328-3p, hsa-miR-323a-3p, hsa-miR-550a-3-5p, age, hsa-miR-323b-3p, | 79.08 | 83.1 | 84.32 |
| 120 | hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-28-3p, hsa-miR-328-3p, hsa-miR-323a-3p, hsa-miR-550a-3-5p, age, hsa-miR-323b-3p, | 79.08 | 84.6 | 81.74 |
| 121 | hsa-miR-326, hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-423-5p, hsa-miR-29a-3p, hsa-miR-7-5p, hsa-miR-28-3p, hsa-miR-328-3p, hsa-miR-323a-3p, age, hsa-miR-323b-3p, | 79.08 | 80.71 | 83.64 |
| 122 | hsa-miR-326, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-28-3p, hsa-miR-323a-3p, hsa-miR-550a-3-5p, age, hsa-miR-323b-3p, | 79.05 | 84.67 | 83.48 |
| 123 | hsa-miR-326, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-7-5p, hsa-miR-28-3p, hsa-miR-323a-3p, hsa-miR-550a-3-5p, age, hsa-miR-323b-3p, | 79.05 | 84.18 | 86.06 |
| 124 | hsa-miR-130a-3p, hsa-miR-4306, hsa-miR-1301-3p, hsa-miR-377-3p, hsa-miR-483-5p, hsa-miR-6803-3p, hsa-miR-181c-3p, hsa-miR-101-3p, hsa-miR-29a-3p, hsa-miR-28-3p, hsa-miR-328-3p, hsa-miR-323a-3p, hsa-miR-550a-3-5p, age, hsa-miR-323b-3p, | 79.05 | 84.75 | 82.73 |
| 125 | hsa-miR-142-5p, hsa-miR-181a-5p, hsa-miR-92b-3p, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-7-5p, hsa-miR-28-3p, hsa-miR-328-3p, hsa-miR-323a-3p, hsa-miR-550a-3-5p, age, hsa-miR-323b-3p, | 79.05 | 82.92 | 85.15 |
| 126 | hsa-miR-326, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, age, | 78.63 | 83.14 | 81.06 |
| 127 | hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-7-5p, hsa-miR-28-3p, hsa-miR-328-3p, hsa-miR-323a-3p, hsa-miR-550a-3-5p, age, hsa-miR-323b-3p, | 78.63 | 83.52 | 80.98 |
| 128 | hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-29a-3p, hsa-miR-7-5p, hsa-miR-28-3p, hsa-miR-15b-3p, hsa-miR-328-3p, hsa-miR-323a-3p, age, | 78.61 | 85.42 | 83.56 |
| 129 | hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-28-3p, hsa-miR-328-3p, hsa-miR-323a-3p, age, hsa-miR-323b-3p, | 78.61 | 83.65 | 82.65 |
| 130 | hsa-miR-142-5p, hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-28-3p, hsa-miR-15b-3p, hsa-miR-323a-3p, age, hsa-miR-323b-3p, | 78.61 | 83.28 | 84.55 |
| 131 | hsa-miR-326, hsa-miR-181a-5p, hsa-miR-1260b, hsa-miR-4306, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, hsa-miR-550a-3-5p, age, hsa-miR-323b-3p, | 78.61 | 86.04 | 84.62 |
| 132 | hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-28-3p, hsa-miR-328-3p, hsa-miR-323a-3p, hsa-miR-550a-3-5p, age, hsa-miR-323b-3p, | 78.61 | 84.62 | 83.48 |
| 133 | hsa-miR-326, hsa-miR-181a-5p, hsa-miR-1260b, hsa-miR-4306, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-15b-3p, hsa-miR-328-3p, hsa-miR-323a-3p, hsa-miR-550a-3-5p, age, | 78.61 | 86.2 | 82.8 |
| 134 | hsa-miR-326, hsa-miR-181a-5p, hsa-miR-1260b, hsa-miR-4306, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 78.61 | 85.22 | 82.8 |
| 135 | hsa-miR-142-5p, hsa-miR-130a-3p, hsa-miR-4306, hsa-miR-1301-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, | 78.61 | 84.25 | 81.89 |
| 136 | hsa-miR-142-5p, hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-7-5p, hsa-miR-28-3p, hsa-miR-328-3p, hsa-miR-323a-3p, hsa-miR-550a-3-5p, age, hsa-miR-323b-3p, | 78.61 | 84.27 | 83.48 |
| 137 | hsa-miR-326, hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-28-3p, hsa-miR-328-3p, hsa-miR-323a-3p, age, | 78.58 | 82.64 | 84.47 |
| 138 | hsa-miR-326, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-15b-3p, hsa-miR-328-3p, hsa-miR-323a-3p, hsa-miR-550a-3-5p, age, | 78.58 | 86.71 | 83.56 |
| 139 | hsa-miR-326, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 78.58 | 83.41 | 83.64 |
| 140 | hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 78.58 | 86 | 82.58 |
| 141 | hsa-miR-30b-5p, hsa-miR-326, hsa-miR-142-5p, hsa-miR-181a-5p, hsa-miR-1260b, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-15b-3p, hsa-miR-328-3p, hsa-miR-323a-3p, hsa-miR-323b-3p, | 78.58 | 84.13 | 81.06 |
| 142 | hsa-miR-30b-5p, hsa-miR-181a-5p, hsa-miR-1260b, hsa-miR-4306, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, hsa-miR-323b-3p, | 78.58 | 84.42 | 80.83 |
| 143 | hsa-miR-142-5p, hsa-miR-1260b, hsa-miR-21-5p, hsa-miR-377-3p, hsa-miR-101-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, hsa-miR-323b-3p, | 78.58 | 81.07 | 85.98 |
| 144 | hsa-miR-142-5p, hsa-miR-181a-5p, hsa-miR-1260b, hsa-miR-4306, hsa-miR-195-5p, hsa-miR-483-5p, hsa-miR-181c-3p, hsa-miR-101-3p, hsa-miR-374a-5p, hsa-miR-423-5p, hsa-miR-29a-3p, hsa-miR-28-3p, hsa-miR-323a-3p, hsa-miR-550a-3-5p, age, hsa-miR-323b-3p, | 78.58 | 84.58 | 81.89 |
| 145 | hsa-miR-326, hsa-miR-181a-5p, hsa-miR-130a-3p, hsa-miR-1260b, hsa-miR-4306, hsa-miR-1301-3p, hsa-miR-21-5p, hsa-miR-195-5p, hsa-miR-483-5p, hsa-miR-6803-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 78.58 | 82.71 | 81.97 |
| 146 | hsa-miR-326, hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-7-5p, hsa-miR-28-3p, hsa-miR-328-3p, hsa-miR-323a-3p, hsa-miR-550a-3-5p, age, hsa-miR-323b-3p, | 78.58 | 84.47 | 83.56 |
| 147 | hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-323a-3p, hsa-miR-550a-3-5p, age, | 78.58 | 85.35 | 83.64 |
| 148 | hsa-miR-326, hsa-miR-142-5p, hsa-miR-181a-5p, hsa-miR-1260b, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-15b-3p, hsa-miR-328-3p, hsa-miR-323a-3p, hsa-miR-550a-3-5p, hsa-miR-323b-3p, | 78.58 | 83.55 | 80.83 |
| 149 | hsa-miR-181a-5p, hsa-miR-130a-3p, hsa-miR-4306, hsa-miR-1301-3p, hsa-miR-21-5p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 78.55 | 84.62 | 82.73 |
| 150 | hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-323a-3p, hsa-miR-550a-3-5p, age, hsa-miR-323b-3p, | 78.55 | 84.55 | 83.56 |
| 151 | hsa-miR-326, hsa-miR-130a-3p, hsa-miR-1260b, hsa-miR-4306, hsa-miR-1301-3p, hsa-miR-21-5p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 78.55 | 85.2 | 83.56 |
| 152 | hsa-miR-326, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-15b-3p, hsa-miR-328-3p, hsa-miR-323a-3p, hsa-miR-550a-3-5p, age, hsa-miR-323b-3p, | 78.55 | 86.36 | 82.73 |
| 153 | hsa-miR-326, hsa-miR-181a-5p, hsa-miR-130a-3p, hsa-miR-1260b, hsa-miR-4306, hsa-miR-1301-3p, hsa-miR-21-5p, hsa-miR-483-5p, hsa-miR-6803-3p, hsa-miR-181c-3p, hsa-miR-423-5p, hsa-miR-29a-3p, hsa-miR-28-3p, hsa-miR-328-3p, hsa-miR-323a-3p, hsa-miR-550a-3-5p, age, hsa-miR-323b-3p, | 78.55 | 85.55 | 82.8 |
| 154 | hsa-miR-142-5p, hsa-miR-181a-5p, hsa-miR-130a-3p, hsa-miR-1260b, hsa-miR-4306, hsa-miR-1301-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, hsa-miR-323b-3p, | 78.55 | 85.3 | 83.64 |
| 155 | hsa-miR-142-5p, hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, hsa-miR-323b-3p, | 78.55 | 84.2 | 83.56 |
| 156 | hsa-miR-326, hsa-miR-142-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-28-3p, hsa-miR-328-3p, hsa-miR-323a-3p, hsa-miR-550a-3-5p, age, hsa-miR-323b-3p, | 78.55 | 84.2 | 83.41 |
| 157 | hsa-miR-326, hsa-miR-181a-5p, hsa-miR-92b-3p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-28-3p, hsa-miR-328-3p, hsa-miR-323a-3p, age, | 78.55 | 82.4 | 81.82 |
| 158 | hsa-miR-181a-5p, hsa-miR-130a-3p, hsa-miR-1260b, hsa-miR-4306, hsa-miR-1301-3p, hsa-miR-21-5p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, | 78.11 | 83 | 81.89 |
| 159 | hsa-miR-326, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 78.11 | 81.18 | 84.7 |
| 160 | hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, | 78.11 | 84.64 | 82.73 |
| 161 | hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, | 78.11 | 83.96 | 82.8 |
| 162 | hsa-miR-326, hsa-miR-181a-5p, hsa-miR-130a-3p, hsa-miR-1260b, hsa-miR-1301-3p, hsa-miR-21-5p, hsa-miR-195-5p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-15b-3p, hsa-miR-328-3p, hsa-miR-550a-3-5p, hsa-miR-323b-3p, | 78.11 | 83.02 | 81.74 |
| 163 | hsa-miR-181a-5p, hsa-miR-1260b, hsa-miR-4306, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, hsa-miR-323b-3p, | 78.08 | 84.56 | 81.89 |
| 164 | hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, | 78.08 | 85.91 | 83.64 |
| 165 | hsa-miR-326, hsa-miR-181a-5p, hsa-miR-130a-3p, hsa-miR-1260b, hsa-miR-4306, hsa-miR-1301-3p, hsa-miR-21-5p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, hsa-miR-550a-3-5p, age, hsa-miR-323b-3p, | 78.08 | 85.23 | 82.88 |
| 166 | hsa-miR-142-5p, hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-28-3p, hsa-miR-323a-3p, age, hsa-miR-323b-3p, | 78.08 | 82.82 | 83.56 |
| 167 | hsa-miR-326, hsa-miR-1260b, hsa-miR-4306, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 78.08 | 85.99 | 81.89 |
| 168 | hsa-miR-326, hsa-miR-142-5p, hsa-miR-181a-5p, hsa-miR-1260b, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, hsa-miR-323b-3p, | 78.08 | 81.83 | 81.74 |
| 169 | hsa-miR-326, hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-29a-3p, hsa-miR-28-3p, hsa-miR-328-3p, hsa-miR-323a-3p, age, | 78.08 | 83.22 | 83.56 |
| 170 | hsa-miR-326, hsa-miR-181a-5p, hsa-miR-1260b, hsa-miR-4306, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 78.08 | 84.46 | 81.89 |
| 171 | hsa-miR-181a-5p, hsa-miR-130a-3p, hsa-miR-1260b, hsa-miR-4306, hsa-miR-1301-3p, hsa-miR-21-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 78.08 | 84.22 | 83.64 |
| 172 | hsa-miR-326, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, hsa-miR-550a-3-5p, age, | 78.08 | 84.94 | 80.08 |
| 173 | hsa-miR-181a-5p, hsa-miR-1260b, hsa-miR-4306, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 78.05 | 85.03 | 81.89 |
| 174 | hsa-miR-142-5p, hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-28-3p, hsa-miR-15b-3p, hsa-miR-323a-3p, hsa-miR-550a-3-5p, age, hsa-miR-323b-3p, | 78.05 | 84.83 | 82.8 |
| 175 | hsa-miR-326, hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-423-5p, hsa-miR-7-5p, hsa-miR-28-3p, hsa-miR-328-3p, hsa-miR-323a-3p, hsa-miR-550a-3-5p, age, hsa-miR-323b-3p, | 78.05 | 84.14 | 83.71 |
| 176 | hsa-miR-142-5p, hsa-miR-181a-5p, hsa-miR-4306, hsa-miR-377-3p, hsa-miR-483-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 78.05 | 82.7 | 84.47 |
| 177 | hsa-miR-326, hsa-miR-181a-5p, hsa-miR-92b-3p, hsa-miR-1260b, hsa-miR-4306, hsa-miR-1301-3p, hsa-miR-195-5p, hsa-miR-483-5p, hsa-miR-6803-3p, hsa-miR-181c-3p, hsa-miR-101-3p, hsa-miR-29a-3p, hsa-miR-28-3p, hsa-miR-15b-3p, hsa-miR-328-3p, hsa-miR-323a-3p, hsa-miR-323b-3p, | 78.05 | 85.44 | 80.98 |
| 178 | hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-323a-3p, hsa-miR-550a-3-5p, age, | 78.05 | 84.96 | 83.56 |
| 179 | hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-423-5p, hsa-miR-29a-3p, hsa-miR-28-3p, hsa-miR-323a-3p, hsa-miR-550a-3-5p, age, hsa-miR-323b-3p, | 78.05 | 84.97 | 84.55 |
| 180 | hsa-miR-326, hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-28-3p, hsa-miR-328-3p, hsa-miR-323a-3p, hsa-miR-550a-3-5p, age, hsa-miR-323b-3p, | 78.05 | 84.83 | 80.15 |
| 181 | hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-15b-3p, hsa-miR-328-3p, hsa-miR-323a-3p, hsa-miR-550a-3-5p, age, | 78.05 | 85.93 | 82.73 |
| 182 | hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 78.05 | 83.66 | 80.76 |
| 183 | hsa-miR-130a-3p, hsa-miR-4306, hsa-miR-1301-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 78.05 | 83.56 | 81.82 |
| 184 | hsa-miR-30b-5p, hsa-miR-142-5p, hsa-miR-181a-5p, hsa-miR-1260b, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-15b-3p, hsa-miR-328-3p, hsa-miR-323a-3p, hsa-miR-550a-3-5p, hsa-miR-323b-3p, | 78.05 | 85.64 | 80.83 |
| 185 | hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, age, hsa-miR-323b-3p, | 78.05 | 84.2 | 82.65 |
| 186 | hsa-miR-326, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-28-3p, hsa-miR-323a-3p, hsa-miR-550a-3-5p, age, hsa-miR-323b-3p, | 78.03 | 84.38 | 82.73 |
| 187 | hsa-miR-326, hsa-miR-1260b, hsa-miR-4306, hsa-miR-21-5p, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-28-3p, hsa-miR-323a-3p, hsa-miR-550a-3-5p, age, hsa-miR-323b-3p, | 78.03 | 84.61 | 80.98 |
| 188 | hsa-miR-142-5p, hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-29a-3p, hsa-miR-7-5p, hsa-miR-28-3p, hsa-miR-328-3p, hsa-miR-323a-3p, age, | 78.03 | 81.71 | 82.65 |
| 189 | hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, hsa-miR-323b-3p, | 78.03 | 85.03 | 80.76 |
| 190 | hsa-miR-326, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, hsa-miR-550a-3-5p, age, hsa-miR-323b-3p, | 78.03 | 85.73 | 79.17 |
| 191 | hsa-miR-326, hsa-miR-142-5p, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-28-3p, hsa-miR-328-3p, hsa-miR-323a-3p, hsa-miR-550a-3-5p, age, | 78.03 | 82.66 | 82.58 |
| 192 | hsa-miR-326, hsa-miR-142-5p, hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-7-5p, hsa-miR-28-3p, hsa-miR-328-3p, hsa-miR-323a-3p, hsa-miR-550a-3-5p, age, | 78.03 | 81.43 | 81.74 |
| 193 | hsa-miR-326, hsa-miR-130a-3p, hsa-miR-1260b, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-550a-3-5p, hsa-miR-323b-3p, | 77.58 | 80.76 | 82.58 |
| 194 | hsa-miR-326, hsa-miR-181a-5p, hsa-miR-1260b, hsa-miR-195-5p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, hsa-miR-323b-3p, | 77.58 | 79.56 | 81.97 |
| 195 | hsa-miR-30b-5p, hsa-miR-142-5p, hsa-miR-181a-5p, hsa-miR-1260b, hsa-miR-1301-3p, hsa-miR-21-5p, hsa-miR-195-5p, hsa-miR-6803-3p, hsa-miR-181c-3p, hsa-miR-101-3p, hsa-miR-374a-5p, hsa-miR-29a-3p, hsa-miR-7-5p, hsa-miR-15b-3p, hsa-miR-328-3p, hsa-miR-550a-3-5p, hsa-miR-323b-3p, | 77.58 | 81.72 | 80.83 |
| 196 | hsa-miR-181a-5p, hsa-miR-1260b, hsa-miR-4306, hsa-miR-21-5p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-323a-3p, hsa-miR-550a-3-5p, age, | 77.58 | 85.14 | 82.73 |
| 197 | hsa-miR-181a-5p, hsa-miR-1260b, hsa-miR-4306, hsa-miR-1301-3p, hsa-miR-21-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, | 77.58 | 83.11 | 80.98 |
| 198 | hsa-miR-30b-5p, hsa-miR-326, hsa-miR-181a-5p, hsa-miR-1260b, hsa-miR-21-5p, hsa-miR-195-5p, hsa-miR-377-3p, hsa-miR-6803-3p, hsa-miR-181c-3p, hsa-miR-374a-5p, hsa-miR-423-5p, hsa-miR-29a-3p, hsa-miR-15b-3p, hsa-miR-328-3p, hsa-miR-323a-3p, hsa-miR-550a-3-5p, age, hsa-miR-323b-3p, | 77.58 | 84.61 | 82.73 |
| 199 | hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 77.55 | 85 | 81.89 |
| 200 | hsa-miR-326, hsa-miR-142-5p, hsa-miR-181a-5p, hsa-miR-92b-3p, hsa-miR-1260b, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-29a-3p, hsa-miR-28-3p, hsa-miR-15b-3p, hsa-miR-328-3p, hsa-miR-323a-3p, hsa-miR-323b-3p, | 77.55 | 83.38 | 82.73 |
| 201 | hsa-miR-326, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-7-5p, hsa-miR-28-3p, hsa-miR-323a-3p, age, hsa-miR-323b-3p, | 77.55 | 81.7 | 81.89 |
| 202 | hsa-miR-181a-5p, hsa-miR-92b-3p, hsa-miR-1260b, hsa-miR-4306, hsa-miR-1301-3p, hsa-miR-195-5p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 77.55 | 84.81 | 80.98 |
| 203 | hsa-miR-326, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 77.55 | 85.01 | 81.82 |
| 204 | hsa-miR-30b-5p, hsa-miR-326, hsa-miR-181a-5p, hsa-miR-130a-3p, hsa-miR-1260b, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-29a-3p, hsa-miR-7-5p, hsa-miR-15b-3p, hsa-miR-323a-3p, hsa-miR-550a-3-5p, hsa-miR-323b-3p, | 77.55 | 84.94 | 85.23 |
| 205 | hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, | 77.55 | 83.36 | 80.98 |
| 206 | hsa-miR-142-5p, hsa-miR-181a-5p, hsa-miR-1260b, hsa-miR-4306, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 77.55 | 84.67 | 80.91 |
| 207 | hsa-miR-30b-5p, hsa-miR-142-5p, hsa-miR-181a-5p, hsa-miR-1260b, hsa-miR-1301-3p, hsa-miR-195-5p, hsa-miR-6803-3p, hsa-miR-181c-3p, hsa-miR-101-3p, hsa-miR-374a-5p, hsa-miR-29a-3p, hsa-miR-15b-3p, hsa-miR-328-3p, hsa-miR-550a-3-5p, hsa-miR-323b-3p, | 77.55 | 81.54 | 79.92 |
| 208 | hsa-miR-326, hsa-miR-181a-5p, hsa-miR-4306, hsa-miR-1301-3p, hsa-miR-195-5p, hsa-miR-483-5p, hsa-miR-6803-3p, hsa-miR-181c-3p, hsa-miR-28-3p, hsa-miR-15b-3p, hsa-miR-328-3p, hsa-miR-323a-3p, | 77.55 | 84.74 | 80.23 |
| 209 | hsa-miR-326, hsa-miR-142-5p, hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-28-3p, hsa-miR-328-3p, hsa-miR-323a-3p, age, | 77.53 | 81.83 | 84.32 |
| 210 | hsa-miR-30b-5p, hsa-miR-326, hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-15b-3p, hsa-miR-328-3p, hsa-miR-323a-3p, age, hsa-miR-323b-3p, | 77.53 | 85.09 | 81.67 |
| 211 | hsa-miR-326, hsa-miR-142-5p, hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-28-3p, hsa-miR-328-3p, hsa-miR-323a-3p, hsa-miR-550a-3-5p, age, | 77.53 | 82.44 | 82.8 |
| 212 | hsa-miR-181a-5p, hsa-miR-130a-3p, hsa-miR-4306, hsa-miR-1301-3p, hsa-miR-21-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 77.53 | 82.71 | 79.24 |
| 213 | hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 77.5 | 84.91 | 80.76 |
| 214 | hsa-miR-142-5p, hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-101-3p, hsa-miR-29a-3p, hsa-miR-7-5p, hsa-miR-28-3p, hsa-miR-328-3p, hsa-miR-323a-3p, hsa-miR-550a-3-5p, age, | 77.5 | 83.41 | 83.64 |
| 215 | hsa-miR-130a-3p, hsa-miR-4306, hsa-miR-1301-3p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, hsa-miR-323a-3p, hsa-miR-550a-3-5p, age, hsa-miR-323b-3p, | 77.5 | 82.7 | 82.73 |
| 216 | hsa-miR-142-5p, hsa-miR-181a-5p, hsa-miR-130a-3p, hsa-miR-4306, hsa-miR-1301-3p, hsa-miR-377-3p, hsa-miR-483-5p, hsa-miR-6803-3p, hsa-miR-101-3p, hsa-miR-29a-3p, hsa-miR-7-5p, hsa-miR-28-3p, hsa-miR-328-3p, hsa-miR-323a-3p, age, | 77.5 | 83.3 | 82.65 |
| 217 | hsa-miR-142-5p, hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-101-3p, hsa-miR-7-5p, hsa-miR-28-3p, hsa-miR-328-3p, hsa-miR-323a-3p, hsa-miR-550a-3-5p, age, hsa-miR-323b-3p, | 77.5 | 83.51 | 81.82 |
| 218 | hsa-miR-326, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-15b-3p, hsa-miR-328-3p, hsa-miR-323a-3p, hsa-miR-550a-3-5p, age, hsa-miR-323b-3p, | 77.5 | 86.62 | 80.98 |
| 219 | hsa-miR-142-5p, hsa-miR-181a-5p, hsa-miR-4306, hsa-miR-1301-3p, hsa-miR-377-3p, hsa-miR-483-5p, hsa-miR-6803-3p, hsa-miR-101-3p, hsa-miR-29a-3p, hsa-miR-7-5p, hsa-miR-28-3p, hsa-miR-328-3p, hsa-miR-323a-3p, age, | 77.5 | 82.39 | 82.73 |
| 220 | hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-323a-3p, hsa-miR-550a-3-5p, age, hsa-miR-323b-3p, | 77.5 | 84.6 | 81.67 |
| 221 | hsa-miR-142-5p, hsa-miR-181a-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-323a-3p, hsa-miR-550a-3-5p, age, hsa-miR-323b-3p, | 77.5 | 84.28 | 81.74 |
| 222 | hsa-miR-130a-3p, hsa-miR-4306, hsa-miR-1301-3p, hsa-miR-21-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, hsa-miR-323b-3p, | 77.47 | 83.49 | 80.91 |
| 223 | hsa-miR-181a-5p, hsa-miR-130a-3p, hsa-miR-1260b, hsa-miR-4306, hsa-miR-1301-3p, hsa-miR-21-5p, hsa-miR-377-3p, hsa-miR-483-5p, hsa-miR-6803-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, | 77.08 | 80.19 | 83.64 |
| 224 | hsa-miR-326, hsa-miR-181a-5p, hsa-miR-1260b, hsa-miR-195-5p, hsa-miR-377-3p, hsa-miR-483-5p, hsa-miR-181c-3p, hsa-miR-29a-3p, hsa-miR-7-5p, hsa-miR-28-3p, hsa-miR-328-3p, hsa-miR-323a-3p, hsa-miR-550a-3-5p, hsa-miR-323b-3p, | 77.05 | 82.15 | 81.89 |
| 225 | hsa-miR-30b-5p, hsa-miR-181a-5p, hsa-miR-92b-3p, hsa-miR-130a-3p, hsa-miR-1260b, hsa-miR-21-5p, hsa-miR-377-3p, hsa-miR-483-5p, hsa-miR-181c-3p, hsa-miR-101-3p, hsa-miR-374a-5p, hsa-miR-29a-3p, hsa-miR-7-5p, hsa-miR-15b-3p, hsa-miR-323a-3p, hsa-miR-550a-3-5p, age, | 77.05 | 84.49 | 82.65 |
| 226 | hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, | 77.05 | 82.85 | 80.91 |
| 227 | hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, | 77.05 | 82.19 | 81.82 |
| 228 | hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 77.05 | 84.64 | 80.15 |
| 229 | hsa-miR-326, hsa-miR-142-5p, hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-28-3p, hsa-miR-328-3p, hsa-miR-323a-3p, age, | 77.03 | 82.41 | 82.65 |
| 230 | hsa-miR-130a-3p, hsa-miR-1260b, hsa-miR-4306, hsa-miR-1301-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 77.03 | 83.45 | 80.15 |
| 231 | hsa-miR-30b-5p, hsa-miR-326, hsa-miR-1260b, hsa-miR-1301-3p, hsa-miR-21-5p, hsa-miR-195-5p, hsa-miR-377-3p, hsa-miR-6803-3p, hsa-miR-181c-3p, hsa-miR-374a-5p, hsa-miR-423-5p, hsa-miR-29a-3p, hsa-miR-7-5p, hsa-miR-15b-3p, hsa-miR-328-3p, hsa-miR-550a-3-5p, | 77.03 | 82.72 | 82.5 |
| 232 | hsa-miR-326, hsa-miR-142-5p, hsa-miR-1260b, hsa-miR-4306, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, age, hsa-miR-323b-3p, | 77.03 | 83.56 | 81.89 |
| 233 | hsa-miR-326, hsa-miR-181a-5p, hsa-miR-1260b, hsa-miR-4306, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, hsa-miR-323b-3p, | 77.03 | 84.13 | 81.06 |
| 234 | hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-15b-3p, hsa-miR-328-3p, hsa-miR-323a-3p, age, | 77.03 | 82.35 | 80.91 |
| 235 | hsa-miR-30b-5p, hsa-miR-142-5p, hsa-miR-181a-5p, hsa-miR-1260b, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-15b-3p, hsa-miR-328-3p, hsa-miR-550a-3-5p, hsa-miR-323b-3p, | 77.03 | 78.58 | 79.02 |
| 236 | hsa-miR-326, hsa-miR-181a-5p, hsa-miR-1260b, hsa-miR-4306, hsa-miR-21-5p, hsa-miR-195-5p, hsa-miR-377-3p, hsa-miR-483-5p, hsa-miR-181c-3p, hsa-miR-374a-5p, hsa-miR-423-5p, hsa-miR-29a-3p, hsa-miR-7-5p, hsa-miR-28-3p, hsa-miR-323a-3p, age, hsa-miR-323b-3p, | 77.03 | 82.22 | 81.89 |
| 237 | hsa-miR-142-5p, hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-423-5p, hsa-miR-29a-3p, hsa-miR-7-5p, hsa-miR-28-3p, hsa-miR-323a-3p, age, hsa-miR-323b-3p, | 77.03 | 82.8 | 81.82 |
| 238 | hsa-miR-181a-5p, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 77.03 | 84.3 | 80.98 |
| 239 | hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 77.03 | 84.79 | 81.74 |
| 240 | hsa-miR-326, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, | 77.00 | 83.30 | 82.65 |
| 241 | hsa-miR-326, hsa-miR-142-5p, hsa-miR-181a-5p, hsa-miR-1260b, hsa-miR-21-5p, hsa-miR-195-5p, hsa-miR-483-5p, hsa-miR-181c-3p, hsa-miR-29a-3p, hsa-miR-7-5p, hsa-miR-28-3p, hsa-miR-15b-3p, hsa-miR-323a-3p, hsa-miR-550a-3-5p, hsa-miR-323b-3p, | 77.00 | 82.07 | 81.67 |
| 242 | hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-374a-5p, hsa-miR-7-5p, hsa-miR-28-3p, hsa-miR-328-3p, hsa-miR-323a-3p, hsa-miR-550a-3-5p, age, hsa-miR-323b-3p, | 76.97 | 83.16 | 84.39 |
| 243 | hsa-miR-326, hsa-miR-130a-3p, hsa-miR-1260b, hsa-miR-4306, hsa-miR-1301-3p, hsa-miR-21-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 76.97 | 84.41 | 80.83 |
| 244 | hsa-miR-326, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-423-5p, hsa-miR-7-5p, hsa-miR-28-3p, hsa-miR-328-3p, hsa-miR-323a-3p, hsa-miR-550a-3-5p, age, hsa-miR-323b-3p, | 76.97 | 84.74 | 81.06 |
| 245 | hsa-miR-142-5p, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-28-3p, hsa-miR-323a-3p, hsa-miR-550a-3-5p, age, hsa-miR-323b-3p, | 76.97 | 84.57 | 82.65 |
| 246 | hsa-miR-326, hsa-miR-142-5p, hsa-miR-181a-5p, hsa-miR-92b-3p, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-28-3p, hsa-miR-328-3p, hsa-miR-323a-3p, hsa-miR-550a-3-5p, age, | 76.97 | 81.69 | 84.17 |
| 247 | hsa-miR-326, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-323a-3p, hsa-miR-550a-3-5p, age, hsa-miR-323b-3p, | 76.95 | 84.4 | 79.92 |
| 248 | hsa-miR-326, hsa-miR-130a-3p, hsa-miR-1260b, hsa-miR-4306, hsa-miR-1301-3p, hsa-miR-21-5p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-550a-3-5p, age, | 76.95 | 85.22 | 82.73 |
| 249 | hsa-miR-30b-5p, hsa-miR-326, hsa-miR-181a-5p, hsa-miR-1260b, hsa-miR-1301-3p, hsa-miR-195-5p, hsa-miR-483-5p, hsa-miR-6803-3p, hsa-miR-181c-3p, hsa-miR-374a-5p, hsa-miR-29a-3p, hsa-miR-7-5p, hsa-miR-28-3p, hsa-miR-15b-3p, hsa-miR-328-3p, hsa-miR-550a-3-5p, hsa-miR-323b-3p, | 76.55 | 84.65 | 80.15 |
| 250 | hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 76.53 | 85.34 | 81.82 |
| 251 | hsa-miR-30b-5p, hsa-miR-142-5p, hsa-miR-1260b, hsa-miR-1301-3p, hsa-miR-21-5p, hsa-miR-195-5p, hsa-miR-6803-3p, hsa-miR-181c-3p, hsa-miR-101-3p, hsa-miR-374a-5p, hsa-miR-29a-3p, hsa-miR-7-5p, hsa-miR-15b-3p, hsa-miR-328-3p, hsa-miR-550a-3-5p, hsa-miR-323b-3p, | 76.53 | 82.56 | 81.59 |
| 252 | hsa-miR-30b-5p, hsa-miR-326, hsa-miR-181a-5p, hsa-miR-92b-3p, hsa-miR-130a-3p, hsa-miR-1260b, hsa-miR-21-5p, hsa-miR-195-5p, hsa-miR-6803-3p, hsa-miR-181c-3p, hsa-miR-101-3p, hsa-miR-423-5p, hsa-miR-29a-3p, hsa-miR-7-5p, hsa-miR-28-3p, hsa-miR-15b-3p, hsa-miR-328-3p, hsa-miR-323a-3p, hsa-miR-550a-3-5p, | 76.53 | 82.83 | 81.97 |
| 253 | hsa-miR-326, hsa-miR-181a-5p, hsa-miR-92b-3p, hsa-miR-1301-3p, hsa-miR-195-5p, hsa-miR-483-5p, hsa-miR-6803-3p, hsa-miR-181c-3p, hsa-miR-101-3p, hsa-miR-28-3p, hsa-miR-15b-3p, hsa-miR-328-3p, hsa-miR-323a-3p, hsa-miR-323b-3p, | 76.53 | 83.43 | 79.32 |
| 254 | hsa-miR-326, hsa-miR-181a-5p, hsa-miR-4306, hsa-miR-1301-3p, hsa-miR-195-5p, hsa-miR-483-5p, hsa-miR-6803-3p, hsa-miR-181c-3p, hsa-miR-101-3p, hsa-miR-28-3p, hsa-miR-15b-3p, hsa-miR-328-3p, hsa-miR-323a-3p, age, | 76.5 | 85.53 | 81.21 |
| 255 | hsa-miR-326, hsa-miR-181a-5p, hsa-miR-92b-3p, hsa-miR-1260b, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, hsa-miR-323b-3p, | 76.5 | 83.58 | 83.56 |
| 256 | hsa-miR-130a-3p, hsa-miR-1260b, hsa-miR-1301-3p, hsa-miR-21-5p, hsa-miR-483-5p, hsa-miR-6803-3p, hsa-miR-101-3p, hsa-miR-374a-5p, hsa-miR-29a-3p, hsa-miR-7-5p, hsa-miR-28-3p, hsa-miR-15b-3p, hsa-miR-328-3p, hsa-miR-323a-3p, hsa-miR-550a-3-5p, hsa-miR-323b-3p, | 76.5 | 82.09 | 81.74 |
| 257 | hsa-miR-181a-5p, hsa-miR-1260b, hsa-miR-4306, hsa-miR-1301-3p, hsa-miR-377-3p, hsa-miR-483-5p, hsa-miR-6803-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 76.5 | 83.47 | 82.8 |
| 258 | hsa-miR-130a-3p, hsa-miR-1260b, hsa-miR-4306, hsa-miR-1301-3p, hsa-miR-377-3p, hsa-miR-483-5p, hsa-miR-6803-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 76.5 | 82.57 | 81.89 |
| 259 | hsa-miR-142-5p, hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-374a-5p, hsa-miR-7-5p, hsa-miR-28-3p, hsa-miR-328-3p, hsa-miR-323a-3p, age, hsa-miR-323b-3p, | 76.47 | 82.33 | 83.56 |
| 260 | hsa-miR-181a-5p, hsa-miR-92b-3p, hsa-miR-4306, hsa-miR-1301-3p, hsa-miR-195-5p, hsa-miR-377-3p, hsa-miR-483-5p, hsa-miR-181c-3p, hsa-miR-101-3p, hsa-miR-374a-5p, hsa-miR-423-5p, hsa-miR-7-5p, hsa-miR-28-3p, hsa-miR-550a-3-5p, age, hsa-miR-323b-3p, | 76.47 | 83.33 | 80.15 |
| 261 | hsa-miR-130a-3p, hsa-miR-4306, hsa-miR-1301-3p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 76.47 | 83.18 | 79.24 |
| 262 | hsa-miR-326, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-15b-3p, hsa-miR-328-3p, hsa-miR-323a-3p, hsa-miR-550a-3-5p, hsa-miR-323b-3p, | 76.47 | 84.77 | 80.91 |
| 263 | hsa-miR-326, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-7-5p, hsa-miR-28-3p, hsa-miR-328-3p, hsa-miR-323a-3p, hsa-miR-550a-3-5p, age, | 76.47 | 83.55 | 80.91 |
| 264 | hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-29a-3p, hsa-miR-7-5p, hsa-miR-28-3p, hsa-miR-328-3p, hsa-miR-323a-3p, age, | 76.47 | 79.71 | 81.82 |
| 265 | hsa-miR-130a-3p, hsa-miR-1260b, hsa-miR-1301-3p, hsa-miR-21-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-323a-3p, hsa-miR-550a-3-5p, hsa-miR-323b-3p, | 76.45 | 79.86 | 79.85 |
| 266 | hsa-miR-30b-5p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-15b-3p, hsa-miR-328-3p, hsa-miR-323a-3p, hsa-miR-550a-3-5p, age, | 76.45 | 84.64 | 80.91 |
| 267 | hsa-miR-326, hsa-miR-181a-5p, hsa-miR-1260b, hsa-miR-1301-3p, hsa-miR-195-5p, hsa-miR-377-3p, hsa-miR-6803-3p, hsa-miR-374a-5p, hsa-miR-29a-3p, hsa-miR-7-5p, hsa-miR-28-3p, hsa-miR-323a-3p, hsa-miR-550a-3-5p, hsa-miR-323b-3p, | 76.45 | 78.29 | 80.83 |
| 268 | hsa-miR-326, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-423-5p, hsa-miR-28-3p, hsa-miR-328-3p, hsa-miR-323a-3p, hsa-miR-550a-3-5p, age, hsa-miR-323b-3p, | 76.42 | 84.16 | 81.97 |
| 269 | hsa-miR-30b-5p, hsa-miR-181a-5p, hsa-miR-1260b, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-15b-3p, hsa-miR-328-3p, hsa-miR-550a-3-5p, | 76.03 | 79.86 | 78.26 |
| 270 | hsa-miR-30b-5p, hsa-miR-181a-5p, hsa-miR-1260b, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-15b-3p, hsa-miR-328-3p, hsa-miR-550a-3-5p, hsa-miR-323b-3p, | 76 | 80.16 | 79.09 |
| 271 | hsa-miR-30b-5p, hsa-miR-326, hsa-miR-142-5p, hsa-miR-181a-5p, hsa-miR-1260b, hsa-miR-1301-3p, hsa-miR-195-5p, hsa-miR-483-5p, hsa-miR-6803-3p, hsa-miR-181c-3p, hsa-miR-101-3p, hsa-miR-29a-3p, hsa-miR-7-5p, hsa-miR-28-3p, hsa-miR-15b-3p, hsa-miR-323a-3p, hsa-miR-550a-3-5p, hsa-miR-323b-3p, | 76 | 83.5 | 79.17 |
| 272 | hsa-miR-130a-3p, hsa-miR-1260b, hsa-miR-4306, hsa-miR-1301-3p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, age, | 75.97 | 84.22 | 78.33 |
| 273 | hsa-miR-142-5p, hsa-miR-181a-5p, hsa-miR-1260b, hsa-miR-4306, hsa-miR-195-5p, hsa-miR-377-3p, hsa-miR-181c-3p, hsa-miR-101-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, age, hsa-miR-323b-3p, | 75.97 | 84.25 | 81.74 |
| 274 | hsa-miR-326, hsa-miR-142-5p, hsa-miR-181a-5p, hsa-miR-1260b, hsa-miR-4306, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 75.97 | 84.16 | 80.08 |
| 275 | hsa-miR-30b-5p, hsa-miR-181a-5p, hsa-miR-1260b, hsa-miR-4306, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 75.97 | 83.38 | 80.08 |
| 276 | hsa-miR-142-5p, hsa-miR-181a-5p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, age, | 75.97 | 83.61 | 81.74 |
| 277 | hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-374a-5p, hsa-miR-7-5p, hsa-miR-28-3p, hsa-miR-328-3p, hsa-miR-323a-3p, age, | 75.97 | 79.33 | 82.58 |
| 278 | hsa-miR-30b-5p, hsa-miR-1260b, hsa-miR-4306, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-15b-3p, hsa-miR-328-3p, hsa-miR-550a-3-5p, age, hsa-miR-323b-3p, | 75.95 | 82.4 | 80.76 |
| 279 | hsa-miR-30b-5p, hsa-miR-130a-3p, hsa-miR-1260b, hsa-miR-4306, hsa-miR-1301-3p, hsa-miR-21-5p, hsa-miR-195-5p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, hsa-miR-550a-3-5p, age, | 75.95 | 81 | 83.33 |
| 280 | hsa-miR-326, hsa-miR-181a-5p, hsa-miR-130a-3p, hsa-miR-4306, hsa-miR-1301-3p, hsa-miR-21-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-323a-3p, age, hsa-miR-323b-3p, | 75.92 | 83.11 | 81.97 |
| 281 | hsa-miR-181a-5p, hsa-miR-130a-3p, hsa-miR-4306, hsa-miR-1301-3p, hsa-miR-483-5p, hsa-miR-6803-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, age, | 75.92 | 80.55 | 81.82 |
| 282 | hsa-miR-181a-5p, hsa-miR-4306, hsa-miR-1301-3p, hsa-miR-195-5p, hsa-miR-377-3p, hsa-miR-483-5p, hsa-miR-6803-3p, hsa-miR-101-3p, hsa-miR-29a-3p, hsa-miR-28-3p, hsa-miR-328-3p, hsa-miR-323a-3p, hsa-miR-550a-3-5p, age, | 75.89 | 81.9 | 80.91 |
| 283 | hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, age, | 75.47 | 84.86 | 80 |
| 284 | hsa-miR-30b-5p, hsa-miR-142-5p, hsa-miR-181a-5p, hsa-miR-1260b, hsa-miR-1301-3p, hsa-miR-195-5p, hsa-miR-6803-3p, hsa-miR-181c-3p, hsa-miR-101-3p, hsa-miR-374a-5p, hsa-miR-29a-3p, hsa-miR-328-3p, hsa-miR-550a-3-5p, hsa-miR-323b-3p, | 75.47 | 79.79 | 77.27 |
| 285 | hsa-miR-142-5p, hsa-miR-181a-5p, hsa-miR-92b-3p, hsa-miR-1260b, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-423-5p, hsa-miR-7-5p, hsa-miR-323a-3p, age, hsa-miR-323b-3p, | 75.47 | 80.85 | 81.82 |
| 286 | hsa-miR-30b-5p, hsa-miR-142-5p, hsa-miR-181a-5p, hsa-miR-1260b, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-15b-3p, hsa-miR-328-3p, hsa-miR-550a-3-5p, | 75.47 | 79.28 | 78.18 |
| 287 | hsa-miR-326, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-15b-3p, hsa-miR-328-3p, hsa-miR-323a-3p, hsa-miR-550a-3-5p, age, hsa-miR-323b-3p, | 75.45 | 86.11 | 79.24 |
| 288 | hsa-miR-181a-5p, hsa-miR-92b-3p, hsa-miR-1260b, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-28-3p, hsa-miR-328-3p, hsa-miR-323a-3p, hsa-miR-550a-3-5p, hsa-miR-323b-3p, | 75.45 | 81.38 | 81.89 |
| 289 | hsa-miR-326, hsa-miR-181a-5p, hsa-miR-1260b, hsa-miR-1301-3p, hsa-miR-21-5p, hsa-miR-195-5p, hsa-miR-374a-5p, hsa-miR-423-5p, hsa-miR-29a-3p, hsa-miR-7-5p, hsa-miR-15b-3p, hsa-miR-328-3p, hsa-miR-323a-3p, hsa-miR-550a-3-5p, hsa-miR-323b-3p, | 75.45 | 82.33 | 80 |
| 290 | hsa-miR-30b-5p, hsa-miR-4306, hsa-miR-1301-3p, hsa-miR-21-5p, hsa-miR-195-5p, hsa-miR-377-3p, hsa-miR-483-5p, hsa-miR-181c-3p, hsa-miR-101-3p, hsa-miR-423-5p, hsa-miR-29a-3p, hsa-miR-7-5p, hsa-miR-15b-3p, hsa-miR-550a-3-5p, hsa-miR-323b-3p, | 75.45 | 82.6 | 80 |
| 291 | hsa-miR-326, hsa-miR-1260b, hsa-miR-4306, hsa-miR-195-5p, hsa-miR-377-3p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-328-3p, hsa-miR-323a-3p, hsa-miR-550a-3-5p, age, | 75.42 | 84.63 | 79.17 |
| 292 | hsa-miR-326, hsa-miR-181a-5p, hsa-miR-92b-3p, hsa-miR-1260b, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-29a-3p, hsa-miR-28-3p, hsa-miR-328-3p, hsa-miR-323a-3p, hsa-miR-550a-3-5p, hsa-miR-323b-3p, | 75.42 | 83.22 | 79.17 |
| 293 | hsa-miR-130a-3p, hsa-miR-1260b, hsa-miR-4306, hsa-miR-1301-3p, hsa-miR-21-5p, hsa-miR-483-5p, hsa-miR-101-3p, hsa-miR-423-5p, hsa-miR-7-5p, hsa-miR-28-3p, hsa-miR-550a-3-5p, hsa-miR-323b-3p, | 75.42 | 80.4 | 79.24 |
| 294 | hsa-miR-130a-3p, hsa-miR-1260b, hsa-miR-1301-3p, hsa-miR-21-5p, hsa-miR-377-3p, hsa-miR-6803-3p, hsa-miR-374a-5p, hsa-miR-423-5p, hsa-miR-29a-3p, hsa-miR-7-5p, hsa-miR-15b-3p, hsa-miR-328-3p, hsa-miR-323a-3p, hsa-miR-550a-3-5p, hsa-miR-323b-3p, | 75.42 | 81.62 | 79.77 |
| 295 | hsa-miR-326, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-28-3p, hsa-miR-15b-3p, hsa-miR-328-3p, hsa-miR-323a-3p, hsa-miR-323b-3p, | 75.42 | 82.24 | 78.33 |
| 296 | hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, age, hsa-miR-323b-3p, | 75.39 | 83.93 | 77.27 |
| 297 | hsa-miR-181a-5p, hsa-miR-4306, hsa-miR-195-5p, hsa-miR-377-3p, hsa-miR-483-5p, hsa-miR-181c-3p, hsa-miR-101-3p, hsa-miR-374a-5p, hsa-miR-423-5p, hsa-miR-7-5p, hsa-miR-28-3p, hsa-miR-550a-3-5p, age, hsa-miR-323b-3p, | 75.39 | 82.98 | 81.06 |
| 298 | hsa-miR-326, hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-423-5p, hsa-miR-7-5p, hsa-miR-28-3p, hsa-miR-328-3p, hsa-miR-323a-3p, hsa-miR-550a-3-5p, age, hsa-miR-323b-3p, | 75.39 | 82.11 | 81.21 |
| 299 | hsa-miR-30b-5p, hsa-miR-326, hsa-miR-1260b, hsa-miR-1301-3p, hsa-miR-195-5p, hsa-miR-377-3p, hsa-miR-6803-3p, hsa-miR-181c-3p, hsa-miR-374a-5p, hsa-miR-423-5p, hsa-miR-29a-3p, hsa-miR-15b-3p, hsa-miR-328-3p, hsa-miR-323a-3p, hsa-miR-550a-3-5p, hsa-miR-323b-3p, | 74.97 | 81.08 | 81.74 |
| 300 | hsa-miR-326, hsa-miR-181a-5p, hsa-miR-1260b, hsa-miR-195-5p, hsa-miR-483-5p, hsa-miR-181c-3p, hsa-miR-101-3p, hsa-miR-29a-3p, hsa-miR-7-5p, hsa-miR-28-3p, hsa-miR-328-3p, hsa-miR-550a-3-5p, hsa-miR-323b-3p, | 74.97 | 80.05 | 80.98 |
| 301 | hsa-miR-326, hsa-miR-181a-5p, hsa-miR-1260b, hsa-miR-1301-3p, hsa-miR-195-5p, hsa-miR-483-5p, hsa-miR-6803-3p, hsa-miR-181c-3p, hsa-miR-374a-5p, hsa-miR-29a-3p, hsa-miR-7-5p, hsa-miR-28-3p, hsa-miR-15b-3p, hsa-miR-328-3p, hsa-miR-550a-3-5p, hsa-miR-323b-3p, | 74.97 | 81.39 | 80.15 |
| 302 | hsa-miR-326, hsa-miR-181a-5p, hsa-miR-1260b, hsa-miR-1301-3p, hsa-miR-195-5p, hsa-miR-6803-3p, hsa-miR-181c-3p, hsa-miR-374a-5p, hsa-miR-29a-3p, hsa-miR-7-5p, hsa-miR-28-3p, hsa-miR-328-3p, hsa-miR-323a-3p, hsa-miR-550a-3-5p, hsa-miR-323b-3p, | 74.97 | 81.58 | 79.17 |
| 303 | hsa-miR-326, hsa-miR-130a-3p, hsa-miR-1260b, hsa-miR-4306, hsa-miR-1301-3p, hsa-miR-195-5p, hsa-miR-28-3p, hsa-miR-15b-3p, hsa-miR-328-3p, age, hsa-miR-323b-3p, | 74.95 | 81.24 | 81.21 |
| 304 | hsa-miR-30b-5p, hsa-miR-181a-5p, hsa-miR-1260b, hsa-miR-4306, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-7-5p, hsa-miR-15b-3p, hsa-miR-328-3p, hsa-miR-550a-3-5p, age, hsa-miR-323b-3p, | 74.95 | 84.63 | 81.06 |
| 305 | hsa-miR-326, hsa-miR-181a-5p, hsa-miR-4306, hsa-miR-1301-3p, hsa-miR-21-5p, hsa-miR-195-5p, hsa-miR-483-5p, hsa-miR-6803-3p, hsa-miR-181c-3p, hsa-miR-101-3p, hsa-miR-7-5p, hsa-miR-28-3p, hsa-miR-328-3p, hsa-miR-323a-3p, hsa-miR-550a-3-5p, age, | 74.92 | 84.45 | 77.58 |
| 306 | hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-7-5p, hsa-miR-28-3p, hsa-miR-328-3p, hsa-miR-323a-3p, hsa-miR-550a-3-5p, age, | 74.92 | 83.27 | 80.08 |
| 307 | hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-7-5p, hsa-miR-28-3p, hsa-miR-328-3p, hsa-miR-323a-3p, hsa-miR-550a-3-5p, age, | 74.92 | 83.38 | 80.08 |
| 308 | hsa-miR-30b-5p, hsa-miR-326, hsa-miR-1260b, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-15b-3p, hsa-miR-328-3p, hsa-miR-550a-3-5p, | 74.92 | 84.13 | 81.59 |
| 309 | hsa-miR-30b-5p, hsa-miR-142-5p, hsa-miR-181a-5p, hsa-miR-1260b, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-328-3p, hsa-miR-550a-3-5p, hsa-miR-323b-3p, | 74.92 | 79.02 | 78.18 |
| 310 | hsa-miR-130a-3p, hsa-miR-1260b, hsa-miR-4306, hsa-miR-1301-3p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-550a-3-5p, age, | 74.92 | 84.55 | 80.15 |
| 311 | hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-7-5p, hsa-miR-28-3p, hsa-miR-323a-3p, hsa-miR-550a-3-5p, age, | 74.89 | 82.61 | 80 |
| 312 | hsa-miR-30b-5p, hsa-miR-181a-5p, hsa-miR-1260b, hsa-miR-4306, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-328-3p, hsa-miR-550a-3-5p, age, hsa-miR-323b-3p, | 74.89 | 81.47 | 79.02 |
| 313 | hsa-miR-326, hsa-miR-130a-3p, hsa-miR-1260b, hsa-miR-4306, hsa-miR-1301-3p, hsa-miR-21-5p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-328-3p, hsa-miR-550a-3-5p, | 74.89 | 82.58 | 79.92 |
| 314 | hsa-miR-30b-5p, hsa-miR-130a-3p, hsa-miR-1260b, hsa-miR-4306, hsa-miR-1301-3p, hsa-miR-195-5p, hsa-miR-6803-3p, hsa-miR-181c-3p, hsa-miR-101-3p, hsa-miR-374a-5p, hsa-miR-29a-3p, hsa-miR-328-3p, hsa-miR-550a-3-5p, age, hsa-miR-323b-3p, | 74.87 | 80.22 | 78.11 |
| 315 | hsa-miR-30b-5p, hsa-miR-1260b, hsa-miR-4306, hsa-miR-1301-3p, hsa-miR-195-5p, hsa-miR-6803-3p, hsa-miR-181c-3p, hsa-miR-101-3p, hsa-miR-374a-5p, hsa-miR-29a-3p, hsa-miR-328-3p, hsa-miR-550a-3-5p, age, hsa-miR-323b-3p, | 74.87 | 81.69 | 78.11 |
| 316 | hsa-miR-326, hsa-miR-142-5p, hsa-miR-181a-5p, hsa-miR-1260b, hsa-miR-1301-3p, hsa-miR-21-5p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-15b-3p, hsa-miR-328-3p, hsa-miR-550a-3-5p, hsa-miR-323b-3p, | 74.45 | 79.57 | 80 |
| 317 | hsa-miR-30b-5p, hsa-miR-142-5p, hsa-miR-181a-5p, hsa-miR-1260b, hsa-miR-1301-3p, hsa-miR-21-5p, hsa-miR-195-5p, hsa-miR-6803-3p, hsa-miR-181c-3p, hsa-miR-101-3p, hsa-miR-374a-5p, hsa-miR-29a-3p, hsa-miR-7-5p, hsa-miR-15b-3p, hsa-miR-328-3p, hsa-miR-323b-3p, | 74.42 | 80.83 | 79.09 |
| 318 | hsa-miR-30b-5p, hsa-miR-326, hsa-miR-181a-5p, hsa-miR-1260b, hsa-miR-1301-3p, hsa-miR-195-5p, hsa-miR-377-3p, hsa-miR-6803-3p, hsa-miR-181c-3p, hsa-miR-101-3p, hsa-miR-423-5p, hsa-miR-29a-3p, hsa-miR-7-5p, hsa-miR-328-3p, hsa-miR-550a-3-5p, | 74.42 | 79.78 | 79.92 |
| 319 | hsa-miR-30b-5p, hsa-miR-142-5p, hsa-miR-181a-5p, hsa-miR-1260b, hsa-miR-1301-3p, hsa-miR-21-5p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, hsa-miR-550a-3-5p, | 74.42 | 82.89 | 77.27 |
| 320 | hsa-miR-30b-5p, hsa-miR-181a-5p, hsa-miR-1260b, hsa-miR-4306, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-28-3p, hsa-miR-328-3p, hsa-miR-323a-3p, age, | 74.42 | 81.51 | 79.47 |
| 321 | hsa-miR-30b-5p, hsa-miR-142-5p, hsa-miR-181a-5p, hsa-miR-1260b, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-15b-3p, hsa-miR-328-3p, hsa-miR-323a-3p, hsa-miR-550a-3-5p, hsa-miR-323b-3p, | 74.42 | 82.19 | 78.33 |
| 322 | hsa-miR-326, hsa-miR-181a-5p, hsa-miR-1260b, hsa-miR-1301-3p, hsa-miR-21-5p, hsa-miR-195-5p, hsa-miR-377-3p, hsa-miR-483-5p, hsa-miR-181c-3p, hsa-miR-101-3p, hsa-miR-374a-5p, hsa-miR-29a-3p, hsa-miR-7-5p, hsa-miR-28-3p, hsa-miR-323a-3p, hsa-miR-550a-3-5p, | 74.42 | 79.07 | 80 |
| 323 | hsa-miR-30b-5p, hsa-miR-326, hsa-miR-1260b, hsa-miR-1301-3p, hsa-miR-21-5p, hsa-miR-195-5p, hsa-miR-377-3p, hsa-miR-6803-3p, hsa-miR-181c-3p, hsa-miR-374a-5p, hsa-miR-423-5p, hsa-miR-29a-3p, hsa-miR-7-5p, hsa-miR-15b-3p, hsa-miR-328-3p, hsa-miR-550a-3-5p, hsa-miR-323b-3p, | 74.42 | 82.46 | 80.68 |
| 324 | hsa-miR-142-5p, hsa-miR-181a-5p, hsa-miR-1260b, hsa-miR-4306, hsa-miR-1301-3p, hsa-miR-21-5p, hsa-miR-195-5p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-550a-3-5p, | 74.42 | 82.34 | 79.17 |
| 325 | hsa-miR-142-5p, hsa-miR-92b-3p, hsa-miR-1260b, hsa-miR-1301-3p, hsa-miR-377-3p, hsa-miR-483-5p, hsa-miR-181c-3p, hsa-miR-101-3p, hsa-miR-29a-3p, hsa-miR-7-5p, hsa-miR-328-3p, hsa-miR-550a-3-5p, age, | 74.39 | 81.12 | 79.09 |
| 326 | hsa-miR-30b-5p, hsa-miR-326, hsa-miR-1260b, hsa-miR-1301-3p, hsa-miR-195-5p, hsa-miR-6803-3p, hsa-miR-181c-3p, hsa-miR-374a-5p, hsa-miR-423-5p, hsa-miR-29a-3p, hsa-miR-15b-3p, hsa-miR-328-3p, hsa-miR-550a-3-5p, hsa-miR-323b-3p, | 74.39 | 81.78 | 81.67 |
| 327 | hsa-miR-326, hsa-miR-142-5p, hsa-miR-181a-5p, hsa-miR-1260b, hsa-miR-195-5p, hsa-miR-377-3p, hsa-miR-483-5p, hsa-miR-6803-3p, hsa-miR-181c-3p, hsa-miR-374a-5p, hsa-miR-423-5p, hsa-miR-29a-3p, hsa-miR-7-5p, hsa-miR-28-3p, hsa-miR-15b-3p, hsa-miR-328-3p, hsa-miR-323a-3p, hsa-miR-323b-3p, | 74.37 | 83.35 | 79.92 |
| 328 | hsa-miR-30b-5p, hsa-miR-326, hsa-miR-142-5p, hsa-miR-181a-5p, hsa-miR-1260b, hsa-miR-1301-3p, hsa-miR-21-5p, hsa-miR-195-5p, hsa-miR-377-3p, hsa-miR-6803-3p, hsa-miR-181c-3p, hsa-miR-374a-5p, hsa-miR-29a-3p, hsa-miR-7-5p, hsa-miR-15b-3p, hsa-miR-328-3p, hsa-miR-550a-3-5p, hsa-miR-323b-3p, | 73.89 | 80.88 | 78.18 |
| 329 | hsa-miR-30b-5p, hsa-miR-326, hsa-miR-142-5p, hsa-miR-92b-3p, hsa-miR-1301-3p, hsa-miR-21-5p, hsa-miR-377-3p, hsa-miR-483-5p, hsa-miR-6803-3p, hsa-miR-181c-3p, hsa-miR-101-3p, hsa-miR-423-5p, hsa-miR-7-5p, hsa-miR-328-3p, hsa-miR-323a-3p, hsa-miR-323b-3p, | 73.87 | 81.85 | 80.08 |
| 330 | hsa-miR-326, hsa-miR-181a-5p, hsa-miR-92b-3p, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-28-3p, hsa-miR-328-3p, hsa-miR-323a-3p, hsa-miR-323b-3p, | 73.87 | 80.9 | 80.08 |
| 331 | hsa-miR-30b-5p, hsa-miR-142-5p, hsa-miR-92b-3p, hsa-miR-195-5p, hsa-miR-483-5p, hsa-miR-28-3p, hsa-miR-15b-3p, hsa-miR-328-3p, hsa-miR-323a-3p, hsa-miR-550a-3-5p, age, hsa-miR-323b-3p, | 73.87 | 80.45 | 80.15 |
| 332 | hsa-miR-130a-3p, hsa-miR-1260b, hsa-miR-4306, hsa-miR-1301-3p, hsa-miR-21-5p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-28-3p, hsa-miR-328-3p, hsa-miR-550a-3-5p, age, | 73.84 | 84.07 | 77.5 |
| 333 | hsa-miR-30b-5p, hsa-miR-181a-5p, hsa-miR-92b-3p, hsa-miR-1260b, hsa-miR-4306, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, hsa-miR-323a-3p, age, hsa-miR-323b-3p, | 73.84 | 82.21 | 77.42 |
| 334 | hsa-miR-326, hsa-miR-181a-5p, hsa-miR-130a-3p, hsa-miR-4306, hsa-miR-1301-3p, hsa-miR-21-5p, hsa-miR-483-5p, hsa-miR-6803-3p, hsa-miR-423-5p, hsa-miR-29a-3p, hsa-miR-28-3p, hsa-miR-328-3p, hsa-miR-323a-3p, hsa-miR-550a-3-5p, age, | 73.82 | 80.7 | 79.24 |
| 335 | hsa-miR-30b-5p, hsa-miR-1260b, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-7-5p, hsa-miR-15b-3p, hsa-miR-328-3p, hsa-miR-550a-3-5p, | 73.42 | 78.81 | 78.26 |
| 336 | hsa-miR-30b-5p, hsa-miR-326, hsa-miR-181a-5p, hsa-miR-1260b, hsa-miR-1301-3p, hsa-miR-21-5p, hsa-miR-195-5p, hsa-miR-6803-3p, hsa-miR-181c-3p, hsa-miR-374a-5p, hsa-miR-423-5p, hsa-miR-29a-3p, hsa-miR-28-3p, hsa-miR-328-3p, hsa-miR-550a-3-5p, hsa-miR-323b-3p, | 73.39 | 83.36 | 76.52 |
| 337 | hsa-miR-30b-5p, hsa-miR-142-5p, hsa-miR-181a-5p, hsa-miR-1260b, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, hsa-miR-550a-3-5p, hsa-miR-323b-3p, | 73.37 | 78.46 | 76.29 |
| 338 | hsa-miR-142-5p, hsa-miR-181a-5p, hsa-miR-130a-3p, hsa-miR-1260b, hsa-miR-1301-3p, hsa-miR-195-5p, hsa-miR-6803-3p, hsa-miR-181c-3p, hsa-miR-101-3p, hsa-miR-374a-5p, hsa-miR-29a-3p, hsa-miR-328-3p, hsa-miR-550a-3-5p, hsa-miR-323b-3p, | 73.37 | 78.99 | 78.26 |
| 339 | hsa-miR-130a-3p, hsa-miR-1260b, hsa-miR-1301-3p, hsa-miR-21-5p, hsa-miR-195-5p, hsa-miR-377-3p, hsa-miR-6803-3p, hsa-miR-181c-3p, hsa-miR-374a-5p, hsa-miR-29a-3p, hsa-miR-328-3p, hsa-miR-323a-3p, hsa-miR-550a-3-5p, age, | 73.37 | 81.54 | 76.36 |
| 340 | hsa-miR-30b-5p, hsa-miR-142-5p, hsa-miR-181a-5p, hsa-miR-130a-3p, hsa-miR-1260b, hsa-miR-1301-3p, hsa-miR-6803-3p, hsa-miR-181c-3p, hsa-miR-101-3p, hsa-miR-374a-5p, hsa-miR-29a-3p, hsa-miR-15b-3p, hsa-miR-328-3p, hsa-miR-323b-3p, | 73.34 | 80.92 | 78.11 |
| 341 | hsa-miR-30b-5p, hsa-miR-1260b, hsa-miR-1301-3p, hsa-miR-195-5p, hsa-miR-377-3p, hsa-miR-6803-3p, hsa-miR-181c-3p, hsa-miR-101-3p, hsa-miR-374a-5p, hsa-miR-29a-3p, hsa-miR-7-5p, hsa-miR-15b-3p, hsa-miR-328-3p, hsa-miR-550a-3-5p, hsa-miR-323b-3p, | 73.34 | 79.57 | 76.52 |
| 342 | hsa-miR-30b-5p, hsa-miR-142-5p, hsa-miR-181a-5p, hsa-miR-1260b, hsa-miR-4306, hsa-miR-1301-3p, hsa-miR-195-5p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, age, | 73.32 | 80.36 | 78.11 |
| 343 | hsa-miR-30b-5p, hsa-miR-181a-5p, hsa-miR-4306, hsa-miR-21-5p, hsa-miR-483-5p, hsa-miR-181c-3p, hsa-miR-101-3p, hsa-miR-7-5p, hsa-miR-28-3p, hsa-miR-328-3p, hsa-miR-323a-3p, hsa-miR-550a-3-5p, age, | 73.32 | 81.65 | 78.41 |
| 344 | hsa-miR-30b-5p, hsa-miR-181a-5p, hsa-miR-130a-3p, hsa-miR-1260b, hsa-miR-1301-3p, hsa-miR-6803-3p, hsa-miR-181c-3p, hsa-miR-101-3p, hsa-miR-374a-5p, hsa-miR-29a-3p, hsa-miR-328-3p, hsa-miR-550a-3-5p, hsa-miR-323b-3p, | 73.32 | 81.51 | 77.95 |
| 345 | hsa-miR-181a-5p, hsa-miR-130a-3p, hsa-miR-4306, hsa-miR-1301-3p, hsa-miR-377-3p, hsa-miR-483-5p, hsa-miR-6803-3p, hsa-miR-101-3p, hsa-miR-29a-3p, hsa-miR-28-3p, hsa-miR-328-3p, hsa-miR-323a-3p, hsa-miR-550a-3-5p, age, | 73.29 | 81.76 | 80.91 |
| 346 | hsa-miR-130a-3p, hsa-miR-1260b, hsa-miR-1301-3p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, hsa-miR-550a-3-5p, hsa-miR-323b-3p, | 72.87 | 77.4 | 79.02 |
| 347 | hsa-miR-30b-5p, hsa-miR-326, hsa-miR-181a-5p, hsa-miR-1260b, hsa-miR-1301-3p, hsa-miR-21-5p, hsa-miR-377-3p, hsa-miR-6803-3p, hsa-miR-181c-3p, hsa-miR-374a-5p, hsa-miR-423-5p, hsa-miR-29a-3p, hsa-miR-15b-3p, hsa-miR-328-3p, hsa-miR-550a-3-5p, hsa-miR-323b-3p, | 72.87 | 80.77 | 79.09 |
| 348 | hsa-miR-30b-5p, hsa-miR-326, hsa-miR-181a-5p, hsa-miR-1260b, hsa-miR-1301-3p, hsa-miR-21-5p, hsa-miR-195-5p, hsa-miR-377-3p, hsa-miR-6803-3p, hsa-miR-181c-3p, hsa-miR-374a-5p, hsa-miR-423-5p, hsa-miR-29a-3p, hsa-miR-328-3p, hsa-miR-550a-3-5p, hsa-miR-323b-3p, | 72.84 | 80.24 | 78.11 |
| 349 | hsa-miR-30b-5p, hsa-miR-181a-5p, hsa-miR-1260b, hsa-miR-4306, hsa-miR-1301-3p, hsa-miR-195-5p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, hsa-miR-550a-3-5p, age, | 72.84 | 81.71 | 77.35 |
| 350 | hsa-miR-181a-5p, hsa-miR-92b-3p, hsa-miR-1260b, hsa-miR-4306, hsa-miR-1301-3p, hsa-miR-21-5p, hsa-miR-195-5p, hsa-miR-377-3p, hsa-miR-483-5p, hsa-miR-6803-3p, hsa-miR-101-3p, hsa-miR-7-5p, hsa-miR-28-3p, hsa-miR-328-3p, hsa-miR-323a-3p, hsa-miR-550a-3-5p, age, | 72.82 | 82.27 | 78.33 |
| 351 | hsa-miR-326, hsa-miR-181a-5p, hsa-miR-92b-3p, hsa-miR-4306, hsa-miR-1301-3p, hsa-miR-21-5p, hsa-miR-195-5p, hsa-miR-483-5p, hsa-miR-6803-3p, hsa-miR-181c-3p, hsa-miR-28-3p, hsa-miR-328-3p, hsa-miR-550a-3-5p, age, | 72.82 | 83.55 | 75.91 |
| 352 | hsa-miR-30b-5p, hsa-miR-326, hsa-miR-181a-5p, hsa-miR-1260b, hsa-miR-1301-3p, hsa-miR-21-5p, hsa-miR-195-5p, hsa-miR-6803-3p, hsa-miR-181c-3p, hsa-miR-374a-5p, hsa-miR-423-5p, hsa-miR-29a-3p, hsa-miR-328-3p, hsa-miR-550a-3-5p, age, hsa-miR-323b-3p, | 72.82 | 81.54 | 78.18 |
| 353 | hsa-miR-130a-3p, hsa-miR-1260b, hsa-miR-4306, hsa-miR-1301-3p, hsa-miR-21-5p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, hsa-miR-550a-3-5p, age, | 72.79 | 82.57 | 77.35 |
| 354 | hsa-miR-30b-5p, hsa-miR-130a-3p, hsa-miR-1260b, hsa-miR-4306, hsa-miR-1301-3p, hsa-miR-195-5p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, hsa-miR-550a-3-5p, age, hsa-miR-323b-3p, | 72.76 | 81.94 | 76.44 |
| 355 | hsa-miR-30b-5p, hsa-miR-326, hsa-miR-1260b, hsa-miR-1301-3p, hsa-miR-21-5p, hsa-miR-195-5p, hsa-miR-377-3p, hsa-miR-6803-3p, hsa-miR-181c-3p, hsa-miR-374a-5p, hsa-miR-423-5p, hsa-miR-29a-3p, hsa-miR-7-5p, hsa-miR-328-3p, hsa-miR-550a-3-5p, age, hsa-miR-323b-3p, | 72.32 | 81.14 | 79.02 |
| 356 | hsa-miR-142-5p, hsa-miR-181a-5p, hsa-miR-92b-3p, hsa-miR-130a-3p, hsa-miR-1260b, hsa-miR-4306, hsa-miR-1301-3p, hsa-miR-21-5p, hsa-miR-377-3p, hsa-miR-423-5p, hsa-miR-29a-3p, hsa-miR-15b-3p, hsa-miR-550a-3-5p, hsa-miR-323b-3p, | 72.29 | 79.72 | 78.11 |
| 357 | hsa-miR-130a-3p, hsa-miR-1260b, hsa-miR-4306, hsa-miR-1301-3p, hsa-miR-21-5p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, hsa-miR-550a-3-5p, age, | 72.26 | 80.78 | 77.2 |
| 358 | hsa-miR-142-5p, hsa-miR-130a-3p, hsa-miR-1260b, hsa-miR-4306, hsa-miR-1301-3p, hsa-miR-377-3p, hsa-miR-483-5p, hsa-miR-6803-3p, hsa-miR-181c-3p, hsa-miR-101-3p, hsa-miR-374a-5p, hsa-miR-29a-3p, hsa-miR-7-5p, hsa-miR-328-3p, age, hsa-miR-323b-3p, | 72.26 | 80.69 | 77.2 |
| 359 | hsa-miR-326, hsa-miR-181a-5p, hsa-miR-130a-3p, hsa-miR-1260b, hsa-miR-4306, hsa-miR-21-5p, hsa-miR-377-3p, hsa-miR-6803-3p, hsa-miR-374a-5p, hsa-miR-29a-3p, hsa-miR-7-5p, hsa-miR-328-3p, hsa-miR-323a-3p, | 71.82 | 76.62 | 77.27 |
| 360 | hsa-miR-30b-5p, hsa-miR-181a-5p, hsa-miR-1260b, hsa-miR-4306, hsa-miR-1301-3p, hsa-miR-21-5p, hsa-miR-195-5p, hsa-miR-377-3p, hsa-miR-6803-3p, hsa-miR-181c-3p, hsa-miR-101-3p, hsa-miR-29a-3p, hsa-miR-7-5p, hsa-miR-328-3p, hsa-miR-550a-3-5p, age, | 71.79 | 80.96 | 77.35 |
| 361 | hsa-miR-30b-5p, hsa-miR-142-5p, hsa-miR-1260b, hsa-miR-377-3p, hsa-miR-6803-3p, hsa-miR-181c-3p, hsa-miR-423-5p, hsa-miR-7-5p, hsa-miR-15b-3p, age, | 71.76 | 79.54 | 78.11 |
| 362 | hsa-miR-30b-5p, hsa-miR-181a-5p, hsa-miR-130a-3p, hsa-miR-21-5p, hsa-miR-29a-3p, hsa-miR-7-5p, hsa-miR-28-3p, hsa-miR-15b-3p, hsa-miR-323a-3p, hsa-miR-550a-3-5p, age, | 71.76 | 81.31 | 79.17 |
| 363 | hsa-miR-30b-5p, hsa-miR-181a-5p, hsa-miR-92b-3p, hsa-miR-4306, hsa-miR-21-5p, hsa-miR-195-5p, hsa-miR-374a-5p, hsa-miR-550a-3-5p, age, | 71.18 | 77.92 | 74.77 |
| 364 | hsa-miR-30b-5p, hsa-miR-1260b, hsa-miR-4306, hsa-miR-483-5p, hsa-miR-181c-3p, hsa-miR-374a-5p, hsa-miR-423-5p, hsa-miR-29a-3p, hsa-miR-7-5p, hsa-miR-323a-3p, age, | 70.76 | 76.96 | 78.33 |
| 365 | hsa-miR-130a-3p, hsa-miR-4306, hsa-miR-195-5p, hsa-miR-377-3p, hsa-miR-483-5p, hsa-miR-6803-3p, hsa-miR-423-5p, hsa-miR-7-5p, hsa-miR-323a-3p, hsa-miR-323b-3p, | 70.71 | 76.37 | 80.91 |
| 366 | hsa-miR-130a-3p, hsa-miR-1260b, hsa-miR-1301-3p, hsa-miR-483-5p, hsa-miR-6803-3p, hsa-miR-181c-3p, hsa-miR-101-3p, hsa-miR-374a-5p, hsa-miR-29a-3p, hsa-miR-328-3p, hsa-miR-550a-3-5p, hsa-miR-323b-3p, | 70.21 | 77.87 | 76.36 |
| 367 | hsa-miR-130a-3p, hsa-miR-1260b, hsa-miR-4306, hsa-miR-1301-3p, hsa-miR-6803-3p, hsa-miR-181c-3p, hsa-miR-101-3p, hsa-miR-374a-5p, hsa-miR-29a-3p, hsa-miR-328-3p, hsa-miR-550a-3-5p, age, hsa-miR-323b-3p, | 70.16 | 80.7 | 74.77 |
| 368 | hsa-miR-30b-5p, hsa-miR-326, hsa-miR-1260b, hsa-miR-1301-3p, hsa-miR-21-5p, hsa-miR-377-3p, hsa-miR-6803-3p, hsa-miR-181c-3p, hsa-miR-374a-5p, hsa-miR-423-5p, hsa-miR-29a-3p, hsa-miR-7-5p, hsa-miR-328-3p, hsa-miR-323b-3p, | 66.55 | 76.16 | 76.36 |
| 369 | combination 1 + rs11862974 | 67.11 | 64.36 | 87.8 |
| 370 | combination 1 + rs7193058 | 74.89 | 79.03 | 86.97 |
| 371 | combination 2 + rs12917707 | 69.63 | 70.47 | 70.15 |
| 372 | combination 2 + rs11647727 | 68.58 | 71.76 | 83.56 |
| 373 | combination 3 + rs7204342 | 69.13 | 73.18 | 83.48 |
| 374 | combination 3 + rs7193058 | 69.11 | 72.14 | 83.23 |
| 375 | combination 4 + rs12917707 | 73.84 | 77.37 | 80.3 |
| 376 | combination 4 + rs11647727 | 72.79 | 77.42 | 83.4 |
| 377 | combination 8 + rs76563024 | 74.92 | 81.29 | 83.56 |
| 378 | combination 8 + rs7193058 | 74.89 | 79.03 | 80.7 |
| 379 | combination 29 + rs76563024 | 80.18 | 87.08 | 87.08 |
| 380 | combination 149 +rs76563024 | 78.57 | 85.51 | 84.51 |
| 381 | combination 150 + rs76563024 | 77.3 | 85.51 | 86.71 |
| 382 | combination 151 + rs76563024 | 75.3 | 85.01 | 84.17 |
| 383 | combination 152 + rs76563024 | 75.3 | 84.7 | 84.20 |
| 384 | combination 153 + rs76563024 | 74.4 | 83.51 | 85.17 |
| 385 | combination 154 + rs76563024 | 74.3 | 82.5 | 86.18 |
| 386 | combination 155+ rs76563024 | 74 | 82 | 84.20 |

This is the list of the best 30 mRNAs selected by the algorithm sorted by importance for diagnosis outcome: hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548l, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-485-3p, hsa-miR-335-5p, hsa-miR-1290, hsa-miR-132-3p, and hsa-miR-337-5p. The inventors combined these miRNAs, together or not with age and UMOD SNP variant, creating unique combinations leading to a further significant increase in sensitivity, specificity, accuracy and predictive power for diagnosis (Table 16).

**Table 16. Combinations that allow higher sensitivity, specificity, accuracy and predictive power for diagnosis.**

| N | Combinations | Accuracy | ROC AUC | Recall |
|---|---|---|---|---|
| 389 | hsa-miR-99a-3p, hsa-miR-1180-3p, hsa-miR-192-5p | 78.26 | 75.95 | 100.0 |
| 390 | hsa-miR-99a-3p, hsa-miR-1180-3p, hsa-miR-192-5p, hsa-miR-4286 | 79.13 | 77.85 | 100.0 |
| 391 | hsa-miR-99a-3p, hsa-miR-1180-3p, hsa-miR-192-5p, hsa-miR-4286, hsa-miR-103a-2-5p | 79.13 | 82.04 | 100.0 |
| 392 | hsa-miR-99a-3p, hsa-miR-1180-3p, hsa-miR-192-5p, hsa-miR-4286, hsa-miR-103a-2-5p, hsa-miR-215-5p | 80 | 82.18 | 95.56 |
| 393 | hsa-miR-99a-3p, hsa-miR-1180-3p, hsa-miR-192-5p, hsa-miR-4286, hsa-miR-103a-2-5p, hsa-miR-215-5p, hsa-miR-590-3p | 80.87 | 82.62 | 94.44 |
| 394 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p | 82.1 | 84.26 | 93.33 |
| 395 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p | 84.5 | 81.67 | 92.22 |
| 396 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p | 83.8 | 80.16 | 93.33 |
| 397 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p | 84.5 | 89.17 | 94.44 |
| 398 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p | 84.5 | 90.42 | 94.44 |
| 399 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I | 83.8 | 91.45 | 93.33 |
| 400 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p | 86.09 | 90.52 | 94.44 |
| 401 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p | 87.83 | 90.38 | 94.44 |
| 402 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p | 87.83 | 91.71 | 93.33 |
| 403 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p | 86.09 | 90.82 | 94.44 |
| 404 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p | 86.96 | 92.99 | 95.56 |
| 405 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p | 89.7 | 95.25 | 92.22 |
| 406 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p, hsa-miR-331-5p | 88.7 | 92.32 | 93.33 |
| 407 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p | 90.3 | 95.96 | 93.33 |
| 408 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a | 89.7 | 95.25 | 93.33 |
| 409 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a, hsa-miR-491-5p | 90.3 | 95.29 | 90.0 |
| 410 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a, hsa-miR-491-5p, hsa-miR-501-5p | 89.7 | 95.29 | 90.0 |
| 411 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p | 90.3 | 93.33 | 90.0 |
| 412 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-485-3p | 90.3 | 92.85 | 91.11 |
| 413 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-485-3p, hsa-miR-335-5p | 88.7 | 93.78 | 88.89 |
| 414 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-485-3p, hsa-miR-335-5p, hsa-miR-1290 | 88.7 | 93.17 | 90.0 |
| 415 | hsa-miR-99a-3p, hsa-miR-1180-3p, hsa-miR-192-5p, hsa-miR-4286, hsa-miR-103a-2-5p, hsa-miR-215-5p, hsa-miR-590-3p, hsa-miR-500a-5p, hsa-miR-590-5p, hsa-miR-382-5p, hsa-miR-30e-3p, hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-485-3p, hsa-miR-335-5p, hsa-miR-1290, hsa-miR-132-3p | 88.7 | 93.21 | 91.11 |
| 416 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 95.8 | 95.93 | 97.89 |
| 417 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-382-5p | 94.02 | 96.35 | 95.89 |
| 418 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 94.02 | 94.56 | 95.89 |
| 419 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 93.94 | 94.93 | 96.78 |
| 420 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-335-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 93.11 | 96.72 | 94.56 |
| 421 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-335-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 93.11 | 96.35 | 95.67 |
| 422 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-451a, hsa-miR-491-5p, hsa-miR-501-5p, hsa-miR-335-5p, hsa-miR-337-5p, hsa-miR-4286, hsa-miR-103a-2-5p, hsa-miR-215-5p, hsa-miR-590-3p, hsa-miR-382-5p | 93.03 | 96.67 | 95.67 |
| 423 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-337-5p, hsa-miR-4286, hsa-miR-103a-2-5p, hsa-miR-215-5p, hsa-miR-590-3p, hsa-miR-500a-5p | 93.03 | 93.76 | 94.56 |
| 424 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-337-5p, hsa-miR-4286, hsa-miR-103a-2-5p, hsa-miR-215-5p, hsa-miR-590-3p, hsa-miR-500a-5p, hsa-miR-382-5p | 93.03 | 95.3 | 94.56 |
| 425 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-382-5p | 93.03 | 95.3 | 94.78 |
| 426 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 93.03 | 94.93 | 94.67 |
| 427 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 93.03 | 93.81 | 94.67 |
| 428 | hsa-miR-30e-3p, hsa-miR-545-5p, hsa-miR-548I, hsa-miR-20b-5p, hsa-miR-193b-3p, hsa-miR-450a-5p, hsa-miR-181c-3p, hsa-miR-451a, hsa-miR-501-5p, hsa-miR-485-3p, hsa-miR-1290, hsa-miR-337-5p, hsa-miR-4286, hsa-miR-103a-2-5p, hsa-miR-215-5p, hsa-miR-590-3p, hsa-miR-500a-5p, hsa-miR-382-5p | 92.95 | 97.28 | 94.56 |
| 429 | hsa-miR-30e-3p, hsa-miR-548I, hsa-miR-20b-5p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 92.5 | 94.74 | 94.89 |
| 430 | hsa-miR-30e-3p, hsa-miR-548l, hsa-miR-20b-5p, hsa-miR-193b-3p, hsa-miR-450a-5p, hsa-miR-433-3p, hsa-miR-221-5p, hsa-miR-331-5p, hsa-miR-181c-3p, hsa-miR-501-5p, hsa-miR-335-5p, hsa-miR-4286, hsa-miR-103a-2-5p, hsa-miR-215-5p, hsa-miR-590-3p, hsa-miR-500a-5p, hsa-miR-382-5p | 92.5 | 98.52 | 95.78 |
| 431 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-337-5p, hsa-miR-4286, hsa-miR-215-5p, hsa-miR-590-3p, hsa-miR-500a-5p, hsa-miR-382-5p | 92.5 | 95.24 | 94.67 |
| 432 | hsa-miR-30e-3p, hsa-miR-548I, hsa-miR-20b-5p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p, hsa-miR-382-5p | 92.27 | 95.06 | 95.78 |
| 433 | hsa-miR-30e-3p, hsa-miR-548I, hsa-miR-20b-5p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p, hsa-miR-382-5p | 92.27 | 96.67 | 93.67 |
| 434 | hsa-miR-30e-3p, hsa-miR-548I, hsa-miR-20b-5p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p, hsa-miR-382-5p | 92.27 | 97.59 | 94.67 |
| 435 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 92.2 | 94.93 | 94.78 |
| 436 | hsa-miR-30e-3p, hsa-miR-548I, hsa-miR-20b-5p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-335-5p, hsa-miR-4286, hsa-miR-103a-2-5p, hsa-miR-215-5p, hsa-miR-590-3p, hsa-miR-500a-5p, hsa-miR-382-5p | 92.2 | 96.85 | 93.56 |
| 437 | hsa-miR-30e-3p, hsa-miR-548I, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p, hsa-miR-382-5p | 92.2 | 94.74 | 94.67 |
| 438 | hsa-miR-30e-3p, hsa-miR-548I, hsa-miR-20b-5p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 92.2 | 94.93 | 95.78 |
| 439 | hsa-miR-30e-3p, hsa-miR-548I, hsa-miR-20b-5p, hsa-miR-450a-5p, hsa-miR-181c-3p, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-590-5p | 92.2 | 93.57 | 94.56 |
| 440 | hsa-miR-30e-3p, hsa-miR-548I, hsa-miR-20b-5p, hsa-miR-193b-3p, hsa-miR-433-3p, hsa-miR-331-5p, hsa-miR-181c-3p, hsa-miR-451a, hsa-miR-491-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 92.2 | 96.22 | 95.78 |
| 441 | hsa-miR-30e-3p, hsa-miR-548I, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 92.2 | 95 | 93.67 |
| 442 | hsa-miR-30e-3p, hsa-miR-548I, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 92.2 | 93.57 | 93.67 |
| 443 | hsa-miR-30e-3p, hsa-miR-548l, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 92.2 | 94.5 | 95.78 |
| 444 | hsa-miR-548I, hsa-miR-20b-5p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-335-5p, hsa-miR-4286, hsa-miR-103a-2-5p, hsa-miR-215-5p, hsa-miR-590-3p, hsa-miR-500a-5p, hsa-miR-382-5p | 92.2 | 95.61 | 94.67 |
| 445 | hsa-miR-30e-3p, hsa-miR-548I, hsa-miR-20b-5p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 92.2 | 95.06 | 93.67 |
| 446 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-4286, hsa-miR-103a-2-5p, hsa-miR-215-5p, hsa-miR-590-3p, hsa-miR-500a-5p, hsa-miR-382-5p | 92.12 | 94.5 | 94.56 |
| 447 | hsa-miR-30e-3p, hsa-miR-548l, hsa-miR-20b-5p, hsa-miR-193b-3p, hsa-miR-450a-5p, hsa-miR-181c-3p, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-590-5p, hsa-miR-382-5p | 92.12 | 94.69 | 94.56 |
| 448 | hsa-miR-30e-3p, hsa-miR-548l, hsa-miR-20b-5p, hsa-miR-193b-3p, hsa-miR-450a-5p, hsa-miR-181c-3p, hsa-miR-491-5p, hsa-miR-501-5p, hsa-miR-485-3p, hsa-miR-1290, hsa-miR-337-5p, hsa-miR-4286, hsa-miR-103a-2-5p, hsa-miR-215-5p, hsa-miR-590-3p, hsa-miR-500a-5p, hsa-miR-590-5p, hsa-miR-382-5p | 92.12 | 94.19 | 94.56 |
| 449 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-337-5p, hsa-miR-4286, hsa-miR-103a-2-5p, hsa-miR-215-5p, hsa-miR-590-3p, hsa-miR-500a-5p | 92.12 | 94.31 | 94.56 |
| 450 | hsa-miR-30e-3p, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-337-5p, hsa-miR-4286, hsa-miR-103a-2-5p, hsa-miR-215-5p, hsa-miR-590-3p, hsa-miR-500a-5p, hsa-miR-382-5p | 92.05 | 97.28 | 94.56 |
| 451 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p, hsa-miR-382-5p | 91.4 | 94.5 | 94.78 |
| 452 | hsa-miR-30e-3p, hsa-miR-545-5p, hsa-miR-548I, hsa-miR-20b-5p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p, hsa-miR-382-5p | 91.6 | 95.56 | 92.56 |
| 453 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-1290, hsa-miR-132-3p, hsa-miR-4286, hsa-miR-103a-2-5p, hsa-miR-215-5p, hsa-miR-590-3p, hsa-miR-500a-5p, hsa-miR-382-5p | 91.6 | 94.87 | 94.78 |
| 454 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 91.6 | 94.74 | 93.67 |
| 455 | hsa-miR-30e-3p, hsa-miR-548l, hsa-miR-20b-5p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-500a-5p | 91.6 | 93.94 | 93.67 |
| 456 | hsa-miR-30e-3p, hsa-miR-548I, hsa-miR-193b-3p, hsa-miR-450a-5p, hsa-miR-221-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 91.6 | 94.37 | 93.67 |
| 457 | hsa-miR-30e-3p, hsa-miR-548I, hsa-miR-20b-5p, hsa-miR-193b-3p, hsa-miR-450a-5p, hsa-miR-221-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 91.6 | 94.19 | 93.56 |
| 458 | hsa-miR-30e-3p, hsa-miR-548I, hsa-miR-450a-5p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p | 91.6 | 92.52 | 93.67 |
| 459 | hsa-miR-30e-3p, hsa-miR-548I, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-335-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 91.6 | 96.48 | 93.67 |
| 460 | hsa-miR-30e-3p, hsa-miR-548l, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 91.6 | 95.48 | 93.67 |
| 461 | hsa-miR-30e-3p, hsa-miR-548I, hsa-miR-20b-5p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p | 91.6 | 93.07 | 93.56 |
| 462 | hsa-miR-30e-3p, hsa-miR-548I, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-4732-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 91.6 | 93.63 | 93.67 |
| 463 | hsa-miR-30e-3p, hsa-miR-548I, hsa-miR-20b-5p, hsa-miR-193b-3p, hsa-miR-450a-5p, hsa-miR-433-3p, hsa-miR-221-5p, hsa-miR-331-5p, hsa-miR-181c-3p, hsa-miR-491-5p, hsa-miR-501-5p, hsa-miR-4286, hsa-miR-103a-2-5p, hsa-miR-215-5p, hsa-miR-590-3p, hsa-miR-500a-5p, hsa-miR-382-5p | 91.6 | 96.48 | 93.56 |
| 464 | hsa-miR-30e-3p, hsa-miR-548I, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 91.6 | 94.56 | 93.67 |
| 465 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-590-5p, hsa-miR-382-5p | 91.29 | 95.24 | 92.33 |
| 466 | hsa-miR-30e-3p, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 91.29 | 94.74 | 94.56 |
| 467 | hsa-miR-30e-3p, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-337-5p, hsa-miR-4286, hsa-miR-103a-2-5p, hsa-miR-215-5p, hsa-miR-590-3p, hsa-miR-500a-5p, hsa-miR-382-5p | 91.29 | 94.56 | 93.44 |
| 468 | hsa-miR-30e-3p, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-4732-5p, hsa-miR-335-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 91.29 | 95.48 | 93.44 |
| 469 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 91.29 | 95.85 | 93.56 |
| 470 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-337-5p, hsa-miR-4286, hsa-miR-103a-2-5p, hsa-miR-215-5p, hsa-miR-590-3p, hsa-miR-500a-5p, hsa-miR-382-5p | 91.29 | 96.35 | 93.56 |
| 471 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-337-5p, hsa-miR-4286, hsa-miR-103a-2-5p, hsa-miR-590-3p, hsa-miR-590-5p, hsa-miR-382-5p | 91.21 | 93.94 | 94.56 |
| 472 | hsa-miR-548I, hsa-miR-20b-5p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-500a-5p, hsa-miR-382-5p | 90.1 | 91.7 | 93.78 |
| 473 | hsa-miR-30e-3p, hsa-miR-548l, hsa-miR-20b-5p, hsa-miR-193b-3p, hsa-miR-433-3p, hsa-miR-331-5p, hsa-miR-181c-3p, hsa-miR-451a, hsa-miR-491-5p, hsa-miR-132-3p, hsa-miR-337-5p, hsa-miR-4286, hsa-miR-103a-2-5p, hsa-miR-215-5p, hsa-miR-500a-5p, hsa-miR-382-5p | 90.1 | 93.81 | 94.67 |
| 474 | hsa-miR-30e-3p, hsa-miR-545-5p, hsa-miR-548I, hsa-miR-20b-5p, hsa-miR-433-3p, hsa-miR-331-5p, hsa-miR-181c-3p, hsa-miR-501-5p, hsa-miR-4732-5p, hsa-miR-132-3p, hsa-miR-4286, hsa-miR-103a-2-5p, hsa-miR-215-5p, hsa-miR-590-3p, hsa-miR-590-5p | 90.1 | 94.87 | 94.56 |
| 475 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-337-5p, hsa-miR-4286, hsa-miR-215-5p, hsa-miR-590-3p, hsa-miR-500a-5p, hsa-miR-382-5p | 90.1 | 94.87 | 92.44 |
| 476 | hsa-miR-30e-3p, hsa-miR-548l, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-590-3p, hsa-miR-500a-5p, hsa-miR-382-5p | 90.1 | 92.63 | 92.67 |
| 477 | hsa-miR-30e-3p, hsa-miR-548I, hsa-miR-20b-5p, hsa-miR-450a-5p, hsa-miR-221-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 90.3 | 96.54 | 92.56 |
| 478 | hsa-miR-30e-3p, hsa-miR-548I, hsa-miR-20b-5p, hsa-miR-193b-3p, hsa-miR-450a-5p, hsa-miR-433-3p, hsa-miR-221-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-500a-5p, hsa-miR-382-5p | 90.3 | 95.8 | 93.56 |
| 479 | hsa-miR-30e-3p, hsa-miR-548I, hsa-miR-20b-5p, hsa-miR-193b-3p, hsa-miR-450a-5p, hsa-miR-221-5p, hsa-miR-181c-3p, hsa-miR-451a, hsa-miR-501-5p, hsa-miR-485-3p, hsa-miR-335-5p, hsa-miR-1290, hsa-miR-337-5p, hsa-miR-4286, hsa-miR-215-5p, hsa-miR-590-3p, hsa-miR-590-5p, hsa-miR-382-5p | 90.3 | 90.85 | 92.33 |
| 480 | hsa-miR-548I, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p, hsa-miR-382-5p | 90.3 | 95.43 | 93.67 |
| 481 | hsa-miR-30e-3p, hsa-miR-548l, hsa-miR-20b-5p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-500a-5p, hsa-miR-382-5p | 90.3 | 93.94 | 93.67 |
| 482 | hsa-miR-30e-3p, hsa-miR-548I, hsa-miR-20b-5p, hsa-miR-193b-3p, hsa-miR-450a-5p, hsa-miR-221-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-500a-5p | 90.3 | 94 | 92.56 |
| 483 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-335-5p, hsa-miR-337-5p, hsa-miR-4286, hsa-miR-215-5p, hsa-miR-590-3p, hsa-miR-500a-5p, hsa-miR-382-5p | 90.3 | 95.85 | 92.33 |
| 484 | hsa-miR-30e-3p, hsa-miR-545-5p, hsa-miR-548I, hsa-miR-20b-5p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-500a-5p, hsa-miR-382-5p | 90.3 | 93.07 | 93.67 |
| 485 | hsa-miR-548I, hsa-miR-20b-5p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p, hsa-miR-382-5p | 90.3 | 95.98 | 92.56 |
| 486 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-337-5p, hsa-miR-4286, hsa-miR-103a-2-5p, hsa-miR-590-3p, hsa-miR-382-5p | 90.3 | 94 | 94.67 |
| 487 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-590-5p | 90.3 | 93.44 | 92.44 |
| 488 | hsa-miR-548I, hsa-miR-20b-5p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 90.3 | 94.31 | 94.56 |
| 489 | hsa-miR-30e-3p, hsa-miR-548I, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-500a-5p | 90.5 | 93.31 | 92.56 |
| 490 | hsa-miR-30e-3p, hsa-miR-548I, hsa-miR-20b-5p, hsa-miR-193b-3p, hsa-miR-450a-5p, hsa-miR-433-3p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-500a-5p | 90.5 | 94 | 94.67 |
| 491 | hsa-miR-30e-3p, hsa-miR-545-5p, hsa-miR-548I, hsa-miR-20b-5p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p, hsa-miR-382-5p | 90.5 | 96.11 | 92.44 |
| 492 | hsa-miR-30e-3p, hsa-miR-545-5p, hsa-miR-20b-5p, hsa-miR-193b-3p, hsa-miR-181c-3p, hsa-miR-451a, hsa-miR-491-5p, hsa-miR-501-5p, hsa-miR-485-3p, hsa-miR-1290, hsa-miR-337-5p, hsa-miR-4286, hsa-miR-103a-2-5p, hsa-miR-215-5p, hsa-miR-590-3p, hsa-miR-500a-5p | 90.5 | 94.5 | 94.56 |
| 493 | hsa-miR-30e-3p, hsa-miR-548I, hsa-miR-20b-5p, hsa-miR-193b-3p, hsa-miR-450a-5p, hsa-miR-181c-3p, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 90.5 | 92.94 | 92.56 |
| 494 | hsa-miR-30e-3p, hsa-miR-548I, hsa-miR-20b-5p, hsa-miR-193b-3p, hsa-miR-450a-5p, hsa-miR-433-3p, hsa-miR-221-5p, hsa-miR-331-5p, hsa-miR-181c-3p, hsa-miR-491-5p, hsa-miR-501-5p, hsa-miR-335-5p, hsa-miR-4286, hsa-miR-103a-2-5p, hsa-miR-215-5p, hsa-miR-590-3p, hsa-miR-500a-5p, hsa-miR-382-5p | 90.5 | 96.48 | 92.44 |
| 495 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 90.5 | 93.81 | 93.56 |
| 496 | hsa-miR-30e-3p, hsa-miR-548I, hsa-miR-20b-5p, hsa-miR-193b-3p, hsa-miR-450a-5p, hsa-miR-181c-3p, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-590-5p, hsa-miR-382-5p | 90.5 | 94.19 | 93.56 |
| 497 | hsa-miR-30e-3p, hsa-miR-545-5p, hsa-miR-548I, hsa-miR-20b-5p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-4286, hsa-miR-103a-2-5p, hsa-miR-215-5p, hsa-miR-590-3p, hsa-miR-500a-5p, hsa-miR-382-5p | 90.5 | 94.44 | 93.56 |
| 498 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-451a, hsa-miR-491-5p, hsa-miR-132-3p, hsa-miR-337-5p, hsa-miR-4286, hsa-miR-103a-2-5p, hsa-miR-215-5p, hsa-miR-590-3p, hsa-miR-382-5p | 90.8 | 94.5 | 93.56 |
| 499 | hsa-miR-30e-3p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-337-5p, hsa-miR-4286, hsa-miR-103a-2-5p, hsa-miR-215-5p, hsa-miR-590-3p, hsa-miR-500a-5p, hsa-miR-382-5p | 90.8 | 94.06 | 93.56 |
| 500 | hsa-miR-30e-3p, hsa-miR-548I, hsa-miR-20b-5p, hsa-miR-193b-3p, hsa-miR-450a-5p, hsa-miR-181c-3p, hsa-miR-491-5p, hsa-miR-501-5p, hsa-miR-485-3p, hsa-miR-1290, hsa-miR-337-5p, hsa-miR-4286, hsa-miR-103a-2-5p, hsa-miR-590-3p, hsa-miR-590-5p, hsa-miR-382-5p | 90.8 | 94 | 93.56 |
| 501 | hsa-miR-30e-3p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 90.8 | 95.17 | 93.56 |
| 502 | hsa-miR-548I, hsa-miR-20b-5p, hsa-miR-450a-5p, hsa-miR-410-3p, hsa-miR-433-3p, hsa-miR-331-5p, hsa-miR-181c-3p, hsa-miR-451a, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 89.77 | 95.98 | 94.56 |
| 503 | hsa-miR-545-5p, hsa-miR-548I, hsa-miR-20b-5p, hsa-miR-450a-5p, hsa-miR-410-3p, hsa-miR-433-3p, hsa-miR-331-5p, hsa-miR-181c-3p, hsa-miR-501-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 89.7 | 95.43 | 94.56 |
| 504 | hsa-miR-545-5p, hsa-miR-548I, hsa-miR-20b-5p, hsa-miR-193b-3p, hsa-miR-450a-5p, hsa-miR-221-5p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 89.7 | 92.94 | 93.67 |
| 505 | hsa-miR-545-5p, hsa-miR-548I, hsa-miR-20b-5p, hsa-miR-193b-3p, hsa-miR-450a-5p, hsa-miR-410-3p, hsa-miR-221-5p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p | 89.7 | 91.59 | 93.44 |
| 506 | hsa-miR-30e-3p, hsa-miR-545-5p, hsa-miR-548I, hsa-miR-20b-5p, hsa-miR-450a-5p, hsa-miR-433-3p, hsa-miR-331-5p, hsa-miR-181c-3p, hsa-miR-451a, hsa-miR-501-5p, hsa-miR-4732-5p, hsa-miR-132-3p, hsa-miR-103a-2-5p, hsa-miR-215-5p, hsa-miR-590-3p, hsa-miR-590-5p | 89.7 | 95.37 | 95.56 |
| 507 | hsa-miR-548l, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 89.7 | 94.93 | 93.67 |
| 508 | hsa-miR-545-5p, hsa-miR-20b-5p, hsa-miR-410-3p, hsa-miR-221-5p, hsa-miR-331-5p, hsa-miR-181c-3p, hsa-miR-501-5p, hsa-miR-4732-5p, hsa-miR-335-5p, hsa-miR-4286, hsa-miR-590-5p, hsa-miR-382-5p | 89.7 | 88.81 | 93.44 |
| 509 | hsa-miR-30e-3p, hsa-miR-548I, hsa-miR-20b-5p, hsa-miR-450a-5p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 89.2 | 96.72 | 92.56 |
| 510 | hsa-miR-30e-3p, hsa-miR-548I, hsa-miR-193b-3p, hsa-miR-450a-5p, hsa-miR-433-3p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-500a-5p | 89.2 | 93.63 | 93.67 |
| 511 | hsa-miR-30e-3p, hsa-miR-548I, hsa-miR-20b-5p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-132-3p, hsa-miR-4286, hsa-miR-103a-2-5p, hsa-miR-215-5p, hsa-miR-590-3p, hsa-miR-500a-5p | 89.2 | 95.56 | 94.56 |
| 512 | hsa-miR-545-5p, hsa-miR-548l, hsa-miR-20b-5p, hsa-miR-450a-5p, hsa-miR-410-3p, hsa-miR-433-3p, hsa-miR-331-5p, hsa-miR-181c-3p, hsa-miR-501-5p, hsa-miR-335-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 89.2 | 92.46 | 92.33 |
| 513 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-337-5p, hsa-miR-4286, hsa-miR-215-5p, hsa-miR-590-3p, hsa-miR-500a-5p, hsa-miR-382-5p | 89.2 | 93.44 | 92.44 |
| 514 | hsa-miR-30e-3p, hsa-miR-548I, hsa-miR-20b-5p, hsa-miR-193b-3p, hsa-miR-450a-5p, hsa-miR-433-3p, hsa-miR-221-5p, hsa-miR-331-5p, hsa-miR-181c-3p, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p, hsa-miR-382-5p | 89.2 | 95.61 | 93.56 |
| 515 | hsa-miR-30e-3p, hsa-miR-548I, hsa-miR-20b-5p, hsa-miR-193b-3p, hsa-miR-450a-5p, hsa-miR-331-5p, hsa-miR-181c-3p, hsa-miR-451a, hsa-miR-501-5p, hsa-miR-335-5p, hsa-miR-1290, hsa-miR-337-5p, hsa-miR-4286, hsa-miR-215-5p, hsa-miR-590-3p, hsa-miR-500a-5p, hsa-miR-382-5p | 89.2 | 92.94 | 91.33 |
| 516 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-500a-5p, hsa-miR-382-5p | 89.2 | 92.07 | 92.67 |
| 517 | hsa-miR-30e-3p, hsa-miR-548I, hsa-miR-20b-5p, hsa-miR-450a-5p, hsa-miR-433-3p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 89.5 | 94 | 93.56 |
| 518 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-590-3p, hsa-miR-500a-5p, hsa-miR-590-5p, hsa-miR-382-5p | 89.5 | 91.78 | 94.67 |
| 519 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-590-3p, hsa-miR-590-5p, hsa-miR-382-5p | 89.5 | 94.87 | 91.44 |
| 520 | hsa-miR-30e-3p, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-4732-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 89.5 | 96.41 | 94.56 |
| 521 | hsa-miR-545-5p, hsa-miR-548I, hsa-miR-20b-5p, hsa-miR-450a-5p, hsa-miR-433-3p, hsa-miR-331-5p, hsa-miR-181c-3p, hsa-miR-491-5p, hsa-miR-501-5p, hsa-miR-335-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 89.5 | 94.69 | 92.33 |
| 522 | hsa-miR-30e-3p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-485-3p, hsa-miR-4286, hsa-miR-103a-2-5p, hsa-miR-215-5p, hsa-miR-590-3p, hsa-miR-500a-5p | 89.5 | 94.61 | 93.44 |
| 523 | hsa-miR-545-5p, hsa-miR-548I, hsa-miR-20b-5p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-335-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 89.5 | 94.5 | 92.33 |
| 524 | hsa-miR-30e-3p, hsa-miR-20b-5p, hsa-miR-193b-3p, hsa-miR-450a-5p, hsa-miR-181c-3p, hsa-miR-491-5p, hsa-miR-501-5p, hsa-miR-4732-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-590-5p | 89.7 | 93.13 | 92.33 |
| 525 | hsa-miR-30e-3p, hsa-miR-548I, hsa-miR-20b-5p, hsa-miR-193b-3p, hsa-miR-433-3p, hsa-miR-331-5p, hsa-miR-451a, hsa-miR-491-5p, hsa-miR-132-3p, hsa-miR-337-5p, hsa-miR-4286, hsa-miR-103a-2-5p, hsa-miR-215-5p, hsa-miR-500a-5p, hsa-miR-382-5p | 89.7 | 92.76 | 92.44 |
| 526 | hsa-miR-30e-3p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-132-3p, hsa-miR-4286, hsa-miR-103a-2-5p, hsa-miR-215-5p, hsa-miR-590-3p, hsa-miR-500a-5p | 89.9 | 94.37 | 92.33 |
| 527 | hsa-miR-30e-3p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-4732-5p, hsa-miR-132-3p, hsa-miR-4286, hsa-miR-103a-2-5p, hsa-miR-215-5p, hsa-miR-590-3p, hsa-miR-500a-5p | 89.9 | 93.13 | 92.33 |
| 528 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-335-5p, hsa-miR-337-5p, hsa-miR-4286, hsa-miR-103a-2-5p, hsa-miR-590-3p, hsa-miR-590-5p, hsa-miR-382-5p | 89.9 | 93.94 | 91.22 |
| 529 | hsa-miR-30e-3p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-4286, hsa-miR-103a-2-5p, hsa-miR-215-5p, hsa-miR-590-3p, hsa-miR-500a-5p | 89.9 | 94.06 | 92.33 |
| 530 | hsa-miR-30e-3p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-4732-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 89.9 | 92.37 | 93.33 |
| 531 | hsa-miR-545-5p, hsa-miR-548I, hsa-miR-20b-5p, hsa-miR-450a-5p, hsa-miR-410-3p, hsa-miR-433-3p, hsa-miR-331-5p, hsa-miR-181c-3p, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 88.86 | 95.43 | 93.56 |
| 532 | hsa-miR-30e-3p, hsa-miR-548l, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-337-5p, hsa-miR-4286, hsa-miR-215-5p, hsa-miR-590-3p, hsa-miR-500a-5p, hsa-miR-382-5p | 88.86 | 93.61 | 93.67 |
| 533 | hsa-miR-545-5p, hsa-miR-548I, hsa-miR-20b-5p, hsa-miR-450a-5p, hsa-miR-410-3p, hsa-miR-433-3p, hsa-miR-331-5p, hsa-miR-181c-3p, hsa-miR-451a, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 88.86 | 95.43 | 93.44 |
| 534 | hsa-miR-545-5p, hsa-miR-548I, hsa-miR-20b-5p, hsa-miR-193b-3p, hsa-miR-450a-5p, hsa-miR-410-3p, hsa-miR-221-5p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 88.86 | 92.57 | 92.56 |
| 535 | hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-331-5p, hsa-miR-181c-3p, hsa-miR-451a, hsa-miR-501-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 88.86 | 96.35 | 93.56 |
| 536 | hsa-miR-545-5p, hsa-miR-548I, hsa-miR-20b-5p, hsa-miR-450a-5p, hsa-miR-410-3p, hsa-miR-221-5p, hsa-miR-181c-3p, hsa-miR-451a, hsa-miR-501-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-500a-5p, hsa-miR-382-5p | 88.86 | 94.69 | 91.56 |
| 537 | hsa-miR-545-5p, hsa-miR-548I, hsa-miR-20b-5p, hsa-miR-450a-5p, hsa-miR-410-3p, hsa-miR-181c-3p, hsa-miR-451a, hsa-miR-501-5p, hsa-miR-485-3p, hsa-miR-1290, hsa-miR-337-5p, hsa-miR-4286, hsa-miR-103a-2-5p, hsa-miR-215-5p, hsa-miR-590-3p, hsa-miR-500a-5p, hsa-miR-382-5p | 88.86 | 94.37 | 93.44 |
| 538 | hsa-miR-545-5p, hsa-miR-548l, hsa-miR-20b-5p, hsa-miR-193b-3p, hsa-miR-450a-5p, hsa-miR-221-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-590-5p | 88.79 | 90.3 | 92.33 |
| 539 | hsa-miR-30e-3p, hsa-miR-548I, hsa-miR-20b-5p, hsa-miR-193b-3p, hsa-miR-433-3p, hsa-miR-491-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 88.79 | 92.2 | 94.67 |
| 540 | hsa-miR-30e-3p, hsa-miR-548l, hsa-miR-20b-5p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-335-5p, hsa-miR-337-5p, hsa-miR-4286, hsa-miR-215-5p, hsa-miR-590-3p, hsa-miR-500a-5p, hsa-miR-382-5p | 88.79 | 94.19 | 91.44 |
| 541 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-337-5p, hsa-miR-4286, hsa-miR-215-5p, hsa-miR-590-3p, hsa-miR-500a-5p | 88.79 | 93.69 | 91.33 |
| 542 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-337-5p, hsa-miR-4286, hsa-miR-215-5p, hsa-miR-590-3p, hsa-miR-500a-5p, hsa-miR-382-5p | 88.79 | 94.24 | 91.33 |
| 543 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-335-5p, hsa-miR-132-3p, hsa-miR-337-5p, hsa-miR-4286, hsa-miR-215-5p, hsa-miR-590-3p, hsa-miR-500a-5p, hsa-miR-382-5p | 88.79 | 94.06 | 91.44 |
| 544 | hsa-miR-30e-3p, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-4732-5p, hsa-miR-337-5p, hsa-miR-4286, hsa-miR-215-5p, hsa-miR-590-3p, hsa-miR-500a-5p, hsa-miR-382-5p | 88.79 | 93.69 | 93.44 |
| 545 | hsa-miR-30e-3p, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-4286, hsa-miR-215-5p, hsa-miR-590-3p, hsa-miR-500a-5p, hsa-miR-382-5p | 88.79 | 88.98 | 92.44 |
| 546 | hsa-miR-30e-3p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 88.71 | 92.02 | 95.67 |
| 547 | hsa-miR-20b-5p, hsa-miR-193b-3p, hsa-miR-450a-5p, hsa-miR-221-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 88.71 | 92.94 | 91.33 |
| 548 | hsa-miR-30e-3p, hsa-miR-548I, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-500a-5p, hsa-miR-382-5p | 88.71 | 93.37 | 91.56 |
| 549 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-590-3p, hsa-miR-590-5p, hsa-miR-382-5p | 88.71 | 92.39 | 93.67 |
| 550 | hsa-miR-20b-5p, hsa-miR-193b-3p, hsa-miR-450a-5p, hsa-miR-433-3p, hsa-miR-221-5p, hsa-miR-181c-3p, hsa-miR-451a, hsa-miR-501-5p, hsa-miR-4732-5p, hsa-miR-485-3p, hsa-miR-4286, hsa-miR-103a-2-5p, hsa-miR-215-5p, hsa-miR-590-5p, hsa-miR-382-5p | 88.71 | 93.44 | 93.44 |
| 551 | hsa-miR-30e-3p, hsa-miR-20b-5p, hsa-miR-193b-3p, hsa-miR-450a-5p, hsa-miR-221-5p, hsa-miR-181c-3p, hsa-miR-451a, hsa-miR-491-5p, hsa-miR-485-3p, hsa-miR-1290, hsa-miR-132-3p, hsa-miR-337-5p, hsa-miR-103a-2-5p, hsa-miR-215-5p, hsa-miR-590-3p, hsa-miR-500a-5p | 88.71 | 93.81 | 92.44 |
| 552 | hsa-miR-30e-3p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-4732-5p, hsa-miR-4286, hsa-miR-103a-2-5p, hsa-miR-215-5p, hsa-miR-590-3p, hsa-miR-500a-5p | 88.4 | 94.56 | 92.33 |
| 553 | hsa-miR-30e-3p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 88.4 | 94.67 | 92.44 |
| 554 | hsa-miR-30e-3p, hsa-miR-548I, hsa-miR-20b-5p, hsa-miR-193b-3p, hsa-miR-450a-5p, hsa-miR-410-3p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-500a-5p | 88.4 | 92.15 | 91.44 |
| 555 | hsa-miR-30e-3p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-337-5p, hsa-miR-4286, hsa-miR-103a-2-5p, hsa-miR-215-5p, hsa-miR-590-3p, hsa-miR-500a-5p | 88.6 | 95.3 | 91.33 |
| 556 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-335-5p, hsa-miR-337-5p, hsa-miR-4286, hsa-miR-103a-2-5p, hsa-miR-590-3p, hsa-miR-500a-5p, hsa-miR-590-5p, hsa-miR-382-5p | 88.6 | 93.39 | 91.22 |
| 557 | hsa-miR-30e-3p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-4732-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 88.6 | 94.3 | 92.33 |
| 558 | hsa-miR-30e-3p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-4732-5p, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 88.8 | 90.89 | 92.22 |
| 559 | hsa-miR-545-5p, hsa-miR-548I, hsa-miR-20b-5p, hsa-miR-450a-5p, hsa-miR-410-3p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p, hsa-miR-382-5p | 88.03 | 94.5 | 92.56 |
| 560 | hsa-miR-30e-3p, hsa-miR-548I, hsa-miR-20b-5p, hsa-miR-193b-3p, hsa-miR-450a-5p, hsa-miR-331-5p, hsa-miR-451a, hsa-miR-491-5p, hsa-miR-501-5p, hsa-miR-132-3p, hsa-miR-337-5p, hsa-miR-4286, hsa-miR-103a-2-5p, hsa-miR-590-3p, hsa-miR-500a-5p | 88.03 | 92.26 | 92.67 |
| 561 | hsa-miR-545-5p, hsa-miR-548I, hsa-miR-20b-5p, hsa-miR-450a-5p, hsa-miR-410-3p, hsa-miR-221-5p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-500a-5p | 88.03 | 94 | 91.44 |
| 562 | hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-331-5p, hsa-miR-181c-3p, hsa-miR-451a, hsa-miR-501-5p, hsa-miR-337-5p, hsa-miR-103a-2-5p, hsa-miR-215-5p, hsa-miR-590-3p, hsa-miR-500a-5p, hsa-miR-382-5p | 87.95 | 91.17 | 91.33 |
| 563 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-500a-5p | 87.95 | 93.19 | 92.67 |
| 564 | hsa-miR-30e-3p, hsa-miR-548I, hsa-miR-20b-5p, hsa-miR-450a-5p, hsa-miR-433-3p, hsa-miR-331-5p, hsa-miR-181c-3p, hsa-miR-451a, hsa-miR-501-5p, hsa-miR-4732-5p, hsa-miR-1290, hsa-miR-132-3p, hsa-miR-4286, hsa-miR-103a-2-5p, hsa-miR-215-5p, hsa-miR-590-3p, hsa-miR-590-5p | 87.95 | 94.31 | 92.33 |
| 565 | hsa-miR-30e-3p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-132-3p, hsa-miR-337-5p, hsa-miR-4286, hsa-miR-215-5p, hsa-miR-590-3p, hsa-miR-500a-5p, hsa-miR-382-5p | 87.88 | 89.28 | 90.11 |
| 566 | hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-331-5p, hsa-miR-181c-3p, hsa-miR-451a, hsa-miR-501-5p, hsa-miR-132-3p, hsa-miR-337-5p, hsa-miR-103a-2-5p, hsa-miR-215-5p, hsa-miR-590-3p, hsa-miR-500a-5p, hsa-miR-382-5p | 87.88 | 92.41 | 91.33 |
| 567 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-335-5p, hsa-miR-1290, hsa-miR-132-3p, hsa-miR-337-5p, hsa-miR-4286, hsa-miR-215-5p, hsa-miR-590-3p, hsa-miR-500a-5p, hsa-miR-382-5p | 87.88 | 92.31 | 90.33 |
| 568 | hsa-miR-30e-3p, hsa-miR-193b-3p, hsa-miR-450a-5p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-337-5p, hsa-miR-4286, hsa-miR-215-5p, hsa-miR-590-3p, hsa-miR-500a-5p | 87.88 | 92.74 | 93.44 |
| 569 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-500a-5p | 87.88 | 91.7 | 92.56 |
| 570 | hsa-miR-30e-3p, hsa-miR-548I, hsa-miR-20b-5p, hsa-miR-193b-3p, hsa-miR-433-3p, hsa-miR-331-5p, hsa-miR-491-5p, hsa-miR-132-3p, hsa-miR-337-5p, hsa-miR-4286, hsa-miR-103a-2-5p, hsa-miR-590-5p, hsa-miR-382-5p | 87.88 | 92.31 | 90.33 |
| 571 | hsa-miR-30e-3p, hsa-miR-548l, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-4732-5p, hsa-miR-335-5p, hsa-miR-132-3p, hsa-miR-337-5p, hsa-miR-4286, hsa-miR-215-5p, hsa-miR-590-3p, hsa-miR-500a-5p | 87.8 | 91.57 | 91.33 |
| 572 | hsa-miR-30e-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-132-3p, hsa-miR-4286, hsa-miR-103a-2-5p, hsa-miR-215-5p, hsa-miR-590-3p, hsa-miR-500a-5p | 87.8 | 93.76 | 91.33 |
| 573 | hsa-miR-30e-3p, hsa-miR-193b-3p, hsa-miR-450a-5p, hsa-miR-331-5p, hsa-miR-181c-3p, hsa-miR-451a, hsa-miR-501-5p, hsa-miR-4732-5p, hsa-miR-132-3p, hsa-miR-337-5p, hsa-miR-4286, hsa-miR-103a-2-5p, hsa-miR-215-5p, hsa-miR-590-3p, hsa-miR-500a-5p | 87.73 | 92.31 | 91.22 |
| 574 | hsa-miR-30e-3p, hsa-miR-548I, hsa-miR-20b-5p, hsa-miR-193b-3p, hsa-miR-181c-3p, hsa-miR-451a, hsa-miR-491-5p, hsa-miR-485-3p, hsa-miR-1290, hsa-miR-337-5p, hsa-miR-103a-2-5p, hsa-miR-215-5p | 87.73 | 88.44 | 90.11 |
| 575 | hsa-miR-30e-3p, hsa-miR-545-5p, hsa-miR-193b-3p, hsa-miR-450a-5p, hsa-miR-433-3p, hsa-miR-181c-3p, hsa-miR-451a, hsa-miR-491-5p, hsa-miR-4732-5p, hsa-miR-215-5p, hsa-miR-590-3p | 87.73 | 89.24 | 92.22 |
| 576 | hsa-miR-30e-3p, hsa-miR-548I, hsa-miR-20b-5p, hsa-miR-193b-3p, hsa-miR-450a-5p, hsa-miR-410-3p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-500a-5p | 87.73 | 91.28 | 91.33 |
| 577 | hsa-miR-30e-3p, hsa-miR-193b-3p, hsa-miR-450a-5p, hsa-miR-331-5p, hsa-miR-181c-3p, hsa-miR-451a, hsa-miR-491-5p, hsa-miR-501-5p, hsa-miR-4732-5p, hsa-miR-132-3p, hsa-miR-337-5p, hsa-miR-4286, hsa-miR-103a-2-5p, hsa-miR-215-5p, hsa-miR-590-3p, hsa-miR-500a-5p | 87.73 | 91.26 | 91.22 |
| 578 | hsa-miR-30e-3p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-4732-5p, hsa-miR-132-3p, hsa-miR-337-5p, hsa-miR-4286, hsa-miR-103a-2-5p, hsa-miR-215-5p, hsa-miR-590-3p, hsa-miR-500a-5p | 87.73 | 90.22 | 94.56 |
| 579 | hsa-miR-30e-3p, hsa-miR-193b-3p, hsa-miR-450a-5p, hsa-miR-433-3p, hsa-miR-331-5p, hsa-miR-181c-3p, hsa-miR-491-5p, hsa-miR-501-5p, hsa-miR-4732-5p, hsa-miR-335-5p, hsa-miR-337-5p, hsa-miR-4286, hsa-miR-103a-2-5p, hsa-miR-215-5p, hsa-miR-590-3p, hsa-miR-500a-5p, hsa-miR-382-5p | 87.5 | 92.2 | 92.22 |
| 580 | hsa-miR-30e-3p, hsa-miR-20b-5p, hsa-miR-450a-5p, hsa-miR-221-5p, hsa-miR-331-5p, hsa-miR-181c-3p, hsa-miR-501-5p, hsa-miR-4286, hsa-miR-215-5p, hsa-miR-590-3p, hsa-miR-590-5p, hsa-miR-382-5p | 87.27 | 90.48 | 90.22 |
| 581 | hsa-miR-545-5p, hsa-miR-548I, hsa-miR-20b-5p, hsa-miR-450a-5p, hsa-miR-410-3p, hsa-miR-221-5p, hsa-miR-181c-3p, hsa-miR-501-5p, hsa-miR-485-3p, hsa-miR-335-5p, hsa-miR-1290, hsa-miR-132-3p, hsa-miR-4286, hsa-miR-103a-2-5p, hsa-miR-590-5p | 87.2 | 86.22 | 91.33 |
| 582 | hsa-miR-30e-3p, hsa-miR-548I, hsa-miR-20b-5p, hsa-miR-193b-3p, hsa-miR-450a-5p, hsa-miR-433-3p, hsa-miR-451a, hsa-miR-491-5p, hsa-miR-501-5p, hsa-miR-485-3p, hsa-miR-132-3p, hsa-miR-337-5p, hsa-miR-4286, hsa-miR-103a-2-5p, hsa-miR-590-3p, hsa-miR-500a-5p | 87.12 | 92.44 | 91.56 |
| 583 | hsa-miR-548I, hsa-miR-20b-5p, hsa-miR-331-5p, hsa-miR-181c-3p, hsa-miR-501-5p, hsa-miR-1290, hsa-miR-4286, hsa-miR-103a-2-5p, hsa-miR-215-5p, hsa-miR-590-5p, hsa-miR-382-5p | 87.12 | 90.11 | 91.44 |
| 584 | hsa-miR-30e-3p, hsa-miR-548I, hsa-miR-20b-5p, hsa-miR-193b-3p, hsa-miR-410-3p, hsa-miR-433-3p, hsa-miR-331-5p, hsa-miR-181c-3p, hsa-miR-451a, hsa-miR-335-5p, hsa-miR-337-5p, hsa-miR-103a-2-5p, hsa-miR-215-5p, hsa-miR-500a-5p, hsa-miR-382-5p | 87.12 | 93.33 | 92.56 |
| 585 | hsa-miR-30e-3p, hsa-miR-548l, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-132-3p, hsa-miR-337-5p, hsa-miR-4286, hsa-miR-215-5p, hsa-miR-590-3p, hsa-miR-500a-5p, hsa-miR-382-5p | 87.12 | 92.44 | 91.44 |
| 586 | hsa-miR-30e-3p, hsa-miR-545-5p, hsa-miR-548I, hsa-miR-20b-5p, hsa-miR-410-3p, hsa-miR-331-5p, hsa-miR-181c-3p, hsa-miR-132-3p, hsa-miR-337-5p, hsa-miR-4286, hsa-miR-215-5p, hsa-miR-590-3p, hsa-miR-590-5p | 87.05 | 90.35 | 91.22 |
| 587 | hsa-miR-30e-3p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-337-5p, hsa-miR-4286, hsa-miR-215-5p, hsa-miR-590-3p, hsa-miR-500a-5p | 87.05 | 90.28 | 92.44 |
| 588 | hsa-miR-30e-3p, hsa-miR-548I, hsa-miR-20b-5p, hsa-miR-193b-3p, hsa-miR-450a-5p, hsa-miR-433-3p, hsa-miR-181c-3p, hsa-miR-451a, hsa-miR-501-5p, hsa-miR-485-3p, hsa-miR-335-5p, hsa-miR-1290, hsa-miR-337-5p, hsa-miR-4286, hsa-miR-215-5p, hsa-miR-590-3p, hsa-miR-500a-5p, hsa-miR-382-5p | 87.05 | 94.06 | 90.22 |
| 589 | hsa-miR-193b-3p, hsa-miR-433-3p, hsa-miR-221-5p, hsa-miR-331-5p, hsa-miR-451a, hsa-miR-491-5p, hsa-miR-501-5p, hsa-miR-4732-5p, hsa-miR-485-3p, hsa-miR-335-5p, hsa-miR-4286, hsa-miR-103a-2-5p, hsa-miR-215-5p, hsa-miR-590-3p, hsa-miR-500a-5p, hsa-miR-590-5p | 87.05 | 90.78 | 93.44 |
| 590 | hsa-miR-548I, hsa-miR-20b-5p, hsa-miR-450a-5p, hsa-miR-410-3p, hsa-miR-221-5p, hsa-miR-181c-3p, hsa-miR-501-5p, hsa-miR-4732-5p, hsa-miR-335-5p, hsa-miR-132-3p, hsa-miR-4286, hsa-miR-103a-2-5p, hsa-miR-215-5p, hsa-miR-590-3p, hsa-miR-590-5p | 87.05 | 92.7 | 94.56 |
| 591 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-491-5p, hsa-miR-501-5p, hsa-miR-335-5p, hsa-miR-337-5p, hsa-miR-4286, hsa-miR-103a-2-5p, hsa-miR-590-3p, hsa-miR-590-5p, hsa-miR-382-5p | 87.05 | 92.83 | 88.11 |
| 592 | hsa-miR-30e-3p, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-335-5p, hsa-miR-1290, hsa-miR-132-3p, hsa-miR-337-5p, hsa-miR-4286, hsa-miR-215-5p, hsa-miR-590-3p, hsa-miR-500a-5p | 87.05 | 90.33 | 92.44 |
| 593 | hsa-miR-30e-3p, hsa-miR-545-5p, hsa-miR-548I, hsa-miR-20b-5p, hsa-miR-450a-5p, hsa-miR-433-3p, hsa-miR-181c-3p, hsa-miR-485-3p, hsa-miR-132-3p, hsa-miR-337-5p, hsa-miR-103a-2-5p, hsa-miR-215-5p, hsa-miR-590-3p, hsa-miR-500a-5p | 87.05 | 92.57 | 90.33 |
| 594 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-337-5p, hsa-miR-4286, hsa-miR-215-5p, hsa-miR-590-3p, hsa-miR-500a-5p | 87.05 | 92.2 | 91.33 |
| 595 | hsa-miR-30e-3p, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-335-5p, hsa-miR-1290, hsa-miR-337-5p, hsa-miR-4286, hsa-miR-215-5p, hsa-miR-590-3p, hsa-miR-500a-5p | 86.97 | 90.2 | 91.22 |
| 596 | hsa-miR-30e-3p, hsa-miR-193b-3p, hsa-miR-450a-5p, hsa-miR-331-5p, hsa-miR-181c-3p, hsa-miR-451a, hsa-miR-491-5p, hsa-miR-501-5p, hsa-miR-4732-5p, hsa-miR-132-3p, hsa-miR-337-5p, hsa-miR-4286, hsa-miR-215-5p, hsa-miR-590-3p, hsa-miR-500a-5p | 86.97 | 90.46 | 91.22 |
| 597 | hsa-miR-30e-3p, hsa-miR-548I, hsa-miR-20b-5p, hsa-miR-193b-3p, hsa-miR-450a-5p, hsa-miR-181c-3p, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-590-5p, hsa-miR-382-5p | 86.97 | 92.94 | 91.44 |
| 598 | hsa-miR-30e-3p, hsa-miR-548I, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-335-5p, hsa-miR-132-3p, hsa-miR-337-5p, hsa-miR-4286, hsa-miR-215-5p, hsa-miR-590-3p, hsa-miR-500a-5p | 86.97 | 89.72 | 90.22 |
| 599 | hsa-miR-30e-3p, hsa-miR-548I, hsa-miR-20b-5p, hsa-miR-193b-3p, hsa-miR-450a-5p, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-590-5p, hsa-miR-382-5p | 86.97 | 92.2 | 91.44 |
| 600 | hsa-miR-30e-3p, hsa-miR-548I, hsa-miR-20b-5p, hsa-miR-193b-3p, hsa-miR-450a-5p, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-500a-5p, hsa-miR-590-5p | 86.97 | 91.65 | 91.44 |
| 601 | hsa-miR-30e-3p, hsa-miR-20b-5p, hsa-miR-193b-3p, hsa-miR-450a-5p, hsa-miR-433-3p, hsa-miR-331-5p, hsa-miR-181c-3p, hsa-miR-451a, hsa-miR-491-5p, hsa-miR-501-5p, hsa-miR-4732-5p, hsa-miR-132-3p, hsa-miR-337-5p, hsa-miR-4286, hsa-miR-215-5p, hsa-miR-590-3p, hsa-miR-500a-5p, hsa-miR-382-5p | 86.97 | 92.93 | 91.22 |
| 602 | hsa-miR-30e-3p, hsa-miR-548I, hsa-miR-20b-5p, hsa-miR-193b-3p, hsa-miR-450a-5p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-500a-5p | 86.97 | 89.19 | 94.67 |
| 603 | hsa-miR-548I, hsa-miR-20b-5p, hsa-miR-193b-3p, hsa-miR-450a-5p, hsa-miR-410-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-500a-5p | 86.97 | 87.63 | 91.33 |
| 604 | hsa-miR-548I, hsa-miR-20b-5p, hsa-miR-193b-3p, hsa-miR-450a-5p, hsa-miR-410-3p, hsa-miR-451a, hsa-miR-491-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-500a-5p | 86.97 | 88.19 | 91.33 |
| 605 | hsa-miR-545-5p, hsa-miR-548I, hsa-miR-20b-5p, hsa-miR-410-3p, hsa-miR-433-3p, hsa-miR-181c-3p, hsa-miR-501-5p, hsa-miR-4732-5p, hsa-miR-485-3p, hsa-miR-335-5p, hsa-miR-132-3p, hsa-miR-337-5p, hsa-miR-4286, hsa-miR-103a-2-5p, hsa-miR-590-5p | 86.97 | 86.09 | 91.33 |
| 606 | hsa-miR-30e-3p, hsa-miR-548l, hsa-miR-20b-5p, hsa-miR-193b-3p, hsa-miR-410-3p, hsa-miR-451a, hsa-miR-491-5p, hsa-miR-485-3p, hsa-miR-1290, hsa-miR-337-5p, hsa-miR-103a-2-5p, hsa-miR-215-5p, hsa-miR-500a-5p | 86.89 | 90.3 | 91.33 |
| 607 | hsa-miR-30e-3p, hsa-miR-545-5p, hsa-miR-548I, hsa-miR-20b-5p, hsa-miR-193b-3p, hsa-miR-221-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-103a-2-5p, hsa-miR-215-5p, hsa-miR-590-3p, hsa-miR-500a-5p | 86.89 | 92.2 | 91.33 |
| 608 | hsa-miR-30e-3p, hsa-miR-548I, hsa-miR-221-5p, hsa-miR-4732-5p, hsa-miR-485-3p, hsa-miR-1290, hsa-miR-103a-2-5p, hsa-miR-590-3p, hsa-miR-500a-5p | 86.82 | 94.06 | 92.44 |
| 609 | hsa-miR-30e-3p, hsa-miR-545-5p, hsa-miR-548I, hsa-miR-20b-5p, hsa-miR-450a-5p, hsa-miR-410-3p, hsa-miR-221-5p, hsa-miR-181c-3p, hsa-miR-501-5p, hsa-miR-4732-5p, hsa-miR-335-5p, hsa-miR-132-3p, hsa-miR-4286, hsa-miR-215-5p, hsa-miR-590-3p, hsa-miR-590-5p, hsa-miR-382-5p | 86.6 | 90.3 | 93.44 |
| 610 | hsa-miR-30e-3p, hsa-miR-450a-5p, hsa-miR-433-3p, hsa-miR-221-5p, hsa-miR-331-5p, hsa-miR-181c-3p, hsa-miR-501-5p, hsa-miR-215-5p, hsa-miR-590-3p, hsa-miR-382-5p | 86.29 | 90.28 | 90.22 |
| 611 | hsa-miR-545-5p, hsa-miR-548I, hsa-miR-20b-5p, hsa-miR-450a-5p, hsa-miR-410-3p, hsa-miR-221-5p, hsa-miR-181c-3p, hsa-miR-501-5p, hsa-miR-485-3p, hsa-miR-335-5p, hsa-miR-1290, hsa-miR-132-3p, hsa-miR-4286, hsa-miR-103a-2-5p, hsa-miR-590-5p, hsa-miR-382-5p | 86.29 | 89.56 | 90.22 |
| 612 | hsa-miR-30e-3p, hsa-miR-548I, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-132-3p, hsa-miR-337-5p, hsa-miR-4286, hsa-miR-215-5p, hsa-miR-590-3p, hsa-miR-500a-5p, hsa-miR-382-5p | 86.29 | 93.56 | 90.33 |
| 613 | hsa-miR-548l, hsa-miR-20b-5p, hsa-miR-193b-3p, hsa-miR-450a-5p, hsa-miR-221-5p, hsa-miR-181c-3p, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-590-3p, hsa-miR-590-5p | 86.29 | 91.04 | 91.22 |
| 614 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-335-5p, hsa-miR-1290, hsa-miR-132-3p, hsa-miR-337-5p, hsa-miR-4286, hsa-miR-215-5p, hsa-miR-590-3p, hsa-miR-500a-5p | 86.21 | 92.81 | 91.33 |
| 615 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-1290, hsa-miR-132-3p, hsa-miR-337-5p, hsa-miR-103a-2-5p, hsa-miR-215-5p, hsa-miR-590-3p, hsa-miR-500a-5p, hsa-miR-382-5p | 86.21 | 94.06 | 90.33 |
| 616 | hsa-miR-30e-3p, hsa-miR-545-5p, hsa-miR-548I, hsa-miR-20b-5p, hsa-miR-193b-3p, hsa-miR-410-3p, hsa-miR-433-3p, hsa-miR-331-5p, hsa-miR-181c-3p, hsa-miR-451a, hsa-miR-501-5p, hsa-miR-132-3p, hsa-miR-337-5p, hsa-miR-103a-2-5p, hsa-miR-215-5p, hsa-miR-500a-5p, hsa-miR-382-5p | 86.21 | 94.5 | 91.33 |
| 617 | hsa-miR-20b-5p, hsa-miR-410-3p, hsa-miR-221-5p, hsa-miR-331-5p, hsa-miR-181c-3p, hsa-miR-501-5p, hsa-miR-485-3p, hsa-miR-1290, hsa-miR-4286, hsa-miR-103a-2-5p, hsa-miR-590-5p | 86.21 | 86.96 | 90.22 |
| 618 | hsa-miR-30e-3p, hsa-miR-548I, hsa-miR-20b-5p, hsa-miR-193b-3p, hsa-miR-433-3p, hsa-miR-331-5p, hsa-miR-181c-3p, hsa-miR-451a, hsa-miR-491-5p, hsa-miR-132-3p, hsa-miR-337-5p, hsa-miR-103a-2-5p, hsa-miR-215-5p, hsa-miR-500a-5p, hsa-miR-382-5p | 86.21 | 90.06 | 92.44 |
| 619 | hsa-miR-30e-3p, hsa-miR-548I, hsa-miR-450a-5p, hsa-miR-433-3p, hsa-miR-331-5p, hsa-miR-451a, hsa-miR-491-5p, hsa-miR-132-3p, hsa-miR-103a-2-5p, hsa-miR-215-5p, hsa-miR-590-3p, hsa-miR-500a-5p | 86.21 | 88.93 | 92.44 |
| 620 | hsa-miR-30e-3p, hsa-miR-20b-5p, hsa-miR-450a-5p, hsa-miR-410-3p, hsa-miR-433-3p, hsa-miR-331-5p, hsa-miR-181c-3p, hsa-miR-491-5p, hsa-miR-1290, hsa-miR-4286, hsa-miR-215-5p, hsa-miR-590-3p, hsa-miR-500a-5p, hsa-miR-382-5p | 86.21 | 92.89 | 91.33 |
| 621 | hsa-miR-30e-3p, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-337-5p, hsa-miR-4286, hsa-miR-215-5p, hsa-miR-590-3p, hsa-miR-500a-5p | 86.21 | 90.46 | 92.44 |
| 622 | hsa-miR-545-5p, hsa-miR-548I, hsa-miR-20b-5p, hsa-miR-193b-3p, hsa-miR-450a-5p, hsa-miR-410-3p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-103a-2-5p, hsa-miR-215-5p, hsa-miR-590-3p, hsa-miR-500a-5p, hsa-miR-382-5p | 86.21 | 89.56 | 93.44 |
| 623 | hsa-miR-30e-3p, hsa-miR-545-5p, hsa-miR-548l, hsa-miR-20b-5p, hsa-miR-193b-3p, hsa-miR-450a-5p, hsa-miR-410-3p, hsa-miR-433-3p, hsa-miR-331-5p, hsa-miR-181c-3p, hsa-miR-451a, hsa-miR-501-5p, hsa-miR-132-3p, hsa-miR-337-5p, hsa-miR-103a-2-5p, hsa-miR-215-5p, hsa-miR-500a-5p, hsa-miR-382-5p | 86.21 | 94.13 | 91.33 |
| 624 | hsa-miR-30e-3p, hsa-miR-548l, hsa-miR-20b-5p, hsa-miR-193b-3p, hsa-miR-450a-5p, hsa-miR-181c-3p, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-500a-5p, hsa-miR-382-5p | 86.21 | 88.74 | 95.78 |
| 625 | hsa-miR-30e-3p, hsa-miR-548I, hsa-miR-20b-5p, hsa-miR-193b-3p, hsa-miR-450a-5p, hsa-miR-491-5p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-590-5p | 86.14 | 92.2 | 91.44 |
| 626 | hsa-miR-30e-3p, hsa-miR-548I, hsa-miR-193b-3p, hsa-miR-410-3p, hsa-miR-331-5p, hsa-miR-181c-3p, hsa-miR-451a, hsa-miR-501-5p, hsa-miR-335-5p, hsa-miR-337-5p, hsa-miR-4286, hsa-miR-215-5p, hsa-miR-500a-5p, hsa-miR-590-5p | 86.14 | 89.19 | 92.33 |
| 627 | hsa-miR-30e-3p, hsa-miR-545-5p, hsa-miR-548I, hsa-miR-20b-5p, hsa-miR-193b-3p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-337-5p, hsa-miR-4286, hsa-miR-215-5p, hsa-miR-590-3p, hsa-miR-500a-5p | 86.14 | 92.2 | 91.33 |
| 628 | hsa-miR-30e-3p, hsa-miR-193b-3p, hsa-miR-450a-5p, hsa-miR-181c-3p, hsa-miR-451a, hsa-miR-491-5p, hsa-miR-501-5p, hsa-miR-4732-5p, hsa-miR-485-3p, hsa-miR-1290, hsa-miR-132-3p, hsa-miR-337-5p, hsa-miR-4286, hsa-miR-215-5p, hsa-miR-590-3p, hsa-miR-500a-5p | 86.14 | 89.91 | 91.33 |
| 629 | hsa-miR-30e-3p, hsa-miR-548I, hsa-miR-20b-5p, hsa-miR-193b-3p, hsa-miR-450a-5p, hsa-miR-491-5p, hsa-miR-485-3p, hsa-miR-1290, hsa-miR-132-3p, hsa-miR-337-5p, hsa-miR-4286, hsa-miR-103a-2-5p, hsa-miR-590-3p, hsa-miR-590-5p | 86.06 | 92.94 | 90.22 |
| 630 | hsa-miR-30e-3p, hsa-miR-548l, hsa-miR-20b-5p, hsa-miR-193b-3p, hsa-miR-410-3p, hsa-miR-491-5p, hsa-miR-485-3p, hsa-miR-1290, hsa-miR-132-3p, hsa-miR-337-5p, hsa-miR-103a-2-5p, hsa-miR-215-5p, hsa-miR-500a-5p | 86.06 | 89.24 | 90.22 |
| 631 | hsa-miR-30e-3p, hsa-miR-545-5p, hsa-miR-548I, hsa-miR-20b-5p, hsa-miR-193b-3p, hsa-miR-410-3p, hsa-miR-181c-3p, hsa-miR-451a, hsa-miR-491-5p, hsa-miR-485-3p, hsa-miR-1290, hsa-miR-132-3p, hsa-miR-337-5p, hsa-miR-103a-2-5p, hsa-miR-215-5p, hsa-miR-500a-5p | 85.98 | 89.3 | 90.22 |
| 632 | hsa-miR-30e-3p, hsa-miR-548I, hsa-miR-433-3p, hsa-miR-181c-3p, hsa-miR-501-5p, hsa-miR-4732-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-590-5p | 85.98 | 91.2 | 90.22 |
| 633 | hsa-miR-545-5p, hsa-miR-548I, hsa-miR-20b-5p, hsa-miR-410-3p, hsa-miR-221-5p, hsa-miR-331-5p, hsa-miR-181c-3p, hsa-miR-501-5p, hsa-miR-4732-5p, hsa-miR-132-3p, hsa-miR-4286, hsa-miR-590-3p, hsa-miR-590-5p | 85.3 | 90.41 | 91.22 |
| 634 | hsa-miR-20b-5p, hsa-miR-193b-3p, hsa-miR-450a-5p, hsa-miR-451a, hsa-miR-501-5p, hsa-miR-4732-5p, hsa-miR-485-3p, hsa-miR-335-5p, hsa-miR-1290, hsa-miR-590-5p, hsa-miR-382-5p | 85.5 | 88.61 | 93.56 |
| 635 | hsa-miR-30e-3p, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-337-5p, hsa-miR-4286, hsa-miR-215-5p, hsa-miR-590-3p, hsa-miR-500a-5p | 85.5 | 90.48 | 91.33 |
| 636 | hsa-miR-30e-3p, hsa-miR-545-5p, hsa-miR-20b-5p, hsa-miR-193b-3p, hsa-miR-450a-5p, hsa-miR-410-3p, hsa-miR-221-5p, hsa-miR-181c-3p, hsa-miR-501-5p, hsa-miR-4732-5p, hsa-miR-485-3p, hsa-miR-335-5p, hsa-miR-132-3p, hsa-miR-4286, hsa-miR-590-3p, hsa-miR-590-5p | 85.5 | 89.67 | 90.22 |
| 637 | hsa-miR-548I, hsa-miR-20b-5p, hsa-miR-410-3p, hsa-miR-221-5p, hsa-miR-331-5p, hsa-miR-181c-3p, hsa-miR-501-5p, hsa-miR-1290, hsa-miR-4286, hsa-miR-103a-2-5p, hsa-miR-215-5p, hsa-miR-590-5p, hsa-miR-382-5p | 85.5 | 88.13 | 90.33 |
| 638 | hsa-miR-30e-3p, hsa-miR-545-5p, hsa-miR-20b-5p, hsa-miR-410-3p, hsa-miR-221-5p, hsa-miR-331-5p, hsa-miR-181c-3p, hsa-miR-501-5p, hsa-miR-485-3p, hsa-miR-1290, hsa-miR-132-3p, hsa-miR-4286, hsa-miR-590-5p, hsa-miR-382-5p | 85.8 | 88.19 | 90.33 |
| 639 | hsa-miR-30e-3p, hsa-miR-548I, hsa-miR-20b-5p, hsa-miR-193b-3p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-132-3p, hsa-miR-337-5p, hsa-miR-4286, hsa-miR-215-5p, hsa-miR-590-3p, hsa-miR-500a-5p | 85.8 | 93.31 | 90.33 |
| 640 | hsa-miR-30e-3p, hsa-miR-545-5p, hsa-miR-548I, hsa-miR-20b-5p, hsa-miR-450a-5p, hsa-miR-410-3p, hsa-miR-221-5p, hsa-miR-181c-3p, hsa-miR-501-5p, hsa-miR-485-3p, hsa-miR-335-5p, hsa-miR-1290, hsa-miR-132-3p, hsa-miR-4286, hsa-miR-103a-2-5p, hsa-miR-590-5p, hsa-miR-382-5p | 85.8 | 87.7 | 90.33 |
| 641 | hsa-miR-548I, hsa-miR-20b-5p, hsa-miR-193b-3p, hsa-miR-410-3p, hsa-miR-433-3p, hsa-miR-331-5p, hsa-miR-181c-3p, hsa-miR-451a, hsa-miR-132-3p, hsa-miR-337-5p, hsa-miR-103a-2-5p, hsa-miR-215-5p, hsa-miR-500a-5p, hsa-miR-382-5p | 85.8 | 92.22 | 92.44 |
| 642 | hsa-miR-545-5p, hsa-miR-20b-5p, hsa-miR-193b-3p, hsa-miR-410-3p, hsa-miR-433-3p, hsa-miR-331-5p, hsa-miR-501-5p, hsa-miR-4732-5p, hsa-miR-485-3p, hsa-miR-335-5p, hsa-miR-337-5p, hsa-miR-4286, hsa-miR-103a-2-5p | 85. | 86.89 | 90.22 |
| 643 | hsa-miR-30e-3p, hsa-miR-548I, hsa-miR-450a-5p, hsa-miR-331-5p, hsa-miR-491-5p, hsa-miR-501-5p, hsa-miR-4732-5p, hsa-miR-335-5p, hsa-miR-132-3p, hsa-miR-337-5p, hsa-miR-4286, hsa-miR-590-3p, hsa-miR-382-5p | 85. | 91.57 | 91.33 |
| 644 | hsa-miR-30e-3p, hsa-miR-548I, hsa-miR-20b-5p, hsa-miR-450a-5p, hsa-miR-331-5p, hsa-miR-451a, hsa-miR-491-5p, hsa-miR-132-3p, hsa-miR-103a-2-5p, hsa-miR-215-5p, hsa-miR-590-3p, hsa-miR-500a-5p | 85. | 88.5 | 91.33 |
| 645 | hsa-miR-30e-3p, hsa-miR-548I, hsa-miR-450a-5p, hsa-miR-433-3p, hsa-miR-451a, hsa-miR-491-5p, hsa-miR-485-3p, hsa-miR-1290, hsa-miR-132-3p, hsa-miR-337-5p, hsa-miR-103a-2-5p, hsa-miR-215-5p, hsa-miR-590-3p, hsa-miR-500a-5p | 85. | 88.74 | 91.33 |
| 646 | hsa-miR-548I, hsa-miR-433-3p, hsa-miR-331-5p, hsa-miR-181c-3p, hsa-miR-1290, hsa-miR-103a-2-5p, hsa-miR-215-5p, hsa-miR-500a-5p, hsa-miR-590-5p, hsa-miR-382-5p | 85. | 84.74 | 92.44 |
| 647 | hsa-miR-30e-3p, hsa-miR-545-5p, hsa-miR-20b-5p, hsa-miR-450a-5p, hsa-miR-331-5p, hsa-miR-501-5p, hsa-miR-335-5p, hsa-miR-1290, hsa-miR-4286, hsa-miR-590-3p, hsa-miR-590-5p | 85. | 88.81 | 89.11 |
| 648 | hsa-miR-450a-5p, hsa-miR-433-3p, hsa-miR-181c-3p, hsa-miR-451a, hsa-miR-501-5p, hsa-miR-4732-5p, hsa-miR-485-3p, hsa-miR-1290, hsa-miR-132-3p, hsa-miR-337-5p, hsa-miR-4286, hsa-miR-215-5p, hsa-miR-590-5p | 85. | 88.93 | 92.44 |
| 649 | hsa-miR-30e-3p, hsa-miR-545-5p, hsa-miR-548I, hsa-miR-20b-5p, hsa-miR-450a-5p, hsa-miR-433-3p, hsa-miR-331-5p, hsa-miR-491-5p, hsa-miR-132-3p, hsa-miR-337-5p, hsa-miR-103a-2-5p, hsa-miR-215-5p, hsa-miR-590-3p, hsa-miR-500a-5p | 85.23 | 90.67 | 89.22 |
| 650 | hsa-miR-30e-3p, hsa-miR-548l, hsa-miR-20b-5p, hsa-miR-193b-3p, hsa-miR-450a-5p, hsa-miR-491-5p, hsa-miR-485-3p, hsa-miR-1290, hsa-miR-132-3p, hsa-miR-337-5p, hsa-miR-103a-2-5p, hsa-miR-590-3p, hsa-miR-590-5p, hsa-miR-382-5p | 85.23 | 84.93 | 91.11 |
| 651 | hsa-miR-30e-3p, hsa-miR-545-5p, hsa-miR-548l, hsa-miR-20b-5p, hsa-miR-193b-3p, hsa-miR-410-3p, hsa-miR-433-3p, hsa-miR-331-5p, hsa-miR-181c-3p, hsa-miR-451a, hsa-miR-335-5p, hsa-miR-337-5p, hsa-miR-103a-2-5p, hsa-miR-215-5p, hsa-miR-500a-5p, hsa-miR-382-5p | 85.23 | 93.89 | 90.22 |
| 652 | hsa-miR-30e-3p, hsa-miR-548I, hsa-miR-20b-5p, hsa-miR-193b-3p, hsa-miR-491-5p, hsa-miR-485-3p, hsa-miR-1290, hsa-miR-132-3p, hsa-miR-337-5p, hsa-miR-4286, hsa-miR-103a-2-5p, hsa-miR-590-5p | 85.15 | 89.06 | 89.11 |
| 653 | hsa-miR-30e-3p, hsa-miR-548l, hsa-miR-20b-5p, hsa-miR-193b-3p, hsa-miR-410-3p, hsa-miR-451a, hsa-miR-485-3p, hsa-miR-1290, hsa-miR-132-3p, hsa-miR-337-5p, hsa-miR-103a-2-5p, hsa-miR-215-5p, hsa-miR-500a-5p, hsa-miR-382-5p | 84.5 | 87.81 | 90.33 |
| 654 | hsa-miR-30e-3p, hsa-miR-548l, hsa-miR-20b-5p, hsa-miR-450a-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-4286, hsa-miR-215-5p, hsa-miR-590-3p, hsa-miR-500a-5p | 84.5 | 90.17 | 93.56 |
| 655 | hsa-miR-30e-3p, hsa-miR-548I, hsa-miR-20b-5p, hsa-miR-193b-3p, hsa-miR-491-5p, hsa-miR-485-3p, hsa-miR-1290, hsa-miR-132-3p, hsa-miR-337-5p, hsa-miR-4286, hsa-miR-103a-2-5p, hsa-miR-590-5p, hsa-miR-382-5p | 84.5 | 93.19 | 88.22 |
| 656 | hsa-miR-30e-3p, hsa-miR-548I, hsa-miR-20b-5p, hsa-miR-193b-3p, hsa-miR-450a-5p, hsa-miR-491-5p, hsa-miR-501-5p, hsa-miR-485-3p, hsa-miR-335-5p, hsa-miR-1290, hsa-miR-132-3p, hsa-miR-337-5p, hsa-miR-4286, hsa-miR-590-3p, hsa-miR-500a-5p | 84.7 | 91.02 | 89.22 |
| 657 | hsa-miR-545-5p, hsa-miR-548l, hsa-miR-20b-5p, hsa-miR-450a-5p, hsa-miR-410-3p, hsa-miR-221-5p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-103a-2-5p, hsa-miR-215-5p, hsa-miR-590-3p, hsa-miR-500a-5p | 84.7 | 91.96 | 91.33 |
| 658 | hsa-miR-545-5p, hsa-miR-548I, hsa-miR-20b-5p, hsa-miR-193b-3p, hsa-miR-410-3p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-103a-2-5p, hsa-miR-215-5p, hsa-miR-500a-5p | 84.7 | 87.39 | 92.44 |
| 659 | hsa-miR-30e-3p, hsa-miR-548I, hsa-miR-20b-5p, hsa-miR-193b-3p, hsa-miR-433-3p, hsa-miR-331-5p, hsa-miR-491-5p, hsa-miR-132-3p, hsa-miR-337-5p, hsa-miR-103a-2-5p, hsa-miR-215-5p, hsa-miR-500a-5p, hsa-miR-382-5p | 84.9 | 86.83 | 90.22 |
| 660 | hsa-miR-30e-3p, hsa-miR-548I, hsa-miR-20b-5p, hsa-miR-193b-3p, hsa-miR-450a-5p, hsa-miR-433-3p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-337-5p, hsa-miR-4286, hsa-miR-215-5p, hsa-miR-590-3p, hsa-miR-500a-5p | 84.9 | 93.81 | 89.11 |
| 661 | hsa-miR-30e-3p, hsa-miR-548I, hsa-miR-20b-5p, hsa-miR-193b-3p, hsa-miR-410-3p, hsa-miR-491-5p, hsa-miR-485-3p, hsa-miR-1290, hsa-miR-132-3p, hsa-miR-337-5p, hsa-miR-103a-2-5p, hsa-miR-590-5p | 84.9 | 85.85 | 90.11 |
| 662 | hsa-miR-30e-3p, hsa-miR-545-5p, hsa-miR-548I, hsa-miR-20b-5p, hsa-miR-331-5p, hsa-miR-181c-3p, hsa-miR-491-5p, hsa-miR-485-3p, hsa-miR-335-5p, hsa-miR-500a-5p, hsa-miR-590-5p, hsa-miR-382-5p | 84.9 | 89.11 | 89.11 |
| 663 | hsa-miR-30e-3p, hsa-miR-548I, hsa-miR-20b-5p, hsa-miR-193b-3p, hsa-miR-433-3p, hsa-miR-331-5p, hsa-miR-451a, hsa-miR-491-5p, hsa-miR-132-3p, hsa-miR-337-5p, hsa-miR-103a-2-5p, hsa-miR-215-5p, hsa-miR-500a-5p, hsa-miR-382-5p | 84.9 | 86.83 | 90.22 |
| 664 | hsa-miR-545-5p, hsa-miR-548I, hsa-miR-20b-5p, hsa-miR-450a-5p, hsa-miR-410-3p, hsa-miR-433-3p, hsa-miR-331-5p, hsa-miR-181c-3p, hsa-miR-451a, hsa-miR-501-5p, hsa-miR-132-3p, hsa-miR-337-5p, hsa-miR-103a-2-5p, hsa-miR-215-5p, hsa-miR-590-3p, hsa-miR-500a-5p, hsa-miR-382-5p | 84.9 | 92.04 | 92.33 |
| 665 | hsa-miR-30e-3p, hsa-miR-548I, hsa-miR-20b-5p, hsa-miR-450a-5p, hsa-miR-433-3p, hsa-miR-331-5p, hsa-miR-451a, hsa-miR-491-5p, hsa-miR-132-3p, hsa-miR-103a-2-5p, hsa-miR-215-5p, hsa-miR-590-3p, hsa-miR-500a-5p | 84.9 | 89 | 90.22 |
| 666 | hsa-miR-30e-3p, hsa-miR-548I, hsa-miR-20b-5p, hsa-miR-193b-3p, hsa-miR-410-3p, hsa-miR-433-3p, hsa-miR-331-5p, hsa-miR-181c-3p, hsa-miR-491-5p, hsa-miR-335-5p, hsa-miR-337-5p, hsa-miR-103a-2-5p, hsa-miR-215-5p, hsa-miR-500a-5p, hsa-miR-382-5p | 84.9 | 90.56 | 91.33 |
| 667 | hsa-miR-545-5p, hsa-miR-548I, hsa-miR-20b-5p, hsa-miR-193b-3p, hsa-miR-450a-5p, hsa-miR-410-3p, hsa-miR-433-3p, hsa-miR-331-5p, hsa-miR-451a, hsa-miR-501-5p, hsa-miR-132-3p, hsa-miR-337-5p, hsa-miR-103a-2-5p, hsa-miR-215-5p, hsa-miR-500a-5p, hsa-miR-382-5p | 84.2 | 88.63 | 89.11 |
| 668 | hsa-miR-30e-3p, hsa-miR-545-5p, hsa-miR-491-5p, hsa-miR-4732-5p, hsa-miR-485-3p, hsa-miR-1290, hsa-miR-132-3p, hsa-miR-103a-2-5p, hsa-miR-590-3p, hsa-miR-382-5p | 84.2 | 88 | 92.33 |
| 669 | hsa-miR-30e-3p, hsa-miR-548I, hsa-miR-20b-5p, hsa-miR-193b-3p, hsa-miR-433-3p, hsa-miR-331-5p, hsa-miR-491-5p, hsa-miR-335-5p, hsa-miR-337-5p, hsa-miR-103a-2-5p, hsa-miR-215-5p, hsa-miR-382-5p | 84.24 | 86.72 | 94.44 |
| 670 | hsa-miR-30e-3p, hsa-miR-548I, hsa-miR-20b-5p, hsa-miR-193b-3p, hsa-miR-451a, hsa-miR-491-5p, hsa-miR-485-3p, hsa-miR-1290, hsa-miR-337-5p, hsa-miR-103a-2-5p, hsa-miR-590-5p | 84.24 | 89.5 | 89 |
| 671 | hsa-miR-30e-3p, hsa-miR-548I, hsa-miR-193b-3p, hsa-miR-433-3p, hsa-miR-181c-3p, hsa-miR-501-5p, hsa-miR-4732-5p, hsa-miR-485-3p, hsa-miR-1290, hsa-miR-132-3p, hsa-miR-4286, hsa-miR-103a-2-5p, hsa-miR-215-5p, hsa-miR-590-3p, hsa-miR-590-5p | 84.17 | 91.96 | 89.11 |
| 672 | hsa-miR-548I, hsa-miR-20b-5p, hsa-miR-193b-3p, hsa-miR-450a-5p, hsa-miR-221-5p, hsa-miR-451a, hsa-miR-491-5p, hsa-miR-485-3p, hsa-miR-1290, hsa-miR-132-3p, hsa-miR-337-5p, hsa-miR-103a-2-5p, hsa-miR-215-5p, hsa-miR-590-3p, hsa-miR-500a-5p | 83.4 | 85.96 | 92.44 |
| 673 | hsa-miR-545-5p, hsa-miR-548I, hsa-miR-20b-5p, hsa-miR-193b-3p, hsa-miR-410-3p, hsa-miR-451a, hsa-miR-485-3p, hsa-miR-1290, hsa-miR-132-3p, hsa-miR-337-5p, hsa-miR-103a-2-5p, hsa-miR-215-5p, hsa-miR-500a-5p, hsa-miR-382-5p | 83.4 | 87.39 | 90.22 |
| 674 | hsa-miR-545-5p, hsa-miR-548I, hsa-miR-20b-5p, hsa-miR-193b-3p, hsa-miR-410-3p, hsa-miR-181c-3p, hsa-miR-451a, hsa-miR-491-5p, hsa-miR-485-3p, hsa-miR-1290, hsa-miR-132-3p, hsa-miR-337-5p, hsa-miR-103a-2-5p, hsa-miR-215-5p, hsa-miR-500a-5p | 83.4 | 86.09 | 90.33 |
| 675 | hsa-miR-545-5p, hsa-miR-20b-5p, hsa-miR-193b-3p, hsa-miR-410-3p, hsa-miR-433-3p, hsa-miR-221-5p, hsa-miR-181c-3p, hsa-miR-491-5p, hsa-miR-4732-5p, hsa-miR-1290, hsa-miR-132-3p, hsa-miR-4286, hsa-miR-103a-2-5p, hsa-miR-215-5p | 83.6 | 85.41 | 89.11 |
| 676 | hsa-miR-30e-3p, hsa-miR-545-5p, hsa-miR-20b-5p, hsa-miR-450a-5p, hsa-miR-331-5p, hsa-miR-451a, hsa-miR-491-5p, hsa-miR-4732-5p, hsa-miR-485-3p, hsa-miR-103a-2-5p, hsa-miR-215-5p, hsa-miR-590-5p, hsa-miR-382-5p | 83.8 | 87.33 | 90 |
| 677 | hsa-miR-30e-3p, hsa-miR-548l, hsa-miR-20b-5p, hsa-miR-193b-3p, hsa-miR-491-5p, hsa-miR-485-3p, hsa-miR-1290, hsa-miR-337-5p, hsa-miR-103a-2-5p, hsa-miR-590-5p, hsa-miR-382-5p | 83.8 | 89.43 | 89 |
| 678 | hsa-miR-30e-3p, hsa-miR-548I, hsa-miR-20b-5p, hsa-miR-193b-3p, hsa-miR-410-3p, hsa-miR-491-5p, hsa-miR-485-3p, hsa-miR-1290, hsa-miR-132-3p, hsa-miR-337-5p, hsa-miR-103a-2-5p, hsa-miR-215-5p, hsa-miR-590-5p | 83.8 | 90.06 | 89 |
| 679 | hsa-miR-30e-3p, hsa-miR-548I, hsa-miR-20b-5p, hsa-miR-193b-3p, hsa-miR-491-5p, hsa-miR-485-3p, hsa-miR-1290, hsa-miR-132-3p, hsa-miR-337-5p, hsa-miR-103a-2-5p, hsa-miR-500a-5p, hsa-miR-590-5p | 83.8 | 85.41 | 92.44 |
| 680 | hsa-miR-30e-3p, hsa-miR-548I, hsa-miR-20b-5p, hsa-miR-193b-3p, hsa-miR-433-3p, hsa-miR-331-5p, hsa-miR-451a, hsa-miR-491-5p, hsa-miR-335-5p, hsa-miR-337-5p, hsa-miR-103a-2-5p, hsa-miR-215-5p, hsa-miR-500a-5p, hsa-miR-382-5p | 83.1 | 86.41 | 93.44 |
| 681 | hsa-miR-30e-3p, hsa-miR-548l, hsa-miR-20b-5p, hsa-miR-193b-3p, hsa-miR-451a, hsa-miR-491-5p, hsa-miR-485-3p, hsa-miR-1290, hsa-miR-132-3p, hsa-miR-337-5p, hsa-miR-103a-2-5p, hsa-miR-215-5p, hsa-miR-590-5p | 83.1 | 87.7 | 87.89 |
| 682 | hsa-miR-545-5p, hsa-miR-548I, hsa-miR-20b-5p, hsa-miR-193b-3p, hsa-miR-450a-5p, hsa-miR-410-3p, hsa-miR-221-5p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-103a-2-5p, hsa-miR-215-5p, hsa-miR-590-3p, hsa-miR-500a-5p | 82.88 | 90.28 | 88.22 |
| 683 | hsa-miR-545-5p, hsa-miR-548I, hsa-miR-20b-5p, hsa-miR-193b-3p, hsa-miR-410-3p, hsa-miR-181c-3p, hsa-miR-451a, hsa-miR-491-5p, hsa-miR-485-3p, hsa-miR-1290, hsa-miR-132-3p, hsa-miR-337-5p, hsa-miR-103a-2-5p, hsa-miR-215-5p, hsa-miR-382-5p | 82.8 | 86.09 | 91.22 |
| 684 | hsa-miR-545-5p, hsa-miR-548I, hsa-miR-20b-5p, hsa-miR-410-3p, hsa-miR-451a, hsa-miR-501-5p, hsa-miR-485-3p, hsa-miR-1290, hsa-miR-132-3p, hsa-miR-337-5p, hsa-miR-103a-2-5p, hsa-miR-382-5p | 82.73 | 83.69 | 92.44 |
| 685 | hsa-miR-545-5p, hsa-miR-548I, hsa-miR-20b-5p, hsa-miR-410-3p, hsa-miR-181c-3p, hsa-miR-451a, hsa-miR-501-5p, hsa-miR-485-3p, hsa-miR-1290, hsa-miR-132-3p, hsa-miR-337-5p, hsa-miR-103a-2-5p, hsa-miR-215-5p, hsa-miR-500a-5p, hsa-miR-382-5p | 82.5 | 85.35 | 91.33 |
| 686 | hsa-miR-30e-3p, hsa-miR-548I, hsa-miR-20b-5p, hsa-miR-193b-3p, hsa-miR-451a, hsa-miR-491-5p, hsa-miR-485-3p, hsa-miR-1290, hsa-miR-132-3p, hsa-miR-337-5p, hsa-miR-103a-2-5p, hsa-miR-382-5p | 82.5 | 87.02 | 89.11 |
| 687 | hsa-miR-30e-3p, hsa-miR-545-5p, hsa-miR-548I, hsa-miR-20b-5p, hsa-miR-193b-3p, hsa-miR-410-3p, hsa-miR-451a, hsa-miR-485-3p, hsa-miR-1290, hsa-miR-132-3p, hsa-miR-337-5p, hsa-miR-103a-2-5p, hsa-miR-500a-5p, hsa-miR-382-5p | 82.5 | 89.11 | 91.44 |
| 688 | hsa-miR-30e-3p, hsa-miR-548I, hsa-miR-20b-5p, hsa-miR-193b-3p, hsa-miR-433-3p, hsa-miR-331-5p, hsa-miR-451a, hsa-miR-491-5p, hsa-miR-132-3p, hsa-miR-337-5p, hsa-miR-103a-2-5p, hsa-miR-215-5p, hsa-miR-500a-5p, hsa-miR-590-5p, hsa-miR-382-5p | 82.8 | 88.44 | 90.11 |
| 689 | hsa-miR-30e-3p, hsa-miR-450a-5p, hsa-miR-181c-3p, hsa-miR-451a, hsa-miR-501-5p, hsa-miR-4732-5p, hsa-miR-335-5p, hsa-miR-1290, hsa-miR-337-5p, hsa-miR-215-5p, hsa-miR-590-3p, hsa-miR-500a-5p, hsa-miR-590-5p, hsa-miR-382-5p | 82.00 | 89.59 | 85.67 |
| 690 | hsa-miR-545-5p, hsa-miR-548I, hsa-miR-20b-5p, hsa-miR-193b-3p, hsa-miR-410-3p, hsa-miR-451a, hsa-miR-485-3p, hsa-miR-1290, hsa-miR-132-3p, hsa-miR-337-5p, hsa-miR-103a-2-5p | 81.89 | 84.48 | 89.11 |
| 691 | hsa-miR-331-5p, hsa-miR-181c-3p, hsa-miR-451a, hsa-miR-491-5p, hsa-miR-501-5p, hsa-miR-485-3p, hsa-miR-335-5p, hsa-miR-1290, hsa-miR-337-5p, hsa-miR-590-3p, hsa-miR-500a-5p, hsa-miR-590-5p, hsa-miR-382-5p | 81.89 | 83.93 | 91.33 |
| 692 | hsa-miR-193b-3p, hsa-miR-450a-5p, hsa-miR-410-3p, hsa-miR-181c-3p, hsa-miR-501-5p, hsa-miR-485-3p, hsa-miR-1290, hsa-miR-132-3p, hsa-miR-337-5p, hsa-miR-4286, hsa-miR-103a-2-5p, hsa-miR-215-5p, hsa-miR-500a-5p | 81.89 | 84.3 | 94.67 |
| 693 | hsa-miR-545-5p, hsa-miR-548I, hsa-miR-20b-5p, hsa-miR-193b-3p, hsa-miR-410-3p, hsa-miR-451a, hsa-miR-491-5p, hsa-miR-485-3p, hsa-miR-1290, hsa-miR-132-3p, hsa-miR-337-5p, hsa-miR-103a-2-5p, hsa-miR-215-5p, hsa-miR-500a-5p | 81.82 | 83.74 | 89.11 |
| 694 | hsa-miR-545-5p, hsa-miR-548I, hsa-miR-20b-5p, hsa-miR-410-3p, hsa-miR-181c-3p, hsa-miR-451a, hsa-miR-485-3p, hsa-miR-1290, hsa-miR-132-3p, hsa-miR-337-5p, hsa-miR-103a-2-5p, hsa-miR-215-5p, hsa-miR-500a-5p, hsa-miR-382-5p | 81.82 | 85.85 | 91.22 |
| 695 | hsa-miR-545-5p, hsa-miR-548I, hsa-miR-450a-5p, hsa-miR-181c-3p, hsa-miR-491-5p, hsa-miR-335-5p, hsa-miR-132-3p, hsa-miR-337-5p, hsa-miR-590-3p, hsa-miR-500a-5p | 80.98 | 83.74 | 93.44 |
| 696 | hsa-miR-548I, hsa-miR-193b-3p, hsa-miR-501-5p, hsa-miR-132-3p, hsa-miR-4286, hsa-miR-103a-2-5p, hsa-miR-215-5p | 80.91 | 85.54 | 94.56 |
| 699 | combination 389+ rs76563024 | 80.87 | 78.71 | 100.0 |
| 700 | combination 390+ rs76563024 | 80.87 | 80.31 | 98.89 |
| 701 | combination 392+ rs76563024 | 81.74 | 79.08 | 97.78 |
| 702 | combination 393+ rs12934455 | 85.22 | 84.73 | 96.67 |
| 703 | combination 395+ rs12934455 | 85.22 | 79.65 | 95.56 |
| 704 | combination 397+ rs71384446 | 86.09 | 87.88 | 97.78 |
| 705 | combiantion 399 + rs7203642 | 86.96 | 87.09 | 95.56 |
| 706 | combination 400 + rs28688991 | 86.96 | 85.75 | 94.44 |
| 707 | combination 652 + rs7203642 | 85.22 | 92.38 | 94.44 |
| 708 | combination 653 +rs7203642 | 85.22 | 92.38 | 92.38 |
| 709 | combination 654 + rs7203642 | 85.22 | 92.38 | 92.38 |

This is the list of the best 30 mRNAs selected by the algorithm sorted by importance for tumour stage: hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p, hsa-miR-181c-5p, hsa-miR-4306, hsa-miR-27a-3p, hsa-miR-134-5p, hsa-miR-1296-5p, and hsa-miR-15a-5p. The inventors combined these miRNAs, together or not with age and UMOD SNP variant, creating unique combinations leading to a further significant increase in sensitivity, specificity, accuracy and predictive power for tumour stage (Table 17).

**Table 17. Combinations that allow higher sensitivity, specificity, accuracy and predictive power for tumour stage.**

| N | Combinations | Accuracy | ROCAUC | Recall |
|---|---|---|---|---|
| 711 | hsa-miR-769-5p, hsa-miR-27b-3p | 74.74 | 80.82 | 23.00 |
| 712 | hsa-miR-769-5p, hsa-miR-27b-3p, hsa-miR-320a-3p | 78.19 | 83.28 | 50.67 |
| 713 | hsa-miR-769-5p, hsa-miR-27b-3p, hsa-miR-320a-3p, hsa-miR-629-5p | 78.13 | 86.31 | 58.00 |
| 714 | hsa-miR-769-5p, hsa-miR-27b-3p, hsa-miR-320a-3p, hsa-miR-629-5p, hsa-miR-100-5p | 79.24 | 87.54 | 62.67 |
| 715 | hsa-miR-769-5p, hsa-miR-27b-3p, hsa-miR-320a-3p, hsa-miR-629-5p, hsa-miR-100-5p, hsa-miR-183-3p | 80.23 | 87.59 | 66.67 |
| 716 | hsa-miR-769-5p, hsa-miR-27b-3p, hsa-miR-320a-3p, hsa-miR-629-5p, hsa-miR-100-5p, hsa-miR-183-3p, hsa-miR-224-5p | 85.85 | 89.38 | 70.0 |
| 717 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p | 86.9 | 90.46 | 69.33 |
| 718 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p | 88.01 | 91.23 | 72.67 |
| 719 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p | 89.12 | 93.03 | 72.67 |
| 720 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p | 88.01 | 93.23 | 76.67 |
| 721 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p | 89.12 | 92.92 | 69.33 |
| 722 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p | 90.06 | 95.64 | 81.33 |
| 723 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p | 85.85 | 95.18 | 73.33 |
| 724 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p | 89.12 | 94.87 | 65.33 |
| 725 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p | 86.96 | 94.05 | 68.67 |
| 726 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p | 85.85 | 92.26 | 68.67 |
| 727 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p | 84.8 | 93.95 | 80.67 |
| 728 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136 | 85.73 | 94.05 | 72.67 |
| 729 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p | 86.84 | 94.67 | 72.67 |
| 730 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p | 86.9 | 94.1 | 72.67 |
| 731 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p | 88.01 | 95.59 | 68.67 |
| 732 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p, hsa-miR-337-5p | 86.9 | 94.92 | 68.67 |
| 733 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p | 86.9 | 94.92 | 76.67 |
| 734 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p, hsa-miR-181c-5p | 86.9 | 94.72 | 76.67 |
| 735 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p, hsa-miR-181c-5p, hsa-miR-4306 | 88.01 | 92.62 | 76.67 |
| 736 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p, hsa-miR-181c-5p, hsa-miR-4306, hsa-miR-27a-3p | 90.12 | 94.97 | 76.67 |
| 737 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p, hsa-miR-181c-5p, hsa-miR-4306, hsa-miR-27a-3p, hsa-miR-134-5p | 87.95 | 95.18 | 80.67 |
| 738 | hsa-miR-769-5p, hsa-miR-27b-3p, hsa-miR-320a-3p, hsa-miR-629-5p, hsa-miR-100-5p, hsa-miR-183-3p, hsa-miR-224-5p, hsa-let-7i-3p, hsa-miR-652-3p, hsa-miR-140-3p, hsa-miR-22-5p, hsa-miR-548a-3p, hsa-miR-1307-5p, hsa-miR-339-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p, hsa-miR-181c-5p, hsa-miR-4306, hsa-miR-27a-3p, hsa-miR-134-5p, hsa-miR-1296-5p | 90.12 | 95.38 | 80.67 |
| 739 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 95.67 | 98.33 | 91.67 |
| 740 | hsa-miR-22-5p, hsa-miR-1307-5p, hsa-miR-339-3p, hsa-miR-10b-5p, hsa-miR-12136, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 92.56 | 97.86 | 86.67 |
| 741 | hsa-miR-22-5p, hsa-miR-1307-5p, hsa-miR-10b-5p, hsa-miR-12136, hsa-miR-34a-3p, hsa-miR-337-5p, hsa-miR-188-5p, hsa-miR-15a-5p, hsa-miR-629-5p, hsa-miR-100-5p, hsa-miR-183-3p, hsa-miR-224-5p, hsa-miR-652-3p | 92.56 | 97.38 | 90.00 |
| 742 | hsa-miR-22-5p, hsa-miR-1307-5p, hsa-miR-339-3p, hsa-miR-195-5p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 92.44 | 98.81 | 88.33 |
| 743 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 92.44 | 96.03 | 81.67 |
| 744 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 92.33 | 95.32 | 85.00 |
| 745 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-224-5p, hsa-miR-652-3p, hsa-miR-140-3p | 91.44 | 96.98 | 86.67 |
| 746 | hsa-miR-22-5p, hsa-miR-1307-5p, hsa-miR-339-3p, hsa-miR-12136, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 91.44 | 97.86 | 86.67 |
| 747 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 91.44 | 97.62 | 80.00 |
| 748 | hsa-miR-22-5p, hsa-miR-1307-5p, hsa-miR-339-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 91.44 | 96.19 | 86.67 |
| 749 | hsa-miR-22-5p, hsa-miR-1307-5p, hsa-miR-339-3p, hsa-miR-485-3p, hsa-miR-10b-5p, hsa-miR-12136, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 91.33 | 95.32 | 81.67 |
| 750 | hsa-miR-22-5p, hsa-miR-1307-5p, hsa-miR-339-3p, hsa-miR-485-3p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 91.33 | 95.32 | 81.67 |
| 751 | hsa-miR-22-5p, hsa-miR-1307-5p, hsa-miR-485-3p, hsa-miR-10b-5p, hsa-miR-12136, hsa-miR-337-5p, hsa-miR-188-5p, hsa-miR-134-5p, hsa-miR-15a-5p, hsa-miR-629-5p, hsa-miR-100-5p, hsa-miR-224-5p, hsa-miR-652-3p | 91.33 | 96.75 | 81.67 |
| 752 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 91.33 | 96.9 | 85.00 |
| 753 | hsa-miR-22-5p, hsa-miR-1307-5p, hsa-miR-339-3p, hsa-miR-485-3p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 91.33 | 97.14 | 83.33 |
| 754 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-224-5p, hsa-miR-140-3p | 91.33 | 95.71 | 83.33 |
| 755 | hsa-miR-22-5p, hsa-miR-1307-5p, hsa-miR-339-3p, hsa-miR-485-3p, hsa-miR-10b-5p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 91.33 | 94.21 | 81.67 |
| 756 | hsa-miR-22-5p, hsa-miR-1307-5p, hsa-miR-339-3p, hsa-miR-485-3p, hsa-miR-10b-5p, hsa-miR-12136, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 91.33 | 96.03 | 81.67 |
| 757 | hsa-miR-22-5p, hsa-miR-1307-5p, hsa-miR-339-3p, hsa-miR-195-5p, hsa-miR-485-3p, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 91.33 | 96.27 | 78.33 |
| 758 | hsa-miR-22-5p, hsa-miR-195-5p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-15a-5p, hsa-miR-629-5p, hsa-miR-100-5p, hsa-miR-224-5p, hsa-miR-652-3p | 91.33 | 93.41 | 78.33 |
| 759 | hsa-miR-22-5p, hsa-miR-1307-5p, hsa-miR-339-3p, hsa-miR-485-3p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 91.22 | 94.21 | 81.67 |
| 760 | hsa-miR-22-5p, hsa-miR-10b-5p, hsa-miR-12136, hsa-miR-34a-3p, hsa-miR-337-5p, hsa-miR-188-5p, hsa-miR-4306, hsa-miR-15a-5p, hsa-miR-629-5p, hsa-miR-100-5p, hsa-miR-183-3p, hsa-miR-224-5p, hsa-miR-652-3p | 90.44 | 94.52 | 85.00 |
| 761 | hsa-miR-22-5p, hsa-miR-10b-5p, hsa-miR-12136, hsa-miR-34a-3p, hsa-miR-337-5p, hsa-miR-188-5p, hsa-miR-15a-5p, hsa-miR-629-5p, hsa-miR-100-5p, hsa-miR-183-3p, hsa-miR-224-5p, hsa-miR-652-3p | 90.44 | 96.35 | 85.00 |
| 762 | hsa-miR-22-5p, hsa-miR-548a-3p, hsa-miR-1307-5p, hsa-miR-339-3p, hsa-miR-744-5p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-miR-652-3p, hsa-miR-140-3p | 90.33 | 96.43 | 81.67 |
| 763 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-224-5p, hsa-let-7i-3p, hsa-miR-652-3p | 90.33 | 96.35 | 81.67 |
| 764 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-15a-5p, hsa-miR-629-5p, hsa-miR-100-5p, hsa-miR-183-3p, hsa-miR-224-5p, hsa-let-7i-3p | 90.33 | 97.06 | 85.00 |
| 765 | hsa-miR-22-5p, hsa-miR-1307-5p, hsa-miR-339-3p, hsa-miR-485-3p, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 90.22 | 95.56 | 81.67 |
| 766 | hsa-miR-22-5p, hsa-miR-548a-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-1296-5p, hsa-miR-15a-5p, hsa-miR-100-5p, hsa-miR-183-3p, hsa-miR-224-5p, hsa-miR-652-3p, hsa-miR-140-3p | 90.22 | 92.94 | 73.33 |
| 767 | hsa-miR-22-5p, hsa-miR-1307-5p, hsa-miR-339-3p, hsa-miR-195-5p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 90.22 | 96.75 | 78.33 |
| 768 | hsa-miR-22-5p, hsa-miR-548a-3p, hsa-miR-1307-5p, hsa-miR-10b-5p, hsa-miR-12136, hsa-miR-337-5p, hsa-miR-188-5p, hsa-miR-4306, hsa-miR-134-5p, hsa-miR-15a-5p, hsa-miR-629-5p, hsa-miR-100-5p, hsa-miR-224-5p, hsa-miR-652-3p, hsa-miR-140-3p | 90.22 | 94.44 | 76.67 |
| 769 | hsa-miR-22-5p, hsa-miR-1307-5p, hsa-miR-339-3p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 90.22 | 96.03 | 85.00 |
| 770 | hsa-miR-22-5p, hsa-miR-548a-3p, hsa-miR-1307-5p, hsa-miR-485-3p, hsa-miR-10b-5p, hsa-miR-12136, hsa-miR-337-5p, hsa-miR-188-5p, hsa-miR-4306, hsa-miR-134-5p, hsa-miR-15a-5p, hsa-miR-629-5p, hsa-miR-100-5p, hsa-miR-224-5p, hsa-miR-652-3p, hsa-miR-140-3p | 90.22 | 95.08 | 78.33 |
| 771 | hsa-miR-22-5p, hsa-miR-548a-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-134-5p, hsa-miR-1296-5p, hsa-miR-15a-5p, hsa-miR-100-5p, hsa-miR-224-5p, hsa-miR-652-3p, hsa-miR-140-3p | 90.22 | 91.11 | 73.33 |
| 772 | hsa-miR-22-5p, hsa-miR-1307-5p, hsa-miR-339-3p, hsa-miR-195-5p, hsa-miR-485-3p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 90.22 | 93.49 | 78.33 |
| 773 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-let-7i-3p | 90.22 | 96.51 | 85.00 |
| 774 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 90.22 | 94.76 | 85.00 |
| 775 | hsa-miR-22-5p, hsa-miR-1307-5p, hsa-miR-339-3p, hsa-miR-195-5p, hsa-miR-485-3p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 90.22 | 96.67 | 81.67 |
| 776 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-let-7i-3p, hsa-miR-652-3p | 90.22 | 97.78 | 81.67 |
| 777 | hsa-miR-22-5p, hsa-miR-1307-5p, hsa-miR-339-3p, hsa-miR-485-3p, hsa-miR-10b-5p, hsa-miR-12136, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p | 90.22 | 96.98 | 90.00 |
| 778 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p | 90.22 | 95.56 | 81.67 |
| 779 | hsa-miR-22-5p, hsa-miR-548a-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-1296-5p, hsa-miR-15a-5p, hsa-miR-100-5p, hsa-miR-224-5p, hsa-miR-652-3p, hsa-miR-140-3p | 90.22 | 91.75 | 73.33 |
| 780 | hsa-miR-22-5p, hsa-miR-1307-5p, hsa-miR-485-3p, hsa-miR-12136, hsa-miR-337-5p, hsa-miR-188-5p, hsa-miR-134-5p, hsa-miR-15a-5p, hsa-miR-629-5p, hsa-miR-100-5p, hsa-miR-224-5p, hsa-let-7i-3p, hsa-miR-652-3p | 90.22 | 96.75 | 81.67 |
| 781 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-miR-140-3p | 90.11 | 94.13 | 73.33 |
| 782 | hsa-miR-22-5p, hsa-miR-1307-5p, hsa-miR-339-3p, hsa-miR-744-5p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-miR-140-3p | 90.11 | 92.7 | 73.33 |
| 783 | hsa-miR-22-5p, hsa-miR-1307-5p, hsa-miR-339-3p, hsa-miR-485-3p, hsa-miR-10b-5p, hsa-miR-12136, hsa-miR-188-5p, hsa-miR-134-5p, hsa-miR-15a-5p, hsa-miR-629-5p, hsa-miR-100-5p, hsa-miR-183-3p, hsa-miR-224-5p, hsa-miR-652-3p | 89.33 | 95.4 | 81.67 |
| 784 | hsa-miR-22-5p, hsa-miR-1307-5p, hsa-miR-339-3p, hsa-miR-744-5p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p | 89.33 | 93.02 | 78.33 |
| 785 | hsa-miR-22-5p, hsa-miR-1307-5p, hsa-miR-339-3p, hsa-miR-195-5p, hsa-miR-10b-5p, hsa-miR-12136, hsa-miR-34a-3p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 89.33 | 94.37 | 81.67 |
| 786 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-15a-5p, hsa-miR-629-5p, hsa-miR-100-5p, hsa-miR-224-5p, hsa-let-7i-3p, hsa-miR-652-3p | 89.22 | 92.94 | 85.00 |
| 787 | hsa-miR-22-5p, hsa-miR-1307-5p, hsa-miR-339-3p, hsa-miR-10b-5p, hsa-miR-12136, hsa-miR-34a-3p, hsa-let-7d-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 89.22 | 95.71 | 86.67 |
| 788 | hsa-miR-22-5p, hsa-miR-1307-5p, hsa-miR-339-3p, hsa-miR-195-5p, hsa-miR-10b-5p, hsa-miR-12136, hsa-miR-34a-3p, hsa-let-7d-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 89.22 | 93.97 | 86.67 |
| 789 | hsa-miR-1307-5p, hsa-miR-339-3p, hsa-miR-485-3p, hsa-miR-10b-5p, hsa-miR-12136, hsa-miR-34a-3p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 89.22 | 95.71 | 85.00 |
| 790 | hsa-miR-22-5p, hsa-miR-1307-5p, hsa-miR-339-3p, hsa-miR-485-3p, hsa-miR-10b-5p, hsa-miR-12136, hsa-miR-455-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 89.22 | 94.37 | 81.67 |
| 791 | hsa-miR-22-5p, hsa-miR-548a-3p, hsa-miR-195-5p, hsa-miR-12136, hsa-miR-34a-3p, hsa-let-7d-3p, hsa-miR-337-5p, hsa-miR-188-5p, hsa-miR-4306, hsa-miR-1296-5p, hsa-miR-15a-5p, hsa-miR-100-5p, hsa-miR-183-3p, hsa-miR-224-5p, hsa-miR-140-3p | 89.22 | 93.49 | 76.67 |
| 792 | hsa-miR-548a-3p, hsa-miR-744-5p, hsa-miR-195-5p, hsa-miR-10b-5p, hsa-miR-34a-3p, hsa-let-7d-3p, hsa-miR-181c-5p, hsa-miR-4306, hsa-miR-1296-5p, hsa-miR-100-5p, hsa-miR-224-5p, hsa-miR-652-3p, hsa-miR-140-3p | 89.22 | 91.59 | 73.33 |
| 793 | hsa-miR-22-5p, hsa-miR-548a-3p, hsa-miR-744-5p, hsa-miR-195-5p, hsa-miR-34a-3p, hsa-let-7d-3p, hsa-miR-337-5p, hsa-miR-188-5p, hsa-miR-181c-5p, hsa-miR-4306, hsa-miR-1296-5p, hsa-miR-15a-5p, hsa-miR-100-5p, hsa-miR-183-3p, hsa-miR-224-5p, hsa-miR-652-3p, hsa-miR-140-3p | 89.22 | 92.3 | 73.33 |
| 794 | hsa-miR-22-5p, hsa-miR-485-3p, hsa-miR-12136, hsa-miR-34a-3p, hsa-miR-455-3p, hsa-miR-337-5p, hsa-miR-188-5p, hsa-miR-15a-5p, hsa-miR-629-5p, hsa-miR-100-5p, hsa-miR-183-3p, hsa-miR-224-5p, hsa-let-7i-3p, hsa-miR-140-3p | 89.22 | 91.9 | 76.67 |
| 795 | hsa-miR-22-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-15a-5p, hsa-miR-629-5p, hsa-miR-100-5p, hsa-miR-224-5p, hsa-let-7i-3p, hsa-miR-652-3p | 89.22 | 95.79 | 85.00 |
| 796 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-224-5p, hsa-let-7i-3p | 89.22 | 92.78 | 86.67 |
| 797 | hsa-miR-22-5p, hsa-miR-548a-3p, hsa-miR-1307-5p, hsa-miR-339-3p, hsa-miR-744-5p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-652-3p, hsa-miR-140-3p | 89.22 | 96.51 | 81.67 |
| 798 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-let-7i-3p | 89.11 | 93.65 | 85.00 |
| 799 | hsa-miR-22-5p, hsa-miR-1307-5p, hsa-miR-744-5p, hsa-miR-485-3p, hsa-miR-10b-5p, hsa-miR-12136, hsa-miR-34a-3p, hsa-let-7d-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-let-7i-3p | 89.11 | 94.84 | 78.33 |
| 800 | hsa-miR-22-5p, hsa-miR-548a-3p, hsa-miR-744-5p, hsa-miR-195-5p, hsa-miR-34a-3p, hsa-let-7d-3p, hsa-miR-455-3p, hsa-miR-337-5p, hsa-miR-181c-5p, hsa-miR-1296-5p, hsa-miR-15a-5p, hsa-miR-224-5p, hsa-let-7i-3p, hsa-miR-652-3p | 89.11 | 91.98 | 73.33 |
| 801 | hsa-miR-22-5p, hsa-miR-1307-5p, hsa-miR-10b-5p, hsa-miR-12136, hsa-miR-337-5p, hsa-miR-188-5p, hsa-miR-4306, hsa-miR-134-5p, hsa-miR-15a-5p, hsa-miR-629-5p, hsa-miR-100-5p, hsa-miR-224-5p, hsa-miR-652-3p, hsa-miR-140-3p | 89.11 | 94.6 | 73.33 |
| 802 | hsa-miR-22-5p, hsa-miR-548a-3p, hsa-miR-195-5p, hsa-miR-10b-5p, hsa-miR-34a-3p, hsa-let-7d-3p, hsa-miR-337-5p, hsa-miR-188-5p, hsa-miR-4306, hsa-miR-1296-5p, hsa-miR-15a-5p, hsa-miR-100-5p, hsa-miR-224-5p, hsa-miR-652-3p, hsa-miR-140-3p | 89.11 | 92.14 | 70.00 |
| 803 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 89.11 | 94.05 | 81.67 |
| 804 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 89.11 | 94.05 | 81.67 |
| 805 | hsa-miR-22-5p, hsa-miR-548a-3p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-4306, hsa-miR-134-5p, hsa-miR-15a-5p, hsa-miR-629-5p, hsa-miR-100-5p, hsa-miR-652-3p, hsa-miR-140-3p | 89.11 | 96.11 | 81.67 |
| 806 | hsa-miR-22-5p, hsa-miR-339-3p, hsa-miR-744-5p, hsa-miR-195-5p, hsa-miR-34a-3p, hsa-let-7d-3p, hsa-miR-337-5p, hsa-miR-181c-5p, hsa-miR-27a-3p, hsa-miR-134-5p, hsa-miR-1296-5p, hsa-miR-15a-5p, hsa-miR-100-5p, hsa-let-7i-3p, hsa-miR-652-3p, hsa-miR-140-3p | 89.11 | 89.76 | 73.33 |
| 807 | hsa-miR-548a-3p, hsa-miR-744-5p, hsa-miR-485-3p, hsa-miR-10b-5p, hsa-miR-34a-3p, hsa-let-7d-3p, hsa-miR-455-3p, hsa-miR-337-5p, hsa-miR-4306, hsa-miR-134-5p, hsa-miR-1296-5p, hsa-miR-15a-5p, hsa-miR-100-5p, hsa-miR-183-3p, hsa-miR-224-5p, hsa-miR-652-3p, hsa-miR-140-3p | 89.11 | 95.63 | 73.33 |
| 808 | hsa-miR-22-5p, hsa-miR-1307-5p, hsa-miR-339-3p, hsa-miR-195-5p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-let-7i-3p, hsa-miR-652-3p | 89.11 | 93.25 | 76.67 |
| 809 | hsa-miR-22-5p, hsa-miR-548a-3p, hsa-miR-1307-5p, hsa-miR-339-3p, hsa-miR-744-5p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-miR-140-3p | 89.11 | 92.54 | 78.33 |
| 810 | hsa-miR-22-5p, hsa-miR-548a-3p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-652-3p, hsa-miR-140-3p | 89.11 | 97.3 | 78.33 |
| 811 | hsa-miR-22-5p, hsa-miR-1307-5p, hsa-miR-339-3p, hsa-miR-485-3p, hsa-miR-12136, hsa-miR-34a-3p, hsa-miR-455-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-224-5p, hsa-miR-652-3p | 89.11 | 94.05 | 81.67 |
| 812 | hsa-miR-22-5p, hsa-miR-1307-5p, hsa-miR-485-3p, hsa-miR-12136, hsa-miR-455-3p, hsa-miR-337-5p, hsa-miR-188-5p, hsa-miR-134-5p, hsa-miR-15a-5p, hsa-miR-629-5p, hsa-miR-100-5p, hsa-miR-224-5p, hsa-let-7i-3p, hsa-miR-652-3p | 89.11 | 95.00 | 81.67 |
| 813 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-let-7i-3p, hsa-miR-140-3p | 89.11 | 93.97 | 81.67 |
| 814 | hsa-miR-22-5p, hsa-miR-548a-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-134-5p, hsa-miR-1296-5p, hsa-miR-15a-5p, hsa-miR-100-5p, hsa-miR-224-5p, hsa-miR-652-3p | 89.11 | 92.46 | 78.33 |
| 815 | hsa-miR-22-5p, hsa-miR-1307-5p, hsa-miR-339-3p, hsa-miR-485-3p, hsa-miR-10b-5p, hsa-miR-12136, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 89.11 | 94.05 | 81.67 |
| 816 | hsa-miR-22-5p, hsa-miR-1307-5p, hsa-miR-339-3p, hsa-miR-485-3p, hsa-miR-10b-5p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 89.11 | 92.94 | 78.33 |
| 817 | hsa-miR-22-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 89.11 | 95.56 | 78.33 |
| 818 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-181c-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-let-7i-3p | 89.11 | 94.68 | 81.67 |
| 819 | hsa-miR-22-5p, hsa-miR-548a-3p, hsa-miR-339-3p, hsa-miR-744-5p, hsa-miR-195-5p, hsa-miR-10b-5p, hsa-let-7d-3p, hsa-miR-337-5p, hsa-miR-181c-5p, hsa-miR-4306, hsa-miR-27a-3p, hsa-miR-1296-5p, hsa-miR-15a-5p, hsa-miR-224-5p, hsa-miR-652-3p, hsa-miR-140-3p | 89.11 | 92.46 | 68.33 |
| 820 | hsa-miR-22-5p, hsa-miR-548a-3p, hsa-miR-1307-5p, hsa-miR-339-3p, hsa-miR-485-3p, hsa-miR-10b-5p, hsa-miR-12136, hsa-miR-34a-3p, hsa-let-7d-3p, hsa-miR-337-5p, hsa-miR-188-5p, hsa-miR-4306, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 89.11 | 94.52 | 81.67 |
| 821 | hsa-miR-22-5p, hsa-miR-548a-3p, hsa-miR-339-3p, hsa-miR-744-5p, hsa-miR-10b-5p, hsa-miR-12136, hsa-miR-34a-3p, hsa-miR-337-5p, hsa-miR-4306, hsa-miR-27a-3p, hsa-miR-134-5p, hsa-miR-15a-5p, hsa-miR-629-5p, hsa-miR-100-5p, hsa-miR-183-3p, hsa-miR-652-3p, hsa-miR-140-3p | 89.11 | 93.65 | 73.33 |
| 822 | hsa-miR-22-5p, hsa-miR-195-5p, hsa-miR-485-3p, hsa-miR-10b-5p, hsa-miR-12136, hsa-miR-34a-3p, hsa-let-7d-3p, hsa-miR-337-5p, hsa-miR-181c-5p, hsa-miR-15a-5p, hsa-miR-629-5p, hsa-miR-100-5p, hsa-miR-183-3p, hsa-miR-224-5p, hsa-let-7i-3p, hsa-miR-652-3p, hsa-miR-140-3p | 89.11 | 94.05 | 76.67 |
| 823 | hsa-miR-22-5p, hsa-miR-1307-5p, hsa-miR-339-3p, hsa-miR-195-5p, hsa-miR-485-3p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 89.11 | 95.95 | 78.33 |
| 824 | hsa-miR-22-5p, hsa-miR-548a-3p, hsa-miR-744-5p, hsa-miR-195-5p, hsa-miR-485-3p, hsa-miR-34a-3p, hsa-let-7d-3p, hsa-miR-455-3p, hsa-miR-337-5p, hsa-miR-188-5p, hsa-miR-181c-5p, hsa-miR-15a-5p, hsa-miR-629-5p, hsa-miR-100-5p, hsa-miR-183-3p, hsa-miR-224-5p, hsa-let-7i-3p, hsa-miR-652-3p, hsa-miR-140-3p | 89.00 | 96.59 | 80.00 |
| 825 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-134-5p, hsa-miR-1296-5p, hsa-miR-15a-5p, hsa-miR-100-5p, hsa-miR-183-3p, hsa-miR-224-5p, hsa-miR-140-3p | 89.00 | 93.57 | 70.00 |
| 826 | hsa-miR-22-5p, hsa-miR-195-5p, hsa-miR-485-3p, hsa-miR-10b-5p, hsa-miR-34a-3p, hsa-let-7d-3p, hsa-miR-337-5p, hsa-miR-181c-5p, hsa-miR-15a-5p, hsa-miR-629-5p, hsa-miR-100-5p, hsa-miR-183-3p, hsa-miR-224-5p, hsa-let-7i-3p, hsa-miR-652-3p, hsa-miR-140-3p | 89.00 | 92.06 | 76.67 |
| 827 | hsa-miR-22-5p, hsa-miR-1307-5p, hsa-miR-339-3p, hsa-miR-195-5p, hsa-miR-10b-5p, hsa-miR-12136, hsa-miR-34a-3p, hsa-let-7d-3p, hsa-miR-337-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 88.22 | 93.33 | 73.33 |
| 828 | hsa-miR-1307-5p, hsa-miR-339-3p, hsa-miR-10b-5p, hsa-miR-12136, hsa-miR-455-3p, hsa-miR-337-5p, hsa-miR-188-5p, hsa-miR-181c-5p, hsa-miR-27a-3p, hsa-miR-134-5p, hsa-miR-629-5p, hsa-miR-100-5p, hsa-miR-183-3p, hsa-miR-224-5p, hsa-miR-652-3p | 88.22 | 93.02 | 70.00 |
| 829 | hsa-miR-22-5p, hsa-miR-1307-5p, hsa-miR-339-3p, hsa-miR-10b-5p, hsa-miR-12136, hsa-miR-34a-3p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 88.22 | 95.08 | 81.67 |
| 830 | hsa-miR-1307-5p, hsa-miR-339-3p, hsa-miR-485-3p, hsa-miR-10b-5p, hsa-miR-12136, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 88.11 | 95.24 | 85.00 |
| 831 | hsa-miR-22-5p, hsa-miR-1307-5p, hsa-miR-339-3p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 88.11 | 93.57 | 73.33 |
| 832 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-15a-5p, hsa-miR-629-5p, hsa-miR-100-5p, hsa-miR-224-5p, hsa-let-7i-3p, hsa-miR-652-3p | 88.11 | 92.94 | 81.67 |
| 833 | hsa-miR-22-5p, hsa-miR-1307-5p, hsa-miR-339-3p, hsa-miR-195-5p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 88.11 | 95.24 | 81.67 |
| 834 | hsa-miR-22-5p, hsa-miR-744-5p, hsa-miR-195-5p, hsa-miR-34a-3p, hsa-let-7d-3p, hsa-miR-455-3p, hsa-miR-181c-5p, hsa-miR-27a-3p, hsa-miR-1296-5p, hsa-miR-100-5p, hsa-miR-224-5p, hsa-let-7i-3p, hsa-miR-140-3p | 88.11 | 89.84 | 73.33 |
| 835 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p | 88.11 | 95.56 | 81.67 |
| 836 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-15a-5p, hsa-miR-629-5p, hsa-miR-100-5p, hsa-let-7i-3p, hsa-miR-652-3p | 88.11 | 97.06 | 80.00 |
| 837 | hsa-miR-1307-5p, hsa-miR-339-3p, hsa-miR-10b-5p, hsa-miR-12136, hsa-miR-188-5p, hsa-miR-27a-3p, hsa-miR-134-5p, hsa-miR-629-5p, hsa-miR-100-5p, hsa-miR-183-3p, hsa-miR-224-5p, hsa-miR-652-3p | 88.11 | 93.49 | 76.67 |
| 838 | hsa-miR-22-5p, hsa-miR-548a-3p, hsa-miR-1307-5p, hsa-miR-339-3p, hsa-miR-744-5p, hsa-miR-10b-5p, hsa-miR-12136, hsa-miR-34a-3p, hsa-miR-337-5p, hsa-miR-4306, hsa-miR-27a-3p, hsa-miR-134-5p, hsa-miR-1296-5p, hsa-miR-15a-5p, hsa-miR-100-5p, hsa-miR-183-3p, hsa-miR-652-3p, hsa-miR-140-3p | 88.11 | 95.79 | 81.67 |
| 839 | hsa-miR-548a-3p, hsa-miR-1307-5p, hsa-miR-339-3p, hsa-miR-485-3p, hsa-miR-181c-5p, hsa-miR-4306, hsa-miR-134-5p, hsa-miR-15a-5p, hsa-miR-100-5p, hsa-miR-183-3p, hsa-miR-224-5p, hsa-miR-652-3p, hsa-miR-140-3p | 88.11 | 93.81 | 86.67 |
| 840 | hsa-miR-22-5p, hsa-miR-548a-3p, hsa-miR-1307-5p, hsa-miR-744-5p, hsa-miR-195-5p, hsa-miR-10b-5p, hsa-miR-12136, hsa-miR-34a-3p, hsa-miR-337-5p, hsa-miR-4306, hsa-miR-134-5p, hsa-miR-1296-5p, hsa-miR-15a-5p, hsa-miR-100-5p, hsa-miR-224-5p, hsa-miR-652-3p | 88.11 | 92.78 | 80.00 |
| 841 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-let-7d-3p, hsa-miR-337-5p, hsa-miR-188-5p, hsa-miR-181c-5p, hsa-miR-27a-3p, hsa-miR-134-5p, hsa-miR-1296-5p, hsa-miR-15a-5p, hsa-miR-100-5p, hsa-miR-224-5p, hsa-let-7i-3p, hsa-miR-140-3p | 88.11 | 93.73 | 70.00 |
| 842 | hsa-miR-1307-5p, hsa-miR-339-3p, hsa-miR-10b-5p, hsa-miR-12136, hsa-miR-337-5p, hsa-miR-188-5p, hsa-miR-181c-5p, hsa-miR-27a-3p, hsa-miR-134-5p, hsa-miR-629-5p, hsa-miR-100-5p, hsa-miR-183-3p, hsa-miR-224-5p, hsa-miR-652-3p | 88.11 | 96.03 | 81.67 |
| 843 | hsa-miR-22-5p, hsa-miR-1307-5p, hsa-miR-339-3p, hsa-miR-195-5p, hsa-miR-10b-5p, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 88.11 | 92.78 | 76.67 |
| 844 | hsa-miR-22-5p, hsa-miR-1307-5p, hsa-miR-339-3p, hsa-miR-195-5p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p | 88.11 | 92.94 | 81.67 |
| 845 | hsa-miR-22-5p, hsa-miR-1307-5p, hsa-miR-339-3p, hsa-miR-10b-5p, hsa-miR-12136, hsa-miR-34a-3p, hsa-let-7d-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-183-3p, hsa-miR-224-5p | 88.11 | 92.86 | 78.33 |
| 846 | hsa-miR-22-5p, hsa-miR-1307-5p, hsa-miR-339-3p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p | 88.11 | 92.38 | 78.33 |
| 847 | hsa-miR-22-5p, hsa-miR-10b-5p, hsa-miR-34a-3p, hsa-let-7d-3p, hsa-miR-455-3p, hsa-miR-337-5p, hsa-miR-188-5p, hsa-miR-181c-5p, hsa-miR-15a-5p, hsa-miR-629-5p, hsa-miR-100-5p, hsa-miR-183-3p, hsa-miR-224-5p, hsa-let-7i-3p, hsa-miR-140-3p | 88.00 | 92.78 | 73.33 |
| 848 | hsa-miR-22-5p, hsa-miR-1307-5p, hsa-miR-485-3p, hsa-miR-10b-5p, hsa-miR-12136, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p | 88.00 | 94.29 | 81.67 |
| 849 | hsa-miR-22-5p, hsa-miR-548a-3p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-4306, hsa-miR-27a-3p, hsa-miR-15a-5p, hsa-miR-629-5p, hsa-miR-100-5p, hsa-miR-652-3p, hsa-miR-140-3p | 88.00 | 93.97 | 81.67 |
| 850 | hsa-miR-22-5p, hsa-miR-1307-5p, hsa-miR-339-3p, hsa-miR-195-5p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p, hsa-miR-140-3p | 88.00 | 95.48 | 76.67 |
| 851 | hsa-miR-22-5p, hsa-miR-1307-5p, hsa-miR-485-3p, hsa-miR-12136, hsa-miR-455-3p, hsa-miR-337-5p, hsa-miR-188-5p, hsa-miR-134-5p, hsa-miR-15a-5p, hsa-miR-629-5p, hsa-miR-224-5p, hsa-let-7i-3p, hsa-miR-652-3p | 88.00 | 92.7 | 81.67 |
| 852 | hsa-miR-22-5p, hsa-miR-10b-5p, hsa-miR-34a-3p, hsa-let-7d-3p, hsa-miR-455-3p, hsa-miR-337-5p, hsa-miR-188-5p, hsa-miR-181c-5p, hsa-miR-15a-5p, hsa-miR-629-5p, hsa-miR-100-5p, hsa-miR-183-3p, hsa-miR-224-5p, hsa-let-7i-3p | 88.00 | 91.35 | 76.67 |
| 853 | hsa-miR-22-5p, hsa-miR-1307-5p, hsa-miR-485-3p, hsa-miR-10b-5p, hsa-miR-12136, hsa-miR-337-5p, hsa-miR-188-5p, hsa-miR-134-5p, hsa-miR-15a-5p, hsa-miR-629-5p, hsa-miR-100-5p, hsa-miR-224-5p | 88.00 | 93.81 | 81.67 |
| 854 | hsa-miR-22-5p, hsa-miR-1307-5p, hsa-miR-485-3p, hsa-miR-10b-5p, hsa-miR-12136, hsa-miR-455-3p, hsa-miR-337-5p, hsa-miR-188-5p, hsa-miR-134-5p, hsa-miR-15a-5p, hsa-miR-629-5p, hsa-miR-224-5p, hsa-miR-652-3p | 88.00 | 93.65 | 85.00 |
| 855 | hsa-miR-22-5p, hsa-miR-1307-5p, hsa-miR-339-3p, hsa-miR-485-3p, hsa-miR-12136, hsa-miR-337-5p, hsa-miR-188-5p, hsa-miR-27a-3p, hsa-miR-134-5p, hsa-miR-15a-5p, hsa-miR-629-5p, hsa-miR-183-3p, hsa-miR-224-5p, hsa-let-7i-3p, hsa-miR-652-3p | 88.00 | 94.29 | 78.33 |
| 856 | hsa-miR-22-5p, hsa-miR-548a-3p, hsa-miR-1307-5p, hsa-miR-485-3p, hsa-miR-10b-5p, hsa-miR-12136, hsa-miR-455-3p, hsa-miR-337-5p, hsa-miR-188-5p, hsa-miR-4306, hsa-miR-134-5p, hsa-miR-15a-5p, hsa-miR-629-5p, hsa-miR-224-5p, hsa-miR-652-3p | 88.00 | 94.68 | 85.00 |
| 857 | hsa-miR-22-5p, hsa-miR-548a-3p, hsa-miR-744-5p, hsa-miR-195-5p, hsa-miR-485-3p, hsa-miR-10b-5p, hsa-miR-12136, hsa-miR-34a-3p, hsa-let-7d-3p, hsa-miR-337-5p, hsa-miR-181c-5p, hsa-miR-15a-5p, hsa-miR-100-5p, hsa-miR-183-3p, hsa-miR-224-5p, hsa-miR-140-3p | 88.00 | 93.73 | 70.00 |
| 858 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-let-7i-3p, hsa-miR-652-3p | 88.00 | 94.13 | 81.67 |
| 859 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-183-3p, hsa-let-7i-3p | 88.00 | 93.25 | 85.00 |
| 860 | hsa-miR-22-5p, hsa-miR-1307-5p, hsa-miR-339-3p, hsa-miR-485-3p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-224-5p, hsa-let-7i-3p, hsa-miR-652-3p | 87.89 | 93.57 | 76.67 |
| 861 | hsa-miR-22-5p, hsa-miR-195-5p, hsa-miR-485-3p, hsa-miR-10b-5p, hsa-miR-34a-3p, hsa-let-7d-3p, hsa-miR-337-5p, hsa-miR-181c-5p, hsa-miR-15a-5p, hsa-miR-629-5p, hsa-miR-100-5p, hsa-miR-183-3p, hsa-miR-224-5p, hsa-miR-140-3p | 87.89 | 92.22 | 76.67 |
| 862 | hsa-miR-22-5p, hsa-miR-548a-3p, hsa-miR-1307-5p, hsa-miR-744-5p, hsa-miR-485-3p, hsa-miR-10b-5p, hsa-miR-12136, hsa-miR-34a-3p, hsa-miR-337-5p, hsa-miR-134-5p, hsa-miR-1296-5p, hsa-miR-15a-5p, hsa-miR-100-5p, hsa-miR-183-3p, hsa-miR-224-5p, hsa-let-7i-3p, hsa-miR-140-3p | 87.89 | 94.68 | 78.33 |
| 863 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-let-7i-3p, hsa-miR-140-3p | 87.89 | 93.25 | 73.33 |
| 864 | hsa-miR-22-5p, hsa-miR-1307-5p, hsa-miR-744-5p, hsa-miR-485-3p, hsa-miR-10b-5p, hsa-miR-12136, hsa-miR-34a-3p, hsa-miR-337-5p, hsa-miR-134-5p, hsa-miR-1296-5p, hsa-miR-15a-5p, hsa-miR-100-5p, hsa-miR-183-3p, hsa-miR-224-5p, hsa-let-7i-3p, hsa-miR-140-3p | 87.89 | 94.6 | 73.33 |
| 865 | hsa-miR-22-5p, hsa-miR-548a-3p, hsa-miR-1307-5p, hsa-miR-744-5p, hsa-miR-485-3p, hsa-miR-10b-5p, hsa-miR-12136, hsa-miR-34a-3p, hsa-miR-337-5p, hsa-miR-4306, hsa-miR-134-5p, hsa-miR-1296-5p, hsa-miR-15a-5p, hsa-miR-100-5p, hsa-miR-183-3p, hsa-miR-224-5p, hsa-let-7i-3p, hsa-miR-140-3p | 87.89 | 95.63 | 81.67 |
| 866 | hsa-miR-1307-5p, hsa-miR-485-3p, hsa-miR-10b-5p, hsa-miR-12136, hsa-miR-188-5p, hsa-miR-134-5p, hsa-miR-629-5p, hsa-miR-100-5p, hsa-miR-183-3p, hsa-miR-224-5p, hsa-miR-652-3p | 87.11 | 93.33 | 78.33 |
| 867 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-15a-5p, hsa-miR-629-5p, hsa-miR-100-5p, hsa-miR-183-3p, hsa-miR-224-5p, hsa-let-7i-3p | 87.11 | 95.24 | 76.67 |
| 868 | hsa-miR-22-5p, hsa-miR-548a-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-1296-5p, hsa-miR-15a-5p, hsa-miR-100-5p, hsa-miR-224-5p, hsa-miR-652-3p | 87.11 | 89.37 | 73.33 |
| 869 | hsa-miR-22-5p, hsa-miR-1307-5p, hsa-miR-339-3p, hsa-miR-195-5p, hsa-miR-10b-5p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-let-7i-3p, hsa-miR-652-3p | 87.11 | 91.9 | 76.67 |
| 870 | hsa-miR-1307-5p, hsa-miR-12136, hsa-miR-455-3p, hsa-miR-337-5p, hsa-miR-181c-5p, hsa-miR-4306, hsa-miR-27a-3p, hsa-miR-1296-5p, hsa-miR-15a-5p, hsa-miR-183-3p, hsa-miR-224-5p | 87.11 | 90.87 | 75.00 |
| 871 | hsa-miR-22-5p, hsa-miR-1307-5p, hsa-miR-339-3p, hsa-miR-10b-5p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 87.11 | 93.17 | 76.67 |
| 872 | hsa-miR-22-5p, hsa-miR-1307-5p, hsa-miR-339-3p, hsa-miR-195-5p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-miR-652-3p | 87.11 | 92.62 | 73.33 |
| 873 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-let-7i-3p | 87.00 | 94.13 | 81.67 |
| 874 | hsa-miR-22-5p, hsa-miR-1307-5p, hsa-miR-744-5p, hsa-miR-195-5p, hsa-miR-10b-5p, hsa-miR-12136, hsa-miR-34a-3p, hsa-let-7d-3p, hsa-miR-337-5p, hsa-miR-188-5p, hsa-miR-134-5p, hsa-miR-15a-5p, hsa-miR-629-5p, hsa-miR-100-5p, hsa-miR-224-5p, hsa-let-7i-3p, hsa-miR-652-3p | 87.00 | 93.49 | 78.33 |
| 875 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-1296-5p, hsa-miR-15a-5p, hsa-miR-100-5p, hsa-miR-224-5p | 87.00 | 92.7 | 78.33 |
| 876 | hsa-miR-22-5p, hsa-miR-548a-3p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-4306, hsa-miR-27a-3p, hsa-miR-15a-5p, hsa-miR-629-5p, hsa-miR-100-5p, hsa-miR-652-3p | 87.00 | 92.94 | 85.00 |
| 877 | hsa-miR-22-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-15a-5p, hsa-miR-629-5p, hsa-miR-100-5p, hsa-let-7i-3p, hsa-miR-652-3p | 87.00 | 90.95 | 85.00 |
| 878 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-455-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 87.00 | 93.81 | 78.33 |
| 879 | hsa-miR-22-5p, hsa-miR-195-5p, hsa-miR-485-3p, hsa-miR-10b-5p, hsa-miR-12136, hsa-miR-34a-3p, hsa-miR-337-5p, hsa-miR-188-5p, hsa-miR-15a-5p, hsa-miR-100-5p, hsa-miR-183-3p, hsa-miR-224-5p, hsa-miR-652-3p, hsa-miR-140-3p | 87.00 | 93.97 | 76.67 |
| 880 | hsa-miR-22-5p, hsa-miR-548a-3p, hsa-miR-744-5p, hsa-miR-10b-5p, hsa-let-7d-3p, hsa-miR-337-5p, hsa-miR-181c-5p, hsa-miR-4306, hsa-miR-27a-3p, hsa-miR-1296-5p, hsa-miR-15a-5p, hsa-miR-100-5p, hsa-miR-183-3p, hsa-miR-224-5p, hsa-miR-652-3p, hsa-miR-140-3p | 87.00 | 93.49 | 70.00 |
| 881 | hsa-miR-22-5p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-4306, hsa-miR-15a-5p, hsa-miR-629-5p, hsa-miR-100-5p, hsa-miR-224-5p, hsa-miR-652-3p | 87.00 | 93.97 | 78.33 |
| 882 | hsa-miR-1307-5p, hsa-miR-339-3p, hsa-miR-485-3p, hsa-miR-10b-5p, hsa-miR-12136, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 87.00 | 95.16 | 85.00 |
| 883 | hsa-miR-22-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-let-7i-3p | 87.00 | 93.65 | 73.33 |
| 884 | hsa-miR-1307-5p, hsa-miR-485-3p, hsa-miR-10b-5p, hsa-miR-12136, hsa-miR-337-5p, hsa-miR-188-5p, hsa-miR-134-5p, hsa-miR-629-5p, hsa-miR-224-5p, hsa-miR-652-3p | 87.00 | 93.41 | 81.67 |
| 885 | hsa-miR-22-5p, hsa-miR-548a-3p, hsa-miR-339-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-1296-5p, hsa-miR-15a-5p, hsa-miR-100-5p, hsa-miR-224-5p, hsa-miR-652-3p, hsa-miR-140-3p | 87.00 | 93.81 | 73.33 |
| 886 | hsa-miR-22-5p, hsa-miR-548a-3p, hsa-miR-744-5p, hsa-miR-10b-5p, hsa-miR-12136, hsa-let-7d-3p, hsa-miR-337-5p, hsa-miR-4306, hsa-miR-134-5p, hsa-miR-1296-5p, hsa-miR-15a-5p, hsa-miR-629-5p, hsa-miR-100-5p, hsa-miR-183-3p, hsa-miR-224-5p, hsa-let-7i-3p | 87.00 | 93.41 | 85.00 |
| 887 | hsa-miR-22-5p, hsa-miR-1307-5p, hsa-miR-339-3p, hsa-miR-485-3p, hsa-miR-10b-5p, hsa-miR-12136, hsa-miR-455-3p, hsa-miR-337-5p, hsa-miR-188-5p, hsa-miR-27a-3p, hsa-miR-134-5p, hsa-miR-629-5p, hsa-miR-224-5p, hsa-miR-652-3p | 87.00 | 91.43 | 76.67 |
| 888 | hsa-miR-22-5p, hsa-miR-548a-3p, hsa-miR-1307-5p, hsa-miR-744-5p, hsa-miR-10b-5p, hsa-let-7d-3p, hsa-miR-337-5p, hsa-miR-181c-5p, hsa-miR-4306, hsa-miR-27a-3p, hsa-miR-134-5p, hsa-miR-1296-5p, hsa-miR-183-3p, hsa-miR-652-3p | 87.00 | 90.87 | 75.00 |
| 889 | hsa-miR-1307-5p, hsa-miR-339-3p, hsa-miR-195-5p, hsa-miR-10b-5p, hsa-miR-12136, hsa-miR-188-5p, hsa-miR-181c-5p, hsa-miR-27a-3p, hsa-miR-134-5p, hsa-miR-629-5p, hsa-miR-100-5p, hsa-miR-183-3p, hsa-miR-224-5p, hsa-miR-652-3p | 87.00 | 93.65 | 70.00 |
| 890 | hsa-miR-22-5p, hsa-miR-548a-3p, hsa-miR-339-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-134-5p, hsa-miR-1296-5p, hsa-miR-15a-5p, hsa-miR-100-5p, hsa-miR-652-3p, hsa-miR-140-3p | 87.00 | 94.21 | 70.00 |
| 891 | hsa-miR-22-5p, hsa-miR-1307-5p, hsa-miR-339-3p, hsa-miR-485-3p, hsa-miR-10b-5p, hsa-miR-12136, hsa-miR-188-5p, hsa-miR-134-5p, hsa-miR-15a-5p, hsa-miR-629-5p, hsa-miR-100-5p, hsa-miR-652-3p | 86.89 | 93.17 | 81.67 |
| 892 | hsa-miR-22-5p, hsa-miR-1307-5p, hsa-miR-339-3p, hsa-miR-485-3p, hsa-miR-12136, hsa-miR-34a-3p, hsa-miR-455-3p, hsa-miR-337-5p, hsa-miR-188-5p, hsa-miR-27a-3p, hsa-miR-134-5p, hsa-miR-15a-5p, hsa-miR-629-5p, hsa-miR-224-5p, hsa-miR-652-3p | 86.89 | 93.1 | 78.33 |
| 893 | hsa-miR-22-5p, hsa-miR-548a-3p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-4306, hsa-miR-27a-3p, hsa-miR-134-5p, hsa-miR-15a-5p, hsa-miR-629-5p, hsa-miR-100-5p, hsa-miR-652-3p | 86.89 | 93.81 | 85.00 |
| 894 | hsa-miR-22-5p, hsa-miR-548a-3p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-181c-5p, hsa-miR-4306, hsa-miR-27a-3p, hsa-miR-134-5p, hsa-miR-15a-5p, hsa-miR-629-5p, hsa-miR-100-5p, hsa-miR-652-3p, hsa-miR-140-3p | 86.89 | 94.13 | 85.00 |
| 895 | hsa-miR-22-5p, hsa-miR-1307-5p, hsa-miR-339-3p, hsa-miR-485-3p, hsa-miR-34a-3p, hsa-miR-455-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-224-5p, hsa-let-7i-3p, hsa-miR-652-3p | 86.89 | 93.1 | 78.33 |
| 896 | hsa-miR-22-5p, hsa-miR-1307-5p, hsa-miR-339-3p, hsa-miR-485-3p, hsa-miR-10b-5p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-224-5p, hsa-miR-652-3p | 86.89 | 93.57 | 78.33 |
| 897 | hsa-miR-22-5p, hsa-miR-548a-3p, hsa-miR-1307-5p, hsa-miR-339-3p, hsa-miR-485-3p, hsa-miR-10b-5p, hsa-miR-12136, hsa-miR-34a-3p, hsa-let-7d-3p, hsa-miR-337-5p, hsa-miR-188-5p, hsa-miR-181c-5p, hsa-miR-4306, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-140-3p | 86.89 | 95.24 | 73.33 |
| 898 | hsa-miR-22-5p, hsa-miR-1307-5p, hsa-miR-339-3p, hsa-miR-485-3p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-224-5p, hsa-miR-652-3p | 86.89 | 93.1 | 78.33 |
| 899 | hsa-miR-22-5p, hsa-miR-1307-5p, hsa-miR-339-3p, hsa-miR-485-3p, hsa-miR-12136, hsa-miR-34a-3p, hsa-miR-455-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-224-5p, hsa-let-7i-3p, hsa-miR-652-3p | 86.89 | 93.1 | 78.33 |
| 900 | hsa-miR-22-5p, hsa-miR-1307-5p, hsa-miR-339-3p, hsa-miR-485-3p, hsa-miR-12136, hsa-miR-34a-3p, hsa-miR-337-5p, hsa-miR-188-5p, hsa-miR-181c-5p, hsa-miR-27a-3p, hsa-miR-15a-5p, hsa-miR-629-5p, hsa-miR-224-5p, hsa-let-7i-3p, hsa-miR-652-3p | 86.89 | 92.3 | 73.33 |
| 901 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-let-7i-3p | 86.89 | 92.46 | 81.67 |
| 902 | hsa-miR-1307-5p, hsa-miR-339-3p, hsa-miR-485-3p, hsa-miR-10b-5p, hsa-miR-12136, hsa-miR-337-5p, hsa-miR-188-5p, hsa-miR-27a-3p, hsa-miR-134-5p, hsa-miR-629-5p, hsa-miR-100-5p, hsa-miR-224-5p, hsa-miR-652-3p | 86.89 | 93.73 | 75.00 |
| 903 | hsa-miR-1307-5p, hsa-miR-339-3p, hsa-miR-485-3p, hsa-miR-10b-5p, hsa-miR-12136, hsa-miR-337-5p, hsa-miR-188-5p, hsa-miR-27a-3p, hsa-miR-134-5p, hsa-miR-629-5p, hsa-miR-100-5p, hsa-miR-224-5p, hsa-let-7i-3p, hsa-miR-652-3p | 86.89 | 90.24 | 78.33 |
| 904 | hsa-miR-22-5p, hsa-miR-1307-5p, hsa-miR-339-3p, hsa-miR-195-5p, hsa-miR-485-3p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p | 86.89 | 89.05 | 76.67 |
| 905 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-455-3p, hsa-miR-337-5p, hsa-miR-188-5p, hsa-miR-27a-3p, hsa-miR-134-5p, hsa-miR-629-5p, hsa-miR-224-5p, hsa-miR-652-3p | 86.89 | 96.11 | 78.33 |
| 906 | hsa-miR-22-5p, hsa-miR-1307-5p, hsa-miR-339-3p, hsa-miR-195-5p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 86.89 | 94.29 | 76.67 |
| 907 | hsa-miR-548a-3p, hsa-miR-1307-5p, hsa-miR-485-3p, hsa-miR-10b-5p, hsa-miR-12136, hsa-miR-337-5p, hsa-miR-188-5p, hsa-miR-4306, hsa-miR-134-5p, hsa-miR-15a-5p, hsa-miR-629-5p, hsa-miR-224-5p, hsa-miR-652-3p | 86.89 | 92.38 | 68.33 |
| 908 | hsa-miR-22-5p, hsa-miR-195-5p, hsa-miR-485-3p, hsa-miR-10b-5p, hsa-miR-12136, hsa-miR-34a-3p, hsa-let-7d-3p, hsa-miR-337-5p, hsa-miR-4306, hsa-miR-1296-5p, hsa-miR-15a-5p, hsa-miR-100-5p, hsa-miR-183-3p, hsa-miR-224-5p, hsa-miR-140-3p | 86.89 | 93.73 | 66.67 |
| 909 | hsa-miR-22-5p, hsa-miR-548a-3p, hsa-miR-744-5p, hsa-miR-34a-3p, hsa-let-7d-3p, hsa-miR-455-3p, hsa-miR-337-5p, hsa-miR-181c-5p, hsa-miR-4306, hsa-miR-1296-5p, hsa-miR-15a-5p, hsa-miR-629-5p, hsa-miR-100-5p, hsa-miR-183-3p, hsa-miR-224-5p, hsa-let-7i-3p, hsa-miR-140-3p | 86.89 | 91.51 | 76.67 |
| 910 | hsa-miR-22-5p, hsa-miR-1307-5p, hsa-miR-339-3p, hsa-miR-195-5p, hsa-miR-10b-5p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 86.89 | 93.02 | 76.67 |
| 911 | hsa-miR-22-5p, hsa-miR-548a-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-134-5p, hsa-miR-15a-5p, hsa-miR-629-5p, hsa-miR-100-5p, hsa-miR-224-5p, hsa-miR-652-3p, hsa-miR-140-3p | 86.78 | 93.49 | 76.67 |
| 912 | hsa-miR-1307-5p, hsa-miR-339-3p, hsa-miR-485-3p, hsa-miR-10b-5p, hsa-miR-12136, hsa-miR-34a-3p, hsa-miR-188-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 86.00 | 93.33 | 85.00 |
| 913 | hsa-miR-548a-3p, hsa-miR-1307-5p, hsa-miR-339-3p, hsa-miR-744-5p, hsa-miR-195-5p, hsa-miR-10b-5p, hsa-miR-34a-3p, hsa-let-7d-3p, hsa-miR-181c-5p, hsa-miR-4306, hsa-miR-134-5p, hsa-miR-1296-5p, hsa-miR-15a-5p, hsa-miR-224-5p, hsa-miR-652-3p, hsa-miR-140-3p | 86.00 | 91.03 | 63.33 |
| 914 | hsa-miR-22-5p, hsa-miR-548a-3p, hsa-miR-1307-5p, hsa-miR-744-5p, hsa-miR-195-5p, hsa-miR-10b-5p, hsa-let-7d-3p, hsa-miR-181c-5p, hsa-miR-4306, hsa-miR-1296-5p, hsa-miR-15a-5p, hsa-miR-100-5p, hsa-miR-224-5p, hsa-miR-652-3p | 86.00 | 90.4 | 66.67 |
| 915 | hsa-miR-22-5p, hsa-miR-1307-5p, hsa-miR-339-3p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 86.00 | 94.29 | 71.67 |
| 916 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-let-7d-3p, hsa-miR-337-5p, hsa-miR-188-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p, hsa-let-7i-3p | 86.00 | 91.83 | 73.33 |
| 917 | hsa-miR-22-5p, hsa-miR-548a-3p, hsa-miR-1307-5p, hsa-miR-339-3p, hsa-miR-12136, hsa-let-7d-3p, hsa-miR-455-3p, hsa-miR-337-5p, hsa-miR-188-5p, hsa-miR-27a-3p, hsa-miR-134-5p, hsa-miR-15a-5p, hsa-miR-100-5p, hsa-miR-224-5p, hsa-miR-652-3p, hsa-miR-140-3p | 86.00 | 92.54 | 71.67 |
| 918 | hsa-miR-1307-5p, hsa-miR-485-3p, hsa-miR-10b-5p, hsa-miR-12136, hsa-miR-188-5p, hsa-miR-134-5p, hsa-miR-629-5p, hsa-miR-100-5p, hsa-miR-224-5p, hsa-miR-652-3p | 86.00 | 91.67 | 75.00 |
| 919 | hsa-miR-22-5p, hsa-miR-1307-5p, hsa-miR-485-3p, hsa-miR-10b-5p, hsa-miR-12136, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-652-3p | 85.89 | 92.14 | 81.67 |
| 920 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p | 85.89 | 94.84 | 81.67 |
| 921 | hsa-miR-548a-3p, hsa-miR-339-3p, hsa-miR-744-5p, hsa-miR-195-5p, hsa-miR-10b-5p, hsa-let-7d-3p, hsa-miR-337-5p, hsa-miR-181c-5p, hsa-miR-4306, hsa-miR-27a-3p, hsa-miR-134-5p, hsa-miR-1296-5p, hsa-miR-224-5p, hsa-miR-652-3p, hsa-miR-140-3p | 85.89 | 91.35 | 73.33 |
| 922 | hsa-miR-22-5p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-181c-5p, hsa-miR-27a-3p, hsa-miR-134-5p, hsa-miR-1296-5p, hsa-miR-15a-5p, hsa-miR-100-5p, hsa-miR-183-3p, hsa-miR-652-3p | 85.89 | 93.81 | 70.00 |
| 923 | hsa-miR-548a-3p, hsa-miR-744-5p, hsa-miR-195-5p, hsa-miR-10b-5p, hsa-miR-34a-3p, hsa-let-7d-3p, hsa-miR-181c-5p, hsa-miR-4306, hsa-miR-134-5p, hsa-miR-1296-5p, hsa-miR-15a-5p, hsa-miR-100-5p, hsa-miR-652-3p, hsa-miR-140-3p | 85.89 | 92.7 | 63.33 |
| 924 | hsa-miR-22-5p, hsa-miR-548a-3p, hsa-miR-1307-5p, hsa-miR-339-3p, hsa-miR-10b-5p, hsa-miR-12136, hsa-miR-337-5p, hsa-miR-188-5p, hsa-miR-4306, hsa-miR-134-5p, hsa-miR-15a-5p, hsa-miR-629-5p, hsa-miR-100-5p, hsa-miR-140-3p | 85.89 | 93.89 | 78.33 |
| 925 | hsa-miR-22-5p, hsa-miR-1307-5p, hsa-miR-339-3p, hsa-miR-195-5p, hsa-miR-10b-5p, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 85.89 | 93.25 | 73.33 |
| 926 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-652-3p | 85.89 | 97.7 | 80.00 |
| 927 | hsa-miR-1307-5p, hsa-miR-339-3p, hsa-miR-485-3p, hsa-miR-10b-5p, hsa-miR-12136, hsa-miR-34a-3p, hsa-miR-455-3p, hsa-miR-188-5p, hsa-miR-181c-5p, hsa-miR-27a-3p, hsa-miR-134-5p, hsa-miR-629-5p, hsa-miR-100-5p, hsa-miR-224-5p, hsa-miR-652-3p | 85.89 | 90.00 | 76.67 |
| 928 | hsa-miR-22-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-4306, hsa-miR-27a-3p, hsa-miR-15a-5p, hsa-miR-629-5p, hsa-miR-100-5p, hsa-miR-652-3p | 85.89 | 93.17 | 85.00 |
| 929 | hsa-miR-1307-5p, hsa-miR-339-3p, hsa-miR-485-3p, hsa-miR-10b-5p, hsa-miR-12136, hsa-miR-34a-3p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-224-5p, hsa-let-7i-3p, hsa-miR-652-3p | 85.89 | 95.00 | 80.00 |
| 930 | hsa-miR-1307-5p, hsa-miR-339-3p, hsa-miR-485-3p, hsa-miR-10b-5p, hsa-miR-12136, hsa-miR-34a-3p, hsa-miR-188-5p, hsa-miR-27a-3p, hsa-miR-134-5p, hsa-miR-629-5p, hsa-miR-100-5p, hsa-miR-224-5p, hsa-miR-652-3p | 85.89 | 91.98 | 73.33 |
| 931 | hsa-miR-22-5p, hsa-miR-744-5p, hsa-miR-195-5p, hsa-miR-188-5p, hsa-miR-181c-5p, hsa-miR-4306, hsa-miR-27a-3p, hsa-miR-15a-5p, hsa-miR-629-5p, hsa-miR-183-3p, hsa-miR-652-3p, hsa-miR-140-3p | 85.89 | 94.52 | 65.00 |
| 932 | hsa-miR-1307-5p, hsa-miR-339-3p, hsa-miR-744-5p, hsa-miR-195-5p, hsa-miR-10b-5p, hsa-miR-12136, hsa-miR-455-3p, hsa-miR-188-5p, hsa-miR-27a-3p, hsa-miR-134-5p, hsa-miR-1296-5p, hsa-miR-100-5p, hsa-miR-183-3p, hsa-let-7i-3p, hsa-miR-652-3p, hsa-miR-140-3p | 85.78 | 94.29 | 73.33 |
| 933 | hsa-miR-22-5p, hsa-miR-1307-5p, hsa-miR-339-3p, hsa-miR-485-3p, hsa-miR-34a-3p, hsa-miR-455-3p, hsa-miR-337-5p, hsa-miR-188-5p, hsa-miR-27a-3p, hsa-miR-134-5p, hsa-miR-15a-5p, hsa-miR-629-5p, hsa-miR-224-5p, hsa-miR-652-3p | 85.78 | 94.05 | 71.67 |
| 934 | hsa-miR-22-5p, hsa-miR-339-3p, hsa-miR-744-5p, hsa-miR-195-5p, hsa-miR-485-3p, hsa-miR-34a-3p, hsa-let-7d-3p, hsa-miR-455-3p, hsa-miR-337-5p, hsa-miR-181c-5p, hsa-miR-27a-3p, hsa-miR-134-5p, hsa-miR-1296-5p, hsa-miR-15a-5p, hsa-miR-100-5p, hsa-miR-183-3p, hsa-let-7i-3p, hsa-miR-140-3p | 85.78 | 91.9 | 73.33 |
| 935 | hsa-miR-22-5p, hsa-miR-548a-3p, hsa-miR-485-3p, hsa-miR-10b-5p, hsa-miR-12136, hsa-miR-34a-3p, hsa-miR-337-5p, hsa-miR-188-5p, hsa-miR-4306, hsa-miR-15a-5p, hsa-miR-629-5p, hsa-miR-100-5p, hsa-miR-652-3p, hsa-miR-140-3p | 85.78 | 91.19 | 68.33 |
| 936 | hsa-miR-1307-5p, hsa-miR-339-3p, hsa-miR-485-3p, hsa-miR-12136, hsa-miR-34a-3p, hsa-miR-455-3p, hsa-miR-337-5p, hsa-miR-188-5p, hsa-miR-27a-3p, hsa-miR-134-5p, hsa-miR-629-5p, hsa-miR-100-5p, hsa-miR-224-5p, hsa-let-7i-3p, hsa-miR-652-3p | 85.78 | 91.43 | 76.67 |
| 937 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-455-3p, hsa-miR-337-5p, hsa-miR-188-5p, hsa-miR-181c-5p, hsa-miR-27a-3p, hsa-miR-134-5p, hsa-miR-629-5p, hsa-miR-224-5p, hsa-let-7i-3p, hsa-miR-652-3p | 85.78 | 93.33 | 76.67 |
| 938 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-455-3p, hsa-miR-337-5p, hsa-miR-188-5p, hsa-miR-181c-5p, hsa-miR-27a-3p, hsa-miR-134-5p, hsa-miR-629-5p, hsa-miR-224-5p, hsa-miR-652-3p | 85.78 | 91.67 | 76.67 |
| 939 | hsa-miR-22-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-15a-5p, hsa-miR-629-5p, hsa-miR-100-5p, hsa-miR-652-3p | 85.78 | 89.92 | 73.33 |
| 940 | hsa-miR-22-5p, hsa-miR-1307-5p, hsa-miR-339-3p, hsa-miR-485-3p, hsa-miR-12136, hsa-miR-34a-3p, hsa-miR-455-3p, hsa-miR-337-5p, hsa-miR-188-5p, hsa-miR-181c-5p, hsa-miR-27a-3p, hsa-miR-134-5p, hsa-miR-629-5p, hsa-miR-224-5p, hsa-let-7i-3p, hsa-miR-652-3p | 85.78 | 93.33 | 76.67 |
| 941 | hsa-miR-22-5p, hsa-miR-548a-3p, hsa-miR-195-5p, hsa-miR-10b-5p, hsa-miR-12136, hsa-miR-34a-3p, hsa-miR-337-5p, hsa-miR-188-5p, hsa-miR-181c-5p, hsa-miR-4306, hsa-miR-15a-5p, hsa-miR-629-5p, hsa-miR-100-5p, hsa-miR-183-3p, hsa-miR-652-3p, hsa-miR-140-3p | 85.78 | 92.94 | 68.33 |
| 942 | hsa-miR-22-5p, hsa-miR-548a-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-27a-3p, hsa-miR-1296-5p, hsa-miR-100-5p, hsa-miR-652-3p, hsa-miR-140-3p | 85.78 | 90.08 | 68.33 |
| 943 | hsa-miR-22-5p, hsa-miR-1307-5p, hsa-miR-339-3p, hsa-miR-10b-5p, hsa-miR-12136, hsa-miR-34a-3p, hsa-let-7d-3p, hsa-miR-337-5p, hsa-miR-188-5p, hsa-miR-181c-5p, hsa-miR-27a-3p, hsa-miR-134-5p, hsa-miR-1296-5p, hsa-miR-15a-5p, hsa-miR-100-5p, hsa-miR-183-3p, hsa-miR-224-5p | 85.00 | 93.25 | 78.33 |
| 944 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-let-7d-3p, hsa-miR-337-5p, hsa-miR-188-5p, hsa-miR-181c-5p, hsa-miR-27a-3p, hsa-miR-134-5p, hsa-miR-1296-5p, hsa-miR-15a-5p, hsa-miR-100-5p, hsa-miR-224-5p, hsa-let-7i-3p, hsa-miR-652-3p | 85.00 | 93.89 | 73.33 |
| 945 | hsa-miR-1307-5p, hsa-miR-339-3p, hsa-miR-10b-5p, hsa-miR-12136, hsa-miR-34a-3p, hsa-miR-455-3p, hsa-miR-188-5p, hsa-miR-181c-5p, hsa-miR-27a-3p, hsa-miR-134-5p, hsa-miR-15a-5p, hsa-miR-629-5p, hsa-miR-100-5p, hsa-miR-183-3p, hsa-miR-224-5p, hsa-miR-652-3p | 85.00 | 94.44 | 70.00 |
| 946 | hsa-miR-548a-3p, hsa-miR-339-3p, hsa-miR-195-5p, hsa-miR-12136, hsa-let-7d-3p, hsa-miR-337-5p, hsa-miR-188-5p, hsa-miR-4306, hsa-miR-134-5p, hsa-miR-1296-5p, hsa-miR-15a-5p, hsa-miR-183-3p, hsa-miR-140-3p | 84.89 | 87.22 | 66.67 |
| 947 | hsa-miR-1307-5p, hsa-miR-339-3p, hsa-miR-10b-5p, hsa-miR-12136, hsa-miR-455-3p, hsa-miR-337-5p, hsa-miR-188-5p, hsa-miR-181c-5p, hsa-miR-27a-3p, hsa-miR-134-5p, hsa-miR-629-5p, hsa-miR-183-3p, hsa-miR-224-5p, hsa-miR-652-3p | 84.89 | 91.35 | 70.00 |
| 948 | hsa-miR-22-5p, hsa-miR-1307-5p, hsa-miR-339-3p, hsa-miR-12136, hsa-miR-34a-3p, hsa-miR-455-3p, hsa-miR-337-5p, hsa-miR-188-5p, hsa-miR-27a-3p, hsa-miR-134-5p, hsa-miR-15a-5p, hsa-miR-629-5p, hsa-miR-183-3p, hsa-miR-224-5p, hsa-miR-652-3p | 84.78 | 93.49 | 68.33 |
| 949 | hsa-miR-1307-5p, hsa-miR-339-3p, hsa-miR-485-3p, hsa-miR-10b-5p, hsa-miR-12136, hsa-miR-455-3p, hsa-miR-337-5p, hsa-miR-188-5p, hsa-miR-27a-3p, hsa-miR-134-5p, hsa-miR-629-5p, hsa-miR-100-5p, hsa-miR-224-5p, hsa-miR-652-3p | 84.78 | 92.38 | 75.00 |
| 950 | hsa-miR-22-5p, hsa-miR-548a-3p, hsa-miR-339-3p, hsa-miR-485-3p, hsa-miR-10b-5p, hsa-miR-12136, hsa-miR-337-5p, hsa-miR-188-5p, hsa-miR-4306, hsa-miR-27a-3p, hsa-miR-15a-5p, hsa-miR-629-5p, hsa-miR-100-5p, hsa-miR-652-3p | 84.78 | 90.87 | 85.00 |
| 951 | hsa-miR-1307-5p, hsa-miR-339-3p, hsa-miR-744-5p, hsa-miR-485-3p, hsa-miR-455-3p, hsa-miR-181c-5p, hsa-miR-4306, hsa-miR-27a-3p, hsa-miR-134-5p, hsa-miR-1296-5p, hsa-miR-183-3p, hsa-miR-224-5p | 84.78 | 92.14 | 71.67 |
| 952 | hsa-miR-22-5p, hsa-miR-1307-5p, hsa-miR-339-3p, hsa-miR-195-5p, hsa-miR-12136, hsa-miR-34a-3p, hsa-miR-455-3p, hsa-miR-337-5p, hsa-miR-188-5p, hsa-miR-181c-5p, hsa-miR-27a-3p, hsa-miR-134-5p, hsa-miR-15a-5p, hsa-miR-629-5p, hsa-miR-183-3p, hsa-miR-224-5p, hsa-miR-652-3p | 84.78 | 92.22 | 70.00 |
| 953 | hsa-miR-22-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-27a-3p, hsa-miR-134-5p, hsa-miR-1296-5p, hsa-miR-15a-5p, hsa-miR-100-5p, hsa-let-7i-3p, hsa-miR-652-3p | 84.78 | 94.13 | 78.33 |
| 954 | hsa-miR-22-5p, hsa-miR-548a-3p, hsa-miR-485-3p, hsa-miR-10b-5p, hsa-miR-12136, hsa-miR-34a-3p, hsa-miR-337-5p, hsa-miR-188-5p, hsa-miR-4306, hsa-miR-15a-5p, hsa-miR-629-5p, hsa-miR-100-5p, hsa-miR-652-3p | 84.78 | 90.4 | 73.33 |
| 955 | hsa-miR-1307-5p, hsa-miR-339-3p, hsa-miR-485-3p, hsa-miR-10b-5p, hsa-miR-12136, hsa-miR-34a-3p, hsa-miR-188-5p, hsa-miR-181c-5p, hsa-miR-27a-3p, hsa-miR-134-5p, hsa-miR-629-5p, hsa-miR-100-5p, hsa-miR-224-5p, hsa-let-7i-3p, hsa-miR-652-3p | 84.78 | 92.3 | 73.33 |
| 956 | hsa-miR-22-5p, hsa-miR-548a-3p, hsa-miR-10b-5p, hsa-miR-12136, hsa-miR-34a-3p, hsa-miR-337-5p, hsa-miR-188-5p, hsa-miR-4306, hsa-miR-15a-5p, hsa-miR-629-5p, hsa-miR-100-5p, hsa-miR-183-3p, hsa-miR-652-3p | 84.78 | 92.46 | 81.67 |
| 957 | hsa-miR-1307-5p, hsa-miR-12136, hsa-miR-188-5p, hsa-miR-134-5p, hsa-miR-629-5p, hsa-miR-100-5p, hsa-miR-224-5p, hsa-let-7i-3p, hsa-miR-140-3p | 84.78 | 88.57 | 73.33 |
| 958 | hsa-miR-22-5p, hsa-miR-1307-5p, hsa-miR-339-3p, hsa-miR-10b-5p, hsa-miR-12136, hsa-miR-337-5p, hsa-miR-188-5p, hsa-miR-27a-3p, hsa-miR-134-5p, hsa-miR-629-5p, hsa-miR-183-3p, hsa-miR-224-5p, hsa-miR-652-3p | 84.78 | 90.71 | 68.33 |
| 959 | hsa-miR-1307-5p, hsa-miR-339-3p, hsa-miR-485-3p, hsa-miR-10b-5p, hsa-miR-12136, hsa-miR-455-3p, hsa-miR-337-5p, hsa-miR-188-5p, hsa-miR-27a-3p, hsa-miR-134-5p, hsa-miR-629-5p, hsa-miR-224-5p, hsa-miR-652-3p | 84.78 | 91.35 | 71.67 |
| 960 | hsa-miR-22-5p, hsa-miR-548a-3p, hsa-miR-339-3p, hsa-miR-744-5p, hsa-miR-195-5p, hsa-miR-485-3p, hsa-miR-10b-5p, hsa-miR-34a-3p, hsa-let-7d-3p, hsa-miR-337-5p, hsa-miR-181c-5p, hsa-miR-4306, hsa-miR-27a-3p, hsa-miR-1296-5p, hsa-miR-15a-5p, hsa-miR-100-5p, hsa-miR-140-3p | 84.78 | 91.83 | 68.33 |
| 961 | hsa-miR-1307-5p, hsa-miR-339-3p, hsa-miR-485-3p, hsa-miR-10b-5p, hsa-miR-12136, hsa-miR-188-5p, hsa-miR-27a-3p, hsa-miR-134-5p, hsa-miR-629-5p, hsa-miR-100-5p, hsa-miR-224-5p, hsa-let-7i-3p, hsa-miR-652-3p | 84.78 | 92.62 | 75.00 |
| 962 | hsa-miR-22-5p, hsa-miR-1307-5p, hsa-miR-339-3p, hsa-miR-485-3p, hsa-miR-10b-5p, hsa-miR-12136, hsa-miR-337-5p, hsa-miR-188-5p, hsa-miR-27a-3p, hsa-miR-134-5p, hsa-miR-15a-5p, hsa-miR-629-5p, hsa-miR-100-5p, hsa-miR-652-3p | 84.78 | 91.35 | 78.33 |
| 963 | hsa-miR-22-5p, hsa-miR-548a-3p, hsa-miR-744-5p, hsa-miR-195-5p, hsa-miR-485-3p, hsa-miR-12136, hsa-miR-34a-3p, hsa-miR-337-5p, hsa-miR-4306, hsa-miR-1296-5p, hsa-miR-15a-5p, hsa-miR-100-5p, hsa-miR-183-3p, hsa-miR-224-5p, hsa-miR-652-3p, hsa-miR-140-3p | 84.78 | 92.54 | 68.33 |
| 964 | hsa-miR-22-5p, hsa-miR-1307-5p, hsa-miR-339-3p, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-183-3p, hsa-miR-224-5p, hsa-miR-652-3p | 84.78 | 91.11 | 71.67 |
| 965 | hsa-miR-548a-3p, hsa-miR-744-5p, hsa-miR-195-5p, hsa-miR-10b-5p, hsa-miR-34a-3p, hsa-let-7d-3p, hsa-miR-455-3p, hsa-miR-181c-5p, hsa-miR-4306, hsa-miR-134-5p, hsa-miR-1296-5p, hsa-miR-183-3p, hsa-miR-224-5p, hsa-miR-652-3p, hsa-miR-140-3p | 84.78 | 92.78 | 75.00 |
| 966 | hsa-miR-22-5p, hsa-miR-548a-3p, hsa-miR-485-3p, hsa-miR-10b-5p, hsa-miR-12136, hsa-miR-34a-3p, hsa-miR-337-5p, hsa-miR-188-5p, hsa-miR-4306, hsa-miR-27a-3p, hsa-miR-15a-5p, hsa-miR-629-5p, hsa-miR-100-5p, hsa-miR-652-3p | 84.78 | 92.22 | 76.67 |
| 967 | hsa-miR-548a-3p, hsa-miR-339-3p, hsa-miR-744-5p, hsa-miR-195-5p, hsa-miR-10b-5p, hsa-miR-34a-3p, hsa-miR-455-3p, hsa-miR-181c-5p, hsa-miR-4306, hsa-miR-134-5p, hsa-miR-1296-5p, hsa-miR-15a-5p, hsa-miR-100-5p, hsa-miR-183-3p, hsa-miR-224-5p, hsa-miR-652-3p, hsa-miR-140-3p | 84.78 | 94.37 | 70.00 |
| 968 | hsa-miR-22-5p, hsa-miR-1307-5p, hsa-miR-339-3p, hsa-miR-10b-5p, hsa-miR-12136, hsa-miR-34a-3p, hsa-miR-455-3p, hsa-miR-337-5p, hsa-miR-188-5p, hsa-miR-27a-3p, hsa-miR-134-5p, hsa-miR-629-5p, hsa-miR-224-5p, hsa-miR-652-3p | 84.78 | 90.79 | 73.33 |
| 969 | hsa-miR-22-5p, hsa-miR-548a-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-1296-5p, hsa-miR-100-5p, hsa-miR-652-3p, hsa-miR-140-3p | 84.78 | 89.44 | 71.67 |
| 970 | hsa-miR-22-5p, hsa-miR-10b-5p, hsa-miR-12136, hsa-miR-34a-3p, hsa-miR-337-5p, hsa-miR-188-5p, hsa-miR-4306, hsa-miR-15a-5p, hsa-miR-629-5p, hsa-miR-100-5p, hsa-miR-183-3p, hsa-miR-652-3p | 84.78 | 94.29 | 81.67 |
| 971 | hsa-miR-22-5p, hsa-miR-1307-5p, hsa-miR-339-3p, hsa-miR-12136, hsa-miR-34a-3p, hsa-miR-337-5p, hsa-miR-188-5p, hsa-miR-27a-3p, hsa-miR-134-5p, hsa-miR-15a-5p, hsa-miR-629-5p, hsa-miR-183-3p, hsa-miR-224-5p, hsa-miR-652-3p | 84.78 | 91.27 | 68.33 |
| 972 | hsa-miR-22-5p, hsa-miR-548a-3p, hsa-miR-1307-5p, hsa-miR-339-3p, hsa-miR-744-5p, hsa-miR-195-5p, hsa-miR-455-3p, hsa-miR-337-5p, hsa-miR-188-5p, hsa-miR-4306, hsa-miR-27a-3p, hsa-miR-1296-5p, hsa-miR-15a-5p, hsa-miR-629-5p, hsa-miR-183-3p, hsa-miR-224-5p, hsa-let-7i-3p, hsa-miR-140-3p | 84.78 | 92.06 | 68.33 |
| 973 | hsa-miR-1307-5p, hsa-miR-339-3p, hsa-miR-485-3p, hsa-miR-10b-5p, hsa-miR-12136, hsa-miR-34a-3p, hsa-miR-188-5p, hsa-miR-27a-3p, hsa-miR-134-5p, hsa-miR-629-5p, hsa-miR-100-5p, hsa-miR-224-5p, hsa-let-7i-3p, hsa-miR-652-3p | 84.78 | 91.59 | 73.33 |
| 974 | hsa-miR-1307-5p, hsa-miR-339-3p, hsa-miR-10b-5p, hsa-miR-12136, hsa-miR-455-3p, hsa-miR-337-5p, hsa-miR-188-5p, hsa-miR-27a-3p, hsa-miR-134-5p, hsa-miR-629-5p, hsa-miR-183-3p, hsa-miR-224-5p, hsa-miR-652-3p | 84.67 | 91.51 | 78.33 |
| 975 | hsa-miR-22-5p, hsa-miR-548a-3p, hsa-miR-1307-5p, hsa-miR-339-3p, hsa-miR-744-5p, hsa-miR-485-3p, hsa-miR-10b-5p, hsa-miR-12136, hsa-miR-34a-3p, hsa-miR-337-5p, hsa-miR-4306, hsa-miR-27a-3p, hsa-miR-134-5p, hsa-miR-15a-5p, hsa-miR-629-5p, hsa-miR-100-5p, hsa-miR-652-3p, hsa-miR-140-3p | 84.67 | 96.11 | 75.00 |
| 976 | hsa-miR-22-5p, hsa-miR-548a-3p, hsa-miR-1307-5p, hsa-miR-485-3p, hsa-miR-10b-5p, hsa-miR-12136, hsa-miR-337-5p, hsa-miR-188-5p, hsa-miR-4306, hsa-miR-15a-5p, hsa-miR-629-5p, hsa-miR-100-5p, hsa-miR-224-5p, hsa-miR-652-3p, hsa-miR-140-3p | 84.67 | 92.94 | 71.67 |
| 977 | hsa-miR-22-5p, hsa-miR-548a-3p, hsa-miR-10b-5p, hsa-miR-12136, hsa-miR-34a-3p, hsa-miR-337-5p, hsa-miR-188-5p, hsa-miR-4306, hsa-miR-15a-5p, hsa-miR-629-5p, hsa-miR-100-5p, hsa-miR-183-3p, hsa-miR-652-3p, hsa-miR-140-3p | 84.67 | 92.46 | 68.33 |
| 978 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-455-3p, hsa-miR-337-5p, hsa-miR-188-5p, hsa-miR-27a-3p, hsa-miR-134-5p, hsa-miR-629-5p, hsa-miR-224-5p, hsa-let-7i-3p, hsa-miR-652-3p | 84.67 | 95.4 | 78.33 |
| 979 | hsa-miR-548a-3p, hsa-miR-744-5p, hsa-miR-195-5p, hsa-miR-10b-5p, hsa-let-7d-3p, hsa-miR-181c-5p, hsa-miR-4306, hsa-miR-1296-5p, hsa-miR-100-5p, hsa-miR-652-3p, hsa-miR-140-3p | 83.78 | 89.76 | 68.33 |
| 980 | hsa-miR-1307-5p, hsa-miR-339-3p, hsa-miR-10b-5p, hsa-miR-455-3p, hsa-miR-337-5p, hsa-miR-188-5p, hsa-miR-181c-5p, hsa-miR-27a-3p, hsa-miR-134-5p, hsa-miR-629-5p, hsa-miR-183-3p, hsa-miR-224-5p, hsa-miR-652-3p | 83.78 | 90.16 | 71.67 |
| 981 | hsa-miR-1307-5p, hsa-miR-744-5p, hsa-miR-10b-5p, hsa-miR-34a-3p, hsa-let-7d-3p, hsa-miR-455-3p, hsa-miR-337-5p, hsa-miR-188-5p, hsa-miR-4306, hsa-miR-134-5p, hsa-miR-1296-5p, hsa-miR-629-5p, hsa-miR-100-5p, hsa-miR-183-3p, hsa-miR-652-3p | 83.78 | 88.97 | 76.67 |
| 982 | hsa-miR-22-5p, hsa-miR-548a-3p, hsa-miR-485-3p, hsa-miR-10b-5p, hsa-miR-12136, hsa-miR-34a-3p, hsa-miR-337-5p, hsa-miR-188-5p, hsa-miR-181c-5p, hsa-miR-4306, hsa-miR-15a-5p, hsa-miR-629-5p, hsa-miR-100-5p, hsa-miR-652-3p | 83.67 | 87.14 | 73.33 |
| 983 | hsa-miR-22-5p, hsa-miR-1307-5p, hsa-miR-339-3p, hsa-miR-485-3p, hsa-miR-10b-5p, hsa-miR-12136, hsa-miR-34a-3p, hsa-miR-337-5p, hsa-miR-188-5p, hsa-miR-27a-3p, hsa-miR-15a-5p, hsa-miR-629-5p, hsa-miR-100-5p, hsa-let-7i-3p, hsa-miR-652-3p | 83.67 | 90.63 | 78.33 |
| 984 | hsa-miR-1307-5p, hsa-miR-339-3p, hsa-miR-485-3p, hsa-miR-10b-5p, hsa-miR-12136, hsa-miR-34a-3p, hsa-miR-455-3p, hsa-miR-337-5p, hsa-miR-188-5p, hsa-miR-181c-5p, hsa-miR-27a-3p, hsa-miR-134-5p, hsa-miR-629-5p, hsa-miR-100-5p, hsa-miR-224-5p, hsa-let-7i-3p, hsa-miR-652-3p | 83.67 | 90.16 | 70.00 |
| 985 | hsa-miR-548a-3p, hsa-miR-1307-5p, hsa-miR-339-3p, hsa-miR-485-3p, hsa-miR-12136, hsa-miR-34a-3p, hsa-miR-455-3p, hsa-miR-181c-5p, hsa-miR-27a-3p, hsa-miR-1296-5p, hsa-miR-15a-5p, hsa-miR-100-5p, hsa-let-7i-3p, hsa-miR-652-3p, hsa-miR-140-3p | 83.56 | 88.49 | 73.33 |
| 986 | hsa-miR-1307-5p, hsa-miR-339-3p, hsa-miR-10b-5p, hsa-let-7d-3p, hsa-miR-455-3p, hsa-miR-337-5p, hsa-miR-188-5p, hsa-miR-181c-5p, hsa-miR-27a-3p, hsa-miR-629-5p, hsa-miR-183-3p, hsa-let-7i-3p | 83.56 | 88.89 | 70.00 |
| 987 | hsa-miR-1307-5p, hsa-miR-339-3p, hsa-miR-485-3p, hsa-miR-10b-5p, hsa-miR-12136, hsa-miR-34a-3p, hsa-miR-455-3p, hsa-miR-337-5p, hsa-miR-188-5p, hsa-miR-27a-3p, hsa-miR-134-5p, hsa-miR-629-5p, hsa-miR-100-5p, hsa-miR-224-5p, hsa-let-7i-3p, hsa-miR-652-3p | 83.56 | 90.87 | 70.00 |
| 988 | hsa-miR-1307-5p, hsa-miR-339-3p, hsa-miR-485-3p, hsa-miR-10b-5p, hsa-miR-12136, hsa-miR-455-3p, hsa-miR-337-5p, hsa-miR-188-5p, hsa-miR-27a-3p, hsa-miR-134-5p, hsa-miR-629-5p, hsa-miR-100-5p, hsa-miR-224-5p, hsa-let-7i-3p, hsa-miR-652-3p | 83.56 | 91.43 | 75.00 |
| 989 | hsa-miR-22-5p, hsa-miR-1307-5p, hsa-miR-744-5p, hsa-miR-10b-5p, hsa-miR-34a-3p, hsa-miR-188-5p, hsa-miR-4306, hsa-miR-134-5p, hsa-miR-15a-5p, hsa-miR-629-5p, hsa-miR-224-5p, hsa-let-7i-3p, hsa-miR-140-3p | 83.44 | 86.83 | 68.33 |
| 990 | hsa-miR-548a-3p, hsa-miR-1307-5p, hsa-miR-339-3p, hsa-miR-12136, hsa-miR-455-3p, hsa-miR-337-5p, hsa-miR-188-5p, hsa-miR-181c-5p, hsa-miR-27a-3p, hsa-let-7i-3p, hsa-miR-652-3p, hsa-miR-140-3p | 82.67 | 86.03 | 61.67 |
| 991 | hsa-miR-22-5p, hsa-miR-548a-3p, hsa-miR-195-5p, hsa-miR-10b-5p, hsa-miR-12136, hsa-miR-34a-3p, hsa-miR-337-5p, hsa-miR-188-5p, hsa-miR-4306, hsa-miR-15a-5p, hsa-miR-629-5p, hsa-miR-100-5p, hsa-miR-183-3p, hsa-miR-652-3p | 82.56 | 91.35 | 65.00 |
| 992 | hsa-miR-22-5p, hsa-miR-548a-3p, hsa-miR-744-5p, hsa-miR-195-5p, hsa-miR-10b-5p, hsa-miR-34a-3p, hsa-let-7d-3p, hsa-miR-337-5p, hsa-miR-181c-5p, hsa-miR-4306, hsa-miR-1296-5p, hsa-miR-652-3p, hsa-miR-140-3p | 82.56 | 87.86 | 66.67 |
| 993 | hsa-miR-22-5p, hsa-miR-548a-3p, hsa-miR-1307-5p, hsa-miR-744-5p, hsa-miR-195-5p, hsa-miR-485-3p, hsa-miR-10b-5p, hsa-miR-12136, hsa-miR-34a-3p, hsa-miR-455-3p, hsa-miR-4306, hsa-miR-27a-3p, hsa-miR-629-5p, hsa-miR-224-5p, hsa-let-7i-3p, hsa-miR-652-3p, hsa-miR-140-3p | 82.56 | 92.14 | 68.33 |
| 994 | hsa-miR-1307-5p, hsa-miR-485-3p, hsa-miR-12136, hsa-miR-455-3p, hsa-miR-337-5p, hsa-miR-188-5p, hsa-miR-134-5p, hsa-miR-629-5p, hsa-miR-224-5p, hsa-let-7i-3p, hsa-miR-652-3p | 82.56 | 90.48 | 75.00 |
| 995 | hsa-miR-1307-5p, hsa-miR-339-3p, hsa-miR-485-3p, hsa-miR-10b-5p, hsa-miR-12136, hsa-miR-455-3p, hsa-miR-337-5p, hsa-miR-188-5p, hsa-miR-27a-3p, hsa-miR-134-5p, hsa-miR-629-5p, hsa-miR-224-5p, hsa-let-7i-3p, hsa-miR-652-3p | 82.56 | 90.4 | 71.67 |
| 996 | hsa-miR-1307-5p, hsa-miR-339-3p, hsa-miR-485-3p, hsa-miR-10b-5p, hsa-miR-12136, hsa-miR-188-5p, hsa-miR-27a-3p, hsa-miR-134-5p, hsa-miR-629-5p, hsa-miR-100-5p, hsa-miR-224-5p, hsa-miR-652-3p | 82.56 | 89.05 | 65.00 |
| 997 | hsa-miR-22-5p, hsa-miR-548a-3p, hsa-miR-744-5p, hsa-miR-485-3p, hsa-miR-10b-5p, hsa-miR-12136, hsa-miR-34a-3p, hsa-miR-337-5p, hsa-miR-4306, hsa-miR-1296-5p, hsa-miR-15a-5p, hsa-miR-100-5p, hsa-miR-183-3p, hsa-miR-140-3p | 82.44 | 92.14 | 65.00 |
| 998 | hsa-miR-1307-5p, hsa-miR-339-3p, hsa-miR-485-3p, hsa-miR-10b-5p, hsa-miR-12136, hsa-miR-34a-3p, hsa-miR-455-3p, hsa-miR-337-5p, hsa-miR-188-5p, hsa-miR-27a-3p, hsa-miR-134-5p, hsa-miR-629-5p, hsa-miR-224-5p, hsa-let-7i-3p, hsa-miR-652-3p | 82.44 | 90.4 | 70.00 |
| 999 | hsa-miR-1307-5p, hsa-miR-339-3p, hsa-miR-485-3p, hsa-miR-10b-5p, hsa-miR-12136, hsa-miR-34a-3p, hsa-miR-455-3p, hsa-miR-337-5p, hsa-miR-188-5p, hsa-miR-181c-5p, hsa-miR-27a-3p, hsa-miR-134-5p, hsa-miR-629-5p, hsa-miR-224-5p, hsa-let-7i-3p, hsa-miR-652-3p | 81.33 | 91.43 | 71.67 |
| 1000 | hsa-miR-1307-5p, hsa-miR-12136, hsa-miR-188-5p, hsa-miR-27a-3p, hsa-miR-1296-5p, hsa-miR-629-5p, hsa-miR-100-5p | 80.56 | 86.43 | 70.00 |
| 1001 | hsa-miR-548a-3p, hsa-miR-1307-5p, hsa-miR-339-3p, hsa-miR-744-5p, hsa-miR-195-5p, hsa-miR-485-3p, hsa-miR-34a-3p, hsa-miR-188-5p, hsa-miR-27a-3p, hsa-miR-1296-5p, hsa-let-7i-3p | 76.00 | 80.95 | 51.67 |
| 1003 | combination 712 + rs28544423 | 80.35 | 83.95 | 73.96 |
| 1004 | combination 713 + rs28544423 | 80.29 | 85.44 | 82.76 |
| 1005 | combination 714 + rs28544423 | 81.46 | 86.51 | 83.77 |
| 1006 | combination 715 + rs28544423 | 84.68 | 87.85 | 88.02 |
| 1007 | combination 718 + rs71384446 | 89.12 | 91.13 | 91.93 |
| 1008 | combination 719 + rs28544423 | 91.29 | 94.21 | 89.92 |
| 1009 | combination 724 + rs11647727 | 89.12 | 92.92 | 92.43 |
| 1010 | combination 725 + rs11647727 | 89.18 | 94.91 | 92.12 |
| 1011 | combination 726 + rs28544423 | 90.18 | 93.13 | 92.71 |
| 1012 | combination 727 + rs7193058 | 86.96 | 93.13 | 92.46 |
| 1013 | combination 728 + s13333226 | 86.96 | 92.92 | 93.04 |
| 1014 | combination 729 + rs4297685 | 88.01 | 94.72 | 92.43 |
| 1015 | combination 730 + rs11647727 | 88.01 | 94.41 | 92.37 |
| 1016 | combination 737 + rs11862974 | 88.01 | 94.26 | 92.98 |
| 1017 | combination 738 + rs28544423 | 91.35 | 96.21 | 92.06 |
| 1018 | combination 711 + rs4293393 | 72.63 | 73.03 | 91.44 |
| 1019 | combination 712 + rs4293393 | 80.35 | 83.95 | 95.14 |
| 1020 | combination 713 + rs4293393 | 80.35 | 85.59 | 93.88 |
| 1021 | combination 714 + rs4293393 | 82.46 | 86.72 | 94.49 |
| 1022 | combination 715 + rs4293393 | 84.68 | 87.85 | 93.57 |
| 1023 | combination 718 + rs4293393 | 88.07 | 94.1 | 94.1 |
| 1024 | combination 719 + rs4293393 | 87.95 | 94.21 | 94.21 |
| 1025 | combination 725 + rs4293393 | 90.18 | 93.13 | 92.52 |
| 1026 | combination 726 + rs4293393 | 89.06 | 93.13 | 92.71 |
| 1027 | combination 727 + rs4293393 | 89.06 | 93.13 | 92.46 |
| 1028 | combination 728 + rs4293393 | 86.96 | 93.74 | 93.24 |
| 1029 | combination 729 + rs4293393 | 89.12 | 94.36 | 92.63 |
| 1030 | combination 737 + rs4293393 | 89.18 | 96.56 | 92.72 |

This is the list of the best 30 mRNAs selected by the algorithm sorted by importance for tumour grade: hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p. The inventors combined these miRNAs, together or not with age and UMOD SNP variant, creating unique combinations leading to a further significant increase in sensitivity, specificity, accuracy and predictive power for tumour grade. (Table 18).

**Table 18. Combinations that allow higher sensitivity, specificity, accuracy and predictive power for tumour grade.**

| N | Combinations | Accuracy | ROC AUC | Recall |
|---|---|---|---|---|
| 1031 | hsa-miR-33a-5p | 73.57 | 60.35 | 60.00 |
| 1032 | hsa-miR-33a-5p, hsa-miR-125a-5p | 73.57 | 77.19 | 75.10 |
| 1033 | hsa-miR-33a-5p, hsa-miR-125a-5p, hsa-miR-424-3p | 73.51 | 73.95 | 70.0 |
| 1034 | hsa-miR-33a-5p, hsa-miR-125a-5p, hsa-miR-424-3p, hsa-miR-744-5p | 82.4 | 88.59 | 80.52 |
| 1035 | hsa-miR-33a-5p, hsa-miR-125a-5p, hsa-miR-424-3p, hsa-miR-744-5p, hsa-miR-125b-5p | 81.29 | 85.55 | 79.5 |
| 1036 | hsa-miR-33a-5p, hsa-miR-125a-5p, hsa-miR-424-3p, hsa-miR-744-5p, hsa-miR-125b-5p, hsa-miR-502-3p | 81.35 | 88.35 | 82.3 |
| 1037 | hsa-miR-33a-5p, hsa-miR-125a-5p, hsa-miR-424-3p, hsa-miR-744-5p, hsa-miR-125b-5p, hsa-miR-502-3p, hsa-miR-629-5p | 82.4 | 92.5 | 85.5 |
| 1038 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p | 82.4 | 91.92 | 84.67 |
| 1039 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p | 84.62 | 90.74 | 88.67 |
| 1040 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p | 85.67 | 88.27 | 86.5 |
| 1041 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p | 86.73 | 94.14 | 87.31 |
| 1042 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p | 86.73 | 91.09 | 86.5 |
| 1043 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p | 88.89 | 94.39 | 88.9 |
| 1044 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p | 88.89 | 97.72 | 90.0 |
| 1045 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p | 88.95 | 97.38 | 88.67 |
| 1046 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p | 88.95 | 96.82 | 96.67 |
| 1047 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p | 90.06 | 96.76 | 92.67 |
| 1048 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p | 88.95 | 96.48 | 92.67 |
| 1049 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p | 88.95 | 96.43 | 92.67 |
| 1050 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-222-3p | 86.73 | 95.2 | 88.67 |
| 1051 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-222-3p, hsa-miR-548am-5p | 86.73 | 95.47 | 92.67 |
| 1052 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-222-3p, hsa-miR-548am-5p, hsa-miR-151a-5p | 87.84 | 95.78 | 92.67 |
| 1053 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p | 87.84 | 95.47 | 85.33 |
| 1054 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p | 88.95 | 96.34 | 85.33 |
| 1055 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p, hsa-miR-664a-3p | 87.84 | 94.83 | 85.33 |
| 1056 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p, hsa-miR-664a-3p, hsa-miR-501-5p | 86.73 | 95.38 | 92.0 |
| 1057 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p, hsa-miR-664a-3p, hsa-miR-501-5p, hsa-miR-424-5p | 88.95 | 95.69 | 92.0 |
| 1058 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p, hsa-miR-664a-3p, hsa-miR-501-5p, hsa-miR-424-5p, hsa-miR-144-3p | 86.73 | 95.41 | 88.0 |
| 1059 | hsa-miR-33a-5p, hsa-miR-125a-5p, hsa-miR-424-3p, hsa-miR-744-5p, hsa-miR-125b-5p, hsa-miR-502-3p, hsa-miR-629-5p, hsa-miR-128-3p, hsa-miR-21-3p, hsa-miR-31-5p, hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p, hsa-miR-664a-3p, hsa-miR-501-5p, hsa-miR-424-5p, hsa-miR-144-3p, hsa-let-7d-3p | 87.89 | 95.82 | 96.67 |
| 1060 | hsa-miR-545-5p, hsa-miR-134-5p, hsa-miR-195-5p, hsa-miR-103a-2-5p, hsa-miR-652-3p, hsa-miR-339-3p, hsa-miR-151a-5p, hsa-miR-126-5p, hsa-miR-579-3p, hsa-miR-664a-3p, hsa-miR-144-3p, hsa-let-7d-3p, hsa-miR-27b-3p, hsa-miR-744-5p, hsa-miR-125b-5p, hsa-miR-502-3p, hsa-miR-31-5p | 97.89 | 100.00 | 100.00 |
| 1061 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-151a-5p, hsa-miR-126-5p, hsa-miR-579-3p, hsa-miR-664a-3p, hsa-miR-144-3p, hsa-let-7d-3p, hsa-miR-27b-3p, hsa-miR-744-5p, hsa-miR-125b-5p, hsa-miR-502-3p, hsa-miR-31-5p | 97.89 | 100.00 | 96.67 |
| 1062 | hsa-miR-545-5p, hsa-miR-134-5p, hsa-miR-195-5p, hsa-miR-103a-2-5p, hsa-miR-652-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 97.78 | 100.00 | 93.33 |
| 1063 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 96.67 | 99.44 | 96.67 |
| 1064 | hsa-miR-545-5p, hsa-miR-134-5p, hsa-miR-195-5p, hsa-miR-103a-2-5p, hsa-miR-652-3p, hsa-miR-483-5p, hsa-miR-151a-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 95.78 | 100.00 | 93.33 |
| 1065 | hsa-miR-545-5p, hsa-miR-134-5p, hsa-miR-195-5p, hsa-miR-103a-2-5p, hsa-miR-652-3p, hsa-miR-151a-5p, hsa-miR-126-5p, hsa-miR-579-3p, hsa-miR-664a-3p, hsa-miR-144-3p, hsa-miR-27b-3p, hsa-miR-744-5p, hsa-miR-502-3p, hsa-miR-31-5p | 95.67 | 98.25 | 91.67 |
| 1066 | hsa-miR-545-5p, hsa-miR-134-5p, hsa-miR-195-5p, hsa-miR-103a-2-5p, hsa-miR-652-3p, hsa-miR-339-3p, hsa-miR-151a-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 95.67 | 97.78 | 91.67 |
| 1067 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-151a-5p, hsa-miR-126-5p, hsa-miR-579-3p, hsa-miR-664a-3p, hsa-miR-501-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 95.67 | 98.33 | 93.33 |
| 1068 | hsa-miR-545-5p, hsa-miR-134-5p, hsa-miR-195-5p, hsa-miR-103a-2-5p, hsa-miR-652-3p, hsa-miR-7-1-3p, hsa-miR-483-5p, hsa-miR-151a-5p, hsa-miR-126-5p, hsa-miR-579-3p, hsa-miR-664a-3p, hsa-let-7d-3p, hsa-miR-27b-3p, hsa-miR-744-5p, hsa-miR-502-3p, hsa-miR-31-5p | 95.67 | 100.00 | 88.33 |
| 1069 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-151a-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 95.67 | 100.00 | 96.67 |
| 1070 | hsa-miR-545-5p, hsa-miR-134-5p, hsa-miR-195-5p, hsa-miR-103a-2-5p, hsa-miR-652-3p, hsa-miR-339-3p, hsa-miR-126-5p, hsa-miR-579-3p, hsa-miR-664a-3p, hsa-miR-424-5p, hsa-miR-144-3p, hsa-let-7d-3p, hsa-miR-744-5p, hsa-miR-125b-5p, hsa-miR-502-3p, hsa-miR-31-5p | 95.67 | 97.78 | 91.67 |
| 1071 | hsa-miR-545-5p, hsa-miR-134-5p, hsa-miR-195-5p, hsa-miR-103a-2-5p, hsa-miR-652-3p, hsa-miR-151a-5p, hsa-miR-126-5p, hsa-miR-579-3p, hsa-miR-664a-3p, hsa-miR-424-5p, hsa-miR-144-3p, hsa-let-7d-3p, hsa-miR-744-5p, hsa-miR-125b-5p, hsa-miR-502-3p, hsa-miR-31-5p | 95.67 | 98.33 | 91.67 |
| 1072 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p, hsa-miR-31-5p | 95.67 | 100.00 | 96.67 |
| 1073 | hsa-miR-545-5p, hsa-miR-134-5p, hsa-miR-195-5p, hsa-miR-103a-2-5p, hsa-miR-652-3p, hsa-miR-339-3p, hsa-miR-7-1-3p, hsa-miR-126-5p, hsa-miR-579-3p, hsa-miR-664a-3p, hsa-miR-144-3p, hsa-miR-27b-3p, hsa-miR-744-5p, hsa-miR-502-3p, hsa-miR-31-5p | 95.56 | 95.95 | 91.67 |
| 1074 | hsa-miR-545-5p, hsa-miR-134-5p, hsa-miR-195-5p, hsa-miR-103a-2-5p, hsa-miR-652-3p, hsa-miR-339-3p, hsa-miR-7-1-3p, hsa-miR-151a-5p, hsa-miR-126-5p, hsa-miR-579-3p, hsa-miR-664a-3p, hsa-miR-144-3p, hsa-let-7d-3p, hsa-miR-27b-3p, hsa-miR-744-5p, hsa-miR-125b-5p, hsa-miR-502-3p, hsa-miR-629-5p, hsa-miR-31-5p | 94.78 | 100.00 | 91.67 |
| 1075 | hsa-miR-545-5p, hsa-miR-134-5p, hsa-miR-103a-2-5p, hsa-miR-652-3p, hsa-miR-339-3p, hsa-miR-483-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p, hsa-miR-31-5p | 94.56 | 98.33 | 93.33 |
| 1076 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 94.56 | 97.78 | 93.33 |
| 1077 | hsa-miR-545-5p, hsa-miR-134-5p, hsa-miR-195-5p, hsa-miR-103a-2-5p, hsa-miR-652-3p, hsa-miR-339-3p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 94.56 | 99.44 | 96.67 |
| 1078 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 94.56 | 97.62 | 93.33 |
| 1079 | hsa-miR-545-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 94.56 | 98.89 | 93.33 |
| 1080 | hsa-miR-545-5p, hsa-miR-134-5p, hsa-miR-195-5p, hsa-miR-103a-2-5p, hsa-miR-652-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-125b-5p, hsa-miR-502-3p, hsa-miR-31-5p | 94.56 | 98.89 | 93.33 |
| 1081 | hsa-miR-545-5p, hsa-miR-134-5p, hsa-miR-195-5p, hsa-miR-103a-2-5p, hsa-miR-652-3p, hsa-miR-483-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 94.56 | 99.44 | 93.33 |
| 1082 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-744-5p, hsa-miR-125b-5p, hsa-miR-502-3p, hsa-miR-31-5p | 94.56 | 99.44 | 96.67 |
| 1083 | hsa-miR-545-5p, hsa-miR-134-5p, hsa-miR-195-5p, hsa-miR-103a-2-5p, hsa-miR-652-3p, hsa-miR-339-3p, hsa-miR-151a-5p, hsa-miR-126-5p, hsa-miR-579-3p, hsa-miR-664a-3p, hsa-miR-424-5p, hsa-miR-144-3p, hsa-let-7d-3p, hsa-miR-744-5p, hsa-miR-125b-5p, hsa-miR-502-3p, hsa-miR-31-5p | 94.56 | 98.33 | 88.33 |
| 1084 | hsa-miR-545-5p, hsa-miR-134-5p, hsa-miR-195-5p, hsa-miR-103a-2-5p, hsa-miR-652-3p, hsa-miR-151a-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 94.56 | 98.33 | 91.67 |
| 1085 | hsa-miR-545-5p, hsa-miR-134-5p, hsa-miR-195-5p, hsa-miR-103a-2-5p, hsa-miR-652-3p, hsa-miR-339-3p, hsa-miR-7-1-3p, hsa-miR-483-5p, hsa-miR-126-5p, hsa-miR-579-3p, hsa-miR-664a-3p, hsa-miR-144-3p, hsa-miR-27b-3p, hsa-miR-744-5p, hsa-miR-502-3p, hsa-miR-31-5p | 94.44 | 96.67 | 91.67 |
| 1086 | hsa-miR-545-5p, hsa-miR-134-5p, hsa-miR-195-5p, hsa-miR-103a-2-5p, hsa-miR-652-3p, hsa-miR-339-3p, hsa-miR-7-1-3p, hsa-miR-483-5p, hsa-miR-151a-5p, hsa-miR-126-5p, hsa-miR-579-3p, hsa-miR-664a-3p, hsa-let-7d-3p, hsa-miR-27b-3p, hsa-miR-744-5p, hsa-miR-502-3p, hsa-miR-31-5p | 94.44 | 98.89 | 88.33 |
| 1087 | hsa-miR-134-5p, hsa-miR-195-5p, hsa-miR-103a-2-5p, hsa-miR-652-3p, hsa-miR-339-3p, hsa-miR-126-5p, hsa-miR-579-3p, hsa-miR-424-5p, hsa-miR-144-3p, hsa-let-7d-3p, hsa-miR-125b-5p, hsa-miR-502-3p, hsa-miR-31-5p | 94.44 | 96.11 | 90.00 |
| 1088 | hsa-miR-134-5p, hsa-miR-195-5p, hsa-miR-103a-2-5p, hsa-miR-652-3p, hsa-miR-339-3p, hsa-miR-151a-5p, hsa-miR-126-5p, hsa-miR-579-3p, hsa-miR-424-5p, hsa-miR-144-3p, hsa-let-7d-3p, hsa-miR-125b-5p, hsa-miR-502-3p, hsa-miR-31-5p | 94.44 | 95.56 | 90.00 |
| 1089 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 93.67 | 99.44 | 96.67 |
| 1090 | hsa-miR-545-5p, hsa-miR-134-5p, hsa-miR-195-5p, hsa-miR-103a-2-5p, hsa-miR-652-3p, hsa-miR-339-3p, hsa-miR-7-1-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-502-3p, hsa-miR-629-5p, hsa-miR-128-3p, hsa-miR-31-5p | 93.56 | 98.33 | 96.67 |
| 1091 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 93.56 | 98.89 | 96.67 |
| 1092 | hsa-miR-545-5p, hsa-miR-134-5p, hsa-miR-195-5p, hsa-miR-103a-2-5p, hsa-miR-652-3p, hsa-miR-339-3p, hsa-miR-7-1-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-629-5p, hsa-miR-31-5p | 93.56 | 98.89 | 91.67 |
| 1093 | hsa-miR-545-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-501-5p, hsa-miR-424-5p, hsa-let-7d-3p, hsa-miR-27b-3p, hsa-miR-744-5p, hsa-miR-125b-5p, hsa-miR-502-3p, hsa-miR-31-5p | 93.56 | 98.33 | 86.67 |
| 1094 | hsa-miR-545-5p, hsa-miR-134-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p, hsa-miR-31-5p | 93.56 | 99.44 | 93.33 |
| 1095 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-548am-5p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p | 93.56 | 97.3 | 93.33 |
| 1096 | hsa-miR-545-5p, hsa-miR-103a-2-5p, hsa-miR-652-3p, hsa-miR-339-3p, hsa-miR-483-5p, hsa-miR-579-3p, hsa-miR-424-5p, hsa-miR-144-3p, hsa-let-7d-3p, hsa-miR-744-5p, hsa-miR-125b-5p, hsa-miR-502-3p, hsa-miR-21-3p, hsa-miR-31-5p | 93.44 | 97.22 | 88.33 |
| 1097 | hsa-miR-545-5p, hsa-miR-134-5p, hsa-miR-195-5p, hsa-miR-103a-2-5p, hsa-miR-652-3p, hsa-miR-7-1-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 93.44 | 99.44 | 96.67 |
| 1098 | hsa-miR-545-5p, hsa-miR-134-5p, hsa-miR-195-5p, hsa-miR-103a-2-5p, hsa-miR-652-3p, hsa-miR-339-3p, hsa-miR-151a-5p, hsa-miR-126-5p, hsa-miR-579-3p, hsa-miR-664a-3p, hsa-miR-501-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 93.44 | 98.33 | 85.00 |
| 1099 | hsa-miR-545-5p, hsa-miR-134-5p, hsa-miR-103a-2-5p, hsa-miR-652-3p, hsa-miR-339-3p, hsa-miR-483-5p, hsa-miR-222-3p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-21-3p, hsa-miR-31-5p | 93.44 | 98.33 | 88.33 |
| 1100 | hsa-miR-545-5p, hsa-miR-134-5p, hsa-miR-103a-2-5p, hsa-miR-652-3p, hsa-miR-339-3p, hsa-miR-483-5p, hsa-miR-151a-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p, hsa-miR-31-5p | 93.44 | 98.33 | 88.33 |
| 1101 | hsa-miR-545-5p, hsa-miR-134-5p, hsa-miR-103a-2-5p, hsa-miR-652-3p, hsa-miR-339-3p, hsa-miR-7-1-3p, hsa-miR-483-5p, hsa-miR-548am-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p, hsa-miR-31-5p | 93.44 | 99.44 | 93.33 |
| 1102 | hsa-miR-545-5p, hsa-miR-134-5p, hsa-miR-195-5p, hsa-miR-103a-2-5p, hsa-miR-652-3p, hsa-miR-7-1-3p, hsa-miR-483-5p, hsa-miR-126-5p, hsa-miR-579-3p, hsa-miR-664a-3p, hsa-miR-144-3p, hsa-miR-27b-3p, hsa-miR-744-5p, hsa-miR-502-3p, hsa-miR-629-5p, hsa-miR-31-5p | 93.44 | 97.06 | 91.67 |
| 1103 | hsa-miR-545-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-501-5p, hsa-miR-424-5p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 93.44 | 98.89 | 86.67 |
| 1104 | hsa-miR-545-5p, hsa-miR-195-5p, hsa-miR-103a-2-5p, hsa-miR-652-3p, hsa-miR-339-3p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 93.44 | 97.22 | 80.00 |
| 1105 | hsa-miR-134-5p, hsa-miR-195-5p, hsa-miR-103a-2-5p, hsa-miR-652-3p, hsa-miR-126-5p, hsa-miR-579-3p, hsa-miR-424-5p, hsa-miR-144-3p, hsa-let-7d-3p, hsa-miR-125b-5p, hsa-miR-502-3p, hsa-miR-31-5p | 93.33 | 96.11 | 86.67 |
| 1106 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-629-5p, hsa-miR-31-5p | 92.44 | 97.78 | 93.33 |
| 1107 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-483-5p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p, hsa-miR-128-3p, hsa-miR-31-5p | 92.44 | 97.62 | 88.33 |
| 1108 | hsa-miR-545-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p, hsa-miR-31-5p | 92.44 | 98.33 | 81.67 |
| 1109 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-502-3p, hsa-miR-128-3p, hsa-miR-31-5p | 92.44 | 99.44 | 96.67 |
| 1110 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-151a-5p, hsa-miR-126-5p, hsa-miR-579-3p, hsa-miR-664a-3p, hsa-miR-144-3p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 92.44 | 99.44 | 96.67 |
| 1111 | hsa-miR-545-5p, hsa-miR-134-5p, hsa-miR-195-5p, hsa-miR-103a-2-5p, hsa-miR-652-3p, hsa-miR-369-3p, hsa-miR-483-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 92.44 | 100.00 | 88.33 |
| 1112 | hsa-miR-545-5p, hsa-miR-134-5p, hsa-miR-195-5p, hsa-miR-103a-2-5p, hsa-miR-652-3p, hsa-miR-369-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 92.44 | 98.33 | 93.33 |
| 1113 | hsa-miR-545-5p, hsa-miR-134-5p, hsa-miR-195-5p, hsa-miR-103a-2-5p, hsa-miR-652-3p, hsa-miR-7-1-3p, hsa-miR-151a-5p, hsa-miR-126-5p, hsa-miR-579-3p, hsa-miR-664a-3p, hsa-miR-501-5p, hsa-miR-144-3p, hsa-miR-27b-3p, hsa-miR-744-5p, hsa-miR-502-3p, hsa-miR-31-5p | 92.44 | 97.78 | 88.33 |
| 1114 | hsa-miR-545-5p, hsa-miR-195-5p, hsa-miR-103a-2-5p, hsa-miR-652-3p, hsa-miR-7-1-3p, hsa-miR-151a-5p, hsa-miR-126-5p, hsa-miR-579-3p, hsa-miR-664a-3p, hsa-miR-501-5p, hsa-miR-144-3p, hsa-miR-27b-3p, hsa-miR-744-5p, hsa-miR-502-3p, hsa-miR-31-5p | 92.44 | 98.33 | 88.33 |
| 1115 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-483-5p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 92.44 | 98.89 | 93.33 |
| 1116 | hsa-miR-545-5p, hsa-miR-134-5p, hsa-miR-195-5p, hsa-miR-103a-2-5p, hsa-miR-652-3p, hsa-miR-339-3p, hsa-miR-7-1-3p, hsa-miR-483-5p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-502-3p, hsa-miR-629-5p, hsa-miR-31-5p | 92.44 | 97.78 | 91.67 |
| 1117 | hsa-miR-545-5p, hsa-miR-195-5p, hsa-miR-103a-2-5p, hsa-miR-652-3p, hsa-miR-339-3p, hsa-miR-369-3p, hsa-miR-483-5p, hsa-miR-126-5p, hsa-miR-579-3p, hsa-miR-664a-3p, hsa-miR-501-5p, hsa-miR-424-5p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 92.44 | 99.44 | 90.00 |
| 1118 | hsa-miR-545-5p, hsa-miR-134-5p, hsa-miR-195-5p, hsa-miR-103a-2-5p, hsa-miR-652-3p, hsa-miR-7-1-3p, hsa-miR-483-5p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-502-3p, hsa-miR-629-5p, hsa-miR-31-5p | 92.44 | 98.33 | 91.67 |
| 1119 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-144-3p, hsa-miR-27b-3p, hsa-miR-744-5p, hsa-miR-125b-5p, hsa-miR-502-3p, hsa-miR-31-5p | 92.33 | 97.22 | 90.00 |
| 1120 | hsa-miR-134-5p, hsa-miR-195-5p, hsa-miR-103a-2-5p, hsa-miR-652-3p, hsa-miR-483-5p, hsa-miR-126-5p, hsa-miR-579-3p, hsa-miR-424-5p, hsa-miR-144-3p, hsa-let-7d-3p, hsa-miR-27b-3p, hsa-miR-125b-5p, hsa-miR-502-3p, hsa-miR-31-5p | 92.33 | 97.06 | 93.33 |
| 1121 | hsa-miR-545-5p, hsa-miR-134-5p, hsa-miR-195-5p, hsa-miR-103a-2-5p, hsa-miR-652-3p, hsa-miR-7-1-3p, hsa-miR-548am-5p, hsa-miR-151a-5p, hsa-miR-126-5p, hsa-miR-579-3p, hsa-miR-664a-3p, hsa-miR-501-5p, hsa-miR-144-3p, hsa-miR-27b-3p, hsa-miR-744-5p, hsa-miR-502-3p, hsa-miR-31-5p | 92.33 | 98.33 | 88.33 |
| 1122 | hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-424-5p, hsa-let-7d-3p, hsa-miR-125b-5p, hsa-miR-502-3p, hsa-miR-21-3p, hsa-miR-31-5p | 92.33 | 97.3 | 93.33 |
| 1123 | hsa-miR-545-5p, hsa-miR-134-5p, hsa-miR-195-5p, hsa-miR-103a-2-5p, hsa-miR-652-3p, hsa-miR-7-1-3p, hsa-miR-483-5p, hsa-miR-126-5p, hsa-miR-579-3p, hsa-miR-664a-3p, hsa-miR-144-3p, hsa-miR-27b-3p, hsa-miR-744-5p, hsa-miR-502-3p, hsa-miR-31-5p | 92.33 | 96.51 | 91.67 |
| 1124 | hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-501-5p, hsa-miR-424-5p, hsa-miR-144-3p, hsa-let-7d-3p, hsa-miR-125b-5p, hsa-miR-502-3p, hsa-miR-21-3p, hsa-miR-31-5p | 92.33 | 95.63 | 90.00 |
| 1125 | hsa-miR-195-5p, hsa-miR-103a-2-5p, hsa-miR-652-3p, hsa-miR-339-3p, hsa-miR-483-5p, hsa-miR-222-3p, hsa-miR-126-5p, hsa-miR-579-3p, hsa-miR-664a-3p, hsa-miR-501-5p, hsa-miR-424-5p, hsa-let-7d-3p, hsa-miR-27b-3p, hsa-miR-125b-5p, hsa-miR-31-5p | 92.33 | 98.33 | 93.33 |
| 1126 | hsa-miR-545-5p, hsa-miR-134-5p, hsa-miR-195-5p, hsa-miR-103a-2-5p, hsa-miR-652-3p, hsa-miR-369-3p, hsa-miR-7-1-3p, hsa-miR-483-5p, hsa-miR-151a-5p, hsa-miR-126-5p, hsa-miR-579-3p, hsa-miR-664a-3p, hsa-miR-501-5p, hsa-let-7d-3p, hsa-miR-27b-3p, hsa-miR-128-3p, hsa-miR-21-3p, hsa-miR-31-5p | 92.33 | 98.89 | 88.33 |
| 1127 | hsa-miR-134-5p, hsa-miR-103a-2-5p, hsa-miR-652-3p, hsa-miR-339-3p, hsa-miR-483-5p, hsa-miR-222-3p, hsa-miR-579-3p, hsa-miR-424-5p, hsa-let-7d-3p, hsa-miR-125b-5p, hsa-miR-21-3p, hsa-miR-31-5p | 92.33 | 97.78 | 93.33 |
| 1128 | hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-125b-5p, hsa-miR-502-3p, hsa-miR-31-5p | 92.33 | 96.11 | 90.00 |
| 1129 | hsa-miR-195-5p, hsa-miR-103a-2-5p, hsa-miR-652-3p, hsa-miR-339-3p, hsa-miR-483-5p, hsa-miR-222-3p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-125b-5p, hsa-miR-31-5p | 92.33 | 96.67 | 90.00 |
| 1130 | hsa-miR-134-5p, hsa-miR-195-5p, hsa-miR-103a-2-5p, hsa-miR-652-3p, hsa-miR-151a-5p, hsa-miR-126-5p, hsa-miR-579-3p, hsa-miR-424-5p, hsa-miR-144-3p, hsa-let-7d-3p, hsa-miR-744-5p, hsa-miR-125b-5p, hsa-miR-502-3p, hsa-miR-21-3p, hsa-miR-31-5p | 92.33 | 96.67 | 88.33 |
| 1131 | hsa-miR-134-5p, hsa-miR-195-5p, hsa-miR-103a-2-5p, hsa-miR-652-3p, hsa-miR-339-3p, hsa-miR-126-5p, hsa-miR-579-3p, hsa-miR-424-5p, hsa-miR-144-3p, hsa-let-7d-3p, hsa-miR-125b-5p, hsa-miR-502-3p, hsa-miR-21-3p, hsa-miR-31-5p | 92.33 | 96.67 | 90.00 |
| 1132 | hsa-miR-545-5p, hsa-miR-134-5p, hsa-miR-195-5p, hsa-miR-103a-2-5p, hsa-miR-652-3p, hsa-miR-7-1-3p, hsa-miR-483-5p, hsa-miR-126-5p, hsa-miR-579-3p, hsa-miR-664a-3p, hsa-miR-27b-3p, hsa-miR-744-5p, hsa-miR-502-3p, hsa-miR-629-5p, hsa-miR-31-5p | 92.33 | 96.35 | 91.67 |
| 1133 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 92.33 | 96.67 | 85.00 |
| 1134 | hsa-miR-545-5p, hsa-miR-195-5p, hsa-miR-103a-2-5p, hsa-miR-652-3p, hsa-miR-7-1-3p, hsa-miR-483-5p, hsa-miR-151a-5p, hsa-miR-126-5p, hsa-miR-579-3p, hsa-miR-664a-3p, hsa-miR-501-5p, hsa-miR-144-3p, hsa-miR-27b-3p, hsa-miR-744-5p, hsa-miR-125b-5p, hsa-miR-502-3p | 92.33 | 96.51 | 80.00 |
| 1135 | hsa-miR-545-5p, hsa-miR-103a-2-5p, hsa-miR-652-3p, hsa-miR-339-3p, hsa-miR-483-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 92.33 | 97.78 | 85.00 |
| 1136 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-579-3p, hsa-miR-424-5p, hsa-let-7d-3p, hsa-miR-125b-5p, hsa-miR-502-3p, hsa-miR-21-3p, hsa-miR-31-5p | 92.33 | 96.75 | 88.33 |
| 1137 | hsa-miR-545-5p, hsa-miR-134-5p, hsa-miR-195-5p, hsa-miR-103a-2-5p, hsa-miR-652-3p, hsa-miR-339-3p, hsa-miR-151a-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-125b-5p, hsa-miR-502-3p, hsa-miR-31-5p | 92.33 | 98.17 | 88.33 |
| 1138 | hsa-miR-134-5p, hsa-miR-195-5p, hsa-miR-103a-2-5p, hsa-miR-652-3p, hsa-miR-7-1-3p, hsa-miR-548am-5p, hsa-miR-151a-5p, hsa-miR-126-5p, hsa-miR-579-3p, hsa-miR-664a-3p, hsa-miR-501-5p, hsa-miR-144-3p, hsa-miR-27b-3p, hsa-miR-744-5p, hsa-miR-502-3p, hsa-miR-31-5p | 92.33 | 97.22 | 85.00 |
| 1139 | hsa-miR-545-5p, hsa-miR-195-5p, hsa-miR-103a-2-5p, hsa-miR-652-3p, hsa-miR-339-3p, hsa-miR-483-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 92.33 | 97.22 | 85.00 |
| 1140 | hsa-miR-545-5p, hsa-miR-134-5p, hsa-miR-103a-2-5p, hsa-miR-652-3p, hsa-miR-339-3p, hsa-miR-483-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p | 92.33 | 97.22 | 83.33 |
| 1141 | hsa-miR-545-5p, hsa-miR-195-5p, hsa-miR-103a-2-5p, hsa-miR-652-3p, hsa-miR-339-3p, hsa-miR-483-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 92.33 | 97.22 | 85.00 |
| 1142 | hsa-miR-545-5p, hsa-miR-134-5p, hsa-miR-195-5p, hsa-miR-103a-2-5p, hsa-miR-652-3p, hsa-miR-339-3p, hsa-miR-151a-5p, hsa-miR-126-5p, hsa-miR-579-3p, hsa-miR-664a-3p, hsa-miR-27b-3p, hsa-miR-744-5p, hsa-miR-502-3p, hsa-miR-31-5p | 92.22 | 95.95 | 88.33 |
| 1143 | hsa-miR-545-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-21-3p, hsa-miR-31-5p | 92.22 | 98.33 | 83.33 |
| 1144 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-502-3p, hsa-miR-629-5p, hsa-miR-128-3p, hsa-miR-31-5p | 91.44 | 98.89 | 96.67 |
| 1145 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-128-3p, hsa-miR-31-5p | 91.33 | 99.44 | 96.67 |
| 1146 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-222-3p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-21-3p, hsa-miR-31-5p | 91.33 | 98.33 | 91.67 |
| 1147 | hsa-miR-545-5p, hsa-miR-134-5p, hsa-miR-195-5p, hsa-miR-103a-2-5p, hsa-miR-652-3p, hsa-miR-369-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-128-3p, hsa-miR-31-5p | 91.33 | 98.89 | 93.33 |
| 1148 | hsa-miR-545-5p, hsa-miR-134-5p, hsa-miR-195-5p, hsa-miR-103a-2-5p, hsa-miR-652-3p, hsa-miR-483-5p, hsa-miR-151a-5p, hsa-miR-126-5p, hsa-miR-579-3p, hsa-miR-664a-3p, hsa-miR-144-3p, hsa-miR-27b-3p, hsa-miR-744-5p, hsa-miR-125b-5p, hsa-miR-502-3p, hsa-miR-31-5p | 91.33 | 97.78 | 90.00 |
| 1149 | hsa-miR-134-5p, hsa-miR-195-5p, hsa-miR-103a-2-5p, hsa-miR-652-3p, hsa-miR-339-3p, hsa-miR-483-5p, hsa-miR-222-3p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-31-5p | 91.33 | 97.22 | 93.33 |
| 1150 | hsa-miR-545-5p, hsa-miR-134-5p, hsa-miR-195-5p, hsa-miR-103a-2-5p, hsa-miR-652-3p, hsa-miR-7-1-3p, hsa-miR-483-5p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-502-3p, hsa-miR-629-5p, hsa-miR-128-3p, hsa-miR-31-5p | 91.33 | 99.44 | 96.67 |
| 1151 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 91.33 | 97.22 | 88.33 |
| 1152 | hsa-miR-545-5p, hsa-miR-134-5p, hsa-miR-195-5p, hsa-miR-103a-2-5p, hsa-miR-652-3p, hsa-miR-7-1-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-502-3p, hsa-miR-629-5p, hsa-miR-31-5p | 91.33 | 97.78 | 91.67 |
| 1153 | hsa-miR-134-5p, hsa-miR-195-5p, hsa-miR-103a-2-5p, hsa-miR-652-3p, hsa-miR-339-3p, hsa-miR-151a-5p, hsa-miR-126-5p, hsa-miR-579-3p, hsa-miR-144-3p, hsa-let-7d-3p, hsa-miR-125b-5p, hsa-miR-502-3p, hsa-miR-21-3p, hsa-miR-31-5p | 91.33 | 96.11 | 88.33 |
| 1154 | hsa-miR-545-5p, hsa-miR-134-5p, hsa-miR-195-5p, hsa-miR-652-3p, hsa-miR-339-3p, hsa-miR-483-5p, hsa-miR-548am-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 91.33 | 97.22 | 88.33 |
| 1155 | hsa-miR-545-5p, hsa-miR-134-5p, hsa-miR-195-5p, hsa-miR-103a-2-5p, hsa-miR-339-3p, hsa-miR-483-5p, hsa-miR-222-3p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-31-5p | 91.33 | 98.89 | 86.67 |
| 1156 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-151a-5p, hsa-miR-126-5p, hsa-miR-579-3p, hsa-miR-664a-3p, hsa-miR-144-3p, hsa-let-7d-3p, hsa-miR-27b-3p, hsa-miR-744-5p, hsa-miR-125b-5p, hsa-miR-502-3p, hsa-miR-128-3p, hsa-miR-31-5p | 91.33 | 98.89 | 85.00 |
| 1157 | hsa-miR-545-5p, hsa-miR-195-5p, hsa-miR-103a-2-5p, hsa-miR-652-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 91.33 | 96.35 | 85.00 |
| 1158 | hsa-miR-134-5p, hsa-miR-195-5p, hsa-miR-103a-2-5p, hsa-miR-652-3p, hsa-miR-339-3p, hsa-miR-483-5p, hsa-miR-151a-5p, hsa-miR-126-5p, hsa-miR-579-3p, hsa-miR-144-3p, hsa-let-7d-3p, hsa-miR-125b-5p, hsa-miR-502-3p, hsa-miR-21-3p, hsa-miR-31-5p | 91.33 | 96.59 | 93.33 |
| 1159 | hsa-miR-545-5p, hsa-miR-134-5p, hsa-miR-195-5p, hsa-miR-652-3p, hsa-miR-339-3p, hsa-miR-7-1-3p, hsa-miR-151a-5p, hsa-miR-126-5p, hsa-miR-664a-3p, hsa-miR-144-3p, hsa-miR-27b-3p, hsa-miR-744-5p, hsa-miR-502-3p, hsa-miR-21-3p, hsa-miR-31-5p | 91.33 | 97.22 | 85.00 |
| 1160 | hsa-miR-545-5p, hsa-miR-195-5p, hsa-miR-103a-2-5p, hsa-miR-652-3p, hsa-miR-339-3p, hsa-miR-369-3p, hsa-miR-483-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 91.33 | 98.89 | 85.00 |
| 1161 | hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-424-5p, hsa-miR-144-3p, hsa-let-7d-3p, hsa-miR-27b-3p, hsa-miR-125b-5p, hsa-miR-21-3p, hsa-miR-31-5p | 91.33 | 95.00 | 90.00 |
| 1162 | hsa-miR-134-5p, hsa-miR-195-5p, hsa-miR-103a-2-5p, hsa-miR-652-3p, hsa-miR-7-1-3p, hsa-miR-151a-5p, hsa-miR-126-5p, hsa-miR-579-3p, hsa-miR-664a-3p, hsa-miR-501-5p, hsa-miR-144-3p, hsa-miR-27b-3p, hsa-miR-744-5p, hsa-miR-502-3p, hsa-miR-31-5p | 91.33 | 96.11 | 88.33 |
| 1163 | hsa-miR-134-5p, hsa-miR-195-5p, hsa-miR-103a-2-5p, hsa-miR-652-3p, hsa-miR-339-3p, hsa-miR-483-5p, hsa-miR-222-3p, hsa-miR-126-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-31-5p | 91.33 | 97.22 | 93.33 |
| 1164 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 91.33 | 98.89 | 93.33 |
| 1165 | hsa-miR-545-5p, hsa-miR-134-5p, hsa-miR-195-5p, hsa-miR-103a-2-5p, hsa-miR-652-3p, hsa-miR-7-1-3p, hsa-miR-483-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-502-3p, hsa-miR-31-5p | 91.22 | 98.33 | 91.67 |
| 1166 | hsa-miR-545-5p, hsa-miR-134-5p, hsa-miR-195-5p, hsa-miR-103a-2-5p, hsa-miR-652-3p, hsa-miR-483-5p, hsa-miR-151a-5p, hsa-miR-126-5p, hsa-miR-579-3p, hsa-miR-664a-3p, hsa-miR-144-3p, hsa-miR-27b-3p, hsa-miR-744-5p, hsa-miR-502-3p, hsa-miR-31-5p | 91.22 | 98.17 | 88.33 |
| 1167 | hsa-miR-134-5p, hsa-miR-195-5p, hsa-miR-103a-2-5p, hsa-miR-7-1-3p, hsa-miR-222-3p, hsa-miR-548am-5p, hsa-miR-151a-5p, hsa-miR-126-5p, hsa-miR-579-3p, hsa-miR-664a-3p, hsa-miR-501-5p, hsa-miR-424-5p, hsa-let-7d-3p, hsa-miR-744-5p, hsa-miR-502-3p, hsa-miR-128-3p, hsa-miR-31-5p | 91.22 | 95.24 | 93.33 |
| 1168 | hsa-miR-545-5p, hsa-miR-134-5p, hsa-miR-103a-2-5p, hsa-miR-652-3p, hsa-miR-339-3p, hsa-miR-369-3p, hsa-miR-483-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 91.22 | 97.06 | 81.67 |
| 1169 | hsa-miR-545-5p, hsa-miR-103a-2-5p, hsa-miR-652-3p, hsa-miR-339-3p, hsa-miR-483-5p, hsa-miR-579-3p, hsa-miR-501-5p, hsa-miR-424-5p, hsa-let-7d-3p, hsa-miR-744-5p, hsa-miR-125b-5p, hsa-miR-502-3p, hsa-miR-31-5p | 91.22 | 97.22 | 80.00 |
| 1170 | hsa-miR-545-5p, hsa-miR-103a-2-5p, hsa-miR-652-3p, hsa-miR-339-3p, hsa-miR-483-5p, hsa-miR-579-3p, hsa-miR-501-5p, hsa-miR-424-5p, hsa-miR-144-3p, hsa-let-7d-3p, hsa-miR-125b-5p, hsa-miR-502-3p, hsa-miR-21-3p, hsa-miR-31-5p | 91.22 | 98.33 | 85.00 |
| 1171 | hsa-miR-545-5p, hsa-miR-134-5p, hsa-miR-103a-2-5p, hsa-miR-652-3p, hsa-miR-339-3p, hsa-miR-483-5p, hsa-miR-579-3p, hsa-miR-664a-3p, hsa-miR-424-5p, hsa-let-7d-3p, hsa-miR-27b-3p, hsa-miR-744-5p, hsa-miR-125b-5p, hsa-miR-31-5p | 91.22 | 95.4 | 73.33 |
| 1172 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-144-3p, hsa-miR-27b-3p, hsa-miR-744-5p, hsa-miR-502-3p, hsa-miR-31-5p | 91.22 | 97.22 | 91.67 |
| 1173 | hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-664a-3p, hsa-miR-501-5p, hsa-miR-424-5p, hsa-let-7d-3p, hsa-miR-27b-3p, hsa-miR-125b-5p, hsa-miR-502-3p, hsa-miR-21-3p, hsa-miR-31-5p | 91.22 | 96.75 | 90.00 |
| 1174 | hsa-miR-545-5p, hsa-miR-134-5p, hsa-miR-195-5p, hsa-miR-103a-2-5p, hsa-miR-652-3p, hsa-miR-483-5p, hsa-miR-126-5p, hsa-miR-579-3p, hsa-miR-664a-3p, hsa-miR-144-3p, hsa-miR-27b-3p, hsa-miR-744-5p, hsa-miR-502-3p, hsa-miR-31-5p | 91.22 | 97.06 | 91.67 |
| 1175 | hsa-miR-545-5p, hsa-miR-134-5p, hsa-miR-195-5p, hsa-miR-103a-2-5p, hsa-miR-652-3p, hsa-miR-339-3p, hsa-miR-126-5p, hsa-miR-579-3p, hsa-miR-424-5p, hsa-miR-144-3p, hsa-let-7d-3p, hsa-miR-125b-5p, hsa-miR-502-3p, hsa-miR-31-5p | 91.22 | 97.78 | 88.33 |
| 1176 | hsa-miR-545-5p, hsa-miR-134-5p, hsa-miR-195-5p, hsa-miR-103a-2-5p, hsa-miR-652-3p, hsa-miR-339-3p, hsa-miR-151a-5p, hsa-miR-126-5p, hsa-miR-579-3p, hsa-miR-664a-3p, hsa-miR-144-3p, hsa-miR-27b-3p, hsa-miR-744-5p, hsa-miR-125b-5p, hsa-miR-502-3p, hsa-miR-31-5p | 91.22 | 95.95 | 86.67 |
| 1177 | hsa-miR-545-5p, hsa-miR-103a-2-5p, hsa-miR-652-3p, hsa-miR-339-3p, hsa-miR-151a-5p, hsa-miR-579-3p, hsa-miR-664a-3p, hsa-miR-144-3p, hsa-let-7d-3p, hsa-miR-27b-3p, hsa-miR-744-5p, hsa-miR-125b-5p, hsa-miR-502-3p | 91.22 | 96.51 | 76.67 |
| 1178 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-151a-5p, hsa-miR-126-5p, hsa-miR-579-3p, hsa-miR-664a-3p, hsa-miR-144-3p, hsa-miR-27b-3p, hsa-miR-744-5p, hsa-miR-125b-5p, hsa-miR-502-3p, hsa-miR-31-5p | 91.11 | 97.22 | 86.67 |
| 1179 | hsa-miR-545-5p, hsa-miR-134-5p, hsa-miR-195-5p, hsa-miR-103a-2-5p, hsa-miR-652-3p, hsa-miR-7-1-3p, hsa-miR-483-5p, hsa-miR-126-5p, hsa-miR-579-3p, hsa-miR-664a-3p, hsa-miR-144-3p, hsa-miR-27b-3p, hsa-miR-744-5p, hsa-miR-502-3p, hsa-miR-629-5p, hsa-miR-128-3p, hsa-miR-31-5p | 91.11 | 95.08 | 83.33 |
| 1180 | hsa-miR-134-5p, hsa-miR-195-5p, hsa-miR-103a-2-5p, hsa-miR-548am-5p, hsa-miR-664a-3p, hsa-miR-424-5p, hsa-miR-125b-5p, hsa-miR-629-5p, hsa-miR-128-3p, hsa-miR-21-3p, hsa-miR-31-5p | 91.11 | 94.68 | 78.33 |
| 1181 | hsa-miR-134-5p, hsa-miR-195-5p, hsa-miR-103a-2-5p, hsa-miR-652-3p, hsa-miR-126-5p, hsa-miR-579-3p, hsa-miR-424-5p, hsa-miR-144-3p, hsa-let-7d-3p, hsa-miR-125b-5p, hsa-miR-502-3p, hsa-miR-21-3p, hsa-miR-31-5p | 91.11 | 96.11 | 93.33 |
| 1182 | hsa-miR-545-5p, hsa-miR-134-5p, hsa-miR-195-5p, hsa-miR-103a-2-5p, hsa-miR-652-3p, hsa-miR-548am-5p, hsa-miR-151a-5p, hsa-miR-126-5p, hsa-miR-579-3p, hsa-miR-664a-3p, hsa-miR-27b-3p, hsa-miR-744-5p, hsa-miR-502-3p, hsa-miR-629-5p, hsa-miR-128-3p, hsa-miR-31-5p | 91.11 | 95.24 | 76.67 |
| 1183 | hsa-miR-545-5p, hsa-miR-103a-2-5p, hsa-miR-652-3p, hsa-miR-7-1-3p, hsa-miR-151a-5p, hsa-miR-126-5p, hsa-miR-579-3p, hsa-miR-664a-3p, hsa-miR-501-5p, hsa-miR-144-3p, hsa-miR-27b-3p, hsa-miR-744-5p, hsa-miR-125b-5p, hsa-miR-502-3p | 91.11 | 95.79 | 76.67 |
| 1184 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-483-5p, hsa-miR-151a-5p, hsa-miR-126-5p, hsa-miR-579-3p, hsa-miR-664a-3p, hsa-miR-144-3p, hsa-let-7d-3p, hsa-miR-27b-3p, hsa-miR-744-5p, hsa-miR-125b-5p, hsa-miR-502-3p, hsa-miR-31-5p | 90.44 | 100.00 | 90.00 |
| 1185 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-483-5p, hsa-miR-151a-5p, hsa-miR-126-5p, hsa-miR-579-3p, hsa-miR-664a-3p, hsa-miR-144-3p, hsa-let-7d-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 90.33 | 98.89 | 93.33 |
| 1186 | hsa-miR-134-5p, hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-424-5p, hsa-miR-144-3p, hsa-let-7d-3p, hsa-miR-125b-5p, hsa-miR-502-3p, hsa-miR-21-3p, hsa-miR-31-5p | 90.22 | 96.75 | 90.00 |
| 1187 | hsa-miR-545-5p, hsa-miR-195-5p, hsa-miR-103a-2-5p, hsa-miR-652-3p, hsa-miR-339-3p, hsa-miR-483-5p, hsa-miR-548am-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p | 90.22 | 96.75 | 80.00 |
| 1188 | hsa-miR-545-5p, hsa-miR-195-5p, hsa-miR-652-3p, hsa-miR-483-5p, hsa-miR-548am-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 90.22 | 96.11 | 83.33 |
| 1189 | hsa-miR-545-5p, hsa-miR-134-5p, hsa-miR-103a-2-5p, hsa-miR-483-5p, hsa-miR-222-3p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-21-3p, hsa-miR-31-5p | 90.22 | 98.89 | 85.00 |
| 1190 | hsa-miR-134-5p, hsa-miR-195-5p, hsa-miR-103a-2-5p, hsa-miR-652-3p, hsa-miR-339-3p, hsa-miR-483-5p, hsa-miR-222-3p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-21-3p, hsa-miR-31-5p | 90.22 | 97.22 | 93.33 |
| 1191 | hsa-miR-545-5p, hsa-miR-134-5p, hsa-miR-195-5p, hsa-miR-103a-2-5p, hsa-miR-652-3p, hsa-miR-339-3p, hsa-miR-483-5p, hsa-miR-548am-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 90.22 | 96.75 | 83.33 |
| 1192 | hsa-miR-545-5p, hsa-miR-103a-2-5p, hsa-miR-652-3p, hsa-miR-483-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 90.22 | 97.22 | 83.33 |
| 1193 | hsa-miR-134-5p, hsa-miR-195-5p, hsa-miR-652-3p, hsa-miR-339-3p, hsa-miR-369-3p, hsa-miR-7-1-3p, hsa-miR-151a-5p, hsa-miR-126-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 90.22 | 95.00 | 90.00 |
| 1194 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-483-5p, hsa-miR-126-5p, hsa-miR-579-3p, hsa-miR-664a-3p, hsa-miR-501-5p, hsa-miR-424-5p, hsa-let-7d-3p, hsa-miR-27b-3p, hsa-miR-744-5p, hsa-miR-125b-5p, hsa-miR-502-3p, hsa-miR-31-5p | 90.22 | 97.78 | 85.00 |
| 1195 | hsa-miR-134-5p, hsa-miR-195-5p, hsa-miR-103a-2-5p, hsa-miR-652-3p, hsa-miR-339-3p, hsa-miR-483-5p, hsa-miR-222-3p, hsa-miR-126-5p, hsa-miR-579-3p, hsa-miR-424-5p, hsa-miR-144-3p, hsa-let-7d-3p, hsa-miR-27b-3p, hsa-miR-125b-5p, hsa-miR-21-3p, hsa-miR-31-5p | 90.22 | 96.03 | 93.33 |
| 1196 | hsa-miR-545-5p, hsa-miR-134-5p, hsa-miR-195-5p, hsa-miR-339-3p, hsa-miR-483-5p, hsa-miR-548am-5p, hsa-miR-126-5p, hsa-miR-664a-3p, hsa-miR-424-5p, hsa-let-7d-3p, hsa-miR-27b-3p, hsa-miR-744-5p, hsa-miR-125b-5p, hsa-miR-502-3p | 90.22 | 94.84 | 86.67 |
| 1197 | hsa-miR-545-5p, hsa-miR-103a-2-5p, hsa-miR-652-3p, hsa-miR-339-3p, hsa-miR-369-3p, hsa-miR-483-5p, hsa-miR-579-3p, hsa-miR-424-5p, hsa-let-7d-3p, hsa-miR-744-5p, hsa-miR-125b-5p, hsa-miR-502-3p, hsa-miR-629-5p, hsa-miR-128-3p, hsa-miR-21-3p, hsa-miR-31-5p | 90.22 | 97.06 | 81.67 |
| 1198 | hsa-miR-545-5p, hsa-miR-195-5p, hsa-miR-103a-2-5p, hsa-miR-652-3p, hsa-miR-339-3p, hsa-miR-369-3p, hsa-miR-483-5p, hsa-miR-126-5p, hsa-miR-579-3p, hsa-miR-664a-3p, hsa-miR-501-5p, hsa-miR-424-5p, hsa-let-7d-3p, hsa-miR-27b-3p, hsa-miR-744-5p, hsa-miR-125b-5p, hsa-miR-502-3p, hsa-miR-31-5p | 90.22 | 99.44 | 85.00 |
| 1199 | hsa-miR-545-5p, hsa-miR-103a-2-5p, hsa-miR-652-3p, hsa-miR-339-3p, hsa-miR-483-5p, hsa-miR-151a-5p, hsa-miR-579-3p, hsa-miR-664a-3p, hsa-miR-501-5p, hsa-miR-144-3p, hsa-let-7d-3p, hsa-miR-27b-3p, hsa-miR-744-5p, hsa-miR-125b-5p, hsa-miR-502-3p, hsa-miR-31-5p | 90.11 | 97.78 | 73.33 |
| 1200 | hsa-miR-134-5p, hsa-miR-195-5p, hsa-miR-103a-2-5p, hsa-miR-652-3p, hsa-miR-339-3p, hsa-miR-483-5p, hsa-miR-222-3p, hsa-miR-126-5p, hsa-miR-579-3p, hsa-miR-424-5p, hsa-miR-144-3p, hsa-let-7d-3p, hsa-miR-125b-5p, hsa-miR-21-3p, hsa-miR-31-5p | 90.11 | 96.19 | 86.67 |
| 1201 | hsa-miR-134-5p, hsa-miR-103a-2-5p, hsa-miR-652-3p, hsa-miR-339-3p, hsa-miR-7-1-3p, hsa-miR-483-5p, hsa-miR-579-3p, hsa-miR-424-5p, hsa-let-7d-3p, hsa-miR-502-3p, hsa-miR-21-3p, hsa-miR-31-5p | 90.11 | 93.89 | 86.67 |
| 1202 | hsa-miR-545-5p, hsa-miR-195-5p, hsa-miR-339-3p, hsa-miR-7-1-3p, hsa-miR-483-5p, hsa-miR-222-3p, hsa-miR-548am-5p, hsa-miR-151a-5p, hsa-miR-579-3p, hsa-miR-424-5p, hsa-let-7d-3p, hsa-miR-27b-3p, hsa-miR-502-3p, hsa-miR-629-5p, hsa-miR-128-3p, hsa-miR-21-3p | 90.11 | 88.89 | 85.00 |
| 1203 | hsa-miR-545-5p, hsa-miR-134-5p, hsa-miR-195-5p, hsa-miR-103a-2-5p, hsa-miR-652-3p, hsa-miR-339-3p, hsa-miR-126-5p, hsa-miR-579-3p, hsa-miR-424-5p, hsa-miR-144-3p, hsa-let-7d-3p, hsa-miR-125b-5p, hsa-miR-502-3p, hsa-miR-21-3p, hsa-miR-31-5p | 90.11 | 95.56 | 86.67 |
| 1204 | hsa-miR-134-5p, hsa-miR-103a-2-5p, hsa-miR-652-3p, hsa-miR-339-3p, hsa-miR-483-5p, hsa-miR-579-3p, hsa-miR-424-5p, hsa-let-7d-3p, hsa-miR-125b-5p, hsa-miR-502-3p, hsa-miR-31-5p | 90.11 | 95.00 | 83.33 |
| 1205 | hsa-miR-545-5p, hsa-miR-222-3p, hsa-miR-151a-5p, hsa-miR-579-3p, hsa-miR-664a-3p, hsa-miR-501-5p, hsa-miR-144-3p, hsa-miR-27b-3p, hsa-miR-744-5p, hsa-miR-125b-5p, hsa-miR-502-3p, hsa-miR-629-5p | 90.11 | 92.3 | 71.67 |
| 1206 | hsa-miR-545-5p, hsa-miR-103a-2-5p, hsa-miR-652-3p, hsa-miR-483-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 90.11 | 97.22 | 80.00 |
| 1207 | hsa-miR-545-5p, hsa-miR-195-5p, hsa-miR-103a-2-5p, hsa-miR-652-3p, hsa-miR-483-5p, hsa-miR-151a-5p, hsa-miR-126-5p, hsa-miR-579-3p, hsa-miR-664a-3p, hsa-miR-501-5p, hsa-miR-144-3p, hsa-miR-27b-3p, hsa-miR-744-5p, hsa-miR-502-3p, hsa-miR-31-5p | 90.11 | 98.89 | 85.00 |
| 1208 | hsa-miR-545-5p, hsa-miR-134-5p, hsa-miR-195-5p, hsa-miR-103a-2-5p, hsa-miR-652-3p, hsa-miR-7-1-3p, hsa-miR-151a-5p, hsa-miR-579-3p, hsa-miR-664a-3p, hsa-miR-501-5p, hsa-miR-144-3p, hsa-miR-27b-3p, hsa-miR-744-5p, hsa-miR-502-3p | 90.00 | 91.43 | 75.00 |
| 1209 | hsa-miR-545-5p, hsa-miR-195-5p, hsa-miR-103a-2-5p, hsa-miR-652-3p, hsa-miR-7-1-3p, hsa-miR-483-5p, hsa-miR-548am-5p, hsa-miR-151a-5p, hsa-miR-579-3p, hsa-miR-144-3p, hsa-miR-502-3p, hsa-miR-21-3p, hsa-miR-31-5p | 90.00 | 95.08 | 80.00 |
| 1210 | hsa-miR-545-5p, hsa-miR-195-5p, hsa-miR-103a-2-5p, hsa-miR-652-3p, hsa-miR-151a-5p, hsa-miR-126-5p, hsa-miR-579-3p, hsa-miR-664a-3p, hsa-miR-501-5p, hsa-miR-144-3p, hsa-miR-27b-3p, hsa-miR-744-5p, hsa-miR-125b-5p, hsa-miR-502-3p, hsa-miR-31-5p | 90.00 | 96.51 | 75.00 |
| 1211 | hsa-miR-545-5p, hsa-miR-134-5p, hsa-miR-195-5p, hsa-miR-103a-2-5p, hsa-miR-652-3p, hsa-miR-339-3p, hsa-miR-369-3p, hsa-miR-7-1-3p, hsa-miR-548am-5p, hsa-miR-151a-5p, hsa-miR-126-5p, hsa-miR-579-3p, hsa-miR-664a-3p, hsa-miR-501-5p, hsa-miR-424-5p, hsa-miR-144-3p, hsa-miR-27b-3p, hsa-miR-744-5p, hsa-miR-502-3p, hsa-miR-629-5p | 90.00 | 92.7 | 75.00 |
| 1212 | hsa-miR-545-5p, hsa-miR-195-5p, hsa-miR-652-3p, hsa-miR-339-3p, hsa-miR-369-3p, hsa-miR-7-1-3p, hsa-miR-483-5p, hsa-miR-548am-5p, hsa-miR-151a-5p, hsa-miR-126-5p, hsa-miR-579-3p, hsa-miR-664a-3p, hsa-miR-501-5p, hsa-miR-424-5p, hsa-miR-144-3p, hsa-miR-27b-3p, hsa-miR-744-5p, hsa-miR-502-3p, hsa-miR-629-5p, hsa-miR-31-5p | 90.00 | 96.51 | 80.00 |
| 1213 | hsa-miR-545-5p, hsa-miR-134-5p, hsa-miR-339-3p, hsa-miR-483-5p, hsa-miR-222-3p, hsa-miR-548am-5p, hsa-miR-664a-3p, hsa-miR-424-5p, hsa-let-7d-3p, hsa-miR-27b-3p, hsa-miR-744-5p, hsa-miR-125b-5p, hsa-miR-31-5p | 89.22 | 95.00 | 86.67 |
| 1214 | hsa-miR-545-5p, hsa-miR-483-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 89.22 | 96.67 | 81.67 |
| 1215 | hsa-miR-545-5p, hsa-miR-483-5p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 89.11 | 96.51 | 86.67 |
| 1216 | hsa-miR-545-5p, hsa-miR-195-5p, hsa-miR-339-3p, hsa-miR-483-5p, hsa-miR-548am-5p, hsa-miR-126-5p, hsa-miR-664a-3p, hsa-miR-501-5p, hsa-miR-424-5p, hsa-let-7d-3p, hsa-miR-27b-3p, hsa-miR-744-5p, hsa-miR-125b-5p, hsa-miR-502-3p | 89.11 | 96.11 | 86.67 |
| 1217 | hsa-miR-545-5p, hsa-miR-134-5p, hsa-miR-483-5p, hsa-miR-222-3p, hsa-miR-548am-5p, hsa-miR-151a-5p, hsa-miR-664a-3p, hsa-miR-144-3p, hsa-let-7d-3p, hsa-miR-27b-3p, hsa-miR-744-5p, hsa-miR-125b-5p | 89.11 | 92.7 | 75.00 |
| 1218 | hsa-miR-545-5p, hsa-miR-652-3p, hsa-miR-483-5p, hsa-miR-548am-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 89.11 | 97.22 | 80.00 |
| 1219 | hsa-miR-545-5p, hsa-miR-134-5p, hsa-miR-195-5p, hsa-miR-103a-2-5p, hsa-miR-483-5p, hsa-miR-222-3p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-21-3p, hsa-miR-31-5p | 89.11 | 98.17 | 81.67 |
| 1220 | hsa-miR-545-5p, hsa-miR-134-5p, hsa-miR-195-5p, hsa-miR-103a-2-5p, hsa-miR-652-3p, hsa-miR-339-3p, hsa-miR-7-1-3p, hsa-miR-483-5p, hsa-miR-548am-5p, hsa-miR-151a-5p, hsa-miR-126-5p, hsa-miR-579-3p, hsa-miR-501-5p, hsa-let-7d-3p, hsa-miR-27b-3p, hsa-miR-125b-5p, hsa-miR-502-3p, hsa-miR-128-3p | 89.11 | 96.19 | 81.67 |
| 1221 | hsa-miR-545-5p, hsa-miR-103a-2-5p, hsa-miR-652-3p, hsa-miR-339-3p, hsa-miR-483-5p, hsa-miR-579-3p, hsa-miR-424-5p, hsa-miR-144-3p, hsa-let-7d-3p, hsa-miR-744-5p, hsa-miR-125b-5p, hsa-miR-502-3p, hsa-miR-21-3p | 89.11 | 97.22 | 80.00 |
| 1222 | hsa-miR-545-5p, hsa-miR-134-5p, hsa-miR-195-5p, hsa-miR-339-3p, hsa-miR-483-5p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 89.11 | 95.95 | 80.00 |
| 1223 | hsa-miR-545-5p, hsa-miR-134-5p, hsa-miR-195-5p, hsa-miR-339-3p, hsa-miR-483-5p, hsa-miR-222-3p, hsa-miR-126-5p, hsa-miR-664a-3p, hsa-miR-424-5p, hsa-let-7d-3p, hsa-miR-27b-3p, hsa-miR-744-5p, hsa-miR-125b-5p, hsa-miR-31-5p | 89.11 | 95.79 | 81.67 |
| 1224 | hsa-miR-545-5p, hsa-miR-134-5p, hsa-miR-483-5p, hsa-miR-222-3p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p | 89.11 | 93.25 | 83.33 |
| 1225 | hsa-miR-134-5p, hsa-miR-195-5p, hsa-miR-103a-2-5p, hsa-miR-652-3p, hsa-miR-339-3p, hsa-miR-483-5p, hsa-miR-222-3p, hsa-miR-126-5p, hsa-miR-579-3p, hsa-miR-424-5p, hsa-miR-144-3p, hsa-let-7d-3p, hsa-miR-744-5p, hsa-miR-125b-5p, hsa-miR-21-3p, hsa-miR-31-5p | 89.11 | 96.75 | 86.67 |
| 1226 | hsa-miR-545-5p, hsa-miR-134-5p, hsa-miR-103a-2-5p, hsa-miR-652-3p, hsa-miR-369-3p, hsa-miR-483-5p, hsa-miR-579-3p, hsa-miR-664a-3p, hsa-miR-144-3p, hsa-miR-27b-3p, hsa-miR-744-5p, hsa-miR-502-3p, hsa-miR-629-5p, hsa-miR-128-3p, hsa-miR-31-5p | 89.00 | 97.62 | 80.00 |
| 1227 | hsa-miR-545-5p, hsa-miR-134-5p, hsa-miR-195-5p, hsa-miR-103a-2-5p, hsa-miR-652-3p, hsa-miR-369-3p, hsa-miR-7-1-3p, hsa-miR-483-5p, hsa-miR-151a-5p, hsa-miR-126-5p, hsa-miR-579-3p, hsa-miR-664a-3p, hsa-miR-144-3p, hsa-miR-27b-3p, hsa-miR-744-5p, hsa-miR-502-3p, hsa-miR-629-5p, hsa-miR-128-3p, hsa-miR-31-5p | 89.00 | 98.17 | 85.00 |
| 1228 | hsa-miR-545-5p, hsa-miR-195-5p, hsa-miR-103a-2-5p, hsa-miR-652-3p, hsa-miR-339-3p, hsa-miR-151a-5p, hsa-miR-579-3p, hsa-miR-664a-3p, hsa-miR-144-3p, hsa-let-7d-3p, hsa-miR-27b-3p, hsa-miR-744-5p, hsa-miR-125b-5p, hsa-miR-502-3p, hsa-miR-31-5p | 89.00 | 96.67 | 70.00 |
| 1229 | hsa-miR-545-5p, hsa-miR-134-5p, hsa-miR-195-5p, hsa-miR-103a-2-5p, hsa-miR-339-3p, hsa-miR-7-1-3p, hsa-miR-424-5p, hsa-miR-144-3p, hsa-miR-744-5p, hsa-miR-502-3p, hsa-miR-31-5p | 89.00 | 95.4 | 76.67 |
| 1230 | hsa-miR-134-5p, hsa-miR-195-5p, hsa-miR-103a-2-5p, hsa-miR-652-3p, hsa-miR-339-3p, hsa-miR-222-3p, hsa-miR-126-5p, hsa-miR-579-3p, hsa-miR-424-5p, hsa-miR-144-3p, hsa-let-7d-3p, hsa-miR-125b-5p, hsa-miR-21-3p, hsa-miR-31-5p | 89.00 | 97.22 | 90.00 |
| 1231 | hsa-miR-545-5p, hsa-miR-195-5p, hsa-miR-652-3p, hsa-miR-369-3p, hsa-miR-483-5p, hsa-miR-222-3p, hsa-miR-126-5p, hsa-miR-579-3p, hsa-miR-664a-3p, hsa-miR-144-3p, hsa-miR-502-3p, hsa-miR-629-5p, hsa-miR-21-3p | 89.00 | 88.17 | 78.33 |
| 1232 | hsa-miR-134-5p, hsa-miR-103a-2-5p, hsa-miR-652-3p, hsa-miR-339-3p, hsa-miR-483-5p, hsa-miR-579-3p, hsa-miR-424-5p, hsa-let-7d-3p, hsa-miR-125b-5p, hsa-miR-502-3p, hsa-miR-21-3p, hsa-miR-31-5p | 89.00 | 95.63 | 83.33 |
| 1233 | hsa-miR-545-5p, hsa-miR-103a-2-5p, hsa-miR-652-3p, hsa-miR-339-3p, hsa-miR-483-5p, hsa-miR-579-3p, hsa-miR-424-5p, hsa-let-7d-3p, hsa-miR-744-5p, hsa-miR-125b-5p, hsa-miR-502-3p, hsa-miR-31-5p | 89.00 | 97.22 | 78.33 |
| 1234 | hsa-miR-545-5p, hsa-miR-195-5p, hsa-miR-103a-2-5p, hsa-miR-652-3p, hsa-miR-222-3p, hsa-miR-579-3p, hsa-miR-664a-3p, hsa-miR-501-5p, hsa-miR-144-3p, hsa-let-7d-3p, hsa-miR-27b-3p, hsa-miR-31-5p | 88.89 | 97.22 | 80.00 |
| 1235 | hsa-miR-545-5p, hsa-miR-134-5p, hsa-miR-339-3p, hsa-miR-483-5p, hsa-miR-548am-5p, hsa-miR-664a-3p, hsa-miR-424-5p, hsa-let-7d-3p, hsa-miR-27b-3p, hsa-miR-744-5p, hsa-miR-125b-5p, hsa-miR-502-3p | 88.11 | 94.44 | 86.67 |
| 1236 | hsa-miR-195-5p, hsa-miR-103a-2-5p, hsa-miR-652-3p, hsa-miR-339-3p, hsa-miR-483-5p, hsa-miR-151a-5p, hsa-miR-126-5p, hsa-miR-579-3p, hsa-miR-144-3p, hsa-let-7d-3p, hsa-miR-125b-5p, hsa-miR-502-3p, hsa-miR-21-3p, hsa-miR-31-5p | 88.11 | 94.6 | 86.67 |
| 1237 | hsa-miR-545-5p, hsa-miR-134-5p, hsa-miR-103a-2-5p, hsa-miR-652-3p, hsa-miR-483-5p, hsa-miR-151a-5p, hsa-miR-579-3p, hsa-miR-664a-3p, hsa-miR-27b-3p, hsa-miR-744-5p, hsa-miR-125b-5p, hsa-miR-502-3p, hsa-miR-31-5p | 88.11 | 96.11 | 88.33 |
| 1238 | hsa-miR-545-5p, hsa-miR-483-5p, hsa-miR-548am-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 88.11 | 96.11 | 81.67 |
| 1239 | hsa-miR-545-5p, hsa-miR-195-5p, hsa-miR-339-3p, hsa-miR-483-5p, hsa-miR-222-3p, hsa-miR-548am-5p, hsa-miR-126-5p, hsa-miR-664a-3p, hsa-miR-501-5p, hsa-miR-424-5p, hsa-let-7d-3p, hsa-miR-27b-3p, hsa-miR-744-5p, hsa-miR-125b-5p | 88.00 | 96.19 | 75.00 |
| 1240 | hsa-miR-545-5p, hsa-miR-134-5p, hsa-miR-195-5p, hsa-miR-339-3p, hsa-miR-483-5p, hsa-miR-222-3p, hsa-miR-548am-5p, hsa-miR-126-5p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p | 88.00 | 93.41 | 81.67 |
| 1241 | hsa-miR-134-5p, hsa-miR-103a-2-5p, hsa-miR-7-1-3p, hsa-miR-151a-5p, hsa-miR-126-5p, hsa-miR-744-5p, hsa-miR-125b-5p, hsa-miR-502-3p, hsa-miR-128-3p, hsa-miR-21-3p, hsa-miR-31-5p | 88.00 | 93.97 | 83.33 |
| 1242 | hsa-miR-545-5p, hsa-miR-134-5p, hsa-miR-339-3p, hsa-miR-222-3p, hsa-miR-548am-5p, hsa-miR-151a-5p, hsa-miR-126-5p, hsa-miR-579-3p, hsa-miR-664a-3p, hsa-miR-144-3p, hsa-let-7d-3p, hsa-miR-27b-3p, hsa-miR-744-5p, hsa-miR-125b-5p | 88.00 | 92.14 | 80.00 |
| 1243 | hsa-miR-545-5p, hsa-miR-652-3p, hsa-miR-483-5p, hsa-miR-548am-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 88.00 | 97.22 | 80.00 |
| 1244 | hsa-miR-545-5p, hsa-miR-134-5p, hsa-miR-483-5p, hsa-miR-222-3p, hsa-miR-548am-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-31-5p | 88.00 | 95.4 | 71.67 |
| 1245 | hsa-miR-195-5p, hsa-miR-103a-2-5p, hsa-miR-652-3p, hsa-miR-339-3p, hsa-miR-126-5p, hsa-miR-579-3p, hsa-miR-501-5p, hsa-miR-424-5p, hsa-miR-144-3p, hsa-let-7d-3p, hsa-miR-125b-5p, hsa-miR-502-3p, hsa-miR-21-3p | 88.00 | 95.56 | 80.00 |
| 1246 | hsa-miR-545-5p, hsa-miR-134-5p, hsa-miR-195-5p, hsa-miR-339-3p, hsa-miR-483-5p, hsa-miR-222-3p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 88.00 | 93.1 | 81.67 |
| 1247 | hsa-miR-545-5p, hsa-miR-134-5p, hsa-miR-195-5p, hsa-miR-339-3p, hsa-miR-483-5p, hsa-miR-548am-5p, hsa-miR-126-5p, hsa-miR-579-3p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 88.00 | 95.4 | 80.00 |
| 1248 | hsa-miR-545-5p, hsa-miR-195-5p, hsa-miR-339-3p, hsa-miR-483-5p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-502-3p | 88.00 | 95.95 | 83.33 |
| 1249 | hsa-miR-545-5p, hsa-miR-134-5p, hsa-miR-103a-2-5p, hsa-miR-652-3p, hsa-miR-339-3p, hsa-miR-369-3p, hsa-miR-7-1-3p, hsa-miR-483-5p, hsa-miR-579-3p, hsa-miR-664a-3p, hsa-miR-424-5p, hsa-let-7d-3p, hsa-miR-27b-3p, hsa-miR-744-5p, hsa-miR-502-3p, hsa-miR-629-5p, hsa-miR-128-3p, hsa-miR-31-5p | 87.89 | 97.78 | 83.33 |
| 1250 | hsa-miR-545-5p, hsa-miR-195-5p, hsa-miR-652-3p, hsa-miR-339-3p, hsa-miR-7-1-3p, hsa-miR-483-5p, hsa-miR-151a-5p, hsa-miR-579-3p, hsa-miR-664a-3p, hsa-miR-501-5p, hsa-miR-424-5p, hsa-miR-144-3p, hsa-miR-27b-3p, hsa-miR-744-5p, hsa-miR-31-5p | 87.89 | 93.02 | 76.67 |
| 1251 | hsa-miR-134-5p, hsa-miR-195-5p, hsa-miR-103a-2-5p, hsa-miR-652-3p, hsa-miR-7-1-3p, hsa-miR-548am-5p, hsa-miR-151a-5p, hsa-miR-126-5p, hsa-miR-664a-3p, hsa-miR-144-3p, hsa-miR-27b-3p, hsa-miR-744-5p, hsa-miR-502-3p, hsa-miR-31-5p | 87.89 | 95.79 | 85.00 |
| 1252 | hsa-miR-545-5p, hsa-miR-103a-2-5p, hsa-miR-652-3p, hsa-miR-483-5p, hsa-miR-579-3p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p | 87.89 | 97.22 | 73.33 |
| 1253 | hsa-miR-545-5p, hsa-miR-195-5p, hsa-miR-103a-2-5p, hsa-miR-652-3p, hsa-miR-222-3p, hsa-miR-151a-5p, hsa-miR-126-5p, hsa-miR-579-3p, hsa-miR-664a-3p, hsa-miR-27b-3p, hsa-miR-125b-5p, hsa-miR-502-3p, hsa-miR-128-3p, hsa-miR-21-3p, hsa-miR-31-5p | 87.89 | 95.24 | 70.00 |
| 1254 | hsa-miR-545-5p, hsa-miR-103a-2-5p, hsa-miR-652-3p, hsa-miR-339-3p, hsa-miR-7-1-3p, hsa-miR-151a-5p, hsa-miR-579-3p, hsa-miR-501-5p, hsa-miR-424-5p, hsa-miR-144-3p, hsa-miR-744-5p, hsa-miR-502-3p | 87.78 | 92.3 | 76.67 |
| 1255 | hsa-miR-545-5p, hsa-miR-195-5p, hsa-miR-652-3p, hsa-miR-369-3p, hsa-miR-7-1-3p, hsa-miR-483-5p, hsa-miR-222-3p, hsa-miR-151a-5p, hsa-miR-126-5p, hsa-miR-579-3p, hsa-miR-664a-3p, hsa-miR-501-5p, hsa-miR-27b-3p, hsa-miR-744-5p, hsa-miR-629-5p, hsa-miR-128-3p, hsa-miR-31-5p | 87.00 | 98.17 | 83.33 |
| 1256 | hsa-miR-545-5p, hsa-miR-134-5p, hsa-miR-195-5p, hsa-miR-103a-2-5p, hsa-miR-369-3p, hsa-miR-126-5p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-502-3p, hsa-miR-629-5p, hsa-miR-128-3p, hsa-miR-21-3p | 86.89 | 94.84 | 80.00 |
| 1257 | hsa-miR-545-5p, hsa-miR-195-5p, hsa-miR-339-3p, hsa-miR-222-3p, hsa-miR-548am-5p, hsa-miR-126-5p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-31-5p | 86.89 | 95.00 | 71.67 |
| 1258 | hsa-miR-545-5p, hsa-miR-195-5p, hsa-miR-339-3p, hsa-miR-483-5p, hsa-miR-222-3p, hsa-miR-548am-5p, hsa-miR-126-5p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-31-5p | 86.89 | 94.13 | 76.67 |
| 1259 | hsa-miR-545-5p, hsa-miR-134-5p, hsa-miR-195-5p, hsa-miR-339-3p, hsa-miR-483-5p, hsa-miR-222-3p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-31-5p | 86.89 | 94.68 | 88.33 |
| 1260 | hsa-miR-103a-2-5p, hsa-miR-652-3p, hsa-miR-369-3p, hsa-miR-7-1-3p, hsa-miR-151a-5p, hsa-miR-126-5p, hsa-miR-579-3p, hsa-miR-664a-3p, hsa-miR-501-5p, hsa-miR-27b-3p, hsa-miR-744-5p, hsa-miR-502-3p, hsa-miR-629-5p, hsa-miR-128-3p, hsa-miR-31-5p | 86.89 | 94.21 | 78.33 |
| 1261 | hsa-miR-134-5p, hsa-miR-195-5p, hsa-miR-103a-2-5p, hsa-miR-652-3p, hsa-miR-339-3p, hsa-miR-222-3p, hsa-miR-548am-5p, hsa-miR-126-5p, hsa-miR-579-3p, hsa-miR-424-5p, hsa-miR-144-3p, hsa-let-7d-3p, hsa-miR-125b-5p, hsa-miR-21-3p, hsa-miR-31-5p | 86.89 | 96.75 | 78.33 |
| 1262 | hsa-miR-545-5p, hsa-miR-134-5p, hsa-miR-195-5p, hsa-miR-339-3p, hsa-miR-483-5p, hsa-miR-222-3p, hsa-miR-548am-5p, hsa-miR-126-5p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p | 86.89 | 93.57 | 76.67 |
| 1263 | hsa-miR-545-5p, hsa-miR-134-5p, hsa-miR-195-5p, hsa-miR-339-3p, hsa-miR-222-3p, hsa-miR-548am-5p, hsa-miR-151a-5p, hsa-miR-126-5p, hsa-miR-664a-3p, hsa-miR-144-3p, hsa-let-7d-3p, hsa-miR-27b-3p, hsa-miR-744-5p, hsa-miR-125b-5p, hsa-miR-31-5p | 86.89 | 96.51 | 71.67 |
| 1264 | hsa-miR-134-5p, hsa-miR-195-5p, hsa-miR-103a-2-5p, hsa-miR-652-3p, hsa-miR-339-3p, hsa-miR-483-5p, hsa-miR-222-3p, hsa-miR-126-5p, hsa-miR-424-5p, hsa-miR-144-3p, hsa-let-7d-3p, hsa-miR-125b-5p, hsa-miR-21-3p, hsa-miR-31-5p | 86.89 | 95.56 | 80.00 |
| 1265 | hsa-miR-545-5p, hsa-miR-134-5p, hsa-miR-195-5p, hsa-miR-339-3p, hsa-miR-483-5p, hsa-miR-222-3p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-31-5p | 86.89 | 96.9 | 80.00 |
| 1266 | hsa-miR-195-5p, hsa-miR-339-3p, hsa-miR-483-5p, hsa-miR-222-3p, hsa-miR-126-5p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-31-5p | 86.89 | 94.13 | 80.00 |
| 1267 | hsa-miR-545-5p, hsa-miR-195-5p, hsa-miR-339-3p, hsa-miR-483-5p, hsa-miR-548am-5p, hsa-miR-126-5p, hsa-miR-664a-3p, hsa-miR-501-5p, hsa-miR-424-5p, hsa-miR-144-3p, hsa-let-7d-3p, hsa-miR-27b-3p, hsa-miR-744-5p, hsa-miR-125b-5p, hsa-miR-502-3p | 86.89 | 95.56 | 75.00 |
| 1268 | hsa-miR-195-5p, hsa-miR-339-3p, hsa-miR-483-5p, hsa-miR-222-3p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-21-3p | 86.89 | 93.65 | 80.00 |
| 1269 | hsa-miR-195-5p, hsa-miR-103a-2-5p, hsa-miR-7-1-3p, hsa-miR-126-5p, hsa-miR-125b-5p, hsa-miR-502-3p, hsa-miR-128-3p, hsa-miR-21-3p, hsa-miR-31-5p | 86.89 | 95.08 | 73.33 |
| 1270 | hsa-miR-545-5p, hsa-miR-134-5p, hsa-miR-195-5p, hsa-miR-483-5p, hsa-miR-222-3p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p | 86.89 | 93.25 | 78.33 |
| 1271 | hsa-miR-545-5p, hsa-miR-103a-2-5p, hsa-miR-652-3p, hsa-miR-7-1-3p, hsa-miR-151a-5p, hsa-miR-126-5p, hsa-miR-579-3p, hsa-miR-664a-3p, hsa-miR-501-5p, hsa-miR-27b-3p, hsa-miR-744-5p, hsa-miR-502-3p, hsa-miR-128-3p | 86.78 | 95.24 | 71.67 |
| 1272 | hsa-miR-545-5p, hsa-miR-195-5p, hsa-miR-103a-2-5p, hsa-miR-7-1-3p, hsa-miR-222-3p, hsa-miR-126-5p, hsa-miR-664a-3p, hsa-miR-501-5p, hsa-miR-144-3p, hsa-miR-27b-3p, hsa-miR-744-5p | 86.67 | 94.13 | 71.67 |
| 1273 | hsa-miR-134-5p, hsa-miR-195-5p, hsa-miR-652-3p, hsa-miR-339-3p, hsa-miR-369-3p, hsa-miR-7-1-3p, hsa-miR-222-3p, hsa-miR-548am-5p, hsa-miR-501-5p, hsa-miR-27b-3p, hsa-miR-744-5p, hsa-miR-125b-5p, hsa-miR-629-5p, hsa-miR-31-5p | 86.67 | 91.75 | 71.67 |
| 1274 | hsa-miR-545-5p, hsa-miR-652-3p, hsa-miR-369-3p, hsa-miR-222-3p, hsa-miR-548am-5p, hsa-miR-151a-5p, hsa-miR-126-5p, hsa-miR-579-3p, hsa-miR-664a-3p, hsa-miR-501-5p, hsa-miR-144-3p, hsa-let-7d-3p, hsa-miR-27b-3p, hsa-miR-744-5p, hsa-miR-629-5p | 85.89 | 96.67 | 76.67 |
| 1275 | hsa-miR-195-5p, hsa-miR-339-3p, hsa-miR-483-5p, hsa-miR-222-3p, hsa-miR-548am-5p, hsa-miR-126-5p, hsa-miR-424-5p, hsa-miR-144-3p, hsa-let-7d-3p, hsa-miR-27b-3p, hsa-miR-125b-5p, hsa-miR-21-3p | 85.78 | 89.44 | 73.33 |
| 1276 | hsa-miR-134-5p, hsa-miR-195-5p, hsa-miR-103a-2-5p, hsa-miR-339-3p, hsa-miR-222-3p, hsa-miR-151a-5p, hsa-miR-126-5p, hsa-miR-579-3p, hsa-miR-424-5p, hsa-miR-144-3p, hsa-let-7d-3p, hsa-miR-125b-5p, hsa-miR-21-3p, hsa-miR-31-5p | 85.78 | 92.22 | 71.67 |
| 1277 | hsa-miR-195-5p, hsa-miR-103a-2-5p, hsa-miR-652-3p, hsa-miR-339-3p, hsa-miR-483-5p, hsa-miR-126-5p, hsa-miR-579-3p, hsa-miR-424-5p, hsa-miR-144-3p, hsa-let-7d-3p, hsa-miR-125b-5p, hsa-miR-21-3p, hsa-miR-31-5p | 85.78 | 94.05 | 75.00 |
| 1278 | hsa-miR-339-3p, hsa-miR-369-3p, hsa-miR-7-1-3p, hsa-miR-548am-5p, hsa-miR-151a-5p, hsa-miR-126-5p, hsa-miR-579-3p, hsa-miR-664a-3p, hsa-miR-125b-5p, hsa-miR-502-3p, hsa-miR-21-3p, hsa-miR-31-5p | 85.78 | 89.68 | 76.67 |
| 1279 | hsa-miR-545-5p, hsa-miR-134-5p, hsa-miR-103a-2-5p, hsa-miR-339-3p, hsa-miR-369-3p, hsa-miR-7-1-3p, hsa-miR-483-5p, hsa-miR-222-3p, hsa-miR-548am-5p, hsa-miR-126-5p, hsa-miR-579-3p, hsa-miR-424-5p, hsa-miR-144-3p, hsa-let-7d-3p, hsa-miR-744-5p, hsa-miR-21-3p, hsa-miR-31-5p | 85.78 | 96.67 | 80.00 |
| 1280 | hsa-miR-545-5p, hsa-miR-134-5p, hsa-miR-195-5p, hsa-miR-339-3p, hsa-miR-483-5p, hsa-miR-222-3p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-21-3p, hsa-miR-31-5p | 85.78 | 95.79 | 80.00 |
| 1281 | hsa-miR-545-5p, hsa-miR-134-5p, hsa-miR-339-3p, hsa-miR-7-1-3p, hsa-miR-483-5p, hsa-miR-548am-5p, hsa-miR-664a-3p, hsa-miR-424-5p, hsa-let-7d-3p, hsa-miR-27b-3p, hsa-miR-744-5p, hsa-miR-502-3p | 85.78 | 95.00 | 75.00 |
| 1282 | hsa-miR-545-5p, hsa-miR-134-5p, hsa-miR-195-5p, hsa-miR-483-5p, hsa-miR-222-3p, hsa-miR-548am-5p, hsa-miR-151a-5p, hsa-miR-126-5p, hsa-miR-664a-3p, hsa-miR-144-3p, hsa-let-7d-3p, hsa-miR-27b-3p, hsa-miR-744-5p, hsa-miR-125b-5p | 85.78 | 93.1 | 75.00 |
| 1283 | hsa-miR-134-5p, hsa-miR-195-5p, hsa-miR-103a-2-5p, hsa-miR-652-3p, hsa-miR-339-3p, hsa-miR-151a-5p, hsa-miR-126-5p, hsa-miR-144-3p, hsa-let-7d-3p, hsa-miR-125b-5p, hsa-miR-502-3p, hsa-miR-21-3p, hsa-miR-31-5p | 85.78 | 95.63 | 81.67 |
| 1284 | hsa-miR-545-5p, hsa-miR-134-5p, hsa-miR-195-5p, hsa-miR-339-3p, hsa-miR-483-5p, hsa-miR-222-3p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p | 85.78 | 93.25 | 75.00 |
| 1285 | hsa-miR-134-5p, hsa-miR-195-5p, hsa-miR-339-3p, hsa-miR-7-1-3p, hsa-miR-222-3p, hsa-miR-151a-5p, hsa-miR-579-3p, hsa-miR-424-5p, hsa-miR-144-3p, hsa-let-7d-3p, hsa-miR-502-3p, hsa-miR-128-3p | 85.67 | 86.59 | 75.00 |
| 1286 | hsa-miR-134-5p, hsa-miR-195-5p, hsa-miR-103a-2-5p, hsa-miR-652-3p, hsa-miR-339-3p, hsa-miR-369-3p, hsa-miR-483-5p, hsa-miR-222-3p, hsa-miR-144-3p, hsa-miR-629-5p | 85.56 | 91.98 | 68.33 |
| 1287 | hsa-miR-545-5p, hsa-miR-134-5p, hsa-miR-103a-2-5p, hsa-miR-7-1-3p, hsa-miR-151a-5p, hsa-miR-126-5p, hsa-miR-501-5p, hsa-miR-144-3p, hsa-miR-502-3p, hsa-miR-21-3p, hsa-miR-31-5p | 85.56 | 95.63 | 76.67 |
| 1288 | hsa-miR-339-3p, hsa-miR-7-1-3p, hsa-miR-548am-5p, hsa-miR-579-3p, hsa-miR-501-5p, hsa-miR-424-5p, hsa-let-7d-3p, hsa-miR-502-3p, hsa-miR-629-5p, hsa-miR-21-3p, hsa-miR-31-5p | 85.56 | 92.22 | 66.67 |
| 1289 | hsa-miR-545-5p, hsa-miR-134-5p, hsa-miR-103a-2-5p, hsa-miR-369-3p, hsa-miR-664a-3p, hsa-miR-501-5p, hsa-let-7d-3p, hsa-miR-21-3p, hsa-miR-31-5p | 85.56 | 89.6 | 73.33 |
| 1290 | hsa-miR-545-5p, hsa-miR-151a-5p, hsa-miR-126-5p, hsa-miR-501-5p, hsa-miR-424-5p, hsa-let-7d-3p, hsa-miR-744-5p, hsa-miR-125b-5p, hsa-miR-629-5p, hsa-miR-128-3p | 84.78 | 91.51 | 73.33 |
| 1291 | hsa-miR-134-5p, hsa-miR-103a-2-5p, hsa-miR-339-3p, hsa-miR-369-3p, hsa-miR-7-1-3p, hsa-miR-222-3p, hsa-miR-548am-5p, hsa-miR-126-5p, hsa-miR-664a-3p, hsa-miR-144-3p, hsa-let-7d-3p, hsa-miR-744-5p, hsa-miR-125b-5p, hsa-miR-502-3p, hsa-miR-629-5p, hsa-miR-128-3p, hsa-miR-21-3p, hsa-miR-31-5p | 84.78 | 92.7 | 80.00 |
| 1292 | hsa-miR-545-5p, hsa-miR-134-5p, hsa-miR-483-5p, hsa-miR-222-3p, hsa-miR-548am-5p, hsa-miR-151a-5p, hsa-miR-664a-3p, hsa-miR-27b-3p, hsa-miR-744-5p, hsa-miR-125b-5p | 84.67 | 88.41 | 66.67 |
| 1293 | hsa-miR-545-5p, hsa-miR-134-5p, hsa-miR-103a-2-5p, hsa-miR-652-3p, hsa-miR-369-3p, hsa-miR-483-5p, hsa-miR-151a-5p, hsa-miR-579-3p, hsa-miR-664a-3p, hsa-miR-27b-3p, hsa-miR-744-5p, hsa-miR-125b-5p, hsa-miR-502-3p, hsa-miR-629-5p, hsa-miR-128-3p, hsa-miR-31-5p | 84.67 | 95.79 | 71.67 |
| 1294 | hsa-miR-134-5p, hsa-miR-195-5p, hsa-miR-652-3p, hsa-miR-7-1-3p, hsa-miR-483-5p, hsa-miR-222-3p, hsa-miR-579-3p, hsa-miR-664a-3p, hsa-miR-501-5p, hsa-miR-144-3p, hsa-let-7d-3p, hsa-miR-27b-3p, hsa-miR-744-5p, hsa-miR-31-5p | 84.56 | 91.67 | 80.00 |
| 1295 | hsa-miR-545-5p, hsa-miR-134-5p, hsa-miR-195-5p, hsa-miR-103a-2-5p, hsa-miR-652-3p, hsa-miR-339-3p, hsa-miR-7-1-3p, hsa-miR-126-5p, hsa-miR-424-5p, hsa-miR-144-3p, hsa-let-7d-3p, hsa-miR-502-3p, hsa-miR-21-3p, hsa-miR-31-5p | 83.56 | 93.33 | 76.67 |
| 1296 | hsa-miR-134-5p, hsa-miR-195-5p, hsa-miR-339-3p, hsa-miR-483-5p, hsa-miR-222-3p, hsa-miR-548am-5p, hsa-miR-126-5p, hsa-miR-664a-3p, hsa-miR-424-5p, hsa-miR-144-3p, hsa-let-7d-3p, hsa-miR-27b-3p, hsa-miR-744-5p, hsa-miR-125b-5p, hsa-miR-21-3p, hsa-miR-31-5p | 83.56 | 90.32 | 68.33 |
| 1297 | hsa-miR-545-5p, hsa-miR-134-5p, hsa-miR-195-5p, hsa-miR-652-3p, hsa-miR-339-3p, hsa-miR-369-3p, hsa-miR-7-1-3p, hsa-miR-483-5p, hsa-miR-151a-5p, hsa-miR-579-3p, hsa-miR-664a-3p, hsa-miR-424-5p, hsa-miR-128-3p, hsa-miR-31-5p | 83.44 | 90.63 | 81.67 |
| 1298 | hsa-miR-134-5p, hsa-miR-195-5p, hsa-miR-652-3p, hsa-miR-369-3p, hsa-miR-7-1-3p, hsa-miR-126-5p, hsa-miR-144-3p, hsa-let-7d-3p, hsa-miR-27b-3p, hsa-miR-125b-5p, hsa-miR-629-5p, hsa-miR-128-3p, hsa-miR-21-3p, hsa-miR-31-5p | 83.44 | 83.65 | 78.33 |
| 1299 | hsa-miR-134-5p, hsa-miR-195-5p, hsa-miR-339-3p, hsa-miR-483-5p, hsa-miR-222-3p, hsa-miR-126-5p, hsa-miR-424-5p, hsa-miR-144-3p, hsa-let-7d-3p, hsa-miR-125b-5p, hsa-miR-502-3p, hsa-miR-21-3p | 82.44 | 87.78 | 66.67 |
| 1300 | hsa-miR-545-5p, hsa-miR-134-5p, hsa-miR-195-5p, hsa-miR-339-3p, hsa-miR-369-3p, hsa-miR-7-1-3p, hsa-miR-222-3p, hsa-miR-501-5p, hsa-miR-144-3p, hsa-let-7d-3p, hsa-miR-27b-3p, hsa-miR-21-3p, hsa-miR-31-5p | 82.33 | 88.1 | 61.67 |
| 1301 | hsa-miR-545-5p, hsa-miR-369-3p, hsa-miR-483-5p, hsa-miR-222-3p, hsa-miR-548am-5p, hsa-miR-126-5p, hsa-miR-501-5p, hsa-miR-424-5p, hsa-miR-144-3p, hsa-miR-27b-3p, hsa-miR-629-5p, hsa-miR-31-5p | 82.33 | 91.35 | 75.00 |
| 1302 | combination 1031 + rs11647727 | 74.68 | 59.34 | 75.65 |
| 1303 | combination 1034 + rs11862974 | 82.46 | 90.46 | 74.46 |
| 1304 | combination 1035 + rs4297685 | 81.35 | 88.02 | 85.85 |
| 1305 | combination 10346 + rs4297685 | 82.46 | 88.88 | 87.82 |
| 1306 | combination 1037 + rs4297685 | 85.73 | 91.06 | 89.18 |
| 1307 | combination 1038 + rs4297685 | 85.73 | 90.75 | 89.06 |
| 1308 | combination 1039 + rs11862974 | 85.79 | 92.77 | 92.51 |
| 1309 | combination 1040 + rs76563024 | 86.78 | 92.5 | 91.06 |
| 1310 | combination 1041 + rs76563024 | 87.95 | 95.69 | 93.0, |
| 1311 | combination 1044 + rs76563024 | 89.01 | 95.67 | 92.08 |
| 1312 | combination 1045 + rs76563024 | 90.12 | 95.62 | 98.24 |
| 1313 | combination 1046 + rs76563024 | 89.01 | 95.34 | 99.69 |
| 1314 | combination 1047 + rs4297685 | 91.17 | 97.12 | 99.38 |
| 1315 | combination 1049 + rs76563024 | 89.01 | 96.7 | 99.08 |
| 1316 | combination 1050 + rs76563024 | 87.89 | 96.08 | 98.8 |
| 1317 | combination 1051 + rs7204342 | 88.89 | 96 | 96.65 |
| 1318 | combination 1052 + rs11647727 | 91.11 | 96.31 | 95.23 |
| 1319 | combination 1054 + rs12934455 | 89.01 | 95.74 | 95.53 |
| 1320 | combination 1055 + rs9928757 | 89.01 | 95.19 | 95.5 |
| 1321 | combination 1056 + rs76563024 | 90 | 96 | 92.51 |
| 1322 | combination 1057 + rs28544423 | 90 | 95.72 | 91.06 |
| 1323 | combination 1058 + rs34356953 | 90 | 94.8 | 93.0 |
| 1324 | combination 1059 + rs28544423 | 88.95 | 96.05 | 92.08 |
| 1325 | combination 1226 + rs11647727 | 89.00 | 90.32 | 92.51 |
| 1326 | combination 1227 + rs11647727 | 89.00 | 90.63 | 91.06 |
| 1327 | combination 1037 + rs4293393 | 82.46 | 91.67 | 93.0 |
| 1328 | combination 1038 + rs4293393 | 83.51 | 91.64 | 92.08 |
| 1329 | combination 1040 + rs4293393 | 84.68 | 92.26 | 98.24 |
| 1330 | combination 1045 + rs4293393 | 90.18 | 99.08 | 99.69 |
| 1331 | combination 1046 + rs4293393 | 93.45 | 97.69 | 99.38 |
| 1332 | combination 1050 + rs4293393 | 91.23 | 98.18 | 99.08 |
| 1333 | combination 1056 + rs4293393 | 92.28 | 97.34 | 95.23 |

List of the best 29 mRNAs selected by the algorithm sorted by importance for clinical metastasis: hsa-miR-3613-3p, hsa-miR-548l, hsa-miR-548k, hsa-miR-377-3p, hsa-miR-153-3p, hsa-miR-590-3p, hsa-miR-582-5p, hsa-miR-129, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, hsa-miR-382-5p, hsa-miR-342-3p, hsa-miR-532-5p, hsa-miR-197-3p, hsa-miR-126-3p, hsa-miR-150-5p, hsa-let-7i-3p, hsa-miR-222-3p. The inventors combined these miRNAs, together or not with age and UMOD SNP variant, creating unique combinations leading to a further significant increase in sensitivity, specificity, accuracy and predictive power for clinical metastasis (Table 19).

**Table 19. Combinations that allow higher sensitivity, specificity, accuracy and predictive power for clinical metastasis.**

| N | Combinations | Accuracy | ROC AUC | Recall |
|---|---|---|---|---|
| 1334 | hsa-miR-3613-3p | 92.34 | 58.01 | 70.0 |
| 1335 | hsa-miR-3613-3p, hsa-miR-548l | 92.34 | 73.27 | 70.0 |
| 1336 | hsa-miR-3613-3p, hsa-miR-548l, hsa-miR-548k | 92.34 | 82.65 | 80.0 |
| 1337 | hsa-miR-3613-3p, hsa-miR-548l, hsa-miR-548k, hsa-miR-377-3p | 92.46 | 94.67 | 80.0 |
| 1338 | hsa-miR-3613-3p, hsa-miR-548l, hsa-miR-548k, hsa-miR-377-3p, hsa-miR-153-3p | 93.51 | 92.9 | 70.0 |
| 1339 | hsa-miR-3613-3p, hsa-miR-548l, hsa-miR-548k, hsa-miR-377-3p, hsa-miR-153-3p, hsa-miR-590-3p | 95.56 | 97.02 | 70.0 |
| 1340 | hsa-miR-3613-3p, hsa-miR-548l, hsa-miR-548k, hsa-miR-377-3p, hsa-miR-153-3p, hsa-miR-590-3p, hsa-miR-582-5p | 95.67 | 98.2 | 80.0 |
| 1341 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290 | 94.5 | 97.61 | 80.0 |
| 1342 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p | 95.61 | 96.47 | 80.0 |
| 1343 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p | 96.67 | 96.47 | 80.0 |
| 1344 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484 | 96.67 | 96.47 | 100.0 |
| 1345 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p | 95.61 | 97.65 | 100.0 |
| 1346 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p | 97.84 | 96.47 | 100.0 |
| 1347 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p | 98.89 | 97.65 | 80.0 |
| 1348 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p | 100.00 | 100.00 | 100.00 |
| 1349 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p | 100.00 | 100.00 | 100.00 |
| 1350 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p | 100.00 | 100.00 | 100.00 |
| 1351 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p | 100.00 | 100.00 | 100.00 |
| 1352 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p | 98.89 | 100.00 | 100.00 |
| 1353 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p | 97.78 | 99.38 | 80.0 |
| 1354 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p | 97.78 | 99.38 | 80.0 |
| 1355 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, age | 97.78 | 99.38 | 80.0 |
| 1356 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, age, hsa-miR-382-5p | 96.67 | 100.00 | 100.00 |
| 1357 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, age, hsa-miR-382-5p, hsa-miR-342-3p | 96.67 | 98.75 | 70.0 |
| 1358 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, age, hsa-miR-382-5p, hsa-miR-342-3p, hsa-miR-532-5p | 96.67 | 97.57 | 70.0 |
| 1359 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, age, hsa-miR-382-5p, hsa-miR-342-3p, hsa-miR-532-5p, hsa-miR-197-3p | 95.56 | 95.22 | 70.0 |
| 1360 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, age, hsa-miR-382-5p, hsa-miR-342-3p, hsa-miR-532-5p, hsa-miR-197-3p, hsa-miR-126-3p | 95.56 | 97.57 | 70.0 |
| 1361 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, age, hsa-miR-382-5p, hsa-miR-342-3p, hsa-miR-532-5p, hsa-miR-197-3p, hsa-miR-126-3p, hsa-miR-150-5p | 96.67 | 96.4 | 70.0 |
| 1362 | hsa-miR-3613-3p, hsa-miR-548l, hsa-miR-548k, hsa-miR-377-3p, hsa-miR-153-3p, hsa-miR-590-3p, hsa-miR-582-5p, hsa-miR-1290, hsa-miR-410-3p, hsa-let-7g-3p, hsa-miR-484, hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, age, hsa-miR-382-5p, hsa-miR-342-3p, hsa-miR-532-5p, hsa-miR-197-3p, hsa-miR-126-3p, hsa-miR-150-5p, hsa-let-7i-3p | 96.67 | 98.75 | 70.0 |
| 1363 | hsa-miR-484, hsa-miR-376c-3p, hsa-miR-545-5p, hsa-miR-590-5p, hsa-miR-7-1-3p, hsa-miR-598-3p, hsa-miR-671-5p, hsa-miR-342-3p, hsa-miR-197-3p, hsa-miR-150-5p, hsa-let-7i-3p, hsa-miR-377-3p, hsa-miR-590-3p | 100.00 | 100.00 | 100.00 |
| 1364 | hsa-miR-484, hsa-miR-590-5p, hsa-let-7d-3p, hsa-miR-7-1-3p, hsa-miR-598-3p, hsa-miR-671-5p, hsa-miR-126-3p, hsa-miR-150-5p, hsa-let-7i-3p, hsa-miR-377-3p, hsa-miR-590-3p | 100.00 | 100.00 | 100.00 |
| 1365 | hsa-miR-484, hsa-miR-376c-3p, hsa-miR-545-5p, hsa-miR-6803-3p, hsa-miR-590-5p, hsa-miR-598-3p, hsa-miR-671-5p, age, hsa-miR-197-3p, hsa-miR-150-5p, hsa-let-7i-3p, hsa-miR-377-3p, hsa-miR-153-3p, hsa-miR-590-3p, hsa-miR-1290 | 100.00 | 100.00 | 100.00 |
| 1366 | hsa-miR-376c-3p, hsa-miR-545-5p, hsa-miR-6803-3p, hsa-miR-455-3p, age, hsa-miR-342-3p, hsa-miR-197-3p, hsa-miR-222-3p, hsa-miR-377-3p, hsa-miR-153-3p, hsa-miR-590-3p, hsa-miR-582-5p, hsa-miR-1290 | 100.00 | 100.00 | 100.00 |
| 1367 | hsa-miR-376c-3p, hsa-miR-545-5p, hsa-miR-6803-3p, hsa-miR-590-5p, hsa-miR-455-3p, hsa-miR-671-5p, hsa-miR-382-5p, hsa-miR-532-5p, hsa-miR-197-3p, hsa-miR-150-5p, hsa-miR-222-3p, hsa-miR-377-3p, hsa-miR-153-3p, hsa-miR-590-3p, hsa-miR-582-5p, hsa-miR-1290 | 100.00 | 100.00 | 100.00 |
| 1368 | hsa-miR-376c-3p, hsa-miR-545-5p, hsa-miR-6803-3p, hsa-miR-590-5p, hsa-miR-455-3p, hsa-miR-671-5p, hsa-miR-382-5p, hsa-miR-532-5p, hsa-miR-197-3p, hsa-miR-150-5p, hsa-let-7i-3p, hsa-miR-377-3p, hsa-miR-153-3p, hsa-miR-590-3p, hsa-miR-582-5p, hsa-miR-1290 | 100.00 | 100.00 | 100.00 |
| 1369 | hsa-miR-484, hsa-miR-376c-3p, hsa-miR-545-5p, hsa-miR-590-5p, hsa-miR-7-1-3p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-590-3p, hsa-miR-410-3p | 100.00 | 100.00 | 100.00 |
| 1370 | hsa-miR-484, hsa-miR-376c-3p, hsa-miR-545-5p, hsa-miR-6803-3p, hsa-miR-590-5p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-153-3p, hsa-miR-590-3p, hsa-miR-1290 | 100.00 | 100.00 | 100.00 |
| 1371 | hsa-miR-484, hsa-miR-590-5p, hsa-miR-7-1-3p, hsa-miR-598-3p, hsa-miR-671-5p, age, hsa-miR-197-3p, hsa-miR-126-3p, hsa-miR-150-5p, hsa-let-7i-3p, hsa-miR-377-3p, hsa-miR-590-3p | 100.00 | 100.00 | 100.00 |
| 1372 | hsa-miR-376c-3p, hsa-miR-545-5p, hsa-miR-6803-3p, hsa-miR-455-3p, hsa-miR-671-5p, hsa-miR-382-5p, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290, hsa-let-7g-3p | 100.00 | 100.00 | 100.00 |
| 1373 | hsa-miR-376c-3p, hsa-miR-545-5p, hsa-miR-6803-3p, hsa-miR-455-3p, hsa-miR-671-5p, hsa-miR-382-5p, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290 | 100.00 | 100.00 | 100.00 |
| 1374 | hsa-miR-484, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-377-3p, hsa-miR-410-3p | 100.00 | 100.00 | 100.00 |
| 1375 | hsa-miR-376c-3p, hsa-miR-545-5p, hsa-miR-6803-3p, hsa-miR-455-3p, hsa-miR-382-5p, hsa-miR-342-3p, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290 | 100.00 | 100.00 | 100.00 |
| 1376 | hsa-miR-484, hsa-miR-376c-3p, hsa-miR-6803-3p, hsa-miR-590-5p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-153-3p, hsa-miR-590-3p, hsa-miR-1290, hsa-miR-410-3p | 100.00 | 100.00 | 100.00 |
| 1377 | hsa-miR-484, hsa-miR-545-5p, hsa-miR-590-5p, hsa-let-7d-3p, hsa-miR-7-1-3p, hsa-miR-598-3p, hsa-miR-671-5p, hsa-miR-342-3p, hsa-miR-126-3p, hsa-miR-150-5p, hsa-let-7i-3p, hsa-miR-377-3p, hsa-miR-590-3p, hsa-miR-410-3p | 100.00 | 100.00 | 100.00 |
| 1378 | hsa-miR-484, hsa-miR-376c-3p, hsa-miR-545-5p, hsa-miR-590-5p, hsa-miR-7-1-3p, hsa-miR-598-3p, hsa-miR-671-5p, age, hsa-miR-342-3p, hsa-miR-197-3p, hsa-miR-126-3p, hsa-miR-150-5p, hsa-let-7i-3p, hsa-miR-377-3p, hsa-miR-590-3p | 100.00 | 100.00 | 100.00 |
| 1379 | hsa-miR-376c-3p, hsa-miR-6803-3p, hsa-miR-455-3p, age, hsa-miR-382-5p, hsa-miR-342-3p, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290 | 100.00 | 100.00 | 100.00 |
| 1380 | hsa-miR-376c-3p, hsa-miR-545-5p, hsa-miR-6803-3p, hsa-miR-455-3p, age, hsa-miR-382-5p, hsa-miR-342-3p, hsa-miR-197-3p, hsa-miR-222-3p, hsa-miR-377-3p, hsa-miR-153-3p, hsa-miR-590-3p, hsa-miR-582-5p, hsa-miR-1290 | 100.00 | 100.00 | 100.00 |
| 1381 | hsa-miR-484, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-590-3p, hsa-miR-410-3p | 100.00 | 100.00 | 100.00 |
| 1382 | hsa-miR-484, hsa-miR-590-5p, hsa-miR-7-1-3p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-590-3p, hsa-miR-410-3p | 100.00 | 100.00 | 100.00 |
| 1383 | hsa-miR-376c-3p, hsa-miR-6803-3p, hsa-miR-455-3p, age, hsa-miR-382-5p, hsa-miR-342-3p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 100.00 | 100.00 | 100.00 |
| 1384 | hsa-miR-376c-3p, hsa-miR-545-5p, hsa-miR-6803-3p, hsa-miR-590-5p, hsa-miR-455-3p, hsa-miR-671-5p, hsa-miR-382-5p, hsa-miR-532-5p, hsa-miR-197-3p, hsa-miR-126-3p, hsa-miR-150-5p, hsa-miR-222-3p, hsa-miR-377-3p, hsa-miR-153-3p, hsa-miR-590-3p, hsa-miR-582-5p, hsa-miR-1290 | 100.00 | 100.00 | 100.00 |
| 1385 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-1290, hsa-miR-410-3p | 100.00 | 100.00 | 100.00 |
| 1386 | hsa-miR-376c-3p, hsa-miR-6803-3p, hsa-miR-455-3p, age, hsa-miR-342-3p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 100.00 | 100.00 | 100.00 |
| 1387 | hsa-miR-484, hsa-miR-30e-3p, hsa-miR-590-5p, hsa-miR-7-1-3p, hsa-miR-598-3p, hsa-miR-671-5p, hsa-miR-342-3p, hsa-miR-197-3p, hsa-miR-126-3p, hsa-miR-150-5p, hsa-let-7i-3p, hsa-miR-590-3p | 100.00 | 100.00 | 100.00 |
| 1388 | hsa-miR-484, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-377-3p, hsa-miR-590-3p | 100.00 | 100.00 | 100.00 |
| 1389 | hsa-miR-484, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-377-3p, hsa-miR-590-3p, hsa-miR-410-3p | 100.00 | 100.00 | 100.00 |
| 1390 | hsa-miR-484, hsa-miR-376c-3p, hsa-miR-545-5p, hsa-miR-590-5p, hsa-miR-7-1-3p, hsa-miR-598-3p, hsa-miR-671-5p, hsa-miR-382-5p, hsa-miR-197-3p, hsa-miR-150-5p, hsa-let-7i-3p, hsa-miR-377-3p, hsa-miR-590-3p | 100.00 | 100.00 | 100.00 |
| 1391 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-126-3p, hsa-miR-150-5p, hsa-let-7i-3p, hsa-miR-377-3p, hsa-miR-153-3p, hsa-miR-590-3p, hsa-miR-1290, hsa-miR-410-3p | 100.00 | 100.00 | 100.00 |
| 1392 | hsa-miR-484, hsa-miR-590-5p, hsa-let-7d-3p, hsa-miR-7-1-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-590-3p | 100.00 | 100.00 | 100.00 |
| 1393 | hsa-miR-484, hsa-miR-376c-3p, hsa-miR-6803-3p, hsa-miR-590-5p, hsa-miR-598-3p, hsa-miR-671-5p, age, hsa-miR-342-3p, hsa-miR-197-3p, hsa-miR-126-3p, hsa-miR-150-5p, hsalet-7i-3p, hsa-miR-377-3p, hsa-miR-153-3p, hsa-miR-590-3p, hsa-miR-1290, hsa-miR-410-3p | 100.00 | 100.00 | 100.00 |
| 1394 | hsa-miR-590-5p, hsa-miR-455-3p, hsa-let-7d-3p, hsa-miR-7-1-3p, hsa-miR-671-5p, age, hsa-miR-532-5p, hsa-miR-126-3p, hsa-miR-150-5p, hsa-let-7i-3p, hsa-miR-377-3p, hsa-miR-590-3p, hsa-miR-582-5p, hsa-miR-410-3p | 100.00 | 100.00 | 100.00 |
| 1395 | hsa-miR-484, hsa-miR-590-5p, hsa-let-7d-3p, hsa-miR-7-1-3p, hsa-miR-598-3p, hsa-miR-671-5p, hsa-miR-126-3p, hsa-miR-150-5p, hsa-let-7i-3p, hsa-miR-377-3p, hsa-miR-410-3p | 100.00 | 100.00 | 100.00 |
| 1396 | hsa-miR-376c-3p, hsa-miR-6803-3p, hsa-miR-455-3p, hsa-let-7d-3p, hsa-miR-671-5p, age, hsa-miR-126-3p, hsa-let-7i-3p, hsa-miR-377-3p, hsa-miR-153-3p, hsa-miR-590-3p, hsa-miR-582-5p, hsa-miR-1290, hsa-miR-410-3p | 100.00 | 100.00 | 100.00 |
| 1397 | hsa-miR-376c-3p, hsa-miR-545-5p, hsa-miR-6803-3p, hsa-miR-590-5p, hsa-miR-455-3p, hsa-let-7d-3p, hsa-miR-671-5p, hsa-miR-382-5p, hsa-miR-532-5p, hsa-miR-126-3p, hsa-miR-150-5p, hsa-let-7i-3p, hsa-miR-377-3p, hsa-miR-590-3p, hsa-miR-582-5p | 100.00 | 100.00 | 100.00 |
| 1398 | hsa-miR-6803-3p, hsa-miR-455-3p, hsa-let-7d-3p, hsa-miR-671-5p, age, hsa-miR-126-3p, hsa-let-7i-3p, hsa-miR-377-3p, hsa-miR-153-3p, hsa-miR-590-3p, hsa-miR-582-5p, hsa-miR-1290, hsa-miR-410-3p | 100.00 | 100.00 | 100.00 |
| 1399 | hsa-miR-484, hsa-miR-376c-3p, hsa-miR-545-5p, hsa-miR-590-5p, hsa-let-7d-3p, hsa-miR-7-1-3p, hsa-miR-598-3p, hsa-miR-671-5p, hsa-miR-342-3p, hsa-miR-126-3p, hsa-miR-150-5p, hsa-let-7i-3p, hsa-miR-377-3p, hsa-miR-590-3p | 100.00 | 100.00 | 100.00 |
| 1400 | hsa-miR-376c-3p, hsa-miR-6803-3p, hsa-miR-455-3p, hsa-miR-382-5p, hsa-miR-342-3p, hsa-miR-197-3p, hsa-miR-222-3p, hsa-miR-377-3p, hsa-miR-153-3p, hsa-miR-590-3p, hsa-miR-582-5p, hsa-miR-1290 | 100.00 | 100.00 | 100.00 |
| 1401 | hsa-miR-484, hsa-miR-376c-3p, hsa-miR-6803-3p, hsa-miR-590-5p, hsa-miR-598-3p, hsa-miR-671-5p, hsa-miR-342-3p, hsa-miR-197-3p, hsa-miR-150-5p, hsa-let-7i-3p, hsa-miR-377-3p, hsa-miR-153-3p, hsa-miR-590-3p, hsa-miR-1290 | 100.00 | 100.00 | 100.00 |
| 1402 | hsa-miR-376c-3p, hsa-miR-6803-3p, hsa-miR-455-3p, hsa-miR-342-3p, hsa-miR-197-3p, hsa-miR-222-3p, hsa-miR-377-3p, hsa-miR-153-3p, hsa-miR-590-3p, hsa-miR-582-5p, hsa-miR-1290 | 100.00 | 100.00 | 100.00 |
| 1403 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-1290 | 100.00 | 100.00 | 100.00 |
| 1404 | hsa-miR-376c-3p, hsa-miR-6803-3p, hsa-miR-455-3p, hsa-let-7d-3p, hsa-miR-671-5p, age, hsa-miR-342-3p, hsa-miR-532-5p, hsa-miR-126-3p, hsa-let-7i-3p, hsa-miR-377-3p, hsa-miR-153-3p, hsa-miR-590-3p, hsa-miR-582-5p, hsa-miR-1290, hsa-miR-410-3p | 100.00 | 100.00 | 100.00 |
| 1405 | hsa-miR-484, hsa-miR-376c-3p, hsa-miR-6803-3p, hsa-miR-590-5p, hsa-miR-598-3p, hsa-miR-671-5p, age, hsa-miR-342-3p, hsa-miR-197-3p, hsa-miR-126-3p, hsa-miR-150-5p, hsa-let-7i-3p, hsa-miR-377-3p, hsa-miR-153-3p, hsa-miR-590-3p, hsa-miR-1290 | 100.00 | 100.00 | 100.00 |
| 1406 | hsa-miR-484, hsa-miR-545-5p, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-377-3p, hsa-miR-590-3p | 100.00 | 100.00 | 100.00 |
| 1407 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-150-5p, hsa-let-7i-3p, hsa-miR-377-3p, hsa-miR-153-3p, hsa-miR-590-3p, hsa-miR-1290, hsa-miR-410-3p | 100.00 | 100.00 | 100.00 |
| 1408 | hsa-miR-376c-3p, hsa-miR-6803-3p, hsa-miR-455-3p, hsa-let-7d-3p, hsa-miR-671-5p, age, hsa-miR-342-3p, hsa-miR-126-3p, hsa-let-7i-3p, hsa-miR-377-3p, hsa-miR-153-3p, hsa-miR-590-3p, hsa-miR-582-5p, hsa-miR-1290, hsa-miR-410-3p | 100.00 | 100.00 | 100.00 |
| 1409 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-1290 | 100.00 | 100.00 | 100.00 |
| 1410 | hsa-miR-484, hsa-miR-376c-3p, hsa-miR-545-5p, hsa-miR-6803-3p, hsa-miR-590-5p, hsa-miR-598-3p, hsa-miR-671-5p, age, hsa-miR-342-3p, hsa-miR-197-3p, hsa-miR-126-3p, hsa-miR-150-5p, hsa-let-7i-3p, hsa-miR-377-3p, hsa-miR-153-3p, hsa-miR-590-3p, hsa-miR-1290 | 100.00 | 100.00 | 100.00 |
| 1411 | hsa-miR-376c-3p, hsa-miR-6803-3p, hsa-miR-455-3p, hsa-let-7d-3p, hsa-miR-671-5p, age, hsa-miR-342-3p, hsa-miR-126-3p, hsa-let-7i-3p, hsa-miR-377-3p, hsa-miR-153-3p, hsa-miR-590-3p, hsa-miR-582-5p, hsa-miR-1290 | 100.00 | 100.00 | 100.00 |
| 1412 | hsa-miR-484, hsa-miR-590-5p, hsa-let-7d-3p, hsa-miR-7-1-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-590-3p, hsa-miR-410-3p | 100.00 | 100.00 | 100.00 |
| 1413 | hsa-miR-545-5p, hsa-miR-6803-3p, hsa-miR-455-3p, hsa-miR-382-5p, hsa-miR-342-3p, hsa-miR-197-3p, hsa-miR-222-3p, hsa-miR-377-3p, hsa-miR-153-3p, hsa-miR-590-3p, hsa-miR-582-5p, hsa-miR-1290 | 100.00 | 100.00 | 100.00 |
| 1414 | hsa-miR-376c-3p, hsa-miR-545-5p, hsa-miR-6803-3p, hsa-miR-455-3p, hsa-miR-671-5p, age, hsa-miR-342-3p, hsa-miR-197-3p, hsa-let-7i-3p, hsa-miR-377-3p, hsa-miR-153-3p, hsa-miR-590-3p, hsa-miR-582-5p, hsa-miR-1290 | 100.00 | 100.00 | 100.00 |
| 1415 | hsa-miR-484, hsa-miR-376c-3p, hsa-miR-590-5p, hsa-miR-7-1-3p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-590-3p, hsa-miR-410-3p | 100.00 | 100.00 | 100.00 |
| 1416 | hsa-miR-484, hsa-miR-545-5p, hsa-miR-590-5p, hsa-let-7d-3p, hsa-miR-7-1-3p, hsa-miR-598-3p, hsa-miR-671-5p, hsa-miR-126-3p, hsa-miR-150-5p, hsa-let-7i-3p, hsa-miR-377-3p, hsa-miR-590-3p | 100.00 | 100.00 | 100.00 |
| 1417 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-1290 | 100.00 | 100.00 | 100.00 |
| 1418 | hsa-miR-376c-3p, hsa-miR-6803-3p, hsa-miR-455-3p, age, hsa-miR-342-3p, hsa-miR-532-5p, hsa-miR-197-3p, hsa-miR-126-3p, hsa-let-7i-3p, hsa-miR-377-3p, hsa-miR-153-3p, hsa-miR-590-3p, hsa-miR-582-5p, hsa-miR-1290, hsa-miR-410-3p | 98.95 | 99.41 | 100.00 |
| 1419 | hsa-miR-484, hsa-miR-376c-3p, hsa-miR-545-5p, hsa-miR-6803-3p, hsa-miR-590-5p, hsa-miR-598-3p, hsa-miR-671-5p, age, hsa-miR-342-3p, hsa-miR-532-5p, hsa-miR-197-3p, hsa-miR-150-5p, hsa-let-7i-3p, hsa-miR-377-3p, hsa-miR-153-3p, hsa-miR-590-3p, hsa-miR-1290 | 98.95 | 100.00 | 90.00 |
| 1420 | hsa-miR-484, hsa-miR-376c-3p, hsa-miR-6803-3p, hsa-miR-590-5p, hsa-miR-598-3p, hsa-miR-671-5p, age, hsa-miR-342-3p, hsa-miR-532-5p, hsa-miR-197-3p, hsa-miR-150-5p, hsa-let-7i-3p, hsa-miR-377-3p, hsa-miR-153-3p, hsa-miR-590-3p, hsa-miR-1290, hsa-miR-410-3p | 98.95 | 100.00 | 90.00 |
| 1421 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-342-3p, hsa-miR-532-5p, hsa-miR-126-3p, hsa-miR-150-5p, hsa-let-7i-3p, hsa-miR-377-3p, hsa-miR-153-3p, hsa-miR-590-3p, hsa-miR-1290 | 98.95 | 99.41 | 90.00 |
| 1422 | hsa-miR-484, hsa-miR-376c-3p, hsa-miR-545-5p, hsa-miR-6803-3p, hsa-miR-590-5p, hsa-miR-598-3p, hsa-miR-671-5p, age, hsa-miR-342-3p, hsa-miR-532-5p, hsa-miR-197-3p, hsa-miR-150-5p, hsa-let-7i-3p, hsa-miR-377-3p, hsa-miR-153-3p, hsa-miR-590-3p, hsa-miR-1290, hsa-miR-410-3p | 98.95 | 100.00 | 90.00 |
| 1423 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, hsa-miR-532-5p, hsa-miR-126-3p, hsa-miR-150-5p, hsa-let-7i-3p, hsa-miR-377-3p, hsa-miR-153-3p, hsa-miR-590-3p, hsa-miR-1290 | 98.95 | 100.00 | 90.00 |
| 1424 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-532-5p, hsa-miR-126-3p, hsa-miR-150-5p, hsa-let-7i-3p, hsa-miR-377-3p, hsa-miR-153-3p, hsa-miR-590-3p, hsa-miR-1290, hsa-miR-410-3p | 98.95 | 100.00 | 90.00 |
| 1425 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-532-5p, hsa-miR-126-3p, hsa-miR-150-5p, hsa-let-7i-3p, hsa-miR-377-3p, hsa-miR-153-3p, hsa-miR-590-3p, hsa-miR-1290 | 98.95 | 100.00 | 90.00 |
| 1426 | hsa-miR-484, hsa-miR-376c-3p, hsa-miR-6803-3p, hsa-miR-590-5p, hsa-miR-598-3p, hsa-miR-671-5p, age, hsa-miR-532-5p, hsa-miR-197-3p, hsa-miR-150-5p, hsa-let-7i-3p, hsa-miR-377-3p, hsa-miR-153-3p, hsa-miR-590-3p, hsa-miR-1290 | 98.95 | 100.00 | 90.00 |
| 1427 | hsa-miR-484, hsa-miR-545-5p, hsa-miR-590-5p, hsa-miR-7-1-3p, hsa-miR-598-3p, hsa-miR-671-5p, age, hsa-miR-197-3p, hsa-miR-150-5p, hsa-let-7i-3p, hsa-miR-377-3p, hsa-miR-590-3p | 98.95 | 100.00 | 90.00 |
| 1428 | hsa-miR-484, hsa-miR-376c-3p, hsa-miR-545-5p, hsa-miR-590-5p, hsa-let-7d-3p, hsa-miR-7-1-3p, hsa-miR-598-3p, hsa-miR-671-5p, hsa-miR-126-3p, hsa-miR-150-5p, hsa-let-7i-3p, hsa-miR-377-3p, hsa-miR-590-3p | 98.95 | 100.00 | 100.00 |
| 1429 | hsa-miR-590-5p, hsa-miR-455-3p, hsa-miR-7-1-3p, hsa-miR-598-3p, hsa-miR-671-5p, age, hsa-miR-342-3p, hsa-miR-532-5p, hsa-miR-197-3p, hsa-miR-150-5p, hsa-let-7i-3p, hsa-miR-377-3p, hsa-miR-590-3p, hsa-miR-582-5p, hsa-miR-410-3p | 98.95 | 99.41 | 90.00 |
| 1430 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-532-5p, hsa-miR-126-3p, hsa-miR-150-5p, hsa-let-7i-3p, hsa-miR-377-3p, hsa-miR-153-3p, hsa-miR-590-3p, hsa-miR-1290 | 98.95 | 100.00 | 90.00 |
| 1431 | hsa-miR-484, hsa-miR-376c-3p, hsa-miR-6803-3p, hsa-miR-590-5p, hsa-miR-598-3p, hsa-miR-671-5p, age, hsa-miR-382-5p, hsa-miR-342-3p, hsa-miR-532-5p, hsa-miR-197-3p, hsa-miR-150-5p, hsa-let-7i-3p, hsa-miR-377-3p, hsa-miR-153-3p, hsa-miR-590-3p, hsa-miR-1290, hsa-miR-410-3p | 98.95 | 100.00 | 90.00 |
| 1432 | hsa-miR-30e-3p, hsa-miR-590-5p, hsa-miR-455-3p, hsa-let-7d-3p, hsa-miR-7-1-3p, hsa-miR-671-5p, age, hsa-miR-532-5p, hsa-miR-126-3p, hsa-miR-150-5p, hsa-let-7i-3p, hsa-miR-590-3p, hsa-miR-582-5p, hsa-miR-410-3p, hsa-let-7g-3p | 98.95 | 100.00 | 100.00 |
| 1433 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-532-5p, hsa-miR-150-5p, hsa-let-7i-3p, hsa-miR-377-3p, hsa-miR-153-3p, hsa-miR-590-3p, hsa-miR-1290 | 98.95 | 100.00 | 90.00 |
| 1434 | hsa-miR-484, hsa-miR-30e-3p, hsa-miR-376c-3p, hsa-miR-545-5p, hsa-miR-6803-3p, hsa-miR-590-5p, hsa-miR-598-3p, hsa-miR-671-5p, hsa-miR-382-5p, hsa-miR-532-5p, hsa-miR-197-3p, hsa-miR-126-3p, hsa-miR-150-5p, hsa-let-7i-3p, hsa-miR-377-3p, hsa-miR-153-3p, hsa-miR-590-3p, hsa-miR-1290 | 98.89 | 100.00 | 90.00 |
| 1435 | hsa-miR-484, hsa-miR-30e-3p, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-153-3p, hsa-miR-590-3p, hsa-miR-1290, hsa-miR-410-3p, hsa-let-7g-3p | 98.89 | 96.88 | 90.00 |
| 1436 | hsa-miR-484, hsa-miR-30e-3p, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-1290 | 98.89 | 98.13 | 90.00 |
| 1437 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, hsa-miR-382-5p, hsa-miR-342-3p, hsa-miR-532-5p, hsa-miR-126-3p, hsa-miR-150-5p, hsa-let-7i-3p, hsa-miR-377-3p, hsa-miR-153-3p, hsa-miR-1290, hsa-miR-410-3p | 98.89 | 99.38 | 90.00 |
| 1438 | hsa-miR-484, hsa-miR-30e-3p, hsa-miR-590-5p, hsa-miR-7-1-3p, hsa-miR-598-3p, hsa-miR-671-5p, age, hsa-miR-342-3p, hsa-miR-197-3p, hsa-miR-150-5p, hsa-let-7i-3p, hsa-miR-590-3p, hsa-miR-410-3p | 98.89 | 99.38 | 90.00 |
| 1439 | hsa-miR-376c-3p, hsa-miR-6803-3p, hsa-miR-455-3p, hsa-miR-382-5p, hsa-miR-342-3p, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 98.89 | 100.00 | 100.00 |
| 1440 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-150-5p, hsa-let-7i-3p, hsa-miR-377-3p, hsa-miR-153-3p, hsa-miR-590-3p, hsa-miR-1290 | 98.89 | 98.75 | 90.00 |
| 1441 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-miR-222-3p, hsa-miR-377-3p, hsa-miR-153-3p, hsa-miR-590-3p, hsa-miR-1290 | 98.89 | 100.00 | 100.00 |
| 1442 | hsa-miR-545-5p, hsa-miR-6803-3p, hsa-miR-455-3p, hsa-let-7d-3p, hsa-miR-382-5p, hsa-miR-342-3p, hsa-miR-126-3p, hsa-let-7i-3p, hsa-miR-377-3p, hsa-miR-153-3p, hsa-miR-582-5p, hsa-miR-1290 | 98.89 | 98.75 | 90.00 |
| 1443 | hsa-miR-484, hsa-miR-30e-3p, hsa-miR-376c-3p, hsa-miR-545-5p, hsa-miR-6803-3p, hsa-miR-590-5p, hsa-miR-598-3p, hsa-miR-671-5p, age, hsa-miR-197-3p, hsa-miR-150-5p, hsa-let-7i-3p, hsa-miR-153-3p, hsa-miR-590-3p, hsa-miR-1290, hsa-let-7g-3p | 98.89 | 100.00 | 90.00 |
| 1444 | hsa-miR-484, hsa-miR-376c-3p, hsa-miR-545-5p, hsa-miR-6803-3p, hsa-miR-590-5p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-153-3p, hsa-miR-590-3p, hsa-miR-1290, hsa-miR-410-3p | 98.89 | 100.00 | 80.00 |
| 1445 | hsa-miR-376c-3p, hsa-miR-545-5p, hsa-miR-6803-3p, hsa-miR-455-3p, hsa-miR-382-5p, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 98.89 | 100.00 | 100.00 |
| 1446 | hsa-miR-376c-3p, hsa-miR-545-5p, hsa-miR-6803-3p, hsa-miR-455-3p, age, hsa-miR-382-5p, hsa-miR-342-3p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 98.89 | 100.00 | 100.00 |
| 1447 | hsa-miR-376c-3p, hsa-miR-6803-3p, hsa-miR-455-3p, hsa-let-7d-3p, age, hsa-miR-342-3p, hsa-miR-126-3p, hsa-miR-222-3p, hsa-miR-377-3p, hsa-miR-153-3p, hsa-miR-590-3p, hsa-miR-582-5p, hsa-miR-1290 | 98.89 | 100.00 | 90.00 |
| 1448 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-1290, hsa-miR-410-3p | 98.89 | 100.00 | 90.00 |
| 1449 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-1290, hsa-miR-410-3p | 98.89 | 100.00 | 80.00 |
| 1450 | hsa-miR-484, hsa-miR-376c-3p, hsa-miR-590-5p, hsa-let-7d-3p, hsa-miR-7-1-3p, hsa-miR-598-3p, hsa-miR-671-5p, age, hsa-miR-342-3p, hsa-miR-150-5p, hsa-let-7i-3p, hsa-miR-377-3p, hsa-miR-590-3p, hsa-miR-410-3p | 98.89 | 99.38 | 90.00 |
| 1451 | hsa-miR-376c-3p, hsa-miR-6803-3p, hsa-miR-455-3p, age, hsa-miR-382-5p, hsa-miR-342-3p, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p, hsa-let-7g-3p | 98.89 | 91.76 | 80.00 |
| 1452 | hsa-miR-484, hsa-miR-590-5p, hsa-let-7d-3p, hsa-miR-7-1-3p, hsa-miR-598-3p, hsa-miR-671-5p, age, hsa-miR-150-5p, hsa-let-7i-3p, hsa-miR-377-3p, hsa-miR-410-3p | 98.89 | 100.00 | 90.00 |
| 1453 | hsa-miR-545-5p, hsa-miR-6803-3p, hsa-miR-590-5p, hsa-miR-455-3p, hsa-let-7d-3p, hsa-miR-671-5p, hsa-miR-382-5p, hsa-miR-342-3p, hsa-miR-532-5p, hsa-miR-126-3p, hsa-miR-150-5p, hsa-let-7i-3p, hsa-miR-377-3p, hsa-miR-590-3p, hsa-miR-582-5p | 98.89 | 100.00 | 90.00 |
| 1454 | hsa-miR-545-5p, hsa-miR-6803-3p, hsa-miR-455-3p, hsa-miR-382-5p, hsa-miR-342-3p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 98.89 | 100.00 | 100.00 |
| 1455 | hsa-miR-6803-3p, hsa-miR-455-3p, hsa-let-7d-3p, hsa-miR-671-5p, age, hsa-let-7i-3p, hsa-miR-377-3p, hsa-miR-153-3p, hsa-miR-582-5p, hsa-miR-1290, hsa-miR-410-3p, hsa-let-7g-3p | 98.89 | 98.82 | 100.00 |
| 1456 | hsa-miR-484, hsa-miR-30e-3p, hsa-miR-590-5p, hsa-miR-7-1-3p, hsa-miR-598-3p, hsa-miR-671-5p, hsa-miR-342-3p, hsa-miR-197-3p, hsa-miR-150-5p, hsa-let-7i-3p, hsa-miR-590-3p | 98.89 | 99.38 | 90.00 |
| 1457 | hsa-miR-484, hsa-miR-376c-3p, hsa-miR-6803-3p, hsa-miR-590-5p, hsa-miR-455-3p, hsa-let-7d-3p, hsa-miR-671-5p, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-582-5p, hsa-miR-1290, hsa-miR-410-3p | 98.89 | 100.00 | 90.00 |
| 1458 | hsa-miR-484, hsa-miR-376c-3p, hsa-miR-545-5p, hsa-miR-6803-3p, hsa-miR-590-5p, hsa-miR-598-3p, hsa-miR-671-5p, hsa-miR-342-3p, hsa-miR-197-3p, hsa-miR-150-5p, hsa-let-7i-3p, hsa-miR-377-3p, hsa-miR-153-3p, hsa-miR-590-3p, hsa-miR-1290 | 98.89 | 100.00 | 90.00 |
| 1459 | hsa-miR-545-5p, hsa-miR-6803-3p, hsa-miR-455-3p, hsa-let-7d-3p, hsa-miR-382-5p, hsa-miR-342-3p, hsa-miR-126-3p, hsa-miR-222-3p, hsa-miR-377-3p, hsa-miR-153-3p, hsa-miR-582-5p, hsa-miR-1290 | 98.89 | 98.75 | 90.00 |
| 1460 | hsa-miR-484, hsa-miR-376c-3p, hsa-miR-545-5p, hsa-miR-6803-3p, hsa-miR-590-5p, hsa-miR-598-3p, hsa-miR-671-5p, age, hsa-miR-342-3p, hsa-miR-197-3p, hsa-miR-126-3p, hsa-miR-150-5p, hsa-let-7i-3p, hsa-miR-153-3p, hsa-miR-590-3p, hsa-miR-1290 | 98.89 | 99.38 | 90.00 |
| 1461 | hsa-miR-484, hsa-miR-376c-3p, hsa-miR-545-5p, hsa-miR-6803-3p, hsa-miR-598-3p, hsa-miR-671-5p, hsa-miR-382-5p, hsa-miR-197-3p, hsa-miR-126-3p, hsa-miR-222-3p, hsa-miR-377-3p, hsa-miR-153-3p, hsa-miR-590-3p, hsa-miR-582-5p, hsa-miR-1290 | 98.89 | 100.00 | 100.00 |
| 1462 | hsa-miR-376c-3p, hsa-miR-6803-3p, hsa-miR-455-3p, hsa-miR-671-5p, hsa-miR-197-3p, hsa-miR-126-3p, hsa-let-7i-3p, hsa-miR-377-3p, hsa-miR-153-3p, hsa-miR-590-3p, hsa-miR-582-5p, hsa-miR-1290 | 98.89 | 100.00 | 100.00 |
| 1463 | hsa-miR-376c-3p, hsa-miR-6803-3p, hsa-miR-455-3p, hsa-let-7d-3p, age, hsa-miR-342-3p, hsa-miR-126-3p, hsa-miR-222-3p, hsa-miR-377-3p, hsa-miR-153-3p, hsa-miR-590-3p, hsa-miR-582-5p, hsa-miR-1290, hsa-miR-410-3p, hsa-let-7g-3p | 98.89 | 95.29 | 80.00 |
| 1464 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, hsa-miR-342-3p, hsa-miR-150-5p, hsa-let-7i-3p, hsa-miR-377-3p, hsa-miR-153-3p, hsa-miR-590-3p, hsa-miR-1290 | 98.89 | 100.00 | 90.00 |
| 1465 | hsa-miR-455-3p, hsa-miR-7-1-3p, hsa-miR-671-5p, age, hsa-miR-342-3p, hsa-miR-197-3p, hsa-let-7i-3p, hsa-miR-377-3p, hsa-miR-590-3p, hsa-miR-582-5p | 98.89 | 98.13 | 90.00 |
| 1466 | hsa-miR-376c-3p, hsa-miR-545-5p, hsa-miR-6803-3p, hsa-miR-455-3p, hsa-miR-671-5p, age, hsa-miR-342-3p, hsa-miR-197-3p, hsa-miR-222-3p, hsa-miR-377-3p, hsa-miR-153-3p, hsa-miR-590-3p, hsa-miR-582-5p, hsa-miR-1290 | 98.89 | 100.00 | 100.00 |
| 1467 | hsa-miR-484, hsa-miR-30e-3p, hsa-miR-590-5p, hsa-let-7d-3p, hsa-miR-7-1-3p, hsa-miR-598-3p, hsa-miR-671-5p, hsa-miR-342-3p, hsa-miR-126-3p, hsa-miR-150-5p, hsa-let-7i-3p, hsa-miR-410-3p | 98.89 | 99.38 | 90.00 |
| 1468 | hsa-miR-484, hsa-miR-545-5p, hsa-miR-6803-3p, hsa-miR-590-5p, hsa-let-7d-3p, hsa-miR-598-3p, age, hsa-miR-342-3p, hsa-miR-197-3p, hsa-miR-222-3p, hsa-miR-377-3p, hsa-miR-590-3p | 98.89 | 100.00 | 90.00 |
| 1469 | hsa-miR-484, hsa-miR-30e-3p, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-153-3p, hsa-miR-590-3p, hsa-miR-1290, hsa-let-7g-3p | 98.89 | 98.13 | 90.00 |
| 1470 | hsa-miR-484, hsa-miR-376c-3p, hsa-miR-590-5p, hsa-let-7d-3p, hsa-miR-7-1-3p, age, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-410-3p | 98.89 | 97.65 | 80.00 |
| 1471 | hsa-miR-484, hsa-miR-590-5p, hsa-miR-7-1-3p, hsa-miR-598-3p, age, hsa-miR-382-5p, hsa-miR-342-3p, hsa-miR-197-3p, hsa-miR-126-3p, hsa-miR-150-5p, hsa-miR-222-3p, hsa-miR-377-3p, hsa-miR-590-3p, hsa-miR-410-3p | 98.89 | 99.38 | 100.00 |
| 1472 | hsa-miR-376c-3p, hsa-miR-6803-3p, hsa-miR-455-3p, age, hsa-miR-382-5p, hsa-miR-342-3p, hsa-miR-222-3p, hsa-miR-377-3p, hsa-miR-153-3p, hsa-miR-590-3p, hsa-miR-582-5p, hsa-miR-1290, hsa-miR-410-3p | 98.89 | 100.00 | 100.00 |
| 1473 | hsa-miR-376c-3p, hsa-miR-6803-3p, hsa-miR-455-3p, hsa-let-7d-3p, hsa-miR-671-5p, age, hsa-miR-382-5p, hsa-miR-342-3p, hsa-miR-532-5p, hsa-miR-126-3p, hsa-miR-222-3p, hsa-miR-377-3p, hsa-miR-153-3p, hsa-miR-590-3p, hsa-miR-582-5p, hsa-miR-1290 | 98.89 | 100.00 | 80.00 |
| 1474 | hsa-miR-376c-3p, hsa-miR-545-5p, hsa-miR-6803-3p, hsa-miR-455-3p, hsa-miR-671-5p, hsa-miR-342-3p, hsa-miR-197-3p, hsa-miR-126-3p, hsa-let-7i-3p, hsa-miR-377-3p, hsa-miR-153-3p, hsa-miR-582-5p, hsa-miR-1290 | 98.89 | 99.38 | 90.00 |
| 1475 | hsa-miR-376c-3p, hsa-miR-455-3p, hsa-miR-7-1-3p, age, hsa-miR-382-5p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 98.89 | 98.82 | 100.00 |
| 1476 | hsa-miR-484, hsa-miR-30e-3p, hsa-miR-376c-3p, hsa-miR-545-5p, hsa-miR-590-5p, hsa-miR-7-1-3p, hsa-miR-598-3p, age, hsa-miR-342-3p, hsa-miR-197-3p, hsa-miR-126-3p, hsa-miR-150-5p, hsa-let-7i-3p, hsa-miR-153-3p, hsa-miR-590-3p, hsa-miR-582-5p, hsa-miR-1290, hsa-let-7g-3p | 98.89 | 97.65 | 80.00 |
| 1477 | hsa-miR-484, hsa-miR-30e-3p, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-126-3p, hsa-miR-150-5p, hsa-let-7i-3p, hsa-miR-153-3p, hsa-miR-590-3p, hsa-miR-1290, hsa-let-7g-3p | 98.89 | 98.13 | 90.00 |
| 1478 | hsa-miR-484, hsa-miR-590-5p, hsa-miR-7-1-3p, hsa-miR-598-3p, hsa-miR-671-5p, hsa-miR-382-5p, hsa-miR-197-3p, hsa-miR-126-3p, hsa-miR-150-5p, hsa-miR-222-3p, hsa-miR-377-3p, hsa-miR-590-3p, hsa-miR-410-3p | 98.89 | 100.00 | 100.00 |
| 1479 | hsa-miR-484, hsa-miR-376c-3p, hsa-miR-545-5p, hsa-miR-6803-3p, hsa-miR-590-5p, hsa-miR-598-3p, hsa-miR-671-5p, hsa-miR-382-5p, hsa-miR-342-3p, hsa-miR-197-3p, hsa-miR-150-5p, hsa-miR-222-3p, hsa-miR-377-3p, hsa-miR-153-3p, hsa-miR-590-3p, hsa-miR-1290 | 98.89 | 100.00 | 90.00 |
| 1480 | hsa-miR-376c-3p, hsa-miR-545-5p, hsa-miR-6803-3p, hsa-miR-455-3p, age, hsa-miR-382-5p, hsa-miR-342-3p, hsa-miR-197-3p, hsa-miR-222-3p, hsa-miR-377-3p, hsa-miR-153-3p, hsa-miR-582-5p, hsa-miR-1290 | 98.89 | 100.00 | 90.00 |
| 1481 | hsa-miR-376c-3p, hsa-miR-545-5p, hsa-miR-6803-3p, hsa-miR-455-3p, hsa-miR-671-5p, age, hsa-miR-342-3p, hsa-miR-197-3p, hsa-let-7i-3p, hsa-miR-377-3p, hsa-miR-153-3p, hsa-miR-590-3p, hsa-miR-582-5p, hsa-miR-1290, hsa-miR-410-3p | 98.89 | 100.00 | 100.00 |
| 1482 | hsa-miR-484, hsa-miR-545-5p, hsa-miR-590-5p, hsa-miR-7-1-3p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-590-3p | 98.89 | 100.00 | 100.00 |
| 1483 | hsa-miR-484, hsa-miR-30e-3p, hsa-miR-376c-3p, hsa-miR-545-5p, hsa-miR-6803-3p, hsa-miR-590-5p, hsa-miR-598-3p, hsa-miR-671-5p, hsa-miR-197-3p, hsa-miR-150-5p, hsa-let-7i-3p, hsa-miR-153-3p, hsa-miR-590-3p, hsa-miR-1290, hsa-let-7g-3p | 98.89 | 97.5 | 90.00 |
| 1484 | hsa-miR-484, hsa-miR-376c-3p, hsa-miR-545-5p, hsa-miR-6803-3p, hsa-miR-590-5p, hsa-miR-598-3p, age, hsa-miR-382-5p, hsa-miR-342-3p, hsa-miR-197-3p, hsa-miR-150-5p, hsa-miR-222-3p, hsa-miR-377-3p, hsa-miR-153-3p, hsa-miR-590-3p, hsa-miR-1290, hsa-miR-410-3p | 98.89 | 99.38 | 90.00 |
| 1485 | hsa-miR-376c-3p, hsa-miR-6803-3p, hsa-miR-455-3p, hsa-miR-671-5p, age, hsa-miR-382-5p, hsa-miR-197-3p, hsa-miR-126-3p, hsa-let-7i-3p, hsa-miR-377-3p, hsa-miR-153-3p, hsa-miR-590-3p, hsa-miR-582-5p, hsa-miR-1290, hsa-miR-410-3p | 98.89 | 100.00 | 100.00 |
| 1486 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-342-3p, hsa-miR-150-5p, hsa-let-7i-3p, hsa-miR-377-3p, hsa-miR-153-3p, hsa-miR-590-3p, hsa-miR-1290 | 98.89 | 100.00 | 90.00 |
| 1487 | hsa-miR-376c-3p, hsa-miR-6803-3p, hsa-miR-455-3p, age, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 98.89 | 100.00 | 100.00 |
| 1488 | hsa-miR-376c-3p, hsa-miR-545-5p, hsa-miR-6803-3p, hsa-miR-455-3p, age, hsa-miR-382-5p, hsa-miR-342-3p, hsa-miR-532-5p, hsa-miR-197-3p, hsa-miR-222-3p, hsa-miR-377-3p, hsa-miR-153-3p, hsa-miR-582-5p, hsa-miR-1290 | 98.89 | 100.00 | 90.00 |
| 1489 | hsa-miR-376c-3p, hsa-miR-6803-3p, hsa-miR-455-3p, hsa-miR-671-5p, age, hsa-miR-342-3p, hsa-let-7i-3p, hsa-miR-377-3p, hsa-miR-153-3p, hsa-miR-590-3p, hsa-miR-582-5p, hsa-miR-1290 | 98.89 | 100.00 | 90.00 |
| 1490 | hsa-miR-484, hsa-miR-376c-3p, hsa-miR-545-5p, hsa-miR-6803-3p, hsa-miR-590-5p, hsa-miR-598-3p, hsa-miR-671-5p, hsa-miR-342-3p, hsa-miR-197-3p, hsa-miR-126-3p, hsa-miR-150-5p, hsa-let-7i-3p, hsa-miR-377-3p, hsa-miR-153-3p, hsa-miR-590-3p, hsa-miR-1290 | 98.89 | 100.00 | 90.00 |
| 1491 | hsa-miR-376c-3p, hsa-miR-6803-3p, hsa-miR-455-3p, hsa-let-7d-3p, age, hsa-miR-382-5p, hsa-miR-342-3p, hsa-miR-126-3p, hsa-miR-222-3p, hsa-miR-377-3p, hsa-miR-153-3p, hsa-miR-590-3p, hsa-miR-582-5p, hsa-miR-1290 | 98.89 | 100.00 | 80.00 |
| 1492 | hsa-miR-376c-3p, hsa-miR-6803-3p, hsa-miR-455-3p, age, hsa-miR-382-5p, hsa-miR-342-3p, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 98.89 | 100.00 | 100.00 |
| 1493 | hsa-miR-376c-3p, hsa-miR-6803-3p, hsa-miR-455-3p, age, hsa-miR-342-3p, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 98.89 | 100.00 | 100.00 |
| 1494 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-382-5p, hsa-miR-342-3p, hsa-miR-150-5p, hsa-let-7i-3p, hsa-miR-377-3p, hsa-miR-153-3p, hsa-miR-590-3p, hsa-miR-1290, hsa-miR-410-3p | 98.89 | 100.00 | 90.00 |
| 1495 | hsa-miR-484, hsa-miR-376c-3p, hsa-miR-545-5p, hsa-miR-6803-3p, hsa-miR-598-3p, age, hsa-miR-382-5p, hsa-miR-342-3p, hsa-miR-197-3p, hsa-miR-222-3p, hsa-miR-377-3p, hsa-miR-153-3p, hsa-miR-590-3p, hsa-miR-1290, hsa-miR-410-3p | 98.89 | 98.75 | 90.00 |
| 1496 | hsa-miR-484, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-miR-222-3p, hsa-miR-377-3p, hsa-miR-153-3p, hsa-miR-590-3p, hsa-miR-1290, hsa-miR-410-3p | 98.89 | 98.75 | 90.00 |
| 1497 | hsa-miR-484, hsa-miR-6803-3p, hsa-miR-590-5p, hsa-let-7d-3p, hsa-miR-598-3p, hsa-miR-671-5p, age, hsa-miR-342-3p, hsa-miR-150-5p, hsa-let-7i-3p, hsa-miR-377-3p, hsa-miR-153-3p, hsa-miR-590-3p, hsa-miR-1290, hsa-miR-410-3p | 98.89 | 98.75 | 90.00 |
| 1498 | hsa-miR-484, hsa-miR-545-5p, hsa-let-7d-3p, hsa-miR-7-1-3p, hsa-miR-598-3p, hsa-miR-671-5p, hsa-miR-126-3p, hsa-let-7i-3p, hsa-miR-377-3p, hsa-miR-410-3p | 98.89 | 100.00 | 80.00 |
| 1499 | hsa-miR-376c-3p, hsa-miR-545-5p, hsa-miR-6803-3p, hsa-miR-455-3p, age, hsa-miR-382-5p, hsa-miR-342-3p, hsa-miR-532-5p, hsa-miR-197-3p, hsa-miR-222-3p, hsa-miR-377-3p, hsa-miR-153-3p, hsa-miR-582-5p, hsa-miR-1290, hsa-let-7g-3p | 98.89 | 98.82 | 80.00 |
| 1500 | hsa-miR-376c-3p, hsa-miR-545-5p, hsa-miR-6803-3p, hsa-miR-455-3p, hsa-let-7d-3p, hsa-miR-671-5p, hsa-miR-342-3p, hsa-let-7i-3p, hsa-miR-377-3p, hsa-miR-153-3p, hsa-miR-582-5p | 98.89 | 96.88 | 90.00 |
| 1501 | hsa-miR-484, hsa-miR-376c-3p, hsa-miR-590-5p, hsa-let-7d-3p, hsa-miR-7-1-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-590-3p, hsa-miR-410-3p | 98.89 | 100.00 | 100.00 |
| 1502 | hsa-miR-484, hsa-miR-30e-3p, hsa-miR-376c-3p, hsa-miR-6803-3p, hsa-miR-590-5p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-153-3p, hsa-miR-590-3p, hsa-miR-1290, hsa-miR-410-3p | 98.89 | 99.38 | 90.00 |
| 1503 | hsa-miR-30e-3p, hsa-miR-376c-3p, hsa-miR-6803-3p, hsa-miR-590-5p, hsa-miR-455-3p, hsa-miR-671-5p, age, hsa-miR-532-5p, hsa-miR-197-3p, hsa-miR-150-5p, hsa-let-7i-3p, hsa-miR-153-3p, hsa-miR-590-3p, hsa-miR-582-5p, hsa-miR-1290, hsa-miR-410-3p | 97.89 | 99.41 | 90.00 |
| 1504 | hsa-miR-484, hsa-miR-545-5p, hsa-miR-590-5p, hsa-miR-7-1-3p, hsa-miR-598-3p, hsa-miR-671-5p, age, hsa-miR-342-3p, hsa-miR-532-5p, hsa-miR-197-3p, hsa-miR-150-5p, hsa-let-7i-3p, hsa-miR-377-3p, hsa-miR-590-3p | 97.89 | 100.00 | 80.00 |
| 1505 | hsa-miR-484, hsa-miR-545-5p, hsa-miR-590-5p, hsa-let-7d-3p, hsa-miR-7-1-3p, hsa-miR-598-3p, hsa-miR-671-5p, age, hsa-miR-532-5p, hsa-miR-126-3p, hsa-miR-150-5p, hsa-let-7i-3p, hsa-miR-377-3p, hsa-miR-590-3p | 97.89 | 100.00 | 80.00 |
| 1506 | hsa-miR-376c-3p, hsa-miR-545-5p, hsa-miR-6803-3p, hsa-miR-590-5p, hsa-miR-455-3p, hsa-miR-671-5p, hsa-miR-382-5p, hsa-miR-532-5p, hsa-miR-197-3p, hsa-miR-126-3p, hsa-miR-150-5p, hsa-let-7i-3p, hsa-miR-377-3p, hsa-miR-590-3p, hsa-miR-582-5p | 97.84 | 100.00 | 80.00 |
| 1507 | hsa-miR-484, hsa-miR-376c-3p, hsa-miR-545-5p, hsa-miR-6803-3p, hsa-miR-590-5p, hsa-miR-598-3p, hsa-miR-671-5p, age, hsa-miR-382-5p, hsa-miR-342-3p, hsa-miR-532-5p, hsa-miR-197-3p, hsa-miR-150-5p, hsa-let-7i-3p, hsa-miR-377-3p, hsa-miR-153-3p, hsa-miR-1290 | 97.84 | 100.00 | 80.00 |
| 1508 | hsa-miR-484, hsa-miR-30e-3p, hsa-miR-376c-3p, hsa-miR-6803-3p, hsa-miR-590-5p, hsa-let-7d-3p, hsa-miR-598-3p, hsa-miR-671-5p, age, hsa-miR-382-5p, hsa-miR-342-3p, hsa-miR-532-5p, hsa-miR-126-3p, hsa-miR-150-5p, hsa-let-7i-3p, hsa-miR-377-3p, hsa-miR-153-3p, hsa-miR-590-3p, hsa-miR-1290, hsa-miR-410-3p | 97.84 | 99.38 | 80.00 |
| 1509 | hsa-miR-484, hsa-miR-590-5p, hsa-miR-7-1-3p, hsa-miR-598-3p, hsa-miR-671-5p, age, hsa-miR-197-3p, hsa-miR-150-5p, hsa-let-7i-3p, hsa-miR-377-3p, hsa-miR-410-3p | 97.84 | 99.38 | 90.00 |
| 1510 | hsa-miR-455-3p, hsa-let-7d-3p, hsa-miR-7-1-3p, hsa-miR-671-5p, age, hsa-miR-342-3p, hsa-let-7i-3p, hsa-miR-377-3p, hsa-miR-590-3p, hsa-miR-582-5p | 97.84 | 98.75 | 90.00 |
| 1511 | hsa-miR-484, hsa-miR-545-5p, hsa-miR-590-5p, hsa-let-7d-3p, hsa-miR-7-1-3p, hsa-miR-598-3p, hsa-miR-671-5p, age, hsa-miR-197-3p, hsa-miR-150-5p, hsa-let-7i-3p, hsa-miR-377-3p, hsa-miR-590-3p | 97.84 | 100.00 | 80.00 |
| 1512 | hsa-miR-590-5p, hsa-miR-455-3p, hsa-miR-7-1-3p, hsa-miR-382-5p, hsa-miR-532-5p, hsa-miR-197-3p, hsa-miR-150-5p, hsa-miR-222-3p, hsa-miR-377-3p, hsa-miR-590-3p, hsa-miR-582-5p, hsa-miR-410-3p | 97.84 | 100.00 | 80.00 |
| 1513 | hsa-miR-484, hsa-miR-30e-3p, hsa-miR-376c-3p, hsa-miR-545-5p, hsa-miR-6803-3p, hsa-miR-590-5p, hsa-miR-598-3p, hsa-miR-671-5p, age, hsa-miR-342-3p, hsa-miR-532-5p, hsa-miR-197-3p, hsa-miR-150-5p, hsa-let-7i-3p, hsa-miR-377-3p, hsa-miR-153-3p, hsa-miR-590-3p, hsa-miR-1290 | 97.84 | 100.00 | 80.00 |
| 1514 | hsa-miR-484, hsa-miR-545-5p, hsa-miR-6803-3p, hsa-miR-590-5p, hsa-let-7d-3p, hsa-miR-598-3p, hsa-miR-671-5p, hsa-miR-342-3p, hsa-miR-197-3p, hsa-miR-150-5p, hsa-miR-377-3p, hsa-miR-153-3p, hsa-miR-1290 | 97.84 | 96.25 | 80.00 |
| 1515 | hsa-miR-376c-3p, hsa-miR-545-5p, hsa-miR-6803-3p, hsa-miR-590-5p, hsa-miR-455-3p, hsa-miR-598-3p, hsa-miR-671-5p, age, hsa-miR-342-3p, hsa-miR-532-5p, hsa-miR-197-3p, hsa-miR-126-3p, hsa-miR-222-3p, hsa-miR-377-3p, hsa-miR-590-3p, hsa-miR-1290 | 97.84 | 100.00 | 80.00 |
| 1516 | hsa-miR-484, hsa-miR-590-5p, hsa-let-7d-3p, hsa-miR-7-1-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-590-3p, hsa-miR-410-3p, hsa-let-7g-3p | 97.84 | 96.47 | 80.00 |
| 1517 | hsa-miR-484, hsa-miR-590-5p, hsa-let-7d-3p, hsa-miR-7-1-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-590-3p, hsa-let-7g-3p | 97.84 | 96.47 | 80.00 |
| 1518 | hsa-miR-484, hsa-miR-376c-3p, hsa-miR-590-5p, hsa-miR-598-3p, hsa-miR-671-5p, age, hsa-miR-342-3p, hsa-miR-197-3p, hsa-miR-126-3p, hsa-miR-150-5p, hsa-let-7i-3p, hsa-miR-377-3p, hsa-miR-153-3p, hsa-miR-590-3p, hsa-miR-1290 | 97.84 | 98.82 | 70.00 |
| 1519 | hsa-miR-376c-3p, hsa-miR-6803-3p, hsa-miR-455-3p, hsa-miR-671-5p, age, hsa-miR-342-3p, hsa-miR-197-3p, hsa-let-7i-3p, hsa-miR-377-3p, hsa-miR-153-3p, hsa-miR-590-3p, hsa-miR-582-5p, hsa-miR-1290, hsa-miR-410-3p | 97.84 | 100.00 | 100.00 |
| 1520 | hsa-miR-376c-3p, hsa-miR-6803-3p, hsa-miR-455-3p, hsa-miR-671-5p, age, hsa-miR-342-3p, hsa-miR-532-5p, hsa-miR-197-3p, hsa-let-7i-3p, hsa-miR-377-3p, hsa-miR-153-3p, hsa-miR-590-3p, hsa-miR-582-5p, hsa-miR-1290, hsa-miR-410-3p | 97.84 | 100.00 | 100.00 |
| 1521 | hsa-miR-484, hsa-miR-376c-3p, hsa-miR-545-5p, hsa-miR-590-5p, hsa-miR-7-1-3p, hsa-miR-598-3p, age, hsa-miR-382-5p, hsa-miR-342-3p, hsa-miR-197-3p, hsa-miR-126-3p, hsa-miR-150-5p, hsa-miR-222-3p, hsa-miR-377-3p, hsa-miR-153-3p, hsa-miR-1290 | 97.84 | 100.00 | 90.00 |
| 1522 | hsa-miR-376c-3p, hsa-miR-545-5p, hsa-miR-6803-3p, hsa-miR-455-3p, age, hsa-miR-532-5p, hsa-miR-197-3p, hsa-miR-222-3p, hsa-miR-377-3p, hsa-miR-153-3p, hsa-miR-590-3p, hsa-miR-582-5p, hsa-miR-1290, hsa-miR-410-3p | 97.84 | 100.00 | 90.00 |
| 1523 | hsa-miR-484, hsa-miR-376c-3p, hsa-miR-545-5p, hsa-miR-590-5p, hsa-let-7d-3p, hsa-miR-7-1-3p, hsa-miR-598-3p, hsa-miR-671-5p, hsa-miR-342-3p, hsa-miR-532-5p, hsa-miR-126-3p, hsa-miR-150-5p, hsa-let-7i-3p, hsa-miR-377-3p, hsa-miR-590-3p, hsa-let-7g-3p | 97.84 | 98.82 | 70.00 |
| 1524 | hsa-miR-484, hsa-miR-590-5p, hsa-let-7d-3p, hsa-miR-7-1-3p, hsa-miR-598-3p, hsa-miR-671-5p, age, hsa-miR-342-3p, hsa-miR-126-3p, hsa-miR-150-5p, hsa-let-7i-3p, hsa-miR-590-3p, hsa-miR-410-3p, hsa-let-7g-3p | 97.84 | 96.47 | 80.00 |
| 1525 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, hsa-miR-342-3p, hsa-miR-126-3p, hsa-miR-150-5p, hsa-let-7i-3p, hsa-miR-377-3p, hsa-miR-153-3p, hsa-miR-1290, hsa-miR-410-3p | 97.78 | 96.95 | 70.00 |
| 1526 | hsa-miR-455-3p, hsa-miR-7-1-3p, hsa-miR-382-5p, hsa-miR-197-3p, hsa-miR-222-3p, hsa-miR-377-3p, hsa-miR-582-5p, hsa-miR-410-3p | 97.78 | 98.75 | 100.00 |
| 1527 | hsa-miR-484, hsa-miR-30e-3p, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-153-3p, hsa-miR-590-3p, hsa-miR-1290, hsa-miR-410-3p, hsa-let-7g-3p | 97.78 | 97.57 | 70.00 |
| 1528 | hsa-miR-484, hsa-miR-545-5p, hsa-miR-590-5p, hsa-let-7d-3p, hsa-miR-598-3p, age, hsa-miR-382-5p, hsa-miR-126-3p, hsa-miR-150-5p, hsa-miR-377-3p, hsa-miR-582-5p | 97.78 | 99.38 | 90.00 |
| 1529 | hsa-miR-484, hsa-miR-376c-3p, hsa-miR-545-5p, hsa-miR-6803-3p, hsa-miR-590-5p, hsa-miR-7-1-3p, age, hsa-miR-197-3p, hsa-miR-126-3p, hsa-miR-150-5p, hsa-let-7i-3p, hsa-miR-377-3p, hsa-miR-153-3p, hsa-miR-590-3p, hsa-miR-1290 | 97.78 | 98.82 | 70.00 |
| 1530 | hsa-miR-484, hsa-miR-30e-3p, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-590-3p, hsa-miR-410-3p, hsa-let-7g-3p | 97.78 | 98.13 | 70.00 |
| 1531 | hsa-miR-484, hsa-miR-30e-3p, hsa-miR-376c-3p, hsa-miR-6803-3p, hsa-miR-590-5p, hsa-miR-598-3p, hsa-miR-671-5p, age, hsa-miR-197-3p, hsa-miR-150-5p, hsa-let-7i-3p, hsa-miR-153-3p, hsa-miR-590-3p, hsa-miR-1290, hsa-miR-410-3p, hsa-let-7g-3p | 97.78 | 97.02 | 70.00 |
| 1532 | hsa-miR-30e-3p, hsa-miR-376c-3p, hsa-miR-590-5p, hsa-let-7d-3p, hsa-miR-7-1-3p, age, hsa-miR-382-5p, hsa-miR-342-3p, hsa-miR-377-3p, hsa-miR-590-3p, hsa-miR-582-5p, hsa-miR-410-3p | 97.78 | 98.13 | 90.00 |
| 1533 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-1290, hsa-let-7g-3p | 97.78 | 95.77 | 70.00 |
| 1534 | hsa-miR-376c-3p, hsa-miR-6803-3p, hsa-miR-455-3p, hsa-let-7d-3p, age, hsa-miR-382-5p, hsa-miR-126-3p, hsa-miR-222-3p, hsa-miR-377-3p, hsa-miR-153-3p, hsa-miR-590-3p, hsa-miR-582-5p, hsa-miR-1290, hsa-miR-410-3p | 97.78 | 100.00 | 80.00 |
| 1535 | hsa-miR-484, hsa-miR-376c-3p, hsa-miR-545-5p, hsa-miR-6803-3p, hsa-miR-590-5p, hsa-miR-7-1-3p, age, hsa-miR-342-3p, hsa-miR-197-3p, hsa-miR-126-3p, hsa-miR-150-5p, hsa-let-7i-3p, hsa-miR-377-3p, hsa-miR-153-3p, hsa-miR-590-3p, hsa-miR-1290, hsa-miR-410-3p | 97.78 | 98.82 | 70.00 |
| 1536 | hsa-miR-376c-3p, hsa-miR-6803-3p, hsa-miR-455-3p, hsa-let-7d-3p, age, hsa-miR-382-5p, hsa-miR-342-3p, hsa-miR-126-3p, hsa-miR-222-3p, hsa-miR-377-3p, hsa-miR-153-3p, hsa-miR-582-5p, hsa-miR-1290, hsa-miR-410-3p | 97.78 | 96.95 | 70.00 |
| 1537 | hsa-miR-484, hsa-miR-30e-3p, hsa-miR-590-5p, hsa-let-7d-3p, hsa-miR-7-1-3p, hsa-miR-598-3p, hsa-miR-671-5p, age, hsa-miR-382-5p, hsa-miR-126-3p, hsa-miR-150-5p, hsa-let-7i-3p, hsa-miR-377-3p, hsa-miR-410-3p | 97.78 | 98.75 | 90.00 |
| 1538 | hsa-miR-484, hsa-miR-376c-3p, hsa-miR-545-5p, hsa-miR-6803-3p, hsa-miR-455-3p, hsa-let-7d-3p, hsa-miR-598-3p, age, hsa-miR-532-5p, hsa-miR-150-5p, hsa-let-7i-3p, hsa-miR-222-3p, hsa-miR-377-3p, hsa-miR-590-3p | 97.78 | 93.97 | 70.00 |
| 1539 | hsa-miR-484, hsa-miR-30e-3p, hsa-miR-590-5p, hsa-let-7d-3p, hsa-miR-7-1-3p, hsa-miR-598-3p, hsa-miR-671-5p, age, hsa-miR-532-5p, hsa-miR-126-3p, hsa-miR-150-5p, hsa-let-7i-3p, hsa-miR-590-3p, hsa-miR-410-3p | 97.78 | 98.75 | 90.00 |
| 1540 | hsa-miR-484, hsa-miR-6803-3p, hsa-miR-590-5p, hsa-miR-598-3p, hsa-miR-671-5p, hsa-miR-342-3p, hsa-miR-197-3p, hsa-miR-150-5p, hsa-let-7i-3p, hsa-miR-377-3p, hsa-miR-153-3p, hsa-miR-590-3p, hsa-miR-1290 | 97.78 | 100.00 | 80.00 |
| 1541 | hsa-miR-484, hsa-miR-590-5p, hsa-let-7d-3p, hsa-miR-7-1-3p, hsa-miR-598-3p, hsa-miR-671-5p, hsa-miR-342-3p, hsa-miR-126-3p, hsa-miR-150-5p, hsa-let-7i-3p, hsa-miR-377-3p | 97.78 | 100.00 | 100.00 |
| 1542 | hsa-miR-484, hsa-miR-30e-3p, hsa-miR-545-5p, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-377-3p, hsa-miR-590-3p | 97.78 | 98.75 | 90.00 |
| 1543 | hsa-miR-484, hsa-miR-590-5p, hsa-miR-7-1-3p, hsa-miR-598-3p, age, hsa-miR-382-5p, hsa-miR-197-3p, hsa-miR-150-5p, hsa-miR-222-3p, hsa-miR-377-3p, hsa-miR-590-3p | 97.78 | 99.38 | 70.00 |
| 1544 | hsa-miR-484, hsa-miR-590-5p, hsa-let-7d-3p, hsa-miR-7-1-3p, hsa-miR-598-3p, hsa-miR-671-5p, hsa-miR-342-3p, hsa-miR-126-3p, hsa-miR-150-5p, hsa-let-7i-3p, hsa-miR-377-3p, hsa-miR-410-3p | 97.78 | 100.00 | 100.00 |
| 1545 | hsa-miR-484, hsa-miR-30e-3p, hsa-miR-590-5p, hsa-let-7d-3p, hsa-miR-7-1-3p, hsa-miR-598-3p, hsa-miR-671-5p, age, hsa-miR-342-3p, hsa-miR-150-5p, hsa-miR-222-3p, hsa-miR-590-3p, hsa-let-7g-3p | 97.78 | 96.88 | 70.00 |
| 1546 | hsa-miR-484, hsa-miR-545-5p, hsa-miR-590-5p, hsa-let-7d-3p, hsa-miR-7-1-3p, hsa-miR-598-3p, hsa-miR-671-5p, hsa-miR-342-3p, hsa-miR-150-5p, hsa-let-7i-3p, hsa-miR-377-3p, hsa-miR-590-3p | 97.78 | 97.57 | 70.00 |
| 1547 | hsa-miR-484, hsa-miR-30e-3p, hsa-miR-376c-3p, hsa-miR-6803-3p, hsa-miR-590-5p, hsa-miR-598-3p, age, hsa-miR-382-5p, hsa-miR-342-3p, hsa-miR-197-3p, hsa-miR-150-5p, hsa-let-7i-3p, hsa-miR-153-3p, hsa-miR-590-3p, hsa-miR-1290, hsa-miR-410-3p | 97.78 | 99.38 | 70.00 |
| 1548 | hsa-miR-484, hsa-miR-6803-3p, hsa-miR-590-5p, hsa-let-7d-3p, hsa-miR-598-3p, hsa-miR-671-5p, hsa-miR-126-3p, hsa-miR-150-5p, hsa-let-7i-3p, hsa-miR-590-3p, hsa-miR-410-3p | 97.78 | 98.75 | 70.00 |
| 1549 | hsa-miR-545-5p, hsa-miR-6803-3p, hsa-miR-455-3p, hsa-let-7d-3p, hsa-miR-671-5p, hsa-miR-342-3p, hsa-let-7i-3p, hsa-miR-377-3p, hsa-miR-153-3p, hsa-miR-590-3p, hsa-miR-582-5p, hsa-miR-1290 | 97.78 | 98.75 | 90.00 |
| 1550 | hsa-miR-484, hsa-miR-7-1-3p, hsa-miR-598-3p, age, hsa-miR-382-5p, hsa-miR-197-3p, hsa-miR-222-3p, hsa-miR-377-3p, hsa-miR-590-3p, hsa-miR-410-3p | 97.78 | 98.82 | 60.00 |
| 1551 | hsa-miR-30e-3p, hsa-miR-376c-3p, hsa-miR-545-5p, hsa-miR-6803-3p, hsa-miR-455-3p, age, hsa-miR-342-3p, hsa-miR-197-3p, hsa-miR-222-3p, hsa-miR-153-3p, hsa-miR-590-3p, hsa-miR-582-5p, hsa-miR-1290 | 97.78 | 99.38 | 70.00 |
| 1552 | hsa-miR-376c-3p, hsa-miR-6803-3p, hsa-miR-455-3p, hsa-let-7d-3p, hsa-miR-382-5p, hsa-miR-342-3p, hsa-miR-126-3p, hsa-let-7i-3p, hsa-miR-377-3p, hsa-miR-153-3p, hsa-miR-590-3p, hsa-miR-582-5p, hsa-miR-1290, hsa-let-7g-3p | 97.78 | 91.69 | 70.00 |
| 1553 | hsa-miR-484, hsa-miR-30e-3p, hsa-miR-376c-3p, hsa-miR-6803-3p, hsa-miR-590-5p, hsa-miR-671-5p, age, hsa-miR-342-3p, hsa-miR-532-5p, hsa-miR-197-3p, hsa-miR-150-5p, hsa-let-7i-3p, hsa-miR-377-3p, hsa-miR-153-3p, hsa-miR-590-3p, hsa-miR-1290, hsa-miR-410-3p, hsa-let-7g-3p | 97.78 | 95.29 | 70.00 |
| 1554 | hsa-miR-484, hsa-miR-30e-3p, hsa-miR-376c-3p, hsa-miR-6803-3p, hsa-let-7d-3p, hsa-miR-598-3p, age, hsa-miR-382-5p, hsa-miR-342-3p, hsa-miR-126-3p, hsa-miR-222-3p, hsa-miR-153-3p, hsa-miR-590-3p, hsa-miR-1290, hsa-let-7g-3p | 97.78 | 84.26 | 70.00 |
| 1555 | hsa-miR-484, hsa-miR-30e-3p, hsa-miR-376c-3p, hsa-miR-6803-3p, hsa-miR-590-5p, hsa-let-7d-3p, hsa-miR-671-5p, age, hsa-miR-342-3p, hsa-miR-126-3p, hsa-miR-150-5p, hsa-let-7i-3p, hsa-miR-153-3p, hsa-miR-590-3p, hsa-miR-1290, hsa-miR-410-3p | 97.78 | 99.38 | 70.00 |
| 1556 | hsa-miR-376c-3p, hsa-miR-6803-3p, hsa-miR-455-3p, hsa-let-7d-3p, hsa-miR-671-5p, age, hsa-miR-382-5p, hsa-miR-342-3p, hsa-miR-532-5p, hsa-miR-126-3p, hsa-let-7i-3p, hsa-miR-222-3p, hsa-miR-377-3p, hsa-miR-153-3p, hsa-miR-590-3p, hsa-miR-410-3p | 97.78 | 98.82 | 70.00 |
| 1557 | hsa-miR-484, hsa-miR-376c-3p, hsa-miR-545-5p, hsa-miR-590-5p, hsa-let-7d-3p, hsa-miR-7-1-3p, hsa-miR-671-5p, age, hsa-miR-126-3p, hsa-miR-150-5p, hsa-let-7i-3p, hsa-miR-377-3p, hsa-miR-153-3p, hsa-miR-590-3p, hsa-miR-1290, hsa-let-7g-3p | 97.78 | 95.29 | 80.00 |
| 1558 | hsa-miR-484, hsa-miR-30e-3p, hsa-miR-376c-3p, hsa-miR-6803-3p, hsa-miR-590-5p, hsa-miR-598-3p, age, hsa-miR-382-5p, hsa-miR-342-3p, hsa-miR-197-3p, hsa-miR-126-3p, hsa-miR-150-5p, hsa-miR-222-3p, hsa-miR-153-3p, hsa-miR-590-3p, hsa-miR-1290, hsa-miR-410-3p, hsa-let-7g-3p | 97.78 | 91.69 | 70.00 |
| 1559 | hsa-miR-484, hsa-miR-30e-3p, hsa-miR-590-5p, hsa-miR-7-1-3p, hsa-miR-598-3p, hsa-miR-671-5p, age, hsa-miR-342-3p, hsa-miR-532-5p, hsa-miR-197-3p, hsa-miR-150-5p, hsa-let-7i-3p, hsa-miR-590-3p, hsa-miR-410-3p, hsa-let-7g-3p | 97.78 | 99.38 | 70.00 |
| 1560 | hsa-miR-376c-3p, hsa-miR-6803-3p, hsa-miR-455-3p, hsa-let-7d-3p, hsa-miR-382-5p, hsa-miR-342-3p, hsa-miR-126-3p, hsa-miR-222-3p, hsa-miR-377-3p, hsa-miR-153-3p, hsa-miR-590-3p, hsa-miR-582-5p, hsa-miR-1290, hsa-miR-410-3p | 97.78 | 97.57 | 70.00 |
| 1561 | hsa-miR-484, hsa-miR-30e-3p, hsa-miR-376c-3p, hsa-miR-6803-3p, hsa-miR-590-5p, hsa-let-7d-3p, hsa-miR-671-5p, age, hsa-miR-382-5p, hsa-miR-342-3p, hsa-miR-126-3p, hsa-miR-222-3p, hsa-miR-377-3p, hsa-miR-153-3p, hsa-miR-590-3p, hsa-miR-410-3p, hsa-let-7g-3p | 97.78 | 97.57 | 70.00 |
| 1562 | hsa-miR-484, hsa-miR-30e-3p, hsa-miR-545-5p, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-590-3p, hsa-miR-410-3p, hsa-let-7g-3p | 97.78 | 98.75 | 70.00 |
| 1563 | hsa-miR-376c-3p, hsa-miR-545-5p, hsa-miR-6803-3p, hsa-miR-455-3p, hsa-miR-671-5p, age, hsa-miR-342-3p, hsa-miR-197-3p, hsa-let-7i-3p, hsa-miR-377-3p, hsa-miR-153-3p, hsa-miR-582-5p, hsa-miR-1290, hsa-miR-410-3p | 97.78 | 98.82 | 90.00 |
| 1564 | hsa-miR-484, hsa-miR-590-5p, hsa-let-7d-3p, hsa-miR-7-1-3p, hsa-miR-598-3p, age, hsa-miR-382-5p, hsa-miR-126-3p, hsa-miR-150-5p, hsa-miR-222-3p, hsa-miR-377-3p, hsa-miR-590-3p, hsa-miR-410-3p | 97.78 | 98.75 | 90.00 |
| 1565 | hsa-miR-30e-3p, hsa-miR-376c-3p, hsa-miR-455-3p, hsa-let-7d-3p, hsa-miR-7-1-3p, hsa-miR-671-5p, age, hsa-miR-126-3p, hsa-let-7i-3p, hsa-miR-153-3p, hsa-miR-590-3p, hsa-miR-582-5p, hsa-miR-1290, hsa-miR-410-3p, hsa-let-7g-3p | 97.78 | 97.65 | 70.00 |
| 1566 | hsa-miR-484, hsa-miR-30e-3p, hsa-miR-376c-3p, hsa-miR-6803-3p, hsa-miR-590-5p, hsa-let-7d-3p, hsa-miR-598-3p, hsa-miR-671-5p, age, hsa-miR-126-3p, hsa-miR-150-5p, hsa-let-7i-3p, hsa-miR-153-3p, hsa-miR-590-3p, hsa-miR-1290, hsa-miR-410-3p, hsa-let-7g-3p | 97.78 | 99.38 | 70.00 |
| 1567 | hsa-miR-484, hsa-miR-30e-3p, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-590-3p, hsa-let-7g-3p | 97.78 | 98.13 | 70.00 |
| 1568 | hsa-miR-455-3p, hsa-miR-7-1-3p, hsa-miR-671-5p, age, hsa-miR-342-3p, hsa-miR-197-3p, hsa-let-7i-3p, hsa-miR-377-3p, hsa-miR-590-3p, hsa-miR-582-5p, hsa-miR-410-3p | 97.78 | 98.13 | 90.00 |
| 1569 | hsa-miR-484, hsa-miR-376c-3p, hsa-miR-545-5p, hsa-miR-6803-3p, hsa-let-7d-3p, hsa-miR-598-3p, hsa-miR-671-5p, age, hsa-miR-342-3p, hsa-miR-532-5p, hsa-miR-126-3p, hsa-let-7i-3p, hsa-miR-377-3p, hsa-miR-153-3p, hsa-miR-590-3p, hsa-miR-1290, hsa-miR-410-3p | 97.78 | 100.00 | 70.00 |
| 1570 | hsa-miR-484, hsa-miR-30e-3p, hsa-miR-376c-3p, hsa-miR-6803-3p, hsa-miR-590-5p, hsa-let-7d-3p, hsa-miR-598-3p, age, hsa-miR-382-5p, hsa-miR-342-3p, hsa-miR-126-3p, hsa-miR-150-5p, hsa-miR-222-3p, hsa-miR-377-3p, hsa-miR-153-3p, hsa-miR-590-3p, hsa-miR-1290, hsa-miR-410-3p | 97.78 | 94.45 | 70.00 |
| 1571 | hsa-miR-376c-3p, hsa-miR-6803-3p, hsa-miR-455-3p, hsa-let-7d-3p, age, hsa-miR-382-5p, hsa-miR-342-3p, hsa-miR-126-3p, hsa-miR-222-3p, hsa-miR-377-3p, hsa-miR-153-3p, hsa-miR-590-3p, hsa-miR-582-5p, hsa-miR-1290, hsa-miR-410-3p | 97.78 | 98.75 | 70.00 |
| 1572 | hsa-miR-376c-3p, hsa-miR-6803-3p, hsa-miR-455-3p, hsa-let-7d-3p, hsa-miR-671-5p, age, hsa-miR-342-3p, hsa-miR-126-3p, hsa-let-7i-3p, hsa-miR-153-3p, hsa-miR-590-3p, hsa-miR-582-5p, hsa-miR-1290, hsa-miR-410-3p, hsa-let-7g-3p | 97.78 | 89.96 | 70.00 |
| 1573 | hsa-miR-376c-3p, hsa-miR-545-5p, hsa-miR-6803-3p, hsa-miR-455-3p, hsa-let-7d-3p, hsa-miR-671-5p, age, hsa-miR-342-3p, hsa-miR-126-3p, hsa-let-7i-3p, hsa-miR-377-3p, hsa-miR-153-3p, hsa-miR-590-3p, hsa-miR-582-5p, hsa-miR-1290 | 97.78 | 99.38 | 90.00 |
| 1574 | hsa-miR-484, hsa-miR-30e-3p, hsa-miR-376c-3p, hsa-miR-545-5p, hsa-miR-6803-3p, hsa-miR-590-5p, hsa-miR-598-3p, hsa-miR-671-5p, age, hsa-miR-342-3p, hsa-miR-197-3p, hsa-miR-150-5p, hsa-let-7i-3p, hsa-miR-153-3p, hsa-miR-590-3p, hsa-miR-1290, hsa-let-7g-3p | 97.78 | 98.75 | 70.00 |
| 1575 | hsa-miR-484, hsa-miR-30e-3p, hsa-miR-376c-3p, hsa-miR-545-5p, hsa-miR-6803-3p, hsa-miR-590-5p, hsa-let-7d-3p, hsa-miR-598-3p, age, hsa-miR-382-5p, hsa-miR-532-5p, hsa-miR-126-3p, hsa-miR-150-5p, hsa-miR-222-3p, hsa-miR-153-3p, hsa-miR-590-3p, hsa-miR-1290, hsa-let-7g-3p | 97.78 | 90.92 | 70.00 |
| 1576 | hsa-miR-484, hsa-miR-30e-3p, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-590-3p | 97.78 | 98.13 | 90.00 |
| 1577 | hsa-miR-484, hsa-miR-30e-3p, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-590-3p, hsa-miR-410-3p | 97.78 | 98.75 | 90.00 |
| 1578 | hsa-miR-30e-3p, hsa-miR-376c-3p, hsa-miR-6803-3p, hsa-miR-455-3p, hsa-let-7d-3p, hsa-miR-7-1-3p, hsa-miR-671-5p, age, hsa-miR-342-3p, hsa-miR-532-5p, hsa-miR-150-5p, hsa-miR-377-3p, hsa-miR-153-3p, hsa-miR-410-3p, hsa-let-7g-3p | 97.78 | 90.37 | 70.00 |
| 1579 | hsa-miR-484, hsa-miR-30e-3p, hsa-miR-376c-3p, hsa-miR-6803-3p, hsa-miR-590-5p, hsa-miR-598-3p, hsa-miR-671-5p, age, hsa-miR-342-3p, hsa-miR-532-5p, hsa-miR-197-3p, hsa-miR-150-5p, hsa-let-7i-3p, hsa-miR-153-3p, hsa-miR-590-3p, hsa-miR-1290, hsa-miR-410-3p | 97.78 | 98.2 | 70.00 |
| 1580 | hsa-miR-484, hsa-miR-30e-3p, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-153-3p, hsa-miR-590-3p, hsa-miR-1290, hsa-miR-410-3p, hsa-let-7g-3p | 97.78 | 97.57 | 70.00 |
| 1581 | hsa-miR-376c-3p, hsa-miR-545-5p, hsa-miR-455-3p, hsa-miR-7-1-3p, age, hsa-miR-197-3p, hsa-miR-222-3p, hsa-miR-377-3p, hsa-miR-153-3p, hsa-miR-590-3p, hsa-miR-582-5p, hsa-miR-1290, hsa-let-7g-3p | 97.78 | 94.12 | 80.00 |
| 1582 | hsa-miR-455-3p, hsa-miR-7-1-3p, hsa-miR-671-5p, age, hsa-miR-342-3p, hsa-miR-532-5p, hsa-miR-197-3p, hsa-let-7i-3p, hsa-miR-377-3p, hsa-miR-590-3p, hsa-miR-582-5p, hsa-miR-410-3p, hsa-let-7g-3p | 97.78 | 99.38 | 70.00 |
| 1583 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-1290, hsa-miR-410-3p, hsa-let-7g-3p | 97.78 | 98.2 | 70.00 |
| 1584 | hsa-miR-30e-3p, hsa-miR-376c-3p, hsa-miR-545-5p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-let-7i-3p, hsa-miR-377-3p, hsa-miR-153-3p, hsa-miR-590-3p, hsa-miR-1290, hsa-miR-410-3p | 97.78 | 98.75 | 70.00 |
| 1585 | hsa-miR-484, hsa-miR-376c-3p, hsa-miR-6803-3p, hsa-miR-590-5p, hsa-let-7d-3p, hsa-miR-598-3p, age, hsa-miR-342-3p, hsa-miR-126-3p, hsa-miR-150-5p, hsa-let-7i-3p, hsa-miR-377-3p, hsa-miR-153-3p, hsa-miR-590-3p, hsa-miR-1290, hsa-miR-410-3p | 97.78 | 98.75 | 70.00 |
| 1586 | hsa-miR-484, hsa-miR-376c-3p, hsa-miR-545-5p, hsa-miR-6803-3p, hsa-let-7d-3p, hsa-miR-598-3p, hsa-miR-671-5p, hsa-miR-342-3p, hsa-miR-126-3p, hsa-let-7i-3p, hsa-miR-377-3p, hsa-miR-153-3p, hsa-miR-590-3p, hsa-miR-1290 | 97.78 | 99.38 | 70.00 |
| 1587 | hsa-miR-484, hsa-miR-30e-3p, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-153-3p, hsa-miR-590-3p, hsa-miR-1290, hsa-miR-410-3p | 97.78 | 98.13 | 70.00 |
| 1588 | hsa-miR-376c-3p, hsa-miR-6803-3p, hsa-miR-455-3p, hsa-let-7d-3p, age, hsa-miR-342-3p, hsa-miR-126-3p, hsa-miR-222-3p, hsa-miR-377-3p, hsa-miR-153-3p, hsa-miR-590-3p, hsa-miR-582-5p, hsa-miR-1290, hsa-miR-410-3p | 97.78 | 99.38 | 70.00 |
| 1589 | hsa-miR-376c-3p, hsa-miR-6803-3p, hsa-miR-455-3p, hsa-miR-598-3p, age, hsa-miR-342-3p, hsa-miR-197-3p, hsa-miR-126-3p, hsa-miR-222-3p, hsa-miR-377-3p, hsa-miR-153-3p, hsa-miR-590-3p, hsa-miR-582-5p, hsa-miR-1290, hsa-miR-410-3p | 97.78 | 100.00 | 90.00 |
| 1590 | hsa-miR-484, hsa-miR-376c-3p, hsa-miR-6803-3p, hsa-miR-590-5p, hsa-miR-455-3p, hsa-let-7d-3p, hsa-miR-598-3p, hsa-miR-671-5p, age, hsa-miR-342-3p, hsa-miR-532-5p, hsa-miR-126-3p, hsa-miR-150-5p, hsa-let-7i-3p, hsa-miR-222-3p, hsa-miR-377-3p, hsa-miR-153-3p, hsa-miR-590-3p, hsa-miR-1290, hsa-miR-410-3p, hsa-let-7g-3p | 97.78 | 93.49 | 70.00 |
| 1591 | hsa-miR-484, hsa-miR-30e-3p, hsa-miR-376c-3p, hsa-miR-6803-3p, hsa-let-7d-3p, hsa-miR-598-3p, hsa-miR-671-5p, age, hsa-miR-382-5p, hsa-miR-126-3p, hsa-miR-150-5p, hsa-let-7i-3p, hsa-miR-153-3p, hsa-miR-590-3p, hsa-miR-1290, hsa-miR-410-3p, hsa-let-7g-3p | 97.78 | 96.95 | 70.00 |
| 1592 | hsa-miR-30e-3p, hsa-miR-376c-3p, hsa-miR-6803-3p, hsa-miR-455-3p, age, hsa-miR-382-5p, hsa-miR-342-3p, hsa-miR-197-3p, hsa-miR-222-3p, hsa-miR-153-3p, hsa-miR-590-3p, hsa-miR-582-5p, hsa-miR-1290, hsa-miR-410-3p, hsa-let-7g-3p | 97.78 | 92.32 | 70.00 |
| 1593 | hsa-miR-484, hsa-miR-30e-3p, hsa-miR-376c-3p, hsa-miR-6803-3p, hsa-miR-590-5p, hsa-miR-598-3p, age, hsa-miR-382-5p, hsa-miR-342-3p, hsa-miR-532-5p, hsa-miR-197-3p, hsa-miR-150-5p, hsa-miR-222-3p, hsa-miR-153-3p, hsa-miR-590-3p, hsa-miR-1290, hsa-miR-410-3p, hsa-let-7g-3p | 97.78 | 92.32 | 70.00 |
| 1594 | hsa-miR-484, hsa-miR-6803-3p, hsa-miR-590-5p, hsa-let-7d-3p, hsa-miR-598-3p, hsa-miR-671-5p, hsa-miR-342-3p, hsa-miR-126-3p, hsa-miR-150-5p, hsa-let-7i-3p, hsa-miR-377-3p, hsa-miR-153-3p, hsa-miR-410-3p | 97.78 | 95.29 | 80.00 |
| 1595 | hsa-miR-484, hsa-miR-590-5p, hsa-let-7d-3p, hsa-miR-7-1-3p, hsa-miR-598-3p, hsa-miR-671-5p, age, hsa-miR-342-3p, hsa-miR-532-5p, hsa-miR-150-5p, hsa-let-7i-3p, hsa-miR-377-3p, hsa-miR-590-3p | 96.78 | 99.38 | 70.00 |
| 1596 | hsa-miR-484, hsa-miR-590-5p, hsa-let-7d-3p, hsa-miR-7-1-3p, hsa-miR-598-3p, hsa-miR-671-5p, age, hsa-miR-382-5p, hsa-miR-126-3p, hsa-miR-150-5p, hsa-let-7i-3p, hsa-miR-377-3p, hsa-miR-590-3p, hsa-miR-410-3p | 96.78 | 99.41 | 90.00 |
| 1597 | hsa-miR-484, hsa-miR-590-5p, hsa-miR-7-1-3p, hsa-miR-598-3p, age, hsa-miR-382-5p, hsa-miR-342-3p, hsa-miR-532-5p, hsa-miR-197-3p, hsa-miR-150-5p, hsa-miR-222-3p, hsa-miR-377-3p, hsa-miR-590-3p, hsa-miR-410-3p | 96.78 | 99.38 | 70.00 |
| 1598 | hsa-miR-484, hsa-miR-590-5p, hsa-let-7d-3p, hsa-miR-7-1-3p, hsa-miR-598-3p, hsa-miR-671-5p, age, hsa-miR-532-5p, hsa-miR-126-3p, hsa-miR-150-5p, hsa-let-7i-3p, hsa-miR-377-3p, hsa-miR-590-3p, hsa-miR-410-3p | 96.78 | 100.00 | 70.00 |
| 1599 | hsa-miR-484, hsa-let-7d-3p, hsa-miR-7-1-3p, hsa-miR-598-3p, hsa-miR-382-5p, hsa-miR-126-3p, hsa-miR-222-3p, hsa-miR-377-3p, hsa-miR-410-3p | 96.73 | 97.57 | 100.00 |
| 1600 | hsa-miR-484, hsa-miR-545-5p, hsa-miR-590-5p, hsa-let-7d-3p, hsa-miR-7-1-3p, hsa-miR-598-3p, hsa-miR-671-5p, hsa-miR-126-3p, hsa-miR-150-5p, hsa-let-7i-3p, hsa-miR-377-3p, hsa-miR-410-3p | 96.73 | 100.00 | 80.00 |
| 1601 | hsa-miR-484, hsa-miR-376c-3p, hsa-miR-6803-3p, hsa-miR-590-5p, hsa-miR-598-3p, age, hsa-miR-382-5p, hsa-miR-342-3p, hsa-miR-197-3p, hsa-miR-150-5p, hsa-miR-222-3p, hsa-miR-377-3p, hsa-miR-153-3p, hsa-miR-590-3p, hsa-miR-1290, hsa-miR-410-3p | 96.73 | 97.02 | 60.00 |
| 1602 | hsa-miR-484, hsa-miR-545-5p, hsa-let-7d-3p, hsa-miR-7-1-3p, hsa-miR-598-3p, hsa-miR-126-3p, hsa-miR-222-3p, hsa-miR-377-3p, hsa-miR-410-3p | 96.73 | 100.00 | 90.00 |
| 1603 | hsa-miR-484, hsa-miR-376c-3p, hsa-miR-545-5p, hsa-miR-6803-3p, hsa-miR-590-5p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-153-3p, hsa-miR-1290 | 96.73 | 98.2 | 60.00 |
| 1604 | hsa-miR-484, hsa-miR-590-5p, hsa-miR-7-1-3p, hsa-miR-598-3p, age, hsa-miR-382-5p, hsa-miR-197-3p, hsa-miR-126-3p, hsa-miR-150-5p, hsa-let-7i-3p, hsa-miR-377-3p, hsa-miR-410-3p | 96.73 | 99.38 | 100.00 |
| 1605 | hsa-miR-484, hsa-miR-6803-3p, hsa-miR-590-5p, hsa-let-7d-3p, hsa-miR-598-3p, hsa-miR-671-5p, hsa-miR-342-3p, hsa-miR-150-5p, hsa-let-7i-3p, hsa-miR-377-3p, hsa-miR-153-3p, hsa-miR-1290, hsa-miR-410-3p | 96.73 | 97.5 | 60.00 |
| 1606 | hsa-miR-484, hsa-miR-30e-3p, hsa-miR-376c-3p, hsa-miR-6803-3p, hsa-miR-590-5p, hsa-miR-598-3p, hsa-miR-671-5p, age, hsa-miR-342-3p, hsa-miR-532-5p, hsa-miR-197-3p, hsa-miR-126-3p, hsa-miR-150-5p, hsa-let-7i-3p, hsa-miR-153-3p, hsa-miR-590-3p, hsa-miR-1290, hsa-miR-410-3p | 96.73 | 99.38 | 60.00 |
| 1607 | hsa-miR-484, hsa-miR-6803-3p, hsa-miR-590-5p, hsa-miR-598-3p, hsa-miR-671-5p, age, hsa-miR-342-3p, hsa-miR-532-5p, hsa-miR-197-3p, hsa-miR-126-3p, hsa-miR-150-5p, hsa-let-7i-3p, hsa-miR-377-3p, hsa-miR-153-3p, hsa-miR-590-3p, hsa-miR-1290 | 96.73 | 100.00 | 60.00 |
| 1608 | hsa-miR-484, hsa-miR-30e-3p, hsa-miR-376c-3p, hsa-miR-6803-3p, hsa-miR-590-5p, hsa-let-7d-3p, hsa-miR-598-3p, hsa-miR-671-5p, age, hsa-miR-532-5p, hsa-miR-126-3p, hsa-miR-150-5p, hsa-let-7i-3p, hsa-miR-153-3p, hsa-miR-590-3p, hsa-miR-1290, hsa-let-7g-3p | 96.73 | 97.57 | 60.00 |
| 1609 | hsa-miR-484, hsa-miR-6803-3p, hsa-miR-590-5p, hsa-let-7d-3p, hsa-miR-598-3p, hsa-miR-671-5p, age, hsa-miR-126-3p, hsa-miR-150-5p, hsa-let-7i-3p, hsa-miR-153-3p, hsa-miR-590-3p, hsa-miR-1290, hsa-miR-410-3p | 96.73 | 98.13 | 60.00 |
| 1610 | hsa-miR-455-3p, hsa-miR-7-1-3p, age, hsa-miR-342-3p, hsa-miR-532-5p, hsa-miR-197-3p, hsa-miR-222-3p, hsa-miR-377-3p, hsa-miR-590-3p, hsa-miR-582-5p, hsa-miR-410-3p | 96.73 | 96.99 | 70.00 |
| 1611 | hsa-miR-484, hsa-miR-376c-3p, hsa-miR-6803-3p, hsa-miR-590-5p, hsa-miR-598-3p, age, hsa-miR-382-5p, hsa-miR-342-3p, hsa-miR-197-3p, hsa-miR-126-3p, hsa-miR-150-5p, hsa-miR-222-3p, hsa-miR-377-3p, hsa-miR-153-3p, hsa-miR-590-3p, hsa-miR-1290, hsa-miR-410-3p | 96.73 | 97.02 | 60.00 |
| 1612 | hsa-miR-484, hsa-miR-376c-3p, hsa-miR-545-5p, hsa-miR-6803-3p, hsa-miR-590-5p, hsa-miR-455-3p, hsa-miR-598-3p, hsa-miR-671-5p, age, hsa-miR-342-3p, hsa-miR-532-5p, hsa-miR-197-3p, hsa-miR-126-3p, hsa-miR-150-5p, hsa-let-7i-3p, hsa-miR-377-3p, hsa-miR-153-3p, hsa-miR-590-3p, hsa-miR-1290, hsa-let-7g-3p | 96.73 | 95.29 | 60.00 |
| 1613 | hsa-miR-484, hsa-miR-590-5p, hsa-let-7d-3p, hsa-miR-7-1-3p, hsa-miR-598-3p, hsa-miR-671-5p, hsa-miR-382-5p, hsa-miR-126-3p, hsa-miR-150-5p, hsa-let-7i-3p, hsa-miR-377-3p, hsa-miR-410-3p | 96.73 | 98.79 | 100.00 |
| 1614 | hsa-miR-7-1-3p, hsa-miR-598-3p, hsa-miR-671-5p, hsa-miR-342-3p, hsa-miR-126-3p, hsa-miR-377-3p, hsa-miR-590-3p, hsa-miR-582-5p, hsa-miR-410-3p | 96.73 | 97.54 | 90.00 |
| 1615 | hsa-miR-484, hsa-miR-30e-3p, hsa-miR-6803-3p, hsa-miR-455-3p, hsa-miR-671-5p, hsa-miR-382-5p, hsa-let-7i-3p, hsa-miR-222-3p, hsa-miR-582-5p | 96.73 | 92.32 | 70.00 |
| 1616 | hsa-miR-376c-3p, hsa-miR-6803-3p, hsa-miR-455-3p, hsa-miR-342-3p, hsa-miR-532-5p, hsa-miR-197-3p, hsa-miR-222-3p, hsa-miR-377-3p, hsa-miR-153-3p, hsa-miR-582-5p, hsa-miR-1290, hsa-miR-410-3p | 96.73 | 98.82 | 60.00 |
| 1617 | hsa-miR-545-5p, hsa-miR-7-1-3p, hsa-miR-342-3p, hsa-miR-532-5p, hsa-miR-126-3p, hsa-miR-377-3p, hsa-miR-153-3p, hsa-miR-582-5p, hsa-miR-1290 | 96.73 | 92.87 | 90.00 |
| 1618 | hsa-miR-484, hsa-miR-590-5p, hsa-let-7d-3p, hsa-miR-7-1-3p, hsa-miR-598-3p, hsa-miR-671-5p, age, hsa-miR-382-5p, hsa-miR-126-3p, hsa-miR-150-5p, hsa-let-7i-3p, hsa-miR-377-3p, hsa-miR-410-3p | 96.73 | 99.38 | 90.00 |
| 1619 | hsa-miR-484, hsa-miR-6803-3p, hsa-miR-590-5p, hsa-let-7d-3p, hsa-miR-598-3p, hsa-miR-671-5p, age, hsa-miR-342-3p, hsa-miR-532-5p, hsa-miR-150-5p, hsa-let-7i-3p, hsa-miR-153-3p, hsa-miR-590-3p, hsa-miR-1290, hsa-miR-410-3p, hsa-let-7g-3p | 96.73 | 95.81 | 70.00 |
| 1620 | hsa-miR-484, hsa-miR-6803-3p, hsa-miR-590-5p, hsa-let-7d-3p, hsa-miR-598-3p, age, hsa-miR-342-3p, hsa-miR-150-5p, hsa-miR-222-3p, hsa-miR-377-3p, hsa-miR-590-3p, hsa-miR-410-3p | 96.73 | 91.91 | 80.00 |
| 1621 | hsa-miR-484, hsa-miR-30e-3p, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-153-3p, hsa-miR-1290, hsa-miR-410-3p, hsa-let-7g-3p | 96.73 | 95.7 | 60.00 |
| 1622 | hsa-miR-484, hsa-miR-30e-3p, hsa-miR-376c-3p, hsa-miR-590-5p, hsa-let-7d-3p, hsa-miR-598-3p, hsa-miR-671-5p, hsa-miR-382-5p, hsa-miR-342-3p, hsa-miR-532-5p, hsa-miR-222-3p, hsa-miR-377-3p, hsa-miR-153-3p, hsa-miR-590-3p, hsa-miR-410-3p, hsa-let-7g-3p | 96.73 | 99.38 | 60.00 |
| 1623 | hsa-miR-484, hsa-miR-376c-3p, hsa-miR-6803-3p, hsa-miR-598-3p, age, hsa-miR-382-5p, hsa-miR-342-3p, hsa-miR-197-3p, hsa-miR-126-3p, hsa-miR-222-3p, hsa-miR-377-3p, hsa-miR-153-3p, hsa-miR-590-3p, hsa-miR-1290, hsa-miR-410-3p | 96.67 | 95.85 | 50.00 |
| 1624 | hsa-miR-376c-3p, hsa-miR-6803-3p, hsa-miR-455-3p, age, hsa-miR-382-5p, hsa-miR-342-3p, hsa-miR-197-3p, hsa-miR-222-3p, hsa-miR-153-3p, hsa-miR-590-3p, hsa-miR-582-5p, hsa-miR-1290, hsa-miR-410-3p, hsa-let-7g-3p | 96.67 | 92.94 | 70.00 |
| 1625 | hsa-miR-484, hsa-miR-376c-3p, hsa-miR-545-5p, hsa-miR-6803-3p, hsa-miR-590-5p, hsa-let-7d-3p, hsa-miR-598-3p, hsa-miR-671-5p, hsa-miR-342-3p, hsa-miR-126-3p, hsa-miR-150-5p, hsa-let-7i-3p, hsa-miR-377-3p, hsa-miR-153-3p, hsa-miR-1290 | 96.67 | 98.13 | 70.00 |
| 1626 | hsa-miR-484, hsa-miR-376c-3p, hsa-miR-6803-3p, hsa-let-7d-3p, hsa-miR-598-3p, age, hsa-miR-382-5p, hsa-miR-342-3p, hsa-miR-126-3p, hsa-miR-222-3p, hsa-miR-377-3p, hsa-miR-153-3p, hsa-miR-590-3p, hsa-miR-1290, hsa-miR-410-3p | 96.67 | 94.52 | 50.00 |
| 1627 | hsa-miR-376c-3p, hsa-miR-545-5p, hsa-miR-6803-3p, hsa-miR-455-3p, hsa-let-7d-3p, hsa-miR-671-5p, hsa-miR-382-5p, hsa-miR-532-5p, hsa-miR-126-3p, hsa-miR-222-3p, hsa-miR-377-3p, hsa-miR-153-3p, hsa-miR-590-3p, hsa-miR-582-5p, hsa-miR-1290, hsa-miR-410-3p | 96.67 | 98.2 | 80.00 |
| 1628 | hsa-miR-484, hsa-miR-376c-3p, hsa-miR-6803-3p, hsa-miR-590-5p, hsa-let-7d-3p, hsa-miR-598-3p, hsa-miR-671-5p, hsa-miR-342-3p, hsa-miR-126-3p, hsa-miR-150-5p, hsa-let-7i-3p, hsa-miR-377-3p, hsa-miR-153-3p, hsa-miR-1290, hsa-miR-410-3p | 96.67 | 97.57 | 50.00 |
| 1629 | hsa-miR-484, hsa-miR-376c-3p, hsa-miR-6803-3p, hsa-miR-590-5p, hsa-let-7d-3p, hsa-miR-598-3p, hsa-miR-671-5p, hsa-miR-342-3p, hsa-miR-126-3p, hsa-miR-150-5p, hsa-let-7i-3p, hsa-miR-377-3p, hsa-miR-153-3p, hsa-miR-1290 | 96.67 | 97.57 | 50.00 |
| 1630 | hsa-miR-376c-3p, hsa-miR-6803-3p, hsa-miR-455-3p, hsa-miR-382-5p, hsa-miR-342-3p, hsa-miR-532-5p, hsa-miR-197-3p, hsa-miR-222-3p, hsa-miR-153-3p, hsa-miR-582-5p, hsa-miR-1290, hsa-miR-410-3p, hsa-let-7g-3p | 96.67 | 88.79 | 70.00 |
| 1631 | hsa-miR-484, hsa-miR-6803-3p, hsa-miR-455-3p, hsa-let-7d-3p, hsa-miR-598-3p, hsa-miR-671-5p, hsa-miR-532-5p, hsa-miR-126-3p, hsa-miR-222-3p, hsa-miR-377-3p, hsa-miR-590-3p, hsa-miR-582-5p, hsa-miR-410-3p, hsa-let-7g-3p | 96.67 | 97.65 | 70.00 |
| 1632 | hsa-miR-484, hsa-miR-376c-3p, hsa-miR-545-5p, hsa-miR-6803-3p, hsa-miR-590-5p, hsa-miR-455-3p, hsa-let-7d-3p, hsa-miR-7-1-3p, hsa-miR-598-3p, hsa-miR-671-5p, age, hsa-miR-382-5p, hsa-miR-532-5p, hsa-miR-126-3p, hsa-miR-150-5p, hsa-miR-377-3p, hsa-miR-590-3p, hsa-miR-582-5p, hsa-miR-1290, hsa-let-7g-3p | 96.67 | 97.65 | 70.00 |
| 1633 | hsa-miR-484, hsa-miR-376c-3p, hsa-miR-545-5p, hsa-miR-6803-3p, hsa-miR-590-5p, hsa-miR-7-1-3p, age, hsa-miR-342-3p, hsa-miR-197-3p, hsa-miR-126-3p, hsa-miR-150-5p, hsa-let-7i-3p, hsa-miR-153-3p, hsa-miR-590-3p, hsa-miR-1290, hsa-miR-410-3p | 96.67 | 98.82 | 50.00 |
| 1634 | hsa-miR-376c-3p, hsa-miR-6803-3p, hsa-miR-455-3p, hsa-let-7d-3p, hsa-miR-671-5p, hsa-miR-342-3p, hsa-miR-126-3p, hsa-miR-222-3p, hsa-miR-377-3p, hsa-miR-153-3p, hsa-miR-582-5p, hsa-miR-1290, hsa-miR-410-3p | 96.67 | 95.7 | 70.00 |
| 1635 | hsa-miR-484, hsa-miR-376c-3p, hsa-miR-7-1-3p, hsa-miR-598-3p, age, hsa-miR-382-5p, hsa-miR-342-3p, hsa-miR-197-3p, hsa-let-7i-3p, hsa-miR-377-3p, hsa-miR-153-3p, hsa-miR-590-3p, hsa-miR-582-5p, hsa-miR-1290, hsa-miR-410-3p | 96.67 | 96.47 | 40.00 |
| 1636 | hsa-miR-484, hsa-miR-376c-3p, hsa-miR-6803-3p, hsa-miR-590-5p, hsa-let-7d-3p, hsa-miR-598-3p, hsa-miR-671-5p, hsa-miR-342-3p, hsa-miR-150-5p, hsa-miR-377-3p, hsa-miR-153-3p, hsa-miR-582-5p, hsa-miR-1290, hsa-miR-410-3p | 96.67 | 93.42 | 70.00 |
| 1637 | hsa-miR-484, hsa-miR-545-5p, hsa-miR-6803-3p, hsa-let-7d-3p, hsa-miR-7-1-3p, hsa-miR-598-3p, hsa-miR-671-5p, hsa-miR-382-5p, hsa-miR-342-3p, hsa-miR-532-5p, hsa-miR-197-3p, hsa-miR-126-3p, hsa-let-7i-3p, hsa-miR-153-3p, hsa-miR-590-3p, hsa-miR-582-5p, hsa-miR-410-3p, hsa-let-7g-3p | 96.67 | 92.87 | 50.00 |
| 1638 | hsa-miR-484, hsa-miR-376c-3p, hsa-miR-545-5p, hsa-miR-590-5p, hsa-miR-7-1-3p, age, hsa-miR-342-3p, hsa-miR-197-3p, hsa-miR-126-3p, hsa-miR-150-5p, hsa-let-7i-3p, hsa-miR-222-3p, hsa-miR-377-3p, hsa-miR-153-3p, hsa-miR-590-3p, hsa-miR-1290 | 96.67 | 97.65 | 60.00 |
| 1639 | hsa-miR-545-5p, hsa-miR-590-5p, hsa-let-7d-3p, age, hsa-miR-382-5p, hsa-miR-222-3p, hsa-miR-377-3p, hsa-miR-590-3p, hsa-miR-582-5p | 96.67 | 98.75 | 70.00 |
| 1640 | hsa-miR-484, hsa-miR-30e-3p, hsa-miR-376c-3p, hsa-miR-545-5p, hsa-miR-590-5p, hsa-let-7d-3p, hsa-miR-7-1-3p, hsa-miR-598-3p, hsa-miR-671-5p, age, hsa-miR-126-3p, hsa-miR-150-5p, hsa-let-7i-3p, hsa-miR-590-3p, hsa-miR-1290, hsa-let-7g-3p | 96.67 | 98.75 | 50.00 |
| 1641 | hsa-miR-30e-3p, hsa-miR-376c-3p, hsa-miR-545-5p, hsa-miR-6803-3p, hsa-miR-590-5p, hsa-miR-455-3p, hsa-let-7d-3p, hsa-miR-598-3p, hsa-miR-671-5p, hsa-miR-342-3p, hsa-miR-126-3p, hsa-miR-222-3p, hsa-miR-153-3p, hsa-miR-582-5p, hsa-miR-1290, hsa-let-7g-3p | 96.67 | 95.63 | 70.00 |
| 1642 | hsa-miR-484, hsa-miR-30e-3p, hsa-miR-376c-3p, hsa-miR-590-5p, hsa-let-7d-3p, hsa-miR-7-1-3p, hsa-miR-598-3p, hsa-miR-671-5p, hsa-miR-382-5p, hsa-miR-126-3p, hsa-miR-150-5p, hsa-let-7i-3p, hsa-miR-590-3p, hsa-let-7g-3p | 96.67 | 98.13 | 70.00 |
| 1643 | hsa-miR-545-5p, hsa-miR-455-3p, hsa-let-7d-3p, hsa-miR-7-1-3p, hsa-miR-382-5p, hsa-miR-342-3p, hsa-miR-126-3p, hsa-let-7i-3p, hsa-miR-377-3p, hsa-miR-590-3p, hsa-miR-582-5p | 96.67 | 97.57 | 60.00 |
| 1644 | hsa-miR-484, hsa-miR-545-5p, hsa-let-7d-3p, hsa-miR-7-1-3p, hsa-miR-598-3p, age, hsa-miR-382-5p, hsa-miR-126-3p, hsa-miR-222-3p, hsa-miR-377-3p | 96.67 | 99.38 | 80.00 |
| 1645 | hsa-miR-484, hsa-miR-545-5p, hsa-let-7d-3p, hsa-miR-7-1-3p, hsa-miR-598-3p, age, hsa-miR-382-5p, hsa-miR-126-3p, hsa-miR-222-3p, hsa-miR-410-3p | 96.67 | 97.57 | 80.00 |
| 1646 | hsa-miR-484, hsa-miR-6803-3p, hsa-miR-590-5p, hsa-miR-671-5p, age, hsa-miR-197-3p, hsa-miR-150-5p, hsa-let-7i-3p, hsa-miR-377-3p, hsa-miR-590-3p, hsa-miR-1290, hsa-miR-410-3p | 96.67 | 100.00 | 60.00 |
| 1647 | hsa-miR-545-5p, hsa-miR-455-3p, hsa-miR-7-1-3p, hsa-miR-382-5p, hsa-miR-197-3p, hsa-miR-126-3p, hsa-miR-222-3p, hsa-miR-377-3p, hsa-miR-582-5p, hsa-miR-410-3p | 96.67 | 96.4 | 80.00 |
| 1648 | hsa-miR-455-3p, hsa-miR-7-1-3p, age, hsa-miR-382-5p, hsa-miR-197-3p, hsa-miR-222-3p, hsa-miR-377-3p, hsa-miR-582-5p, hsa-miR-410-3p | 96.67 | 97.57 | 100.00 |
| 1649 | hsa-miR-484, hsa-miR-30e-3p, hsa-miR-376c-3p, hsa-miR-590-5p, hsa-let-7d-3p, hsa-miR-7-1-3p, hsa-miR-598-3p, age, hsa-miR-532-5p, hsa-miR-126-3p, hsa-miR-150-5p, hsa-let-7i-3p, hsa-miR-590-3p, hsa-miR-410-3p, hsa-let-7g-3p | 96.67 | 95.77 | 70.00 |
| 1650 | hsa-miR-484, hsa-miR-30e-3p, hsa-miR-376c-3p, hsa-miR-545-5p, hsa-miR-6803-3p, hsa-miR-590-5p, hsa-let-7d-3p, hsa-miR-598-3p, age, hsa-miR-382-5p, hsa-miR-342-3p, hsa-miR-126-3p, hsa-miR-150-5p, hsa-miR-222-3p, hsa-miR-153-3p, hsa-miR-590-3p, hsa-miR-1290, hsa-miR-410-3p, hsa-let-7g-3p | 96.67 | 98.13 | 50.00 |
| 1651 | hsa-miR-484, hsa-miR-30e-3p, hsa-miR-545-5p, hsa-miR-6803-3p, hsa-miR-455-3p, hsa-let-7d-3p, hsa-miR-598-3p, hsa-miR-671-5p, hsa-miR-382-5p, hsa-miR-197-3p, hsa-miR-126-3p, hsa-miR-150-5p, hsa-let-7i-3p, hsa-miR-222-3p, hsa-miR-153-3p, hsa-miR-582-5p, hsa-let-7g-3p | 96.67 | 91.88 | 70.00 |
| 1652 | hsa-miR-590-5p, hsa-miR-455-3p, hsa-miR-7-1-3p, hsa-miR-671-5p, age, hsa-miR-532-5p, hsa-miR-197-3p, hsa-miR-126-3p, hsa-miR-150-5p, hsa-let-7i-3p, hsa-miR-377-3p, hsa-miR-590-3p, hsa-miR-582-5p | 95.73 | 96.43 | 80.00 |
| 1653 | hsa-miR-376c-3p, hsa-miR-455-3p, hsa-miR-7-1-3p, age, hsa-miR-197-3p, hsa-miR-150-5p, hsa-miR-222-3p, hsa-miR-377-3p, hsa-miR-153-3p, hsa-miR-590-3p, hsa-miR-582-5p, hsa-miR-1290, hsa-miR-410-3p | 95.67 | 98.24 | 70.00 |
| 1654 | hsa-miR-484, hsa-miR-545-5p, hsa-miR-590-5p, hsa-let-7d-3p, hsa-miR-7-1-3p, hsa-miR-598-3p, hsa-miR-671-5p, hsa-miR-532-5p, hsa-miR-126-3p, hsa-miR-150-5p, hsa-let-7i-3p, hsa-miR-377-3p | 95.67 | 99.41 | 60.00 |
| 1655 | hsa-miR-590-5p, hsa-miR-455-3p, hsa-let-7d-3p, hsa-miR-7-1-3p, hsa-miR-671-5p, age, hsa-miR-532-5p, hsa-miR-126-3p, hsa-miR-150-5p, hsa-let-7i-3p, hsa-miR-377-3p, hsa-miR-582-5p, hsa-miR-410-3p | 95.67 | 98.75 | 70.00 |
| 1656 | hsa-miR-30e-3p, hsa-miR-455-3p, hsa-miR-7-1-3p, age, hsa-miR-342-3p, hsa-miR-532-5p, hsa-miR-197-3p, hsa-miR-222-3p, hsa-miR-377-3p, hsa-miR-590-3p, hsa-miR-582-5p, hsa-miR-410-3p | 95.67 | 97.57 | 60.00 |
| 1657 | hsa-miR-484, hsa-miR-376c-3p, hsa-miR-545-5p, hsa-miR-590-5p, hsa-miR-7-1-3p, hsa-miR-598-3p, hsa-miR-671-5p, hsa-miR-382-5p, hsa-miR-197-3p, hsa-miR-150-5p, hsa-let-7i-3p, hsa-miR-377-3p | 95.67 | 98.2 | 50.00 |
| 1658 | hsa-miR-590-5p, hsa-miR-455-3p, hsa-miR-7-1-3p, hsa-miR-671-5p, hsa-miR-532-5p, hsa-miR-197-3p, hsa-miR-150-5p, hsa-let-7i-3p, hsa-miR-377-3p, hsa-miR-582-5p, hsa-miR-410-3p | 95.67 | 94.6 | 50.00 |
| 1659 | hsa-miR-484, hsa-miR-30e-3p, hsa-miR-590-5p, hsa-let-7d-3p, hsa-miR-7-1-3p, hsa-miR-598-3p, hsa-miR-671-5p, age, hsa-miR-532-5p, hsa-miR-126-3p, hsa-miR-150-5p, hsa-let-7i-3p, hsa-miR-410-3p, hsa-let-7g-3p | 95.67 | 95.7 | 50.00 |
| 1660 | hsa-miR-484, hsa-miR-376c-3p, hsa-miR-590-5p, hsa-miR-7-1-3p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-410-3p | 95.67 | 98.2 | 80.00 |
| 1661 | hsa-miR-376c-3p, hsa-miR-6803-3p, hsa-miR-590-5p, hsa-miR-455-3p, hsa-miR-598-3p, hsa-miR-671-5p, hsa-miR-342-3p, hsa-miR-197-3p, hsa-miR-150-5p, hsa-miR-222-3p, hsa-miR-377-3p, hsa-miR-153-3p, hsa-miR-410-3p | 95.61 | 98.13 | 50.00 |
| 1662 | hsa-miR-484, hsa-miR-376c-3p, hsa-miR-6803-3p, hsa-miR-590-5p, hsa-let-7d-3p, hsa-miR-598-3p, hsa-miR-671-5p, age, hsa-miR-342-3p, hsa-miR-126-3p, hsa-miR-150-5p, hsa-let-7i-3p, hsa-miR-377-3p, hsa-miR-153-3p, hsa-miR-1290, hsa-miR-410-3p | 95.61 | 97.57 | 40.00 |
| 1663 | hsa-miR-30e-3p, hsa-miR-545-5p, hsa-miR-455-3p, hsa-miR-7-1-3p, age, hsa-miR-197-3p, hsa-let-7i-3p, hsa-miR-590-3p, hsa-miR-582-5p, hsa-miR-1290, hsa-let-7g-3p | 95.61 | 98.2 | 60.00 |
| 1664 | hsa-miR-484, hsa-miR-545-5p, hsa-miR-6803-3p, hsa-miR-590-5p, hsa-let-7d-3p, hsa-miR-598-3p, hsa-miR-382-5p, hsa-miR-342-3p, hsa-miR-126-3p, hsa-miR-150-5p, hsa-miR-222-3p, hsa-miR-153-3p, hsa-miR-590-3p, hsa-miR-1290, hsa-let-7g-3p | 95.61 | 87.32 | 50.00 |
| 1665 | hsa-miR-376c-3p, hsa-miR-545-5p, hsa-miR-6803-3p, hsa-miR-455-3p, hsa-let-7d-3p, hsa-miR-598-3p, hsa-miR-671-5p, age, hsa-miR-342-3p, hsa-miR-126-3p, hsa-let-7i-3p, hsa-miR-377-3p, hsa-miR-590-3p, hsa-miR-582-5p | 95.61 | 96.29 | 90.00 |
| 1666 | hsa-miR-30e-3p, hsa-miR-376c-3p, hsa-miR-6803-3p, hsa-miR-455-3p, hsa-let-7d-3p, age, hsa-miR-342-3p, hsa-miR-532-5p, hsa-miR-126-3p, hsa-miR-222-3p, hsa-miR-153-3p, hsa-miR-590-3p, hsa-miR-582-5p, hsa-miR-1290, hsa-miR-410-3p | 95.61 | 94.45 | 50.00 |
| 1667 | hsa-miR-455-3p, hsa-let-7d-3p, hsa-miR-7-1-3p, age, hsa-miR-382-5p, hsa-miR-532-5p, hsa-miR-126-3p, hsa-miR-222-3p, hsa-miR-377-3p, hsa-miR-582-5p, hsa-miR-410-3p | 95.61 | 95.74 | 80.00 |
| 1668 | hsa-miR-376c-3p, hsa-miR-6803-3p, hsa-miR-455-3p, hsa-miR-382-5p, hsa-miR-342-3p, hsa-miR-197-3p, hsa-miR-222-3p, hsa-miR-377-3p, hsa-miR-153-3p, hsa-miR-582-5p, hsa-miR-1290, hsa-miR-410-3p | 95.61 | 95.85 | 60.00 |
| 1669 | hsa-miR-376c-3p, hsa-miR-6803-3p, hsa-miR-598-3p, hsa-miR-671-5p, age, hsa-miR-342-3p, hsa-miR-197-3p, hsa-let-7i-3p, hsa-miR-377-3p, hsa-miR-153-3p, hsa-miR-590-3p, hsa-miR-1290 | 95.61 | 99.41 | 50.00 |
| 1670 | hsa-miR-6803-3p, hsa-miR-590-5p, hsa-miR-598-3p, hsa-miR-671-5p, hsa-miR-342-3p, hsa-miR-197-3p, hsa-let-7i-3p, hsa-miR-377-3p, hsa-miR-1290, hsa-miR-410-3p | 95.61 | 96.32 | 80.00 |
| 1671 | hsa-miR-376c-3p, hsa-miR-6803-3p, hsa-miR-455-3p, hsa-let-7d-3p, hsa-miR-671-5p, age, hsa-miR-342-3p, hsa-miR-126-3p, hsa-let-7i-3p, hsa-miR-377-3p, hsa-miR-153-3p, hsa-miR-582-5p, hsa-miR-1290 | 95.61 | 95.7 | 60.00 |
| 1672 | hsa-miR-484, hsa-miR-590-5p, hsa-miR-7-1-3p, hsa-miR-671-5p, age, hsa-miR-342-3p, hsa-miR-532-5p, hsa-miR-197-3p, hsa-miR-150-5p, hsa-let-7i-3p, hsa-miR-377-3p, hsa-miR-590-3p, hsa-miR-410-3p, hsa-let-7g-3p | 95.61 | 95.29 | 50.00 |
| 1673 | hsa-miR-484, hsa-miR-545-5p, hsa-miR-590-5p, hsa-let-7d-3p, hsa-miR-7-1-3p, hsa-miR-598-3p, hsa-miR-671-5p, hsa-miR-126-3p, hsa-miR-150-5p, hsa-let-7i-3p, hsa-miR-377-3p | 95.61 | 99.38 | 60.00 |
| 1674 | hsa-miR-376c-3p, hsa-miR-455-3p, hsa-let-7d-3p, hsa-miR-671-5p, hsa-miR-382-5p, hsa-miR-342-3p, hsa-miR-150-5p, hsa-miR-222-3p, hsa-miR-377-3p, hsa-miR-582-5p, hsa-let-7g-3p | 95.61 | 95.77 | 60.00 |
| 1675 | hsa-miR-376c-3p, hsa-miR-545-5p, hsa-miR-6803-3p, hsa-miR-590-5p, hsa-miR-455-3p, hsa-let-7d-3p, hsa-miR-598-3p, age, hsa-miR-197-3p, hsa-miR-126-3p, hsa-miR-410-3p, hsa-let-7g-3p | 95.61 | 100.00 | 40.00 |
| 1676 | hsa-miR-545-5p, hsa-miR-455-3p, hsa-miR-7-1-3p, hsa-miR-382-5p, hsa-miR-222-3p, hsa-miR-377-3p, hsa-miR-590-3p, hsa-miR-582-5p | 95.61 | 98.75 | 60.00 |
| 1677 | hsa-miR-6803-3p, hsa-miR-590-5p, hsa-let-7d-3p, hsa-miR-7-1-3p, hsa-miR-671-5p, hsa-miR-342-3p, hsa-miR-197-3p, hsa-miR-150-5p, hsa-let-7i-3p, hsa-miR-222-3p, hsa-miR-377-3p, hsa-miR-153-3p, hsa-miR-582-5p, hsa-miR-1290, hsa-miR-410-3p, hsa-let-7g-3p | 95.61 | 89.89 | 50.00 |
| 1678 | hsa-miR-545-5p, hsa-miR-455-3p, hsa-miR-7-1-3p, age, hsa-miR-382-5p, hsa-miR-342-3p, hsa-miR-197-3p, hsa-miR-222-3p, hsa-miR-377-3p, hsa-miR-590-3p, hsa-miR-582-5p | 95.61 | 98.13 | 60.00 |
| 1679 | hsa-miR-484, hsa-miR-376c-3p, hsa-miR-590-5p, hsa-miR-7-1-3p, age, hsa-miR-342-3p, hsa-miR-197-3p, hsa-miR-126-3p, hsa-miR-150-5p, hsa-let-7i-3p, hsa-miR-377-3p, hsa-miR-153-3p, hsa-miR-590-3p, hsa-miR-1290, hsa-miR-410-3p | 95.61 | 96.47 | 40.00 |
| 1680 | hsa-miR-484, hsa-let-7d-3p, hsa-miR-7-1-3p, hsa-miR-598-3p, age, hsa-miR-382-5p, hsa-miR-126-3p, hsa-miR-222-3p, hsa-miR-377-3p, hsa-miR-410-3p | 95.61 | 97.57 | 80.00 |
| 1681 | hsa-miR-455-3p, hsa-miR-7-1-3p, age, hsa-miR-382-5p, hsa-miR-342-3p, hsa-miR-197-3p, hsa-miR-222-3p, hsa-miR-377-3p, hsa-miR-590-3p, hsa-miR-582-5p, hsa-miR-410-3p | 95.56 | 97.57 | 90.00 |
| 1682 | hsa-miR-455-3p, hsa-let-7d-3p, hsa-miR-7-1-3p, hsa-miR-671-5p, hsa-miR-126-3p, hsa-let-7i-3p, hsa-miR-377-3p, hsa-miR-590-3p, hsa-miR-582-5p | 95.56 | 97.57 | 50.00 |
| 1683 | hsa-miR-455-3p, hsa-miR-7-1-3p, hsa-miR-382-5p, hsa-miR-342-3p, hsa-miR-197-3p, hsa-miR-222-3p, hsa-miR-377-3p, hsa-miR-590-3p, hsa-miR-582-5p, hsa-miR-410-3p | 95.56 | 97.57 | 90.00 |
| 1684 | hsa-miR-484, hsa-miR-376c-3p, hsa-miR-6803-3p, hsa-let-7d-3p, hsa-miR-598-3p, hsa-miR-671-5p, age, hsa-miR-382-5p, hsa-miR-126-3p, hsa-miR-150-5p, hsa-let-7i-3p, hsa-miR-377-3p, hsa-miR-590-3p, hsa-miR-410-3p | 95.56 | 97.5 | 40.00 |
| 1685 | hsa-miR-455-3p, hsa-miR-7-1-3p, age, hsa-miR-382-5p, hsa-miR-197-3p, hsa-miR-222-3p, hsa-miR-377-3p, hsa-miR-590-3p, hsa-miR-582-5p, hsa-miR-410-3p | 95.56 | 96.95 | 60.00 |
| 1686 | hsa-miR-484, hsa-let-7d-3p, hsa-miR-7-1-3p, hsa-miR-598-3p, age, hsa-miR-126-3p, hsa-miR-150-5p, hsa-let-7i-3p, hsa-miR-590-3p, hsa-miR-1290, hsa-miR-410-3p | 95.56 | 93.9 | 40.00 |
| 1687 | hsa-miR-545-5p, hsa-miR-455-3p, hsa-let-7d-3p, hsa-miR-7-1-3p, hsa-miR-671-5p, age, hsa-miR-126-3p, hsa-let-7i-3p, hsa-miR-377-3p, hsa-miR-590-3p, hsa-miR-582-5p | 95.56 | 97.57 | 50.00 |
| 1688 | hsa-miR-455-3p, hsa-let-7d-3p, hsa-miR-7-1-3p, hsa-miR-671-5p, age, hsa-miR-342-3p, hsa-miR-532-5p, hsa-miR-126-3p, hsa-let-7i-3p, hsa-miR-377-3p, hsa-miR-590-3p, hsa-miR-582-5p, hsa-miR-410-3p | 94.62 | 98.82 | 60.00 |
| 1689 | hsa-miR-376c-3p, hsa-miR-455-3p, hsa-let-7d-3p, hsa-miR-7-1-3p, hsa-miR-671-5p, age, hsa-miR-342-3p, hsa-miR-532-5p, hsa-miR-126-3p, hsa-let-7i-3p, hsa-miR-377-3p, hsa-miR-590-3p, hsa-miR-582-5p | 94.62 | 98.82 | 60.00 |
| 1690 | hsa-miR-484, hsa-miR-376c-3p, hsa-miR-6803-3p, hsa-miR-598-3p, age, hsa-miR-382-5p, hsa-miR-342-3p, hsa-miR-532-5p, hsa-miR-197-3p, hsa-miR-222-3p, hsa-miR-377-3p, hsa-miR-153-3p, hsa-miR-590-3p, hsa-miR-1290, hsa-miR-410-3p | 94.56 | 95.22 | 30.00 |
| 1691 | hsa-miR-545-5p, hsa-miR-455-3p, hsa-miR-7-1-3p, age, hsa-miR-382-5p, hsa-miR-197-3p, hsa-miR-126-3p, hsa-miR-222-3p, hsa-miR-377-3p, hsa-miR-590-3p, hsa-miR-582-5p, hsa-miR-410-3p | 94.56 | 95.85 | 30.00 |
| 1692 | hsa-miR-455-3p, hsa-let-7d-3p, hsa-miR-7-1-3p, hsa-miR-532-5p, hsa-miR-126-3p, hsa-miR-222-3p, hsa-miR-377-3p, hsa-miR-582-5p, hsa-miR-410-3p | 94.56 | 90.96 | 80.00 |
| 1693 | hsa-miR-30e-3p, hsa-miR-455-3p, hsa-let-7d-3p, hsa-miR-7-1-3p, hsa-miR-671-5p, age, hsa-miR-382-5p, hsa-miR-532-5p, hsa-miR-126-3p, hsa-let-7i-3p, hsa-miR-590-3p, hsa-miR-582-5p, hsa-let-7g-3p | 94.56 | 94.67 | 80.00 |
| 1694 | hsa-miR-484, hsa-miR-590-5p, hsa-miR-7-1-3p, hsa-miR-598-3p, hsa-miR-671-5p, age, hsa-miR-342-3p, hsa-miR-197-3p, hsa-miR-150-5p, hsa-let-7i-3p, hsa-miR-377-3p, hsa-miR-410-3p | 94.56 | 95.77 | 40.00 |
| 1695 | hsa-miR-484, hsa-miR-545-5p, hsa-miR-590-5p, hsa-let-7d-3p, hsa-miR-7-1-3p, hsa-miR-598-3p, hsa-miR-671-5p, hsa-miR-126-3p, hsa-miR-150-5p, hsa-let-7i-3p, hsa-miR-377-3p, hsa-miR-410-3p, hsa-let-7g-3p | 94.5 | 96.95 | 20.00 |
| 1696 | hsa-miR-30e-3p, hsa-miR-455-3p, hsa-miR-7-1-3p, age, hsa-miR-197-3p, hsa-miR-222-3p, hsa-miR-590-3p, hsa-miR-582-5p, hsa-miR-410-3p, hsa-let-7g-3p | 94.5 | 96.43 | 50.00 |
| 1697 | hsa-miR-545-5p, hsa-miR-455-3p, hsa-let-7d-3p, hsa-miR-7-1-3p, age, hsa-miR-382-5p, hsa-miR-126-3p, hsa-miR-222-3p, hsa-miR-377-3p, hsa-miR-582-5p | 94.5 | 93.42 | 40.00 |
| 1698 | hsa-miR-30e-3p, hsa-miR-376c-3p, hsa-miR-455-3p, age, hsa-miR-382-5p, hsa-miR-342-3p, hsa-miR-532-5p, hsa-miR-126-3p, hsa-miR-150-5p, hsa-miR-222-3p, hsa-miR-377-3p, hsa-miR-582-5p, hsa-miR-410-3p | 94.5 | 98.75 | 60.00 |
| 1699 | hsa-miR-455-3p, hsa-let-7d-3p, hsa-miR-7-1-3p, age, hsa-miR-382-5p, hsa-miR-342-3p, hsa-miR-126-3p, hsa-miR-222-3p, hsa-miR-377-3p, hsa-miR-582-5p, hsa-miR-410-3p | 94.5 | 96.32 | 80.00 |
| 1700 | hsa-miR-484, hsa-miR-376c-3p, hsa-miR-6803-3p, hsa-miR-590-5p, hsa-let-7d-3p, hsa-miR-598-3p, age, hsa-miR-382-5p, hsa-miR-126-3p, hsa-miR-150-5p, hsa-miR-222-3p, hsa-miR-377-3p, hsa-miR-153-3p, hsa-miR-1290, hsa-miR-410-3p | 94.5 | 97.57 | 60.00 |
| 1701 | hsa-miR-545-5p, hsa-miR-6803-3p, hsa-miR-455-3p, hsa-let-7d-3p, hsa-miR-671-5p, hsa-miR-342-3p, hsa-miR-126-3p, hsa-let-7i-3p, hsa-miR-377-3p, hsa-miR-582-5p | 94.5 | 91.99 | 70.00 |
| 1702 | hsa-miR-484, hsa-let-7d-3p, hsa-miR-7-1-3p, hsa-miR-598-3p, age, hsa-miR-382-5p, hsa-miR-342-3p, hsa-miR-532-5p, hsa-miR-150-5p, hsa-let-7i-3p, hsa-miR-377-3p, hsa-miR-153-3p, hsa-miR-590-3p | 94.5 | 97.02 | 40.00 |
| 1703 | hsa-miR-545-5p, hsa-miR-455-3p, hsa-let-7d-3p, hsa-miR-7-1-3p, hsa-miR-382-5p, hsa-miR-222-3p, hsa-miR-377-3p, hsa-miR-590-3p, hsa-miR-582-5p, hsa-miR-410-3p | 94.44 | 96.4 | 50.00 |
| 1704 | hsa-miR-455-3p, hsa-miR-7-1-3p, hsa-miR-382-5p, hsa-miR-197-3p, hsa-miR-126-3p, hsa-miR-222-3p, hsa-miR-377-3p, hsa-miR-590-3p, hsa-miR-582-5p, hsa-miR-410-3p | 93.45 | 93.97 | 60.00 |
| 1705 | hsa-miR-30e-3p, hsa-miR-376c-3p, hsa-miR-455-3p, hsa-miR-7-1-3p, hsa-miR-598-3p, hsa-miR-197-3p, hsa-miR-126-3p, hsa-let-7i-3p, hsa-miR-582-5p, hsa-miR-410-3p | 93.45 | 96.4 | 20.00 |
| 1706 | hsa-miR-376c-3p, hsa-miR-545-5p, hsa-miR-6803-3p, hsa-miR-590-5p, hsa-miR-455-3p, hsa-let-7d-3p, hsa-miR-598-3p, hsa-miR-671-5p, hsa-miR-126-3p, hsa-miR-150-5p, hsa-let-7i-3p, hsa-miR-377-3p, hsa-miR-582-5p, hsa-miR-1290, hsa-miR-410-3p | 93.33 | 97.57 | 50.00 |
| 1707 | hsa-miR-455-3p, hsa-miR-7-1-3p, hsa-miR-197-3p, hsa-miR-222-3p, hsa-miR-377-3p, hsa-miR-582-5p, hsa-miR-410-3p | 92.34 | 92.79 | 0.00 |
| 1708 | hsa-miR-455-3p, hsa-let-7d-3p, hsa-miR-7-1-3p, hsa-miR-222-3p, hsa-miR-377-3p, hsa-miR-582-5p, hsa-miR-410-3p | 92.34 | 92.79 | 0.00 |
| 1709 | combination 1337 + rs11647727 | 93.51 | 94.71 | 86.62 |
| 1710 | combination 1338 + rs11647727 | 94.56 | 86.36 | 85.99 |
| 1711 | combination 1339 + rs13329952 | 96.67 | 93.42 | 94.12 |
| 1712 | combination 1340 + rs9928757 | 96.73 | 96.36 | 94.12 |
| 1713 | combination 1341 + rs11647727 | 94.5 | 94.04 | 94.12 |
| 1714 | combination 1344 + rs4297685 | 96.73 | 85.88 | 91.76 |
| 1715 | combination 1345 + rs4297685 | 95.67 | 85.88 | 92.94 |
| 1716 | combination 1353 + rs4293393 | 97.78 | 95.29 | 88.2 |
| 1717 | combination 1354 + rs4293393 | 97.78 | 92.94 | 87.3 |
| 1718 | combination 1356 + rs13335818 | 96.67 | 90.59 | 95.1 |
| 1719 | combination 1359 + rs13335818 | 95.61 | 90.44 | 97.1 |
| 1720 | combination 1360 + rs13335818 | 95.61 | 88.64 | 92.94 |
| 1721 | combination 1681 + rs11647727 | 95.61 | 89.12 | 91.76 |
| 1722 | combination 1682 + rs11647727 | 95.61 | 89.12 | 94.12 |

Example 3: Circulating plasma miRNAs profiling in the prospective multicentric cohort
Then, the inventors repeat the same type of statistical analysis restricted to the prospective multicentric cohort only, consisting of 59 patients with a renal mass (either benign or malignant) and 51 controls.

The inventors found a set of 6 miRNAs that was significantly differentially expressed (p value < 0.05) in plasma of patients with a renal mass (n = 59) compared with controls (n = 51). In addition, 12 miRNAs, even if did not reach statistical significance, showed a trend that could become significant by increasing the study cohort (0.05 < p value < 0.1)(Table 20).

**Table 20. Differentially expressed miRNAs in patients with a renal mass (n = 59) compared with controls (n = 51).**

| miRNA | FC* | p value |
|---|---|---|
| hsa-miR-423-5p | 1.21 | 0.006 |
| hsa-miR-378a-3p | 1.22 | 0.02 |
| hsa-miR-877-5p | 1.32 | 0.03 |
| hsa-miR-505-3p | 1.18 | 0.03 |
| hsa-miR-2110 | 1.31 | 0.03 |
| hsa-miR-500a-3p | 1.15 | 0.047 |
| hsa-miR-4306 | 1.16 | 0.05 |
| hsa-miR-499a-5p | 1.36 | 0.05 |
| hsa-miR-483-5p | 1.31 | 0.06 |
| hsa-miR-376c-3p | 0.76 | 0.06 |
| hsa-miR-361-5p | 1.17 | 0.07 |
| hsa-miR-494-3p | 0.69 | 0.08 |
| hsa-miR-28-3p | 1.21 | 0.08 |
| hsa-miR-377-3p | 0.84 | 0.08 |
| hsa-miR-154-3p | 0.86 | 0.08 |
| hsa-miR-320e | 1.17 | 0.08 |
| hsa-miR-331-3p | 0.88 | 0.09 |
| hsa-miR-30b-5p | 0.90 | 0.09 |

| | | |
|---|---|---|
| *fold changes (>1 increased; <1 decreased expression in patients with a renal mass) | | |

Then, the inventors investigated whether patients with a benign mass were associated with a specific miRNA signature that significantly differs from patients with a malignant lesion (RCC). The inventors observed26 miRNAs significantly differentially expressed in patients with a benign mass (n = 17) compared with RCC patients (n = 42).In addition, 17 miRNAs showed a trend towards statistical significance (0.05 < p value < 0.1)(Table 21).

**Table 21. Differentially expressed miRNAs in patients with a benign mass (n = 17) compared with RCC patients (n = 42).**

| miRNA | FC | p value* |
|---|---|---|
| hsa-miR-337-5p | 1.40 | 0.002 |
| hsa-miR-550a-3-5p | 1.85 | 0.003 |
| hsa-miR-574-5p | 1.71 | 0.01 |
| hsa-miR-130b-5p | 1.45 | 0.01 |
| hsa-miR-132-3p | 1.49 | 0.01 |
| hsa-miR-103a-2-5p | 1.18 | 0.01 |
| hsa-miR-99a-3p | 1.78 | 0.02 |
| hsa-miR-1249-3p | 1.35 | 0.02 |
| hsa-miR-375-3p | 1.43 | 0.02 |
| hsa-miR-1290 | 1.45 | 0.02 |
| hsa-miR-1180-3p | 0.55 | 0.02 |
| hsa-miR-29c-5p | 0.81 | 0.03 |
| hsa-miR-214-3p | 1.38 | 0.03 |
| hsa-miR-362-3p | 1.27 | 0.03 |
| hsa-miR-223-3p | 0.82 | 0.03 |
| hsa-miR-181a-5p | 0.77 | 0.03 |
| hsa-miR-205-5p | 1.44 | 0.03 |
| hsa-miR-582-5p | 1.33 | 0.03 |
| hsa-miR-196b-5p | 1.46 | 0.03 |
| hsa-miR-497-5p | 1.14 | 0.04 |
| hsa-miR-151a-3p | 0.77 | 0.04 |
| hsa-miR-361-3p | 0.76 | 0.04 |
| hsa-miR-450a-5p | 1.43 | 0.04 |
| hsa-miR-532-3p | 1.28 | 0.04 |
| hsa-miR-410-3p | 1.49 | 0.047 |
| hsa-miR-6803-3p | 1.28 | 0.047 |
| hsa-let-7d-5p | 0.76 | 0.053 |
| hsa-miR-1277-3p | 1.38 | 0.053 |
| hsa-miR-1260a | 1.28 | 0.059 |
| hsa-miR-96-5p | 1.16 | 0.059 |
| hsa-miR-31-5p | 1.29 | 0.061 |
| hsa-miR-942-5p | 1.28 | 0.061 |
| hsa-miR-1260b | 1.21 | 0.074 |
| hsa-miR-26a-5p | 0.88 | 0.074 |
| hsa-miR-30d-5p | 0.88 | 0.074 |
| hsa-miR-181c-5p | 0.75 | 0.076 |
| hsa-miR-7-1-3p | 1.22 | 0.079 |
| hsa-miR-590-3p | 1.16 | 0.082 |
| hsa-miR-590-5p | 1.19 | 0.085 |
| hsa-miR-421 | 1.22 | 0.088 |
| hsa-miR-32-3p | 1.31 | 0.091 |
| hsa-miR-326 | 0.76 | 0.098 |
| hsa-miR-3613-3p | 0.79 | 0.098 |

| | | |
|---|---|---|
| *fold changes (>1 increased; <1 decreased expression in patients with a benign mass) | | |

A panel of 11 miRNAs was found to be significantly differentially expressed between low grade RCC patients (n = 31) and high grade cases (n = 11), while 8 miRNAs showed a trend towards statistical significance (0.05 < p value < 0.1) (Table22).

**Table 22. Differentially expressed miRNAs (p value ≤ 0.05) in low grade RCC patients (n = 31) compared with high grade RCC patients (n = 11).**

| miRNA | FC* | p value |
|---|---|---|
| hsa-miR-103a-2-5p | 1.23 | 0.03 |
| hsa-miR-150-5p | 0.61 | 0.03 |
| hsa-miR-629-5p | 1.38 | 0.03 |
| hsa-miR-433-3p | 1.89 | 0.04 |
| hsa-miR-148a-3p | 0.74 | 0.04 |
| hsa-miR-29a-3p | 0.72 | 0.04 |
| hsa-miR-29b-3p | 0.73 | 0.04 |
| hsa-miR-29c-5p | 0.76 | 0.04 |
| hsa-let-7e-5p | 1.17 | 0.0497 |
| hsa-miR-27a-3p | 0.76 | 0.0497 |
| hsa-miR-342-3p | 0.73 | 0.0497 |
| hsa-miR-382-5p | 2.17 | 0.060 |
| hsa-miR-151a-3p | 1.29 | 0.068 |
| hsa-miR-10a-5p | 0.73 | 0.087 |
| hsa-miR-202-5p | 0.79 | 0.087 |
| hsa-miR-4306 | 1.32 | 0.087 |
| hsa-miR-34a-5p | 0.74 | 0.098 |
| hsa-miR-369-3p | 1.37 | 0.098 |
| hsa-miR-584-5p | 1.22 | 0.098 |

| | | |
|---|---|---|
| *fold changes (>1 increased; <1 decreased expression in high grade RCC patients) | | |

Similarly, the inventors found that 7 miRNAs can significantly distinguish between low stage RCC patients (n = 34) and high stage RCC patients (n = 8), while 6 miRNAs showed a p value between 0.06 and 0.09, indicating a trend towards statistical significance(Table 23).

**Table 23. Differentially expressed miRNAs in low stage RCC patients (n = 34) compared with high stage RCC patients (n = 8).**

| miRNA | FC | p value* |
|---|---|---|
| hsa-miR-324-3p | 0.64 | 0.01 |
| hsa-miR-215-5p | 0.54 | 0.01 |
| hsa-miR-769-5p | 2.06 | 0.01 |
| hsa-miR-598-3p | 1.67 | 0.03 |
| hsa-miR-320a-3p | 1.32 | 0.04 |
| hsa-miR-548a-3p | 1.55 | 0.04 |
| hsa-miR-190a-5p | 0.70 | 0.04 |
| hsa-miR-491-5p | 1.34 | 0.06 |
| hsa-miR-335-5p | 0.83 | 0.07 |
| hsa-miR-423-3p | 1.32 | 0.09 |
| hsa-miR-100-5p | 0.60 | 0.09 |
| hsa-miR-224-5p | 0.61 | 0.09 |
| hsa-miR-421 | 0.72 | 0.09 |

| | | |
|---|---|---|
| *fold changes (>1 increased; <1 decreased expression in high stage RCC patients) | | |

No analysis regarding miRNAs' signatures related to metastatic outcome were performed in the prospectic multicentic phase because only one patient was affected by a metastatic process.

Similarly to the total study cohort analysis reported above, in addition to single biomarker analysis, the inventors identified unique sets of miRNAs (miRNA signatures) leading to a significant increase in sensitivity, specificity, accuracy and predictive power for screening, diagnosing and prognosing of RCC patients by using machine learning algorithms.

Again, the inventors investigated clinical parameters in association with miRNAs biomarkers, using the feature selection algorithm, and the variable age resulted predictive in association with some miRNAs in screening, and diagnosis. It is important to underline that the single variable age, using the same evaluation metric, performed significantly less than the presented combinations with an accuracy of 52.72%, recall of 38.33%, and ROC AUC of 57.82% for screening and an accuracy of 38.78%, recall of 62.5%, and ROC AUC of 56.64% for diagnosis.

In addition, the inventors combined some of the identified miRNAs signatures (together or not with age) with SNPs, creating unique combinations leading to a further significant increase in sensitivity, specificity, accuracy and predictive power for screening, diagnosing and prognosing of RCC patients by using machine learning algorithms (Table 24, 25, 26, 27).

This is the list of the best 29 mRNA selected by the algorithm sorted by importance for screening outcome: hsa-miR-378a-3p, hsa-miR-328-3p, hsa-miR-28-3p, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p, hsa-miR-18a-5p, hsa-miR-130a-3p, hsa-miR-4306, hsa-miR-423-3p, hsa-miR-6803-3p, hsa-miR-664a-3p, hsa-miR-323b-3p, hsa-miR-128-3p, and hsa-miR-494-3p. The inventors combined these miRNAs, together or not with age and UMOD SNP variant, creating unique combinations leading to a further significant increase in sensitivity, specificity, accuracy and predictive power for screening (Table 24).

**Table 24. Combinations that allow higher sensitivity, specificity, accuracy and predictive power for screening.**

| N | Combinations | Accuracy | ROC AUC | Recall |
|---|---|---|---|---|
| 1723 | hsa-miR-378a-3p | 60.61 | 64.57 | 86.8 |
| 1724 | hsa-miR-378a-3p, hsa-miR-328-3p | 59.7 | 62.72 | 80.71 |
| 1725 | hsa-miR-378a-3p, hsa-miR-328-3p, hsa-miR-28-3p | 69.74 | 69.33 | 81.66 |
| 1726 | hsa-miR-378a-3p, hsa-miR-328-3p, hsa-miR-28-3p, age | 68.83 | 75.54 | 85.14 |
| 1727 | hsa-miR-378a-3p, hsa-miR-328-3p, hsa-miR-28-3p, age, hsa-miR-361-5p | 68.83 | 74.94 | 82.37 |
| 1728 | hsa-miR-378a-3p, hsa-miR-328-3p, hsa-miR-28-3p, age, hsa-miR-361-5p, hsa-miR-1260b | 70.69 | 75.89 | 84.15 |
| 1729 | hsa-miR-378a-3p, hsa-miR-328-3p, hsa-miR-28-3p, age, hsa-miR-361-5p, hsa-miR-1260b, hsa-miR-579-3p | 72.55 | 81.78 | 79.84 |
| 1730 | hsa-miR-378a-3p, hsa-miR-328-3p, hsa-miR-28-3p, age, hsa-miR-361-5p, hsa-miR-1260b, hsa-miR-579-3p, hsa-miR-532-3p | 75.24 | 82.96 | 81.5 |
| 1731 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p | 74.37 | 83.15 | 83.28 |
| 1732 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p | 78.92 | 85 | 83.28 |
| 1733 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p | 79.87 | 85.67 | 79.84 |
| 1734 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p | 77.14 | 86.35 | 81.58 |
| 1735 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p | 74.24 | 86.59 | 81.66 |
| 1736 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p | 79.83 | 87.87 | 79.80 |
| 1737 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p | 82.64 | 88.87 | 82.45 |
| 1738 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p | 81.65 | 88.35 | 79.76 |
| 1739 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p | 77.92 | 88.43 | 80.67 |
| 1740 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326 | 81.65 | 90.81 | 77.94 |
| 1741 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110 | 81.65 | 90.31 | 78.02 |
| 1742 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p | 82.6 | 92.35 | 78.02 |
| 1743 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p | 81.69 | 91.35 | 81.54 |
| 1744 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p, hsa-miR-18a-5p | 80.82 | 89.56 | 80.67 |
| 1745 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p, hsa-miR-18a-5p, hsa-miR-130a-3p | 80.65 | 89.8 | 81.54 |
| 1746 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p, hsa-miR-18a-5p, hsa-miR-130a-3p, hsa-miR-4306 | 78.92 | 90.35 | 80.63 |
| 1747 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p, hsa-miR-18a-5p, hsa-miR-130a-3p, hsa-miR-4306, hsa-miR-423-3p | 79.74 | 90.67 | 82.37 |
| 1748 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p, hsa-miR-18a-5p, hsa-miR-130a-3p, hsa-miR-4306, hsa-miR-423-3p, hsa-miR-6803-3p | 80.69 | 90.67 | 80.59 |
| 1749 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p, hsa-miR-18a-5p, hsa-miR-130a-3p, hsa-miR-4306, hsa-miR-423-3p, hsa-miR-6803-3p, hsa-miR-664a-3p | 81.65 | 91.17 | 81.5 |
| 1750 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p, hsa-miR-18a-5p, hsa-miR-130a-3p, hsa-miR-4306, hsa-miR-423-3p, hsa-miR-6803-3p, hsa-miR-664a-3p, hsa-miR-323b-3p | 82.6 | 91.48 | 81.5 |
| 1751 | hsa-miR-378a-3p, hsa-miR-328-3p, hsa-miR-28-3p, age, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-30b-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-101-3p, hsa-miR-18a-5p, hsa-miR-130a-3p, hsa-miR-4306, hsa-miR-423-3p, hsa-miR-6803-3p, hsa-miR-664a-3p, hsa-miR-323b-3p, hsa-miR-128-3p | 83.46 | 91.07 | 80.63 |
| 1752 | hsa-miR-550a-3-5p, hsa-miR-423-5p, hsa-miR-29c-5p, hsa-miR-326, hsa-miR-197-3p, hsa-miR-130a-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 85.41 | 91.3 | 85 |
| 1753 | hsa-miR-550a-3-5p, hsa-miR-423-5p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 85.41 | 89.93 | 90 |
| 1754 | hsa-miR-550a-3-5p, hsa-miR-423-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 85.41 | 89.8 | 90 |
| 1755 | hsa-miR-550a-3-5p, hsa-miR-423-5p, hsa-miR-29c-5p, hsa-miR-326, hsa-miR-197-3p, hsa-miR-423-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 85.32 | 89.94 | 86.67 |
| 1756 | hsa-miR-550a-3-5p, hsa-miR-423-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-494-3p, age, hsa-miR-1260b, hsa-miR-532-3p, hsa-miR-15b-3p | 84.5 | 89.78 | 88.33 |
| 1757 | hsa-miR-550a-3-5p, hsa-miR-423-5p, hsa-miR-1301-3p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p | 84.46 | 89.76 | 86.67 |
| 1758 | hsa-miR-550a-3-5p, hsa-miR-423-5p, hsa-miR-1301-3p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 84.46 | 90.43 | 88.33 |
| 1759 | hsa-miR-550a-3-5p, hsa-miR-181c-3p, hsa-miR-202-5p, hsa-miR-423-5p, hsa-miR-29c-5p, hsa-miR-326, hsa-miR-2110, hsa-miR-197-3p, hsa-miR-130a-3p, hsa-miR-6803-3p, hsa-miR-323b-3p, hsa-miR-494-3p, age, hsa-miR-1260b, hsa-miR-579-3p, hsa-miR-15b-3p | 84.46 | 90.41 | 86.67 |
| 1760 | hsa-miR-550a-3-5p, hsa-miR-1301-3p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-361-5p, hsa-miR-532-3p, hsa-miR-15b-3p | 84.46 | 86.39 | 85 |
| 1761 | hsa-miR-550a-3-5p, hsa-miR-423-5p, hsa-miR-1301-3p, hsa-miR-29c-5p, hsa-miR-326, hsa-miR-197-3p, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p | 84.46 | 90.61 | 86.67 |
| 1762 | hsa-miR-550a-3-5p, hsa-miR-423-5p, hsa-miR-29c-5p, hsa-miR-326, hsa-miR-197-3p, hsa-miR-18a-5p, hsa-miR-423-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 84.42 | 88.52 | 83.33 |
| 1763 | hsa-miR-550a-3-5p, hsa-miR-1301-3p, hsa-miR-29c-5p, hsa-miR-197-3p, hsa-miR-423-3p, hsa-miR-6803-3p, hsa-miR-664a-3p, hsa-miR-323b-3p, hsa-miR-128-3p, hsa-miR-361-5p, hsa-miR-532-3p | 84.42 | 86.15 | 86.67 |
| 1764 | hsa-miR-550a-3-5p, hsa-miR-423-5p, hsa-miR-1301-3p, hsa-miR-326, hsa-miR-197-3p, hsa-miR-18a-5p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p | 84.42 | 89.94 | 85 |
| 1765 | hsa-miR-550a-3-5p, hsa-miR-202-5p, hsa-miR-423-5p, hsa-miR-29c-5p, hsa-miR-326, hsa-miR-197-3p, hsa-miR-423-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 84.42 | 91.78 | 83.33 |
| 1766 | hsa-miR-550a-3-5p, hsa-miR-423-5p, hsa-miR-29c-5p, hsa-miR-326, hsa-miR-197-3p, hsa-miR-423-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 84.37 | 93.67 | 85 |
| 1767 | hsa-miR-550a-3-5p, hsa-miR-423-5p, hsa-miR-29c-5p, hsa-miR-326, hsa-miR-197-3p, hsa-miR-130a-3p, hsa-miR-423-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 84.37 | 93.31 | 83.33 |
| 1768 | hsa-miR-550a-3-5p, hsa-miR-423-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-128-3p, hsa-miR-494-3p, hsa-miR-1260b, hsa-miR-532-3p, hsa-miR-15b-3p | 83.59 | 86.39 | 86.67 |
| 1769 | hsa-miR-550a-3-5p, hsa-miR-423-5p, hsa-miR-29c-5p, hsa-miR-326, hsa-miR-197-3p, hsa-miR-101-3p, hsa-miR-4306, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 83.59 | 90.43 | 86.67 |
| 1770 | hsa-miR-550a-3-5p, hsa-miR-423-5p, hsa-miR-29c-5p, hsa-miR-326, hsa-miR-197-3p, hsa-miR-101-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 83.59 | 90.09 | 86.67 |
| 1771 | hsa-miR-550a-3-5p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 83.55 | 89.74 | 86.67 |
| 1772 | hsa-miR-550a-3-5p, hsa-miR-181c-3p, hsa-miR-423-5p, hsa-miR-29c-5p, hsa-miR-326, hsa-miR-2110, hsa-miR-197-3p, hsa-miR-130a-3p, hsa-miR-6803-3p, hsa-miR-323b-3p, hsa-miR-494-3p, age, hsa-miR-1260b, hsa-miR-579-3p, hsa-miR-15b-3p | 83.55 | 90.91 | 85 |
| 1773 | hsa-miR-550a-3-5p, hsa-miR-423-5p, hsa-miR-29c-5p, hsa-miR-326, hsa-miR-197-3p, hsa-miR-130a-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 83.55 | 91.8 | 83.33 |
| 1774 | hsa-miR-550a-3-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-128-3p, hsa-miR-494-3p, age, hsa-miR-1260b, hsa-miR-579-3p, hsa-miR-532-3p, hsa-miR-15b-3p | 83.55 | 88.33 | 86.67 |
| 1775 | hsa-miR-550a-3-5p, hsa-miR-181c-3p, hsa-miR-202-5p, hsa-miR-423-5p, hsa-miR-29c-5p, hsa-miR-326, hsa-miR-197-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 83.55 | 90.3 | 88.33 |
| 1776 | hsa-miR-550a-3-5p, hsa-miR-423-5p, hsa-miR-326, hsa-miR-197-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 83.55 | 91 | 86.67 |
| 1777 | hsa-miR-550a-3-5p, hsa-miR-423-5p, hsa-miR-29c-5p, hsa-miR-326, hsa-miR-2110, hsa-miR-197-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-15b-3p | 83.55 | 87.87 | 83.33 |
| 1778 | hsa-miR-550a-3-5p, hsa-miR-423-5p, hsa-miR-1301-3p, hsa-miR-29c-5p, hsa-miR-326, hsa-miR-197-3p, hsa-miR-101-3p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p | 83.51 | 89.91 | 86.67 |
| 1779 | hsa-miR-550a-3-5p, hsa-miR-423-5p, hsa-miR-1301-3p, hsa-miR-29c-5p, hsa-miR-326, hsa-miR-197-3p, hsa-miR-101-3p, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p | 83.51 | 90.74 | 85 |
| 1780 | hsa-miR-550a-3-5p, hsa-miR-1301-3p, hsa-miR-29c-5p, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-361-5p, hsa-miR-532-3p | 83.51 | 85.46 | 85 |
| 1781 | hsa-miR-550a-3-5p, hsa-miR-30b-5p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 82.68 | 84.63 | 85 |
| 1782 | hsa-miR-550a-3-5p, hsa-miR-423-5p, hsa-miR-1301-3p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-361-5p, hsa-miR-532-3p, hsa-miR-15b-3p | 82.64 | 85.94 | 83.33 |
| 1783 | hsa-miR-550a-3-5p, hsa-miR-181c-3p, hsa-miR-423-5p, hsa-miR-29c-5p, hsa-miR-326, hsa-miR-197-3p, hsa-miR-130a-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 82.64 | 90.28 | 83.33 |
| 1784 | hsa-miR-550a-3-5p, hsa-miR-423-5p, hsa-miR-1301-3p, hsa-miR-29c-5p, hsa-miR-326, hsa-miR-197-3p, hsa-miR-101-3p, hsa-miR-130a-3p, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p | 82.64 | 90.24 | 86.67 |
| 1785 | hsa-miR-550a-3-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 82.64 | 89.15 | 85 |
| 1786 | hsa-miR-550a-3-5p, hsa-miR-423-5p, hsa-miR-29c-5p, hsa-miR-197-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 82.64 | 88.43 | 83.33 |
| 1787 | hsa-miR-550a-3-5p, hsa-miR-423-5p, hsa-miR-29c-5p, hsa-miR-197-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p | 82.64 | 88.07 | 81.67 |
| 1788 | hsa-miR-550a-3-5p, hsa-miR-30b-5p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-494-3p, age, hsa-miR-361-5p, hsa-miR-1260b, hsa-miR-579-3p, hsa-miR-532-3p, hsa-miR-15b-3p | 82.64 | 89.76 | 85 |
| 1789 | hsa-miR-550a-3-5p, hsa-miR-423-5p, hsa-miR-1301-3p, hsa-miR-29c-5p, hsa-miR-326, hsa-miR-197-3p, hsa-miR-101-3p, hsa-miR-18a-5p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p | 82.6 | 89.39 | 85 |
| 1790 | hsa-miR-550a-3-5p, hsa-miR-181c-3p, hsa-miR-202-5p, hsa-miR-423-5p, hsa-miR-29c-5p, hsa-miR-326, hsa-miR-197-3p, hsa-miR-130a-3p, hsa-miR-423-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 82.6 | 92.11 | 83.33 |
| 1791 | hsa-miR-550a-3-5p, hsa-miR-423-5p, hsa-miR-1301-3p, hsa-miR-326, hsa-miR-197-3p, hsa-miR-101-3p, hsa-miR-18a-5p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p | 82.6 | 88.93 | 83.33 |
| 1792 | hsa-miR-550a-3-5p, hsa-miR-181c-3p, hsa-miR-202-5p, hsa-miR-29c-5p, hsa-miR-326, hsa-miR-2110, hsa-miR-197-3p, hsa-miR-130a-3p, hsa-miR-4306, hsa-miR-6803-3p, hsa-miR-323b-3p, hsa-miR-494-3p, age, hsa-miR-1260b, hsa-miR-579-3p, hsa-miR-15b-3p | 82.6 | 89.65 | 85 |
| 1793 | hsa-miR-550a-3-5p, hsa-miR-423-5p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 82.51 | 92.37 | 81.67 |
| 1794 | hsa-miR-550a-3-5p, hsa-miR-423-5p, hsa-miR-29c-5p, hsa-miR-326, hsa-miR-197-3p, hsa-miR-130a-3p, hsa-miR-4306, hsa-miR-423-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 82.51 | 92 | 80 |
| 1795 | hsa-miR-550a-3-5p, hsa-miR-30b-5p, hsa-miR-326, hsa-miR-197-3p, hsa-miR-4306, hsa-miR-6803-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 81.73 | 83.63 | 83.33 |
| 1796 | hsa-miR-550a-3-5p, hsa-miR-181c-3p, hsa-miR-423-5p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-494-3p, age, hsa-miR-361-5p, hsa-miR-1260b, hsa-miR-532-3p, hsa-miR-15b-3p | 81.73 | 90.63 | 80 |
| 1797 | hsa-miR-550a-3-5p, hsa-miR-181c-3p, hsa-miR-202-5p, hsa-miR-423-5p, hsa-miR-29c-5p, hsa-miR-326, hsa-miR-197-3p, hsa-miR-130a-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 81.73 | 92.13 | 83.33 |
| 1798 | hsa-miR-550a-3-5p, hsa-miR-181c-3p, hsa-miR-423-5p, hsa-miR-29c-5p, hsa-miR-326, hsa-miR-197-3p, hsa-miR-4306, hsa-miR-423-3p, hsa-miR-6803-3p, hsa-miR-323b-3p, hsa-miR-128-3p, age, hsa-miR-361-5p, hsa-miR-1260b, hsa-miR-532-3p | 81.73 | 88.72 | 80 |
| 1799 | hsa-miR-550a-3-5p, hsa-miR-423-5p, hsa-miR-1301-3p, hsa-miR-29c-5p, hsa-miR-326, hsa-miR-2110, hsa-miR-197-3p, hsa-miR-6803-3p, hsa-miR-323b-3p, age, hsa-miR-1260b, hsa-miR-579-3p, hsa-miR-15b-3p | 81.73 | 87.48 | 83.33 |
| 1800 | hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 81.73 | 87.28 | 83.33 |
| 1801 | hsa-miR-30b-5p, hsa-miR-423-5p, hsa-miR-29c-5p, hsa-miR-326, hsa-miR-4306, hsa-miR-423-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 81.73 | 87.04 | 83.33 |
| 1802 | hsa-miR-550a-3-5p, hsa-miR-181c-3p, hsa-miR-202-5p, hsa-miR-423-5p, hsa-miR-29c-5p, hsa-miR-326, hsa-miR-197-3p, hsa-miR-130a-3p, hsa-miR-4306, hsa-miR-423-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 81.69 | 91.19 | 81.67 |
| 1803 | hsa-miR-30b-5p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 81.69 | 86.39 | 83.33 |
| 1804 | hsa-miR-550a-3-5p, hsa-miR-423-5p, hsa-miR-29c-5p, hsa-miR-326, hsa-miR-197-3p, hsa-miR-18a-5p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p | 81.69 | 89.93 | 85 |
| 1805 | hsa-miR-550a-3-5p, hsa-miR-423-5p, hsa-miR-1301-3p, hsa-miR-29c-5p, hsa-miR-326, hsa-miR-423-3p, hsa-miR-664a-3p, hsa-miR-323b-3p, age, hsa-miR-1260b, hsa-miR-579-3p, hsa-miR-532-3p, hsa-miR-15b-3p | 81.69 | 89.28 | 81.67 |
| 1806 | hsa-miR-550a-3-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-128-3p, hsa-miR-494-3p, age, hsa-miR-361-5p, hsa-miR-1260b, hsa-miR-579-3p, hsa-miR-532-3p, hsa-miR-15b-3p | 81.69 | 87.5 | 83.33 |
| 1807 | hsa-miR-550a-3-5p, hsa-miR-423-5p, hsa-miR-29c-5p, hsa-miR-326, hsa-miR-197-3p, hsa-miR-101-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p | 81.69 | 89.22 | 81.67 |
| 1808 | hsa-miR-550a-3-5p, hsa-miR-1301-3p, hsa-miR-197-3p, hsa-miR-101-3p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p | 81.69 | 85.89 | 85 |
| 1809 | hsa-miR-550a-3-5p, hsa-miR-423-5p, hsa-miR-29c-5p, hsa-miR-197-3p, hsa-miR-101-3p, hsa-miR-423-3p, hsa-miR-6803-3p, hsa-miR-664a-3p, hsa-miR-323b-3p, age, hsa-miR-1260b, hsa-miR-579-3p, hsa-miR-532-3p | 81.65 | 87.69 | 80 |
| 1810 | hsa-miR-550a-3-5p, hsa-miR-1301-3p, hsa-miR-197-3p, hsa-miR-101-3p, hsa-miR-4306, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 81.65 | 86.17 | 86.67 |
| 1811 | hsa-miR-550a-3-5p, hsa-miR-423-5p, hsa-miR-29c-5p, hsa-miR-197-3p, hsa-miR-4306, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p | 81.65 | 86.7 | 81.67 |
| 1812 | hsa-miR-550a-3-5p, hsa-miR-423-5p, hsa-miR-1301-3p, hsa-miR-326, hsa-miR-197-3p, hsa-miR-18a-5p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p, hsa-miR-7-5p | 81.65 | 92 | 81.67 |
| 1813 | hsa-miR-550a-3-5p, hsa-miR-181c-3p, hsa-miR-202-5p, hsa-miR-423-5p, hsa-miR-29c-5p, hsa-miR-326, hsa-miR-197-3p, hsa-miR-130a-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 81.65 | 90.15 | 83.33 |
| 1814 | hsa-miR-550a-3-5p, hsa-miR-181c-3p, hsa-miR-202-5p, hsa-miR-423-5p, hsa-miR-1301-3p, hsa-miR-326, hsa-miR-2110, hsa-miR-197-3p, hsa-miR-18a-5p, hsa-miR-130a-3p, hsa-miR-4306, hsa-miR-6803-3p, hsa-miR-323b-3p, age, hsa-miR-1260b, hsa-miR-579-3p, hsa-miR-15b-3p | 81.65 | 88.15 | 83.33 |
| 1815 | hsa-miR-550a-3-5p, hsa-miR-30b-5p, hsa-miR-423-5p, hsa-miR-326, hsa-miR-197-3p, hsa-miR-18a-5p, hsa-miR-4306, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 81.65 | 88.93 | 85 |
| 1816 | hsa-miR-550a-3-5p, hsa-miR-423-5p, hsa-miR-1301-3p, hsa-miR-326, hsa-miR-197-3p, hsa-miR-18a-5p, hsa-miR-4306, hsa-miR-423-3p, hsa-miR-6803-3p, hsa-miR-664a-3p, age, hsa-miR-1260b, hsa-miR-579-3p, hsa-miR-532-3p, hsa-miR-15b-3p, hsa-miR-7-5p | 81.65 | 88.5 | 83.33 |
| 1817 | hsa-miR-181c-3p, hsa-miR-202-5p, hsa-miR-1301-3p, hsa-miR-29c-5p, hsa-miR-326, hsa-miR-2110, hsa-miR-4306, hsa-miR-423-3p, hsa-miR-323b-3p, age, hsa-miR-1260b, hsa-miR-579-3p, hsa-miR-15b-3p | 81.6 | 86.13 | 81.67 |
| 1818 | hsa-miR-550a-3-5p, hsa-miR-29c-5p, hsa-miR-326, hsa-miR-197-3p, hsa-miR-18a-5p, hsa-miR-4306, hsa-miR-423-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 81.6 | 91.35 | 81.67 |
| 1819 | hsa-miR-550a-3-5p, hsa-miR-181c-3p, hsa-miR-202-5p, hsa-miR-423-5p, hsa-miR-1301-3p, hsa-miR-29c-5p, hsa-miR-2110, hsa-miR-197-3p, hsa-miR-18a-5p, hsa-miR-130a-3p, hsa-miR-423-3p, hsa-miR-6803-3p, hsa-miR-128-3p, age, hsa-miR-361-5p, hsa-miR-1260b, hsa-miR-579-3p, hsa-miR-7-5p | 81.6 | 88.22 | 81.67 |
| 1820 | hsa-miR-550a-3-5p, hsa-miR-30b-5p, hsa-miR-29c-5p, hsa-miR-326, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 80.87 | 83.78 | 80 |
| 1821 | hsa-miR-30b-5p, hsa-miR-29c-5p, hsa-miR-326, hsa-miR-4306, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 80.82 | 86.56 | 81.67 |
| 1822 | hsa-miR-550a-3-5p, hsa-miR-423-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-128-3p, age, hsa-miR-361-5p, hsa-miR-1260b, hsa-miR-15b-3p | 80.82 | 88.09 | 78.33 |
| 1823 | hsa-miR-550a-3-5p, hsa-miR-30b-5p, hsa-miR-181c-3p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 80.82 | 85.43 | 83.33 |
| 1824 | hsa-miR-423-5p, hsa-miR-1301-3p, hsa-miR-326, hsa-miR-18a-5p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p | 80.78 | 87.11 | 81.67 |
| 1825 | hsa-miR-550a-3-5p, hsa-miR-423-5p, hsa-miR-1301-3p, hsa-miR-29c-5p, hsa-miR-197-3p, hsa-miR-101-3p, hsa-miR-18a-5p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p | 80.78 | 86 | 83.33 |
| 1826 | hsa-miR-550a-3-5p, hsa-miR-423-5p, hsa-miR-326, hsa-miR-197-3p, hsa-miR-18a-5p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 80.78 | 90.46 | 85 |
| 1827 | hsa-miR-550a-3-5p, hsa-miR-181c-3p, hsa-miR-202-5p, hsa-miR-1301-3p, hsa-miR-326, hsa-miR-197-3p, hsa-miR-4306, hsa-miR-6803-3p, hsa-miR-664a-3p, hsa-miR-323b-3p, hsa-miR-128-3p, hsa-miR-494-3p, age, hsa-miR-361-5p, hsa-miR-1260b | 80.78 | 83.74 | 83.33 |
| 1828 | hsa-miR-550a-3-5p, hsa-miR-423-5p, hsa-miR-1301-3p, hsa-miR-29c-5p, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-361-5p, hsa-miR-532-3p | 80.78 | 84.65 | 85 |
| 1829 | hsa-miR-550a-3-5p, hsa-miR-181c-3p, hsa-miR-202-5p, hsa-miR-1301-3p, hsa-miR-29c-5p, hsa-miR-326, hsa-miR-197-3p, hsa-miR-130a-3p, hsa-miR-4306, hsa-miR-6803-3p, hsa-miR-664a-3p, hsa-miR-323b-3p, hsa-miR-128-3p, age, hsa-miR-361-5p, hsa-miR-1260b, hsa-miR-579-3p, hsa-miR-532-3p, hsa-miR-15b-3p | 80.78 | 88.56 | 85 |
| 1830 | hsa-miR-550a-3-5p, hsa-miR-1301-3p, hsa-miR-29c-5p, hsa-miR-197-3p, hsa-miR-4306, hsa-miR-6803-3p, hsa-miR-664a-3p, hsa-miR-323b-3p, hsa-miR-128-3p, hsa-miR-361-5p, hsa-miR-15b-3p | 80.78 | 83.37 | 83.33 |
| 1831 | hsa-miR-1301-3p, hsa-miR-29c-5p, hsa-miR-326, hsa-miR-423-3p, hsa-miR-664a-3p, hsa-miR-323b-3p, age, hsa-miR-1260b, hsa-miR-579-3p, hsa-miR-532-3p, hsa-miR-15b-3p | 80.78 | 85.7 | 81.67 |
| 1832 | hsa-miR-30b-5p, hsa-miR-29c-5p, hsa-miR-326, hsa-miR-4306, hsa-miR-423-3p, hsa-miR-664a-3p, hsa-miR-323b-3p, hsa-miR-494-3p, age, hsa-miR-361-5p, hsa-miR-1260b, hsa-miR-579-3p, hsa-miR-532-3p, hsa-miR-15b-3p | 80.78 | 88.24 | 81.67 |
| 1833 | hsa-miR-550a-3-5p, hsa-miR-30b-5p, hsa-miR-1301-3p, hsa-miR-29c-5p, hsa-miR-326, hsa-miR-197-3p, hsa-miR-4306, hsa-miR-6803-3p, hsa-miR-664a-3p, hsa-miR-323b-3p, hsa-miR-128-3p, hsa-miR-494-3p, hsa-miR-361-5p, hsa-miR-579-3p, hsa-miR-532-3p, hsa-miR-15b-3p | 80.78 | 83.46 | 86.67 |
| 1834 | hsa-miR-423-5p, hsa-miR-29c-5p, hsa-miR-326, hsa-miR-2110, hsa-miR-197-3p, hsa-miR-18a-5p, hsa-miR-323b-3p, hsa-miR-128-3p, age, hsa-miR-361-5p, hsa-miR-1260b, hsa-miR-532-3p, hsa-miR-15b-3p, hsa-miR-7-5p | 80.78 | 88.24 | 83.33 |
| 1835 | hsa-miR-550a-3-5p, hsa-miR-181c-3p, hsa-miR-202-5p, hsa-miR-423-5p, hsa-miR-29c-5p, hsa-miR-326, hsa-miR-2110, hsa-miR-197-3p, hsa-miR-423-3p, hsa-miR-6803-3p, hsa-miR-323b-3p, hsa-miR-494-3p, age, hsa-miR-1260b, hsa-miR-579-3p, hsa-miR-15b-3p | 80.78 | 88.31 | 80 |
| 1836 | hsa-miR-550a-3-5p, hsa-miR-423-5p, hsa-miR-326, hsa-miR-18a-5p, hsa-miR-423-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 80.78 | 87.76 | 78.33 |
| 1837 | hsa-miR-550a-3-5p, hsa-miR-423-5p, hsa-miR-1301-3p, hsa-miR-326, hsa-miR-197-3p, hsa-miR-18a-5p, hsa-miR-423-3p, hsa-miR-6803-3p, hsa-miR-664a-3p, hsa-miR-323b-3p, age, hsa-miR-1260b, hsa-miR-579-3p, hsa-miR-532-3p, hsa-miR-15b-3p | 80.78 | 89.78 | 81.67 |
| 1838 | hsa-miR-550a-3-5p, hsa-miR-181c-3p, hsa-miR-423-5p, hsa-miR-1301-3p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-128-3p, age, hsa-miR-361-5p, hsa-miR-1260b, hsa-miR-532-3p | 80.74 | 89.31 | 81.67 |
| 1839 | hsa-miR-550a-3-5p, hsa-miR-181c-3p, hsa-miR-423-5p, hsa-miR-326, hsa-miR-197-3p, hsa-miR-18a-5p, hsa-miR-130a-3p, hsa-miR-4306, hsa-miR-423-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 80.74 | 90.2 | 81.67 |
| 1840 | hsa-miR-550a-3-5p, hsa-miR-181c-3p, hsa-miR-202-5p, hsa-miR-423-5p, hsa-miR-29c-5p, hsa-miR-326, hsa-miR-2110, hsa-miR-197-3p, hsa-miR-130a-3p, hsa-miR-6803-3p, hsa-miR-494-3p, age, hsa-miR-1260b, hsa-miR-579-3p, hsa-miR-15b-3p, hsa-miR-7-5p | 80.74 | 88.13 | 83.33 |
| 1841 | hsa-miR-550a-3-5p, hsa-miR-1301-3p, hsa-miR-29c-5p, hsa-miR-2110, hsa-miR-197-3p, hsa-miR-130a-3p, hsa-miR-4306, hsa-miR-423-3p, hsa-miR-6803-3p, hsa-miR-664a-3p, hsa-miR-323b-3p, hsa-miR-128-3p, age, hsa-miR-361-5p, hsa-miR-579-3p, hsa-miR-532-3p, hsa-miR-15b-3p | 80.74 | 88.89 | 81.67 |
| 1842 | hsa-miR-550a-3-5p, hsa-miR-181c-3p, hsa-miR-202-5p, hsa-miR-423-5p, hsa-miR-326, hsa-miR-2110, hsa-miR-197-3p, hsa-miR-18a-5p, hsa-miR-130a-3p, hsa-miR-4306, hsa-miR-6803-3p, hsa-miR-323b-3p, hsa-miR-494-3p, age, hsa-miR-1260b, hsa-miR-579-3p, hsa-miR-15b-3p | 80.74 | 88.48 | 81.67 |
| 1843 | hsa-miR-550a-3-5p, hsa-miR-423-5p, hsa-miR-1301-3p, hsa-miR-29c-5p, hsa-miR-197-3p, hsa-miR-101-3p, hsa-miR-423-3p, hsa-miR-6803-3p, hsa-miR-664a-3p, hsa-miR-323b-3p, age, hsa-miR-1260b, hsa-miR-579-3p, hsa-miR-532-3p | 80.69 | 87.65 | 81.67 |
| 1844 | hsa-miR-550a-3-5p, hsa-miR-181c-3p, hsa-miR-29c-5p, hsa-miR-326, hsa-miR-197-3p, hsa-miR-130a-3p, hsa-miR-4306, hsa-miR-423-3p, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 80.69 | 91.22 | 81.67 |
| 1845 | hsa-miR-550a-3-5p, hsa-miR-181c-3p, hsa-miR-29c-5p, hsa-miR-326, hsa-miR-197-3p, hsa-miR-130a-3p, hsa-miR-4306, hsa-miR-423-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 80.69 | 89.85 | 81.67 |
| 1846 | hsa-miR-550a-3-5p, hsa-miR-30b-5p, hsa-miR-1301-3p, hsa-miR-29c-5p, hsa-miR-326, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 79.96 | 85.61 | 81.67 |
| 1847 | hsa-miR-30b-5p, hsa-miR-423-5p, hsa-miR-29c-5p, hsa-miR-326, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 79.91 | 86.43 | 81.67 |
| 1848 | hsa-miR-550a-3-5p, hsa-miR-181c-3p, hsa-miR-423-5p, hsa-miR-1301-3p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-361-5p, hsa-miR-1260b, hsa-miR-579-3p, hsa-miR-532-3p | 79.91 | 88.56 | 81.67 |
| 1849 | hsa-miR-550a-3-5p, hsa-miR-423-5p, hsa-miR-29c-5p, hsa-miR-326, hsa-miR-18a-5p, hsa-miR-423-3p, hsa-miR-664a-3p, hsa-miR-323b-3p, hsa-miR-128-3p, hsa-miR-494-3p, age, hsa-miR-361-5p, hsa-miR-1260b, hsa-miR-579-3p, hsa-miR-532-3p, hsa-miR-15b-3p | 79.91 | 84.81 | 85 |
| 1850 | hsa-miR-30b-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-128-3p, hsa-miR-494-3p, hsa-miR-532-3p, hsa-miR-15b-3p | 79.91 | 81.26 | 85 |
| 1851 | hsa-miR-550a-3-5p, hsa-miR-423-5p, hsa-miR-29c-5p, hsa-miR-326, hsa-miR-2110, hsa-miR-197-3p, hsa-miR-423-3p, hsa-miR-6803-3p, hsa-miR-323b-3p, hsa-miR-494-3p, age, hsa-miR-1260b, hsa-miR-579-3p, hsa-miR-15b-3p | 79.91 | 87.04 | 80 |
| 1852 | hsa-miR-550a-3-5p, hsa-miR-423-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-128-3p, hsa-miR-494-3p, hsa-miR-532-3p, hsa-miR-15b-3p | 79.91 | 83.74 | 86.67 |
| 1853 | hsa-miR-550a-3-5p, hsa-miR-423-5p, hsa-miR-1301-3p, hsa-miR-326, hsa-miR-197-3p, hsa-miR-18a-5p, hsa-miR-4306, hsa-miR-423-3p, hsa-miR-6803-3p, hsa-miR-664a-3p, hsa-miR-323b-3p, hsa-miR-128-3p, hsa-miR-532-3p, hsa-miR-15b-3p | 79.91 | 85.61 | 83.33 |
| 1854 | hsa-miR-550a-3-5p, hsa-miR-423-5p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-532-3p, hsa-miR-15b-3p | 79.91 | 86.44 | 83.33 |
| 1855 | hsa-miR-550a-3-5p, hsa-miR-423-5p, hsa-miR-29c-5p, hsa-miR-326, hsa-miR-18a-5p, hsa-miR-423-3p, hsa-miR-664a-3p, hsa-miR-323b-3p, hsa-miR-494-3p, age, hsa-miR-361-5p, hsa-miR-1260b, hsa-miR-579-3p, hsa-miR-532-3p, hsa-miR-15b-3p | 79.91 | 88.44 | 81.67 |
| 1856 | hsa-miR-550a-3-5p, hsa-miR-423-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-494-3p, hsa-miR-532-3p, hsa-miR-15b-3p | 79.91 | 86.59 | 83.33 |
| 1857 | hsa-miR-550a-3-5p, hsa-miR-423-5p, hsa-miR-29c-5p, hsa-miR-326, hsa-miR-2110, hsa-miR-197-3p, hsa-miR-6803-3p, hsa-miR-323b-3p, hsa-miR-494-3p, age, hsa-miR-1260b, hsa-miR-579-3p, hsa-miR-15b-3p | 79.91 | 86.7 | 80 |
| 1858 | hsa-miR-30b-5p, hsa-miR-423-5p, hsa-miR-29c-5p, hsa-miR-326, hsa-miR-423-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 79.91 | 86.06 | 80 |
| 1859 | hsa-miR-550a-3-5p, hsa-miR-181c-3p, hsa-miR-1301-3p, hsa-miR-29c-5p, hsa-miR-326, hsa-miR-2110, hsa-miR-197-3p, hsa-miR-130a-3p, hsa-miR-423-3p, hsa-miR-6803-3p, hsa-miR-664a-3p, hsa-miR-128-3p, hsa-miR-361-5p, hsa-miR-579-3p, hsa-miR-15b-3p | 79.87 | 85.91 | 86.67 |
| 1860 | hsa-miR-30b-5p, hsa-miR-181c-3p, hsa-miR-423-5p, hsa-miR-1301-3p, hsa-miR-326, hsa-miR-197-3p, hsa-miR-130a-3p, hsa-miR-4306, hsa-miR-423-3p, hsa-miR-6803-3p, hsa-miR-664a-3p, hsa-miR-323b-3p, hsa-miR-128-3p, hsa-miR-494-3p, age, hsa-miR-1260b, hsa-miR-579-3p, hsa-miR-532-3p | 79.87 | 86.94 | 83.33 |
| 1861 | hsa-miR-550a-3-5p, hsa-miR-30b-5p, hsa-miR-1301-3p, hsa-miR-29c-5p, hsa-miR-197-3p, hsa-miR-4306, hsa-miR-423-3p, hsa-miR-6803-3p, hsa-miR-664a-3p, hsa-miR-128-3p, hsa-miR-494-3p, hsa-miR-361-5p, hsa-miR-579-3p, hsa-miR-532-3p, hsa-miR-15b-3p | 79.87 | 83.65 | 85 |
| 1862 | hsa-miR-423-5p, hsa-miR-1301-3p, hsa-miR-29c-5p, hsa-miR-326, hsa-miR-423-3p, hsa-miR-664a-3p, hsa-miR-323b-3p, age, hsa-miR-1260b, hsa-miR-579-3p, hsa-miR-532-3p, hsa-miR-15b-3p | 79.87 | 86.3 | 78.33 |
| 1863 | hsa-miR-202-5p, hsa-miR-1301-3p, hsa-miR-29c-5p, hsa-miR-326, hsa-miR-2110, hsa-miR-423-3p, hsa-miR-6803-3p, hsa-miR-323b-3p, age, hsa-miR-1260b, hsa-miR-579-3p, hsa-miR-15b-3p | 79.87 | 85.65 | 80 |
| 1864 | hsa-miR-550a-3-5p, hsa-miR-423-5p, hsa-miR-1301-3p, hsa-miR-197-3p, hsa-miR-101-3p, hsa-miR-18a-5p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p | 79.87 | 84.72 | 81.67 |
| 1865 | hsa-miR-550a-3-5p, hsa-miR-423-5p, hsa-miR-1301-3p, hsa-miR-326, hsa-miR-197-3p, hsa-miR-18a-5p, hsa-miR-130a-3p, hsa-miR-4306, hsa-miR-423-3p, hsa-miR-6803-3p, hsa-miR-664a-3p, age, hsa-miR-361-5p, hsa-miR-1260b, hsa-miR-579-3p, hsa-miR-532-3p, hsa-miR-15b-3p, hsa-miR-7-5p | 79.87 | 89.59 | 81.67 |
| 1866 | hsa-miR-550a-3-5p, hsa-miR-423-5p, hsa-miR-29c-5p, hsa-miR-197-3p, hsa-miR-101-3p, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p | 79.87 | 85.56 | 81.67 |
| 1867 | hsa-miR-550a-3-5p, hsa-miR-181c-3p, hsa-miR-202-5p, hsa-miR-423-5p, hsa-miR-1301-3p, hsa-miR-326, hsa-miR-197-3p, hsa-miR-18a-5p, hsa-miR-130a-3p, hsa-miR-4306, hsa-miR-6803-3p, age, hsa-miR-1260b, hsa-miR-579-3p, hsa-miR-532-3p, hsa-miR-15b-3p | 79.83 | 89.17 | 81.67 |
| 1868 | hsa-miR-550a-3-5p, hsa-miR-423-5p, hsa-miR-326, hsa-miR-197-3p, hsa-miR-18a-5p, hsa-miR-423-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 79.83 | 89.61 | 81.67 |
| 1869 | hsa-miR-550a-3-5p, hsa-miR-30b-5p, hsa-miR-423-5p, hsa-miR-1301-3p, hsa-miR-197-3p, hsa-miR-101-3p, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p | 79.83 | 83.8 | 85 |
| 1870 | hsa-miR-550a-3-5p, hsa-miR-181c-3p, hsa-miR-202-5p, hsa-miR-423-5p, hsa-miR-1301-3p, hsa-miR-326, hsa-miR-2110, hsa-miR-197-3p, hsa-miR-18a-5p, hsa-miR-4306, hsa-miR-6803-3p, hsa-miR-323b-3p, age, hsa-miR-1260b, hsa-miR-579-3p, hsa-miR-15b-3p | 79.83 | 87.94 | 80 |
| 1871 | hsa-miR-550a-3-5p, hsa-miR-181c-3p, hsa-miR-202-5p, hsa-miR-423-5p, hsa-miR-29c-5p, hsa-miR-326, hsa-miR-2110, hsa-miR-197-3p, hsa-miR-6803-3p, hsa-miR-323b-3p, hsa-miR-494-3p, age, hsa-miR-1260b, hsa-miR-579-3p, hsa-miR-15b-3p | 79.83 | 89.2 | 81.67 |
| 1872 | hsa-miR-550a-3-5p, hsa-miR-181c-3p, hsa-miR-202-5p, hsa-miR-1301-3p, hsa-miR-29c-5p, hsa-miR-326, hsa-miR-2110, hsa-miR-4306, hsa-miR-423-3p, hsa-miR-323b-3p, age, hsa-miR-1260b, hsa-miR-579-3p, hsa-miR-15b-3p | 79.83 | 88.35 | 81.67 |
| 1873 | hsa-miR-550a-3-5p, hsa-miR-181c-3p, hsa-miR-29c-5p, hsa-miR-326, hsa-miR-197-3p, hsa-miR-18a-5p, hsa-miR-4306, hsa-miR-423-3p, hsa-miR-6803-3p, hsa-miR-664a-3p, hsa-miR-128-3p, hsa-miR-361-5p, hsa-miR-532-3p, hsa-miR-15b-3p, hsa-miR-7-5p | 79.83 | 87.09 | 85 |
| 1874 | hsa-miR-550a-3-5p, hsa-miR-1301-3p, hsa-miR-29c-5p, hsa-miR-197-3p, hsa-miR-101-3p, hsa-miR-423-3p, hsa-miR-6803-3p, hsa-miR-664a-3p, hsa-miR-323b-3p, hsa-miR-128-3p, hsa-miR-361-5p, hsa-miR-532-3p | 79.78 | 85.17 | 81.67 |
| 1875 | hsa-miR-550a-3-5p, hsa-miR-181c-3p, hsa-miR-202-5p, hsa-miR-423-5p, hsa-miR-29c-5p, hsa-miR-326, hsa-miR-197-3p, hsa-miR-130a-3p, hsa-miR-6803-3p, hsa-miR-494-3p, age, hsa-miR-1260b, hsa-miR-579-3p, hsa-miR-532-3p, hsa-miR-15b-3p, hsa-miR-7-5p | 79.78 | 87.61 | 78.33 |
| 1876 | hsa-miR-550a-3-5p, hsa-miR-423-5p, hsa-miR-1301-3p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-361-5p, hsa-miR-532-3p, hsa-miR-15b-3p | 79 | 85.63 | 80 |
| 1877 | hsa-miR-550a-3-5p, hsa-miR-423-5p, hsa-miR-326, hsa-miR-4306, hsa-miR-423-3p, hsa-miR-6803-3p, hsa-miR-323b-3p, hsa-miR-128-3p, hsa-miR-494-3p, age, hsa-miR-361-5p, hsa-miR-579-3p, hsa-miR-532-3p, hsa-miR-15b-3p | 79 | 85.61 | 81.67 |
| 1878 | hsa-miR-550a-3-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-128-3p, hsa-miR-494-3p, hsa-miR-532-3p, hsa-miR-15b-3p | 79 | 85.91 | 83.33 |
| 1879 | hsa-miR-550a-3-5p, hsa-miR-423-5p, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p | 79 | 83.2 | 81.67 |
| 1880 | hsa-miR-30b-5p, hsa-miR-29c-5p, hsa-miR-326, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 79 | 81.44 | 83.33 |
| 1881 | hsa-miR-30b-5p, hsa-miR-326, hsa-miR-18a-5p, hsa-miR-423-3p, hsa-miR-664a-3p, hsa-miR-323b-3p, hsa-miR-494-3p, age, hsa-miR-361-5p, hsa-miR-1260b, hsa-miR-579-3p, hsa-miR-532-3p, hsa-miR-15b-3p | 79 | 86.81 | 80 |
| 1882 | hsa-miR-550a-3-5p, hsa-miR-423-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-128-3p, hsa-miR-494-3p, hsa-miR-579-3p, hsa-miR-532-3p, hsa-miR-15b-3p | 79 | 83.59 | 85 |
| 1883 | hsa-miR-30b-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-494-3p, hsa-miR-532-3p, hsa-miR-15b-3p | 78.96 | 79.7 | 81.67 |
| 1884 | hsa-miR-550a-3-5p, hsa-miR-423-5p, hsa-miR-1301-3p, hsa-miR-29c-5p, hsa-miR-197-3p, hsa-miR-101-3p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p | 78.96 | 86.78 | 80 |
| 1885 | hsa-miR-550a-3-5p, hsa-miR-423-5p, hsa-miR-1301-3p, hsa-miR-197-3p, hsa-miR-101-3p, hsa-miR-4306, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p, hsa-miR-15b-3p | 78.96 | 84.56 | 81.67 |
| 1886 | hsa-miR-550a-3-5p, hsa-miR-423-5p, hsa-miR-1301-3p, hsa-miR-326, hsa-miR-2110, hsa-miR-197-3p, hsa-miR-18a-5p, hsa-miR-423-3p, hsa-miR-664a-3p, hsa-miR-323b-3p, age, hsa-miR-1260b, hsa-miR-579-3p, hsa-miR-15b-3p | 78.96 | 87.15 | 78.33 |
| 1887 | hsa-miR-550a-3-5p, hsa-miR-423-5p, hsa-miR-1301-3p, hsa-miR-197-3p, hsa-miR-101-3p, hsa-miR-4306, hsa-miR-423-3p, hsa-miR-6803-3p, hsa-miR-664a-3p, hsa-miR-323b-3p, age, hsa-miR-1260b, hsa-miR-579-3p, hsa-miR-532-3p | 78.96 | 86.39 | 81.67 |
| 1888 | hsa-miR-30b-5p, hsa-miR-29c-5p, hsa-miR-326, hsa-miR-197-3p, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 78.96 | 82.61 | 83.33 |
| 1889 | hsa-miR-550a-3-5p, hsa-miR-30b-5p, hsa-miR-181c-3p, hsa-miR-423-5p, hsa-miR-326, hsa-miR-4306, hsa-miR-664a-3p, hsa-miR-128-3p, age, hsa-miR-361-5p, hsa-miR-1260b, hsa-miR-532-3p | 78.92 | 84.81 | 80 |
| 1890 | hsa-miR-550a-3-5p, hsa-miR-181c-3p, hsa-miR-202-5p, hsa-miR-423-5p, hsa-miR-29c-5p, hsa-miR-326, hsa-miR-197-3p, hsa-miR-130a-3p, hsa-miR-6803-3p, hsa-miR-323b-3p, hsa-miR-494-3p, age, hsa-miR-1260b, hsa-miR-579-3p, hsa-miR-532-3p, hsa-miR-15b-3p | 78.92 | 86.52 | 80 |
| 1891 | hsa-miR-550a-3-5p, hsa-miR-423-5p, hsa-miR-29c-5p, hsa-miR-197-3p, hsa-miR-423-3p, hsa-miR-6803-3p, hsa-miR-664a-3p, hsa-miR-323b-3p, hsa-miR-494-3p, age, hsa-miR-579-3p, hsa-miR-532-3p | 78.87 | 86.83 | 80 |
| 1892 | hsa-miR-550a-3-5p, hsa-miR-202-5p, hsa-miR-1301-3p, hsa-miR-29c-5p, hsa-miR-2110, hsa-miR-197-3p, hsa-miR-18a-5p, hsa-miR-130a-3p, hsa-miR-423-3p, hsa-miR-6803-3p, hsa-miR-664a-3p, hsa-miR-128-3p, age, hsa-miR-361-5p, hsa-miR-579-3p, hsa-miR-15b-3p, hsa-miR-7-5p | 78.87 | 84.19 | 81.67 |
| 1893 | hsa-miR-550a-3-5p, hsa-miR-29c-5p, hsa-miR-326, hsa-miR-197-3p, hsa-miR-130a-3p, hsa-miR-4306, hsa-miR-6803-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 78.87 | 87.26 | 80 |
| 1894 | hsa-miR-30b-5p, hsa-miR-181c-3p, hsa-miR-202-5p, hsa-miR-423-5p, hsa-miR-1301-3p, hsa-miR-29c-5p, hsa-miR-197-3p, hsa-miR-101-3p, hsa-miR-664a-3p, hsa-miR-494-3p, age, hsa-miR-1260b, hsa-miR-579-3p, hsa-miR-532-3p, hsa-miR-15b-3p, hsa-miR-7-5p | 78.79 | 87.35 | 76.67 |
| 1895 | hsa-miR-423-5p, hsa-miR-1301-3p, hsa-miR-326, hsa-miR-197-3p, hsa-miR-18a-5p, hsa-miR-4306, hsa-miR-423-3p, hsa-miR-6803-3p, hsa-miR-664a-3p, hsa-miR-323b-3p, hsa-miR-128-3p, hsa-miR-361-5p, hsa-miR-532-3p, hsa-miR-15b-3p | 78.1 | 84.65 | 78.33 |
| 1896 | hsa-miR-550a-3-5p, hsa-miR-30b-5p, hsa-miR-326, hsa-miR-197-3p, hsa-miR-4306, hsa-miR-664a-3p, hsa-miR-323b-3p, hsa-miR-128-3p, hsa-miR-494-3p, hsa-miR-361-5p, hsa-miR-532-3p, hsa-miR-15b-3p | 78.1 | 80.59 | 83.33 |
| 1897 | hsa-miR-550a-3-5p, hsa-miR-202-5p, hsa-miR-29c-5p, hsa-miR-326, hsa-miR-197-3p, hsa-miR-101-3p, hsa-miR-130a-3p, hsa-miR-4306, hsa-miR-423-3p, hsa-miR-6803-3p, hsa-miR-664a-3p, hsa-miR-323b-3p, hsa-miR-128-3p, hsa-miR-532-3p, hsa-miR-15b-3p | 78.1 | 84.06 | 85 |
| 1898 | hsa-miR-550a-3-5p, hsa-miR-181c-3p, hsa-miR-29c-5p, hsa-miR-326, hsa-miR-197-3p, hsa-miR-4306, hsa-miR-423-3p, hsa-miR-664a-3p, hsa-miR-323b-3p, hsa-miR-128-3p, hsa-miR-494-3p, hsa-miR-361-5p, hsa-miR-1260b, hsa-miR-532-3p, hsa-miR-15b-3p | 78.1 | 85.17 | 81.67 |
| 1899 | hsa-miR-30b-5p, hsa-miR-29c-5p, hsa-miR-326, hsa-miR-423-3p, hsa-miR-664a-3p, hsa-miR-323b-3p, hsa-miR-494-3p, age, hsa-miR-361-5p, hsa-miR-1260b, hsa-miR-579-3p, hsa-miR-532-3p, hsa-miR-15b-3p | 78.1 | 86.63 | 76.67 |
| 1900 | hsa-miR-550a-3-5p, hsa-miR-30b-5p, hsa-miR-29c-5p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p | 78.05 | 84.43 | 83.33 |
| 1901 | hsa-miR-550a-3-5p, hsa-miR-29c-5p, hsa-miR-326, hsa-miR-197-3p, hsa-miR-18a-5p, hsa-miR-4306, hsa-miR-6803-3p, hsa-miR-664a-3p, hsa-miR-323b-3p, age, hsa-miR-1260b, hsa-miR-579-3p, hsa-miR-532-3p | 78.05 | 87.48 | 81.67 |
| 1902 | hsa-miR-30b-5p, hsa-miR-423-5p, hsa-miR-1301-3p, hsa-miR-29c-5p, hsa-miR-326, hsa-miR-18a-5p, hsa-miR-130a-3p, hsa-miR-4306, hsa-miR-323b-3p, hsa-miR-128-3p, age, hsa-miR-361-5p, hsa-miR-1260b, hsa-miR-579-3p, hsa-miR-532-3p, hsa-miR-7-5p | 78.01 | 84.5 | 78.33 |
| 1903 | hsa-miR-550a-3-5p, hsa-miR-1301-3p, hsa-miR-326, hsa-miR-197-3p, hsa-miR-4306, hsa-miR-423-3p, hsa-miR-6803-3p, hsa-miR-664a-3p, hsa-miR-323b-3p, hsa-miR-128-3p, hsa-miR-361-5p, hsa-miR-15b-3p | 78.01 | 81.85 | 81.67 |
| 1904 | hsa-miR-550a-3-5p, hsa-miR-423-5p, hsa-miR-1301-3p, hsa-miR-29c-5p, hsa-miR-197-3p, hsa-miR-101-3p, hsa-miR-4306, hsa-miR-423-3p, hsa-miR-6803-3p, hsa-miR-664a-3p, hsa-miR-323b-3p, hsa-miR-128-3p, hsa-miR-361-5p, hsa-miR-532-3p | 78.01 | 84.37 | 80 |
| 1905 | hsa-miR-550a-3-5p, hsa-miR-423-5p, hsa-miR-1301-3p, hsa-miR-197-3p, hsa-miR-101-3p, hsa-miR-18a-5p, hsa-miR-4306, hsa-miR-423-3p, hsa-miR-6803-3p, hsa-miR-664a-3p, hsa-miR-323b-3p, hsa-miR-128-3p, hsa-miR-361-5p, hsa-miR-532-3p | 78.01 | 84.56 | 80 |
| 1906 | hsa-miR-550a-3-5p, hsa-miR-202-5p, hsa-miR-1301-3p, hsa-miR-29c-5p, hsa-miR-326, hsa-miR-130a-3p, hsa-miR-4306, hsa-miR-423-3p, hsa-miR-6803-3p, hsa-miR-664a-3p, hsa-miR-361-5p | 78.01 | 79.31 | 83.33 |
| 1907 | hsa-miR-550a-3-5p, hsa-miR-181c-3p, hsa-miR-202-5p, hsa-miR-423-5p, hsa-miR-29c-5p, hsa-miR-326, hsa-miR-2110, hsa-miR-197-3p, hsa-miR-4306, hsa-miR-6803-3p, hsa-miR-323b-3p, hsa-miR-494-3p, age, hsa-miR-1260b, hsa-miR-579-3p, hsa-miR-15b-3p | 78.01 | 87.3 | 80 |
| 1908 | hsa-miR-423-5p, hsa-miR-1301-3p, hsa-miR-29c-5p, hsa-miR-326, hsa-miR-4306, hsa-miR-423-3p, hsa-miR-664a-3p, hsa-miR-323b-3p, age, hsa-miR-1260b, hsa-miR-579-3p, hsa-miR-532-3p, hsa-miR-15b-3p | 78.01 | 86.22 | 80 |
| 1909 | hsa-miR-30b-5p, hsa-miR-1301-3p, hsa-miR-29c-5p, hsa-miR-326, hsa-miR-197-3p, hsa-miR-101-3p, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-532-3p, hsa-miR-15b-3p | 78.01 | 83.67 | 83.33 |
| 1910 | hsa-miR-550a-3-5p, hsa-miR-423-5p, hsa-miR-1301-3p, hsa-miR-29c-5p, hsa-miR-197-3p, hsa-miR-101-3p, hsa-miR-4306, hsa-miR-423-3p, hsa-miR-6803-3p, hsa-miR-664a-3p, hsa-miR-323b-3p, hsa-miR-128-3p, hsa-miR-532-3p | 77.97 | 85.43 | 81.67 |
| 1911 | hsa-miR-550a-3-5p, hsa-miR-181c-3p, hsa-miR-202-5p, hsa-miR-423-5p, hsa-miR-1301-3p, hsa-miR-29c-5p, hsa-miR-197-3p, hsa-miR-130a-3p, hsa-miR-423-3p, hsa-miR-6803-3p, hsa-miR-128-3p, age, hsa-miR-361-5p, hsa-miR-1260b, hsa-miR-15b-3p, hsa-miR-7-5p | 77.97 | 86.59 | 76.67 |
| 1912 | hsa-miR-30b-5p, hsa-miR-181c-3p, hsa-miR-423-5p, hsa-miR-1301-3p, hsa-miR-29c-5p, hsa-miR-326, hsa-miR-197-3p, hsa-miR-101-3p, hsa-miR-18a-5p, hsa-miR-130a-3p, hsa-miR-6803-3p, hsa-miR-323b-3p, hsa-miR-1260b, hsa-miR-579-3p, hsa-miR-7-5p | 77.97 | 83.8 | 78.33 |
| 1913 | hsa-miR-550a-3-5p, hsa-miR-202-5p, hsa-miR-1301-3p, hsa-miR-29c-5p, hsa-miR-326, hsa-miR-2110, hsa-miR-197-3p, hsa-miR-18a-5p, hsa-miR-423-3p, hsa-miR-6803-3p, hsa-miR-664a-3p, hsa-miR-128-3p, hsa-miR-361-5p, hsa-miR-532-3p | 77.97 | 82.22 | 81.67 |
| 1914 | hsa-miR-550a-3-5p, hsa-miR-30b-5p, hsa-miR-326, hsa-miR-101-3p, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 77.23 | 81.94 | 80 |
| 1915 | hsa-miR-30b-5p, hsa-miR-29c-5p, hsa-miR-326, hsa-miR-4306, hsa-miR-423-3p, hsa-miR-6803-3p, hsa-miR-664a-3p, hsa-miR-323b-3p, hsa-miR-494-3p, hsa-miR-532-3p, hsa-miR-15b-3p | 77.14 | 80.06 | 81.67 |
| 1916 | hsa-miR-30b-5p, hsa-miR-423-5p, hsa-miR-326, hsa-miR-18a-5p, hsa-miR-423-3p, hsa-miR-664a-3p, hsa-miR-323b-3p, hsa-miR-494-3p, age, hsa-miR-361-5p, hsa-miR-1260b, hsa-miR-579-3p, hsa-miR-532-3p, hsa-miR-15b-3p | 77.14 | 87.83 | 80 |
| 1917 | hsa-miR-423-5p, hsa-miR-1301-3p, hsa-miR-326, hsa-miR-18a-5p, hsa-miR-423-3p, hsa-miR-664a-3p, hsa-miR-323b-3p, age, hsa-miR-1260b, hsa-miR-579-3p, hsa-miR-532-3p, hsa-miR-15b-3p | 77.14 | 85.41 | 81.67 |
| 1918 | hsa-miR-550a-3-5p, hsa-miR-423-5p, hsa-miR-1301-3p, hsa-miR-326, hsa-miR-197-3p, hsa-miR-18a-5p, hsa-miR-130a-3p, hsa-miR-4306, hsa-miR-423-3p, hsa-miR-6803-3p, hsa-miR-664a-3p, hsa-miR-128-3p, hsa-miR-361-5p, hsa-miR-532-3p, hsa-miR-15b-3p, hsa-miR-7-5p | 77.14 | 83.43 | 78.33 |
| 1919 | hsa-miR-423-5p, hsa-miR-1301-3p, hsa-miR-326, hsa-miR-18a-5p, hsa-miR-423-3p, hsa-miR-664a-3p, hsa-miR-323b-3p, age, hsa-miR-361-5p, hsa-miR-1260b, hsa-miR-579-3p, hsa-miR-532-3p, hsa-miR-15b-3p | 77.14 | 87.31 | 76.67 |
| 1920 | hsa-miR-550a-3-5p, hsa-miR-181c-3p, hsa-miR-202-5p, hsa-miR-29c-5p, hsa-miR-326, hsa-miR-197-3p, hsa-miR-130a-3p, hsa-miR-423-3p, hsa-miR-6803-3p, hsa-miR-664a-3p, hsa-miR-128-3p, hsa-miR-494-3p, hsa-miR-361-5p, hsa-miR-532-3p, hsa-miR-15b-3p, hsa-miR-7-5p | 77.14 | 85.54 | 81.67 |
| 1921 | hsa-miR-550a-3-5p, hsa-miR-423-5p, hsa-miR-1301-3p, hsa-miR-326, hsa-miR-18a-5p, hsa-miR-423-3p, hsa-miR-664a-3p, hsa-miR-323b-3p, age, hsa-miR-1260b, hsa-miR-579-3p, hsa-miR-532-3p, hsa-miR-15b-3p | 77.14 | 87.11 | 75 |
| 1922 | hsa-miR-550a-3-5p, hsa-miR-423-5p, hsa-miR-29c-5p, hsa-miR-197-3p, hsa-miR-101-3p, hsa-miR-4306, hsa-miR-423-3p, hsa-miR-6803-3p, hsa-miR-664a-3p, hsa-miR-323b-3p, hsa-miR-494-3p, age, hsa-miR-1260b, hsa-miR-579-3p, hsa-miR-532-3p | 77.14 | 85.56 | 78.33 |
| 1923 | hsa-miR-30b-5p, hsa-miR-1301-3p, hsa-miR-29c-5p, hsa-miR-326, hsa-miR-18a-5p, hsa-miR-4306, hsa-miR-423-3p, hsa-miR-664a-3p, hsa-miR-323b-3p, hsa-miR-494-3p, hsa-miR-361-5p, hsa-miR-579-3p, hsa-miR-532-3p, hsa-miR-15b-3p, hsa-miR-7-5p | 77.1 | 84.44 | 80 |
| 1924 | hsa-miR-550a-3-5p, hsa-miR-30b-5p, hsa-miR-181c-3p, hsa-miR-202-5p, hsa-miR-423-5p, hsa-miR-197-3p, hsa-miR-664a-3p, hsa-miR-128-3p, hsa-miR-361-5p, hsa-miR-1260b, hsa-miR-579-3p, hsa-miR-15b-3p, hsa-miR-7-5p | 77.1 | 83.65 | 78.33 |
| 1925 | hsa-miR-30b-5p, hsa-miR-1301-3p, hsa-miR-29c-5p, hsa-miR-326, hsa-miR-197-3p, hsa-miR-101-3p, hsa-miR-4306, hsa-miR-423-3p, hsa-miR-664a-3p, hsa-miR-323b-3p, hsa-miR-494-3p, hsa-miR-532-3p, hsa-miR-15b-3p | 77.1 | 78.39 | 81.67 |
| 1926 | hsa-miR-202-5p, hsa-miR-423-5p, hsa-miR-2110, hsa-miR-130a-3p, hsa-miR-4306, hsa-miR-423-3p, hsa-miR-664a-3p, hsa-miR-323b-3p, age, hsa-miR-1260b, hsa-miR-579-3p, hsa-miR-15b-3p | 77.1 | 82.46 | 75 |
| 1927 | hsa-miR-550a-3-5p, hsa-miR-30b-5p, hsa-miR-181c-3p, hsa-miR-202-5p, hsa-miR-326, hsa-miR-2110, hsa-miR-197-3p, hsa-miR-101-3p, hsa-miR-18a-5p, hsa-miR-4306, hsa-miR-6803-3p, hsa-miR-664a-3p, age, hsa-miR-361-5p, hsa-miR-579-3p, hsa-miR-532-3p, hsa-miR-15b-3p, hsa-miR-7-5p | 77.06 | 89.8 | 78.33 |
| 1928 | hsa-miR-550a-3-5p, hsa-miR-181c-3p, hsa-miR-202-5p, hsa-miR-423-5p, hsa-miR-29c-5p, hsa-miR-101-3p, hsa-miR-130a-3p, hsa-miR-423-3p, hsa-miR-128-3p, hsa-miR-494-3p, age, hsa-miR-1260b, hsa-miR-15b-3p, hsa-miR-7-5p | 77.06 | 85.72 | 76.67 |
| 1929 | hsa-miR-550a-3-5p, hsa-miR-30b-5p, hsa-miR-29c-5p, hsa-miR-326, hsa-miR-101-3p, hsa-miR-4306, hsa-miR-423-3p, hsa-miR-6803-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 76.32 | 83.76 | 76.67 |
| 1930 | hsa-miR-550a-3-5p, hsa-miR-202-5p, hsa-miR-326, hsa-miR-197-3p, hsa-miR-101-3p, hsa-miR-130a-3p, hsa-miR-423-3p, hsa-miR-6803-3p, hsa-miR-664a-3p, hsa-miR-323b-3p, hsa-miR-128-3p, hsa-miR-361-5p, hsa-miR-532-3p, hsa-miR-15b-3p, hsa-miR-7-5p | 76.28 | 83.94 | 76.67 |
| 1931 | hsa-miR-550a-3-5p, hsa-miR-423-5p, hsa-miR-1301-3p, hsa-miR-326, hsa-miR-197-3p, hsa-miR-18a-5p, hsa-miR-4306, hsa-miR-423-3p, hsa-miR-6803-3p, hsa-miR-664a-3p, hsa-miR-128-3p, hsa-miR-361-5p, hsa-miR-532-3p, hsa-miR-15b-3p, hsa-miR-7-5p | 76.23 | 83.78 | 80 |
| 1932 | hsa-miR-550a-3-5p, hsa-miR-30b-5p, hsa-miR-202-5p, hsa-miR-29c-5p, hsa-miR-326, hsa-miR-2110, hsa-miR-197-3p, hsa-miR-130a-3p, hsa-miR-4306, hsa-miR-423-3p, hsa-miR-6803-3p, hsa-miR-323b-3p, hsa-miR-128-3p, hsa-miR-494-3p, hsa-miR-361-5p, hsa-miR-532-3p, hsa-miR-15b-3p | 76.23 | 82.54 | 81.67 |
| 1933 | hsa-miR-550a-3-5p, hsa-miR-1301-3p, hsa-miR-29c-5p, hsa-miR-326, hsa-miR-2110, hsa-miR-197-3p, hsa-miR-6803-3p, hsa-miR-323b-3p, hsa-miR-128-3p, hsa-miR-361-5p, hsa-miR-15b-3p | 76.23 | 84.09 | 78.33 |
| 1934 | hsa-miR-550a-3-5p, hsa-miR-30b-5p, hsa-miR-423-5p, hsa-miR-29c-5p, hsa-miR-326, hsa-miR-2110, hsa-miR-101-3p, hsa-miR-18a-5p, hsa-miR-6803-3p, hsa-miR-664a-3p, hsa-miR-323b-3p, hsa-miR-128-3p, hsa-miR-1260b, hsa-miR-532-3p, hsa-miR-7-5p | 76.23 | 81.52 | 78.33 |
| 1935 | hsa-miR-550a-3-5p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-4306, hsa-miR-6803-3p, hsa-miR-323b-3p, hsa-miR-128-3p, hsa-miR-361-5p, hsa-miR-15b-3p | 76.23 | 80.96 | 81.67 |
| 1936 | hsa-miR-550a-3-5p, hsa-miR-181c-3p, hsa-miR-202-5p, hsa-miR-423-5p, hsa-miR-1301-3p, hsa-miR-326, hsa-miR-2110, hsa-miR-197-3p, hsa-miR-18a-5p, hsa-miR-130a-3p, hsa-miR-4306, hsa-miR-6803-3p, hsa-miR-323b-3p, hsa-miR-128-3p, hsa-miR-361-5p, hsa-miR-15b-3p | 76.23 | 84.35 | 76.67 |
| 1937 | hsa-miR-550a-3-5p, hsa-miR-423-5p, hsa-miR-326, hsa-miR-18a-5p, hsa-miR-423-3p, hsa-miR-664a-3p, hsa-miR-323b-3p, hsa-miR-494-3p, age, hsa-miR-361-5p, hsa-miR-1260b, hsa-miR-579-3p, hsa-miR-532-3p, hsa-miR-15b-3p | 76.23 | 88.17 | 80 |
| 1938 | hsa-miR-550a-3-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-4306, hsa-miR-6803-3p, hsa-miR-323b-3p, hsa-miR-128-3p, hsa-miR-361-5p, hsa-miR-15b-3p | 76.19 | 79.41 | 80 |
| 1939 | hsa-miR-550a-3-5p, hsa-miR-1301-3p, hsa-miR-29c-5p, hsa-miR-2110, hsa-miR-197-3p, hsa-miR-18a-5p, hsa-miR-130a-3p, hsa-miR-423-3p, hsa-miR-6803-3p, hsa-miR-128-3p, hsa-miR-361-5p | 76.15 | 80.65 | 80 |
| 1940 | hsa-miR-550a-3-5p, hsa-miR-423-5p, hsa-miR-1301-3p, hsa-miR-29c-5p, hsa-miR-326, hsa-miR-2110, hsa-miR-197-3p, hsa-miR-423-3p, hsa-miR-323b-3p, hsa-miR-15b-3p | 76.15 | 81.61 | 80 |
| 1941 | hsa-miR-550a-3-5p, hsa-miR-30b-5p, hsa-miR-29c-5p, hsa-miR-326, hsa-miR-4306, hsa-miR-423-3p, hsa-miR-664a-3p, hsa-miR-323b-3p, hsa-miR-361-5p, hsa-miR-532-3p, hsa-miR-15b-3p | 75.37 | 80.35 | 78.33 |
| 1942 | hsa-miR-30b-5p, hsa-miR-29c-5p, hsa-miR-326, hsa-miR-197-3p, hsa-miR-4306, hsa-miR-664a-3p, hsa-miR-323b-3p, hsa-miR-494-3p, hsa-miR-532-3p, hsa-miR-15b-3p | 75.28 | 75.44 | 80 |
| 1943 | hsa-miR-550a-3-5p, hsa-miR-30b-5p, hsa-miR-202-5p, hsa-miR-101-3p, hsa-miR-18a-5p, hsa-miR-4306, hsa-miR-6803-3p, hsa-miR-361-5p, hsa-miR-532-3p, hsa-miR-15b-3p, hsa-miR-7-5p | 75.28 | 81.57 | 78.33 |
| 1944 | hsa-miR-30b-5p, hsa-miR-326, hsa-miR-18a-5p, hsa-miR-423-3p, hsa-miR-664a-3p, hsa-miR-323b-3p, hsa-miR-494-3p, age, hsa-miR-361-5p, hsa-miR-1260b, hsa-miR-532-3p, hsa-miR-15b-3p | 75.28 | 81.22 | 76.67 |
| 1945 | hsa-miR-550a-3-5p, hsa-miR-30b-5p, hsa-miR-1301-3p, hsa-miR-29c-5p, hsa-miR-2110, hsa-miR-197-3p, hsa-miR-18a-5p, hsa-miR-4306, hsa-miR-423-3p, hsa-miR-6803-3p, hsa-miR-323b-3p, hsa-miR-128-3p, hsa-miR-494-3p, hsa-miR-579-3p, hsa-miR-532-3p, hsa-miR-15b-3p | 75.28 | 82.94 | 80 |
| 1946 | hsa-miR-550a-3-5p, hsa-miR-181c-3p, hsa-miR-202-5p, hsa-miR-1301-3p, hsa-miR-29c-5p, hsa-miR-326, hsa-miR-2110, hsa-miR-197-3p, hsa-miR-18a-5p, hsa-miR-423-3p, hsa-miR-6803-3p, hsa-miR-128-3p, hsa-miR-361-5p, hsa-miR-1260b, hsa-miR-7-5p | 75.24 | 84.85 | 78.33 |
| 1947 | hsa-miR-202-5p, hsa-miR-423-5p, hsa-miR-1301-3p, hsa-miR-326, hsa-miR-101-3p, hsa-miR-18a-5p, hsa-miR-130a-3p, hsa-miR-4306, hsa-miR-423-3p, hsa-miR-323b-3p, hsa-miR-494-3p, hsa-miR-361-5p, hsa-miR-1260b, hsa-miR-15b-3p, hsa-miR-7-5p | 75.19 | 80.24 | 80 |
| 1948 | hsa-miR-550a-3-5p, hsa-miR-202-5p, hsa-miR-1301-3p, hsa-miR-29c-5p, hsa-miR-2110, hsa-miR-197-3p, hsa-miR-18a-5p, hsa-miR-423-3p, hsa-miR-6803-3p, hsa-miR-128-3p, hsa-miR-361-5p, hsa-miR-532-3p, hsa-miR-7-5p | 75.19 | 80.83 | 78.33 |
| 1949 | hsa-miR-30b-5p, hsa-miR-326, hsa-miR-197-3p, hsa-miR-101-3p, hsa-miR-4306, hsa-miR-664a-3p, hsa-miR-323b-3p, hsa-miR-494-3p, hsa-miR-532-3p, hsa-miR-15b-3p | 74.46 | 79.13 | 76.67 |
| 1950 | hsa-miR-30b-5p, hsa-miR-326, hsa-miR-197-3p, hsa-miR-4306, hsa-miR-423-3p, hsa-miR-664a-3p, hsa-miR-323b-3p, hsa-miR-128-3p, hsa-miR-494-3p, hsa-miR-532-3p, hsa-miR-15b-3p | 74.46 | 76.76 | 78.33 |
| 1951 | hsa-miR-550a-3-5p, hsa-miR-181c-3p, hsa-miR-202-5p, hsa-miR-1301-3p, hsa-miR-29c-5p, hsa-miR-326, hsa-miR-2110, hsa-miR-197-3p, hsa-miR-6803-3p, hsa-miR-323b-3p, hsa-miR-128-3p, hsa-miR-361-5p, hsa-miR-15b-3p | 74.37 | 82.94 | 76.67 |
| 1952 | hsa-miR-550a-3-5p, hsa-miR-202-5p, hsa-miR-1301-3p, hsa-miR-29c-5p, hsa-miR-2110, hsa-miR-197-3p, hsa-miR-18a-5p, hsa-miR-130a-3p, hsa-miR-423-3p, hsa-miR-6803-3p, hsa-miR-323b-3p, hsa-miR-361-5p, hsa-miR-579-3p, hsa-miR-15b-3p, hsa-miR-7-5p | 74.37 | 81.44 | 76.67 |
| 1953 | hsa-miR-423-5p, hsa-miR-29c-5p, hsa-miR-326, hsa-miR-197-3p, hsa-miR-4306, hsa-miR-423-3p, hsa-miR-6803-3p, hsa-miR-323b-3p, age, hsa-miR-361-5p, hsa-miR-532-3p | 74.37 | 84.28 | 76.67 |
| 1954 | hsa-miR-202-5p, hsa-miR-1301-3p, hsa-miR-29c-5p, hsa-miR-101-3p, hsa-miR-130a-3p, hsa-miR-4306, hsa-miR-6803-3p, hsa-miR-323b-3p, hsa-miR-128-3p, hsa-miR-494-3p, hsa-miR-1260b, hsa-miR-532-3p, hsa-miR-15b-3p | 74.37 | 80.87 | 80 |
| 1955 | hsa-miR-181c-3p, hsa-miR-1301-3p, hsa-miR-29c-5p, hsa-miR-326, hsa-miR-2110, hsa-miR-101-3p, hsa-miR-6803-3p, hsa-miR-664a-3p, hsa-miR-323b-3p, hsa-miR-128-3p, hsa-miR-494-3p, age, hsa-miR-579-3p, hsa-miR-7-5p | 74.2 | 83.07 | 76.67 |
| 1956 | hsa-miR-423-5p, hsa-miR-1301-3p, hsa-miR-326, hsa-miR-18a-5p, hsa-miR-423-3p, hsa-miR-664a-3p, hsa-miR-323b-3p, hsa-miR-128-3p, hsa-miR-361-5p, hsa-miR-532-3p, hsa-miR-15b-3p | 73.55 | 81.39 | 73.33 |
| 1957 | hsa-miR-550a-3-5p, hsa-miR-181c-3p, hsa-miR-202-5p, hsa-miR-1301-3p, hsa-miR-29c-5p, hsa-miR-326, hsa-miR-2110, hsa-miR-197-3p, hsa-miR-4306, hsa-miR-6803-3p, hsa-miR-323b-3p, hsa-miR-128-3p, hsa-miR-361-5p, hsa-miR-15b-3p | 73.51 | 78.74 | 76.67 |
| 1958 | hsa-miR-550a-3-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-197-3p, hsa-miR-4306, hsa-miR-6803-3p, hsa-miR-323b-3p, hsa-miR-128-3p, hsa-miR-15b-3p | 73.46 | 78.2 | 76.67 |
| 1959 | hsa-miR-550a-3-5p, hsa-miR-181c-3p, hsa-miR-1301-3p, hsa-miR-29c-5p, hsa-miR-2110, hsa-miR-197-3p, hsa-miR-130a-3p, hsa-miR-423-3p, hsa-miR-6803-3p, hsa-miR-323b-3p, hsa-miR-128-3p, hsa-miR-361-5p, hsa-miR-579-3p, hsa-miR-15b-3p, hsa-miR-7-5p | 73.33 | 83.43 | 71.67 |
| 1960 | hsa-miR-30b-5p, hsa-miR-423-5p, hsa-miR-29c-5p, hsa-miR-326, hsa-miR-423-3p, hsa-miR-664a-3p, hsa-miR-323b-3p, hsa-miR-494-3p, hsa-miR-532-3p, hsa-miR-15b-3p | 72.6 | 79.83 | 76.67 |
| 1961 | hsa-miR-30b-5p, hsa-miR-29c-5p, hsa-miR-326, hsa-miR-423-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 72.55 | 79.56 | 78.33 |
| 1962 | hsa-miR-550a-3-5p, hsa-miR-30b-5p, hsa-miR-202-5p, hsa-miR-423-5p, hsa-miR-1301-3p, hsa-miR-197-3p, hsa-miR-101-3p, hsa-miR-18a-5p, hsa-miR-130a-3p, hsa-miR-4306, hsa-miR-323b-3p, hsa-miR-128-3p, hsa-miR-494-3p, age, hsa-miR-579-3p, hsa-miR-15b-3p | 71.6 | 83.37 | 76.67 |
| 1963 | hsa-miR-550a-3-5p, hsa-miR-181c-3p, hsa-miR-423-5p, hsa-miR-2110, hsa-miR-101-3p, hsa-miR-18a-5p, hsa-miR-4306, hsa-miR-423-3p, hsa-miR-664a-3p, hsa-miR-128-3p, age, hsa-miR-532-3p | 71.56 | 78.3 | 71.67 |
| 1964 | hsa-miR-30b-5p, hsa-miR-29c-5p, hsa-miR-326, hsa-miR-101-3p, hsa-miR-423-3p, hsa-miR-664a-3p, hsa-miR-323b-3p, hsa-miR-128-3p, hsa-miR-494-3p, hsa-miR-361-5p, hsa-miR-532-3p, hsa-miR-15b-3p | 70.78 | 77.81 | 71.67 |
| 1965 | combination 1723 + rs71384446 | 63.38 | 64.5 | 85.97 |
| 1966 | combination 1723 + rs11647727 | 53.46 | 57.78 | 80.70 |
| 1967 | combination 1724 + rs71384446 | 61.52 | 59 | 84.27 |
| 1968 | combination 1725 + rs7193058 | 71.6 | 71 | 81.58 |
| 1969 | combination 1725 + rs11647727 | 70.74 | 70.13 | 84.15 |
| 1970 | combination 1735 + rs13335818 | 78.01 | 85.35 | 81.58 |
| 1971 | combination 1735 + rs7193058 | 78.87 | 89.7 | 85.1 |
| 1972 | combination 1739 + rs9928757 | 80.74 | 87.02 | 84.19 |
| 1973 | combination 1744 + rs11862974 | 81.69 | 90.7 | 84.19 |
| 1974 | combination 1745 + rs11647727 | 81.65 | 90.72 | 90.00 |
| 1975 | combination 1747 + rs76563024 | 81.65 | 88.28 | 85.2 |
| 1976 | combination 1750 + rs13335818 | 83.46 | 91.35 | 90.3 |
| 1977 | combination 1877 + rs76563024 | 79.78 | 90.31 | 89.35 |
| 1978 | combination 1877 + rs76563024 | 79.78 | 90.31 | 90.45 |

This is the list of the best 29 mRNA selected by the algorithm sorted by importance for diagnosis outcome: hsa-miR-374a-5p, hsa-miR-550a-3-5p, hsa-miR-132-3p, hsa-miR-1180-3p, hsa-miR-590-5p, hsa-miR-590-3p, hsa-miR-500a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, hsa-miR-181b-5p, hsa-miR-192-5p, hsa-miR-433-3p, hsa-miR-12136, hsa-miR-598-3p, hsa-miR-628-3p, hsa-miR-19a-3p, hsa-miR-122-5p, hsa-miR-196b-5p, hsa-miR-215-5p, hsa-miR-491-5p, and hsa-miR-153-3p. The inventors combined these miRNAs, together or not with age and UMOD SNP variant, creating unique combinations leading to a further significant increase in sensitivity, specificity, accuracy and predictive power for diagnosis (Table 25).

**Table 25. Combinations that allow higher sensitivity, specificity, accuracy and predictive power for diagnosis.**

| N | Combinations | Accuracy | ROC AUC | Recall |
|---|---|---|---|---|
| 1980 | hsa-miR-550a-3-5p, hsa-miR-132-3p | 78.33 | 81.83 | 88.89 |
| 1981 | hsa-miR-550a-3-5p, hsa-miR-132-3p, hsa-miR-1180-3p | 80 | 82.06 | 95.78 |
| 1982 | hsa-miR-550a-3-5p, hsa-miR-132-3p, hsa-miR-1180-3p, hsa-miR-590-5p | 76.67 | 80.14 | 96.89 |
| 1983 | hsa-miR-550a-3-5p, hsa-miR-132-3p, hsa-miR-1180-3p, hsa-miR-590-5p, hsa-miR-590-3p | 78.33 | 81.39 | 90.89 |
| 1984 | hsa-miR-550a-3-5p, hsa-miR-132-3p, hsa-miR-1180-3p, hsa-miR-590-5p, hsa-miR-590-3p, hsa-miR-500a-5p | 81.67 | 83.89 | 96.89 |
| 1985 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p | 81.67 | 80.65 | 97.78 |
| 1986 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p | 76.67 | 79.79 | 98.89 |
| 1987 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p | 71.67 | 78.89 | 100.0 |
| 1988 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p | 78.33 | 84.4 | 100.0 |
| 1989 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p | 76.67 | 81.55 | 100.0 |
| 1990 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p | 76.67 | 82.36 | 100.0 |
| 1991 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p | 85 | 91.06 | 100.0 |
| 1992 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p | 83.33 | 90.95 | 100.0 |
| 1993 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l | 81.67 | 90.32 | 100.0 |
| 1994 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p | 81.67 | 88.22 | 100.0 |
| 1995 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p | 85 | 88.96 | 100.0 |
| 1996 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age | 88.33 | 95.05 | 100.0 |
| 1997 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p | 88.33 | 95.16 | 100.0 |
| 1998 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-192-5p | 86.67 | 94.19 | 100.0 |
| 1999 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-192-5p, hsa-miR-433-3p | 86.67 | 92.94 | 100.0 |
| 2000 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-192-5p, hsa-miR-433-3p, hsa-miR-12136 | 86.67 | 97.04 | 100.0 |
| 2001 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-192-5p, hsa-miR-433-3p, hsa-miR-12136, hsa-miR-598-3p | 86.67 | 97.04 | 100.0 |
| 2002 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-192-5p, hsa-miR-433-3p, hsa-miR-12136, hsa-miR-598-3p, hsa-miR-628-3p | 85 | 94.65 | 100.0 |
| 2003 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-192-5p, hsa-miR-433-3p, hsa-miR-12136, hsa-miR-598-3p, hsa-miR-628-3p, hsa-miR-19a-3p | 85 | 95.39 | 100.0 |
| 2004 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-192-5p, hsa-miR-433-3p, hsa-miR-12136, hsa-miR-598-3p, hsa-miR-628-3p, hsa-miR-19a-3p, hsa-miR-122-5p | 83.33 | 93.52 | 100.0 |
| 2005 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-192-5p, hsa-miR-433-3p, hsa-miR-12136, hsa-miR-598-3p, hsa-miR-628-3p, hsa-miR-19a-3p, hsa-miR-122-5p, hsa-miR-196b-5p | 85 | 93.91 | 100.0 |
| 2006 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-192-5p, hsa-miR-433-3p, hsa-miR-12136, hsa-miR-598-3p, hsa-miR-628-3p, hsa-miR-19a-3p, hsa-miR-122-5p, hsa-miR-196b-5p, hsa-miR-215-5p | 80 | 94.54 | 100.0 |
| 2007 | hsa-miR-550a-3-5p, hsa-miR-132-3p, hsa-miR-1180-3p, hsa-miR-590-5p, hsa-miR-590-3p, hsa-miR-500a-5p, hsa-miR-374a-5p, hsa-miR-99a-3p, hsa-miR-130b-5p, hsa-miR-337-5p, hsa-miR-4732-5p, hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-192-5p, hsa-miR-433-3p, hsa-miR-12136, hsa-miR-598-3p, hsa-miR-628-3p, hsa-miR-19a-3p, hsa-miR-122-5p, hsa-miR-196b-5p, hsa-miR-215-5p, hsa-miR-491-5p | 85 | 93.91 | 100.0 |
| 2008 | hsa-miR-4732-5p, hsa-miR-3613-3p, hsa-miR-103a-2-5p, hsa-miR-548l, age, hsa-miR-192-5p, hsa-miR-12136, hsa-miR-598-3p, hsa-miR-19a-3p, hsa-miR-590-3p, hsa-miR-500a-5p | 95 | 97.78 | 97.78 |
| 2009 | hsa-miR-4732-5p, hsa-miR-374a-3p, hsa-miR-3613-3p, hsa-miR-103a-2-5p, age, hsa-miR-192-5p, hsa-miR-590-5p, hsa-miR-590-3p | 95 | 90.09 | 95.28 |
| 2010 | hsa-miR-4732-5p, hsa-miR-3613-3p, hsa-miR-103a-2-5p, age, hsa-miR-192-5p, hsa-miR-590-5p, hsa-miR-590-3p, hsa-miR-500a-5p | 95 | 95.56 | 97.78 |
| 2011 | hsa-miR-4732-5p, hsa-miR-3613-3p, hsa-miR-103a-2-5p, age, hsa-miR-192-5p, hsa-miR-590-5p, hsa-miR-590-3p, hsa-miR-500a-5p, hsa-miR-374a-5p | 95 | 95.56 | 97.78 |
| 2012 | hsa-miR-4732-5p, hsa-miR-3613-3p, hsa-miR-103a-2-5p, age, hsa-miR-192-5p, hsa-miR-122-5p, hsa-miR-590-5p, hsa-miR-590-3p, hsa-miR-500a-5p | 95 | 95.56 | 97.78 |
| 2013 | hsa-miR-4732-5p, hsa-miR-3613-3p, hsa-miR-103a-2-5p, age, hsa-miR-192-5p, hsa-miR-122-5p, hsa-miR-590-5p, hsa-miR-590-3p, hsa-miR-500a-5p, hsa-miR-374a-5p | 95 | 95.56 | 97.78 |
| 2014 | hsa-miR-4732-5p, hsa-miR-3613-3p, hsa-miR-103a-2-5p, age, hsa-miR-192-5p, hsa-miR-598-3p, hsa-miR-590-5p, hsa-miR-590-3p, hsa-miR-500a-5p, hsa-miR-374a-5p | 93.33 | 96.3 | 95.56 |
| 2015 | hsa-miR-4732-5p, hsa-miR-3613-3p, hsa-miR-103a-2-5p, hsa-miR-1277-3p, age, hsa-miR-181b-5p, hsa-miR-192-5p, hsa-miR-122-5p, hsa-miR-491-5p, hsa-miR-590-5p, hsa-miR-374a-5p | 93.33 | 94.77 | 95 |
| 2016 | hsa-miR-4732-5p, hsa-miR-3613-3p, hsa-miR-103a-2-5p, age, hsa-miR-181b-5p, hsa-miR-192-5p, hsa-miR-491-5p, hsa-miR-590-5p, hsa-miR-590-3p | 93.33 | 93.45 | 95.28 |
| 2017 | hsa-miR-4732-5p, hsa-miR-3613-3p, age, hsa-miR-192-5p, hsa-miR-122-5p, hsa-miR-153-3p, hsa-miR-590-5p, hsa-miR-590-3p, hsa-miR-500a-5p, hsa-miR-374a-5p | 93.33 | 94.81 | 97.78 |
| 2018 | hsa-miR-4732-5p, hsa-miR-3613-3p, hsa-miR-103a-2-5p, age, hsa-miR-192-5p, hsa-miR-122-5p, hsa-miR-153-3p, hsa-miR-590-5p, hsa-miR-590-3p, hsa-miR-500a-5p, hsa-miR-374a-5p | 93.33 | 94.81 | 97.78 |
| 2019 | hsa-miR-4732-5p, hsa-miR-3613-3p, hsa-miR-103a-2-5p, hsa-miR-548l, age, hsa-miR-192-5p, hsa-miR-12136, hsa-miR-598-3p, hsa-miR-19a-3p, hsa-miR-122-5p, hsa-miR-590-5p, hsa-miR-590-3p, hsa-miR-500a-5p | 93.33 | 98.52 | 95.28 |
| 2020 | hsa-miR-4732-5p, hsa-miR-3613-3p, hsa-miR-103a-2-5p, age, hsa-miR-192-5p, hsa-miR-433-3p, hsa-miR-590-5p, hsa-miR-590-3p, hsa-miR-500a-5p, hsa-miR-374a-5p | 93.33 | 96.3 | 95.56 |
| 2021 | hsa-miR-4732-5p, hsa-miR-3613-3p, hsa-miR-103a-2-5p, hsa-miR-548l, age, hsa-miR-192-5p, hsa-miR-590-5p, hsa-miR-590-3p, hsa-miR-500a-5p | 93.33 | 95.56 | 95.28 |
| 2022 | hsa-miR-4732-5p, hsa-miR-3613-3p, hsa-miR-103a-2-5p, age, hsa-miR-192-5p, hsa-miR-122-5p, hsa-miR-590-5p, hsa-miR-590-3p, hsa-miR-374a-5p | 93.33 | 93.45 | 95.28 |
| 2023 | hsa-miR-4732-5p, hsa-miR-3613-3p, hsa-miR-103a-2-5p, age, hsa-miR-192-5p, hsa-miR-433-3p, hsa-miR-122-5p, hsa-miR-590-5p, hsa-miR-590-3p, hsa-miR-500a-5p, hsa-miR-374a-5p | 93.33 | 96.3 | 95.56 |
| 2024 | hsa-miR-4732-5p, hsa-miR-374a-3p, hsa-miR-3613-3p, hsa-miR-103a-2-5p, age, hsa-miR-192-5p, hsa-miR-122-5p, hsa-miR-590-5p, hsa-miR-590-3p, hsa-miR-374a-5p | 93.33 | 90.83 | 95.28 |
| 2025 | hsa-miR-4732-5p, hsa-miR-3613-3p, hsa-miR-103a-2-5p, hsa-miR-548l, age, hsa-miR-192-5p, hsa-miR-122-5p, hsa-miR-590-5p, hsa-miR-590-3p, hsa-miR-500a-5p | 93.33 | 94.81 | 95.28 |
| 2026 | hsa-miR-4732-5p, hsa-miR-3613-3p, hsa-miR-1277-3p, age, hsa-miR-192-5p, hsa-miR-628-3p, hsa-miR-153-3p, hsa-miR-590-5p, hsa-miR-500a-5p | 93.33 | 94.81 | 97.78 |
| 2027 | hsa-miR-4732-5p, hsa-miR-3613-3p, hsa-miR-103a-2-5p, hsa-miR-192-5p, hsa-miR-433-3p, hsa-miR-590-5p, hsa-miR-590-3p, hsa-miR-500a-5p, hsa-miR-374a-5p | 93.33 | 94.81 | 95.56 |
| 2028 | hsa-miR-4732-5p, hsa-miR-3613-3p, hsa-miR-1277-3p, age, hsa-miR-192-5p, hsa-miR-628-3p, hsa-miR-153-3p, hsa-miR-590-5p, hsa-miR-500a-5p, hsa-miR-374a-5p | 93.33 | 93.06 | 97.78 |
| 2029 | hsa-miR-4732-5p, hsa-miR-3613-3p, hsa-miR-103a-2-5p, hsa-miR-548l, age, hsa-miR-598-3p, hsa-miR-19a-3p, hsa-miR-122-5p, hsa-miR-590-5p, hsa-miR-590-3p, hsa-miR-500a-5p | 93.33 | 96.3 | 95.28 |
| 2030 | hsa-miR-4732-5p, hsa-miR-374a-3p, hsa-miR-3613-3p, hsa-miR-103a-2-5p, age, hsa-miR-192-5p, hsa-miR-122-5p, hsa-miR-590-5p, hsa-miR-590-3p, hsa-miR-500a-5p, hsa-miR-374a-5p | 93.33 | 91.57 | 95.28 |
| 2031 | hsa-miR-4732-5p, hsa-miR-3613-3p, hsa-miR-103a-2-5p, hsa-miR-1277-3p, age, hsa-miR-192-5p, hsa-miR-153-3p, hsa-miR-590-5p, hsa-miR-500a-5p, hsa-miR-374a-5p | 93.33 | 94.31 | 95.56 |
| 2032 | hsa-miR-4732-5p, hsa-miR-3613-3p, hsa-miR-103a-2-5p, hsa-miR-548l, hsa-miR-29c-5p, age, hsa-miR-12136, hsa-miR-598-3p, hsa-miR-628-3p, hsa-miR-19a-3p, hsa-miR-122-5p, hsa-miR-590-3p, hsa-miR-500a-5p, hsa-miR-99a-3p, hsa-miR-130b-5p | 91.67 | 96.3 | 93.33 |
| 2033 | hsa-miR-4732-5p, hsa-miR-3613-3p, hsa-miR-103a-2-5p, age, hsa-miR-192-5p, hsa-miR-122-5p, hsa-miR-491-5p, hsa-miR-590-5p, hsa-miR-590-3p, hsa-miR-500a-5p, hsa-miR-374a-5p | 91.67 | 94.07 | 95.28 |
| 2034 | hsa-miR-4732-5p, hsa-miR-3613-3p, hsa-miR-103a-2-5p, hsa-miR-1277-3p, age, hsa-miR-192-5p, hsa-miR-628-3p, hsa-miR-153-3p, hsa-miR-590-5p, hsa-miR-500a-5p | 91.67 | 92.08 | 95.56 |
| 2035 | hsa-miR-4732-5p, hsa-miR-374a-3p, hsa-miR-3613-3p, hsa-miR-103a-2-5p, hsa-miR-1277-3p, age, hsa-miR-181b-5p, hsa-miR-192-5p, hsa-miR-491-5p, hsa-miR-590-5p | 91.67 | 89.07 | 95 |
| 2036 | hsa-miR-4732-5p, hsa-miR-3613-3p, hsa-miR-103a-2-5p, hsa-miR-548l, age, hsa-miR-192-5p, hsa-miR-12136, hsa-miR-598-3p, hsa-miR-19a-3p, hsa-miR-122-5p, hsa-miR-590-3p, hsa-miR-500a-5p | 91.67 | 98.52 | 95.28 |
| 2037 | hsa-miR-374a-3p, hsa-miR-103a-2-5p, hsa-miR-548l, hsa-miR-192-5p, hsa-miR-433-3p, hsa-miR-12136, hsa-miR-598-3p, hsa-miR-19a-3p, hsa-miR-122-5p, hsa-miR-196b-5p, hsa-miR-215-5p, hsa-miR-590-5p, hsa-miR-590-3p, hsa-miR-500a-5p, hsa-miR-337-5p | 91.67 | 95.51 | 92.78 |
| 2038 | hsa-miR-4732-5p, hsa-miR-3613-3p, hsa-miR-1277-3p, age, hsa-miR-192-5p, hsa-miR-628-3p, hsa-miR-491-5p, hsa-miR-153-3p, hsa-miR-590-5p, hsa-miR-500a-5p | 91.67 | 92.82 | 95.56 |
| 2039 | hsa-miR-4732-5p, hsa-miR-103a-2-5p, hsa-miR-548l, hsa-miR-192-5p, hsa-miR-433-3p, hsa-miR-12136, hsa-miR-598-3p, hsa-miR-19a-3p, hsa-miR-122-5p, hsa-miR-196b-5p, hsa-miR-215-5p, hsa-miR-590-5p, hsa-miR-590-3p, hsa-miR-500a-5p, hsa-miR-337-5p | 91.67 | 97.04 | 92.78 |
| 2040 | hsa-miR-4732-5p, hsa-miR-3613-3p, hsa-miR-103a-2-5p, hsa-miR-192-5p, hsa-miR-433-3p, hsa-miR-12136, hsa-miR-598-3p, hsa-miR-19a-3p, hsa-miR-590-3p, hsa-miR-500a-5p, hsa-miR-374a-5p | 91.67 | 95.56 | 95.28 |
| 2041 | hsa-miR-4732-5p, hsa-miR-3613-3p, hsa-miR-103a-2-5p, hsa-miR-548l, age, hsa-miR-192-5p, hsa-miR-12136, hsa-miR-598-3p, hsa-miR-628-3p, hsa-miR-19a-3p, hsa-miR-590-3p, hsa-miR-500a-5p | 91.67 | 97.04 | 95.56 |
| 2042 | hsa-miR-4732-5p, hsa-miR-3613-3p, hsa-miR-103a-2-5p, age, hsa-miR-192-5p, hsa-miR-628-3p, hsa-miR-153-3p, hsa-miR-590-5p, hsa-miR-590-3p, hsa-miR-500a-5p, hsa-miR-374a-5p | 91.67 | 92.43 | 95.28 |
| 2043 | hsa-miR-4732-5p, hsa-miR-103a-2-5p, hsa-miR-548l, hsa-miR-192-5p, hsa-miR-433-3p, hsa-miR-12136, hsa-miR-598-3p, hsa-miR-19a-3p, hsa-miR-196b-5p, hsa-miR-215-5p, hsa-miR-590-5p, hsa-miR-590-3p, hsa-miR-500a-5p, hsa-miR-337-5p | 91.67 | 97.04 | 92.78 |
| 2044 | hsa-miR-4732-5p, hsa-miR-3613-3p, hsa-miR-103a-2-5p, hsa-miR-548l, age, hsa-miR-192-5p, hsa-miR-122-5p, hsa-miR-590-5p, hsa-miR-590-3p, hsa-miR-500a-5p, hsa-miR-374a-5p | 90 | 94.81 | 90.56 |
| 2045 | hsa-miR-4732-5p, hsa-miR-3613-3p, hsa-miR-103a-2-5p, hsa-miR-1277-3p, age, hsa-miR-192-5p, hsa-miR-122-5p, hsa-miR-153-3p, hsa-miR-590-5p, hsa-miR-500a-5p, hsa-miR-374a-5p | 90 | 94.31 | 93.06 |
| 2046 | hsa-miR-4732-5p, hsa-miR-3613-3p, hsa-miR-103a-2-5p, hsa-miR-1277-3p, age, hsa-miR-192-5p, hsa-miR-628-3p, hsa-miR-122-5p, hsa-miR-153-3p, hsa-miR-590-5p, hsa-miR-500a-5p, hsa-miR-374a-5p | 90 | 94.31 | 93.06 |
| 2047 | hsa-miR-4732-5p, hsa-miR-3613-3p, hsa-miR-1277-3p, age, hsa-miR-192-5p, hsa-miR-122-5p, hsa-miR-153-3p, hsa-miR-590-5p, hsa-miR-500a-5p, hsa-miR-374a-5p | 90 | 94.31 | 93.06 |
| 2048 | hsa-miR-4732-5p, hsa-miR-3613-3p, hsa-miR-1277-3p, age, hsa-miR-192-5p, hsa-miR-628-3p, hsa-miR-122-5p, hsa-miR-153-3p, hsa-miR-590-5p, hsa-miR-500a-5p, hsa-miR-374a-5p | 90 | 94.31 | 93.06 |
| 2049 | hsa-miR-4732-5p, hsa-miR-3613-3p, hsa-miR-103a-2-5p, hsa-miR-29c-5p, age, hsa-miR-192-5p, hsa-miR-598-3p, hsa-miR-19a-3p, hsa-miR-122-5p, hsa-miR-590-5p, hsa-miR-590-3p, hsa-miR-500a-5p, hsa-miR-374a-5p | 90 | 94.03 | 92.78 |
| 2050 | hsa-miR-4732-5p, hsa-miR-374a-3p, hsa-miR-3613-3p, hsa-miR-103a-2-5p, age, hsa-miR-181b-5p, hsa-miR-192-5p, hsa-miR-122-5p, hsa-miR-590-5p, hsa-miR-590-3p, hsa-miR-374a-5p | 90 | 88.33 | 92.78 |
| 2051 | hsa-miR-4732-5p, hsa-miR-3613-3p, hsa-miR-1277-3p, age, hsa-miR-181b-5p, hsa-miR-192-5p, hsa-miR-628-3p, hsa-miR-122-5p, hsa-miR-491-5p, hsa-miR-153-3p, hsa-miR-590-5p, hsa-miR-374a-5p | 90 | 92.31 | 90.28 |
| 2052 | hsa-miR-4732-5p, hsa-miR-3613-3p, hsa-miR-103a-2-5p, hsa-miR-1277-3p, age, hsa-miR-192-5p, hsa-miR-122-5p, hsa-miR-590-5p, hsa-miR-500a-5p, hsa-miR-374a-5p | 90 | 97.15 | 90.56 |
| 2053 | hsa-miR-4732-5p, hsa-miR-3613-3p, age, hsa-miR-192-5p, hsa-miR-122-5p, hsa-miR-590-5p, hsa-miR-590-3p, hsa-miR-500a-5p, hsa-miR-374a-5p | 90 | 94.81 | 90.83 |
| 2054 | hsa-miR-4732-5p, hsa-miR-3613-3p, hsa-miR-548l, hsa-miR-29c-5p, age, hsa-miR-12136, hsa-miR-598-3p, hsa-miR-19a-3p, hsa-miR-122-5p, hsa-miR-153-3p, hsa-miR-590-3p, hsa-miR-500a-5p, hsa-miR-99a-3p, hsa-miR-130b-5p | 90 | 97.78 | 90.56 |
| 2055 | hsa-miR-4732-5p, hsa-miR-3613-3p, hsa-miR-548l, age, hsa-miR-122-5p, hsa-miR-153-3p, hsa-miR-590-5p, hsa-miR-590-3p, hsa-miR-500a-5p | 90 | 91.85 | 90.83 |
| 2056 | hsa-miR-4732-5p, hsa-miR-103a-2-5p, hsa-miR-192-5p, hsa-miR-433-3p, hsa-miR-12136, hsa-miR-598-3p, hsa-miR-19a-3p, hsa-miR-196b-5p, hsa-miR-215-5p, hsa-miR-590-5p, hsa-miR-590-3p, hsa-miR-500a-5p, hsa-miR-337-5p | 90 | 95.67 | 93.06 |
| 2057 | hsa-miR-374a-3p, hsa-miR-103a-2-5p, hsa-miR-548l, hsa-miR-1277-3p, hsa-miR-181b-5p, hsa-miR-192-5p, hsa-miR-433-3p, hsa-miR-12136, hsa-miR-598-3p, hsa-miR-19a-3p, hsa-miR-122-5p, hsa-miR-196b-5p, hsa-miR-215-5p, hsa-miR-491-5p, hsa-miR-590-5p, hsa-miR-590-3p, hsa-miR-500a-5p, hsa-miR-337-5p | 90 | 93.52 | 90.28 |
| 2058 | hsa-miR-4732-5p, hsa-miR-3613-3p, hsa-miR-103a-2-5p, age, hsa-miR-192-5p, hsa-miR-12136, hsa-miR-598-3p, hsa-miR-19a-3p, hsa-miR-122-5p, hsa-miR-590-3p, hsa-miR-500a-5p | 90 | 97.04 | 95.28 |
| 2059 | hsa-miR-374a-3p, hsa-miR-3613-3p, hsa-miR-103a-2-5p, hsa-miR-548l, age, hsa-miR-192-5p, hsa-miR-12136, hsa-miR-598-3p, hsa-miR-19a-3p, hsa-miR-590-3p, hsa-miR-500a-5p | 90 | 98.63 | 92.78 |
| 2060 | hsa-miR-4732-5p, hsa-miR-3613-3p, hsa-miR-103a-2-5p, hsa-miR-1277-3p, age, hsa-miR-192-5p, hsa-miR-628-3p, hsa-miR-153-3p, hsa-miR-590-5p, hsa-miR-500a-5p, hsa-miR-374a-5p | 90 | 88.84 | 91.11 |
| 2061 | hsa-miR-4732-5p, hsa-miR-3613-3p, hsa-miR-103a-2-5p, age, hsa-miR-192-5p, hsa-miR-628-3p, hsa-miR-590-5p, hsa-miR-590-3p, hsa-miR-500a-5p, hsa-miR-374a-5p | 90 | 92.43 | 95.28 |
| 2062 | hsa-miR-4732-5p, hsa-miR-3613-3p, hsa-miR-103a-2-5p, age, hsa-miR-433-3p, hsa-miR-122-5p, hsa-miR-196b-5p, hsa-miR-590-5p, hsa-miR-590-3p, hsa-miR-500a-5p, hsa-miR-374a-5p | 90 | 94.81 | 93.06 |
| 2063 | hsa-miR-4732-5p, hsa-miR-3613-3p, hsa-miR-103a-2-5p, hsa-miR-548l, age, hsa-miR-192-5p, hsa-miR-12136, hsa-miR-598-3p, hsa-miR-628-3p, hsa-miR-19a-3p, hsa-miR-590-3p, hsa-miR-500a-5p, hsa-miR-374a-5p | 90 | 97.78 | 92.78 |
| 2064 | hsa-miR-4732-5p, hsa-miR-3613-3p, hsa-miR-103a-2-5p, age, hsa-miR-192-5p, hsa-miR-122-5p, hsa-miR-590-5p, hsa-miR-590-3p | 90 | 92.71 | 90.56 |
| 2065 | hsa-miR-4732-5p, hsa-miR-3613-3p, hsa-miR-103a-2-5p, age, hsa-miR-192-5p, hsa-miR-122-5p, hsa-miR-590-5p, hsa-miR-590-3p, hsa-miR-500a-5p, hsa-miR-374a-5p, hsa-miR-130b-5p | 90 | 95.56 | 93.33 |
| 2066 | hsa-miR-4732-5p, hsa-miR-374a-3p, hsa-miR-3613-3p, hsa-miR-103a-2-5p, age, hsa-miR-181b-5p, hsa-miR-192-5p, hsa-miR-590-5p, hsa-miR-590-3p, hsa-miR-374a-5p | 90 | 88.96 | 92.78 |
| 2067 | hsa-miR-4732-5p, hsa-miR-3613-3p, hsa-miR-548l, age, hsa-miR-192-5p, hsa-miR-598-3p, hsa-miR-19a-3p, hsa-miR-122-5p, hsa-miR-153-3p, hsa-miR-590-5p, hsa-miR-590-3p, hsa-miR-500a-5p | 88.33 | 95.79 | 95.28 |
| 2068 | hsa-miR-4732-5p, hsa-miR-3613-3p, hsa-miR-103a-2-5p, hsa-miR-29c-5p, age, hsa-miR-192-5p, hsa-miR-122-5p, hsa-miR-590-5p, hsa-miR-590-3p, hsa-miR-500a-5p, hsa-miR-374a-5p, hsa-miR-99a-3p, hsa-miR-130b-5p | 88.33 | 95.79 | 85.83 |
| 2069 | hsa-miR-4732-5p, hsa-miR-3613-3p, hsa-miR-103a-2-5p, age, hsa-miR-181b-5p, hsa-miR-192-5p, hsa-miR-491-5p, hsa-miR-590-5p, hsa-miR-590-3p, hsa-miR-374a-5p | 88.33 | 92.71 | 88.06 |
| 2070 | hsa-miR-4732-5p, hsa-miR-374a-3p, hsa-miR-3613-3p, hsa-miR-103a-2-5p, age, hsa-miR-181b-5p, hsa-miR-192-5p, hsa-miR-122-5p, hsa-miR-590-5p, hsa-miR-590-3p | 88.33 | 87.59 | 90.28 |
| 2071 | hsa-miR-4732-5p, hsa-miR-3613-3p, hsa-miR-103a-2-5p, hsa-miR-29c-5p, age, hsa-miR-192-5p, hsa-miR-598-3p, hsa-miR-19a-3p, hsa-miR-122-5p, hsa-miR-153-3p, hsa-miR-590-5p, hsa-miR-590-3p, hsa-miR-500a-5p, hsa-miR-374a-5p, hsa-miR-99a-3p | 88.33 | 92.94 | 93.06 |
| 2072 | hsa-miR-4732-5p, hsa-miR-3613-3p, hsa-miR-103a-2-5p, age, hsa-miR-192-5p, hsa-miR-598-3p, hsa-miR-19a-3p, hsa-miR-122-5p, hsa-miR-590-5p, hsa-miR-590-3p, hsa-miR-500a-5p | 88.33 | 96.41 | 93.06 |
| 2073 | hsa-miR-4732-5p, hsa-miR-3613-3p, hsa-miR-103a-2-5p, hsa-miR-29c-5p, age, hsa-miR-192-5p, hsa-miR-598-3p, hsa-miR-19a-3p, hsa-miR-122-5p, hsa-miR-196b-5p, hsa-miR-590-5p, hsa-miR-590-3p, hsa-miR-500a-5p, hsa-miR-99a-3p, hsa-miR-130b-5p | 88.33 | 92.94 | 86.11 |
| 2074 | hsa-miR-4732-5p, hsa-miR-3613-3p, age, hsa-miR-192-5p, hsa-miR-628-3p, hsa-miR-153-3p, hsa-miR-590-5p, hsa-miR-590-3p, hsa-miR-500a-5p | 88.33 | 95.56 | 95.56 |
| 2075 | hsa-miR-4732-5p, hsa-miR-3613-3p, hsa-miR-103a-2-5p, age, hsa-miR-598-3p, hsa-miR-19a-3p, hsa-miR-122-5p, hsa-miR-590-5p, hsa-miR-590-3p, hsa-miR-500a-5p | 88.33 | 96.41 | 93.06 |
| 2076 | hsa-miR-4732-5p, hsa-miR-3613-3p, hsa-miR-103a-2-5p, hsa-miR-548l, hsa-miR-29c-5p, age, hsa-miR-12136, hsa-miR-628-3p, hsa-miR-153-3p, hsa-miR-590-3p | 88.33 | 90.83 | 90.56 |
| 2077 | hsa-miR-4732-5p, hsa-miR-103a-2-5p, hsa-miR-548l, hsa-miR-29c-5p, hsa-miR-598-3p, hsa-miR-19a-3p, hsa-miR-196b-5p, hsa-miR-215-5p, hsa-miR-590-5p, hsa-miR-590-3p, hsa-miR-500a-5p, hsa-miR-374a-5p, hsa-miR-99a-3p, hsa-miR-130b-5p, hsa-miR-337-5p | 88.33 | 95.9 | 88.33 |
| 2078 | hsa-miR-374a-3p, hsa-miR-3613-3p, hsa-miR-103a-2-5p, hsa-miR-548l, age, hsa-miR-192-5p, hsa-miR-12136, hsa-miR-598-3p, hsa-miR-628-3p, hsa-miR-19a-3p, hsa-miR-122-5p, hsa-miR-590-3p, hsa-miR-500a-5p | 88.33 | 97.27 | 90.28 |
| 2079 | hsa-miR-4732-5p, hsa-miR-374a-3p, hsa-miR-3613-3p, hsa-miR-103a-2-5p, hsa-miR-548l, hsa-miR-1277-3p, age, hsa-miR-192-5p, hsa-miR-12136, hsa-miR-598-3p, hsa-miR-19a-3p, hsa-miR-590-3p, hsa-miR-500a-5p, hsa-miR-99a-3p, hsa-miR-337-5p | 88.33 | 95.67 | 88.06 |
| 2080 | hsa-miR-4732-5p, hsa-miR-3613-3p, hsa-miR-103a-2-5p, hsa-miR-548l, age, hsa-miR-192-5p, hsa-miR-12136, hsa-miR-628-3p, hsa-miR-590-3p | 88.33 | 89 | 88.33 |
| 2081 | hsa-miR-374a-3p, hsa-miR-3613-3p, hsa-miR-103a-2-5p, hsa-miR-548l, age, hsa-miR-192-5p, hsa-miR-12136, hsa-miR-598-3p, hsa-miR-19a-3p, hsa-miR-122-5p, hsa-miR-590-3p, hsa-miR-500a-5p | 88.33 | 98.63 | 90.28 |
| 2082 | hsa-miR-4732-5p, hsa-miR-3613-3p, hsa-miR-103a-2-5p, hsa-miR-548l, hsa-miR-29c-5p, age, hsa-miR-181b-5p, hsa-miR-192-5p, hsa-miR-12136, hsa-miR-598-3p, hsa-miR-628-3p, hsa-miR-19a-3p, hsa-miR-590-3p, hsa-miR-500a-5p | 88.33 | 90.72 | 90.83 |
| 2083 | hsa-miR-4732-5p, hsa-miR-3613-3p, hsa-miR-103a-2-5p, hsa-miR-548l, age, hsa-miR-12136, hsa-miR-628-3p, hsa-miR-590-3p | 88.33 | 86.9 | 90.83 |
| 2084 | hsa-miR-4732-5p, hsa-miR-3613-3p, hsa-miR-103a-2-5p, age, hsa-miR-192-5p, hsa-miR-122-5p, hsa-miR-590-3p, hsa-miR-500a-5p, hsa-miR-374a-5p | 88.33 | 91.85 | 92.78 |
| 2085 | hsa-miR-4732-5p, hsa-miR-3613-3p, hsa-miR-1277-3p, age, hsa-miR-192-5p, hsa-miR-12136, hsa-miR-628-3p, hsa-miR-122-5p, hsa-miR-500a-5p | 88.33 | 90.56 | 90.56 |
| 2086 | hsa-miR-4732-5p, hsa-miR-374a-3p, hsa-miR-3613-3p, hsa-miR-103a-2-5p, hsa-miR-1277-3p, age, hsa-miR-192-5p, hsa-miR-433-3p, hsa-miR-12136, hsa-miR-598-3p, hsa-miR-19a-3p, hsa-miR-590-3p, hsa-miR-500a-5p | 88.33 | 95.05 | 85.56 |
| 2087 | hsa-miR-4732-5p, hsa-miR-3613-3p, hsa-miR-103a-2-5p, age, hsa-miR-192-5p, hsa-miR-590-3p, hsa-miR-500a-5p, hsa-miR-374a-5p | 88.33 | 92.08 | 92.78 |
| 2088 | hsa-miR-3613-3p, hsa-miR-103a-2-5p, age, hsa-miR-192-5p, hsa-miR-590-5p, hsa-miR-590-3p, hsa-miR-500a-5p, hsa-miR-374a-5p | 88.33 | 94.19 | 93.06 |
| 2089 | hsa-miR-4732-5p, hsa-miR-3613-3p, hsa-miR-103a-2-5p, age, hsa-miR-192-5p, hsa-miR-628-3p, hsa-miR-122-5p, hsa-miR-590-5p, hsa-miR-590-3p, hsa-miR-500a-5p, hsa-miR-374a-5p | 88.33 | 93.06 | 95.28 |
| 2090 | hsa-miR-103a-2-5p, hsa-miR-548l, hsa-miR-181b-5p, hsa-miR-192-5p, hsa-miR-12136, hsa-miR-598-3p, hsa-miR-19a-3p, hsa-miR-196b-5p, hsa-miR-215-5p, hsa-miR-491-5p, hsa-miR-590-3p, hsa-miR-500a-5p | 88.33 | 94.07 | 92.78 |
| 2091 | hsa-miR-374a-3p, hsa-miR-3613-3p, hsa-miR-103a-2-5p, age, hsa-miR-192-5p, hsa-miR-12136, hsa-miR-122-5p, hsa-miR-590-5p, hsa-miR-590-3p, hsa-miR-500a-5p | 88.33 | 93.06 | 88.33 |
| 2092 | hsa-miR-4732-5p, hsa-miR-3613-3p, hsa-miR-103a-2-5p, hsa-miR-548l, hsa-miR-29c-5p, age, hsa-miR-192-5p, hsa-miR-433-3p, hsa-miR-12136, hsa-miR-598-3p, hsa-miR-19a-3p, hsa-miR-590-3p, hsa-miR-500a-5p, hsa-miR-99a-3p, hsa-miR-130b-5p, hsa-miR-337-5p | 88.33 | 96.3 | 86.67 |
| 2093 | hsa-miR-374a-3p, hsa-miR-3613-3p, hsa-miR-103a-2-5p, hsa-miR-548l, hsa-miR-1277-3p, age, hsa-miR-192-5p, hsa-miR-598-3p, hsa-miR-19a-3p, hsa-miR-590-5p, hsa-miR-590-3p, hsa-miR-500a-5p, hsa-miR-337-5p | 88.33 | 97.38 | 88.06 |
| 2094 | hsa-miR-4732-5p, hsa-miR-3613-3p, hsa-miR-103a-2-5p, hsa-miR-548l, hsa-miR-29c-5p, age, hsa-miR-12136, hsa-miR-598-3p, hsa-miR-628-3p, hsa-miR-19a-3p, hsa-miR-590-3p, hsa-miR-500a-5p, hsa-miR-99a-3p, hsa-miR-130b-5p | 88.33 | 91.11 | 88.61 |
| 2095 | hsa-miR-374a-3p, hsa-miR-3613-3p, hsa-miR-548l, hsa-miR-29c-5p, age, hsa-miR-12136, hsa-miR-598-3p, hsa-miR-19a-3p, hsa-miR-122-5p, hsa-miR-153-3p, hsa-miR-590-3p, hsa-miR-500a-5p, hsa-miR-99a-3p, hsa-miR-130b-5p | 88.33 | 95.67 | 88.33 |
| 2096 | hsa-miR-4732-5p, hsa-miR-103a-2-5p, hsa-miR-192-5p, hsa-miR-215-5p, hsa-miR-590-5p, hsa-miR-590-3p, hsa-miR-500a-5p, hsa-miR-374a-5p, hsa-miR-337-5p | 88.33 | 91.69 | 92.78 |
| 2097 | hsa-miR-4732-5p, hsa-miR-3613-3p, hsa-miR-103a-2-5p, hsa-miR-1277-3p, age, hsa-miR-192-5p, hsa-miR-491-5p, hsa-miR-590-5p, hsa-miR-500a-5p, hsa-miR-374a-5p | 88.33 | 93.68 | 90.56 |
| 2098 | hsa-miR-374a-3p, hsa-miR-103a-2-5p, hsa-miR-548l, hsa-miR-192-5p, hsa-miR-433-3p, hsa-miR-12136, hsa-miR-598-3p, hsa-miR-19a-3p, hsa-miR-122-5p, hsa-miR-196b-5p, hsa-miR-215-5p, hsa-miR-590-5p, hsa-miR-590-3p, hsa-miR-500a-5p, hsa-miR-99a-3p, hsa-miR-130b-5p, hsa-miR-337-5p | 88.33 | 93.91 | 88.33 |
| 2099 | hsa-miR-4732-5p, hsa-miR-3613-3p, hsa-miR-103a-2-5p, hsa-miR-548l, age, hsa-miR-192-5p, hsa-miR-12136, hsa-miR-590-5p, hsa-miR-590-3p, hsa-miR-500a-5p, hsa-miR-374a-5p | 88.33 | 96.3 | 92.78 |
| 2100 | hsa-miR-4732-5p, hsa-miR-3613-3p, hsa-miR-1277-3p, age, hsa-miR-192-5p, hsa-miR-628-3p, hsa-miR-122-5p, hsa-miR-491-5p, hsa-miR-153-3p, hsa-miR-590-5p, hsa-miR-500a-5p, hsa-miR-374a-5p | 88.33 | 92.82 | 90.83 |
| 2101 | hsa-miR-4732-5p, hsa-miR-3613-3p, hsa-miR-103a-2-5p, hsa-miR-1277-3p, age, hsa-miR-192-5p, hsa-miR-628-3p, hsa-miR-122-5p, hsa-miR-491-5p, hsa-miR-153-3p, hsa-miR-590-5p, hsa-miR-500a-5p, hsa-miR-374a-5p | 86.67 | 93.56 | 88.61 |
| 2102 | hsa-miR-4732-5p, hsa-miR-3613-3p, hsa-miR-103a-2-5p, age, hsa-miR-192-5p, hsa-miR-433-3p, hsa-miR-590-5p, hsa-miR-590-3p, hsa-miR-500a-5p, hsa-miR-374a-5p, hsa-miR-99a-3p, hsa-miR-130b-5p | 86.67 | 92.71 | 88.89 |
| 2103 | hsa-miR-4732-5p, hsa-miR-3613-3p, hsa-miR-548l, hsa-miR-29c-5p, age, hsa-miR-12136, hsa-miR-628-3p, hsa-miR-122-5p, hsa-miR-153-3p, hsa-miR-590-3p, hsa-miR-374a-5p, hsa-miR-130b-5p | 86.67 | 93.45 | 85.83 |
| 2104 | hsa-miR-4732-5p, hsa-miR-3613-3p, hsa-miR-103a-2-5p, hsa-miR-29c-5p, age, hsa-miR-192-5p, hsa-miR-598-3p, hsa-miR-19a-3p, hsa-miR-590-5p, hsa-miR-590-3p, hsa-miR-500a-5p, hsa-miR-374a-5p, hsa-miR-99a-3p, hsa-miR-130b-5p | 86.67 | 93.68 | 84.17 |
| 2105 | hsa-miR-3613-3p, hsa-miR-103a-2-5p, age, hsa-miR-192-5p, hsa-miR-122-5p, hsa-miR-590-5p, hsa-miR-590-3p, hsa-miR-500a-5p, hsa-miR-374a-5p | 86.67 | 93.45 | 93.06 |
| 2106 | hsa-miR-4732-5p, hsa-miR-3613-3p, hsa-miR-103a-2-5p, hsa-miR-1277-3p, age, hsa-miR-181b-5p, hsa-miR-192-5p, hsa-miR-628-3p, hsa-miR-122-5p, hsa-miR-491-5p, hsa-miR-153-3p, hsa-miR-590-5p, hsa-miR-500a-5p, hsa-miR-374a-5p | 86.67 | 88.22 | 88.06 |
| 2107 | hsa-miR-4732-5p, hsa-miR-548l, hsa-miR-192-5p, hsa-miR-433-3p, hsa-miR-12136, hsa-miR-598-3p, hsa-miR-19a-3p, hsa-miR-196b-5p, hsa-miR-215-5p, hsa-miR-590-5p, hsa-miR-590-3p, hsa-miR-500a-5p, hsa-miR-99a-3p, hsa-miR-130b-5p, hsa-miR-337-5p | 86.67 | 94.19 | 90.83 |
| 2108 | hsa-miR-4732-5p, hsa-miR-3613-3p, hsa-miR-103a-2-5p, hsa-miR-548l, age, hsa-miR-192-5p, hsa-miR-598-3p, hsa-miR-19a-3p, hsa-miR-500a-5p, hsa-miR-374a-5p | 86.67 | 89.24 | 88.33 |
| 2109 | hsa-miR-4732-5p, hsa-miR-3613-3p, hsa-miR-103a-2-5p, hsa-miR-548l, age, hsa-miR-192-5p, hsa-miR-12136, hsa-miR-598-3p, hsa-miR-628-3p, hsa-miR-19a-3p, hsa-miR-153-3p, hsa-miR-590-3p, hsa-miR-500a-5p, hsa-miR-99a-3p, hsa-miR-130b-5p | 86.67 | 92.59 | 88.89 |
| 2110 | hsa-miR-4732-5p, hsa-miR-3613-3p, hsa-miR-103a-2-5p, hsa-miR-29c-5p, age, hsa-miR-192-5p, hsa-miR-122-5p, hsa-miR-590-5p, hsa-miR-590-3p, hsa-miR-500a-5p, hsa-miR-99a-3p, hsa-miR-130b-5p | 86.67 | 93.45 | 86.11 |
| 2111 | hsa-miR-4732-5p, hsa-miR-3613-3p, hsa-miR-103a-2-5p, hsa-miR-29c-5p, age, hsa-miR-192-5p, hsa-miR-598-3p, hsa-miR-19a-3p, hsa-miR-590-5p, hsa-miR-590-3p, hsa-miR-500a-5p, hsa-miR-99a-3p, hsa-miR-130b-5p | 86.67 | 91.46 | 86.39 |
| 2112 | hsa-miR-4732-5p, hsa-miR-3613-3p, hsa-miR-103a-2-5p, age, hsa-miR-181b-5p, hsa-miR-192-5p, hsa-miR-590-5p, hsa-miR-590-3p, hsa-miR-374a-5p | 86.67 | 90.83 | 85.56 |
| 2113 | hsa-miR-4732-5p, hsa-miR-3613-3p, hsa-miR-548l, hsa-miR-1277-3p, hsa-miR-29c-5p, age, hsa-miR-12136, hsa-miR-598-3p, hsa-miR-628-3p, hsa-miR-19a-3p, hsa-miR-122-5p, hsa-miR-153-3p, hsa-miR-500a-5p, hsa-miR-99a-3p, hsa-miR-130b-5p | 86.67 | 94.07 | 83.89 |
| 2114 | hsa-miR-4732-5p, hsa-miR-3613-3p, hsa-miR-103a-2-5p, hsa-miR-29c-5p, age, hsa-miR-192-5p, hsa-miR-598-3p, hsa-miR-19a-3p, hsa-miR-122-5p, hsa-miR-590-5p, hsa-miR-590-3p, hsa-miR-500a-5p, hsa-miR-99a-3p, hsa-miR-130b-5p | 86.67 | 91.57 | 86.11 |
| 2115 | hsa-miR-374a-3p, hsa-miR-3613-3p, hsa-miR-103a-2-5p, age, hsa-miR-192-5p, hsa-miR-590-5p, hsa-miR-590-3p | 86.67 | 88.73 | 95.28 |
| 2116 | hsa-miR-374a-3p, hsa-miR-103a-2-5p, hsa-miR-548l, hsa-miR-181b-5p, hsa-miR-192-5p, hsa-miR-433-3p, hsa-miR-12136, hsa-miR-598-3p, hsa-miR-19a-3p, hsa-miR-196b-5p, hsa-miR-215-5p, hsa-miR-491-5p, hsa-miR-590-5p, hsa-miR-590-3p, hsa-miR-500a-5p, hsa-miR-337-5p | 86.67 | 95.51 | 90.56 |
| 2117 | hsa-miR-374a-3p, hsa-miR-3613-3p, hsa-miR-103a-2-5p, age, hsa-miR-181b-5p, hsa-miR-192-5p, hsa-miR-590-5p, hsa-miR-590-3p, hsa-miR-374a-5p | 86.67 | 82.25 | 93.06 |
| 2118 | hsa-miR-374a-3p, hsa-miR-3613-3p, hsa-miR-103a-2-5p, age, hsa-miR-192-5p, hsa-miR-12136, hsa-miR-122-5p, hsa-miR-590-5p, hsa-miR-590-3p, hsa-miR-374a-5p | 86.67 | 92.66 | 88.06 |
| 2119 | hsa-miR-103a-2-5p, hsa-miR-548l, hsa-miR-1277-3p, hsa-miR-192-5p, hsa-miR-12136, hsa-miR-598-3p, hsa-miR-19a-3p, hsa-miR-196b-5p, hsa-miR-215-5p, hsa-miR-590-5p, hsa-miR-590-3p, hsa-miR-500a-5p, hsa-miR-337-5p | 86.67 | 94.81 | 85.83 |
| 2120 | hsa-miR-4732-5p, hsa-miR-103a-2-5p, hsa-miR-548l, hsa-miR-29c-5p, hsa-miR-433-3p, hsa-miR-598-3p, hsa-miR-19a-3p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 86.67 | 95.28 | 87.78 |
| 2121 | hsa-miR-4732-5p, hsa-miR-3613-3p, hsa-miR-103a-2-5p, hsa-miR-548l, hsa-miR-29c-5p, age, hsa-miR-12136, hsa-miR-628-3p, hsa-miR-19a-3p, hsa-miR-153-3p, hsa-miR-590-3p | 86.67 | 86.16 | 88.89 |
| 2122 | hsa-miR-4732-5p, hsa-miR-3613-3p, hsa-miR-103a-2-5p, hsa-miR-1277-3p, age, hsa-miR-181b-5p, hsa-miR-192-5p, hsa-miR-628-3p, hsa-miR-491-5p, hsa-miR-153-3p, hsa-miR-590-5p, hsa-miR-500a-5p, hsa-miR-374a-5p | 86.67 | 87.36 | 92.78 |
| 2123 | hsa-miR-4732-5p, hsa-miR-3613-3p, hsa-miR-103a-2-5p, hsa-miR-548l, hsa-miR-29c-5p, age, hsa-miR-628-3p, hsa-miR-122-5p, hsa-miR-153-3p, hsa-miR-590-3p | 86.67 | 88.38 | 92.5 |
| 2124 | hsa-miR-374a-3p, hsa-miR-3613-3p, hsa-miR-103a-2-5p, hsa-miR-548l, age, hsa-miR-192-5p, hsa-miR-12136, hsa-miR-122-5p, hsa-miR-590-5p, hsa-miR-590-3p, hsa-miR-500a-5p | 86.67 | 91.57 | 88.33 |
| 2125 | hsa-miR-4732-5p, hsa-miR-3613-3p, hsa-miR-103a-2-5p, hsa-miR-548l, hsa-miR-29c-5p, age, hsa-miR-628-3p, hsa-miR-122-5p, hsa-miR-153-3p, hsa-miR-590-5p, hsa-miR-590-3p | 86.67 | 84.12 | 90 |
| 2126 | hsa-miR-4732-5p, hsa-miR-3613-3p, hsa-miR-103a-2-5p, hsa-miR-1277-3p, age, hsa-miR-181b-5p, hsa-miR-192-5p, hsa-miR-122-5p, hsa-miR-491-5p, hsa-miR-153-3p, hsa-miR-590-5p, hsa-miR-500a-5p, hsa-miR-374a-5p | 86.67 | 91.57 | 87.78 |
| 2127 | hsa-miR-374a-3p, hsa-miR-3613-3p, hsa-miR-103a-2-5p, hsa-miR-548l, age, hsa-miR-192-5p, hsa-miR-12136, hsa-miR-590-5p, hsa-miR-590-3p, hsa-miR-500a-5p, hsa-miR-374a-5p | 86.67 | 92.43 | 88.06 |
| 2128 | hsa-miR-4732-5p, hsa-miR-3613-3p, hsa-miR-103a-2-5p, hsa-miR-548l, hsa-miR-29c-5p, age, hsa-miR-12136, hsa-miR-628-3p, hsa-miR-122-5p, hsa-miR-153-3p, hsa-miR-590-3p | 86.67 | 92.71 | 88.06 |
| 2129 | hsa-miR-4732-5p, hsa-miR-3613-3p, age, hsa-miR-192-5p, hsa-miR-12136, hsa-miR-598-3p, hsa-miR-628-3p, hsa-miR-19a-3p, hsa-miR-500a-5p | 86.67 | 89.63 | 88.61 |
| 2130 | hsa-miR-4732-5p, hsa-miR-3613-3p, hsa-miR-103a-2-5p, hsa-miR-548l, age, hsa-miR-192-5p, hsa-miR-12136, hsa-miR-628-3p, hsa-miR-122-5p, hsa-miR-590-3p, hsa-miR-500a-5p, hsa-miR-374a-5p | 86.67 | 92.2 | 88.33 |
| 2131 | hsa-miR-4732-5p, hsa-miR-3613-3p, hsa-miR-103a-2-5p, hsa-miR-1277-3p, age, hsa-miR-192-5p, hsa-miR-122-5p, hsa-miR-491-5p, hsa-miR-590-5p, hsa-miR-500a-5p | 86.67 | 93.8 | 88.33 |
| 2132 | hsa-miR-4732-5p, hsa-miR-3613-3p, hsa-miR-103a-2-5p, hsa-miR-1277-3p, age, hsa-miR-181b-5p, hsa-miR-192-5p, hsa-miR-491-5p, hsa-miR-590-5p, hsa-miR-374a-5p | 86.67 | 91.81 | 87.78 |
| 2133 | hsa-miR-374a-3p, hsa-miR-3613-3p, hsa-miR-103a-2-5p, hsa-miR-548l, hsa-miR-29c-5p, age, hsa-miR-12136, hsa-miR-598-3p, hsa-miR-19a-3p, hsa-miR-122-5p, hsa-miR-590-3p, hsa-miR-500a-5p, hsa-miR-99a-3p, hsa-miR-130b-5p | 86.67 | 96.41 | 83.61 |
| 2134 | hsa-miR-374a-3p, hsa-miR-103a-2-5p, hsa-miR-192-5p, hsa-miR-433-3p, hsa-miR-12136, hsa-miR-598-3p, hsa-miR-19a-3p, hsa-miR-122-5p, hsa-miR-196b-5p, hsa-miR-215-5p, hsa-miR-590-5p, hsa-miR-590-3p, hsa-miR-500a-5p, hsa-miR-337-5p | 86.67 | 92.15 | 85.56 |
| 2135 | hsa-miR-4732-5p, hsa-miR-3613-3p, hsa-miR-103a-2-5p, hsa-miR-548l, hsa-miR-29c-5p, age, hsa-miR-598-3p, hsa-miR-628-3p, hsa-miR-19a-3p, hsa-miR-590-3p, hsa-miR-500a-5p, hsa-miR-374a-5p, hsa-miR-99a-3p, hsa-miR-130b-5p | 86.67 | 93.33 | 86.39 |
| 2136 | hsa-miR-4732-5p, hsa-miR-3613-3p, hsa-miR-103a-2-5p, hsa-miR-29c-5p, age, hsa-miR-192-5p, hsa-miR-598-3p, hsa-miR-19a-3p, hsa-miR-122-5p, hsa-miR-590-5p, hsa-miR-590-3p, hsa-miR-500a-5p, hsa-miR-374a-5p, hsa-miR-99a-3p, hsa-miR-130b-5p | 86.67 | 95.67 | 86.39 |
| 2137 | hsa-miR-4732-5p, hsa-miR-3613-3p, age, hsa-miR-192-5p, hsa-miR-628-3p, hsa-miR-122-5p, hsa-miR-153-3p, hsa-miR-590-5p, hsa-miR-590-3p, hsa-miR-500a-5p, hsa-miR-374a-5p | 86.67 | 91.57 | 95.28 |
| 2138 | hsa-miR-4732-5p, hsa-miR-3613-3p, age, hsa-miR-192-5p, hsa-miR-590-5p, hsa-miR-590-3p, hsa-miR-500a-5p, hsa-miR-374a-5p | 86.67 | 94.93 | 88.61 |
| 2139 | hsa-miR-374a-3p, hsa-miR-3613-3p, hsa-miR-103a-2-5p, age, hsa-miR-192-5p, hsa-miR-12136, hsa-miR-598-3p, hsa-miR-19a-3p, hsa-miR-122-5p, hsa-miR-590-3p, hsa-miR-500a-5p, hsa-miR-374a-5p | 86.67 | 96.41 | 88.33 |
| 2140 | hsa-miR-4732-5p, hsa-miR-1277-3p, age, hsa-miR-192-5p, hsa-miR-12136, hsa-miR-628-3p, hsa-miR-196b-5p, hsa-miR-215-5p, hsa-miR-153-3p, hsa-miR-500a-5p, hsa-miR-374a-5p, hsa-miR-337-5p | 86.67 | 96.41 | 87.78 |
| 2141 | hsa-miR-4732-5p, hsa-miR-3613-3p, hsa-miR-103a-2-5p, hsa-miR-29c-5p, age, hsa-miR-192-5p, hsa-miR-433-3p, hsa-miR-122-5p, hsa-miR-590-5p, hsa-miR-590-3p, hsa-miR-500a-5p, hsa-miR-374a-5p, hsa-miR-99a-3p, hsa-miR-130b-5p | 86.67 | 93.56 | 85.83 |
| 2142 | hsa-miR-4732-5p, hsa-miR-3613-3p, hsa-miR-103a-2-5p, hsa-miR-548l, age, hsa-miR-181b-5p, hsa-miR-192-5p, hsa-miR-12136, hsa-miR-598-3p, hsa-miR-19a-3p, hsa-miR-122-5p, hsa-miR-491-5p, hsa-miR-590-3p | 86.67 | 87.41 | 88.61 |
| 2143 | hsa-miR-4732-5p, hsa-miR-3613-3p, hsa-miR-29c-5p, age, hsa-miR-192-5p, hsa-miR-433-3p, hsa-miR-122-5p, hsa-miR-590-5p, hsa-miR-590-3p, hsa-miR-500a-5p, hsa-miR-374a-5p, hsa-miR-99a-3p, hsa-miR-130b-5p | 86.67 | 92.2 | 86.11 |
| 2144 | hsa-miR-3613-3p, hsa-miR-103a-2-5p, hsa-miR-548l, hsa-miR-29c-5p, age, hsa-miR-192-5p, hsa-miR-12136, hsa-miR-122-5p, hsa-miR-590-3p, hsa-miR-500a-5p, hsa-miR-374a-5p, hsa-miR-99a-3p, hsa-miR-130b-5p | 86.67 | 93.33 | 88.61 |
| 2145 | hsa-miR-4732-5p, hsa-miR-103a-2-5p, hsa-miR-192-5p, hsa-miR-433-3p, hsa-miR-598-3p, hsa-miR-19a-3p, hsa-miR-196b-5p, hsa-miR-215-5p, hsa-miR-590-5p, hsa-miR-500a-5p, hsa-miR-374a-5p, hsa-miR-337-5p | 85 | 92.43 | 88.61 |
| 2146 | hsa-miR-4732-5p, hsa-miR-3613-3p, age, hsa-miR-192-5p, hsa-miR-122-5p, hsa-miR-153-3p, hsa-miR-590-3p, hsa-miR-500a-5p, hsa-miR-374a-5p | 85 | 91.11 | 92.78 |
| 2147 | hsa-miR-4732-5p, hsa-miR-548l, hsa-miR-192-5p, hsa-miR-433-3p, hsa-miR-12136, hsa-miR-598-3p, hsa-miR-19a-3p, hsa-miR-196b-5p, hsa-miR-215-5p, hsa-miR-491-5p, hsa-miR-500a-5p | 85 | 90.72 | 88.33 |
| 2148 | hsa-miR-4732-5p, hsa-miR-3613-3p, hsa-miR-103a-2-5p, hsa-miR-548l, hsa-miR-1277-3p, hsa-miR-29c-5p, hsa-miR-181b-5p, hsa-miR-628-3p, hsa-miR-122-5p, hsa-miR-196b-5p, hsa-miR-491-5p, hsa-miR-153-3p, hsa-miR-590-5p, hsa-miR-99a-3p, hsa-miR-337-5p | 85 | 88.22 | 88.61 |
| 2149 | hsa-miR-374a-3p, hsa-miR-103a-2-5p, hsa-miR-548l, hsa-miR-181b-5p, hsa-miR-192-5p, hsa-miR-433-3p, hsa-miR-598-3p, hsa-miR-19a-3p, hsa-miR-122-5p, hsa-miR-196b-5p, hsa-miR-215-5p, hsa-miR-491-5p, hsa-miR-590-5p, hsa-miR-590-3p, hsa-miR-500a-5p, hsa-miR-337-5p | 85 | 88.45 | 88.33 |
| 2150 | hsa-miR-4732-5p, hsa-miR-3613-3p, hsa-miR-103a-2-5p, hsa-miR-29c-5p, age, hsa-miR-192-5p, hsa-miR-590-5p, hsa-miR-590-3p, hsa-miR-500a-5p, hsa-miR-374a-5p, hsa-miR-99a-3p, hsa-miR-130b-5p | 85 | 92.94 | 83.89 |
| 2151 | hsa-miR-4732-5p, hsa-miR-3613-3p, hsa-miR-548l, hsa-miR-12136, hsa-miR-628-3p, hsa-miR-19a-3p, hsa-miR-590-5p, hsa-miR-590-3p, hsa-miR-374a-5p | 85 | 90.21 | 90.56 |
| 2152 | hsa-miR-374a-3p, hsa-miR-3613-3p, hsa-miR-103a-2-5p, age, hsa-miR-192-5p, hsa-miR-122-5p, hsa-miR-590-5p, hsa-miR-590-3p, hsa-miR-500a-5p, hsa-miR-374a-5p | 85 | 88.56 | 93.06 |
| 2153 | hsa-miR-4732-5p, hsa-miR-3613-3p, hsa-miR-103a-2-5p, age, hsa-miR-192-5p, hsa-miR-12136, hsa-miR-122-5p, hsa-miR-590-3p, hsa-miR-500a-5p, hsa-miR-374a-5p | 85 | 94.81 | 87.78 |
| 2154 | hsa-miR-4732-5p, hsa-miR-433-3p, hsa-miR-628-3p, hsa-miR-122-5p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 85 | 86.85 | 90.83 |
| 2155 | hsa-miR-374a-3p, hsa-miR-3613-3p, hsa-miR-103a-2-5p, age, hsa-miR-192-5p, hsa-miR-590-5p, hsa-miR-590-3p, hsa-miR-500a-5p, hsa-miR-374a-5p | 85 | 93.06 | 93.06 |
| 2156 | hsa-miR-4732-5p, hsa-miR-3613-3p, hsa-miR-103a-2-5p, age, hsa-miR-181b-5p, hsa-miR-192-5p, hsa-miR-122-5p, hsa-miR-491-5p, hsa-miR-590-5p, hsa-miR-590-3p, hsa-miR-374a-5p | 85 | 87.36 | 88.06 |
| 2157 | hsa-miR-4732-5p, hsa-miR-3613-3p, hsa-miR-103a-2-5p, hsa-miR-548l, age, hsa-miR-192-5p, hsa-miR-12136, hsa-miR-590-3p, hsa-miR-500a-5p, hsa-miR-374a-5p | 85 | 94.81 | 87.78 |
| 2158 | hsa-miR-3613-3p, hsa-miR-103a-2-5p, hsa-miR-548l, age, hsa-miR-192-5p, hsa-miR-12136, hsa-miR-122-5p, hsa-miR-590-5p, hsa-miR-590-3p, hsa-miR-500a-5p, hsa-miR-374a-5p | 85 | 93.56 | 90.28 |
| 2159 | hsa-miR-4732-5p, hsa-miR-374a-3p, hsa-miR-103a-2-5p, hsa-miR-548l, hsa-miR-1277-3p, hsa-miR-29c-5p, hsa-miR-122-5p, hsa-miR-215-5p, hsa-miR-491-5p, hsa-miR-590-5p, hsa-miR-590-3p, hsa-miR-500a-5p, hsa-miR-99a-3p | 85 | 84.75 | 85.83 |
| 2160 | hsa-miR-374a-3p, hsa-miR-103a-2-5p, hsa-miR-548l, hsa-miR-29c-5p, hsa-miR-433-3p, hsa-miR-12136, hsa-miR-122-5p, hsa-miR-196b-5p, hsa-miR-215-5p, hsa-miR-590-5p, hsa-miR-590-3p, hsa-miR-500a-5p, hsa-miR-99a-3p, hsa-miR-130b-5p, hsa-miR-337-5p | 85 | 91.3 | 81.39 |
| 2161 | hsa-miR-548l, hsa-miR-29c-5p, age, hsa-miR-192-5p, hsa-miR-598-3p, hsa-miR-122-5p, hsa-miR-196b-5p, hsa-miR-215-5p, hsa-miR-153-3p, hsa-miR-500a-5p, hsa-miR-374a-5p, hsa-miR-99a-3p, hsa-miR-130b-5p, hsa-miR-337-5p | 85 | 94.07 | 86.67 |
| 2162 | hsa-miR-4732-5p, hsa-miR-3613-3p, hsa-miR-103a-2-5p, age, hsa-miR-181b-5p, hsa-miR-192-5p, hsa-miR-122-5p, hsa-miR-491-5p, hsa-miR-590-5p, hsa-miR-590-3p | 85 | 91.97 | 88.33 |
| 2163 | hsa-miR-4732-5p, hsa-miR-3613-3p, hsa-miR-103a-2-5p, age, hsa-miR-192-5p, hsa-miR-433-3p, hsa-miR-12136, hsa-miR-598-3p, hsa-miR-628-3p, hsa-miR-19a-3p, hsa-miR-590-3p, hsa-miR-500a-5p | 85 | 92.71 | 88.33 |
| 2164 | hsa-miR-4732-5p, hsa-miR-3613-3p, hsa-miR-103a-2-5p, age, hsa-miR-181b-5p, hsa-miR-192-5p, hsa-miR-433-3p, hsa-miR-12136, hsa-miR-598-3p, hsa-miR-19a-3p, hsa-miR-491-5p, hsa-miR-590-3p, hsa-miR-500a-5p, hsa-miR-130b-5p | 85 | 92.59 | 86.11 |
| 2165 | hsa-miR-374a-3p, hsa-miR-3613-3p, hsa-miR-103a-2-5p, hsa-miR-548l, age, hsa-miR-192-5p, hsa-miR-12136, hsa-miR-122-5p, hsa-miR-590-5p, hsa-miR-590-3p, hsa-miR-500a-5p, hsa-miR-374a-5p | 85 | 92.43 | 88.06 |
| 2166 | hsa-miR-4732-5p, hsa-miR-433-3p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 85 | 91.57 | 88.33 |
| 2167 | hsa-miR-4732-5p, hsa-miR-3613-3p, hsa-miR-103a-2-5p, hsa-miR-548l, hsa-miR-1277-3p, age, hsa-miR-181b-5p, hsa-miR-192-5p, hsa-miR-12136, hsa-miR-122-5p, hsa-miR-491-5p, hsa-miR-374a-5p | 85 | 91.57 | 85.28 |
| 2168 | hsa-miR-4732-5p, hsa-miR-103a-2-5p, hsa-miR-181b-5p, hsa-miR-192-5p, hsa-miR-433-3p, hsa-miR-12136, hsa-miR-598-3p, hsa-miR-19a-3p, hsa-miR-196b-5p, hsa-miR-215-5p, hsa-miR-491-5p, hsa-miR-590-5p, hsa-miR-590-3p, hsa-miR-500a-5p, hsa-miR-337-5p | 85 | 92.31 | 88.33 |
| 2169 | hsa-miR-4732-5p, hsa-miR-3613-3p, hsa-miR-103a-2-5p, hsa-miR-548l, age, hsa-miR-12136, hsa-miR-628-3p, hsa-miR-153-3p, hsa-miR-590-3p | 85 | 87.41 | 86.11 |
| 2170 | hsa-miR-4732-5p, hsa-miR-3613-3p, hsa-miR-103a-2-5p, hsa-miR-1277-3p, age, hsa-miR-181b-5p, hsa-miR-192-5p, hsa-miR-491-5p, hsa-miR-590-5p, hsa-miR-500a-5p, hsa-miR-374a-5p | 85 | 90.95 | 86.11 |
| 2171 | hsa-miR-4732-5p, hsa-miR-103a-2-5p, hsa-miR-433-3p, hsa-miR-628-3p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 85 | 87.48 | 90.83 |
| 2172 | hsa-miR-374a-3p, hsa-miR-3613-3p, hsa-miR-103a-2-5p, hsa-miR-548l, hsa-miR-29c-5p, age, hsa-miR-12136, hsa-miR-122-5p, hsa-miR-590-3p, hsa-miR-500a-5p, hsa-miR-99a-3p, hsa-miR-130b-5p | 85 | 89.24 | 88.06 |
| 2173 | hsa-miR-374a-3p, hsa-miR-103a-2-5p, hsa-miR-548l, hsa-miR-29c-5p, hsa-miR-433-3p, hsa-miR-598-3p, hsa-miR-19a-3p, hsa-miR-122-5p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 85 | 87.94 | 81.11 |
| 2174 | hsa-miR-3613-3p, hsa-miR-103a-2-5p, hsa-miR-548l, hsa-miR-1277-3p, age, hsa-miR-192-5p, hsa-miR-12136, hsa-miR-598-3p, hsa-miR-628-3p, hsa-miR-19a-3p, hsa-miR-590-3p, hsa-miR-500a-5p | 85 | 97.78 | 83.61 |
| 2175 | hsa-miR-4732-5p, hsa-miR-103a-2-5p, hsa-miR-548l, hsa-miR-433-3p, hsa-miR-12136, hsa-miR-598-3p, hsa-miR-628-3p, hsa-miR-19a-3p, hsa-miR-122-5p, hsa-miR-215-5p, hsa-miR-491-5p, hsa-miR-153-3p, hsa-miR-590-5p, hsa-miR-500a-5p, hsa-miR-374a-5p, hsa-miR-130b-5p, hsa-miR-337-5p | 83.33 | 92.82 | 81.39 |
| 2176 | hsa-miR-4732-5p, hsa-miR-3613-3p, hsa-miR-103a-2-5p, hsa-miR-548l, age, hsa-miR-192-5p, hsa-miR-12136, hsa-miR-122-5p, hsa-miR-590-3p, hsa-miR-500a-5p, hsa-miR-374a-5p | 83.33 | 94.81 | 85.28 |
| 2177 | hsa-miR-4732-5p, hsa-miR-3613-3p, hsa-miR-103a-2-5p, hsa-miR-548l, hsa-miR-29c-5p, age, hsa-miR-590-3p, hsa-miR-500a-5p, hsa-miR-374a-5p, hsa-miR-99a-3p, hsa-miR-130b-5p | 83.33 | 92.08 | 86.39 |
| 2178 | hsa-miR-4732-5p, hsa-miR-3613-3p, hsa-miR-103a-2-5p, hsa-miR-29c-5p, age, hsa-miR-12136, hsa-miR-598-3p, hsa-miR-628-3p, hsa-miR-19a-3p, hsa-miR-590-3p, hsa-miR-500a-5p, hsa-miR-99a-3p, hsa-miR-130b-5p | 83.33 | 84.56 | 86.67 |
| 2179 | hsa-miR-374a-3p, hsa-miR-3613-3p, hsa-miR-1277-3p, hsa-miR-29c-5p, age, hsa-miR-181b-5p, hsa-miR-192-5p, hsa-miR-12136, hsa-miR-598-3p, hsa-miR-628-3p, hsa-miR-122-5p, hsa-miR-196b-5p, hsa-miR-153-3p, hsa-miR-590-5p, hsa-miR-500a-5p, hsa-miR-374a-5p, hsa-miR-130b-5p, hsa-miR-337-5p | 83.33 | 88.29 | 81.11 |
| 2180 | hsa-miR-4732-5p, hsa-miR-3613-3p, hsa-miR-1277-3p, age, hsa-miR-181b-5p, hsa-miR-192-5p, hsa-miR-628-3p, hsa-miR-122-5p, hsa-miR-491-5p, hsa-miR-153-3p, hsa-miR-590-5p, hsa-miR-500a-5p, hsa-miR-374a-5p | 83.33 | 90.09 | 83.33 |
| 2181 | hsa-miR-374a-3p, hsa-miR-3613-3p, hsa-miR-103a-2-5p, hsa-miR-548l, age, hsa-miR-433-3p, hsa-miR-12136, hsa-miR-598-3p, hsa-miR-19a-3p, hsa-miR-122-5p, hsa-miR-590-3p, hsa-miR-500a-5p | 83.33 | 93.29 | 85.56 |
| 2182 | hsa-miR-374a-3p, hsa-miR-3613-3p, hsa-miR-103a-2-5p, hsa-miR-548l, age, hsa-miR-181b-5p, hsa-miR-192-5p, hsa-miR-12136, hsa-miR-598-3p, hsa-miR-19a-3p, hsa-miR-122-5p, hsa-miR-491-5p, hsa-miR-590-3p | 83.33 | 86 | 85.83 |
| 2183 | hsa-miR-4732-5p, hsa-miR-29c-5p, hsa-miR-433-3p, hsa-miR-19a-3p, hsa-miR-196b-5p, hsa-miR-215-5p, hsa-miR-590-5p, hsa-miR-590-3p, hsa-miR-500a-5p, hsa-miR-374a-5p, hsa-miR-99a-3p, hsa-miR-130b-5p, hsa-miR-337-5p | 83.33 | 90.44 | 83.61 |
| 2184 | hsa-miR-4732-5p, hsa-miR-374a-3p, hsa-miR-3613-3p, hsa-miR-103a-2-5p, age, hsa-miR-181b-5p, hsa-miR-192-5p, hsa-miR-12136, hsa-miR-598-3p, hsa-miR-19a-3p, hsa-miR-590-3p, hsa-miR-500a-5p, hsa-miR-99a-3p, hsa-miR-130b-5p | 83.33 | 90.49 | 81.39 |
| 2185 | hsa-miR-4732-5p, hsa-miR-3613-3p, hsa-miR-1277-3p, hsa-miR-29c-5p, age, hsa-miR-181b-5p, hsa-miR-192-5p, hsa-miR-433-3p, hsa-miR-12136, hsa-miR-628-3p, hsa-miR-196b-5p, hsa-miR-590-3p, hsa-miR-99a-3p, hsa-miR-130b-5p, hsa-miR-337-5p | 83.33 | 91.34 | 85.83 |
| 2186 | hsa-miR-4732-5p, hsa-miR-3613-3p, hsa-miR-103a-2-5p, hsa-miR-29c-5p, age, hsa-miR-433-3p, hsa-miR-122-5p, hsa-miR-590-5p, hsa-miR-590-3p, hsa-miR-500a-5p, hsa-miR-374a-5p, hsa-miR-99a-3p, hsa-miR-130b-5p | 83.33 | 91.46 | 83.89 |
| 2187 | hsa-miR-4732-5p, hsa-miR-3613-3p, hsa-miR-103a-2-5p, hsa-miR-548l, hsa-miR-29c-5p, age, hsa-miR-181b-5p, hsa-miR-12136, hsa-miR-598-3p, hsa-miR-19a-3p, hsa-miR-122-5p, hsa-miR-491-5p, hsa-miR-590-3p, hsa-miR-99a-3p, hsa-miR-130b-5p | 83.33 | 91.23 | 86.39 |
| 2188 | hsa-miR-4732-5p, hsa-miR-3613-3p, hsa-miR-103a-2-5p, age, hsa-miR-181b-5p, hsa-miR-192-5p, hsa-miR-122-5p, hsa-miR-590-5p, hsa-miR-590-3p, hsa-miR-374a-5p | 83.33 | 90.09 | 85.56 |
| 2189 | hsa-miR-4732-5p, hsa-miR-103a-2-5p, hsa-miR-548l, hsa-miR-192-5p, hsa-miR-433-3p, hsa-miR-12136, hsa-miR-196b-5p, hsa-miR-215-5p, hsa-miR-590-5p, hsa-miR-590-3p, hsa-miR-500a-5p, hsa-miR-337-5p | 83.33 | 90.83 | 80.83 |
| 2190 | hsa-miR-374a-3p, hsa-miR-103a-2-5p, hsa-miR-548l, hsa-miR-192-5p, hsa-miR-433-3p, hsa-miR-12136, hsa-miR-598-3p, hsa-miR-19a-3p, hsa-miR-196b-5p, hsa-miR-215-5p, hsa-miR-153-3p, hsa-miR-590-5p, hsa-miR-590-3p, hsa-miR-99a-3p, hsa-miR-130b-5p, hsa-miR-337-5p | 83.33 | 91.06 | 86.11 |
| 2191 | hsa-miR-4732-5p, hsa-miR-3613-3p, hsa-miR-103a-2-5p, hsa-miR-29c-5p, age, hsa-miR-192-5p, hsa-miR-598-3p, hsa-miR-628-3p, hsa-miR-19a-3p, hsa-miR-590-5p, hsa-miR-590-3p, hsa-miR-500a-5p, hsa-miR-374a-5p, hsa-miR-99a-3p, hsa-miR-130b-5p | 83.33 | 91.06 | 84.17 |
| 2192 | hsa-miR-374a-3p, hsa-miR-103a-2-5p, hsa-miR-548l, hsa-miR-1277-3p, hsa-miR-181b-5p, hsa-miR-192-5p, hsa-miR-433-3p, hsa-miR-598-3p, hsa-miR-19a-3p, hsa-miR-122-5p, hsa-miR-196b-5p, hsa-miR-215-5p, hsa-miR-491-5p, hsa-miR-590-5p, hsa-miR-590-3p, hsa-miR-500a-5p, hsa-miR-374a-5p, hsa-miR-337-5p | 83.33 | 88.06 | 83.33 |
| 2193 | hsa-miR-3613-3p, hsa-miR-103a-2-5p, age, hsa-miR-181b-5p, hsa-miR-192-5p, hsa-miR-491-5p, hsa-miR-590-5p, hsa-miR-590-3p, hsa-miR-500a-5p, hsa-miR-374a-5p | 83.33 | 84 | 90.56 |
| 2194 | hsa-miR-4732-5p, hsa-miR-548l, hsa-miR-1277-3p, hsa-miR-192-5p, hsa-miR-12136, hsa-miR-598-3p, hsa-miR-628-3p, hsa-miR-19a-3p, hsa-miR-122-5p, hsa-miR-196b-5p, hsa-miR-215-5p, hsa-miR-153-3p, hsa-miR-590-5p, hsa-miR-500a-5p, hsa-miR-337-5p | 83.33 | 89.98 | 88.61 |
| 2195 | hsa-miR-4732-5p, hsa-miR-3613-3p, hsa-miR-103a-2-5p, hsa-miR-548l, hsa-miR-1277-3p, age, hsa-miR-181b-5p, hsa-miR-192-5p, hsa-miR-598-3p, hsa-miR-19a-3p, hsa-miR-122-5p, hsa-miR-491-5p, hsa-miR-590-5p | 83.33 | 91.81 | 83.33 |
| 2196 | hsa-miR-4732-5p, hsa-miR-103a-2-5p, hsa-miR-433-3p, hsa-miR-196b-5p, hsa-miR-215-5p, hsa-miR-590-5p, hsa-miR-590-3p, hsa-miR-500a-5p, hsa-miR-337-5p | 83.33 | 92.08 | 88.33 |
| 2197 | hsa-miR-4732-5p, hsa-miR-3613-3p, hsa-miR-103a-2-5p, age, hsa-miR-181b-5p, hsa-miR-192-5p, hsa-miR-122-5p, hsa-miR-491-5p, hsa-miR-590-5p, hsa-miR-590-3p, hsa-miR-500a-5p | 83.33 | 87.36 | 90.56 |
| 2198 | hsa-miR-4732-5p, hsa-miR-103a-2-5p, hsa-miR-433-3p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 83.33 | 90.83 | 88.33 |
| 2199 | hsa-miR-4732-5p, hsa-miR-3613-3p, hsa-miR-103a-2-5p, age, hsa-miR-181b-5p, hsa-miR-192-5p, hsa-miR-491-5p, hsa-miR-590-5p, hsa-miR-590-3p, hsa-miR-500a-5p, hsa-miR-374a-5p | 83.33 | 89.47 | 88.06 |
| 2200 | hsa-miR-4732-5p, hsa-miR-433-3p, hsa-miR-628-3p, hsa-miR-122-5p, hsa-miR-196b-5p, hsa-miR-215-5p, hsa-miR-153-3p, hsa-miR-590-5p, hsa-miR-590-3p, hsa-miR-500a-5p, hsa-miR-337-5p | 83.33 | 88.26 | 88.89 |
| 2201 | hsa-miR-4732-5p, hsa-miR-3613-3p, hsa-miR-103a-2-5p, hsa-miR-29c-5p, age, hsa-miR-12136, hsa-miR-598-3p, hsa-miR-19a-3p, hsa-miR-590-3p, hsa-miR-500a-5p, hsa-miR-99a-3p, hsa-miR-130b-5p | 81.67 | 89.86 | 84.17 |
| 2202 | hsa-miR-374a-3p, hsa-miR-3613-3p, hsa-miR-103a-2-5p, hsa-miR-548l, age, hsa-miR-192-5p, hsa-miR-12136, hsa-miR-590-3p, hsa-miR-500a-5p, hsa-miR-374a-5p | 81.67 | 89.58 | 85.56 |
| 2203 | hsa-miR-4732-5p, hsa-miR-1277-3p, hsa-miR-433-3p, hsa-miR-196b-5p, hsa-miR-215-5p, hsa-miR-153-3p, hsa-miR-590-5p, hsa-miR-500a-5p, hsa-miR-374a-5p, hsa-miR-337-5p | 81.67 | 87.59 | 81.39 |
| 2204 | hsa-miR-374a-3p, hsa-miR-3613-3p, hsa-miR-103a-2-5p, hsa-miR-548l, age, hsa-miR-192-5p, hsa-miR-598-3p, hsa-miR-628-3p, hsa-miR-19a-3p, hsa-miR-590-3p, hsa-miR-500a-5p, hsa-miR-374a-5p, hsa-miR-99a-3p, hsa-miR-130b-5p | 81.67 | 90.83 | 83.89 |
| 2205 | hsa-miR-4732-5p, hsa-miR-192-5p, hsa-miR-122-5p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 81.67 | 86.74 | 85.56 |
| 2206 | hsa-miR-4732-5p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 81.67 | 87.48 | 85.83 |
| 2207 | hsa-miR-4732-5p, hsa-miR-103a-2-5p, hsa-miR-192-5p, hsa-miR-433-3p, hsa-miR-122-5p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 81.67 | 91.46 | 88.33 |
| 2208 | hsa-miR-4732-5p, hsa-miR-103a-2-5p, hsa-miR-433-3p, hsa-miR-122-5p, hsa-miR-196b-5p, hsa-miR-215-5p, hsa-miR-590-5p, hsa-miR-590-3p, hsa-miR-500a-5p, hsa-miR-337-5p | 81.67 | 86.74 | 85.83 |
| 2209 | hsa-miR-374a-3p, hsa-miR-3613-3p, hsa-miR-103a-2-5p, hsa-miR-548l, age, hsa-miR-192-5p, hsa-miR-122-5p, hsa-miR-590-5p, hsa-miR-590-3p, hsa-miR-500a-5p, hsa-miR-374a-5p | 81.67 | 83.96 | 85.56 |
| 2210 | hsa-miR-4732-5p, hsa-miR-374a-3p, hsa-miR-103a-2-5p, hsa-miR-548l, hsa-miR-1277-3p, hsa-miR-181b-5p, hsa-miR-433-3p, hsa-miR-12136, hsa-miR-628-3p, hsa-miR-122-5p, hsa-miR-590-3p, hsa-miR-374a-5p, hsa-miR-99a-3p, hsa-miR-337-5p | 81.67 | 88.45 | 81.11 |
| 2211 | hsa-miR-4732-5p, hsa-miR-374a-3p, hsa-miR-103a-2-5p, hsa-miR-29c-5p, hsa-miR-433-3p, hsa-miR-12136, hsa-miR-19a-3p, hsa-miR-122-5p, hsa-miR-196b-5p, hsa-miR-215-5p, hsa-miR-99a-3p, hsa-miR-130b-5p, hsa-miR-337-5p | 81.67 | 88.33 | 80.83 |
| 2212 | hsa-miR-374a-3p, hsa-miR-548l, hsa-miR-1277-3p, hsa-miR-29c-5p, age, hsa-miR-181b-5p, hsa-miR-433-3p, hsa-miR-628-3p, hsa-miR-122-5p, hsa-miR-196b-5p, hsa-miR-491-5p, hsa-miR-153-3p, hsa-miR-590-5p, hsa-miR-500a-5p, hsa-miR-374a-5p, hsa-miR-130b-5p | 81.67 | 83.56 | 79.17 |
| 2213 | hsa-miR-374a-3p, hsa-miR-3613-3p, hsa-miR-548l, hsa-miR-29c-5p, hsa-miR-181b-5p, hsa-miR-192-5p, hsa-miR-433-3p, hsa-miR-598-3p, hsa-miR-19a-3p, hsa-miR-122-5p, hsa-miR-196b-5p, hsa-miR-491-5p, hsa-miR-153-3p, hsa-miR-590-5p, hsa-miR-500a-5p, hsa-miR-374a-5p, hsa-miR-130b-5p | 81.67 | 84.24 | 83.61 |
| 2214 | hsa-miR-374a-3p, hsa-miR-3613-3p, hsa-miR-103a-2-5p, hsa-miR-548l, age, hsa-miR-192-5p, hsa-miR-12136, hsa-miR-122-5p, hsa-miR-590-3p, hsa-miR-500a-5p, hsa-miR-374a-5p | 81.67 | 88.1 | 85.56 |
| 2215 | hsa-miR-374a-3p, hsa-miR-3613-3p, hsa-miR-103a-2-5p, age, hsa-miR-192-5p, hsa-miR-122-5p, hsa-miR-590-3p, hsa-miR-500a-5p, hsa-miR-374a-5p | 81.67 | 82.01 | 90.56 |
| 2216 | hsa-miR-4732-5p, hsa-miR-3613-3p, hsa-miR-29c-5p, age, hsa-miR-12136, hsa-miR-598-3p, hsa-miR-628-3p, hsa-miR-19a-3p, hsa-miR-153-3p, hsa-miR-590-3p, hsa-miR-99a-3p, hsa-miR-130b-5p | 81.67 | 87.13 | 86.67 |
| 2217 | hsa-miR-3613-3p, hsa-miR-548l, hsa-miR-29c-5p, age, hsa-miR-192-5p, hsa-miR-598-3p, hsa-miR-122-5p, hsa-miR-196b-5p, hsa-miR-215-5p, hsa-miR-153-3p, hsa-miR-590-3p, hsa-miR-374a-5p, hsa-miR-99a-3p, hsa-miR-130b-5p | 81.67 | 91.11 | 86.67 |
| 2218 | hsa-miR-4732-5p, hsa-miR-3613-3p, hsa-miR-103a-2-5p, hsa-miR-548l, hsa-miR-29c-5p, hsa-miR-628-3p, hsa-miR-122-5p, hsa-miR-153-3p, hsa-miR-590-5p, hsa-miR-590-3p, hsa-miR-99a-3p | 81.67 | 90.09 | 88.61 |
| 2219 | hsa-miR-374a-3p, hsa-miR-103a-2-5p, hsa-miR-548l, hsa-miR-29c-5p, hsa-miR-192-5p, hsa-miR-433-3p, hsa-miR-628-3p, hsa-miR-122-5p, hsa-miR-196b-5p, hsa-miR-215-5p, hsa-miR-590-5p, hsa-miR-590-3p, hsa-miR-500a-5p, hsa-miR-374a-5p, hsa-miR-99a-3p, hsa-miR-130b-5p, hsa-miR-337-5p | 81.67 | 90.44 | 79.17 |
| 2220 | hsa-miR-4732-5p, hsa-miR-103a-2-5p, hsa-miR-192-5p, hsa-miR-122-5p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 81.67 | 88.96 | 83.33 |
| 2221 | hsa-miR-4732-5p, hsa-miR-3613-3p, hsa-miR-103a-2-5p, age, hsa-miR-181b-5p, hsa-miR-192-5p, hsa-miR-122-5p, hsa-miR-590-5p, hsa-miR-590-3p, hsa-miR-500a-5p, hsa-miR-374a-5p | 81.67 | 88.73 | 88.06 |
| 2222 | hsa-miR-4732-5p, hsa-miR-3613-3p, hsa-miR-548l, hsa-miR-1277-3p, age, hsa-miR-192-5p, hsa-miR-12136, hsa-miR-598-3p, hsa-miR-628-3p, hsa-miR-19a-3p, hsa-miR-500a-5p | 80 | 86 | 83.61 |
| 2223 | hsa-miR-3613-3p, hsa-miR-103a-2-5p, hsa-miR-548l, age, hsa-miR-192-5p, hsa-miR-12136, hsa-miR-122-5p, hsa-miR-590-3p, hsa-miR-500a-5p, hsa-miR-374a-5p | 80 | 88.61 | 88.06 |
| 2224 | hsa-miR-4732-5p, hsa-miR-1277-3p, hsa-miR-628-3p, hsa-miR-196b-5p, hsa-miR-215-5p, hsa-miR-153-3p, hsa-miR-590-5p, hsa-miR-500a-5p, hsa-miR-374a-5p, hsa-miR-337-5p | 80 | 83.61 | 79.17 |
| 2225 | hsa-miR-374a-3p, hsa-miR-3613-3p, hsa-miR-103a-2-5p, hsa-miR-548l, hsa-miR-29c-5p, age, hsa-miR-628-3p, hsa-miR-122-5p, hsa-miR-590-3p, hsa-miR-500a-5p, hsa-miR-374a-5p, hsa-miR-99a-3p, hsa-miR-130b-5p | 80 | 90.95 | 81.39 |
| 2226 | hsa-miR-4732-5p, hsa-miR-3613-3p, hsa-miR-548l, hsa-miR-1277-3p, age, hsa-miR-192-5p, hsa-miR-12136, hsa-miR-598-3p, hsa-miR-628-3p, hsa-miR-19a-3p, hsa-miR-491-5p, hsa-miR-500a-5p | 80 | 86 | 83.33 |
| 2227 | hsa-miR-374a-3p, hsa-miR-3613-3p, hsa-miR-103a-2-5p, hsa-miR-29c-5p, age, hsa-miR-192-5p, hsa-miR-12136, hsa-miR-590-3p, hsa-miR-500a-5p, hsa-miR-374a-5p, hsa-miR-99a-3p, hsa-miR-130b-5p | 80 | 89.98 | 81.39 |
| 2228 | hsa-miR-4732-5p, hsa-miR-103a-2-5p, hsa-miR-122-5p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 80 | 86.34 | 87.78 |
| 2229 | hsa-miR-4732-5p, hsa-miR-3613-3p, hsa-miR-103a-2-5p, hsa-miR-548l, hsa-miR-1277-3p, hsa-miR-29c-5p, age, hsa-miR-181b-5p, hsa-miR-12136, hsa-miR-598-3p, hsa-miR-628-3p, hsa-miR-19a-3p, hsa-miR-491-5p, hsa-miR-99a-3p, hsa-miR-130b-5p | 80 | 86.27 | 74.72 |
| 2230 | hsa-miR-4732-5p, hsa-miR-103a-2-5p, hsa-miR-433-3p, hsa-miR-122-5p, hsa-miR-215-5p, hsa-miR-590-5p, hsa-miR-590-3p, hsa-miR-500a-5p, hsa-miR-374a-5p, hsa-miR-337-5p | 80 | 90.32 | 88.33 |
| 2231 | hsa-miR-374a-3p, hsa-miR-3613-3p, hsa-miR-103a-2-5p, age, hsa-miR-181b-5p, hsa-miR-192-5p, hsa-miR-122-5p, hsa-miR-491-5p, hsa-miR-590-5p, hsa-miR-590-3p, hsa-miR-374a-5p | 80 | 76.39 | 85.83 |
| 2232 | hsa-miR-4732-5p, hsa-miR-1277-3p, hsa-miR-433-3p, hsa-miR-628-3p, hsa-miR-122-5p, hsa-miR-196b-5p, hsa-miR-215-5p, hsa-miR-491-5p, hsa-miR-153-3p, hsa-miR-590-5p, hsa-miR-500a-5p, hsa-miR-374a-5p, hsa-miR-337-5p | 80 | 83.15 | 83.89 |
| 2233 | hsa-miR-4732-5p, hsa-miR-3613-3p, hsa-miR-103a-2-5p, hsa-miR-1277-3p, age, hsa-miR-181b-5p, hsa-miR-433-3p, hsa-miR-19a-3p, hsa-miR-491-5p, hsa-miR-590-5p, hsa-miR-374a-5p, hsa-miR-337-5p | 80 | 84.86 | 78.89 |
| 2234 | hsa-miR-4732-5p, hsa-miR-103a-2-5p, hsa-miR-433-3p, hsa-miR-122-5p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 80 | 89.58 | 88.33 |
| 2235 | hsa-miR-374a-3p, hsa-miR-103a-2-5p, hsa-miR-548l, hsa-miR-1277-3p, hsa-miR-29c-5p, age, hsa-miR-181b-5p, hsa-miR-192-5p, hsa-miR-12136, hsa-miR-628-3p, hsa-miR-19a-3p, hsa-miR-122-5p, hsa-miR-196b-5p, hsa-miR-215-5p, hsa-miR-491-5p, hsa-miR-153-3p, hsa-miR-590-3p, hsa-miR-500a-5p, hsa-miR-374a-5p, hsa-miR-130b-5p, hsa-miR-337-5p | 80 | 87.31 | 78.89 |
| 2236 | hsa-miR-4732-5p, hsa-miR-122-5p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 80 | 89.58 | 88.06 |
| 2237 | hsa-miR-4732-5p, hsa-miR-103a-2-5p, hsa-miR-122-5p, hsa-miR-196b-5p, hsa-miR-215-5p, hsa-miR-590-5p, hsa-miR-590-3p, hsa-miR-500a-5p, hsa-miR-337-5p | 80 | 85.72 | 85.83 |
| 2238 | hsa-miR-4732-5p, hsa-miR-3613-3p, age, hsa-miR-192-5p, hsa-miR-12136, hsa-miR-598-3p, hsa-miR-628-3p, hsa-miR-19a-3p, hsa-miR-153-3p, hsa-miR-590-3p | 78.33 | 86.39 | 83.89 |
| 2239 | hsa-miR-374a-3p, hsa-miR-3613-3p, hsa-miR-103a-2-5p, hsa-miR-548l, hsa-miR-1277-3p, hsa-miR-29c-5p, age, hsa-miR-181b-5p, hsa-miR-12136, hsa-miR-598-3p, hsa-miR-19a-3p, hsa-miR-122-5p, hsa-miR-491-5p, hsa-miR-99a-3p, hsa-miR-130b-5p | 78.33 | 84.86 | 79.44 |
| 2240 | hsa-miR-374a-3p, hsa-miR-548l, age, hsa-miR-181b-5p, hsa-miR-192-5p, hsa-miR-433-3p, hsa-miR-598-3p, hsa-miR-122-5p, hsa-miR-215-5p, hsa-miR-491-5p, hsa-miR-590-5p, hsa-miR-374a-5p, hsa-miR-337-5p | 78.33 | 82.31 | 81.11 |
| 2241 | hsa-miR-374a-3p, hsa-miR-3613-3p, hsa-miR-103a-2-5p, hsa-miR-29c-5p, age, hsa-miR-433-3p, hsa-miR-12136, hsa-miR-598-3p, hsa-miR-19a-3p, hsa-miR-122-5p, hsa-miR-590-3p, hsa-miR-500a-5p, hsa-miR-99a-3p, hsa-miR-130b-5p | 78.33 | 89.58 | 79.17 |
| 2242 | hsa-miR-4732-5p, hsa-miR-433-3p, hsa-miR-122-5p, hsa-miR-196b-5p, hsa-miR-215-5p, hsa-miR-153-3p, hsa-miR-590-5p, hsa-miR-590-3p, hsa-miR-500a-5p, hsa-miR-374a-5p, hsa-miR-337-5p | 78.33 | 88.73 | 86.11 |
| 2243 | hsa-miR-4732-5p, age, hsa-miR-122-5p, hsa-miR-196b-5p, hsa-miR-215-5p, hsa-miR-590-5p, hsa-miR-590-3p, hsa-miR-500a-5p, hsa-miR-374a-5p, hsa-miR-337-5p | 78.33 | 87.94 | 80.83 |
| 2244 | hsa-miR-374a-3p, hsa-miR-548l, hsa-miR-1277-3p, hsa-miR-192-5p, hsa-miR-433-3p, hsa-miR-598-3p, hsa-miR-19a-3p, hsa-miR-196b-5p, hsa-miR-215-5p, hsa-miR-590-5p, hsa-miR-500a-5p, hsa-miR-374a-5p, hsa-miR-99a-3p, hsa-miR-130b-5p, hsa-miR-337-5p | 78.33 | 87.2 | 76.67 |
| 2245 | hsa-miR-374a-3p, hsa-miR-1277-3p, hsa-miR-29c-5p, hsa-miR-181b-5p, hsa-miR-192-5p, hsa-miR-12136, hsa-miR-628-3p, hsa-miR-19a-3p, hsa-miR-122-5p, hsa-miR-215-5p, hsa-miR-491-5p, hsa-miR-153-3p, hsa-miR-590-5p, hsa-miR-130b-5p | 78.33 | 79.58 | 81.39 |
| 2246 | hsa-miR-4732-5p, hsa-miR-628-3p, hsa-miR-122-5p, hsa-miR-196b-5p, hsa-miR-215-5p, hsa-miR-153-3p, hsa-miR-590-5p, hsa-miR-590-3p, hsa-miR-500a-5p, hsa-miR-374a-5p | 78.33 | 79.56 | 83.33 |
| 2247 | hsa-miR-374a-3p, hsa-miR-3613-3p, hsa-miR-29c-5p, age, hsa-miR-181b-5p, hsa-miR-192-5p, hsa-miR-12136, hsa-miR-598-3p, hsa-miR-19a-3p, hsa-miR-122-5p, hsa-miR-215-5p, hsa-miR-153-3p, hsa-miR-500a-5p, hsa-miR-374a-5p, hsa-miR-130b-5p | 78.33 | 82.25 | 83.06 |
| 2248 | hsa-miR-374a-3p, hsa-miR-1277-3p, hsa-miR-181b-5p, hsa-miR-628-3p, hsa-miR-196b-5p, hsa-miR-215-5p, hsa-miR-491-5p, hsa-miR-153-3p, hsa-miR-374a-5p, hsa-miR-99a-3p | 76.67 | 83.5 | 81.67 |
| 2249 | hsa-miR-4732-5p, hsa-miR-374a-3p, hsa-miR-29c-5p, hsa-miR-433-3p, hsa-miR-12136, hsa-miR-598-3p, hsa-miR-122-5p, hsa-miR-196b-5p, hsa-miR-491-5p, hsa-miR-153-3p, hsa-miR-500a-5p, hsa-miR-374a-5p, hsa-miR-99a-3p, hsa-miR-130b-5p, hsa-miR-337-5p | 76.67 | 81.27 | 76.39 |
| 2250 | hsa-miR-4732-5p, hsa-miR-3613-3p, age, hsa-miR-181b-5p, hsa-miR-192-5p, hsa-miR-12136, hsa-miR-598-3p, hsa-miR-19a-3p, hsa-miR-491-5p, hsa-miR-500a-5p, hsa-miR-374a-5p, hsa-miR-99a-3p, hsa-miR-130b-5p | 76.67 | 81.83 | 78.89 |
| 2251 | hsa-miR-4732-5p, hsa-miR-103a-2-5p, hsa-miR-548l, hsa-miR-1277-3p, hsa-miR-181b-5p, hsa-miR-192-5p, hsa-miR-433-3p, hsa-miR-12136, hsa-miR-598-3p, hsa-miR-19a-3p, hsa-miR-196b-5p, hsa-miR-215-5p, hsa-miR-491-5p, hsa-miR-590-5p, hsa-miR-500a-5p, hsa-miR-337-5p | 76.67 | 83.38 | 76.67 |
| 2252 | hsa-miR-4732-5p, hsa-miR-3613-3p, hsa-miR-103a-2-5p, hsa-miR-548l, hsa-miR-1277-3p, hsa-miR-29c-5p, age, hsa-miR-181b-5p, hsa-miR-12136, hsa-miR-598-3p, hsa-miR-628-3p, hsa-miR-19a-3p, hsa-miR-491-5p, hsa-miR-500a-5p, hsa-miR-99a-3p, hsa-miR-130b-5p | 76.67 | 79.33 | 79.17 |
| 2253 | hsa-miR-4732-5p, hsa-miR-628-3p, hsa-miR-196b-5p, hsa-miR-215-5p, hsa-miR-153-3p, hsa-miR-590-5p, hsa-miR-590-3p, hsa-miR-500a-5p, hsa-miR-374a-5p, hsa-miR-337-5p | 73.33 | 81.55 | 79.17 |
| 2254 | hsa-miR-4732-5p, hsa-miR-103a-2-5p, hsa-miR-1277-3p, age, hsa-miR-181b-5p, hsa-miR-12136, hsa-miR-598-3p, hsa-miR-19a-3p, hsa-miR-491-5p, hsa-miR-153-3p, hsa-miR-500a-5p, hsa-miR-99a-3p, hsa-miR-130b-5p | 73.33 | 82.29 | 76.94 |
| 2257 | combinazione 1980 + rs7204342 | 83.33 | 81.71 | 81.71 |
| 2258 | combinazione 1980 + rs7193058 | 73.33 | 77.57 | 77.57 |
| 2259 | combinazione 1982 + rs4297685 | 81.67 | 83.26 | 83.26 |
| 2260 | combinazione 1982 + rs4297685 | 80 | 84.75 | 84.75 |
| 2261 | combinazione 1983 + rs4297685 | 80 | 82.01 | 82.01 |
| 2262 | combinazione 1934 + rs28640218 | 83.33 | 80.02 | 80.02 |
| 2263 | combinazione 1985 + rs13335818 | 83.33 | 83.03 | 83.03 |
| 2264 | combinazione 1986 + rs13335818 | 76.67 | 78.26 | 78.26 |
| 2265 | combinazione 1987 + rs76563024 | 75 | 79.91 | 79.91 |
| 2266 | combinazione 1993 + rs11647727 | 85 | 87.64 | 87.64 |
| 2267 | combinazione 1994 + rs4297685 | 85 | 85.76 | 85.76 |
| 2268 | combinazione 1995 + rs13333226 | 88.33 | 91.57 | 91.57 |
| 2269 | combinazione 2006 + rs13333226 | 80 | 84.24 | 84.24 |

This is the list of the best 30 miRNA selected by the algorithm sorted by importance for tumour stage: hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p, hsa-miR-1296-5p, hsa-miR-1249-3p, hsa-miR-100-5p, hsa-miR-181c-5p, hsa-miR-323b-3p, hsa-miR-452-3p, hsa-miR-20b-5p, and hsa-miR-1301-3p. The inventors combined these miRNAs, together or not with age and UMOD SNP variant, creating unique combinations leading to a further significant increase in sensitivity, specificity, accuracy and predictive power for tumour stage (Table 26).

**Table 26. Combinations that allow higher sensitivity, specificity, accuracy and predictive power for tumour stage.**

| N | Combinations | Accuracy | ROC AUC | Recall |
|---|---|---|---|---|
| 2272 | hsa-miR-769-5p, hsa-miR-548a-3p, hsa-miR-598-3p | 83.89 | 90 | 85.83 |
| 2273 | hsa-miR-769-5p, hsa-miR-548a-3p, hsa-miR-598-3p, hsa-miR-190a-5p | 88.89 | 95.71 | 86.11 |
| 2274 | hsa-miR-769-5p, hsa-miR-548a-3p, hsa-miR-598-3p, hsa-miR-190a-5p, hsa-miR-215-5p | 86.39 | 91.43 | 93.33 |
| 2275 | hsa-miR-769-5p, hsa-miR-548a-3p, hsa-miR-598-3p, hsa-miR-190a-5p, hsa-miR-215-5p, hsa-miR-324-3p | 86.11 | 95.71 | 91.39 |
| 2276 | hsa-miR-769-5p, hsa-miR-548a-3p, hsa-miR-598-3p, hsa-miR-190a-5p, hsa-miR-215-5p, hsa-miR-324-3p, hsa-miR-143-3p | 81.67 | 95.71 | 96.39 |
| 2277 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421 | 83.89 | 94.29 | 96.11 |
| 2278 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p | 88.61 | 91.43 | 99.17 |
| 2279 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306 | 86.11 | 94.29 | 98.06 |
| 2280 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p | 88.33 | 95.71 | 100.0 |
| 2281 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p | 88.33 | 90 | 98.89 |
| 2282 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p | 90.83 | 95.71 | 97.78 |
| 2283 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p | 88.33 | 98.57 | 98.89 |
| 2284 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p | 86.11 | 98.57 | 100.0 |
| 2285 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p | 83.61 | 98.57 | 98.89 |
| 2286 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941 | 86.39 | 98.57 | 98.89 |
| 2287 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p | 85.83 | 97.14 | 98.89 |
| 2288 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p | 83.33 | 97.14 | 97.78 |
| 2289 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p | 81.11 | 97.14 | 98.89 |
| 2290 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p | 81.11 | 97.14 | 98.89 |
| 2291 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p | 83.33 | 97.14 | 100.0 |
| 2292 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p, hsa-miR-1296-5p | 86.11 | 94.29 | 100.0 |
| 2293 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p, hsa-miR-1296-5p, hsa-miR-1249-3p | 86.11 | 92.86 | 99.17 |
| 2294 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p, hsa-miR-1296-5p, hsa-miR-1249-3p, hsa-miR-100-5p | 86.11 | 92.86 | 99.17 |
| 2295 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p, hsa-miR-1296-5p, hsa-miR-1249-3p, hsa-miR-100-5p, hsa-miR-181c-5p | 86.11 | 90 | 99.17 |
| 2296 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p, hsa-miR-1296-5p, hsa-miR-1249-3p, hsa-miR-100-5p, hsa-miR-181c-5p, hsa-miR-323b-3p | 85.83 | 91.43 | 99.17 |
| 2297 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p, hsa-miR-1296-5p, hsa-miR-1249-3p, hsa-miR-100-5p, hsa-miR-181c-5p, hsa-miR-323b-3p, hsa-miR-452-3p | 85.83 | 94.29 | 97.22 |
| 2298 | hsa-miR-769-5p, hsa-miR-548a-3p, hsa-miR-598-3p, hsa-miR-190a-5p, hsa-miR-215-5p, hsa-miR-324-3p, hsa-miR-143-3p, hsa-miR-421, hsa-miR-22-5p, hsa-miR-4306, hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-503-5p, hsa-miR-30e-5p, hsa-let-7i-5p, hsa-miR-1296-5p, hsa-miR-1249-3p, hsa-miR-100-5p, hsa-miR-181c-5p, hsa-miR-323b-3p, hsa-miR-452-3p, hsa-miR-20b-5p | 88.33 | 91.43 | 97.22 |
| 2299 | hsa-miR-335-5p, hsa-miR-30b-5p, hsa-miR-664a-3p, hsa-miR-941, hsa-miR-503-5p, hsa-miR-30e-5p, hsa-miR-1296-5p, hsa-miR-323b-3p, hsa-miR-1301-3p, hsa-miR-215-5p, hsa-miR-324-3p, hsa-miR-143-3p | 100 | 100 | 100 |
| 2300 | hsa-miR-106b-3p, hsa-miR-335-5p, hsa-miR-30b-5p, hsa-miR-664a-3p, hsa-miR-30e-5p, hsa-let-7i-5p, hsa-miR-1296-5p, hsa-miR-100-5p, hsa-miR-323b-3p, hsa-miR-215-5p, hsa-miR-324-3p, hsa-miR-143-3p, hsa-miR-421 | 100 | 100 | 100 |
| 2301 | hsa-miR-106b-3p, hsa-miR-335-5p, hsa-miR-30b-5p, hsa-miR-664a-3p, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p, hsa-miR-421 | 100 | 100 | 100 |
| 2302 | hsa-miR-132-3p, hsa-miR-30b-5p, hsa-miR-503-5p, hsa-miR-30e-5p, hsa-miR-1296-5p, hsa-miR-323b-3p, hsa-miR-452-3p, hsa-miR-215-5p, hsa-miR-324-3p, hsa-miR-421, hsa-miR-22-5p | 100 | 100 | 100 |
| 2303 | hsa-miR-106b-3p, hsa-miR-335-5p, hsa-miR-30b-5p, hsa-miR-664a-3p, hsa-let-7i-5p, hsa-miR-1296-5p, hsa-miR-100-5p, hsa-miR-181c-5p, hsa-miR-323b-3p, hsa-miR-1301-3p, hsa-miR-215-5p, hsa-miR-143-3p, hsa-miR-421 | 100 | 100 | 100 |
| 2304 | hsa-miR-106b-3p, hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p, hsa-miR-421 | 100 | 100 | 100 |
| 2305 | hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-323b-3p, hsa-miR-1301-3p, hsa-miR-215-5p, hsa-miR-324-3p, hsa-miR-143-3p, hsa-miR-421 | 100 | 100 | 100 |
| 2306 | hsa-miR-106b-3p, hsa-miR-335-5p, hsa-miR-664a-3p, hsa-miR-30e-5p, hsa-let-7i-5p, hsa-miR-1296-5p, hsa-miR-100-5p, hsa-miR-181c-5p, hsa-miR-1301-3p, hsa-miR-215-5p, hsa-miR-143-3p, hsa-miR-421 | 100 | 100 | 100 |
| 2307 | hsa-miR-106b-3p, hsa-miR-335-5p, hsa-miR-30b-5p, hsa-miR-664a-3p, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-324-3p, hsa-miR-421 | 100 | 100 | 100 |
| 2308 | hsa-miR-106b-3p, hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-323b-3p, hsa-miR-1301-3p, hsa-miR-215-5p, hsa-miR-324-3p, hsa-miR-143-3p, hsa-miR-421 | 100 | 100 | 100 |
| 2309 | hsa-miR-106b-3p, hsa-miR-335-5p, hsa-miR-664a-3p, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p, hsa-miR-421 | 97.78 | 100 | 100 |
| 2310 | hsa-miR-106b-3p, hsa-miR-335-5p, hsa-miR-30b-5p, hsa-miR-30e-5p, hsa-let-7i-5p, hsa-miR-1296-5p, hsa-miR-100-5p, hsa-miR-323b-3p, hsa-miR-452-3p, hsa-miR-1301-3p, hsa-miR-215-5p, hsa-miR-324-3p, hsa-miR-143-3p, hsa-miR-421 | 97.78 | 100 | 100 |
| 2311 | hsa-miR-106b-3p, hsa-miR-335-5p, hsa-miR-30b-5p, hsa-miR-664a-3p, hsa-miR-941, hsa-miR-32-3p, hsa-miR-140-3p, hsa-miR-503-5p, hsa-miR-30e-5p, hsa-miR-1296-5p, hsa-miR-100-5p, hsa-miR-323b-3p, hsa-miR-1301-3p, hsa-miR-215-5p, hsa-miR-324-3p | 97.78 | 100 | 100 |
| 2312 | hsa-miR-335-5p, hsa-miR-30b-5p, hsa-miR-664a-3p, hsa-miR-503-5p, hsa-miR-30e-5p, hsa-let-7i-5p, hsa-miR-1296-5p, hsa-miR-323b-3p, hsa-miR-20b-5p, hsa-miR-1301-3p, hsa-miR-324-3p, hsa-miR-421 | 97.78 | 100 | 100 |
| 2313 | hsa-miR-106b-3p, hsa-miR-335-5p, hsa-miR-30b-5p, hsa-miR-664a-3p, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-143-3p, hsa-miR-421 | 97.78 | 100 | 100 |
| 2314 | hsa-miR-132-3p, hsa-miR-30b-5p, hsa-miR-31-5p, hsa-miR-32-3p, hsa-miR-140-3p, hsa-miR-30e-5p, hsa-let-7i-5p, hsa-miR-1296-5p, hsa-miR-323b-3p, hsa-miR-452-3p, hsa-miR-190a-5p, hsa-miR-143-3p, hsa-miR-421, hsa-miR-22-5p, hsa-miR-4306 | 97.78 | 100 | 100 |
| 2315 | hsa-miR-106b-3p, hsa-miR-335-5p, hsa-miR-30b-5p, hsa-miR-941, hsa-miR-32-3p, hsa-miR-503-5p, hsa-miR-30e-5p, hsa-miR-1296-5p, hsa-miR-100-5p, hsa-miR-323b-3p, hsa-miR-1301-3p, hsa-miR-215-5p, hsa-miR-324-3p | 97.78 | 100 | 100 |
| 2316 | hsa-miR-335-5p, hsa-miR-30b-5p, hsa-miR-941, hsa-miR-30e-5p, hsa-let-7i-5p, hsa-miR-1296-5p, hsa-miR-323b-3p, hsa-miR-452-3p, hsa-miR-1301-3p, hsa-miR-215-5p, hsa-miR-324-3p, hsa-miR-143-3p, hsa-miR-421 | 97.78 | 100 | 100 |
| 2317 | hsa-miR-335-5p, hsa-miR-30b-5p, hsa-miR-664a-3p, hsa-miR-503-5p, hsa-let-7i-5p, hsa-miR-1296-5p, hsa-miR-181c-5p, hsa-miR-323b-3p, hsa-miR-20b-5p, hsa-miR-1301-3p, hsa-miR-324-3p, hsa-miR-421 | 97.78 | 100 | 90 |
| 2318 | hsa-miR-106b-3p, hsa-miR-335-5p, hsa-miR-30b-5p, hsa-miR-664a-3p, hsa-miR-941, hsa-miR-32-3p, hsa-miR-503-5p, hsa-miR-30e-5p, hsa-miR-1296-5p, hsa-miR-100-5p, hsa-miR-323b-3p, hsa-miR-1301-3p, hsa-miR-215-5p, hsa-miR-324-3p | 97.78 | 100 | 100 |
| 2319 | hsa-miR-106b-3p, hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-143-3p, hsa-miR-421 | 97.78 | 100 | 100 |
| 2320 | hsa-miR-106b-3p, hsa-miR-335-5p, hsa-miR-30b-5p, hsa-miR-664a-3p, hsa-miR-30e-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p, hsa-miR-421 | 97.78 | 100 | 90 |
| 2321 | hsa-miR-335-5p, hsa-miR-30b-5p, hsa-miR-664a-3p, hsa-miR-31-5p, hsa-miR-30e-5p, hsa-let-7i-5p, hsa-miR-1296-5p, hsa-miR-323b-3p, hsa-miR-1301-3p, hsa-miR-324-3p, hsa-miR-143-3p, hsa-miR-421 | 97.78 | 100 | 100 |
| 2322 | hsa-miR-106b-3p, hsa-miR-335-5p, hsa-miR-30b-5p, hsa-miR-664a-3p, hsa-let-7i-5p, hsa-miR-1296-5p, hsa-miR-100-5p, hsa-miR-181c-5p, hsa-miR-323b-3p, hsa-miR-452-3p, hsa-miR-215-5p, hsa-miR-143-3p, hsa-miR-421 | 97.78 | 100 | 90 |
| 2323 | hsa-miR-335-5p, hsa-miR-30b-5p, hsa-miR-941, hsa-miR-30e-5p, hsa-let-7i-5p, hsa-miR-1296-5p, hsa-miR-323b-3p, hsa-miR-452-3p, hsa-miR-215-5p, hsa-miR-324-3p, hsa-miR-143-3p, hsa-miR-421 | 97.78 | 100 | 100 |
| 2324 | hsa-miR-335-5p, hsa-miR-30b-5p, hsa-miR-503-5p, hsa-miR-30e-5p, hsa-let-7i-5p, hsa-miR-1296-5p, hsa-miR-323b-3p, hsa-miR-452-3p, hsa-miR-20b-5p | 97.78 | 97.14 | 100 |
| 2325 | hsa-miR-106b-3p, hsa-miR-335-5p, hsa-miR-30b-5p, hsa-miR-664a-3p, hsa-miR-30e-5p, hsa-let-7i-5p, hsa-miR-1296-5p, hsa-miR-1249-3p, hsa-miR-100-5p, hsa-miR-323b-3p, hsa-miR-1301-3p, hsa-miR-215-5p, hsa-miR-324-3p, hsa-miR-143-3p, hsa-miR-421 | 97.78 | 100 | 90 |
| 2326 | hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-100-5p, hsa-miR-323b-3p, hsa-miR-452-3p, hsa-miR-215-5p, hsa-miR-324-3p, hsa-miR-143-3p, hsa-miR-421 | 97.5 | 100 | 100 |
| 2327 | hsa-miR-106b-3p, hsa-miR-335-5p, hsa-miR-30b-5p, hsa-miR-664a-3p, hsa-miR-941, hsa-miR-503-5p, hsa-miR-30e-5p, hsa-miR-1296-5p, hsa-miR-100-5p, hsa-miR-323b-3p, hsa-miR-1301-3p, hsa-miR-215-5p, hsa-miR-324-3p, hsa-miR-421 | 97.5 | 100 | 100 |
| 2328 | hsa-miR-335-5p, hsa-miR-30b-5p, hsa-miR-664a-3p, hsa-miR-30e-5p, hsa-let-7i-5p, hsa-miR-1296-5p, hsa-miR-323b-3p, hsa-miR-1301-3p, hsa-miR-215-5p, hsa-miR-324-3p, hsa-miR-143-3p, hsa-miR-421 | 97.5 | 100 | 80 |
| 2329 | hsa-miR-106b-3p, hsa-miR-335-5p, hsa-miR-30b-5p, hsa-miR-664a-3p, hsa-miR-31-5p, hsa-miR-140-3p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-324-3p, hsa-miR-421 | 97.5 | 100 | 100 |
| 2330 | hsa-miR-132-3p, hsa-miR-30b-5p, hsa-miR-31-5p, hsa-miR-32-3p, hsa-miR-140-3p, hsa-miR-503-5p, hsa-miR-30e-5p, hsa-miR-1296-5p, hsa-miR-323b-3p, hsa-miR-452-3p, hsa-miR-20b-5p, hsa-miR-190a-5p, hsa-miR-22-5p, hsa-miR-4306 | 97.5 | 97.14 | 100 |
| 2331 | hsa-miR-132-3p, hsa-miR-30b-5p, hsa-miR-664a-3p, hsa-miR-941, hsa-miR-30e-5p, hsa-let-7i-5p, hsa-miR-1296-5p, hsa-miR-323b-3p, hsa-miR-1301-3p, hsa-miR-215-5p, hsa-miR-324-3p, hsa-miR-143-3p, hsa-miR-421, hsa-miR-22-5p | 97.5 | 100 | 80 |
| 2332 | hsa-miR-132-3p, hsa-miR-335-5p, hsa-miR-30b-5p, hsa-miR-664a-3p, hsa-let-7i-5p, hsa-miR-1249-3p, hsa-miR-100-5p, hsa-miR-181c-5p, hsa-miR-323b-3p, hsa-miR-1301-3p, hsa-miR-324-3p, hsa-miR-143-3p, hsa-miR-421 | 97.5 | 100 | 80 |
| 2333 | hsa-miR-106b-3p, hsa-miR-335-5p, hsa-miR-30b-5p, hsa-miR-664a-3p, hsa-miR-31-5p, hsa-miR-32-3p, hsa-miR-140-3p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-190a-5p, hsa-miR-324-3p, hsa-miR-421 | 97.5 | 97.14 | 100 |
| 2334 | hsa-miR-106b-3p, hsa-miR-335-5p, hsa-miR-30b-5p, hsa-let-7i-5p, hsa-miR-1296-5p, hsa-miR-100-5p, hsa-miR-181c-5p, hsa-miR-323b-3p, hsa-miR-452-3p, hsa-miR-1301-3p, hsa-miR-215-5p, hsa-miR-143-3p, hsa-miR-421 | 97.5 | 100 | 100 |
| 2335 | hsa-miR-106b-3p, hsa-miR-335-5p, hsa-miR-30b-5p, hsa-miR-664a-3p, hsa-miR-31-5p, hsa-miR-32-3p, hsa-miR-140-3p, hsa-miR-30e-5p, hsa-let-7i-5p, hsa-miR-1296-5p, hsa-miR-100-5p, hsa-miR-323b-3p, hsa-miR-20b-5p, hsa-miR-324-3p, hsa-miR-421 | 97.5 | 97.14 | 100 |
| 2336 | hsa-miR-106b-3p, hsa-miR-335-5p, hsa-miR-30b-5p, hsa-let-7i-5p, hsa-miR-1296-5p, hsa-miR-100-5p, hsa-miR-181c-5p, hsa-miR-323b-3p, hsa-miR-1301-3p, hsa-miR-215-5p, hsa-miR-143-3p, hsa-miR-421 | 97.5 | 100 | 100 |
| 2337 | hsa-miR-106b-3p, hsa-miR-335-5p, hsa-miR-664a-3p, hsa-miR-941, hsa-miR-503-5p, hsa-miR-30e-5p, hsa-let-7i-5p, hsa-miR-1296-5p, hsa-miR-100-5p, hsa-miR-323b-3p, hsa-miR-1301-3p, hsa-miR-324-3p, hsa-miR-421 | 97.5 | 100 | 100 |
| 2338 | hsa-miR-132-3p, hsa-miR-106b-3p, hsa-miR-30b-5p, hsa-miR-31-5p, hsa-miR-32-3p, hsa-miR-140-3p, hsa-miR-30e-5p, hsa-let-7i-5p, hsa-miR-1296-5p, hsa-miR-323b-3p, hsa-miR-452-3p, hsa-miR-190a-5p, hsa-miR-143-3p, hsa-miR-421 | 97.5 | 100 | 100 |
| 2339 | hsa-miR-106b-3p, hsa-miR-335-5p, hsa-miR-30b-5p, hsa-miR-941, hsa-miR-30e-5p, hsa-let-7i-5p, hsa-miR-1296-5p, hsa-miR-100-5p, hsa-miR-323b-3p, hsa-miR-452-3p, hsa-miR-215-5p, hsa-miR-324-3p, hsa-miR-143-3p, hsa-miR-421 | 95.56 | 100 | 100 |
| 2340 | hsa-miR-106b-3p, hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-100-5p, hsa-miR-323b-3p, hsa-miR-1301-3p, hsa-miR-190a-5p, hsa-miR-215-5p, hsa-miR-324-3p, hsa-miR-143-3p, hsa-miR-421 | 95.56 | 100 | 100 |
| 2341 | hsa-miR-106b-3p, hsa-miR-335-5p, hsa-miR-30b-5p, hsa-miR-941, hsa-miR-30e-5p, hsa-let-7i-5p, hsa-miR-1296-5p, hsa-miR-100-5p, hsa-miR-323b-3p, hsa-miR-452-3p, hsa-miR-1301-3p, hsa-miR-215-5p, hsa-miR-324-3p, hsa-miR-143-3p, hsa-miR-421 | 95.56 | 100 | 100 |
| 2342 | hsa-miR-132-3p, hsa-miR-30b-5p, hsa-miR-503-5p, hsa-miR-30e-5p, hsa-let-7i-5p, hsa-miR-1296-5p, hsa-miR-100-5p, hsa-miR-323b-3p, hsa-miR-452-3p, hsa-miR-20b-5p, hsa-miR-1301-3p, hsa-miR-215-5p, hsa-miR-22-5p, hsa-miR-4306 | 95.56 | 95.71 | 100 |
| 2343 | hsa-miR-106b-3p, hsa-miR-335-5p, hsa-miR-30b-5p, hsa-miR-30e-5p, hsa-let-7i-5p, hsa-miR-1296-5p, hsa-miR-100-5p, hsa-miR-323b-3p, hsa-miR-452-3p, hsa-miR-1301-3p, hsa-miR-215-5p, hsa-miR-143-3p, hsa-miR-421 | 95.56 | 100 | 90 |
| 2344 | hsa-miR-335-5p, hsa-miR-30b-5p, hsa-miR-664a-3p, hsa-miR-941, hsa-miR-30e-5p, hsa-let-7i-5p, hsa-miR-323b-3p, hsa-miR-1301-3p, hsa-miR-215-5p, hsa-miR-324-3p, hsa-miR-143-3p, hsa-miR-421 | 95.56 | 98.57 | 100 |
| 2345 | hsa-miR-106b-3p, hsa-miR-335-5p, hsa-miR-30b-5p, hsa-miR-664a-3p, hsa-miR-32-3p, hsa-miR-503-5p, hsa-miR-30e-5p, hsa-miR-1296-5p, hsa-miR-100-5p, hsa-miR-323b-3p, hsa-miR-215-5p, hsa-miR-324-3p | 95.56 | 100 | 100 |
| 2346 | hsa-miR-132-3p, hsa-miR-335-5p, hsa-miR-30b-5p, hsa-miR-30e-5p, hsa-let-7i-5p, hsa-miR-1296-5p, hsa-miR-100-5p, hsa-miR-323b-3p, hsa-miR-452-3p, hsa-miR-324-3p, hsa-miR-421, hsa-miR-22-5p | 95.56 | 95.71 | 100 |
| 2347 | hsa-miR-132-3p, hsa-miR-30b-5p, hsa-miR-664a-3p, hsa-miR-30e-5p, hsa-let-7i-5p, hsa-miR-1296-5p, hsa-miR-452-3p, hsa-miR-215-5p, hsa-miR-324-3p, hsa-miR-143-3p, hsa-miR-421, hsa-miR-22-5p, hsa-miR-4306 | 95.56 | 100 | 100 |
| 2348 | hsa-miR-132-3p, hsa-miR-30b-5p, hsa-miR-664a-3p, hsa-miR-941, hsa-miR-30e-5p, hsa-let-7i-5p, hsa-miR-1296-5p, hsa-miR-1249-3p, hsa-miR-1301-3p, hsa-miR-215-5p, hsa-miR-324-3p, hsa-miR-143-3p, hsa-miR-421, hsa-miR-22-5p, hsa-miR-4306 | 95.56 | 100 | 100 |
| 2349 | hsa-miR-106b-3p, hsa-miR-335-5p, hsa-miR-664a-3p, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p, hsa-miR-421, hsa-miR-4306 | 95.56 | 100 | 100 |
| 2350 | hsa-miR-335-5p, hsa-miR-30b-5p, hsa-miR-664a-3p, hsa-miR-32-3p, hsa-miR-503-5p, hsa-miR-30e-5p, hsa-miR-1296-5p, hsa-miR-323b-3p, hsa-miR-1301-3p, hsa-miR-215-5p, hsa-miR-324-3p | 95.56 | 100 | 100 |
| 2351 | hsa-miR-132-3p, hsa-miR-106b-3p, hsa-miR-335-5p, hsa-miR-30b-5p, hsa-miR-664a-3p, hsa-miR-140-3p, hsa-miR-30e-5p, hsa-let-7i-5p, hsa-miR-1296-5p, hsa-miR-1249-3p, hsa-miR-100-5p, hsa-miR-181c-5p, hsa-miR-1301-3p, hsa-miR-215-5p, hsa-miR-143-3p, hsa-miR-421, hsa-miR-22-5p | 95.56 | 97.14 | 100 |
| 2352 | hsa-miR-132-3p, hsa-miR-30b-5p, hsa-miR-31-5p, hsa-miR-503-5p, hsa-miR-30e-5p, hsa-let-7i-5p, hsa-miR-1296-5p, hsa-miR-323b-3p, hsa-miR-452-3p, hsa-miR-20b-5p, hsa-miR-190a-5p, hsa-miR-143-3p, hsa-miR-421, hsa-miR-22-5p, hsa-miR-4306 | 95.56 | 98.57 | 90 |
| 2353 | hsa-miR-30b-5p, hsa-miR-31-5p, hsa-miR-941, hsa-miR-140-3p, hsa-miR-30e-5p, hsa-miR-1296-5p, hsa-miR-323b-3p, hsa-miR-452-3p, hsa-miR-190a-5p, hsa-miR-421, hsa-miR-22-5p, hsa-miR-4306 | 95.56 | 100 | 80 |
| 2354 | hsa-miR-132-3p, hsa-miR-30b-5p, hsa-miR-664a-3p, hsa-miR-31-5p, hsa-miR-941, hsa-miR-503-5p, hsa-miR-30e-5p, hsa-let-7i-5p, hsa-miR-1296-5p, hsa-miR-100-5p, hsa-miR-323b-3p, hsa-miR-452-3p, hsa-miR-190a-5p, hsa-miR-215-5p, hsa-miR-324-3p, hsa-miR-143-3p | 95.28 | 100 | 100 |
| 2355 | hsa-miR-335-5p, hsa-miR-30b-5p, hsa-miR-664a-3p, hsa-miR-941, hsa-let-7i-5p, hsa-miR-1296-5p, hsa-miR-181c-5p, hsa-miR-323b-3p, hsa-miR-1301-3p, hsa-miR-215-5p, hsa-miR-143-3p, hsa-miR-421 | 95.28 | 100 | 70 |
| 2356 | hsa-miR-106b-3p, hsa-miR-335-5p, hsa-miR-664a-3p, hsa-let-7i-5p, hsa-miR-100-5p, hsa-miR-181c-5p, hsa-miR-323b-3p, hsa-miR-1301-3p, hsa-miR-215-5p, hsa-miR-143-3p, hsa-miR-421 | 95.28 | 100 | 100 |
| 2357 | hsa-miR-106b-3p, hsa-miR-335-5p, hsa-miR-30b-5p, hsa-miR-664a-3p, hsa-miR-503-5p, hsa-miR-30e-5p, hsa-miR-1296-5p, hsa-miR-100-5p, hsa-miR-323b-3p, hsa-miR-1301-3p, hsa-miR-215-5p, hsa-miR-324-3p | 95.28 | 100 | 80 |
| 2358 | hsa-miR-106b-3p, hsa-miR-335-5p, hsa-miR-664a-3p, hsa-miR-941, hsa-let-7i-5p, hsa-miR-100-5p, hsa-miR-181c-5p, hsa-miR-323b-3p, hsa-miR-1301-3p, hsa-miR-215-5p, hsa-miR-143-3p, hsa-miR-421 | 95.28 | 95.71 | 100 |
| 2359 | hsa-miR-106b-3p, hsa-miR-335-5p, hsa-miR-30b-5p, hsa-miR-664a-3p, hsa-miR-31-5p, hsa-miR-32-3p, hsa-miR-140-3p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-324-3p, hsa-miR-143-3p, hsa-miR-421 | 95.28 | 97.14 | 100 |
| 2360 | hsa-miR-106b-3p, hsa-miR-335-5p, hsa-miR-30b-5p, hsa-miR-664a-3p, hsa-miR-31-5p, hsa-miR-140-3p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-324-3p, hsa-miR-143-3p, hsa-miR-421 | 95.28 | 100 | 100 |
| 2361 | hsa-miR-106b-3p, hsa-miR-335-5p, hsa-miR-30b-5p, hsa-miR-664a-3p, hsa-let-7i-5p, hsa-miR-1296-5p, hsa-miR-100-5p, hsa-miR-181c-5p, hsa-miR-323b-3p, hsa-miR-1301-3p, hsa-miR-215-5p, hsa-miR-324-3p, hsa-miR-143-3p, hsa-miR-421 | 95.28 | 100 | 80 |
| 2362 | hsa-miR-106b-3p, hsa-miR-335-5p, hsa-miR-30b-5p, hsa-miR-664a-3p, hsa-miR-503-5p, hsa-let-7i-5p, hsa-miR-1296-5p, hsa-miR-100-5p, hsa-miR-181c-5p, hsa-miR-323b-3p, hsa-miR-20b-5p, hsa-miR-1301-3p, hsa-miR-215-5p, hsa-miR-324-3p, hsa-miR-421 | 95.28 | 100 | 100 |
| 2363 | hsa-miR-132-3p, hsa-miR-335-5p, hsa-miR-30b-5p, hsa-miR-31-5p, hsa-miR-32-3p, hsa-miR-140-3p, hsa-miR-503-5p, hsa-miR-30e-5p, hsa-miR-1296-5p, hsa-miR-323b-3p, hsa-miR-452-3p, hsa-miR-20b-5p, hsa-miR-190a-5p | 95.28 | 100 | 90 |
| 2364 | hsa-miR-132-3p, hsa-miR-30b-5p, hsa-miR-31-5p, hsa-miR-941, hsa-miR-32-3p, hsa-miR-140-3p, hsa-miR-30e-5p, hsa-miR-1296-5p, hsa-miR-181c-5p, hsa-miR-323b-3p, hsa-miR-452-3p, hsa-miR-190a-5p, hsa-miR-215-5p, hsa-miR-324-3p, hsa-miR-143-3p, hsa-miR-22-5p, hsa-miR-4306 | 95.28 | 97.14 | 100 |
| 2365 | hsa-miR-106b-3p, hsa-miR-335-5p, hsa-miR-30b-5p, hsa-miR-503-5p, hsa-let-7i-5p, hsa-miR-1296-5p, hsa-miR-100-5p, hsa-miR-323b-3p, hsa-miR-452-3p, hsa-miR-421, hsa-miR-22-5p | 95.28 | 100 | 90 |
| 2366 | hsa-miR-132-3p, hsa-miR-106b-3p, hsa-miR-30b-5p, hsa-miR-31-5p, hsa-miR-32-3p, hsa-miR-140-3p, hsa-miR-30e-5p, hsa-miR-1296-5p, hsa-miR-323b-3p, hsa-miR-20b-5p, hsa-miR-190a-5p, hsa-miR-215-5p, hsa-miR-324-3p, hsa-miR-143-3p, hsa-miR-421 | 95.28 | 100 | 100 |
| 2367 | hsa-miR-132-3p, hsa-miR-30b-5p, hsa-miR-941, hsa-miR-32-3p, hsa-miR-503-5p, hsa-miR-30e-5p, hsa-let-7i-5p, hsa-miR-1296-5p, hsa-miR-323b-3p, hsa-miR-452-3p, hsa-miR-20b-5p, hsa-miR-190a-5p, hsa-miR-324-3p, hsa-miR-421, hsa-miR-22-5p, hsa-miR-4306 | 95 | 100 | 100 |
| 2368 | hsa-miR-132-3p, hsa-miR-106b-3p, hsa-miR-30b-5p, hsa-miR-31-5p, hsa-miR-32-3p, hsa-miR-140-3p, hsa-miR-503-5p, hsa-miR-30e-5p, hsa-let-7i-5p, hsa-miR-1296-5p, hsa-miR-323b-3p, hsa-miR-452-3p, hsa-miR-190a-5p, hsa-miR-215-5p, hsa-miR-421, hsa-miR-22-5p | 95 | 94.29 | 100 |
| 2369 | hsa-miR-132-3p, hsa-miR-106b-3p, hsa-miR-30b-5p, hsa-miR-31-5p, hsa-miR-32-3p, hsa-miR-140-3p, hsa-miR-503-5p, hsa-miR-30e-5p, hsa-miR-1296-5p, hsa-miR-323b-3p, hsa-miR-452-3p, hsa-miR-190a-5p, hsa-miR-215-5p, hsa-miR-421 | 95 | 94.29 | 100 |
| 2370 | hsa-miR-132-3p, hsa-miR-335-5p, hsa-miR-30b-5p, hsa-miR-664a-3p, hsa-miR-31-5p, hsa-miR-941, hsa-miR-30e-5p, hsa-let-7i-5p, hsa-miR-452-3p, hsa-miR-20b-5p, hsa-miR-1301-3p, hsa-miR-190a-5p | 95 | 97.14 | 80 |
| 2371 | hsa-miR-132-3p, hsa-miR-106b-3p, hsa-miR-30b-5p, hsa-miR-664a-3p, hsa-miR-941, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p, hsa-miR-421, hsa-miR-4306 | 93.33 | 100 | 100 |
| 2372 | hsa-miR-106b-3p, hsa-miR-335-5p, hsa-miR-30b-5p, hsa-miR-664a-3p, hsa-miR-30e-5p, hsa-let-7i-5p, hsa-miR-1296-5p, hsa-miR-1249-3p, hsa-miR-100-5p, hsa-miR-1301-3p, hsa-miR-215-5p, hsa-miR-324-3p, hsa-miR-143-3p, hsa-miR-421 | 93.33 | 98.57 | 90 |
| 2373 | hsa-miR-106b-3p, hsa-miR-335-5p, hsa-miR-30b-5p, hsa-miR-664a-3p, hsa-miR-30e-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p | 93.33 | 100 | 90 |
| 2374 | hsa-miR-132-3p, hsa-miR-30b-5p, hsa-miR-503-5p, hsa-miR-30e-5p, hsa-let-7i-5p, hsa-miR-1296-5p, hsa-miR-452-3p, hsa-miR-324-3p, hsa-miR-421, hsa-miR-22-5p, hsa-miR-4306 | 93.33 | 94.29 | 90 |
| 2375 | hsa-miR-106b-3p, hsa-miR-335-5p, hsa-miR-30b-5p, hsa-miR-30e-5p, hsa-let-7i-5p, hsa-miR-1296-5p, hsa-miR-100-5p, hsa-miR-323b-3p, hsa-miR-452-3p, hsa-miR-215-5p, hsa-miR-324-3p, hsa-miR-143-3p, hsa-miR-421 | 93.33 | 100 | 90 |
| 2376 | hsa-miR-132-3p, hsa-miR-335-5p, hsa-miR-30b-5p, hsa-miR-503-5p, hsa-let-7i-5p, hsa-miR-1296-5p, hsa-miR-1249-3p, hsa-miR-100-5p, hsa-miR-323b-3p, hsa-miR-215-5p, hsa-miR-324-3p, hsa-miR-421, hsa-miR-22-5p | 93.33 | 98.57 | 90 |
| 2377 | hsa-miR-132-3p, hsa-miR-30b-5p, hsa-miR-32-3p, hsa-miR-30e-5p, hsa-miR-1296-5p, hsa-miR-323b-3p, hsa-miR-452-3p, hsa-miR-215-5p, hsa-miR-324-3p, hsa-miR-22-5p, hsa-miR-4306 | 93.33 | 94.29 | 100 |
| 2378 | hsa-miR-132-3p, hsa-miR-30b-5p, hsa-miR-140-3p, hsa-miR-30e-5p, hsa-miR-1296-5p, hsa-miR-323b-3p, hsa-miR-452-3p, hsa-miR-215-5p, hsa-miR-324-3p, hsa-miR-143-3p, hsa-miR-421, hsa-miR-22-5p, hsa-miR-4306 | 93.33 | 100 | 100 |
| 2379 | hsa-miR-132-3p, hsa-miR-941, hsa-miR-32-3p, hsa-miR-30e-5p, hsa-let-7i-5p, hsa-miR-1296-5p, hsa-miR-452-3p, hsa-miR-215-5p, hsa-miR-324-3p, hsa-miR-143-3p, hsa-miR-421, hsa-miR-22-5p | 93.33 | 97.14 | 80 |
| 2380 | hsa-miR-106b-3p, hsa-miR-335-5p, hsa-miR-30b-5p, hsa-miR-664a-3p, hsa-miR-503-5p, hsa-miR-30e-5p, hsa-miR-1296-5p, hsa-miR-100-5p, hsa-miR-323b-3p, hsa-miR-215-5p, hsa-miR-324-3p, hsa-miR-143-3p | 93.33 | 98.57 | 100 |
| 2381 | hsa-miR-132-3p, hsa-miR-106b-3p, hsa-miR-30b-5p, hsa-miR-664a-3p, hsa-miR-31-5p, hsa-miR-140-3p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-190a-5p, hsa-miR-324-3p, hsa-miR-143-3p, hsa-miR-421, hsa-miR-22-5p, hsa-miR-4306 | 93.33 | 98.57 | 100 |
| 2382 | hsa-miR-335-5p, hsa-miR-30b-5p, hsa-miR-503-5p, hsa-let-7i-5p, hsa-miR-1296-5p, hsa-miR-323b-3p, hsa-miR-452-3p, hsa-miR-20b-5p, hsa-miR-421 | 93.33 | 97.14 | 90 |
| 2383 | hsa-miR-106b-3p, hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-1249-3p, hsa-miR-100-5p, hsa-miR-1301-3p, hsa-miR-215-5p, hsa-miR-324-3p, hsa-miR-143-3p | 93.33 | 98.57 | 90 |
| 2384 | hsa-miR-132-3p, hsa-miR-106b-3p, hsa-miR-30b-5p, hsa-miR-664a-3p, hsa-miR-941, hsa-miR-30e-5p, hsa-let-7i-5p, hsa-miR-1296-5p, hsa-miR-1249-3p, hsa-miR-100-5p, hsa-miR-1301-3p, hsa-miR-190a-5p, hsa-miR-215-5p, hsa-miR-143-3p, hsa-miR-421, hsa-miR-22-5p, hsa-miR-4306 | 93.33 | 95.71 | 100 |
| 2385 | hsa-miR-335-5p, hsa-miR-30b-5p, hsa-miR-30e-5p, hsa-let-7i-5p, hsa-miR-1296-5p, hsa-miR-323b-3p, hsa-miR-452-3p, hsa-miR-1301-3p, hsa-miR-215-5p, hsa-miR-324-3p, hsa-miR-143-3p, hsa-miR-421 | 93.33 | 97.14 | 90 |
| 2386 | hsa-miR-132-3p, hsa-miR-30b-5p, hsa-miR-31-5p, hsa-miR-941, hsa-miR-32-3p, hsa-miR-140-3p, hsa-miR-30e-5p, hsa-miR-1296-5p, hsa-miR-181c-5p, hsa-miR-323b-3p, hsa-miR-452-3p, hsa-miR-20b-5p, hsa-miR-190a-5p, hsa-miR-215-5p, hsa-miR-324-3p, hsa-miR-143-3p, hsa-miR-22-5p, hsa-miR-4306 | 93.06 | 97.14 | 100 |
| 2387 | hsa-miR-106b-3p, hsa-miR-335-5p, hsa-miR-30b-5p, hsa-miR-664a-3p, hsa-miR-503-5p, hsa-miR-30e-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p | 93.06 | 100 | 80 |
| 2388 | hsa-miR-106b-3p, hsa-miR-335-5p, hsa-miR-30b-5p, hsa-miR-503-5p, hsa-miR-30e-5p, hsa-let-7i-5p, hsa-miR-1296-5p, hsa-miR-100-5p, hsa-miR-323b-3p, hsa-miR-452-3p, hsa-miR-1301-3p, hsa-miR-215-5p | 93.06 | 98.57 | 80 |
| 2389 | hsa-miR-132-3p, hsa-miR-106b-3p, hsa-miR-664a-3p, hsa-miR-31-5p, hsa-miR-30e-5p, hsa-let-7i-5p, hsa-miR-1296-5p, hsa-miR-100-5p, hsa-miR-323b-3p, hsa-miR-1301-3p, hsa-miR-190a-5p, hsa-miR-215-5p, hsa-miR-324-3p, hsa-miR-143-3p, hsa-miR-421, hsa-miR-22-5p | 93.06 | 95.71 | 100 |
| 2390 | hsa-miR-132-3p, hsa-miR-335-5p, hsa-miR-30b-5p, hsa-miR-664a-3p, hsa-miR-1249-3p, hsa-miR-100-5p, hsa-miR-181c-5p, hsa-miR-323b-3p, hsa-miR-1301-3p, hsa-miR-324-3p, hsa-miR-143-3p, hsa-miR-421 | 93.06 | 98.57 | 70 |
| 2391 | hsa-miR-106b-3p, hsa-miR-335-5p, hsa-miR-30b-5p, hsa-miR-664a-3p, hsa-miR-941, hsa-let-7i-5p, hsa-miR-100-5p, hsa-miR-181c-5p, hsa-miR-323b-3p, hsa-miR-1301-3p, hsa-miR-215-5p, hsa-miR-143-3p, hsa-miR-421 | 93.06 | 97.14 | 90 |
| 2392 | hsa-miR-132-3p, hsa-miR-31-5p, hsa-miR-941, hsa-miR-503-5p, hsa-miR-30e-5p, hsa-let-7i-5p, hsa-miR-1296-5p, hsa-miR-181c-5p, hsa-miR-452-3p, hsa-miR-190a-5p, hsa-miR-215-5p, hsa-miR-143-3p, hsa-miR-421, hsa-miR-22-5p, hsa-miR-4306 | 93.06 | 98.57 | 100 |
| 2393 | hsa-miR-106b-3p, hsa-miR-335-5p, hsa-miR-30b-5p, hsa-miR-664a-3p, hsa-miR-941, hsa-miR-32-3p, hsa-miR-140-3p, hsa-miR-503-5p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-215-5p, hsa-miR-324-3p, hsa-miR-421 | 93.06 | 100 | 100 |
| 2394 | hsa-miR-132-3p, hsa-miR-31-5p, hsa-miR-941, hsa-miR-140-3p, hsa-miR-503-5p, hsa-miR-30e-5p, hsa-miR-323b-3p, hsa-miR-452-3p, hsa-miR-190a-5p, hsa-miR-215-5p, hsa-miR-143-3p, hsa-miR-421, hsa-miR-22-5p, hsa-miR-4306 | 93.06 | 95.71 | 90 |
| 2395 | hsa-miR-106b-3p, hsa-miR-335-5p, hsa-miR-30b-5p, hsa-miR-664a-3p, hsa-miR-31-5p, hsa-miR-32-3p, hsa-miR-140-3p, hsa-miR-503-5p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-190a-5p, hsa-miR-324-3p | 93.06 | 97.14 | 100 |
| 2396 | hsa-miR-106b-3p, hsa-miR-335-5p, hsa-miR-30b-5p, hsa-miR-664a-3p, hsa-let-7i-5p, hsa-miR-100-5p, hsa-miR-181c-5p, hsa-miR-323b-3p, hsa-miR-1301-3p, hsa-miR-215-5p, hsa-miR-143-3p, hsa-miR-421 | 93.06 | 100 | 90 |
| 2397 | hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-324-3p, hsa-miR-421 | 93.06 | 97.14 | 100 |
| 2398 | hsa-miR-106b-3p, hsa-miR-335-5p, hsa-miR-664a-3p, hsa-miR-941, hsa-miR-30e-5p, hsa-let-7i-5p, hsa-miR-100-5p, hsa-miR-323b-3p, hsa-miR-1301-3p, hsa-miR-215-5p, hsa-miR-324-3p, hsa-miR-143-3p, hsa-miR-421, hsa-miR-22-5p | 93.06 | 97.14 | 100 |
| 2399 | hsa-miR-335-5p, hsa-miR-30b-5p, hsa-miR-30e-5p, hsa-let-7i-5p, hsa-miR-1296-5p, hsa-miR-181c-5p, hsa-miR-323b-3p, hsa-miR-452-3p, hsa-miR-324-3p, hsa-miR-143-3p | 93.06 | 94.29 | 80 |
| 2400 | hsa-miR-132-3p, hsa-miR-106b-3p, hsa-miR-30b-5p, hsa-miR-31-5p, hsa-miR-32-3p, hsa-miR-140-3p, hsa-miR-30e-5p, hsa-miR-1296-5p, hsa-miR-323b-3p, hsa-miR-452-3p, hsa-miR-1301-3p, hsa-miR-190a-5p, hsa-miR-215-5p, hsa-miR-143-3p, hsa-miR-421, hsa-miR-22-5p, hsa-miR-4306 | 92.78 | 100 | 100 |
| 2401 | hsa-miR-132-3p, hsa-miR-106b-3p, hsa-miR-30b-5p, hsa-miR-31-5p, hsa-miR-32-3p, hsa-miR-140-3p, hsa-miR-30e-5p, hsa-miR-1296-5p, hsa-miR-323b-3p, hsa-miR-452-3p, hsa-miR-190a-5p, hsa-miR-215-5p, hsa-miR-421 | 92.78 | 92.86 | 100 |
| 2402 | hsa-miR-132-3p, hsa-miR-106b-3p, hsa-miR-335-5p, hsa-miR-664a-3p, hsa-let-7i-5p, hsa-miR-100-5p, hsa-miR-181c-5p, hsa-miR-323b-3p, hsa-miR-1301-3p, hsa-miR-215-5p, hsa-miR-143-3p, hsa-miR-421 | 92.78 | 100 | 100 |
| 2403 | hsa-miR-132-3p, hsa-miR-106b-3p, hsa-miR-30b-5p, hsa-miR-664a-3p, hsa-miR-31-5p, hsa-miR-32-3p, hsa-miR-140-3p, hsa-miR-30e-5p, hsa-miR-1296-5p, hsa-miR-323b-3p, hsa-miR-452-3p, hsa-miR-20b-5p, hsa-miR-1301-3p, hsa-miR-190a-5p, hsa-miR-324-3p, hsa-miR-143-3p, hsa-miR-421, hsa-miR-22-5p, hsa-miR-4306 | 92.78 | 97.14 | 100 |
| 2404 | hsa-miR-335-5p, hsa-miR-30b-5p, hsa-miR-664a-3p, hsa-miR-503-5p, hsa-let-7i-5p, hsa-miR-1296-5p, hsa-miR-181c-5p, hsa-miR-323b-3p, hsa-miR-20b-5p, hsa-miR-1301-3p, hsa-miR-324-3p | 91.11 | 100 | 100 |
| 2405 | hsa-miR-132-3p, hsa-miR-106b-3p, hsa-miR-30b-5p, hsa-miR-664a-3p, hsa-let-7i-5p, hsa-miR-1296-5p, hsa-miR-1249-3p, hsa-miR-100-5p, hsa-miR-181c-5p, hsa-miR-1301-3p, hsa-miR-190a-5p, hsa-miR-215-5p, hsa-miR-143-3p, hsa-miR-421, hsa-miR-22-5p, hsa-miR-4306 | 91.11 | 94.29 | 90 |
| 2406 | hsa-miR-132-3p, hsa-miR-106b-3p, hsa-miR-30b-5p, hsa-miR-664a-3p, hsa-miR-941, hsa-miR-30e-5p, hsa-let-7i-5p, hsa-miR-100-5p, hsa-miR-323b-3p, hsa-miR-1301-3p, hsa-miR-215-5p, hsa-miR-324-3p, hsa-miR-143-3p, hsa-miR-421, hsa-miR-22-5p | 91.11 | 97.14 | 100 |
| 2407 | hsa-miR-132-3p, hsa-miR-106b-3p, hsa-miR-30b-5p, hsa-miR-664a-3p, hsa-miR-30e-5p, hsa-let-7i-5p, hsa-miR-1296-5p, hsa-miR-1249-3p, hsa-miR-100-5p, hsa-miR-1301-3p, hsa-miR-190a-5p, hsa-miR-215-5p, hsa-miR-324-3p, hsa-miR-143-3p, hsa-miR-421, hsa-miR-22-5p, hsa-miR-4306 | 91.11 | 95.71 | 100 |
| 2408 | hsa-miR-106b-3p, hsa-miR-335-5p, hsa-miR-664a-3p, hsa-miR-941, hsa-miR-503-5p, hsa-miR-30e-5p, hsa-let-7i-5p, hsa-miR-1249-3p, hsa-miR-100-5p, hsa-miR-1301-3p, hsa-miR-215-5p, hsa-miR-324-3p | 91.11 | 98.57 | 90 |
| 2409 | hsa-miR-132-3p, hsa-miR-106b-3p, hsa-miR-30b-5p, hsa-miR-664a-3p, hsa-miR-941, hsa-miR-30e-5p, hsa-let-7i-5p, hsa-miR-1296-5p, hsa-miR-1249-3p, hsa-miR-100-5p, hsa-miR-1301-3p, hsa-miR-190a-5p, hsa-miR-215-5p, hsa-miR-324-3p, hsa-miR-143-3p, hsa-miR-421, hsa-miR-22-5p, hsa-miR-4306 | 91.11 | 97.14 | 100 |
| 2410 | hsa-miR-132-3p, hsa-miR-335-5p, hsa-miR-30b-5p, hsa-miR-664a-3p, hsa-let-7i-5p, hsa-miR-1249-3p, hsa-miR-100-5p, hsa-miR-181c-5p, hsa-miR-323b-3p, hsa-miR-1301-3p, hsa-miR-324-3p, hsa-miR-421, hsa-miR-22-5p | 91.11 | 98.57 | 90 |
| 2411 | hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-324-3p, hsa-miR-143-3p | 91.11 | 97.14 | 100 |
| 2412 | hsa-miR-106b-3p, hsa-miR-335-5p, hsa-miR-30b-5p, hsa-miR-664a-3p, hsa-miR-32-3p, hsa-miR-140-3p, hsa-miR-503-5p, hsa-miR-30e-5p, hsa-miR-1296-5p, hsa-miR-100-5p, hsa-miR-323b-3p, hsa-miR-20b-5p, hsa-miR-1301-3p, hsa-miR-215-5p, hsa-miR-324-3p | 91.11 | 98.57 | 90 |
| 2413 | hsa-miR-106b-3p, hsa-miR-335-5p, hsa-miR-30b-5p, hsa-miR-664a-3p, hsa-miR-31-5p, hsa-let-7i-5p, hsa-miR-1296-5p, hsa-miR-1249-3p, hsa-miR-100-5p, hsa-miR-181c-5p, hsa-miR-1301-3p, hsa-miR-190a-5p, hsa-miR-143-3p, hsa-miR-421, hsa-miR-4306 | 91.11 | 97.14 | 90 |
| 2414 | hsa-miR-132-3p, hsa-miR-32-3p, hsa-miR-30e-5p, hsa-let-7i-5p, hsa-miR-1296-5p, hsa-miR-323b-3p, hsa-miR-452-3p, hsa-miR-190a-5p, hsa-miR-22-5p, hsa-miR-4306 | 91.11 | 91.43 | 80 |
| 2415 | hsa-miR-30b-5p, hsa-miR-31-5p, hsa-miR-140-3p, hsa-miR-30e-5p, hsa-miR-1296-5p, hsa-miR-323b-3p, hsa-miR-452-3p, hsa-miR-20b-5p, hsa-miR-190a-5p, hsa-miR-215-5p, hsa-miR-143-3p, hsa-miR-22-5p, hsa-miR-4306 | 91.11 | 97.14 | 80 |
| 2416 | hsa-miR-106b-3p, hsa-miR-335-5p, hsa-miR-30b-5p, hsa-miR-664a-3p, hsa-miR-30e-5p, hsa-let-7i-5p, hsa-miR-1296-5p, hsa-miR-1249-3p, hsa-miR-100-5p, hsa-miR-1301-3p, hsa-miR-215-5p, hsa-miR-324-3p, hsa-miR-143-3p | 91.11 | 95.71 | 90 |
| 2417 | hsa-miR-106b-3p, hsa-miR-335-5p, hsa-miR-30b-5p, hsa-miR-30e-5p, hsa-let-7i-5p, hsa-miR-1296-5p, hsa-miR-100-5p, hsa-miR-323b-3p, hsa-miR-452-3p, hsa-miR-215-5p, hsa-miR-143-3p | 90.83 | 98.57 | 70 |
| 2418 | hsa-miR-132-3p, hsa-miR-31-5p, hsa-miR-941, hsa-miR-503-5p, hsa-miR-30e-5p, hsa-let-7i-5p, hsa-miR-1249-3p, hsa-miR-323b-3p, hsa-miR-452-3p, hsa-miR-20b-5p, hsa-miR-190a-5p, hsa-miR-215-5p, hsa-miR-421, hsa-miR-22-5p, hsa-miR-4306 | 90.83 | 97.14 | 80 |
| 2419 | hsa-miR-106b-3p, hsa-miR-30b-5p, hsa-miR-32-3p, hsa-miR-503-5p, hsa-miR-30e-5p, hsa-let-7i-5p, hsa-miR-1296-5p, hsa-miR-100-5p, hsa-miR-323b-3p, hsa-miR-452-3p, hsa-miR-20b-5p, hsa-miR-1301-3p, hsa-miR-421, hsa-miR-22-5p, hsa-miR-4306 | 90.83 | 91.43 | 100 |
| 2420 | hsa-miR-335-5p, hsa-miR-30b-5p, hsa-miR-664a-3p, hsa-miR-503-5p, hsa-miR-30e-5p, hsa-let-7i-5p, hsa-miR-1296-5p, hsa-miR-323b-3p, hsa-miR-20b-5p, hsa-miR-324-3p | 90.83 | 94.29 | 80 |
| 2421 | hsa-miR-132-3p, hsa-miR-30b-5p, hsa-miR-32-3p, hsa-miR-140-3p, hsa-miR-503-5p, hsa-miR-30e-5p, hsa-miR-1296-5p, hsa-miR-22-5p | 90.83 | 92.86 | 60 |
| 2422 | hsa-miR-106b-3p, hsa-miR-335-5p, hsa-miR-30b-5p, hsa-miR-664a-3p, hsa-miR-941, hsa-miR-30e-5p, hsa-let-7i-5p, hsa-miR-100-5p, hsa-miR-323b-3p, hsa-miR-1301-3p, hsa-miR-215-5p, hsa-miR-324-3p, hsa-miR-143-3p, hsa-miR-421 | 90.83 | 100 | 100 |
| 2423 | hsa-miR-106b-3p, hsa-miR-335-5p, hsa-miR-30b-5p, hsa-miR-664a-3p, hsa-miR-31-5p, hsa-miR-32-3p, hsa-miR-140-3p, hsa-miR-503-5p, hsa-miR-30e-5p, hsa-let-7i-5p, hsa-miR-1296-5p, hsa-miR-1249-3p, hsa-miR-100-5p, hsa-miR-20b-5p, hsa-miR-1301-3p, hsa-miR-190a-5p, hsa-miR-215-5p, hsa-miR-324-3p, hsa-miR-421 | 90.83 | 95.71 | 90 |
| 2424 | hsa-miR-132-3p, hsa-miR-31-5p, hsa-miR-941, hsa-miR-30e-5p, hsa-let-7i-5p, hsa-miR-1249-3p, hsa-miR-323b-3p, hsa-miR-452-3p, hsa-miR-20b-5p, hsa-miR-190a-5p, hsa-miR-215-5p, hsa-miR-143-3p, hsa-miR-421, hsa-miR-22-5p, hsa-miR-4306 | 90.83 | 98.57 | 80 |
| 2425 | hsa-miR-335-5p, hsa-miR-30b-5p, hsa-miR-664a-3p, hsa-miR-30e-5p, hsa-miR-1296-5p, hsa-miR-323b-3p, hsa-miR-1301-3p, hsa-miR-215-5p, hsa-miR-324-3p, hsa-miR-143-3p, hsa-miR-421 | 90.83 | 100 | 70 |
| 2426 | hsa-miR-106b-3p, hsa-miR-335-5p, hsa-miR-664a-3p, hsa-miR-941, hsa-miR-30e-5p, hsa-let-7i-5p, hsa-miR-100-5p, hsa-miR-323b-3p, hsa-miR-1301-3p, hsa-miR-215-5p, hsa-miR-324-3p, hsa-miR-143-3p, hsa-miR-421 | 90.83 | 100 | 100 |
| 2427 | hsa-miR-132-3p, hsa-miR-106b-3p, hsa-miR-30b-5p, hsa-miR-664a-3p, hsa-miR-941, hsa-miR-30e-5p, hsa-let-7i-5p, hsa-miR-1296-5p, hsa-miR-1249-3p, hsa-miR-100-5p, hsa-miR-1301-3p, hsa-miR-190a-5p, hsa-miR-215-5p, hsa-miR-324-3p, hsa-miR-143-3p, hsa-miR-421, hsa-miR-4306 | 90.83 | 98.57 | 100 |
| 2428 | hsa-miR-132-3p, hsa-miR-106b-3p, hsa-miR-664a-3p, hsa-miR-30e-5p, hsa-let-7i-5p, hsa-miR-1296-5p, hsa-miR-1249-3p, hsa-miR-100-5p, hsa-miR-1301-3p, hsa-miR-215-5p, hsa-miR-143-3p, hsa-miR-421, hsa-miR-22-5p, hsa-miR-4306 | 90.83 | 95.71 | 70 |
| 2429 | hsa-miR-106b-3p, hsa-miR-335-5p, hsa-miR-30b-5p, hsa-miR-664a-3p, hsa-miR-32-3p, hsa-miR-140-3p, hsa-miR-30e-5p, hsa-let-7i-5p, hsa-miR-1296-5p, hsa-miR-1249-3p, hsa-miR-100-5p, hsa-miR-20b-5p, hsa-miR-1301-3p, hsa-miR-324-3p, hsa-miR-421 | 90.83 | 95.71 | 90 |
| 2430 | hsa-miR-106b-3p, hsa-miR-335-5p, hsa-miR-30b-5p, hsa-miR-664a-3p, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p | 90.83 | 97.14 | 80 |
| 2431 | hsa-miR-106b-3p, hsa-miR-335-5p, hsa-miR-30b-5p, hsa-miR-664a-3p, hsa-miR-32-3p, hsa-miR-503-5p, hsa-miR-30e-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p | 90.83 | 100 | 80 |
| 2432 | hsa-miR-106b-3p, hsa-miR-335-5p, hsa-miR-30b-5p, hsa-miR-664a-3p, hsa-miR-941, hsa-miR-30e-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p | 90.83 | 98.57 | 80 |
| 2433 | hsa-miR-106b-3p, hsa-miR-335-5p, hsa-miR-664a-3p, hsa-miR-30e-5p, hsa-let-7i-5p, hsa-miR-100-5p, hsa-miR-323b-3p, hsa-miR-1301-3p, hsa-miR-215-5p, hsa-miR-324-3p, hsa-miR-143-3p, hsa-miR-421 | 90.83 | 100 | 100 |
| 2434 | hsa-miR-132-3p, hsa-miR-30b-5p, hsa-miR-140-3p, hsa-miR-30e-5p, hsa-miR-1296-5p, hsa-miR-323b-3p, hsa-miR-452-3p, hsa-miR-190a-5p, hsa-miR-215-5p, hsa-miR-143-3p, hsa-miR-22-5p, hsa-miR-4306 | 90.83 | 100 | 70 |
| 2435 | hsa-miR-106b-3p, hsa-miR-335-5p, hsa-miR-30b-5p, hsa-miR-664a-3p, hsa-miR-941, hsa-let-7i-5p, hsa-miR-1296-5p, hsa-miR-100-5p, hsa-miR-181c-5p, hsa-miR-323b-3p, hsa-miR-1301-3p, hsa-miR-215-5p, hsa-miR-143-3p, hsa-miR-421 | 90.83 | 100 | 90 |
| 2436 | hsa-miR-132-3p, hsa-miR-335-5p, hsa-miR-664a-3p, hsa-miR-503-5p, hsa-miR-30e-5p, hsa-let-7i-5p, hsa-miR-1296-5p, hsa-miR-100-5p, hsa-miR-323b-3p, hsa-miR-452-3p, hsa-miR-1301-3p, hsa-miR-421, hsa-miR-22-5p | 90.83 | 91.43 | 90 |
| 2437 | hsa-miR-30b-5p, hsa-miR-503-5p, hsa-let-7i-5p, hsa-miR-1249-3p, hsa-miR-181c-5p, hsa-miR-452-3p, hsa-miR-421, hsa-miR-22-5p, hsa-miR-4306 | 90.83 | 98.57 | 90 |
| 2438 | hsa-miR-106b-3p, hsa-miR-335-5p, hsa-miR-30b-5p, hsa-miR-664a-3p, hsa-miR-941, hsa-let-7i-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p, hsa-miR-421 | 90.83 | 100 | 100 |
| 2439 | hsa-miR-106b-3p, hsa-miR-335-5p, hsa-miR-30b-5p, hsa-miR-664a-3p, hsa-miR-31-5p, hsa-let-7i-5p, hsa-miR-1296-5p, hsa-miR-100-5p, hsa-miR-181c-5p, hsa-miR-323b-3p, hsa-miR-20b-5p, hsa-miR-1301-3p, hsa-miR-190a-5p, hsa-miR-143-3p, hsa-miR-421 | 90.83 | 98.57 | 90 |
| 2440 | hsa-miR-335-5p, hsa-miR-30b-5p, hsa-miR-503-5p, hsa-let-7i-5p, hsa-miR-1296-5p, hsa-miR-100-5p, hsa-miR-323b-3p, hsa-miR-452-3p, hsa-miR-20b-5p, hsa-miR-421 | 90.83 | 97.14 | 100 |
| 2441 | hsa-miR-106b-3p, hsa-miR-335-5p, hsa-miR-30b-5p, hsa-miR-664a-3p, hsa-miR-31-5p, hsa-miR-941, hsa-miR-140-3p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-324-3p, hsa-miR-143-3p, hsa-miR-421 | 90.56 | 100 | 100 |
| 2442 | hsa-miR-106b-3p, hsa-miR-335-5p, hsa-miR-30b-5p, hsa-miR-664a-3p, hsa-miR-503-5p, hsa-miR-1296-5p, hsa-miR-100-5p, hsa-miR-181c-5p, hsa-miR-323b-3p, hsa-miR-1301-3p, hsa-miR-215-5p | 90.56 | 94.29 | 60 |
| 2443 | hsa-miR-106b-3p, hsa-miR-335-5p, hsa-miR-30b-5p, hsa-miR-664a-3p, hsa-miR-941, hsa-miR-503-5p, hsa-miR-1296-5p, hsa-miR-100-5p, hsa-miR-181c-5p, hsa-miR-323b-3p, hsa-miR-1301-3p, hsa-miR-215-5p | 90.56 | 94.29 | 60 |
| 2444 | hsa-miR-335-5p, hsa-miR-664a-3p, hsa-miR-503-5p, hsa-miR-30e-5p, hsa-let-7i-5p, hsa-miR-1296-5p, hsa-miR-1249-3p, hsa-miR-1301-3p, hsa-miR-324-3p, hsa-miR-421 | 90.56 | 95.71 | 80 |
| 2445 | hsa-miR-106b-3p, hsa-miR-335-5p, hsa-miR-30b-5p, hsa-miR-664a-3p, hsa-miR-941, hsa-let-7i-5p, hsa-miR-1296-5p, hsa-miR-100-5p, hsa-miR-181c-5p, hsa-miR-323b-3p, hsa-miR-1301-3p, hsa-miR-215-5p, hsa-miR-324-3p, hsa-miR-143-3p, hsa-miR-421 | 90.56 | 100 | 80 |
| 2446 | hsa-miR-106b-3p, hsa-miR-30b-5p, hsa-miR-664a-3p, hsa-miR-941, hsa-miR-32-3p, hsa-miR-30e-5p, hsa-let-7i-5p, hsa-miR-1296-5p, hsa-miR-1249-3p, hsa-miR-181c-5p, hsa-miR-190a-5p, hsa-miR-215-5p, hsa-miR-324-3p, hsa-miR-143-3p, hsa-miR-421, hsa-miR-22-5p | 90.56 | 95.71 | 100 |
| 2447 | hsa-miR-106b-3p, hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-100-5p, hsa-miR-1301-3p, hsa-miR-190a-5p, hsa-miR-215-5p, hsa-miR-324-3p, hsa-miR-421 | 90.56 | 100 | 90 |
| 2448 | hsa-miR-132-3p, hsa-miR-106b-3p, hsa-miR-30b-5p, hsa-miR-31-5p, hsa-miR-32-3p, hsa-miR-140-3p, hsa-miR-503-5p, hsa-miR-30e-5p, hsa-let-7i-5p, hsa-miR-1296-5p, hsa-miR-323b-3p, hsa-miR-452-3p, hsa-miR-190a-5p, hsa-miR-215-5p, hsa-miR-143-3p, hsa-miR-421 | 90.56 | 94.29 | 100 |
| 2449 | hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-324-3p, hsa-miR-143-3p, hsa-miR-421 | 90.56 | 100 | 100 |
| 2450 | hsa-miR-106b-3p, hsa-miR-31-5p, hsa-miR-32-3p, hsa-miR-140-3p, hsa-miR-30e-5p, hsa-miR-1296-5p, hsa-miR-323b-3p, hsa-miR-452-3p, hsa-miR-190a-5p, hsa-miR-421, hsa-miR-22-5p | 90.56 | 90 | 90 |
| 2451 | hsa-miR-30b-5p, hsa-miR-503-5p, hsa-miR-30e-5p, hsa-let-7i-5p, hsa-miR-1296-5p, hsa-miR-421 | 90.56 | 95.71 | 80 |
| 2452 | hsa-miR-106b-3p, hsa-miR-335-5p, hsa-miR-664a-3p, hsa-miR-31-5p, hsa-miR-941, hsa-miR-32-3p, hsa-miR-140-3p, hsa-miR-30e-5p, hsa-let-7i-5p, hsa-miR-100-5p, hsa-miR-323b-3p, hsa-miR-1301-3p, hsa-miR-190a-5p, hsa-miR-215-5p, hsa-miR-324-3p, hsa-miR-143-3p, hsa-miR-421 | 90.56 | 92.86 | 100 |
| 2453 | hsa-miR-132-3p, hsa-miR-30b-5p, hsa-miR-664a-3p, hsa-miR-31-5p, hsa-miR-140-3p, hsa-miR-503-5p, hsa-miR-30e-5p, hsa-miR-1296-5p, hsa-miR-323b-3p, hsa-miR-190a-5p, hsa-miR-215-5p, hsa-miR-143-3p, hsa-miR-421, hsa-miR-22-5p, hsa-miR-4306 | 90.56 | 95.71 | 100 |
| 2454 | hsa-miR-106b-3p, hsa-miR-335-5p, hsa-miR-30b-5p, hsa-miR-31-5p, hsa-miR-32-3p, hsa-miR-140-3p, hsa-miR-1296-5p, hsa-miR-323b-3p, hsa-miR-452-3p, hsa-miR-190a-5p, hsa-miR-324-3p, hsa-miR-421 | 90.56 | 98.57 | 90 |
| 2455 | hsa-miR-106b-3p, hsa-miR-335-5p, hsa-miR-30b-5p, hsa-miR-664a-3p, hsa-miR-32-3p, hsa-miR-140-3p, hsa-miR-503-5p, hsa-let-7i-5p, hsa-miR-1296-5p, hsa-miR-100-5p, hsa-miR-181c-5p, hsa-miR-323b-3p, hsa-miR-20b-5p, hsa-miR-1301-3p, hsa-miR-215-5p, hsa-miR-421 | 90.28 | 92.86 | 60 |
| 2456 | hsa-miR-30b-5p, hsa-miR-664a-3p, hsa-miR-31-5p, hsa-miR-140-3p, hsa-miR-503-5p, hsa-miR-1296-5p, hsa-miR-181c-5p, hsa-miR-323b-3p, hsa-miR-452-3p, hsa-miR-190a-5p, hsa-miR-215-5p, hsa-miR-421, hsa-miR-22-5p, hsa-miR-4306 | 90.28 | 92.86 | 80 |
| 2457 | hsa-miR-106b-3p, hsa-miR-335-5p, hsa-miR-30b-5p, hsa-miR-664a-3p, hsa-miR-31-5p, hsa-miR-30e-5p, hsa-let-7i-5p, hsa-miR-1296-5p, hsa-miR-1249-3p, hsa-miR-100-5p, hsa-miR-1301-3p, hsa-miR-190a-5p, hsa-miR-324-3p, hsa-miR-143-3p, hsa-miR-421, hsa-miR-4306 | 88.89 | 92.86 | 80 |
| 2458 | hsa-miR-132-3p, hsa-miR-106b-3p, hsa-miR-664a-3p, hsa-miR-30e-5p, hsa-let-7i-5p, hsa-miR-1296-5p, hsa-miR-100-5p, hsa-miR-323b-3p, hsa-miR-1301-3p, hsa-miR-324-3p, hsa-miR-143-3p, hsa-miR-421, hsa-miR-22-5p, hsa-miR-4306 | 88.89 | 95.71 | 90 |
| 2459 | hsa-miR-132-3p, hsa-miR-30b-5p, hsa-miR-32-3p, hsa-miR-503-5p, hsa-miR-30e-5p, hsa-let-7i-5p, hsa-miR-1296-5p, hsa-miR-452-3p, hsa-miR-20b-5p, hsa-miR-324-3p, hsa-miR-143-3p, hsa-miR-22-5p, hsa-miR-4306 | 88.89 | 94.29 | 90 |
| 2460 | hsa-miR-132-3p, hsa-miR-106b-3p, hsa-miR-664a-3p, hsa-miR-30e-5p, hsa-let-7i-5p, hsa-miR-1296-5p, hsa-miR-1249-3p, hsa-miR-100-5p, hsa-miR-1301-3p, hsa-miR-190a-5p, hsa-miR-215-5p, hsa-miR-324-3p, hsa-miR-143-3p, hsa-miR-421, hsa-miR-22-5p, hsa-miR-4306 | 88.89 | 94.29 | 90 |
| 2461 | hsa-miR-132-3p, hsa-miR-106b-3p, hsa-miR-30b-5p, hsa-miR-664a-3p, hsa-miR-30e-5p, hsa-let-7i-5p, hsa-miR-1296-5p, hsa-miR-1249-3p, hsa-miR-100-5p, hsa-miR-1301-3p, hsa-miR-215-5p, hsa-miR-324-3p, hsa-miR-143-3p, hsa-miR-421, hsa-miR-22-5p, hsa-miR-4306 | 88.89 | 94.29 | 100 |
| 2462 | hsa-miR-106b-3p, hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-1249-3p, hsa-miR-100-5p, hsa-miR-1301-3p, hsa-miR-190a-5p, hsa-miR-215-5p, hsa-miR-143-3p, hsa-miR-421, hsa-miR-22-5p | 88.61 | 95.71 | 90 |
| 2463 | hsa-miR-106b-3p, hsa-miR-30b-5p, hsa-miR-664a-3p, hsa-miR-30e-5p, hsa-let-7i-5p, hsa-miR-1296-5p, hsa-miR-1249-3p, hsa-miR-100-5p, hsa-miR-1301-3p, hsa-miR-190a-5p, hsa-miR-215-5p, hsa-miR-324-3p, hsa-miR-143-3p, hsa-miR-421, hsa-miR-22-5p, hsa-miR-4306 | 88.61 | 94.29 | 90 |
| 2464 | hsa-miR-335-5p, hsa-miR-30b-5p, hsa-miR-503-5p, hsa-miR-30e-5p, hsa-let-7i-5p, hsa-miR-1296-5p, hsa-miR-100-5p, hsa-miR-323b-3p, hsa-miR-452-3p, hsa-miR-215-5p | 88.61 | 100 | 60 |
| 2465 | hsa-miR-106b-3p, hsa-miR-335-5p, hsa-miR-664a-3p, hsa-miR-941, hsa-miR-30e-5p, hsa-let-7i-5p, hsa-miR-1296-5p, hsa-miR-1249-3p, hsa-miR-100-5p, hsa-miR-1301-3p, hsa-miR-215-5p, hsa-miR-324-3p, hsa-miR-143-3p, hsa-miR-421 | 88.61 | 98.57 | 90 |
| 2466 | hsa-miR-106b-3p, hsa-miR-335-5p, hsa-miR-664a-3p, hsa-miR-941, hsa-miR-30e-5p, hsa-let-7i-5p, hsa-miR-100-5p, hsa-miR-323b-3p, hsa-miR-20b-5p, hsa-miR-1301-3p, hsa-miR-215-5p, hsa-miR-324-3p, hsa-miR-143-3p, hsa-miR-421 | 88.61 | 98.57 | 100 |
| 2467 | hsa-miR-132-3p, hsa-miR-335-5p, hsa-miR-941, hsa-miR-32-3p, hsa-miR-30e-5p, hsa-miR-1296-5p, hsa-miR-100-5p, hsa-miR-181c-5p, hsa-miR-20b-5p, hsa-miR-190a-5p, hsa-miR-324-3p, hsa-miR-143-3p, hsa-miR-4306 | 88.61 | 98.57 | 100 |
| 2468 | hsa-miR-132-3p, hsa-miR-106b-3p, hsa-miR-335-5p, hsa-miR-30b-5p, hsa-miR-664a-3p, hsa-miR-31-5p, hsa-miR-941, hsa-miR-30e-5p, hsa-miR-100-5p, hsa-miR-181c-5p, hsa-miR-323b-3p, hsa-miR-1301-3p, hsa-miR-22-5p, hsa-miR-4306 | 88.61 | 94.29 | 70 |
| 2469 | hsa-miR-132-3p, hsa-miR-106b-3p, hsa-miR-30b-5p, hsa-miR-664a-3p, hsa-miR-31-5p, hsa-miR-941, hsa-miR-30e-5p, hsa-let-7i-5p, hsa-miR-1296-5p, hsa-miR-1249-3p, hsa-miR-100-5p, hsa-miR-1301-3p, hsa-miR-190a-5p, hsa-miR-324-3p, hsa-miR-143-3p, hsa-miR-421, hsa-miR-22-5p, hsa-miR-4306 | 88.61 | 92.86 | 100 |
| 2470 | hsa-miR-30b-5p, hsa-miR-31-5p, hsa-miR-32-3p, hsa-miR-140-3p, hsa-miR-503-5p, hsa-miR-30e-5p, hsa-miR-1296-5p, hsa-miR-323b-3p, hsa-miR-452-3p, hsa-miR-20b-5p, hsa-miR-143-3p, hsa-miR-421, hsa-miR-22-5p, hsa-miR-4306 | 88.61 | 91.43 | 80 |
| 2471 | hsa-miR-106b-3p, hsa-miR-335-5p, hsa-miR-30b-5p, hsa-miR-664a-3p, hsa-miR-30e-5p, hsa-miR-1296-5p, hsa-miR-1249-3p, hsa-miR-100-5p, hsa-miR-20b-5p, hsa-miR-1301-3p, hsa-miR-215-5p, hsa-miR-324-3p, hsa-miR-143-3p, hsa-miR-421 | 88.61 | 95.71 | 90 |
| 2472 | hsa-miR-106b-3p, hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-1249-3p, hsa-miR-100-5p, hsa-miR-1301-3p, hsa-miR-215-5p, hsa-miR-324-3p, hsa-miR-143-3p, hsa-miR-421 | 88.61 | 98.57 | 90 |
| 2473 | hsa-miR-106b-3p, hsa-miR-335-5p, hsa-miR-30b-5p, hsa-miR-503-5p, hsa-miR-30e-5p, hsa-miR-1296-5p, hsa-miR-100-5p, hsa-miR-323b-3p, hsa-miR-452-3p, hsa-miR-1301-3p, hsa-miR-215-5p, hsa-miR-324-3p, hsa-miR-143-3p | 88.61 | 98.57 | 80 |
| 2474 | hsa-miR-106b-3p, hsa-miR-335-5p, hsa-miR-664a-3p, hsa-miR-941, hsa-let-7i-5p, hsa-miR-100-5p, hsa-miR-181c-5p, hsa-miR-323b-3p, hsa-miR-1301-3p, hsa-miR-215-5p, hsa-miR-324-3p, hsa-miR-143-3p, hsa-miR-421 | 88.61 | 97.14 | 100 |
| 2475 | hsa-miR-106b-3p, hsa-miR-335-5p, hsa-miR-664a-3p, hsa-miR-30e-5p, hsa-let-7i-5p, hsa-miR-100-5p, hsa-miR-323b-3p, hsa-miR-1301-3p, hsa-miR-324-3p, hsa-miR-143-3p, hsa-miR-421 | 88.61 | 100 | 100 |
| 2476 | hsa-miR-106b-3p, hsa-miR-335-5p, hsa-miR-30b-5p, hsa-miR-664a-3p, hsa-miR-941, hsa-miR-30e-5p, hsa-let-7i-5p, hsa-miR-1296-5p, hsa-miR-1249-3p, hsa-miR-100-5p, hsa-miR-1301-3p, hsa-miR-324-3p, hsa-miR-143-3p, hsa-miR-421 | 88.61 | 97.14 | 90 |
| 2477 | hsa-miR-106b-3p, hsa-miR-335-5p, hsa-miR-30b-5p, hsa-miR-664a-3p, hsa-miR-31-5p, hsa-miR-32-3p, hsa-miR-140-3p, hsa-miR-503-5p, hsa-miR-30e-5p, hsa-let-7i-5p, hsa-miR-1296-5p, hsa-miR-100-5p, hsa-miR-323b-3p, hsa-miR-20b-5p, hsa-miR-1301-3p, hsa-miR-324-3p | 88.61 | 92.86 | 80 |
| 2478 | hsa-miR-106b-3p, hsa-miR-335-5p, hsa-miR-30b-5p, hsa-miR-664a-3p, hsa-miR-941, hsa-let-7i-5p, hsa-miR-1296-5p, hsa-miR-100-5p, hsa-miR-181c-5p, hsa-miR-323b-3p, hsa-miR-1301-3p, hsa-miR-215-5p, hsa-miR-324-3p, hsa-miR-143-3p, hsa-miR-421, hsa-miR-4306 | 88.61 | 97.14 | 100 |
| 2479 | hsa-miR-132-3p, hsa-miR-30b-5p, hsa-miR-32-3p, hsa-miR-140-3p, hsa-miR-30e-5p, hsa-miR-1249-3p, hsa-miR-323b-3p, hsa-miR-452-3p, hsa-miR-20b-5p, hsa-miR-324-3p, hsa-miR-22-5p | 88.61 | 94.29 | 90 |
| 2480 | hsa-miR-106b-3p, hsa-miR-335-5p, hsa-miR-30b-5p, hsa-miR-664a-3p, hsa-miR-30e-5p, hsa-miR-1296-5p, hsa-miR-100-5p, hsa-miR-323b-3p, hsa-miR-215-5p, hsa-miR-324-3p, hsa-miR-143-3p | 88.61 | 98.57 | 70 |
| 2481 | hsa-miR-132-3p, hsa-miR-106b-3p, hsa-miR-30b-5p, hsa-miR-664a-3p, hsa-miR-31-5p, hsa-miR-941, hsa-miR-140-3p, hsa-miR-30e-5p, hsa-let-7i-5p, hsa-miR-1296-5p, hsa-miR-1249-3p, hsa-miR-100-5p, hsa-miR-323b-3p, hsa-miR-20b-5p, hsa-miR-1301-3p, hsa-miR-190a-5p, hsa-miR-324-3p, hsa-miR-143-3p, hsa-miR-421, hsa-miR-22-5p, hsa-miR-4306 | 88.61 | 94.29 | 100 |
| 2482 | hsa-miR-106b-3p, hsa-miR-335-5p, hsa-miR-30b-5p, hsa-miR-664a-3p, hsa-miR-31-5p, hsa-miR-941, hsa-miR-140-3p, hsa-miR-30e-5p, hsa-let-7i-5p, hsa-miR-1296-5p, hsa-miR-1249-3p, hsa-miR-100-5p, hsa-miR-20b-5p, hsa-miR-1301-3p, hsa-miR-190a-5p, hsa-miR-215-5p, hsa-miR-324-3p, hsa-miR-143-3p, hsa-miR-421 | 88.33 | 98.57 | 90 |
| 2483 | hsa-miR-132-3p, hsa-miR-106b-3p, hsa-miR-335-5p, hsa-miR-30b-5p, hsa-miR-31-5p, hsa-miR-32-3p, hsa-miR-140-3p, hsa-miR-30e-5p, hsa-miR-1296-5p, hsa-miR-100-5p, hsa-miR-181c-5p, hsa-miR-452-3p, hsa-miR-190a-5p, hsa-miR-143-3p, hsa-miR-421, hsa-miR-22-5p | 88.33 | 95.71 | 100 |
| 2484 | hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-30e-5p, hsa-let-7i-5p, hsa-miR-1296-5p, hsa-miR-1249-3p, hsa-miR-100-5p, hsa-miR-20b-5p, hsa-miR-1301-3p, hsa-miR-190a-5p, hsa-miR-215-5p, hsa-miR-324-3p, hsa-miR-143-3p, hsa-miR-421 | 88.33 | 95.71 | 90 |
| 2485 | hsa-miR-132-3p, hsa-miR-106b-3p, hsa-miR-664a-3p, hsa-miR-941, hsa-miR-30e-5p, hsa-let-7i-5p, hsa-miR-1296-5p, hsa-miR-1249-3p, hsa-miR-100-5p, hsa-miR-1301-3p, hsa-miR-190a-5p, hsa-miR-215-5p, hsa-miR-324-3p, hsa-miR-143-3p, hsa-miR-421, hsa-miR-22-5p, hsa-miR-4306 | 88.33 | 98.57 | 90 |
| 2486 | hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-30e-5p, hsa-let-7i-5p, hsa-miR-1296-5p, hsa-miR-1249-3p, hsa-miR-100-5p, hsa-miR-20b-5p, hsa-miR-1301-3p, hsa-miR-190a-5p, hsa-miR-324-3p, hsa-miR-143-3p, hsa-miR-421 | 88.33 | 92.86 | 90 |
| 2487 | hsa-miR-132-3p, hsa-miR-30b-5p, hsa-miR-664a-3p, hsa-miR-31-5p, hsa-miR-941, hsa-miR-30e-5p, hsa-let-7i-5p, hsa-miR-1249-3p, hsa-miR-190a-5p, hsa-miR-4306 | 88.33 | 87.14 | 50 |
| 2488 | hsa-miR-106b-3p, hsa-miR-335-5p, hsa-miR-30b-5p, hsa-miR-664a-3p, hsa-miR-31-5p, hsa-miR-941, hsa-miR-140-3p, hsa-miR-30e-5p, hsa-let-7i-5p, hsa-miR-1296-5p, hsa-miR-1249-3p, hsa-miR-100-5p, hsa-miR-20b-5p, hsa-miR-1301-3p, hsa-miR-190a-5p, hsa-miR-324-3p, hsa-miR-143-3p, hsa-miR-421 | 88.33 | 97.14 | 90 |
| 2489 | hsa-miR-132-3p, hsa-miR-31-5p, hsa-miR-941, hsa-miR-32-3p, hsa-miR-140-3p, hsa-miR-30e-5p, hsa-miR-1296-5p, hsa-miR-323b-3p, hsa-miR-452-3p, hsa-miR-190a-5p, hsa-miR-215-5p, hsa-miR-143-3p, hsa-miR-421, hsa-miR-22-5p, hsa-miR-4306 | 88.33 | 94.29 | 90 |
| 2490 | hsa-miR-132-3p, hsa-miR-30b-5p, hsa-miR-664a-3p, hsa-miR-31-5p, hsa-miR-32-3p, hsa-miR-140-3p, hsa-miR-30e-5p, hsa-miR-1296-5p, hsa-miR-323b-3p, hsa-miR-20b-5p, hsa-miR-190a-5p, hsa-miR-324-3p, hsa-miR-143-3p, hsa-miR-421, hsa-miR-22-5p, hsa-miR-4306 | 88.33 | 95.71 | 100 |
| 2491 | hsa-miR-132-3p, hsa-miR-30b-5p, hsa-miR-664a-3p, hsa-miR-31-5p, hsa-miR-32-3p, hsa-miR-140-3p, hsa-miR-30e-5p, hsa-miR-1296-5p, hsa-miR-1249-3p, hsa-miR-323b-3p, hsa-miR-190a-5p, hsa-miR-324-3p, hsa-miR-143-3p, hsa-miR-421, hsa-miR-22-5p, hsa-miR-4306 | 88.33 | 91.43 | 90 |
| 2492 | hsa-miR-503-5p, hsa-miR-1249-3p, hsa-miR-100-5p, hsa-miR-181c-5p, hsa-miR-323b-3p, hsa-miR-452-3p, hsa-miR-1301-3p, hsa-miR-215-5p, hsa-miR-324-3p, hsa-miR-143-3p, hsa-miR-421 | 88.33 | 95.71 | 70 |
| 2493 | hsa-miR-132-3p, hsa-miR-106b-3p, hsa-miR-335-5p, hsa-miR-30b-5p, hsa-miR-664a-3p, hsa-miR-31-5p, hsa-miR-140-3p, hsa-miR-30e-5p, hsa-let-7i-5p, hsa-miR-1249-3p, hsa-miR-323b-3p, hsa-miR-452-3p, hsa-miR-1301-3p, hsa-miR-190a-5p, hsa-miR-324-3p, hsa-miR-421, hsa-miR-4306 | 88.33 | 94.29 | 90 |
| 2494 | hsa-miR-335-5p, hsa-miR-30b-5p, hsa-miR-664a-3p, hsa-miR-140-3p, hsa-miR-503-5p, hsa-miR-1296-5p, hsa-miR-181c-5p, hsa-miR-20b-5p, hsa-miR-1301-3p, hsa-miR-143-3p, hsa-miR-22-5p, hsa-miR-4306 | 88.33 | 88.57 | 50 |
| 2495 | hsa-miR-106b-3p, hsa-miR-335-5p, hsa-miR-30b-5p, hsa-miR-664a-3p, hsa-let-7i-5p, hsa-miR-100-5p, hsa-miR-181c-5p, hsa-miR-323b-3p, hsa-miR-1301-3p, hsa-miR-215-5p, hsa-miR-143-3p, hsa-miR-421, hsa-miR-4306 | 88.33 | 90 | 80 |
| 2496 | hsa-miR-132-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-30e-5p, hsa-miR-1296-5p, hsa-miR-100-5p, hsa-miR-323b-3p, hsa-miR-452-3p, hsa-miR-1301-3p, hsa-miR-190a-5p, hsa-miR-215-5p, hsa-miR-324-3p, hsa-miR-143-3p, hsa-miR-421, hsa-miR-22-5p | 88.06 | 95.71 | 100 |
| 2497 | hsa-miR-31-5p, hsa-miR-140-3p, hsa-miR-30e-5p, hsa-let-7i-5p, hsa-miR-1296-5p, hsa-miR-323b-3p, hsa-miR-452-3p, hsa-miR-143-3p, hsa-miR-421, hsa-miR-22-5p, hsa-miR-4306 | 88.06 | 92.86 | 70 |
| 2498 | hsa-miR-132-3p, hsa-miR-30b-5p, hsa-miR-32-3p, hsa-miR-503-5p, hsa-miR-30e-5p, hsa-let-7i-5p, hsa-miR-1296-5p, hsa-miR-1249-3p, hsa-miR-452-3p, hsa-miR-20b-5p, hsa-miR-22-5p | 88.06 | 92.86 | 70 |
| 2499 | hsa-miR-132-3p, hsa-miR-30b-5p, hsa-miR-31-5p, hsa-miR-32-3p, hsa-miR-140-3p, hsa-miR-503-5p, hsa-miR-1296-5p, hsa-miR-181c-5p, hsa-miR-323b-3p, hsa-miR-452-3p, hsa-miR-190a-5p, hsa-miR-324-3p, hsa-miR-421, hsa-miR-22-5p, hsa-miR-4306 | 88.06 | 97.14 | 100 |
| 2500 | hsa-miR-132-3p, hsa-miR-106b-3p, hsa-miR-664a-3p, hsa-miR-941, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p, hsa-miR-421, hsa-miR-22-5p, hsa-miR-4306 | 86.67 | 95.71 | 90 |
| 2501 | hsa-miR-132-3p, hsa-miR-106b-3p, hsa-miR-664a-3p, hsa-miR-941, hsa-miR-30e-5p, hsa-let-7i-5p, hsa-miR-1249-3p, hsa-miR-100-5p, hsa-miR-323b-3p, hsa-miR-1301-3p, hsa-miR-215-5p, hsa-miR-324-3p, hsa-miR-421, hsa-miR-22-5p, hsa-miR-4306 | 86.67 | 94.29 | 80 |
| 2502 | hsa-miR-132-3p, hsa-miR-106b-3p, hsa-miR-941, hsa-miR-503-5p, hsa-miR-30e-5p, hsa-let-7i-5p, hsa-miR-1249-3p, hsa-miR-181c-5p, hsa-miR-452-3p, hsa-miR-20b-5p, hsa-miR-1301-3p, hsa-miR-215-5p, hsa-miR-324-3p, hsa-miR-22-5p, hsa-miR-4306 | 86.67 | 92.86 | 90 |
| 2503 | hsa-miR-132-3p, hsa-miR-106b-3p, hsa-miR-664a-3p, hsa-miR-30e-5p, hsa-let-7i-5p, hsa-miR-100-5p, hsa-miR-323b-3p, hsa-miR-1301-3p, hsa-miR-215-5p, hsa-miR-324-3p, hsa-miR-143-3p, hsa-miR-421, hsa-miR-22-5p | 86.67 | 95.71 | 80 |
| 2504 | hsa-miR-132-3p, hsa-miR-106b-3p, hsa-miR-664a-3p, hsa-miR-30e-5p, hsa-let-7i-5p, hsa-miR-1296-5p, hsa-miR-100-5p, hsa-miR-323b-3p, hsa-miR-1301-3p, hsa-miR-215-5p, hsa-miR-324-3p, hsa-miR-143-3p, hsa-miR-421, hsa-miR-22-5p, hsa-miR-4306 | 86.67 | 92.86 | 90 |
| 2505 | hsa-miR-132-3p, hsa-miR-503-5p, hsa-miR-30e-5p, hsa-let-7i-5p, hsa-miR-1249-3p, hsa-miR-452-3p, hsa-miR-421, hsa-miR-22-5p | 86.39 | 91.43 | 70 |
| 2506 | hsa-miR-106b-3p, hsa-miR-335-5p, hsa-miR-664a-3p, hsa-miR-30e-5p, hsa-let-7i-5p, hsa-miR-1249-3p, hsa-miR-100-5p, hsa-miR-1301-3p, hsa-miR-215-5p, hsa-miR-324-3p, hsa-miR-143-3p, hsa-miR-421 | 86.39 | 97.14 | 80 |
| 2507 | hsa-miR-106b-3p, hsa-miR-335-5p, hsa-miR-664a-3p, hsa-miR-30e-5p, hsa-let-7i-5p, hsa-miR-100-5p, hsa-miR-1301-3p, hsa-miR-215-5p, hsa-miR-324-3p, hsa-miR-143-3p, hsa-miR-421 | 86.39 | 95.71 | 80 |
| 2508 | hsa-miR-132-3p, hsa-miR-106b-3p, hsa-miR-32-3p, hsa-miR-140-3p, hsa-miR-503-5p, hsa-miR-30e-5p, hsa-miR-1296-5p, hsa-miR-323b-3p, hsa-miR-452-3p, hsa-miR-215-5p, hsa-miR-421, hsa-miR-22-5p | 86.39 | 75.71 | 50 |
| 2509 | hsa-miR-132-3p, hsa-miR-30b-5p, hsa-miR-31-5p, hsa-miR-32-3p, hsa-miR-140-3p, hsa-miR-1296-5p, hsa-miR-181c-5p, hsa-miR-323b-3p, hsa-miR-452-3p, hsa-miR-190a-5p, hsa-miR-324-3p, hsa-miR-421, hsa-miR-22-5p, hsa-miR-4306 | 86.11 | 92.86 | 90 |
| 2510 | hsa-miR-132-3p, hsa-miR-30b-5p, hsa-miR-664a-3p, hsa-miR-31-5p, hsa-miR-32-3p, hsa-miR-140-3p, hsa-miR-30e-5p, hsa-miR-1296-5p, hsa-miR-323b-3p, hsa-miR-1301-3p, hsa-miR-190a-5p, hsa-miR-215-5p, hsa-miR-324-3p, hsa-miR-143-3p, hsa-miR-421, hsa-miR-4306 | 86.11 | 97.14 | 100 |
| 2511 | hsa-miR-106b-3p, hsa-miR-335-5p, hsa-miR-664a-3p, hsa-let-7i-5p, hsa-miR-100-5p, hsa-miR-181c-5p, hsa-miR-323b-3p, hsa-miR-1301-3p, hsa-miR-215-5p, hsa-miR-324-3p, hsa-miR-143-3p, hsa-miR-421 | 86.11 | 98.57 | 80 |
| 2512 | hsa-miR-132-3p, hsa-miR-30b-5p, hsa-miR-664a-3p, hsa-miR-32-3p, hsa-miR-140-3p, hsa-miR-503-5p, hsa-miR-30e-5p, hsa-miR-1296-5p, hsa-miR-324-3p, hsa-miR-22-5p | 86.11 | 88.57 | 60 |
| 2513 | hsa-miR-106b-3p, hsa-miR-335-5p, hsa-miR-30b-5p, hsa-miR-664a-3p, hsa-let-7i-5p, hsa-miR-100-5p, hsa-miR-181c-5p, hsa-miR-323b-3p, hsa-miR-1301-3p, hsa-miR-215-5p, hsa-miR-324-3p, hsa-miR-143-3p, hsa-miR-421 | 86.11 | 100 | 80 |
| 2514 | hsa-miR-132-3p, hsa-miR-30b-5p, hsa-miR-31-5p, hsa-miR-32-3p, hsa-miR-140-3p, hsa-miR-1296-5p, hsa-miR-181c-5p, hsa-miR-323b-3p, hsa-miR-452-3p, hsa-miR-190a-5p, hsa-miR-324-3p, hsa-miR-143-3p, hsa-miR-421, hsa-miR-22-5p, hsa-miR-4306 | 86.11 | 88.57 | 90 |
| 2515 | hsa-miR-106b-3p, hsa-miR-335-5p, hsa-miR-30b-5p, hsa-miR-664a-3p, hsa-miR-1296-5p, hsa-miR-100-5p, hsa-miR-181c-5p, hsa-miR-323b-3p, hsa-miR-1301-3p, hsa-miR-215-5p, hsa-miR-143-3p | 86.11 | 94.29 | 50 |
| 2516 | hsa-miR-335-5p, hsa-miR-30b-5p, hsa-miR-664a-3p, hsa-miR-31-5p, hsa-let-7i-5p, hsa-miR-1249-3p, hsa-miR-181c-5p, hsa-miR-452-3p, hsa-miR-190a-5p, hsa-miR-22-5p | 86.11 | 92.86 | 60 |
| 2517 | hsa-miR-132-3p, hsa-miR-335-5p, hsa-miR-30b-5p, hsa-miR-503-5p, hsa-miR-100-5p, hsa-miR-323b-3p, hsa-miR-215-5p, hsa-miR-324-3p, hsa-miR-421 | 86.11 | 95.71 | 100 |
| 2518 | hsa-miR-132-3p, hsa-miR-335-5p, hsa-miR-30b-5p, hsa-miR-503-5p, hsa-let-7i-5p, hsa-miR-1249-3p, hsa-miR-100-5p, hsa-miR-323b-3p, hsa-miR-1301-3p, hsa-miR-215-5p, hsa-miR-324-3p, hsa-miR-421, hsa-miR-22-5p | 86.11 | 98.57 | 60 |
| 2519 | hsa-miR-106b-3p, hsa-miR-335-5p, hsa-miR-30b-5p, hsa-miR-664a-3p, hsa-miR-941, hsa-let-7i-5p, hsa-miR-100-5p, hsa-miR-181c-5p, hsa-miR-323b-3p, hsa-miR-1301-3p, hsa-miR-215-5p, hsa-miR-324-3p, hsa-miR-143-3p, hsa-miR-421 | 86.11 | 95.71 | 80 |
| 2520 | hsa-miR-664a-3p, hsa-miR-31-5p, hsa-miR-941, hsa-miR-32-3p, hsa-miR-140-3p, hsa-miR-503-5p, hsa-miR-30e-5p, hsa-miR-181c-5p, hsa-miR-452-3p, hsa-miR-20b-5p, hsa-miR-1301-3p, hsa-miR-215-5p, hsa-miR-324-3p, hsa-miR-143-3p, hsa-miR-22-5p, hsa-miR-4306 | 85.83 | 90 | 80 |
| 2521 | hsa-miR-132-3p, hsa-miR-30b-5p, hsa-miR-31-5p, hsa-miR-32-3p, hsa-miR-140-3p, hsa-miR-181c-5p, hsa-miR-323b-3p, hsa-miR-452-3p, hsa-miR-190a-5p, hsa-miR-215-5p, hsa-miR-324-3p, hsa-miR-143-3p, hsa-miR-421, hsa-miR-22-5p, hsa-miR-4306 | 85.83 | 91.43 | 100 |
| 2522 | hsa-miR-132-3p, hsa-miR-335-5p, hsa-miR-30b-5p, hsa-miR-31-5p, hsa-miR-32-3p, hsa-miR-140-3p, hsa-miR-30e-5p, hsa-miR-100-5p, hsa-miR-323b-3p, hsa-miR-452-3p, hsa-miR-1301-3p, hsa-miR-190a-5p, hsa-miR-215-5p, hsa-miR-324-3p, hsa-miR-143-3p, hsa-miR-421, hsa-miR-22-5p | 85.83 | 94.29 | 100 |
| 2523 | hsa-miR-106b-3p, hsa-miR-335-5p, hsa-miR-30b-5p, hsa-miR-664a-3p, hsa-miR-31-5p, hsa-miR-941, hsa-miR-32-3p, hsa-miR-140-3p, hsa-miR-30e-5p, hsa-let-7i-5p, hsa-miR-1296-5p, hsa-miR-1249-3p, hsa-miR-100-5p, hsa-miR-20b-5p, hsa-miR-1301-3p, hsa-miR-190a-5p, hsa-miR-215-5p, hsa-miR-324-3p, hsa-miR-143-3p, hsa-miR-421, hsa-miR-4306 | 85.83 | 94.29 | 100 |
| 2524 | hsa-miR-106b-3p, hsa-miR-335-5p, hsa-miR-664a-3p, hsa-miR-31-5p, hsa-miR-140-3p, hsa-miR-30e-5p, hsa-let-7i-5p, hsa-miR-100-5p, hsa-miR-323b-3p, hsa-miR-1301-3p, hsa-miR-190a-5p, hsa-miR-215-5p, hsa-miR-324-3p, hsa-miR-143-3p, hsa-miR-421, hsa-miR-4306 | 85.83 | 100 | 100 |
| 2525 | hsa-miR-106b-3p, hsa-miR-941, hsa-miR-140-3p, hsa-miR-503-5p, hsa-let-7i-5p, hsa-miR-1296-5p, hsa-miR-1249-3p, hsa-miR-181c-5p, hsa-miR-323b-3p, hsa-miR-20b-5p, hsa-miR-1301-3p, hsa-miR-190a-5p, hsa-miR-215-5p, hsa-miR-143-3p, hsa-miR-421 | 85.83 | 90 | 70 |
| 2526 | hsa-miR-132-3p, hsa-miR-106b-3p, hsa-miR-664a-3p, hsa-miR-30e-5p, hsa-let-7i-5p, hsa-miR-100-5p, hsa-miR-323b-3p, hsa-miR-1301-3p, hsa-miR-324-3p, hsa-miR-143-3p, hsa-miR-421, hsa-miR-22-5p, hsa-miR-4306 | 84.44 | 91.43 | 80 |
| 2527 | hsa-miR-335-5p, hsa-miR-30b-5p, hsa-miR-664a-3p, hsa-miR-503-5p, hsa-miR-1249-3p, hsa-miR-100-5p, hsa-miR-181c-5p, hsa-miR-323b-3p, hsa-miR-20b-5p, hsa-miR-1301-3p, hsa-miR-324-3p, hsa-miR-143-3p, hsa-miR-421, hsa-miR-22-5p | 84.17 | 91.43 | 70 |
| 2528 | hsa-miR-106b-3p, hsa-miR-335-5p, hsa-miR-30b-5p, hsa-miR-664a-3p, hsa-miR-32-3p, hsa-miR-30e-5p, hsa-miR-1296-5p, hsa-miR-1249-3p, hsa-miR-100-5p, hsa-miR-20b-5p, hsa-miR-1301-3p, hsa-miR-215-5p, hsa-miR-324-3p, hsa-miR-143-3p, hsa-miR-421 | 84.17 | 91.43 | 80 |
| 2529 | hsa-miR-132-3p, hsa-miR-503-5p, hsa-miR-30e-5p, hsa-let-7i-5p, hsa-miR-452-3p, hsa-miR-421, hsa-miR-22-5p | 84.17 | 94.29 | 70 |
| 2530 | hsa-miR-132-3p, hsa-miR-106b-3p, hsa-miR-664a-3p, hsa-miR-31-5p, hsa-miR-30e-5p, hsa-let-7i-5p, hsa-miR-100-5p, hsa-miR-323b-3p, hsa-miR-1301-3p, hsa-miR-190a-5p, hsa-miR-215-5p, hsa-miR-324-3p, hsa-miR-143-3p, hsa-miR-421, hsa-miR-22-5p, hsa-miR-4306 | 83.89 | 94.29 | 100 |
| 2531 | hsa-miR-106b-3p, hsa-miR-335-5p, hsa-miR-30b-5p, hsa-miR-664a-3p, hsa-miR-941, hsa-let-7i-5p, hsa-miR-100-5p, hsa-miR-323b-3p, hsa-miR-1301-3p, hsa-miR-215-5p, hsa-miR-324-3p, hsa-miR-143-3p, hsa-miR-421 | 83.89 | 97.14 | 80 |
| 2532 | hsa-miR-335-5p, hsa-miR-941, hsa-miR-503-5p, hsa-miR-30e-5p, hsa-let-7i-5p, hsa-miR-1249-3p, hsa-miR-452-3p, hsa-miR-215-5p, hsa-miR-421 | 83.89 | 92.86 | 70 |
| 2533 | hsa-miR-132-3p, hsa-miR-664a-3p, hsa-miR-503-5p, hsa-let-7i-5p, hsa-miR-1296-5p, hsa-miR-1249-3p, hsa-miR-181c-5p, hsa-miR-323b-3p, hsa-miR-452-3p, hsa-miR-20b-5p, hsa-miR-421, hsa-miR-22-5p, hsa-miR-4306 | 83.89 | 88.57 | 60 |
| 2534 | hsa-miR-106b-3p, hsa-miR-335-5p, hsa-miR-30b-5p, hsa-miR-664a-3p, hsa-miR-31-5p, hsa-miR-32-3p, hsa-miR-140-3p, hsa-let-7i-5p, hsa-miR-1296-5p, hsa-miR-1249-3p, hsa-miR-100-5p, hsa-miR-181c-5p, hsa-miR-20b-5p, hsa-miR-1301-3p, hsa-miR-190a-5p, hsa-miR-143-3p, hsa-miR-421 | 83.61 | 88.57 | 80 |
| 2535 | hsa-miR-132-3p, hsa-miR-106b-3p, hsa-miR-30b-5p, hsa-miR-31-5p, hsa-miR-941, hsa-miR-32-3p, hsa-miR-140-3p, hsa-miR-1249-3p, hsa-miR-181c-5p, hsa-miR-323b-3p, hsa-miR-452-3p, hsa-miR-190a-5p, hsa-miR-215-5p, hsa-miR-324-3p, hsa-miR-143-3p, hsa-miR-22-5p, hsa-miR-4306 | 83.61 | 88.57 | 100 |
| 2536 | hsa-miR-132-3p, hsa-miR-30b-5p, hsa-miR-664a-3p, hsa-miR-31-5p, hsa-miR-941, hsa-miR-32-3p, hsa-miR-140-3p, hsa-miR-100-5p, hsa-miR-181c-5p, hsa-miR-1301-3p, hsa-miR-324-3p, hsa-miR-143-3p | 83.61 | 90 | 60 |
| 2537 | hsa-miR-106b-3p, hsa-miR-335-5p, hsa-miR-30b-5p, hsa-miR-664a-3p, hsa-miR-31-5p, hsa-miR-32-3p, hsa-miR-140-3p, hsa-miR-30e-5p, hsa-let-7i-5p, hsa-miR-1296-5p, hsa-miR-1249-3p, hsa-miR-100-5p, hsa-miR-20b-5p, hsa-miR-1301-3p, hsa-miR-190a-5p, hsa-miR-215-5p, hsa-miR-324-3p, hsa-miR-143-3p, hsa-miR-421, hsa-miR-4306 | 81.67 | 92.86 | 90 |
| 2538 | hsa-miR-132-3p, hsa-miR-106b-3p, hsa-miR-503-5p, hsa-miR-30e-5p, hsa-let-7i-5p, hsa-miR-1296-5p, hsa-miR-1249-3p, hsa-miR-323b-3p | 81.67 | 82.86 | 40 |
| 2539 | hsa-miR-132-3p, hsa-miR-335-5p, hsa-miR-30b-5p, hsa-miR-664a-3p, hsa-miR-941, hsa-miR-32-3p, hsa-miR-140-3p, hsa-miR-1296-5p, hsa-miR-1249-3p, hsa-miR-100-5p, hsa-miR-452-3p, hsa-miR-20b-5p, hsa-miR-1301-3p, hsa-miR-324-3p, hsa-miR-143-3p, hsa-miR-421, hsa-miR-22-5p, hsa-miR-4306 | 81.67 | 90 | 90 |
| 2540 | hsa-miR-106b-3p, hsa-miR-335-5p, hsa-miR-30b-5p, hsa-miR-664a-3p, hsa-miR-32-3p, hsa-miR-503-5p, hsa-miR-1296-5p, hsa-miR-100-5p, hsa-miR-181c-5p, hsa-miR-323b-3p, hsa-miR-215-5p, hsa-miR-143-3p | 81.39 | 91.43 | 50 |
| 2541 | hsa-miR-132-3p, hsa-miR-30b-5p, hsa-miR-31-5p, hsa-miR-941, hsa-miR-32-3p, hsa-miR-140-3p, hsa-miR-503-5p, hsa-miR-30e-5p, hsa-miR-1296-5p, hsa-miR-1301-3p, hsa-miR-190a-5p, hsa-miR-215-5p, hsa-miR-324-3p, hsa-miR-143-3p, hsa-miR-22-5p | 81.11 | 94.29 | 70 |
| 2542 | hsa-miR-132-3p, hsa-miR-106b-3p, hsa-miR-30b-5p, hsa-miR-31-5p, hsa-miR-32-3p, hsa-miR-503-5p, hsa-miR-1249-3p, hsa-miR-190a-5p, hsa-miR-324-3p, hsa-miR-421 | 81.11 | 87.14 | 80 |
| 2543 | hsa-miR-132-3p, hsa-miR-31-5p, hsa-miR-941, hsa-miR-32-3p, hsa-miR-503-5p, hsa-miR-30e-5p, hsa-let-7i-5p, hsa-miR-1296-5p, hsa-miR-323b-3p, hsa-miR-452-3p, hsa-miR-324-3p, hsa-miR-421, hsa-miR-22-5p, hsa-miR-4306 | 79.44 | 94.29 | 70 |
| 2544 | hsa-miR-132-3p, hsa-miR-106b-3p, hsa-miR-664a-3p, hsa-miR-30e-5p, hsa-let-7i-5p, hsa-miR-1249-3p, hsa-miR-100-5p, hsa-miR-181c-5p, hsa-miR-1301-3p, hsa-miR-215-5p, hsa-miR-143-3p, hsa-miR-421, hsa-miR-22-5p, hsa-miR-4306 | 76.94 | 85.71 | 70 |
| 2545 | combination 2272 + rs12917707 | 84.17 | 80 | 53.33 |
| 2546 | combination 2274 + rs9928757 | 84.44 | 90 | 63.33 |
| 2547 | combination 2275 + rs13335818 | 91.11 | 95.71 | 71.67 |
| 2548 | combination 2276 + rs13335818 | 88.61 | 94.29 | 78.33 |
| 2549 | combination 2277 + rs76563024 | 88.89 | 95.71 | 81.67 |
| 2550 | combination 2278 + rs4297685 | 86.67 | 94.29 | 93.33 |
| 2551 | combination 2279 + rs9928757 | 88.89 | 92.86 | 91.39 |
| 2552 | combination 2280 + rs71384446 | 91.11 | 97.14 | 96.39 |
| 2553 | combination 2281 + rs13333226 | 93.33 | 98.57 | 96.11 |
| 2554 | combination 2282 + rs7204342 | 93.33 | 100 | 100 |
| 2555 | combination 2283 + rs11862974 | 90.83 | 95.71 | 99.17 |
| 2556 | combination 2284 + rs13335818 | 93.33 | 98.57 | 98.06 |
| 2557 | combination 2285 + rs13335818 | 93.33 | 98.57 | 100.0 |
| 2558 | combination 2286 + rs13333226 | 88.89 | 100 | 100 |
| 2559 | combination 2287 + rs13333226 | 90.83 | 95.71 | 93.33 |
| 2560 | combination 2288 + rs13335818 | 93.06 | 95.71 | 91.39 |
| 2561 | combination 2289 + rs4297685 | 88.33 | 94.29 | 96.39 |
| 2562 | combination 2290 + rs76563024 | 88.33 | 94.29 | 96.11 |
| 2563 | combination 2291 + rs71384446 | 86.11 | 94.29 | 99.17 |
| 2564 | combination 2292 + rs71384446 | 86.11 | 94.29 | 98.06 |
| 2565 | combination 2293 + rs71384446 | 95 | 94.29 | 100.0 |
| 2566 | combination 2294 + rs11647727 | 95 | 94.29 | 98.89 |
| 2567 | combination 2295 + rs11647727 | 92.78 | 92.86 | 97.78 |
| 2568 | combination 2296 + rs11647727 | 92.78 | 92.86 | 98.89 |
| 2569 | combination 2297 + rs13335818 | 87.78 | 90 | 98.89 |
| 2570 | combination 2528 + rs12934455 | 85.28 | 90 | 98.89 |
| 2571 | combination 2529 + rs12934455 | 85.28 | 90 | 98.89 |
| 2572 | combination 2274 + rs4293393 | 88.61 | 91.43 | 97.78 |
| 2573 | combination 2275 + rs4293393 | 91.11 | 95.71 | 98.89 |
| 2574 | combination 2277 + rs4293393 | 88.89 | 94.29 | 98.89 |
| 2575 | combination 2278 + rs4293393 | 91.11 | 94.29 | 100.0 |
| 2576 | combination 2279 + rs4293393 | 86.67 | 90 | 100.0 |
| 2577 | combination 2280 + rs4293393 | 88.61 | 88.57 | 99.17 |
| 2578 | combination 2282 + rs4293393 | 90.83 | 94.29 | 99.17 |
| 2579 | combination 2283 + rs4293393 | 92.78 | 94.29 | 99.17 |
| 2580 | combination 2287 + rs4293393 | 90.56 | 95.71 | 99.17 |
| 2581 | combination 2288 + rs4293393 | 90.56 | 92.86 | 97.22 |
| 2582 | combination 2289 + rs4293393 | 90.56 | 95.71 | 97.22 |
| 2583 | combination 2290 + rs4293393 | 90.56 | 92.86 | 91.39 |
| 2584 | combination 2292 + rs4293393 | 95.28 | 97.14 | 96.39 |
| 2585 | combination 2293 + rs4293393 | 92.78 | 95.71 | 96.11 |
| 2586 | combination 2294 + rs4293393 | 90.56 | 95.71 | 99.17 |
| 2587 | combination 2295 + rs4293393 | 90.56 | 94.29 | 98.06 |
| 2588 | combination 2296 + rs4293393 | 88.06 | 91.43 | 93.33 |
| 2589 | combination 2297 + rs4293393 | 88.06 | 91.43 | 91.39 |

This is the list of the best 30 mRNA selected by the algorithm sorted by importance for tumour grade: hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-29b-3p, hsa-miR-590-5p, hsa-miR-340-5p, hsa-miR-15a-5p, hsa-miR-505-5p, hsa-miR-433-3p, hsa-miR-29c-5p, and hsa-miR-15a-3p. The inventors combined these miRNAs, together or not with age and UMOD SNP variant, creating unique combinations leading to a further significant increase in sensitivity, specificity, accuracy and predictive power for tumour grade (Table 27).

**Table 27. Combinations that allow higher sensitivity, specificity, accuracy and predictive power for tumour grade.**

| N | Combinations | Accuracy | ROC AUC | Recall |
|---|---|---|---|---|
| 2590 | hsa-miR-4306 | 76.67 | 74.76 | 58.57 |
| 2591 | hsa-miR-4306, hsa-miR-132-3p | 70 | 63.33 | 76.40 |
| 2592 | hsa-miR-4306, hsa-miR-132-3p, hsa-miR-125b-5p | 79.17 | 80.79 | 85.51 |
| 2593 | hsa-miR-4306, hsa-miR-132-3p, hsa-miR-125b-5p, hsa-miR-148a-3p | 86.11 | 88.65 | 87.24 |
| 2594 | hsa-miR-4306, hsa-miR-132-3p, hsa-miR-125b-5p, hsa-miR-148a-3p, hsa-miR-629-5p | 83.89 | 84.92 | 89.21 |
| 2595 | hsa-miR-4306, hsa-miR-132-3p, hsa-miR-125b-5p, hsa-miR-148a-3p, hsa-miR-629-5p, hsa-miR-545-5p | 93.06 | 96.9 | 89.43 |
| 2596 | hsa-miR-4306, hsa-miR-132-3p, hsa-miR-125b-5p, hsa-miR-148a-3p, hsa-miR-629-5p, hsa-miR-545-5p, hsa-miR-1296-5p | 90.56 | 96.9 | 88.79 |
| 2597 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p | 90.56 | 93.81 | 83.31 |
| 2598 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p | 90.83 | 95.24 | 89.77 |
| 2599 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p | 90.56 | 100 | 91.19 |
| 2600 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p | 93.06 | 98.33 | 92.39 |
| 2601 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p | 90.83 | 98.57 | 92.61 |
| 2602 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p | 86.39 | 95.24 | 95.90 |
| 2603 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p | 85.83 | 95 | 95.84 |
| 2604 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p | 86.39 | 93.81 | 97.10 |
| 2605 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p | 90.56 | 95.24 | 96.40 |
| 2606 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p | 92.78 | 95.24 | 99.11 |
| 2607 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p | 90.56 | 98.57 | 99.11 |
| 2608 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p | 90.83 | 93.81 | 99.14 |
| 2609 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p | 88.33 | 92.62 | 98.52 |
| 2610 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p | 93.06 | 94.29 | 98.83 |
| 2611 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p | 81.11 | 92.38 | 100.00 |
| 2612 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-29b-3p | 81.39 | 93.81 | 100.00 |
| 2613 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-29b-3p, hsa-miR-590-5p | 81.39 | 92.14 | 100.00 |
| 2614 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-29b-3p, hsa-miR-590-5p, hsa-miR-340-5p | 88.33 | 90.48 | 98.77 |
| 2615 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-29b-3p, hsa-miR-590-5p, hsa-miR-340-5p, hsa-miR-15a-5p | 88.33 | 90.48 | 97.85 |
| 2616 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-29b-3p, hsa-miR-590-5p, hsa-miR-340-5p, hsa-miR-15a-5p, hsa-miR-505-5p | 83.89 | 90.71 | 97.23 |
| 2617 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-29b-3p, hsa-miR-590-5p, hsa-miR-340-5p, hsa-miR-15a-5p, hsa-miR-505-5p, hsa-miR-433-3p | 83.61 | 86.19 | 97.57 |
| 2618 | hsa-miR-4306, hsa-miR-132-3p, hsa-miR-125b-5p, hsa-miR-148a-3p, hsa-miR-629-5p, hsa-miR-545-5p, hsa-miR-1296-5p, hsa-miR-103a-2-5p, hsa-let-7i-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-29b-3p, hsa-miR-590-5p, hsa-miR-340-5p, hsa-miR-15a-5p, hsa-miR-505-5p, hsa-miR-433-3p, hsa-miR-29c-5p | 83.89 | 87.62 | 97.26 |
| 2619 | hsa-miR-128-3p, hsa-miR-424-5p, hsa-miR-32-3p, hsa-miR-202-5p, hsa-miR-744-5p, hsa-miR-1537-3p, hsa-miR-590-5p, hsa-miR-15a-5p, hsa-miR-433-3p, hsa-miR-15a-3p, hsa-let-7i-5p | 97.78 | 98.57 | 100.00 |
| 2620 | hsa-miR-128-3p, hsa-miR-424-5p, hsa-miR-32-3p, hsa-miR-584-5p, hsa-miR-1537-3p, hsa-miR-590-5p, hsa-miR-340-5p, hsa-miR-15a-5p, hsa-miR-433-3p, hsa-miR-15a-3p, hsa-miR-148a-3p, hsa-let-7i-5p, hsa-miR-652-5p | 97.78 | 97.14 | 90.00 |
| 2621 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-15a-5p, hsa-miR-433-3p, hsa-miR-15a-3p, hsa-miR-1296-5p, hsa-let-7i-5p | 97.78 | 100.00 | 90.00 |
| 2622 | hsa-miR-128-3p, hsa-miR-424-5p, hsa-miR-32-3p, hsa-miR-202-5p, hsa-miR-744-5p, hsa-miR-1537-3p, hsa-miR-15a-5p, hsa-miR-433-3p, hsa-miR-15a-3p, hsa-let-7i-5p | 97.78 | 98.57 | 100.00 |
| 2623 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-15a-5p, hsa-miR-433-3p, hsa-miR-15a-3p, hsa-let-7i-5p | 97.78 | 100.00 | 90.00 |
| 2624 | hsa-miR-128-3p, hsa-miR-424-5p, hsa-miR-202-5p, hsa-miR-584-5p, hsa-miR-1537-3p, hsa-miR-590-5p, hsa-miR-340-5p, hsa-miR-433-3p, hsa-miR-29c-5p, hsa-miR-15a-3p, hsa-miR-148a-3p, hsa-miR-545-5p, hsa-let-7i-5p, hsa-miR-652-5p | 95.56 | 97.46 | 90.00 |
| 2625 | hsa-miR-128-3p, hsa-miR-424-5p, hsa-miR-202-5p, hsa-miR-584-5p, hsa-miR-1537-3p, hsa-miR-590-5p, hsa-miR-340-5p, hsa-miR-15a-5p, hsa-miR-433-3p, hsa-miR-15a-3p, hsa-miR-148a-3p, hsa-let-7i-5p, hsa-miR-652-5p | 95.56 | 97.46 | 90.00 |
| 2626 | hsa-miR-128-3p, hsa-miR-424-5p, hsa-miR-34a-5p, hsa-miR-202-5p, hsa-miR-584-5p, hsa-miR-1537-3p, hsa-miR-590-5p, hsa-miR-340-5p, hsa-miR-433-3p, hsa-miR-29c-5p, hsa-miR-15a-3p, hsa-miR-148a-3p, hsa-miR-545-5p, hsa-let-7i-5p, hsa-miR-652-5p | 95.56 | 98.89 | 90.00 |
| 2627 | hsa-miR-128-3p, hsa-miR-424-5p, hsa-miR-202-5p, hsa-miR-584-5p, hsa-miR-1537-3p, hsa-miR-590-5p, hsa-miR-340-5p, hsa-miR-15a-5p, hsa-miR-433-3p, hsa-miR-15a-3p, hsa-miR-148a-3p, hsa-miR-545-5p, hsa-let-7i-5p, hsa-miR-652-5p | 95.56 | 97.46 | 90.00 |
| 2628 | hsa-miR-128-3p, hsa-miR-424-5p, hsa-miR-34a-5p, hsa-miR-584-5p, hsa-miR-1537-3p, hsa-miR-590-5p, hsa-miR-340-5p, hsa-miR-15a-5p, hsa-miR-433-3p, hsa-miR-15a-3p, hsa-miR-148a-3p, hsa-miR-1296-5p, hsa-let-7i-5p, hsa-miR-652-5p | 95.56 | 93.17 | 90.00 |
| 2629 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-433-3p, hsa-miR-15a-3p, hsa-let-7i-5p | 95.56 | 97.46 | 83.33 |
| 2630 | hsa-miR-128-3p, hsa-miR-424-5p, hsa-miR-202-5p, hsa-miR-584-5p, hsa-miR-1537-3p, hsa-miR-590-5p, hsa-miR-340-5p, hsa-miR-15a-5p, hsa-miR-433-3p, hsa-miR-29c-5p, hsa-miR-15a-3p, hsa-miR-148a-3p, hsa-miR-545-5p, hsa-let-7i-5p, hsa-miR-652-5p | 95.56 | 97.46 | 90.00 |
| 2631 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-15a-5p, hsa-miR-433-3p, hsa-miR-15a-3p, hsa-let-7i-5p | 95.56 | 100.00 | 90.00 |
| 2632 | hsa-miR-128-3p, hsa-miR-424-5p, hsa-miR-32-3p, hsa-miR-410-3p, hsa-miR-584-5p, hsa-miR-1537-3p, hsa-miR-590-5p, hsa-miR-340-5p, hsa-miR-15a-5p, hsa-miR-433-3p, hsa-miR-29c-5p, hsa-miR-15a-3p, hsa-miR-148a-3p, hsa-miR-545-5p, hsa-let-7i-5p, hsa-miR-652-5p | 95.56 | 98.57 | 100.00 |
| 2633 | hsa-miR-128-3p, hsa-miR-424-5p, hsa-miR-34a-5p, hsa-miR-202-5p, hsa-miR-1537-3p, hsa-miR-590-5p, hsa-miR-340-5p, hsa-miR-433-3p, hsa-miR-29c-5p, hsa-miR-15a-3p, hsa-miR-148a-3p, hsa-miR-545-5p, hsa-miR-652-5p | 95.56 | 98.57 | 90.00 |
| 2634 | hsa-miR-424-5p, hsa-miR-202-5p, hsa-miR-410-3p, hsa-miR-584-5p, hsa-miR-590-5p, hsa-miR-340-5p, hsa-miR-433-3p, hsa-miR-29c-5p, hsa-miR-15a-3p, hsa-miR-148a-3p, hsa-miR-545-5p, hsa-let-7i-5p, hsa-miR-652-5p | 95.56 | 100.00 | 90.00 |
| 2635 | hsa-miR-424-5p, hsa-miR-32-3p, hsa-miR-202-5p, hsa-miR-584-5p, hsa-miR-744-5p, hsa-miR-1537-3p, hsa-miR-29b-3p, hsa-miR-15a-5p, hsa-miR-15a-3p, hsa-miR-545-5p, hsa-miR-1296-5p, hsa-miR-103a-2-5p, hsa-let-7i-5p | 95.28 | 98.57 | 90.00 |
| 2636 | hsa-miR-369-3p, hsa-miR-128-3p, hsa-miR-584-5p, hsa-miR-1537-3p, hsa-miR-590-5p, hsa-miR-340-5p, hsa-miR-15a-5p, hsa-miR-505-5p, hsa-miR-433-3p, hsa-miR-15a-3p, hsa-miR-148a-3p, hsa-let-7i-5p, hsa-miR-652-5p | 95.28 | 98.57 | 80.00 |
| 2637 | hsa-miR-369-3p, hsa-miR-128-3p, hsa-miR-34a-5p, hsa-miR-584-5p, hsa-miR-1537-3p, hsa-miR-590-5p, hsa-miR-340-5p, hsa-miR-505-5p, hsa-miR-433-3p, hsa-miR-29c-5p, hsa-miR-15a-3p, hsa-miR-148a-3p, hsa-miR-545-5p, hsa-let-7i-5p, hsa-miR-652-5p | 95.28 | 100.00 | 90.00 |
| 2638 | hsa-miR-369-3p, hsa-miR-128-3p, hsa-miR-34a-5p, hsa-miR-1537-3p, hsa-miR-590-5p, hsa-miR-340-5p, hsa-miR-505-5p, hsa-miR-433-3p, hsa-miR-29c-5p, hsa-miR-15a-3p, hsa-miR-148a-3p, hsa-miR-545-5p, hsa-let-7i-5p, hsa-miR-652-5p | 95.28 | 96.67 | 90.00 |
| 2639 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-15a-5p, hsa-miR-433-3p, hsa-miR-15a-3p, hsa-miR-545-5p, hsa-let-7i-5p | 95.28 | 98.57 | 90.00 |
| 2640 | hsa-miR-128-3p, hsa-miR-424-5p, hsa-miR-32-3p, hsa-miR-202-5p, hsa-miR-584-5p, hsa-miR-744-5p, hsa-miR-150-5p, hsa-miR-15a-5p, hsa-miR-433-3p, hsa-miR-629-5p, hsa-miR-545-5p | 95.28 | 98.33 | 100.00 |
| 2641 | hsa-miR-424-5p, hsa-miR-34a-5p, hsa-miR-202-5p, hsa-miR-410-3p, hsa-miR-584-5p, hsa-miR-150-5p, hsa-miR-590-5p, hsa-miR-340-5p, hsa-miR-433-3p, hsa-miR-29c-5p, hsa-miR-148a-3p, hsa-miR-629-5p, hsa-miR-545-5p, hsa-let-7i-5p, hsa-miR-652-5p | 95.00 | 98.33 | 90.00 |
| 2642 | hsa-miR-369-3p, hsa-miR-128-3p, hsa-miR-1537-3p, hsa-miR-590-5p, hsa-miR-340-5p, hsa-miR-15a-5p, hsa-miR-505-5p, hsa-miR-433-3p, hsa-miR-29c-5p, hsa-miR-15a-3p, hsa-miR-148a-3p, hsa-miR-545-5p, hsa-miR-652-5p | 95.00 | 98.33 | 80.00 |
| 2643 | hsa-miR-369-3p, hsa-miR-128-3p, hsa-miR-34a-5p, hsa-miR-202-5p, hsa-miR-584-5p, hsa-miR-1537-3p, hsa-miR-590-5p, hsa-miR-505-5p, hsa-miR-433-3p, hsa-miR-15a-3p, hsa-miR-148a-3p, hsa-let-7i-5p, hsa-miR-652-5p | 93.33 | 96.35 | 93.33 |
| 2644 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-433-3p, hsa-miR-15a-3p, hsa-let-7i-5p | 93.33 | 98.57 | 83.33 |
| 2645 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-15a-5p, hsa-miR-15a-3p, hsa-miR-1296-5p, hsa-let-7i-5p | 93.33 | 92.86 | 100.00 |
| 2646 | hsa-miR-128-3p, hsa-miR-424-5p, hsa-miR-32-3p, hsa-miR-202-5p, hsa-miR-744-5p, hsa-miR-1537-3p, hsa-miR-590-5p, hsa-miR-340-5p, hsa-miR-15a-5p, hsa-miR-433-3p, hsa-miR-15a-3p, hsa-miR-148a-3p | 93.33 | 95.71 | 90.00 |
| 2647 | hsa-miR-424-5p, hsa-miR-34a-5p, hsa-miR-32-3p, hsa-miR-202-5p, hsa-miR-744-5p, hsa-miR-433-3p, hsa-miR-148a-3p, hsa-miR-629-5p | 93.33 | 96.03 | 93.33 |
| 2648 | hsa-miR-128-3p, hsa-miR-424-5p, hsa-miR-202-5p, hsa-miR-584-5p, hsa-miR-664a-3p, hsa-miR-1537-3p, hsa-miR-29b-3p, hsa-miR-590-5p, hsa-miR-340-5p, hsa-miR-29c-5p, hsa-miR-15a-3p, hsa-miR-148a-3p, hsa-miR-545-5p, hsa-miR-1296-5p, hsa-let-7i-5p, hsa-miR-652-5p | 93.33 | 91.43 | 90.00 |
| 2649 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-15a-5p, hsa-miR-433-3p, hsa-miR-15a-3p, hsa-miR-1296-5p, hsa-let-7i-5p | 93.33 | 92.86 | 100.00 |
| 2650 | hsa-miR-128-3p, hsa-miR-424-5p, hsa-miR-34a-5p, hsa-miR-202-5p, hsa-miR-584-5p, hsa-miR-664a-3p, hsa-miR-1537-3p, hsa-miR-590-5p, hsa-miR-340-5p, hsa-miR-433-3p, hsa-miR-29c-5p, hsa-miR-15a-3p, hsa-miR-148a-3p, hsa-miR-545-5p, hsa-miR-1296-5p, hsa-let-7i-5p, hsa-miR-652-5p | 93.33 | 91.43 | 90.00 |
| 2651 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-433-3p, hsa-miR-15a-3p, hsa-miR-1296-5p, hsa-let-7i-5p | 93.33 | 98.57 | 83.33 |
| 2652 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-433-3p, hsa-miR-15a-3p, hsa-miR-148a-3p, hsa-let-7i-5p | 93.33 | 97.14 | 93.33 |
| 2653 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-505-5p, hsa-miR-433-3p, hsa-miR-15a-3p, hsa-let-7i-5p | 93.33 | 97.14 | 83.33 |
| 2654 | hsa-miR-128-3p, hsa-miR-424-5p, hsa-miR-32-3p, hsa-miR-202-5p, hsa-miR-744-5p, hsa-miR-1537-3p, hsa-miR-433-3p, hsa-miR-15a-3p, hsa-let-7i-5p | 93.33 | 98.57 | 83.33 |
| 2655 | hsa-miR-128-3p, hsa-miR-424-5p, hsa-miR-34a-5p, hsa-miR-202-5p, hsa-miR-584-5p, hsa-miR-664a-3p, hsa-miR-1537-3p, hsa-miR-590-5p, hsa-miR-340-5p, hsa-miR-29c-5p, hsa-miR-15a-3p, hsa-miR-148a-3p, hsa-miR-545-5p, hsa-miR-1296-5p, hsa-let-7i-5p, hsa-miR-652-5p | 93.33 | 91.43 | 90.00 |
| 2656 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-590-5p, hsa-miR-433-3p, hsa-miR-15a-3p, hsa-miR-148a-3p | 93.33 | 94.29 | 90.00 |
| 2657 | hsa-miR-128-3p, hsa-miR-32-3p, hsa-miR-202-5p, hsa-miR-584-5p, hsa-miR-744-5p, hsa-miR-1537-3p, hsa-miR-15a-5p, hsa-miR-505-5p, hsa-miR-433-3p, hsa-miR-15a-3p, hsa-let-7i-5p | 93.33 | 97.14 | 90.00 |
| 2658 | hsa-miR-128-3p, hsa-miR-424-5p, hsa-miR-34a-5p, hsa-miR-584-5p, hsa-miR-1537-3p, hsa-miR-150-5p, hsa-miR-590-5p, hsa-miR-340-5p, hsa-miR-433-3p, hsa-miR-29c-5p, hsa-miR-15a-3p, hsa-miR-148a-3p, hsa-miR-629-5p, hsa-miR-545-5p, hsa-miR-652-5p | 93.06 | 93.81 | 100.00 |
| 2659 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-15a-5p, hsa-miR-433-3p, hsa-miR-15a-3p, hsa-miR-545-5p | 93.06 | 98.89 | 83.33 |
| 2660 | hsa-miR-128-3p, hsa-miR-424-5p, hsa-miR-34a-5p, hsa-miR-32-3p, hsa-miR-202-5p, hsa-miR-744-5p, hsa-miR-1537-3p, hsa-miR-433-3p, hsa-miR-15a-3p | 93.06 | 98.57 | 73.33 |
| 2661 | hsa-miR-424-5p, hsa-miR-32-3p, hsa-miR-202-5p, hsa-miR-584-5p, hsa-miR-744-5p, hsa-miR-29b-3p, hsa-miR-15a-5p, hsa-miR-29c-5p, hsa-miR-15a-3p, hsa-miR-1296-5p, hsa-miR-103a-2-5p, hsa-let-7i-5p | 93.06 | 94.05 | 90.00 |
| 2662 | hsa-miR-424-5p, hsa-miR-34a-5p, hsa-miR-202-5p, hsa-miR-410-3p, hsa-miR-584-5p, hsa-miR-590-5p, hsa-miR-340-5p, hsa-miR-433-3p, hsa-miR-29c-5p, hsa-miR-148a-3p, hsa-miR-545-5p, hsa-let-7i-5p, hsa-miR-652-5p | 93.06 | 91.90 | 90.00 |
| 2664 | hsa-miR-128-3p, hsa-miR-424-5p, hsa-miR-34a-5p, hsa-miR-202-5p, hsa-miR-584-5p, hsa-miR-1537-3p, hsa-miR-29b-3p, hsa-miR-590-5p, hsa-miR-340-5p, hsa-miR-29c-5p, hsa-miR-15a-3p, hsa-miR-148a-3p, hsa-miR-545-5p, hsa-miR-103a-2-5p, hsa-let-7i-5p, hsa-miR-652-5p | 93.06 | 100.00 | 80.00 |
| 2665 | hsa-miR-128-3p, hsa-miR-424-5p, hsa-miR-32-3p, hsa-miR-202-5p, hsa-miR-744-5p, hsa-miR-1537-3p, hsa-miR-15a-5p, hsa-miR-433-3p, hsa-miR-15a-3p, hsa-miR-545-5p | 93.06 | 98.89 | 83.33 |
| 2666 | hsa-miR-424-5p, hsa-miR-32-3p, hsa-miR-202-5p, hsa-miR-584-5p, hsa-miR-744-5p, hsa-miR-1537-3p, hsa-miR-15a-5p, hsa-miR-433-3p, hsa-miR-29c-5p, hsa-miR-15a-3p, hsa-let-7i-5p | 93.06 | 100.00 | 100.00 |
| 2667 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-29b-3p, hsa-miR-15a-3p, hsa-miR-1296-5p, hsa-miR-103a-2-5p, hsa-let-7i-5p | 92.78 | 96.90 | 80.00 |
| 2668 | hsa-miR-128-3p, hsa-miR-424-5p, hsa-miR-32-3p, hsa-miR-1537-3p, hsa-miR-590-5p, hsa-miR-340-5p, hsa-miR-433-3p, hsa-miR-15a-3p, hsa-miR-148a-3p, hsa-let-7i-5p, hsa-miR-652-5p | 92.78 | 94.05 | 90.00 |
| 2669 | hsa-miR-369-3p, hsa-miR-128-3p, hsa-miR-1537-3p, hsa-miR-590-5p, hsa-miR-340-5p, hsa-miR-15a-5p, hsa-miR-505-5p, hsa-miR-433-3p, hsa-miR-15a-3p, hsa-miR-148a-3p, hsa-let-7i-5p, hsa-miR-652-5p | 92.78 | 92.38 | 70.00 |
| 2670 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-29b-3p, hsa-miR-15a-5p, hsa-miR-15a-3p, hsa-miR-1296-5p, hsa-miR-103a-2-5p, hsa-let-7i-5p | 92.78 | 93.57 | 80.00 |
| 2671 | hsa-miR-424-5p, hsa-miR-34a-5p, hsa-miR-202-5p, hsa-miR-410-3p, hsa-miR-584-5p, hsa-miR-150-5p, hsa-miR-590-5p, hsa-miR-340-5p, hsa-miR-433-3p, hsa-miR-29c-5p, hsa-miR-148a-3p, hsa-miR-629-5p, hsa-miR-545-5p, hsa-miR-652-5p | 92.50 | 96.67 | 80.00 |
| 2672 | hsa-miR-424-5p, hsa-miR-34a-5p, hsa-miR-202-5p, hsa-miR-410-3p, hsa-miR-584-5p, hsa-miR-29b-3p, hsa-miR-590-5p, hsa-miR-340-5p, hsa-miR-433-3p, hsa-miR-148a-3p, hsa-miR-629-5p, hsa-miR-103a-2-5p, hsa-let-7i-5p, hsa-miR-652-5p | 92.50 | 93.33 | 80.00 |
| 2673 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-29b-3p, hsa-miR-15a-5p, hsa-miR-15a-3p, hsa-miR-1296-5p, hsa-let-7i-5p | 91.11 | 92.86 | 100.00 |
| 2674 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-15a-5p, hsa-miR-433-3p, hsa-miR-15a-3p, hsa-let-7i-5p | 91.11 | 91.43 | 100.00 |
| 2675 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-590-5p, hsa-miR-340-5p, hsa-miR-433-3p, hsa-miR-15a-3p, hsa-let-7i-5p | 91.11 | 94.60 | 73.33 |
| 2676 | hsa-miR-128-3p, hsa-miR-424-5p, hsa-miR-32-3p, hsa-miR-202-5p, hsa-miR-664a-3p, hsa-miR-744-5p, hsa-miR-1537-3p, hsa-miR-15a-5p, hsa-miR-433-3p, hsa-miR-15a-3p, hsa-let-7i-5p | 91.11 | 91.43 | 100.00 |
| 2677 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-15a-5p, hsa-miR-15a-3p, hsa-let-7i-5p | 91.11 | 91.43 | 100.00 |
| 2678 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-15a-3p, hsa-miR-1296-5p, hsa-let-7i-5p | 91.11 | 91.43 | 93.33 |
| 2679 | hsa-miR-128-3p, hsa-miR-424-5p, hsa-miR-32-3p, hsa-miR-202-5p, hsa-miR-744-5p, hsa-miR-1537-3p, hsa-miR-150-5p, hsa-miR-15a-5p, hsa-miR-433-3p, hsa-miR-15a-3p | 90.83 | 95.48 | 70.00 |
| 2680 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-505-5p, hsa-miR-433-3p, hsa-let-7i-5p | 90.83 | 94.92 | 83.33 |
| 2681 | hsa-miR-128-3p, hsa-miR-424-5p, hsa-miR-32-3p, hsa-miR-202-5p, hsa-miR-744-5p, hsa-miR-1537-3p, hsa-miR-15a-5p, hsa-miR-433-3p, hsa-miR-15a-3p | 90.83 | 97.14 | 80.00 |
| 2682 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-150-5p, hsa-miR-590-5p, hsa-miR-340-5p, hsa-miR-15a-5p, hsa-miR-433-3p, hsa-miR-15a-3p, hsa-miR-148a-3p, hsa-miR-652-5p | 90.83 | 92.62 | 80.00 |
| 2683 | hsa-miR-424-5p, hsa-miR-34a-5p, hsa-miR-32-3p, hsa-miR-202-5p, hsa-miR-410-3p, hsa-miR-744-5p, hsa-miR-150-5p, hsa-miR-433-3p, hsa-miR-629-5p, hsa-miR-103a-2-5p | 90.83 | 95.24 | 83.33 |
| 2684 | hsa-miR-128-3p, hsa-miR-424-5p, hsa-miR-34a-5p, hsa-miR-32-3p, hsa-miR-202-5p, hsa-miR-744-5p, hsa-miR-1537-3p, hsa-miR-590-5p, hsa-miR-340-5p, hsa-miR-15a-5p, hsa-miR-433-3p, hsa-miR-15a-3p, hsa-let-7i-5p | 90.83 | 95.71 | 80.00 |
| 2685 | hsa-miR-424-5p, hsa-miR-34a-5p, hsa-miR-32-3p, hsa-miR-664a-3p, hsa-miR-744-5p, hsa-miR-1537-3p, hsa-miR-340-5p, hsa-miR-433-3p, hsa-miR-15a-3p, hsa-miR-629-5p, hsa-let-7i-5p | 90.83 | 93.17 | 93.33 |
| 2686 | hsa-miR-128-3p, hsa-miR-424-5p, hsa-miR-34a-5p, hsa-miR-32-3p, hsa-miR-202-5p, hsa-miR-744-5p, hsa-miR-1537-3p, hsa-miR-150-5p, hsa-miR-433-3p, hsa-miR-15a-3p, hsa-miR-629-5p | 90.83 | 92.70 | 83.33 |
| 2687 | hsa-miR-128-3p, hsa-miR-424-5p, hsa-miR-34a-5p, hsa-miR-32-3p, hsa-miR-202-5p, hsa-miR-584-5p, hsa-miR-664a-3p, hsa-miR-744-5p, hsa-miR-1537-3p, hsa-miR-15a-3p, hsa-miR-545-5p, hsa-miR-1296-5p, hsa-let-7i-5p | 90.83 | 93.49 | 90.00 |
| 2688 | hsa-miR-128-3p, hsa-miR-424-5p, hsa-miR-32-3p, hsa-miR-202-5p, hsa-miR-744-5p, hsa-miR-1537-3p, hsa-miR-15a-5p, hsa-miR-433-3p, hsa-miR-15a-3p, hsa-miR-545-5p, hsa-let-7i-5p | 90.83 | 97.46 | 83.33 |
| 2689 | hsa-miR-369-3p, hsa-miR-128-3p, hsa-miR-424-5p, hsa-miR-34a-5p, hsa-miR-584-5p, hsa-miR-1537-3p, hsa-miR-590-5p, hsa-miR-340-5p, hsa-miR-15a-5p, hsa-miR-505-5p, hsa-miR-433-3p, hsa-miR-15a-3p, hsa-miR-148a-3p, hsa-let-7i-5p, hsa-miR-652-5p | 90.83 | 97.22 | 83.33 |
| 2690 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-15a-5p, hsa-miR-433-3p, hsa-miR-15a-3p, hsa-miR-103a-2-5p, hsa-let-7i-5p | 90.56 | 93.81 | 80.00 |
| 2691 | hsa-miR-128-3p, hsa-miR-424-5p, hsa-miR-32-3p, hsa-miR-202-5p, hsa-miR-410-3p, hsa-miR-584-5p, hsa-miR-744-5p, hsa-miR-1537-3p, hsa-miR-15a-5p, hsa-miR-433-3p, hsa-let-7i-5p | 90.56 | 95.48 | 90.00 |
| 2692 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-15a-5p, hsa-miR-433-3p, hsa-miR-29c-5p, hsa-miR-15a-3p, hsa-let-7i-5p | 90.56 | 98.57 | 80.00 |
| 2693 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-29b-3p, hsa-miR-15a-5p, hsa-miR-15a-3p, hsa-miR-103a-2-5p, hsa-let-7i-5p | 90.56 | 95.48 | 80.00 |
| 2694 | hsa-miR-369-3p, hsa-miR-128-3p, hsa-miR-34a-5p, hsa-miR-1537-3p, hsa-miR-590-5p, hsa-miR-340-5p, hsa-miR-15a-5p, hsa-miR-505-5p, hsa-miR-433-3p, hsa-miR-29c-5p, hsa-miR-15a-3p, hsa-miR-148a-3p, hsa-miR-545-5p, hsa-let-7i-5p, hsa-miR-652-5p | 90.56 | 98.33 | 80.00 |
| 2695 | hsa-miR-424-5p, hsa-miR-34a-5p, hsa-miR-32-3p, hsa-miR-202-5p, hsa-miR-410-3p, hsa-miR-584-5p, hsa-miR-744-5p, hsa-miR-29b-3p, hsa-miR-340-5p, hsa-miR-433-3p, hsa-miR-15a-3p, hsa-miR-545-5p, hsa-miR-103a-2-5p, hsa-let-7i-5p | 90.56 | 95.48 | 80.00 |
| 2696 | hsa-miR-128-3p, hsa-miR-424-5p, hsa-miR-202-5p, hsa-miR-1537-3p, hsa-miR-15a-5p, hsa-miR-433-3p, hsa-miR-15a-3p, hsa-miR-148a-3p, hsa-let-7i-5p, hsa-miR-652-5p | 90.56 | 98.57 | 80.00 |
| 2697 | hsa-miR-128-3p, hsa-miR-424-5p, hsa-miR-34a-5p, hsa-miR-32-3p, hsa-miR-202-5p, hsa-miR-744-5p, hsa-miR-1537-3p, hsa-miR-150-5p, hsa-miR-433-3p, hsa-miR-15a-3p | 90.56 | 94.13 | 73.33 |
| 2698 | hsa-miR-424-5p, hsa-miR-32-3p, hsa-miR-202-5p, hsa-miR-410-3p, hsa-miR-584-5p, hsa-miR-744-5p, hsa-miR-15a-5p, hsa-miR-433-3p, hsa-miR-29c-5p, hsa-miR-15a-3p, hsa-let-7i-5p | 90.56 | 96.90 | 90.00 |
| 2699 | hsa-miR-128-3p, hsa-miR-32-3p, hsa-miR-202-5p, hsa-miR-584-5p, hsa-miR-744-5p, hsa-miR-1537-3p, hsa-miR-150-5p, hsa-miR-15a-5p, hsa-miR-433-3p, hsa-miR-629-5p, hsa-miR-545-5p | 90.56 | 95.00 | 90.00 |
| 2700 | hsa-miR-128-3p, hsa-miR-424-5p, hsa-miR-32-3p, hsa-miR-202-5p, hsa-miR-744-5p, hsa-miR-1537-3p, hsa-miR-29b-3p, hsa-miR-15a-5p, hsa-miR-433-3p, hsa-miR-15a-3p, hsa-miR-103a-2-5p, hsa-let-7i-5p | 90.56 | 95.48 | 80.00 |
| 2701 | hsa-miR-128-3p, hsa-miR-424-5p, hsa-miR-32-3p, hsa-miR-744-5p, hsa-miR-1537-3p, hsa-miR-340-5p, hsa-miR-15a-5p, hsa-miR-433-3p, hsa-miR-15a-3p, hsa-miR-545-5p | 90.56 | 95.48 | 80.00 |
| 2702 | hsa-miR-424-5p, hsa-miR-32-3p, hsa-miR-202-5p, hsa-miR-584-5p, hsa-miR-744-5p, hsa-miR-1537-3p, hsa-miR-29b-3p, hsa-miR-15a-5p, hsa-miR-433-3p, hsa-miR-15a-3p, hsa-miR-103a-2-5p, hsa-let-7i-5p | 90.56 | 95.48 | 80.00 |
| 2703 | hsa-miR-128-3p, hsa-miR-424-5p, hsa-miR-202-5p, hsa-miR-584-5p, hsa-miR-1537-3p, hsa-miR-590-5p, hsa-miR-15a-5p, hsa-miR-433-3p, hsa-miR-15a-3p, hsa-miR-148a-3p, hsa-miR-652-5p | 90.56 | 97.14 | 70.00 |
| 2704 | hsa-miR-424-5p, hsa-miR-32-3p, hsa-miR-410-3p, hsa-miR-150-5p, hsa-miR-590-5p, hsa-miR-340-5p, hsa-miR-15a-5p, hsa-miR-433-3p, hsa-miR-148a-3p, hsa-miR-629-5p, hsa-miR-545-5p, hsa-miR-652-5p | 90.28 | 92.14 | 90.00 |
| 2705 | hsa-miR-128-3p, hsa-miR-424-5p, hsa-miR-32-3p, hsa-miR-202-5p, hsa-miR-410-3p, hsa-miR-584-5p, hsa-miR-744-5p, hsa-miR-1537-3p, hsa-miR-29b-3p, hsa-miR-433-3p, hsa-miR-15a-3p, hsa-let-7i-5p | 90.28 | 95.24 | 80.00 |
| 2706 | hsa-miR-369-3p, hsa-miR-128-3p, hsa-miR-424-5p, hsa-miR-34a-5p, hsa-miR-202-5p, hsa-miR-584-5p, hsa-miR-1537-3p, hsa-miR-590-5p, hsa-miR-340-5p, hsa-miR-15a-5p, hsa-miR-433-3p, hsa-miR-29c-5p, hsa-miR-15a-3p, hsa-miR-148a-3p, hsa-miR-545-5p, hsa-miR-1296-5p, hsa-miR-103a-2-5p, hsa-let-7i-5p, hsa-miR-652-5p | 90.28 | 93.33 | 70.00 |
| 2707 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-433-3p, hsa-miR-15a-3p, hsa-let-7i-5p | 88.89 | 90.00 | 93.33 |
| 2708 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-433-3p, hsa-miR-15a-3p, hsa-miR-545-5p, hsa-let-7i-5p | 88.89 | 96.35 | 86.67 |
| 2709 | hsa-miR-128-3p, hsa-miR-424-5p, hsa-miR-32-3p, hsa-miR-202-5p, hsa-miR-664a-3p, hsa-miR-744-5p, hsa-miR-1537-3p, hsa-miR-15a-5p, hsa-miR-15a-3p, hsa-let-7i-5p | 88.89 | 91.43 | 100.00 |
| 2710 | hsa-miR-128-3p, hsa-miR-424-5p, hsa-miR-34a-5p, hsa-miR-32-3p, hsa-miR-202-5p, hsa-miR-584-5p, hsa-miR-664a-3p, hsa-miR-744-5p, hsa-miR-1537-3p, hsa-miR-15a-3p, hsa-miR-1296-5p, hsa-let-7i-5p | 88.89 | 92.86 | 83.33 |
| 2711 | hsa-miR-128-3p, hsa-miR-424-5p, hsa-miR-32-3p, hsa-miR-202-5p, hsa-miR-744-5p, hsa-miR-1537-3p, hsa-miR-150-5p, hsa-miR-15a-5p, hsa-miR-433-3p, hsa-miR-15a-3p, hsa-miR-629-5p | 88.61 | 98.57 | 100.00 |
| 2712 | hsa-miR-128-3p, hsa-miR-424-5p, hsa-miR-32-3p, hsa-miR-202-5p, hsa-miR-744-5p, hsa-miR-1537-3p, hsa-miR-15a-5p, hsa-miR-505-5p, hsa-miR-433-3p, hsa-miR-15a-3p | 88.61 | 97.14 | 80.00 |
| 2713 | hsa-miR-128-3p, hsa-miR-32-3p, hsa-miR-202-5p, hsa-miR-584-5p, hsa-miR-664a-3p, hsa-miR-744-5p, hsa-miR-1537-3p, hsa-miR-29b-3p, hsa-miR-15a-3p, hsa-miR-103a-2-5p, hsa-let-7i-5p | 88.61 | 91.19 | 90.00 |
| 2714 | hsa-miR-128-3p, hsa-miR-424-5p, hsa-miR-32-3p, hsa-miR-202-5p, hsa-miR-744-5p, hsa-miR-150-5p, hsa-miR-15a-5p, hsa-miR-433-3p, hsa-miR-629-5p | 88.61 | 88.41 | 90.00 |
| 2715 | hsa-miR-424-5p, hsa-miR-34a-5p, hsa-miR-32-3p, hsa-miR-202-5p, hsa-miR-410-3p, hsa-miR-744-5p, hsa-miR-505-5p, hsa-miR-433-3p, hsa-miR-1296-5p, hsa-let-7i-5p | 88.61 | 96.03 | 76.67 |
| 2716 | hsa-miR-424-5p, hsa-miR-34a-5p, hsa-miR-32-3p, hsa-miR-202-5p, hsa-miR-584-5p, hsa-miR-744-5p, hsa-miR-150-5p, hsa-miR-590-5p, hsa-miR-340-5p, hsa-miR-15a-5p, hsa-miR-433-3p, hsa-miR-629-5p | 88.61 | 91.43 | 80.00 |
| 2717 | hsa-miR-369-3p, hsa-miR-128-3p, hsa-miR-424-5p, hsa-miR-34a-5p, hsa-miR-32-3p, hsa-miR-744-5p, hsa-miR-1537-3p, hsa-miR-150-5p, hsa-miR-15a-5p, hsa-miR-505-5p, hsa-miR-433-3p, hsa-miR-15a-3p, hsa-miR-629-5p, hsa-let-7i-5p | 88.61 | 97.14 | 83.33 |
| 2718 | hsa-miR-128-3p, hsa-miR-424-5p, hsa-miR-34a-5p, hsa-miR-202-5p, hsa-miR-584-5p, hsa-miR-1537-3p, hsa-miR-433-3p, hsa-miR-29c-5p, hsa-miR-15a-3p, hsa-miR-148a-3p, hsa-let-7i-5p, hsa-miR-652-5p | 88.61 | 97.46 | 83.33 |
| 2719 | hsa-miR-424-5p, hsa-miR-32-3p, hsa-miR-664a-3p, hsa-miR-1537-3p, hsa-miR-150-5p, hsa-miR-590-5p, hsa-miR-340-5p, hsa-miR-505-5p, hsa-miR-29c-5p, hsa-miR-1296-5p, hsa-let-7i-5p | 88.61 | 86.90 | 80.00 |
| 2720 | hsa-miR-34a-5p, hsa-miR-32-3p, hsa-miR-202-5p, hsa-miR-410-3p, hsa-miR-664a-3p, hsa-miR-29b-3p, hsa-miR-629-5p, hsa-miR-1296-5p, hsa-let-7i-5p | 88.61 | 95.48 | 83.33 |
| 2721 | hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-744-5p, hsa-miR-1537-3p, hsa-miR-340-5p, hsa-miR-15a-3p, hsa-let-7i-5p | 88.61 | 93.25 | 73.33 |
| 2722 | hsa-miR-424-5p, hsa-miR-34a-5p, hsa-miR-32-3p, hsa-miR-744-5p, hsa-miR-29b-3p, hsa-miR-505-5p, hsa-miR-433-3p, hsa-miR-629-5p, hsa-miR-1296-5p, hsa-miR-103a-2-5p, hsa-let-7i-5p, hsa-miR-652-5p | 88.33 | 97.14 | 70.00 |
| 2723 | hsa-miR-369-3p, hsa-miR-128-3p, hsa-miR-424-5p, hsa-miR-34a-5p, hsa-miR-32-3p, hsa-miR-584-5p, hsa-miR-744-5p, hsa-miR-1537-3p, hsa-miR-150-5p, hsa-miR-590-5p, hsa-miR-340-5p, hsa-miR-15a-5p, hsa-miR-505-5p, hsa-miR-433-3p, hsa-miR-15a-3p, hsa-let-7i-5p | 88.33 | 95.48 | 80.00 |
| 2724 | hsa-miR-128-3p, hsa-miR-424-5p, hsa-miR-32-3p, hsa-miR-202-5p, hsa-miR-744-5p, hsa-miR-150-5p, hsa-miR-15a-5p, hsa-miR-433-3p, hsa-miR-629-5p, hsa-miR-545-5p | 88.33 | 93.81 | 83.33 |
| 2725 | hsa-miR-369-3p, hsa-miR-128-3p, hsa-miR-32-3p, hsa-miR-202-5p, hsa-miR-584-5p, hsa-miR-744-5p, hsa-miR-1537-3p, hsa-miR-15a-5p, hsa-miR-505-5p, hsa-miR-433-3p, hsa-miR-15a-3p, hsa-let-7i-5p | 88.33 | 94.68 | 73.33 |
| 2726 | hsa-miR-128-3p, hsa-miR-424-5p, hsa-miR-202-5p, hsa-miR-584-5p, hsa-miR-664a-3p, hsa-miR-1537-3p, hsa-miR-29b-3p, hsa-miR-590-5p, hsa-miR-340-5p, hsa-miR-15a-5p, hsa-miR-29c-5p, hsa-miR-15a-3p, hsa-miR-148a-3p, hsa-miR-545-5p, hsa-miR-1296-5p, hsa-miR-103a-2-5p, hsa-let-7i-5p, hsa-miR-652-5p | 88.33 | 91.43 | 70.00 |
| 2727 | hsa-miR-424-5p, hsa-miR-32-3p, hsa-miR-202-5p, hsa-miR-410-3p, hsa-miR-744-5p, hsa-miR-15a-5p, hsa-miR-433-3p, hsa-miR-629-5p, hsa-miR-545-5p, hsa-let-7i-5p | 88.33 | 92.14 | 83.33 |
| 2728 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-29b-3p, hsa-miR-15a-5p, hsa-miR-29c-5p, hsa-miR-15a-3p, hsa-miR-103a-2-5p, hsa-let-7i-5p | 88.33 | 90.95 | 80.00 |
| 2729 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-29b-3p, hsa-miR-15a-5p, hsa-miR-433-3p, hsa-miR-15a-3p, hsa-miR-103a-2-5p, hsa-let-7i-5p | 88.33 | 93.81 | 80.00 |
| 2730 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-29b-3p, hsa-miR-15a-5p, hsa-miR-433-3p, hsa-miR-29c-5p, hsa-miR-15a-3p, hsa-miR-103a-2-5p, hsa-let-7i-5p | 88.33 | 92.62 | 80.00 |
| 2731 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-29b-3p, hsa-miR-15a-5p, hsa-miR-433-3p, hsa-miR-15a-3p, hsa-miR-103a-2-5p, hsa-let-7i-5p | 88.33 | 93.81 | 80.00 |
| 2732 | hsa-miR-424-5p, hsa-miR-664a-3p, hsa-miR-1537-3p, hsa-miR-150-5p, hsa-miR-590-5p, hsa-miR-340-5p, hsa-miR-15a-5p, hsa-miR-505-5p, hsa-miR-15a-3p, hsa-miR-629-5p, hsa-miR-545-5p, hsa-miR-1296-5p, hsa-let-7i-5p, hsa-miR-652-5p | 88.06 | 92.38 | 80.00 |
| 2733 | hsa-miR-128-3p, hsa-miR-424-5p, hsa-miR-34a-5p, hsa-miR-202-5p, hsa-miR-1537-3p, hsa-miR-150-5p, hsa-miR-590-5p, hsa-miR-340-5p, hsa-miR-433-3p, hsa-miR-29c-5p, hsa-miR-15a-3p, hsa-miR-148a-3p, hsa-miR-545-5p, hsa-miR-652-5p | 88.06 | 95.24 | 70.00 |
| 2734 | hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-744-5p, hsa-miR-1537-3p, hsa-miR-433-3p, hsa-miR-15a-3p, hsa-let-7i-5p | 88.06 | 95.24 | 83.33 |
| 2735 | hsa-miR-128-3p, hsa-miR-424-5p, hsa-miR-202-5p, hsa-miR-584-5p, hsa-miR-744-5p, hsa-miR-1537-3p, hsa-miR-150-5p, hsa-miR-590-5p, hsa-miR-340-5p, hsa-miR-433-3p, hsa-miR-629-5p, hsa-miR-545-5p, hsa-miR-652-5p | 88.06 | 96.67 | 80.00 |
| 2736 | hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-15a-5p, hsa-miR-433-3p, hsa-miR-629-5p, hsa-miR-545-5p | 88.06 | 91.90 | 80.00 |
| 2737 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-150-5p, hsa-miR-15a-5p, hsa-miR-433-3p, hsa-miR-15a-3p, hsa-miR-545-5p | 88.06 | 95.56 | 73.33 |
| 2738 | hsa-miR-128-3p, hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-15a-5p, hsa-miR-433-3p, hsa-miR-629-5p, hsa-miR-545-5p | 88.06 | 91.90 | 90.00 |
| 2739 | hsa-miR-369-3p, hsa-miR-128-3p, hsa-miR-202-5p, hsa-miR-584-5p, hsa-miR-664a-3p, hsa-miR-1537-3p, hsa-miR-15a-5p, hsa-miR-29c-5p, hsa-miR-15a-3p, hsa-let-7i-5p | 87.78 | 91.11 | 70.00 |
| 2740 | hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-340-5p, hsa-miR-15a-5p, hsa-miR-433-3p, hsa-miR-629-5p, hsa-miR-545-5p, hsa-let-7i-5p | 87.78 | 93.81 | 90.00 |
| 2741 | hsa-miR-369-3p, hsa-miR-128-3p, hsa-miR-584-5p, hsa-miR-1537-3p, hsa-miR-15a-5p, hsa-miR-505-5p, hsa-miR-433-3p, hsa-miR-15a-3p, hsa-miR-148a-3p, hsa-let-7i-5p, hsa-miR-652-5p | 86.39 | 98.89 | 83.33 |
| 2742 | hsa-miR-369-3p, hsa-miR-128-3p, hsa-miR-202-5p, hsa-miR-744-5p, hsa-miR-1537-3p, hsa-miR-15a-5p, hsa-miR-505-5p, hsa-miR-433-3p, hsa-miR-15a-3p, hsa-let-7i-5p | 86.39 | 96.11 | 76.67 |
| 2743 | hsa-miR-128-3p, hsa-miR-32-3p, hsa-miR-202-5p, hsa-miR-584-5p, hsa-miR-744-5p, hsa-miR-433-3p, hsa-miR-15a-3p | 86.39 | 91.19 | 86.67 |
| 2744 | hsa-miR-128-3p, hsa-miR-424-5p, hsa-miR-32-3p, hsa-miR-202-5p, hsa-miR-584-5p, hsa-miR-744-5p, hsa-miR-150-5p, hsa-miR-15a-5p, hsa-miR-433-3p, hsa-miR-629-5p | 86.39 | 88.10 | 90.00 |
| 2745 | hsa-miR-369-3p, hsa-miR-410-3p, hsa-miR-664a-3p, hsa-miR-744-5p, hsa-miR-1537-3p, hsa-miR-590-5p, hsa-miR-340-5p, hsa-miR-15a-3p, hsa-miR-148a-3p, hsa-miR-1296-5p, hsa-let-7i-5p, hsa-miR-652-5p | 86.39 | 84.84 | 73.33 |
| 2746 | hsa-miR-128-3p, hsa-miR-424-5p, hsa-miR-34a-5p, hsa-miR-202-5p, hsa-miR-410-3p, hsa-miR-584-5p, hsa-miR-664a-3p, hsa-miR-1537-3p, hsa-miR-29b-3p, hsa-miR-590-5p, hsa-miR-340-5p, hsa-miR-29c-5p, hsa-miR-15a-3p, hsa-miR-148a-3p, hsa-miR-545-5p, hsa-miR-1296-5p, hsa-let-7i-5p, hsa-miR-652-5p | 86.39 | 90.32 | 80.00 |
| 2747 | hsa-miR-128-3p, hsa-miR-424-5p, hsa-miR-34a-5p, hsa-miR-32-3p, hsa-miR-202-5p, hsa-miR-584-5p, hsa-miR-664a-3p, hsa-miR-744-5p, hsa-miR-1537-3p, hsa-miR-433-3p, hsa-miR-29c-5p, hsa-miR-15a-3p, hsa-let-7i-5p | 86.39 | 92.14 | 73.33 |
| 2748 | hsa-miR-369-3p, hsa-miR-128-3p, hsa-miR-584-5p, hsa-miR-1537-3p, hsa-miR-505-5p, hsa-miR-433-3p, hsa-miR-15a-3p, hsa-miR-148a-3p, hsa-let-7i-5p, hsa-miR-652-5p | 86.39 | 94.92 | 83.33 |
| 2749 | hsa-miR-128-3p, hsa-miR-424-5p, hsa-miR-34a-5p, hsa-miR-32-3p, hsa-miR-202-5p, hsa-miR-584-5p, hsa-miR-664a-3p, hsa-miR-744-5p, hsa-miR-1537-3p, hsa-miR-29b-3p, hsa-miR-433-3p, hsa-miR-15a-3p, hsa-miR-545-5p, hsa-miR-1296-5p, hsa-miR-103a-2-5p, hsa-let-7i-5p | 86.11 | 95.71 | 80.00 |
| 2750 | hsa-miR-128-3p, hsa-miR-424-5p, hsa-miR-34a-5p, hsa-miR-32-3p, hsa-miR-202-5p, hsa-miR-584-5p, hsa-miR-664a-3p, hsa-miR-744-5p, hsa-miR-1537-3p, hsa-miR-29b-3p, hsa-miR-15a-3p, hsa-miR-1296-5p, hsa-miR-103a-2-5p, hsa-let-7i-5p | 86.11 | 92.62 | 70.00 |
| 2751 | hsa-miR-369-3p, hsa-miR-128-3p, hsa-miR-424-5p, hsa-miR-32-3p, hsa-miR-744-5p, hsa-miR-1537-3p, hsa-miR-15a-5p, hsa-miR-433-3p, hsa-miR-15a-3p, hsa-let-7i-5p | 86.11 | 94.37 | 73.33 |
| 2752 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-29b-3p, hsa-miR-15a-5p, hsa-miR-15a-3p, hsa-miR-103a-2-5p, hsa-let-7i-5p | 86.11 | 90.95 | 80.00 |
| 2753 | hsa-miR-128-3p, hsa-miR-424-5p, hsa-miR-32-3p, hsa-miR-202-5p, hsa-miR-744-5p, hsa-miR-1537-3p, hsa-miR-150-5p, hsa-miR-15a-5p, hsa-miR-433-3p, hsa-miR-629-5p | 86.11 | 90.95 | 90.00 |
| 2754 | hsa-miR-369-3p, hsa-miR-128-3p, hsa-miR-34a-5p, hsa-miR-32-3p, hsa-miR-202-5p, hsa-miR-410-3p, hsa-miR-664a-3p, hsa-miR-1537-3p, hsa-miR-340-5p, hsa-miR-15a-3p, hsa-miR-545-5p, hsa-let-7i-5p | 86.11 | 96.35 | 66.67 |
| 2755 | hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-590-5p, hsa-miR-340-5p, hsa-miR-15a-5p, hsa-miR-433-3p, hsa-miR-148a-3p, hsa-miR-629-5p, hsa-miR-652-5p | 86.11 | 92.62 | 70.00 |
| 2756 | hsa-miR-369-3p, hsa-miR-128-3p, hsa-miR-584-5p, hsa-miR-1537-3p, hsa-miR-15a-5p, hsa-miR-505-5p, hsa-miR-433-3p, hsa-miR-15a-3p, hsa-let-7i-5p, hsa-miR-652-5p | 86.11 | 96.67 | 73.33 |
| 2757 | hsa-miR-128-3p, hsa-miR-424-5p, hsa-miR-202-5p, hsa-miR-584-5p, hsa-miR-1537-3p, hsa-miR-590-5p, hsa-miR-15a-5p, hsa-miR-433-3p, hsa-miR-15a-3p, hsa-miR-148a-3p, hsa-let-7i-5p | 86.11 | 96.03 | 63.33 |
| 2758 | hsa-miR-369-3p, hsa-miR-128-3p, hsa-miR-202-5p, hsa-miR-584-5p, hsa-miR-1537-3p, hsa-miR-590-5p, hsa-miR-15a-5p, hsa-miR-505-5p, hsa-miR-433-3p, hsa-miR-15a-3p, hsa-miR-148a-3p, hsa-let-7i-5p, hsa-miR-652-5p | 86.11 | 94.13 | 66.67 |
| 2759 | hsa-miR-128-3p, hsa-miR-424-5p, hsa-miR-34a-5p, hsa-miR-410-3p, hsa-miR-744-5p, hsa-miR-150-5p, hsa-miR-15a-5p, hsa-miR-505-5p, hsa-miR-148a-3p, hsa-miR-629-5p, hsa-miR-545-5p | 86.11 | 95.48 | 90.00 |
| 2760 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-29b-3p, hsa-miR-15a-5p, hsa-miR-29c-5p, hsa-miR-15a-3p, hsa-miR-103a-2-5p, hsa-let-7i-5p | 86.11 | 91.19 | 70.00 |
| 2761 | hsa-miR-424-5p, hsa-miR-32-3p, hsa-miR-202-5p, hsa-miR-410-3p, hsa-miR-744-5p, hsa-miR-150-5p, hsa-miR-15a-5p, hsa-miR-433-3p, hsa-miR-29c-5p, hsa-let-7i-5p | 86.11 | 92.38 | 90.00 |
| 2762 | hsa-miR-128-3p, hsa-miR-32-3p, hsa-miR-202-5p, hsa-miR-744-5p, hsa-miR-1537-3p, hsa-miR-15a-5p, hsa-miR-433-3p, hsa-miR-15a-3p | 86.11 | 90.63 | 73.33 |
| 2763 | hsa-miR-128-3p, hsa-miR-34a-5p, hsa-miR-202-5p, hsa-miR-584-5p, hsa-miR-1537-3p, hsa-miR-15a-5p, hsa-miR-505-5p, hsa-miR-433-3p, hsa-miR-29c-5p, hsa-miR-15a-3p, hsa-miR-148a-3p, hsa-let-7i-5p, hsa-miR-652-5p | 86.11 | 92.38 | 70.00 |
| 2764 | hsa-miR-369-3p, hsa-miR-128-3p, hsa-miR-424-5p, hsa-miR-32-3p, hsa-miR-584-5p, hsa-miR-744-5p, hsa-miR-1537-3p, hsa-miR-15a-5p, hsa-miR-505-5p, hsa-miR-433-3p, hsa-miR-15a-3p, hsa-let-7i-5p | 86.11 | 95.48 | 90.00 |
| 2765 | hsa-miR-128-3p, hsa-miR-424-5p, hsa-miR-34a-5p, hsa-miR-32-3p, hsa-miR-202-5p, hsa-miR-744-5p, hsa-miR-1537-3p, hsa-miR-340-5p, hsa-miR-15a-5p, hsa-miR-433-3p, hsa-miR-15a-3p, hsa-miR-545-5p, hsa-miR-652-5p | 86.11 | 93.49 | 73.33 |
| 2766 | hsa-miR-590-5p, hsa-miR-15a-5p, hsa-miR-15a-3p, hsa-miR-148a-3p | 85.83 | 97.14 | 50.00 |
| 2767 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-150-5p, hsa-miR-15a-5p, hsa-miR-433-3p, hsa-miR-15a-3p, hsa-miR-545-5p, hsa-let-7i-5p | 85.83 | 94.13 | 73.33 |
| 2768 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-29b-3p, hsa-miR-15a-5p, hsa-miR-15a-3p, hsa-miR-1296-5p, hsa-miR-103a-2-5p, hsa-let-7i-5p | 85.83 | 89.29 | 70.00 |
| 2769 | hsa-miR-424-5p, hsa-miR-32-3p, hsa-miR-202-5p, hsa-miR-410-3p, hsa-miR-584-5p, hsa-miR-664a-3p, hsa-miR-744-5p, hsa-miR-29b-3p, hsa-miR-15a-3p, hsa-miR-1296-5p, hsa-miR-103a-2-5p, hsa-let-7i-5p | 85.83 | 86.19 | 80.00 |
| 2770 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-150-5p, hsa-miR-29b-3p, hsa-miR-505-5p, hsa-miR-433-3p, hsa-miR-29c-5p, hsa-miR-629-5p, hsa-miR-1296-5p, hsa-let-7i-5p, hsa-miR-652-5p | 85.83 | 93.81 | 80.00 |
| 2771 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-150-5p, hsa-miR-15a-5p, hsa-miR-433-3p, hsa-miR-629-5p, hsa-let-7i-5p | 85.83 | 90.71 | 90.00 |
| 2772 | hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-433-3p, hsa-miR-29c-5p, hsa-miR-629-5p, hsa-miR-545-5p, hsa-let-7i-5p | 85.83 | 91.90 | 83.33 |
| 2773 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-150-5p, hsa-miR-15a-5p, hsa-miR-433-3p, hsa-miR-15a-3p, hsa-miR-545-5p | 85.83 | 95.56 | 73.33 |
| 2774 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-15a-5p, hsa-miR-433-3p, hsa-miR-15a-3p, hsa-miR-103a-2-5p, hsa-miR-652-5p | 85.83 | 86.27 | 73.33 |
| 2775 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-150-5p, hsa-miR-340-5p, hsa-miR-15a-5p, hsa-miR-433-3p, hsa-miR-15a-3p, hsa-miR-629-5p | 85.83 | 91.27 | 73.33 |
| 2776 | hsa-miR-424-5p, hsa-miR-32-3p, hsa-miR-202-5p, hsa-miR-410-3p, hsa-miR-584-5p, hsa-miR-744-5p, hsa-miR-29b-3p, hsa-miR-15a-5p, hsa-miR-433-3p, hsa-miR-103a-2-5p, hsa-let-7i-5p | 85.83 | 93.81 | 70.00 |
| 2777 | hsa-miR-424-5p, hsa-miR-32-3p, hsa-miR-202-5p, hsa-miR-410-3p, hsa-miR-584-5p, hsa-miR-744-5p, hsa-miR-15a-5p, hsa-miR-433-3p, hsa-miR-29c-5p, hsa-miR-629-5p, hsa-let-7i-5p | 85.83 | 89.92 | 83.33 |
| 2778 | hsa-miR-369-3p, hsa-miR-128-3p, hsa-miR-424-5p, hsa-miR-32-3p, hsa-miR-202-5p, hsa-miR-664a-3p, hsa-miR-150-5p, hsa-miR-29b-3p, hsa-miR-340-5p, hsa-miR-15a-5p, hsa-miR-433-3p, hsa-miR-15a-3p, hsa-miR-103a-2-5p | 85.56 | 89.05 | 70.00 |
| 2779 | hsa-miR-424-5p, hsa-miR-32-3p, hsa-miR-202-5p, hsa-miR-410-3p, hsa-miR-584-5p, hsa-miR-744-5p, hsa-miR-1537-3p, hsa-miR-15a-5p, hsa-miR-433-3p, hsa-miR-15a-3p, hsa-let-7i-5p | 85.56 | 93.57 | 73.33 |
| 2780 | hsa-miR-424-5p, hsa-miR-32-3p, hsa-miR-202-5p, hsa-miR-410-3p, hsa-miR-744-5p, hsa-miR-150-5p, hsa-miR-15a-5p, hsa-miR-433-3p, hsa-miR-629-5p, hsa-miR-545-5p | 85.56 | 93.57 | 83.33 |
| 2781 | hsa-miR-128-3p, hsa-miR-424-5p, hsa-miR-34a-5p, hsa-miR-202-5p, hsa-miR-744-5p, hsa-miR-1537-3p, hsa-miR-15a-5p, hsa-miR-433-3p, hsa-miR-15a-3p, hsa-let-7i-5p | 85.56 | 84.92 | 60.00 |
| 2782 | hsa-miR-424-5p, hsa-miR-34a-5p, hsa-miR-32-3p, hsa-miR-202-5p, hsa-miR-410-3p, hsa-miR-744-5p, hsa-miR-150-5p, hsa-miR-15a-5p, hsa-miR-433-3p, hsa-miR-629-5p, hsa-miR-545-5p | 85.56 | 91.90 | 83.33 |
| 2783 | hsa-miR-369-3p, hsa-miR-202-5p, hsa-miR-1537-3p, hsa-miR-150-5p, hsa-miR-590-5p, hsa-miR-340-5p, hsa-miR-15a-5p, hsa-miR-433-3p, hsa-miR-29c-5p, hsa-miR-15a-3p, hsa-miR-545-5p, hsa-miR-103a-2-5p, hsa-miR-652-5p | 85.28 | 92.22 | 70.00 |
| 2784 | hsa-miR-369-3p, hsa-miR-128-3p, hsa-miR-32-3p, hsa-miR-744-5p, hsa-miR-1537-3p, hsa-miR-15a-5p, hsa-miR-505-5p, hsa-miR-433-3p, hsa-miR-15a-3p, hsa-let-7i-5p | 84.17 | 92.38 | 76.67 |
| 2785 | hsa-miR-424-5p, hsa-miR-34a-5p, hsa-miR-32-3p, hsa-miR-202-5p, hsa-miR-584-5p, hsa-miR-744-5p, hsa-miR-433-3p | 84.17 | 93.17 | 73.33 |
| 2786 | hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-744-5p, hsa-miR-1537-3p, hsa-miR-433-3p, hsa-miR-15a-3p, hsa-miR-148a-3p, hsa-miR-103a-2-5p, hsa-let-7i-5p | 84.17 | 91.83 | 73.33 |
| 2787 | hsa-miR-369-3p, hsa-miR-128-3p, hsa-miR-34a-5p, hsa-miR-32-3p, hsa-miR-584-5p, hsa-miR-744-5p, hsa-miR-1537-3p, hsa-miR-505-5p, hsa-miR-433-3p, hsa-miR-15a-3p, hsa-let-7i-5p | 84.17 | 94.92 | 76.67 |
| 2788 | hsa-miR-128-3p, hsa-miR-32-3p, hsa-miR-584-5p, hsa-miR-744-5p, hsa-miR-1537-3p, hsa-miR-15a-5p, hsa-miR-505-5p, hsa-miR-433-3p, hsa-miR-15a-3p, hsa-let-7i-5p | 84.17 | 91.43 | 70.00 |
| 2789 | hsa-miR-369-3p, hsa-miR-128-3p, hsa-miR-32-3p, hsa-miR-584-5p, hsa-miR-744-5p, hsa-miR-1537-3p, hsa-miR-505-5p, hsa-miR-433-3p, hsa-miR-15a-3p, hsa-miR-1296-5p, hsa-let-7i-5p | 84.17 | 90.16 | 86.67 |
| 2790 | hsa-miR-369-3p, hsa-miR-128-3p, hsa-miR-202-5p, hsa-miR-584-5p, hsa-miR-744-5p, hsa-miR-1537-3p, hsa-miR-15a-5p, hsa-miR-505-5p, hsa-miR-433-3p, hsa-miR-15a-3p, hsa-let-7i-5p | 84.17 | 96.11 | 76.67 |
| 2791 | hsa-miR-369-3p, hsa-miR-424-5p, hsa-miR-32-3p, hsa-miR-202-5p, hsa-miR-410-3p, hsa-miR-744-5p, hsa-miR-150-5p, hsa-miR-590-5p, hsa-miR-340-5p, hsa-miR-15a-5p, hsa-miR-433-3p, hsa-miR-148a-3p, hsa-miR-629-5p, hsa-miR-652-5p | 83.89 | 84.76 | 70.00 |
| 2792 | hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-15a-3p, hsa-miR-1296-5p, hsa-let-7i-5p | 83.89 | 89.52 | 73.33 |
| 2793 | hsa-miR-369-3p, hsa-miR-128-3p, hsa-miR-32-3p, hsa-miR-584-5p, hsa-miR-744-5p, hsa-miR-1537-3p, hsa-miR-15a-5p, hsa-miR-433-3p, hsa-miR-15a-3p, hsa-let-7i-5p | 83.89 | 96.03 | 80.00 |
| 2794 | hsa-miR-369-3p, hsa-miR-34a-5p, hsa-miR-32-3p, hsa-miR-584-5p, hsa-miR-1537-3p, hsa-miR-150-5p, hsa-miR-29c-5p, hsa-miR-15a-3p, hsa-miR-629-5p, hsa-miR-1296-5p, hsa-let-7i-5p, hsa-miR-652-5p | 83.89 | 91.35 | 83.33 |
| 2795 | hsa-miR-369-3p, hsa-miR-128-3p, hsa-miR-32-3p, hsa-miR-202-5p, hsa-miR-584-5p, hsa-miR-744-5p, hsa-miR-1537-3p, hsa-miR-505-5p, hsa-miR-433-3p, hsa-miR-15a-3p, hsa-let-7i-5p | 83.89 | 93.25 | 83.33 |
| 2796 | hsa-miR-424-5p, hsa-miR-32-3p, hsa-miR-202-5p, hsa-miR-410-3p, hsa-miR-584-5p, hsa-miR-744-5p, hsa-miR-15a-5p, hsa-miR-433-3p, hsa-let-7i-5p | 83.89 | 92.94 | 83.33 |
| 2797 | hsa-miR-369-3p, hsa-miR-128-3p, hsa-miR-34a-5p, hsa-miR-32-3p, hsa-miR-584-5p, hsa-miR-744-5p, hsa-miR-1537-3p, hsa-miR-15a-5p, hsa-miR-505-5p, hsa-miR-433-3p, hsa-miR-15a-3p, hsa-let-7i-5p | 83.89 | 96.35 | 73.33 |
| 2798 | hsa-miR-369-3p, hsa-miR-128-3p, hsa-miR-584-5p, hsa-miR-1537-3p, hsa-miR-15a-5p, hsa-miR-505-5p, hsa-miR-433-3p, hsa-miR-15a-3p, hsa-let-7i-5p | 83.89 | 92.22 | 73.33 |
| 2799 | hsa-miR-424-5p, hsa-miR-32-3p, hsa-miR-202-5p, hsa-miR-410-3p, hsa-miR-584-5p, hsa-miR-744-5p, hsa-miR-1537-3p, hsa-miR-15a-5p, hsa-miR-433-3p, hsa-miR-29c-5p, hsa-miR-15a-3p, hsa-miR-1296-5p, hsa-let-7i-5p | 83.89 | 91.51 | 73.33 |
| 2800 | hsa-miR-369-3p, hsa-miR-128-3p, hsa-miR-34a-5p, hsa-miR-32-3p, hsa-miR-1537-3p, hsa-miR-433-3p, hsa-miR-15a-3p, hsa-let-7i-5p | 83.89 | 91.90 | 66.67 |
| 2801 | hsa-miR-128-3p, hsa-miR-424-5p, hsa-miR-32-3p, hsa-miR-584-5p, hsa-miR-1537-3p, hsa-miR-29b-3p, hsa-miR-505-5p, hsa-miR-433-3p, hsa-miR-29c-5p, hsa-miR-148a-3p, hsa-miR-103a-2-5p, hsa-let-7i-5p, hsa-miR-652-5p | 83.89 | 84.29 | 70.00 |
| 2802 | hsa-miR-424-5p, hsa-miR-34a-5p, hsa-miR-32-3p, hsa-miR-202-5p, hsa-miR-584-5p, hsa-miR-744-5p, hsa-miR-150-5p, hsa-miR-590-5p, hsa-miR-15a-5p, hsa-miR-433-3p, hsa-miR-148a-3p, hsa-miR-629-5p, hsa-miR-652-5p | 83.89 | 86.43 | 83.33 |
| 2803 | hsa-miR-128-3p, hsa-miR-584-5p, hsa-miR-1537-3p, hsa-miR-15a-5p, hsa-miR-505-5p, hsa-miR-433-3p, hsa-miR-15a-3p, hsa-let-7i-5p | 83.89 | 92.14 | 63.33 |
| 2804 | hsa-miR-369-3p, hsa-miR-128-3p, hsa-miR-34a-5p, hsa-miR-202-5p, hsa-miR-584-5p, hsa-miR-744-5p, hsa-miR-1537-3p, hsa-miR-15a-5p, hsa-miR-505-5p, hsa-miR-433-3p, hsa-miR-15a-3p, hsa-let-7i-5p | 83.89 | 94.44 | 66.67 |
| 2805 | hsa-miR-128-3p, hsa-miR-424-5p, hsa-miR-34a-5p, hsa-miR-32-3p, hsa-miR-202-5p, hsa-miR-584-5p, hsa-miR-744-5p, hsa-miR-1537-3p, hsa-miR-150-5p, hsa-miR-433-3p, hsa-miR-629-5p, hsa-miR-1296-5p, hsa-let-7i-5p | 83.89 | 90.95 | 83.33 |
| 2806 | hsa-miR-128-3p, hsa-miR-424-5p, hsa-miR-32-3p, hsa-miR-202-5p, hsa-miR-584-5p, hsa-miR-744-5p, hsa-miR-1537-3p, hsa-miR-29b-3p, hsa-miR-15a-5p, hsa-miR-433-3p, hsa-miR-15a-3p, hsa-miR-629-5p, hsa-miR-103a-2-5p, hsa-let-7i-5p | 83.89 | 96.67 | 73.33 |
| 2807 | hsa-miR-369-3p, hsa-miR-34a-5p, hsa-miR-32-3p, hsa-miR-202-5p, hsa-miR-410-3p, hsa-miR-584-5p, hsa-miR-744-5p, hsa-miR-1537-3p, hsa-miR-29b-3p, hsa-miR-340-5p, hsa-miR-15a-5p, hsa-miR-433-3p, hsa-miR-15a-3p, hsa-let-7i-5p | 83.61 | 89.60 | 73.33 |
| 2808 | hsa-miR-424-5p, hsa-miR-34a-5p, hsa-miR-584-5p, hsa-miR-744-5p, hsa-miR-150-5p, hsa-miR-590-5p, hsa-miR-433-3p, hsa-miR-1296-5p, hsa-miR-103a-2-5p | 83.61 | 87.62 | 60.00 |
| 2809 | hsa-miR-424-5p, hsa-miR-32-3p, hsa-miR-202-5p, hsa-miR-410-3p, hsa-miR-584-5p, hsa-miR-744-5p, hsa-miR-15a-5p, hsa-miR-433-3p, hsa-miR-629-5p, hsa-let-7i-5p | 83.61 | 88.17 | 73.33 |
| 2810 | hsa-miR-369-3p, hsa-miR-128-3p, hsa-miR-424-5p, hsa-miR-34a-5p, hsa-miR-202-5p, hsa-miR-664a-3p, hsa-miR-150-5p, hsa-miR-590-5p, hsa-miR-29c-5p, hsa-miR-545-5p, hsa-let-7i-5p | 83.61 | 92.46 | 70.00 |
| 2811 | hsa-miR-128-3p, hsa-miR-424-5p, hsa-miR-202-5p, hsa-miR-584-5p, hsa-miR-744-5p, hsa-miR-1537-3p, hsa-miR-29b-3p, hsa-miR-505-5p, hsa-miR-433-3p, hsa-miR-29c-5p, hsa-miR-15a-3p, hsa-miR-545-5p, hsa-miR-103a-2-5p, hsa-let-7i-5p | 83.61 | 94.37 | 70.00 |
| 2812 | hsa-miR-424-5p, hsa-miR-34a-5p, hsa-miR-32-3p, hsa-miR-202-5p, hsa-miR-410-3p, hsa-miR-584-5p, hsa-miR-744-5p, hsa-miR-29b-3p, hsa-miR-433-3p, hsa-miR-629-5p, hsa-miR-103a-2-5p, hsa-let-7i-5p | 83.33 | 92.14 | 70.00 |
| 2813 | hsa-miR-424-5p, hsa-miR-32-3p, hsa-miR-202-5p, hsa-miR-410-3p, hsa-miR-584-5p, hsa-miR-744-5p, hsa-miR-1537-3p, hsa-miR-29b-3p, hsa-miR-15a-5p, hsa-miR-433-3p, hsa-miR-15a-3p, hsa-miR-103a-2-5p, hsa-let-7i-5p | 83.33 | 87.14 | 70.00 |
| 2814 | hsa-miR-369-3p, hsa-miR-34a-5p, hsa-miR-410-3p, hsa-miR-584-5p, hsa-miR-744-5p, hsa-miR-150-5p, hsa-miR-29b-3p, hsa-miR-590-5p, hsa-miR-340-5p, hsa-miR-505-5p, hsa-miR-433-3p, hsa-miR-29c-5p, hsa-miR-15a-3p, hsa-miR-629-5p, hsa-miR-545-5p, hsa-miR-1296-5p | 83.33 | 87.14 | 90.00 |
| 2815 | hsa-miR-424-5p, hsa-miR-34a-5p, hsa-miR-32-3p, hsa-miR-202-5p, hsa-miR-410-3p, hsa-miR-584-5p, hsa-miR-744-5p, hsa-miR-150-5p, hsa-miR-29b-3p, hsa-miR-433-3p, hsa-miR-629-5p, hsa-miR-103a-2-5p, hsa-let-7i-5p | 83.33 | 90.48 | 70.00 |
| 2816 | hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-150-5p, hsa-miR-340-5p, hsa-miR-433-3p, hsa-miR-15a-3p, hsa-miR-545-5p, hsa-let-7i-5p | 83.33 | 91.03 | 70.00 |
| 2817 | hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-15a-5p, hsa-miR-433-3p, hsa-miR-629-5p, hsa-miR-545-5p, hsa-let-7i-5p | 83.33 | 89.13 | 73.33 |
| 2818 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-505-5p, hsa-miR-29c-5p, hsa-miR-15a-3p, hsa-miR-629-5p, hsa-let-7i-5p, hsa-miR-652-5p | 81.94 | 86.51 | 83.33 |
| 2819 | hsa-miR-128-3p, hsa-miR-34a-5p, hsa-miR-32-3p, hsa-miR-202-5p, hsa-miR-664a-3p, hsa-miR-744-5p, hsa-miR-1537-3p, hsa-miR-340-5p, hsa-miR-29c-5p, hsa-miR-148a-3p, hsa-miR-652-5p | 81.67 | 83.57 | 60.00 |
| 2820 | hsa-miR-369-3p, hsa-miR-128-3p, hsa-miR-32-3p, hsa-miR-584-5p, hsa-miR-744-5p, hsa-miR-1537-3p, hsa-miR-505-5p, hsa-miR-433-3p, hsa-miR-15a-3p, hsa-let-7i-5p | 81.67 | 89.05 | 66.67 |
| 2821 | hsa-miR-128-3p, hsa-miR-424-5p, hsa-miR-32-3p, hsa-miR-202-5p, hsa-miR-584-5p, hsa-miR-664a-3p, hsa-miR-744-5p, hsa-miR-1537-3p, hsa-miR-29b-3p, hsa-miR-15a-5p, hsa-miR-15a-3p, hsa-miR-629-5p, hsa-miR-103a-2-5p, hsa-let-7i-5p | 81.67 | 92.62 | 73.33 |
| 2822 | hsa-miR-128-3p, hsa-miR-32-3p, hsa-miR-202-5p, hsa-miR-584-5p, hsa-miR-744-5p, hsa-miR-1537-3p, hsa-miR-433-3p, hsa-miR-15a-3p, hsa-miR-103a-2-5p, hsa-let-7i-5p | 81.67 | 90.95 | 56.67 |
| 2823 | hsa-miR-369-3p, hsa-miR-128-3p, hsa-miR-32-3p, hsa-miR-584-5p, hsa-miR-744-5p, hsa-miR-1537-3p, hsa-miR-15a-5p, hsa-miR-505-5p, hsa-miR-433-3p, hsa-miR-15a-3p, hsa-let-7i-5p | 81.67 | 93.81 | 73.33 |
| 2824 | hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-15a-5p, hsa-miR-433-3p | 81.39 | 83.65 | 90.00 |
| 2825 | hsa-miR-128-3p, hsa-miR-424-5p, hsa-miR-202-5p, hsa-miR-584-5p, hsa-miR-744-5p, hsa-miR-1537-3p, hsa-miR-15a-5p, hsa-miR-433-3p, hsa-miR-15a-3p, hsa-let-7i-5p | 81.39 | 93.02 | 56.67 |
| 2826 | hsa-miR-128-3p, hsa-miR-424-5p, hsa-miR-34a-5p, hsa-miR-202-5p, hsa-miR-584-5p, hsa-miR-1537-3p, hsa-miR-150-5p, hsa-miR-590-5p, hsa-miR-433-3p, hsa-miR-29c-5p, hsa-miR-148a-3p, hsa-miR-629-5p, hsa-miR-652-5p | 81.39 | 92.70 | 73.33 |
| 2827 | hsa-miR-128-3p, hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-15a-5p, hsa-miR-433-3p, hsa-miR-629-5p | 81.39 | 81.98 | 73.33 |
| 2828 | hsa-miR-424-5p, hsa-miR-32-3p, hsa-miR-202-5p, hsa-miR-410-3p, hsa-miR-744-5p, hsa-miR-15a-5p, hsa-miR-433-3p, hsa-miR-29c-5p, hsa-miR-629-5p, hsa-let-7i-5p | 81.39 | 92.38 | 73.33 |
| 2829 | hsa-miR-128-3p, hsa-miR-424-5p, hsa-miR-34a-5p, hsa-miR-32-3p, hsa-miR-584-5p, hsa-miR-150-5p, hsa-miR-29b-3p, hsa-miR-15a-5p, hsa-miR-505-5p, hsa-miR-103a-2-5p | 81.39 | 94.05 | 70.00 |
| 2830 | hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-15a-5p, hsa-miR-433-3p, hsa-miR-629-5p, hsa-let-7i-5p | 81.11 | 89.05 | 73.33 |
| 2831 | hsa-miR-424-5p, hsa-miR-32-3p, hsa-miR-202-5p, hsa-miR-584-5p, hsa-miR-744-5p, hsa-miR-150-5p, hsa-miR-15a-5p, hsa-miR-433-3p, hsa-miR-29c-5p, hsa-miR-629-5p, hsa-let-7i-5p | 80.83 | 88.25 | 63.33 |
| 2832 | hsa-miR-128-3p, hsa-miR-424-5p, hsa-miR-32-3p, hsa-miR-202-5p, hsa-miR-410-3p, hsa-miR-584-5p, hsa-miR-664a-3p, hsa-miR-744-5p, hsa-miR-1537-3p, hsa-miR-29b-3p, hsa-miR-15a-5p, hsa-miR-15a-3p, hsa-miR-1296-5p, hsa-miR-103a-2-5p, hsa-let-7i-5p | 80.83 | 82.62 | 60.00 |
| 2833 | hsa-miR-369-3p, hsa-miR-664a-3p, hsa-miR-744-5p, hsa-miR-29b-3p, hsa-miR-590-5p, hsa-miR-15a-5p, hsa-miR-505-5p, hsa-miR-29c-5p, hsa-miR-15a-3p, hsa-miR-629-5p, hsa-miR-103a-2-5p | 80.83 | 84.29 | 60.00 |
| 2834 | hsa-miR-128-3p, hsa-miR-34a-5p, hsa-miR-32-3p, hsa-miR-410-3p, hsa-miR-664a-3p, hsa-miR-744-5p, hsa-miR-1537-3p, hsa-miR-29b-3p, hsa-miR-340-5p, hsa-miR-15a-5p, hsa-miR-29c-5p, hsa-miR-148a-3p, hsa-miR-1296-5p, hsa-let-7i-5p, hsa-miR-652-5p | 79.44 | 87.22 | 63.33 |
| 2835 | hsa-miR-34a-5p, hsa-miR-202-5p, hsa-miR-584-5p, hsa-miR-664a-3p, hsa-miR-744-5p, hsa-miR-150-5p, hsa-miR-505-5p, hsa-miR-433-3p, hsa-miR-629-5p, hsa-miR-545-5p, hsa-miR-1296-5p, hsa-miR-652-5p | 79.17 | 83.10 | 63.33 |
| 2836 | hsa-miR-369-3p, hsa-miR-128-3p, hsa-miR-424-5p, hsa-miR-34a-5p, hsa-miR-32-3p, hsa-miR-744-5p, hsa-miR-1537-3p, hsa-miR-590-5p, hsa-miR-340-5p, hsa-miR-433-3p, hsa-miR-15a-3p | 78.89 | 87.94 | 43.33 |
| 2837 | hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-15a-5p, hsa-miR-433-3p, hsa-miR-29c-5p, hsa-miR-629-5p, hsa-let-7i-5p | 78.61 | 86.59 | 63.33 |
| 2838 | hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-29b-3p, hsa-miR-15a-5p, hsa-miR-433-3p, hsa-miR-629-5p, hsa-miR-103a-2-5p, hsa-let-7i-5p | 78.61 | 93.33 | 70.00 |
| 2839 | hsa-miR-424-5p, hsa-miR-32-3p, hsa-miR-202-5p, hsa-miR-410-3p, hsa-miR-584-5p, hsa-miR-744-5p, hsa-miR-150-5p, hsa-miR-15a-5p, hsa-miR-433-3p, hsa-miR-629-5p | 78.61 | 80.08 | 53.33 |
| 2840 | hsa-miR-424-5p, hsa-miR-32-3p, hsa-miR-202-5p, hsa-miR-410-3p, hsa-miR-744-5p, hsa-miR-150-5p, hsa-miR-15a-5p, hsa-miR-433-3p, hsa-miR-629-5p | 76.94 | 82.22 | 73.33 |
| 2841 | hsa-miR-369-3p, hsa-miR-128-3p, hsa-miR-34a-5p, hsa-miR-32-3p, hsa-miR-410-3p, hsa-miR-744-5p, hsa-miR-150-5p, hsa-miR-505-5p, hsa-miR-433-3p, hsa-miR-629-5p | 76.67 | 88.41 | 63.33 |
| 2842 | hsa-miR-424-5p, hsa-miR-34a-5p, hsa-miR-32-3p, hsa-miR-202-5p, hsa-miR-410-3p, hsa-miR-744-5p, hsa-miR-150-5p, hsa-miR-433-3p, hsa-miR-629-5p | 76.67 | 82.86 | 63.33 |
| 2843 | hsa-miR-369-3p, hsa-miR-32-3p, hsa-miR-584-5p, hsa-miR-744-5p, hsa-miR-150-5p, hsa-miR-505-5p, hsa-miR-433-3p, hsa-miR-629-5p, hsa-miR-1296-5p, hsa-miR-103a-2-5p, hsa-let-7i-5p | 76.67 | 89.60 | 66.67 |
| 2844 | hsa-miR-369-3p, hsa-miR-424-5p, hsa-miR-32-3p, hsa-miR-202-5p, hsa-miR-410-3p, hsa-miR-744-5p, hsa-miR-150-5p, hsa-miR-15a-5p, hsa-miR-433-3p, hsa-miR-629-5p | 76.67 | 86.19 | 63.33 |
| 2845 | combination 2591 + rs13335818 | 74.44 | 49.29 | 57.31 |
| 2846 | combination 2592 + rs11647727 | 83.61 | 85.32 | 75.17 |
| 2847 | combination 2595 + rs4297685 | 95.28 | 98.33 | 85.85 |
| 2848 | combination 2596 + rs4297685 | 95.28 | 96.9 | 87.82 |
| 2849 | combination 2597 + rs4297685 | 95.28 | 98.33 | 89.49 |
| 2850 | combination 2598 + rs13335818 | 93.06 | 98.33 | 89.37 |
| 2851 | combination 2598 + rs4293393 | 90.83 | 90.71 | 80 |
| 2852 | combination 2599 + rs4297685 | 95.28 | 98.57 | 89.31 |
| 2853 | combination 2599 + rs4293393 | 90.83 | 90.71 | 86.51 |
| 2854 | combination 2600 + rs13335818 | 97.5 | 100 | 89.31 |
| 2855 | combination 2600 + rs4293393 | 90.83 | 91.03 | 83.44 |
| 2856 | combination 2601 + rs13335818 | 93.06 | 98.57 | 94.09 |
| 2857 | combination 2601 + rs4293393 | 93.06 | 93.81 | 87.15 |
| 2858 | combination 2602 + rs4297685 | 95.28 | 98.57 | 97.66 |
| 2859 | combination 2603 + rs11647727 | 90.83 | 98.33 | 99.14 |
| 2860 | combination 2604 + rs13335818 | 93.06 | 100 | 99.14 |
| 2861 | combination 2605 + rs11647727 | 90.83 | 96.9 | 99.14 |
| 2862 | combination 2617 + rs11647727 | 85.56 | 87.62 | 99.14 |
| 2863 | combination 2818 + rs11647727 | 83.33 | 87.62 | 99.08 |
| 2864 | combination 2819 + rs11647727 | 83.33 | 87.62 | 95.63 |
| 2865 | combination 2591 + rs4293393 | 74.44 | 49.29 | 58.92 |

Through statistical analysis, the inventors demonstrated that UMOD variants are significantly associated with renal mass presence, malignancy, and aggressiveness in patients. The present data also revealed multiple potential biomarkers (miRNAs) of presence, stage, grade, and metastatic potential of RCC. The combined use of genetic and miRNAs data, together with clinical parameters, could have the unique potential to aid patient management in the setting of screening, diagnosis and therapeutic decision-making with respect to surgical and medical strategy and prognostic. The use of such combined biomarkers panel can lead to a tailored and personalized treatment to avoid unnecessary and associated wasteful costs. Then the present molecular tool can reduce the burden on public and private health care system.

### BIBLIOGRAFIC REFERENCES

- Adams KF, Leitzmann MF, Albanes D, et al. Body size and renal cell cancer incidence in a large US cohort study. Am J Epidemiol., 2008; 168(3):268-277.
- Alam NA, Bevan S, Churchman M, et al. Localization of a gene (MCUL1) for multiple cutaneous leiomyomata and uterine fibroids to chromosome 1q42.3-q43. Am J Hum Genet., 2001; 68(5):1264-1269.
- Babaian KN, Merrill MM, Matin S, et al. Partial nephrectomy in the setting of metastatic renal cell carcinoma. J Urol., 2014; 192(1):36-42.
- Bachmann S, Koeppen-Hagemann I, Kriz W. Ultrastructural localization of TammHorsfall glycoprotein (THP) in rat kidney as revealed by protein A-gold immunocytochemistry. Histochemistry., 1985; 83(6):531-538.
- Baek D, et al. The impact of microRNAs on protein output. Nature, 2008; 455:64-71.
- Baillargeon-Gagné S, Jeldres C, Lughezzani G, et al. A comparative population based analysis of the rate of partial vs radical nephrectomy for clinically localized renal cell carcinoma. BJU Int., 2010; 105(3):359-364.
- Bartel D P. MicroRNAs: genomics, biogenesis, mechanism, and function. Cell. 2004 Jan. 23; 116(2):281-97.
- Bates JM, Raffi HM, Prasadan K, et al. Tamm-Horsfall protein knockout mice are more prone to urinary tract infection: rapid communication. Kidney Int., 2004; 65(3):791-797.
- Bergström a, Hsieh CC, Lindblad P, Lu CM, Cook NR, Wolk a. Obesity and renal cell cancer--a quantitative review. Br J Cancer., 2001; 85(7):984-990.
- Bex A, Albiges L, Ljungberg B, et al. Updated European Association of Urology Guidelines for Cytoreductive Nephrectomy in Patients with Synchronous Metastatic Clear-cell Renal Cell Carcinoma., J Immunother Cancer. 2018 Jan 22;6(1):5.
- Bex A, Ljungberg B., Comparing Everolimus to Sunitinib in Non-clear-cell Renal Cell Carcinoma.Eur Urol. 2016 May;69(5):875-6.
- Bhindi B, Thompson RH, Lohse CM, et al. The Probability of Aggressive Versus Indolent Histology Based on Renal Tumor Size: Implications for Surveillance and Treatment.Eur Urol. 2018 Oct;74(4):489-497.
- Bleyer AJ, Ẑivná M, Kmoch S. Uromodulin-associated kidney disease. Nephron - ClinPract., 2010; 118(1).
- Bracken CP, Scott HS & Goodall G.J. A network-biology perspective of microRNA function and dysfunction in cancer. Nat. Rev. Genet., 2016; 17:719-732.
- Brookman-May SD, May M, Wolff et al. CORONA Project; European Association of Urology (EAU) Young Academic Urologists (YAU) Renal Cancer Group. Evaluation of the prognostic significance of perirenal fat invasion and tumor size in patients with pT1-pT3a localized renal cell carcinoma in a comprehensive multicenter study of the CORONA project. Can we improve prognostic discrimination for patients with stage pT3a tumors? Eur Urol. 2015 May;67(5):943-51.
- Bui MH, Zisman A, Pantuck AJ et al. Prognostic factors and molecular markers for renal cell carcinoma. Expert Rev Anticancer Ther. 2001, 1.4: 565-575.
- Calin G A, Croce C M. MicroRNA-cancer connection: the beginning of a new tale. Cancer research. 2006 Aug. 1; 66(15):7390-4.
- Calin G A, Ferracin M, Cimmino A, Di Leva G, Shimizu M, Wojcik S E, et al. A MicroRNA signature associated with prognosis and progression in chronic lymphocytic leukemia. The New England journal of medicine. 2005 Oct. 27; 353(17):1793-801.
- Capitanio U, Terrone C, Antonelli A, et al. Nephron-sparing techniques independently decrease the risk of cardiovascular events relative to radical nephrectomy in patients with a T1a-T1b renal mass and normal preoperative renal function. Eur Urol., 2015; 67(4):683-689.
- ChanudetE,Wozniak M B, Bouaoun L, Byrnes G, et al. Large-scale genome-wide screening of circulating microRNAs in clear cell renal cell carcinoma reveals specific signatures in late-stage disease. International journal of cancer, 2017; 141.9:1730-1740.
- Cho E, Curhan G, Hankinson SE, et al. Prospective evaluation of analgesic use and risk of renal cell cancer. Arch Int Med., 2013; 171(16):1487-1493.
- Chow W, Dong L, Devesa S. Epidemiology and risk factors for kidney cancer. Nat Rev Urol. 2010; 7(5):245-257.
- Cumberbatch MG, Rota M, Catto JWF, La Vecchia C. The Role of Tobacco Smoke in Bladder and Kidney Carcinogenesis: A Comparison of Exposures and Metaanalysis of Incidence and Mortality Risks. Eur Urol., 2016; 70(3):458-466.
- D'Avella C, Abbosh P, Pal SK, et al, Mutations in renalcell carcinoma UrolOncol. 2018 Nov 23. pii: S1078-1439(18)30436-8.
- Delahunt B, Cheville JC, Martignoni G, Humphrey PA, Magi-Galluzzi C, McKenney J, et al. The International Society of Urological Pathology (ISUP) Grading System for Renal Cell Carcinoma and Other Prognostic Parameters. The American Journal of Surgical Pathology, 2013; 37:1490-504.
- Dellavalle CT, Daniel CR, Aschebrook-Kilfoy B, et al. Dietary intake of nitrate and nitrite and risk of renal cell carcinoma in the NIH-AARP Diet and Health Study. Br J Cancer., 2013; 108(1):205-212.
- Denby, L. et al. MicroRNA-214 Antagonism Protects against Renal Fibrosis. J. Am. Soc. Nephrol., 2014; 25:65-80.
- Edge S, Byrd DR, Compton CC, Fritz AG, Greene FL, Trotti A. AJCC Cancer Staging Manual. Springer; 2010: 718.
- Eichhorn SW, et al. mRNA Destabilization Is the Dominant Effect of Mammalian MicroRNAs by the Time Substantial Repression Ensues. Mol. Cell, 2014; 56:104-115.
- El-achkar TM, Wu X. Uromodulin in Kidney Injury: An Instigator, Bystander, or Protector? Am J Kidney Dis., 2012; 59(3):452-461.
- Esquela-Kerscher A, Slack F J. Oncomirs-microRNAs with a role in cancer. Nature reviews. 2006 April; 6(4):259-69.
- Fedorko M, Stanik M, Iliev R, et al. Combination of MiR-378 and MiR-210 serum levels enables sensitive detection of renal cell carcinoma. IntJMolSci, 2015; 16:23382-9.
- Ferlay J, Steliarova-Foucher E, Lortet-Tieulent J, et al. Cancer incidence and mortality patterns in Europe: Estimates for 40 countries in 2012. Eur J Cancer., 2013; 49(6):1374-1403.
- Ficarra V, Novara G, Secco S, et al. Preoperative Aspects and Dimensions Used for an Anatomical (PADUA) Classification of Renal Tumours in Patients who are Candidates for Nephron-Sparing Surgery. Eur Urol., 2009; 56(5):786-793.
- Graham LA, Padmanabhan S, Fraser NJ, et al. Validation of uromodulin as a candidate gene for human essential hypertension. Hypertension., 2014; 63(3):551558.
- Gresh L, Fischer E, Reimann A, et al. A transcriptional network in polycystic kidney disease. EMBO J., 2004; 23(7):1657-1668.
- Gudbjartsson, Daniel F., et al. "Association of variants at UMOD with chronic kidney disease and kidney stones-role of age and comorbid diseases." PLoS genetics 6.7 (2010): e1001039
- Hauser S, Wulfken LM, Holdenrieder S, et al. Analysis of serum microRNAs (miR-26a-2*, miR-191, miR-337-3p and miR-378) as potential biomarkers in renal cell carcinoma. Cancer Epidemiol,2012; 36:391-4.
- Heidenreich A, Ravery V. Preoperative imaging in renal cell cancer. World J Urol., 2004; 22:307-315.
- Hiroki E, Akahira J, Suzuki F, Nagase S, Ito K, Suzuki T, et al. Changes in microRNA expression levels correlate with clinicopathological features and prognoses in endometrial serous adenocarcinomas. Cancer science. 2010 January; 101(1):241-9.
- Hollingsworth JM, Miller DC, Daignault S, Hollenbeck BK. Five-year survival after surgical treatment for kidney cancer: A population-based competing risk analysis. Cancer., 2007; 109(9):1763-1768.
- Hunter MP, Ismail N, Zhang X, Aguda BD, Lee EJ, Yu L, et al. Detection of microRNA expression in human peripheral blood microvesicles. PLoS ONE, 2008; 3:e3694.
- Hunter MP, Ismail N, Zhang X, Aguda BD, Lee EJ, Yu L, et al. Detection of microRNA expression in human peripheral blood microvesicles. PLoS ONE 2008;3:e3694.
- Ibragimova I, Maradeo ME, Dulaimi E, Cairns P. Aberrant promoter hypermethylation of PBRM1, BAP1, SETD2, KDM6A and other chromatin-modifying genes is absent or rare in clear cell RCC. Epigenetics., 2013; 8(5):486-493.
- lorio MV, Croce CM. MicroRNA dysregulation in cancer: diagnostics, monitoring and therapeutics. A comprehensive review. EMBO Mol Med, 2012; 4:143-59.
- Iwamoto H, Kanda Y, Sejima T, et al. Serum miR-210 as a potential biomarker of early clear cell renal cell carcinoma. Int J Oncol, 2014; 44:53-8.
- Kabat GC, Silvera S a N, Miller a B, Rohan TE. A cohort study of reproductive and hormonal factors and renal cell cancer risk in women. Br J Cancer., 2007; 96(5):845849.
- Keith D, Torres V, King B. Renal cell carcinoma in autosomal dominant polycystic kidney disease. J Am SocNephrol., 1994; 4 (9): 1661-1669.
- Keller A, Leidinger P, Bauer A, Elsharawy A, Haas J, Backes C, et al. Toward the blood-borne miRNome of human diseases. Nat Methods, 2011; 8:841-3.
- Kimchi-Sarfaty C, Oh JM, Kim IW, Sauna ZE, Calcagno AM, Ambudkar SV, Gottesman MM: A "silent" polymorphism in the MDR1 gene changes substrate specificity. Science 2007, 315:525-528
- Kottgen A, Hwang S-J, Larson MG, et al. Uromodulin Levels Associate with a Common UMOD Variant and Risk for Incident CKD. J Am SocNephrol., 2010; 21(2):337-344.
- Krol J, Loedige I &Filipowicz W. The widespread regulation of microRNA biogenesis, function and decay. Nat. Rev. Genet., 2010; 11, 597.
- Kutikov A, Uzzo RG. The R.E.N.A.L. Nephrometry Score: A Comprehensive Standardized System for Quantitating Renal Tumor Size, Location and Depth. J Urol., 2009; 182(3):844-853.
- Lalani AA, Xie W, Martini DJ et al, Change in Neutrophil-to-lymphocyte ratio (NLR) in response to immune checkpoint blockade for metastatic renal cell carcinoma, J Immunother Cancer. 2018 Jan 22;6(1):5.
- Leibovich BC, Lohse CM, Crispen PL, et al. Histological subtype is an independent predictor of outcome for patients with renal cell carcinoma. J Urol. 2010 Apr;183(4):1309-15.
- Levi F, Ferlay J, Galeone C, et al. The changing pattern of kidney cancer incidence and mortality in Europe. BJU Int. 2008; 101(8):949-958.
- Li M, Wang Y, Song Y, et al. MicroRNAs in renal cell carcinoma: a systematic review of clinical implications (review). Oncol Rep, 2015; 33: 1571-8.
- Lim LP et al. Microarray analysis shows that some microRNAs downregulate large numbers of target mRNAs. Nature, 2005; 433, 769-773.
- Linehan WM, Pinto P, Srinivasan R, et al. Innovations and Challenges in Renal Cancer Identification of the Genes for Kidney Cancer: Opportunity for DiseaseSpecific Targeted Therapeutics. Clin Cancer Res., 2007; 13:671-680.
- Lipworth L, Tarone RE, McLaughlin JK. The Epidemiology of Renal Cell Carcinoma. J Urol., 2006; 176(6):2353-2358.
- Ljungberg B, Bensalah K, Canfield S, et al. EAU guidelines on renal cell carcinoma: 2014 update. Eur Urol., 2015; 67(5):913-924.
- Ljungberg B, Hedin O, Lundstam S et al. Nephron Sparing Surgery Associated With Better Survival Than Radical Nephrectomy in Patients Treated for Unforeseen Benign Renal Tumors, UrolOncol. 2018 Nov 23. pii: S1078-1439(18)30436-8.
- Lorenzen JM &Thum T. Circulating and Urinary microRNAs in Kidney Disease. Clin. J. Am. Soc. Nephrol., 2012; 7:1528-1533.
- Lytle JR, Yario TA &Steitz JA. Target mRNAs are repressed as efficiently by microRNA-binding sites in the 5' UTR as in the 3' UTR. Proc. Natl. Acad. Sci., 2017; 104:9667- 9672.
- Michael M Z, S M O C, van Hoist Pellekaan N G, Young G P, James R J. Reduced accumulation of specific microRNAs in colorectal neoplasia. MolCancer Res. 2003 October; 1(12):882-91.
- Mitchell PS, Parkin RK, Kroh EM, et al. Circulating microRNAs as stable blood-based markers for cancer detection. Proc Natl AcadSci U S A, 2008; 105:10513-8.
- Mo L, Huang HY, Zhu XH, Shapiro E, Hasty DL, Wu XR. Tamm-Horsfall protein is a critical renal defense factor protecting against calcium oxalate crystal formation. Kidney Int., 2004; 66(3):1159-1166.
- Mooney C, Raoof R, El-Naggar H, Sanz-Rodriguez A,et al. High throughput qPCR expression profiling of circulating MicroRNAs reveals minimal sex-and sample timing-related variation in plasma of healthy volunteers.PloSone, 2015; 10.12: e0145316.
- Muchmore a V, Decker JM. Uromodulin: a unique 85-kilodalton immunosuppressive glycoprotein isolated from urine of pregnant women. Science., 1985; 229(4712):479-481.
- Nackley AG, Shabalina SA, Tchivileva IE, Satterfield K, Korchynskyi O, Makarov SS, Maixner W, Diatchenko L: Human catechol-O-methyltransferase haplotypes modulate protein expression by altering mRNA secondary structure. Science 2006, 314:1930-1933
- Nini A, Larcher A, CazzanigaW,et al. The side and the location of the primary tumor does not affect the probability of lymph node invasion in patients with renal cell carcinoma. World J Urol. 2018 Nov 24 1-7.
- Pal SK, Ali SM, Yakirevich E et al. Characterization of Clinical Cases of Advanced Papillary Renal Cell Carcinoma via Comprehensive Genomic Profiling. Eur Urol. 2018 Jan;73(1):71-78.
- Pantuck a J, Zisman a, Belldegrun AS. The changing natural history of renal cell carcinoma. J Urol., 2001; 166(5):1611-1623.
- Pennica D, Kohr WJ, Kuang WJ, et al. Identification of human uromodulin as the Tamm-Horsfall urinary glycoprotein. Science., 1987; 236(4797):83-88.
- Prajczer S, Heidenreich U, Pfaller W, Kotanko P, Lhotta K, Jennings P. Evidence for a role of uromodulin in chronic kidney disease progression. NephrolDialTransplant. 2010; 25(6):1896-1903.
- Rampoldi L, Caridi G, Santon D, et al. Allelism of MCKD, FJHN and GCKD caused by impairment of uromodulin export dynamics. Hum MolGenet., 2003; 12(24):33693384.
- Rampoldi L, Scolari F, Amoroso A, Ghiggeri G, Devuyst O. The rediscovery of uromodulin (Tamm-Horsfall protein): from tubulointerstitial nephropathy to chronic kidney disease. Kidney Int., 2011; 80(4):338-347.
- Redova M, Poprach A, Nekvindova J, et al. Circulating miR-378 and miR-451 in serum are potential biomarkers for renal cell carcinoma. J Transl Med 2012; 10:55.
- Richard PO, Jewett MAS, Bhatt JR, et al. Renal Tumor Biopsy for Small Renal Masses: A Single-center 13-year Experience. Eur Urol., 2015; 68(6):1007-1013.
- Ricketts C, Woodward ER, Killick P, et al. Germline SDHB mutations and familial renal cell carcinoma. Journal of the National Cancer Institute, 2008; 100.17: 1260-1262.
- Ritchie ME, Phipson B, Wu D, Hu Y, Law CW, Shi W, et al. limma powers differential expression analysesfor RNA-sequencing and microarray studies. NucleicAcidsResearch. 2015; 43(7):e47.
- Schaefer A, Stephan C, Busch J, Yousef GM, Jung K. Diagnostic, prognostic and therapeutic implications of microRNAs in urologic tumors. Nat Rev Urol, 2010; 7:286-297.
- Schaeffer C, Santambrogio S, Perucca S, Casari G, Rampoldi L. Analysis of Uromodulin Polymerization Provides New Insights into the Mechanisms Regulating ZP Domain-mediated Protein Assembly. MolBiol Cell., 2009; 82(4):327-331.
- Siegel R, Naishadham D, Jemal A. Cancer Statistics , 2017. CA Cancer J Clin., 2012; 62(1):10-29.
- Siemer S, Hack M, Lehmann J, Becker F, Stöckle M. Outcome of renal tumors in young adults. J Urol. 2006; 175(4):1240-3-4.
- Taneja K, Arora S, Rogers CG, Gupta et al. Pathological staging of renal cell carcinoma: a review of 300 consecutive cases with emphasis on retrograde venous invasion. Histopathology. 2018 Oct; 73(4):681-691.
- Tang K, Xu H. Prognostic value of metasignature miRNAs in renal cell carcinoma: an integrated miRNA expression profiling analysis. Sci Rep, 2015; 5:10272.
- Teixeira AL, Ferreira M, Silva J, et al. Higher circulating expression levels of miR-221 associated with poor overall survival in renal cell carcinoma patients. Tumour Biol, 2014; 35:4057-66.
- Thompson RH, Ordonez MA, lasonos A, et al. Renal cell carcinoma in young and old patients--is there a difference? J Urol., 2008; 180(4):1262-6.
- Torffvit O, Melander O, Hulten UL. Urinary excretion rate of Tamm-Horsfall protein is related to salt intake in humans. NephronPhysiol., 2004; 97(1):p31-6.
- Trevisani F. et al. MicroRNA 193b-3p as a predictive biomarker of chronic kidney disease in patients undergoing radical nephrectomy for renal cell carcinoma. Br. J. Cancer, 2016; 115, 1343-1350.
- Trevisani, Francesco, et al. "The association of uromodulin genotype with renal cancer aggressiveness." European urology focus (2017).
- Trionfini P, Benigni A, Remuzzi G. MicroRNAs in kidneyphysiology and disease. Nature Reviews Nephrology, 2015; 11:23-33.
- Trudu M, Janas S, Lanzani C, et al. Common noncoding UMOD gene variants induce salt-sensitive hypertension and kidney damage by increasing uromodulin expression. Nat Med. 2014; 19(12):1-19.
- Tsivian M, Moreira DM, Caso JR, Mouraviev V, Polascik TJ. Cigarette smoking is associated with advanced renal cell carcinoma. J ClinOncol., 2011; 29(15):20272031.
- Vaz-Drago, R., Custódio, N., & Carmo-Fonseca, M. (2017). Deep intronic mutations and human disease. Human genetics, 136(9), 1093-1111
- Valdés Olmos RA, de Geus-Oei LF, TichelerCH et al.From Incidentaloma to Oncocytoma: A Role for Hybrid Molecular Imaging in Characterising Renal Masses? Eur Urol. 2016 Mar;69(3):417-8.
- Volinia S, Calin G A, Liu C G, Ambs S, Cimmino A, Petrocca F, et al. A microRNA expression signature of human solid tumors defines cancer gene targets. Proceedings of the National Academy of Sciences of the United States of America. 2006 Feb. 14; 103(7):2257-61.
- Wagener N, Edelmann D, Benner A, et al. European Association of Urology (EAU) Young Academic Urologists (YAU) Kidney Cancer Group. Outcome of papillary versus clear cell renal cell carcinoma varies significantly in non-metastatic disease. PLoS One. 2017 Sep 21;12(9):e0184173.
- Wang C, Hu J, Lu M, et al. A panel of five serum miRNAs as a potential diagnostic tool for earlystage renal cell carcinoma. Sci Rep, 2015; 5:7610.
- Wang K, Yuan Y, Cho JH, et al. Comparing the MicroRNA spectrum between serum and plasma. PLoS One, 2012; 7:e41561.
- Wein, Kavoussi, Novick, Partin, Peters. Campbell-Walsh Urology. 10th Ed. (Saunders Elsevier, ed.). Philadelphia; 2012.
- Weng, L. et al. MicroRNA profiling of clear cell renal cell carcinoma by wholegenome small RNA deep sequencing of paired frozen and formalin-fixed, paraffin-embedded tissue specimens. J Pathol, 2010; 222:41-51.
- Wulfken LM, Moritz R, Ohlmann C, et al. Micro-RNAs in renal cell carcinoma: diagnostic implications of serum miR-1233 levels. PLoSOne, 2011; 6:e25787.
- Yamaguchi, N. et al. Identification of MicroRNAs Involved in Resistance to Sunitinib in Renal Cell Carcinoma Cells. Anticancer Res., 2017; 37:2985-2992.
- Yanaihara N, Caplen N, Bowman E, Seike M, Kumamoto K, Yi M, et al. Unique microRNA molecular profiles in lung cancer diagnosis and prognosis. Cancer cell. 2006 March; 9(3):189-98.
- Yu S L, Chen H Y, Chang G C, Chen C Y, Chen H W, Singh S, et al. MicroRNA signature predicts survival and relapse in lung cancer. Cancer cell. 2008 January; 13(1):48-57.
- Zbar B, Glenn G, Merino M, et al. Familial renal carcinoma: clinical evaluation, clinical subtypes and risk of renal carcinoma development. J Urol., 2007; 177(2):4615.
- Zhai Q, Zhou L, Zhao C, et al. Identification ofmiR-508-3p and miR-509-3p that are associated with cell invasion and migration and involved in the apoptosis of renal cell carcinoma. BiochemBiophys Res Commun, 2012; 419:621-6.
- Zhang G, Yu Z, Fu S,et al. ERCC6L that is up-regulated in high grade of renal cell carcinoma enhances cell viability in vitro and promotes tumor growth in vivo potentially through modulating MAPK signalling pathway. Cancer Gene Ther. 2018 Nov 21: 1.
- Zhang WB, Pan ZQ, Yang QS, et al. Tumor suppressive miR-509-5p contributes to cell migration, proliferation and antiapoptosis in renal cell carcinoma. Ir J Med Sci, 2013; 182:621-7.
- Zhao A, Li G, Peoc'h M, et al. Serum miR-210 as a novel biomarker for molecular diagnosis of clear cell renal cell carcinoma. ExpMolPathol,2013; 94:115-20.
- Zhu Y, et al. Identification of biomarker microRNAs for predicting the response of colorectal cancer to neoadjuvant chemoradiotherapy based on microRNA regulatory network. Oncotarget, 2016; 8, 2233-2248.
- Znaor A, Lortet-Tieulent J, Laversanne M, Jemal A, Bray F. International variations and trends in renal cell carcinoma incidence and mortality. Eur Urol. 2015; 67(3):519-530.

## Claims

1. A method for the prognosis of RCC comprising measuring the level of at least one miRNA or a combination thereof in a biological sample of the subject and comparing said measured level to a reference level, wherein the at least one miRNA is indicated in Table 12 or Table 13 or Table 14 or Table 22 or Table 23 or is selected from the group consisting of: hsa-miR-744-5p, hsa-miR-4306, hsa-miR-140-3p, hsa-miR-15a-5p, hsa-miR-195-5p, hsa-miR-27b-3p, hsa-miR-30b-5p, hsa-let-7i-5p, hsa-miR-103a-2-5p, hsa-miR-132-3p, hsa-miR-197-3p, hsa-miR-215-5p, hsa-miR-33a-5p, hsa-miR-3613-3p, hsa-miR-377-3p, hsa-miR-590-3p, hsa-miR-598-3p, hsa-miR-629-5p, hsa-miR-664a-3p, hsa-miR-6803-3p, hsa-miR-769-5p, hsa-miR-320a-3p, hsa-miR-100-5p, hsa-miR-183-3p, hsa-miR-224-5p, hsa-let-7i-3p, hsa-miR-652-3, hsa-miR-22-5p, hsa-miR-548a-3p, hsa-miR-1307-5p, hsa-miR-339-3p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p, hsa-miR-181c-5p, hsa-miR-27a-3p, hsa-miR-134-5p, hsa-miR-1296-5p, hsa-miR-125a-5p, hsa-miR-424-3p, hsa-miR-125b-5p, hsa-miR-502-3p, hsa-miR-128-3p, hsa-miR-21-3p, hsa-miR-31-5p, hsa-miR-545-5p, hsa-miR-134-5p, , hsa-miR-652-3, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p, , hsa-miR-501-5p, hsa-miR-424-5p, hsa-miR-144-3p, hsa-let-7d-3p, hsa-miR-548l, hsa-miR-548k, , hsa-miR-153-3p, , hsa-miR-582-5p, hsa-miR-129, hsa-miR-410-3p, hsa-let-7g-3p, hsa-miR-484, hsa-miR-30e-3p, hsa-miR-376c-3p, hsa-miR-545-5p, , hsa-miR-590-5p, hsa-miR-455-3p, hsa-let-7d-3p, hsa-miR-7-1-3p, , hsa-miR-671-5p, hsa-miR-382-5p, hsa-miR-342-3p, hsa-miR-532-5p, hsa-miR-126-3p, hsa-miR-150-5p, hsa-let-7i-3p, hsa-miR-222-3p, hsa-miR-769-5p, hsa-miR-548a-3p, hsa-miR-190a-5p, , hsa-miR-324-3p, hsa-miR-143-3p, hsa-miR-421, hsa-miR-22-5p, , hsa-miR-106b-3p, hsa-miR-335-5p, hsa-miR-31-5p, hsa-miR-941, hsa-miR-32-3p, hsa-miR-503-5p, hsa-miR-30e-5p, hsa-miR-1296-5p, hsa-miR-1249-3p, hsa-miR-100-5p, hsa-miR-181c-5p, hsa-miR-323b-3p, hsa-miR-452-3p, hsa-miR-20b-5p, and hsa-miR-1301-3p, hsa-miR-125b-5p, hsa-miR-148a-3p, hsa-miR-545-5p, hsa-miR-1296-5p, hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-1537-3p, hsa-miR-150-5p, hsa-miR-29b-3p, hsa-miR-590-5p, hsa-miR-340-5p, hsa-miR-505-5p, hsa-miR-433-3p, hsa-miR-29c-5p, and hsa-miR-15a-3p and wherein the combination is indicated in Table 17 or Table 18 or Table 19 or Table 26 or Table 27.

2. The method according to claim 9 further comprising determining the genotype of UMOD SNPs rs11647727, rs12917707, rs12934320, rs12934455, rs13329952, rs13333226, rs13335818, rs28362063, rs28544423, rs28640218, rs28688991, rs34356953, rs4293393, rs60136849, rs71384446, rs7203642, and rs9928757 or of any other UMOD SNPs in linkage disequilibrium with them (preferably with a R² > 0.8) in any ethnic background, preferably rs111266260, rs111483946, rs111904030, rs111992415, rs112166439, rs11342813, rs113452476, rs113923200, rs113957052, rs114997829, rs116563128, rs117774819, rs118059635, rs11862974, rs12102292, rs12922822, rs13334589, rs138755971, rs145279202, rs147946789, rs149233634, rs149786037, rs150044343, rs150052437, rs181711435, rs181820305, rs182188833, rs182605229, rs182744661, rs183590048, rs184483102, rs185128245, rs185578691, rs185630961, rs186260541, rs187620954, rs188045848, rs189908977, rs190450490, rs191065984, rs191171015, rs192180621, rs192280820, rs34115067, rs34262842, rs34857077, rs34882080, rs35650857, rs35830321, rs36060036, rs369494277, rs372456426, rs377257136, rs4238595, rs4997081, rs528859496, rs544951514, rs554722277, rs55808501, rs58768874, rs59027126, rs6497474, rs6497475, rs6497476, rs71149134, rs71149135, rs71377648, rs7193058, rs7204342, rs7204775, rs74011921, rs74011925, rs75864273, rs76563024, rs77225526, rs77875418, rs78052469, rs79245268, rs80142672, rs80159442, rs8060932, rs8062123, rs9923990, rs9924088, rs9926225, rs9928003, rs9928936, rs9933330, and rs9937393.

3. A method to discriminate a subject affected by a low stage RCC from a subject affected by a high stage RCC comprising measuring the level of at least one miRNA or a combination thereof in a biological sample of the subject and comparing said measured level to a reference level, wherein the at least one is indicated in Table 13 or Table 23 or is selected from the group consisting of: hsa-miR-744-5p, hsa-miR-4306, hsa-miR-140-3p, hsa-miR-15a-5p, hsa-miR-195-5p , hsa-miR-27b-3p, hsa-miR-30b-5p, hsa-let-7i-5p, hsa-miR-132-3p, hsa-miR-215-5p, hsa-miR-598-3p, hsa-miR-629-5p, hsa-miR-664a-3p, hsa-miR-769-5p, hsa-miR-320a-3p, hsa-miR-100-5p, hsa-miR-183-3p, hsa-miR-224-5p, hsa-let-7i-3p, hsa-miR-652-3, hsa-miR-22-5p, hsa-miR-548a-3p, hsa-miR-1307-5p, hsa-miR-339-3p, hsa-miR-485-3p, hsa-miR-10b-5p, hsa-miR-12136, hsa-miR-34a-3p, hsa-let-7d-3p, hsa-miR-455-3p, hsa-miR-337-5p, hsa-miR-188-5p, hsa-miR-181c-5p, hsa-miR-27a-3p, hsa-miR-134-5p, hsa-miR-1296-5p, hsa-miR-769-5p, hsa-miR-548a-3p, hsa-miR-190a-5p, , hsa-miR-324-3p, hsa-miR-143-3p, hsa-miR-421, hsa-miR-22-5p, hsa-miR-106b-3p, hsa-miR-335-5p, hsa-miR-31-5p, hsa-miR-941, hsa-miR-32-3p, hsa-miR-503-5p, hsa-miR-30e-5p, hsa-miR-1296-5p, hsa-miR-1249-3p, hsa-miR-100-5p, hsa-miR-181c-5p, hsa-miR-323b-3p, hsa-miR-452-3p, hsa-miR-20b-5p, and hsa-miR-1301-3p and wherein said combination is indicated in Table 17 or Table 26.

4. The method according to claim 3 further comprising determining the genotype of UMOD SNPs rs71384446, rs9928757, rs60136849, rs11647727, rs34356953, rs12934320, rs12934455, rs28640218, rs28544423, rs13335818, rs4293393, rs28362063, rs13333226, rs13329952, rs7203642, rs28688991, and rs12917707 or of any other UMOD SNPs in linkage disequilibrium with them (preferably with a R² > 0.8) in any ethnic background, preferably said other UMOD SNPs is selected from the group consisting of: rs12922822, rs9933330, rs13334589, rs34115067, rs9928936, rs71149134, rs7204775, rs71377648, rs9924088, rs9923990, rs9926225, rs111483946, rs112166439, rs113923200, rs554722277, rs111992415, rs544951514, rs113957052, rs111266260, rs192180621, rs12102292, rs116563128, rs145279202, rs9937393, rs150044343, rs7204342, rs80159442, rs11342813, rs11862974, rs6497476, rs76563024, rs79245268, rs77875418, rs80142672, rs190450490, rs59027126, rs58768874, rs118059635, rs138755971, rs189908977, rs8060932, rs8062123, rs4238595, rs7193058, rs34857077, rs6497475, rs6497474, rs181820305, rs117774819, rs528859496, rs149233634, rs147946789, rs181711435, rs184483102, rs183590048, rs187620954, rs191065984, rs182744661, rs185578691, rs182605229, rs191171015, rs185630961, rs192280820, rs182188833, rs186260541, rs185128245, rs77225526, rs74011925, rs78052469, rs75864273, rs150052437, rs74011921, and rs372456426.

5. A method to discriminate a subject affected by a low grade RCC from a subject affected by a high grade RCC comprising measuring the level of at least one miRNA or a combination thereof in a biological sample of the subject and comparing said measured level to a reference level, wherein the at least one miRNA is indicated in Table 12 or Table 22 or is selected from the group consisting of: hsa-miR-744-5, hsa-miR-4306, hsa-miR-15a-5p ,hsa-miR-195-5p, hsa-miR-27b-3p, hsa-let-7i-5p, hsa-miR-103a-2-5p, hsa-miR-132-3p, hsa-miR-33a-5p, hsa-miR-629-5p, hsa-miR-664a-3p, hsa-miR-125a-5p, hsa-miR-424-3p, hsa-miR-125b-5p, hsa-miR-502-3p, hsa-miR-128-3p, hsa-miR-21-3p, hsa-miR-31-5p, hsa-miR-545-5p, hsa-miR-134-5p, hsa-miR-652-3, hsa-miR-339-3p, hsa-miR-369-3p, hsa-miR-7-1-3p, hsa-miR-483-5p, hsa-miR-222-3p, hsa-miR-548am-5p, hsa-miR-151a-5p, hsa-miR-126-5p, hsa-miR-579-3p, hsa-miR-501-5p, hsa-miR-424-5p, hsa-miR-144-3p, hsa-let-7d-3p, hsa-miR-125b-5p, hsa-miR-148a-3p, , hsa-miR-545-5p, hsa-miR-1296-5p, , hsa-miR-652-5p, hsa-miR-369-3p, hsa-miR-128-3p, hsa-miR-424-5p, hsa-miR-34a-5p, hsa-miR-32-3p, hsa-miR-202-5p, hsa-miR-410-3p, hsa-miR-584-5p, hsa-miR-1537-3p, hsa-miR-150-5p, hsa-miR-29b-3p, hsa-miR-590-5p, hsa-miR-340-5p, hsa-miR-505-5p, hsa-miR-433-3p, hsa-miR-29c-5p, and hsa-miR-15a-3p and wherein the combination is indicated in Table 18 or Table 27.

6. The method according to claim 5 further comprising determining the genotype of UMOD SNP rs13333226 or of any other UMOD SNPs in linkage disequilibrium with rs13333226 (preferably with a R² > 0.8) in any ethnic background, preferably said other UMOD SNP is selected from the group consisting of: rs7204775, rs7203642, rs4293393, rs4997081, rs28688991, rs28640218, rs13329952, rs13335818, rs34115067, rs60136849, rs9928936, rs35650857, rs34882080, rs71149135, rs34356953, rs11862974, rs6497476, rs11342813, rs7204342, rs76563024, rs80159442, rs79245268, rs77875418, rs80142672, rs190450490, rs59027126, rs58768874, rs118059635, rs138755971, rs189908977, rs55808501, rs28362063, rs13334589, rs9933330, rs12922822, rs12917707, rs28544423, rs9928003, rs12934320, rs36060036, rs71149134, rs12934455, rs34262842, rs35830321, rs71377648, rs9928757, and rs71384446.

7. A method to determine the presence of clinical metastasis in a RCC patient comprising measuring the level of at least one miRNA or a combination thereof in a biological sample of the subject and comparing said measured level to a reference level, wherein the at least one miRNA is indicated in Table 14 or is selected from the group consisting of: hsa-miR-197-3p, hsa-miR-3613-3p, hsa-miR-377-3p, hsa-miR-590-3p, hsa-miR-598-3p, hsa-miR-6803-3p hsa-miR-548l, hsa-miR-548k, hsa-miR-153-3p, hsa-miR-582-5p, hsa-miR-129, hsa-miR-410-3p, hsa-let-7g-3p, hsa-miR-484, hsa-miR-30e-3p, hsa-miR-376c-3p, hsa-miR-545-5p, hsa-miR-590-5p, hsa-miR-455-3p, hsa-let-7d-3p, hsa-miR-7-1-3p, hsa-miR-671-5p, hsa-miR-382-5p, hsa-miR-342-3p, hsa-miR-532-5p, hsa-miR-126-3p, hsa-miR-150-5p, hsa-let-7i-3p, hsa-miR-222-3p, and wherein the combination is indicated in Table 19.

8. The method according to claim 7 further comprising determining the genotype of UMOD SNPs rs71384446, rs9928757, rs60136849, rs34356953, rs12934320, rs12934455, rs28544423, rs13335818, rs4293393, rs28362063, rs13333226, rs13329952, rs7203642, rs28688991, and rs12917707 or of any other UMOD SNPs in linkage disequilibrium with them (preferably with a R² > 0.8) in any ethnic background, preferably rs12922822, rs9933330, rs13334589, rs35650857, rs34882080, rs9928003, rs34115067, rs9928936, rs36060036, rs71149135, rs71149134, rs34262842, rs35830321, rs7204775, rs4997081, rs28640218, rs71377648, rs9924088, rs9923990, rs9926225, rs369494277, rs111483946, rs112166439, rs188045848, rs111904030, rs113923200, rs149786037, rs554722277, rs113452476, rs111992415, rs377257136, rs114997829, rs544951514, rs113957052, rs111266260, rs192180621, rs12102292, rs116563128, rs145279202, rs9937393, rs150044343, rs7204342, rs80159442, rs11342813, rs11862974, rs6497476, rs76563024, rs79245268, rs77875418, rs80142672, rs190450490, rs59027126, rs58768874, rs118059635, rs138755971, rs189908977, rs55808501, rs372456426, rs6497475, rs34857077, rs77225526, rs74011925, rs78052469, rs75864273, rs150052437, rs74011921, rs181820305, rs4238595, rs117774819, rs528859496, rs149233634, rs147946789, rs181711435, rs184483102, rs183590048, rs187620954, rs191065984, rs182744661, rs185578691, rs182605229, rs191171015, rs185630961, rs192280820, rs182188833, rs186260541, rs185128245, rs8060932, and rs8062123.

9. A method to determine if a renal mass present in a subject is a benign or a malignant renal mass comprising measuring the level of at least one miRNA or a combination thereof in a biological sample of the subject and comparing said measured level to a reference level, wherein the at least one miRNA is i is selected from the group consisting of: hsa-miR-374a-5p, hsa-miR-103a-2-5p, hsa-miR-132-3p, hsa-miR-192-5p, hsa-miR-215-5p, hsa-miR-3613-3p, hsa-miR-590-3p hsa-miR-598-3p, hsa-miR-99a-3p, hsa-miR-1180-3p, hsa-miR-590-5p, hsa-miR-4286, hsa-miR-500a-5p, hsa-miR-382-5p, hsa-miR-30e-3p, hsa-miR-545-5p, hsa-miR-548l, hsa-miR-20b-5p, hsa-miR-193b-3p, hsa-miR-450a-5p, hsa-miR-410-3p, hsa-miR-433-3p, hsa-miR-221-5p, hsa-miR-331-5p, hsa-miR-181c-3p, hsa-miR-451a, hsa-miR-491-5p, hsa-miR-501-5p, hsa-miR-4732-5p, hsa-miR-485-3p, hsa-miR-335-5p, hsa-miR-1290, , hsa-miR-337-5p, hsa-miR-550a-3-5p, hsa-miR-1180-3p, hsa-miR-500a-5p, , hsa-miR-99a-3p, hsa-miR-130b-5p, hsa-miR-337-5p, hsa-miR-4732-5p, hsa-miR-374a-3p, hsa-miR-548l, hsa-miR-1277-3p, hsa-miR-29c-5p, hsa-miR-181b-5p, hsa-miR-433-3p, hsa-miR-12136, hsa-miR-628-3p, hsa-miR-19a-3p, hsa-miR-122-5p, hsa-miR-196b-5p, hsa-miR-491-5p, and hsa-miR-153-3p or is ndicated in Table 11 or Table 21 and wherein said combination is indicated in Table 16 or Table 25.

10. The method according to claim 1 further comprising determining the genotype of UMOD SNPs rs71384446, rs9928757, rs11647727, rs28640218, rs7193058, rs76563024, rs11862974, rs13329952, rs7203642, rs7204342, and rs28688991 or of any other UMOD SNPs in linkage disequilibrium with them (preferably with a R² > 0.8) in any ethnic background, preferably said other UMOD SNPs are selected from the group consisting of: rs7204775, rs4997081, rs12922822, rs12917707, rs9933330, rs13334589, rs13333226, rs28362063, rs4293393, rs35650857, rs34882080, rs13335818, rs28544423, rs9928003, rs12934320, rs34356953, rs34115067, rs60136849, rs9928936, rs36060036, rs71149134, rs12934455, rs34262842, rs35830321, rs71149135, rs71377648, rs80159442, rs11342813, rs6497476, rs79245268, rs77875418, rs80142672, rs190450490, rs59027126, rs58768874, rs118059635, rs138755971, rs189908977, rs55808501, rs74842838, rs143657120, rs145915865, rs75645968, rs114333799, rs114112267, rs75459600, rs76625281, rs76236484, rs4500734, rs58351658, rs4490010, rs147846395, rs34857077, rs6497475, rs7204210, rs6497474, rs8060932, rs8062123, and rs4238595.

11. A method to predict the presence of a renal mass in a subject comprising measuring the level of at least one miRNA or a combination thereof in a biological sample of the subject and comparing said measured level to a reference level, wherein the at least one miRNA is selected from the group consisting of: hsa-miR-374a-5p, hsa-miR-4306, hsa-miR-195-5p, hsa-miR-30b-5p, hsa-miR-197-3p, hsa-miR-378a-3p, hsa-miR-664a-3p, hsa-miR-6803-3p, hsa-miR-12136, hsa-miR-28-3p, hsa-miR-15b, hsa-miR-328-3p, hsa-miR-323a-3p, hsa-miR-550a-3-5p, hsa-miR-323b-3p, hsa-miR-326, hsa-miR-142-5p, hsa-miR-181a-5p, hsa-miR-92b-3p, hsa-miR-130a-3p, hsa-miR-1260b, , hsa-miR-1301-3p, hsa-miR-21-5p, , hsa-miR-377-3, hsa-miR-483-5p, , hsa-miR-181c-3p, hsa-miR-101-3p, hsa-miR-423-5p, hsa-miR-29a-3p, hsa-miR-7-5p, hsa-miR-328-3p, hsa-miR-28-3p, hsa-miR-361-5p, hsa-miR-1260b, hsa-miR-579-3p, hsa-miR-532-3p, hsa-miR-15b-3p, hsa-miR-7-5p, hsa-miR-550a-3-5p, hsa-miR-181c-3p, hsa-miR-202-5p, hsa-miR-423-5p, hsa-miR-1301-3p, hsa-miR-29c-5p, hsa-miR-326, hsa-miR-2110, hsa-miR-101-3p, hsa-miR-18a-5p, hsa-miR-130a-3p, hsa-miR-423-3p, hsa-miR-323b-3p, hsa-miR-128-3p, and hsa-miR-494-3p or is indicated in Table 10 or Table 20 and wherein the combination is indicated in Table 15 or Table 24.

12. The method according to claim 11 wherein the subject is affected by obesity, autosomal polycystic kidney disease, multicystic kidney disease, hypertension or diabetes or wherein the subject is a smoker or has a family history of kidney cancer.

13. The method according to any one of previous claim further comprising measuring the level of uromodulin in a biological sample of the subject and comparing said measured level to a reference level, preferably the biological sample is selected from the group consisting of: plasma, serum and urine sample.

14. The method according to any one of previous claim further comprising the analysis of a phenotypic characteristic, preferably selected from the group consisting of:
- clinical parameters, such as age at diagnosis/treatment, comorbidity index and specific comorbidities or history of previous renal or non-renal cancer;
- radiological parameters, such as clinical size defined as clinical diameter at axial imaging, tumor volume, clinical tumor stage, clinical nodal stage, PADUA score, RENAL score, ABC score or other nephrometric scores;
- biohumoral parameters, such as hemoglobin, platelets, creatinine, urea, blood electrolytes, total protein serum level and electrophoresis, measured and estimated glomerular filtration rate, proteinuria and urine tests.

15. The method according to any one of previous claim wherein the biological sample is selected from: blood, blood cells, serum, plasma, urine, saliva, sputum, tears, kidney tissue.

16. A kit for use in a method according to any one of previous claim comprising means to quantify said miRNA and control means and optionally comprising means to determine at least one UMOD genotype, preferably said kit comprises an array.
